# EUROPEAN PATENT APPLICATION

(11) **EP 4 389 751 A1**
(43) Date of publication of application: **26.06.2024**
(21) Application number: 23158856.7
(22) Date of filing: 03.09.2022
(51) Int. Cl.: C07D 487/04, C07D 498/10, C07D 519/00, C07D 417/14, C07D 487/08, A61P 35/00, A61K 31/517, A61K 31/519

(54) **HETEROCYCLIC COMPOUNDS AND USES THEREOF**

(30) Priority: 03.09.2021 US 202163240824 P; 04.05.2022 US 202263338383 P
(62) Divisional of application: 22851005.3
(71) Applicant: Kumquat Biosciences Inc., San Diego, CA 92121 (US)
(72) Inventor: LI, Xiaoming, San Diego, California, 92121 (US); LI, Liansheng, San Diego, California, 92121 (US); WU, Baogen, San Diego, California, 92121 (US); REN, Pingda, San Diego, California, 92121 (US); HANSEN, Rasmus, San Diego, California, 92121 (US); CHEN, Zhiyong, San Diego, California, 92121 (US); LIU, Yuan, San Diego, California, 92121 (US)
(74) Representative: Mewburn Ellis LLP

(57) **Abstract**

The present disclosure provides compounds and pharmaceutically acceptable salts thereof, and methods of using the same. The compounds and methods have a range of utilities as therapeutics, diagnostics, and research tools. In particular, the subject compositions and methods are useful for reducing signaling output of oncogenic proteins.

## Description

### CROSS-REFERENCE

This application claims the benefit of U.S. Provisional Patent Application Nos. 63/240,824 filed on September 3, 2021 and 63/338,383 filed on May 4, 2022, each of which is incorporated by reference in its entirety.

### BACKGROUND

Cancer (e.g., tumor, neoplasm, metastases) is the second leading cause of death worldwide estimated to be responsible for about 10 million deaths each year. Many types of cancers are marked with mutations in one or more proteins involved in various signaling pathways leading to unregulated growth of cancerous cells. In some cases, about 25 to 30 percent (%) of tumors are known to harbor Rat sarcoma (Ras) mutations. Ras proteins such as human H-Ras, K-Ras, and N-Ras are small GTPase proteins involved in signal transduction pathways that regulate diverse cellular behaviors. When Ras proteins are activated or switched on by upstream signals, they in turn activate downstream components of signal transductions pathways, culminating in dysregulated cellular activities responsible for abnormal cell growth, differentiation, and/or survival.

Various types of Ras mutations have been found to associate with a variety of cancers. For example, K-Ras mutations have been found in lung cancer (e.g., lung adenocarcinoma), mucinous adenoma, pancreatic cancer (e.g., ductal carcinoma of the pancreas), colorectal cancer, and leukemia. Mutation and dysregulation of the function of N-Ras are associated with different lung cancers and melanoma. H-Ras mutations have been found associated with head and neck cancer and other types of cancer as well.

Ras proteins have long been considered to be "undruggable," due to, in part, high affinity to their substrate Guanosine-5'-triphosphate (GTP) and/or their smooth surfaces without any obvious targeting region. Recently, a specific G12C Ras gene mutation has been identified as a potential druggable target. However, such therapeutic approach is still limiting, as the G12C mutation in Ras has a low prevalence rate (e.g., about 3% in pancreatic ductal adenocarcinoma) as compared to other known Ras mutations. Additional Ras mutations include G12D, G12V, and G12S, among others.

### SUMMARY

In view of the foregoing, there remains a considerable need for a new design of therapeutics and diagnostics that can specifically target Ras mutants and/or associated proteins of Ras to reduce Ras signaling output. Of particular interest are inhibitors of mutant Ras proteins such as Ras G12S, for the treatment of Ras-associated diseases (e.g., cancer). Such compositions and methods can be particularly useful for treating a variety of the diseases including, but not limited to, cancers and neoplasia conditions. The present disclosure addresses these needs, and provides additional advantages applicable for diagnosis, prognosis, and treatment for a wide diversity of diseases.

In an aspect is provided a modified Ras mutant protein comprising a compound covalently bonded to its serine residue, wherein the serine residue corresponds to position 12 of SEQ ID No: 1. In some embodiment, such covalently bonded modified Ras mutant protein exhibits a reduced Ras signaling output (e.g., compared to a corresponding unmodified Ras mutant absent of the covalently bonded compound). In some embodiments, a modified Ras mutant protein is a K-Ras G12S mutant, an H-Ras G12S mutant, or a N-Ras G12S mutant. In some embodiments, a modified Ras mutant protein comprises an amino acid sequence selected from the group consisting of SEQ ID No. 1, SEQ ID No. 3, SEQ ID No. 5, and a respective fragment thereof comprising the serine residue corresponding to position 12 of SEQ ID No: 1

In embodiments of a modified Ras mutant protein described herein, the reduced Ras signaling output is evidenced by one or more a reduced output selected from the group consisting of (i) an increase in steady state level of GDP-bound modified protein; (ii) a reduction of phosphorylated AKTs473, (iii) a reduction of phosphorylated ERK T202/Y204, (iv) a reduction of phosphorylated S6 S235/236, (v) reduction of cell growth of a tumor cell expressing a Ras G12S mutant protein, and (vi) reduction in Ras interaction with a Ras-pathway signaling protein

In embodiments the modified Ras mutant protein described herein is is formed by contacting a precursor compound with the serine residue of an unmodified Ras G12S mutant protein, wherein the precursor compound comprises a moiety susceptible to reacting with a nucleophilic serine residue corresponding to position 12 of SEQ ID No: 1. In some embodiments, the modified Ras mutant protein is formed by contacting a precursor compound with the serine residue of an unmodified Ras G12S mutant protein, wherein the precursor compound comprises a staying group and a leaving group, and upon contacting the precursor compound with an unmodified Ras G12S mutant protein, said leaving group separates from remainder of the precursor compound with an electron pair that previously formed a covalent bond between the leaving group and the remainder of the precursor compound. In some embodiments, the precursor compound comprises a staying group and a leaving group, and wherein said contacting results in release of the leaving group and formation of said modified protein.
In some embodiments, the precursor compound selectively labels the serine residue corresponding to position 12 of SEQ ID No. 1 (a G12S mutant) relative to an aspartate (G12D) or valine (G13V) residue at the same position. In some embodiments, the precursor compound selectively labels the serine residue as compared to (i) an aspartate residue of a K-Ras G12D mutant protein, said aspartate corresponding to residue 12 of SEQ ID NO: 7, and/or (ii) a valine residue of a K-Ras G12V mutant protein, said valine corresponding to residue 12 of SEQ ID NO: 8. In some embodiments, the precursor compound selectively labels the serine residue as compared to (i) an aspartate residue of a K-Ras G12D mutant protein, said aspartate corresponding to residue 12 of SEQ ID NO: 7, and/or (ii) a valine residue of a K-Ras G12V mutant protein, said valine corresponding to residue 12 of SEQ ID NO: 8, by at least 1, 2, 3, 4, 5, 10 folds or more, when assayed under comparable conditions.

In embodiments of the modified Ras mutant protein described herein, the precursor compound contacts the serine residue of an unmodified Ras G12S protein corresponding to position 12 of SEQ ID No: 1 in vitro.

In embodiments of the modified Ras mutant protein described herein, the precursor compound contacts the serine residue of an unmodified K-Ras G12S protein corresponding to position 12 of SEQ ID No: 1 in vivo.

In embodiments of the modified Ras mutant protein described herein, the covalently bonded compound comprises a staying group having a formula: 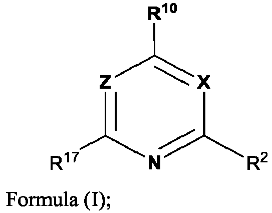 wherein
Z is N and X is C(R³); or Z is C(R⁸) and X is N;
R¹⁰ is -L⁷-R⁷;
   L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
   R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are substituted with one R⁶ directly bonded to a ring N and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20a};
   R⁶ is -C(O)-; wherein R⁶ is directly bonded to the K-Ras G12S serine corresponding to position 12 of SEQ ID No: 1;
   R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c};
   R¹⁷ is selected from -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
   L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
   R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
   R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋9heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R^{14c} is independently selected from C₁₋₆alkyl, and C₁₋₆haloalkyl;
   each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
   each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C_{1- 6}alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   ------ indicates a single or double bond such that all valences are satisfied.

In some embodiments of the modified Ras mutant protein described herein, the covalently bonded compound comprises a staying group having a formula: 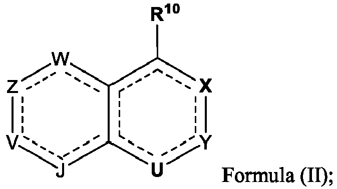 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
   L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
   R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are substituted with one R⁶ directly bonded to a ring N and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20a};
   R⁶ is -C(O)-; wherein R⁶ is directly bonded to the K-Ras G12S serine corresponding to position 12 of SEQ ID No: 1;
   R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R¹⁷ is selected from -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
   R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
   R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
   R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
   R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
   R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
   each R^{20a}, R^{20b}, R^{20c}, R^{20d}, R^{20e}, R^{20f}, R^{20g}, R^{20h}, and R²⁰ⁱ are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C_{1- 6}alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³) , and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   ------ indicates a single or double bond such that all valences are satisfied.

In embodiments of the modified Ras mutant protein described herein, the leaving group has a formula:
A) 5 membered partially unsaturated heterocycloalkyl or a 5 membered heteroaryl, each optionally substituted with one, two or three R^{20k}, wherein the partially unsaturated heterocycloalkyl or heteroaryl comprises two or three ring nitrogen atoms; or
B) 5 or 6 membered partially unsaturated heterocycloalkyl or a 5 or 6 membered heteroaryl, each optionally substituted with one, two or three R^{20k}, wherein the partially unsaturated heterocycloalkyl or heteroaryl comprises two or three ring nitrogen atoms; and further wherein the partially unsaturated heterocycloalkyl or heteroaryl is substituted with a -NH(R¹⁴) bonded to a ring carbon of the partially unsaturated heterocycloalkyl or heteroaryl.
   each R¹⁴ is independently selected from hydrogen, C₁₋₆alkyl, and C₁₋₆haloalkyl;
   each R^{20k} is independently selected from halogen, -CN, C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and-OC(O)R²⁵; or two R^{20k} bonded to the same or adjacent atoms may optionally be joined to form a C_{3- 6}cycloalkyl, C₂₋₉heterocycloalkyl, C₆₋₁₀aryl, -CH₂-C₆₋₁₀aryl, or C₁₋₉heteroaryl; wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from Hand C₁₋₆alkyl;
   each R²⁴ is independently selected from Hand C₁₋₆alkyl; and
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl.

In embodiments of the modified Ras mutant protein described herein, the leaving group has a formula: 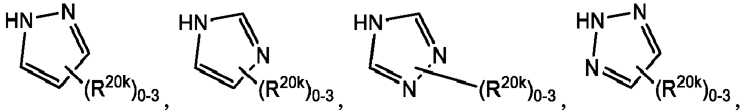 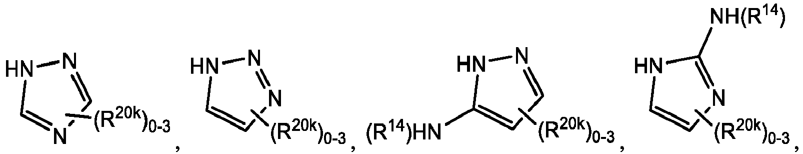 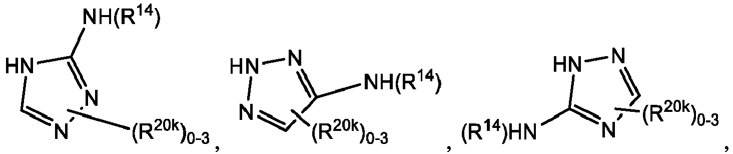 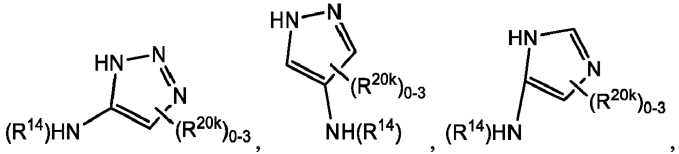 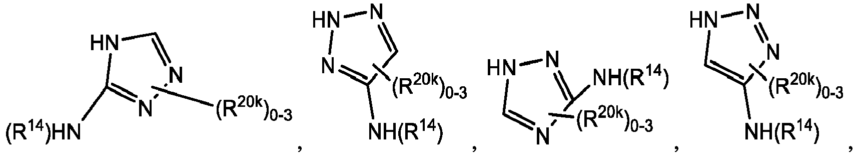 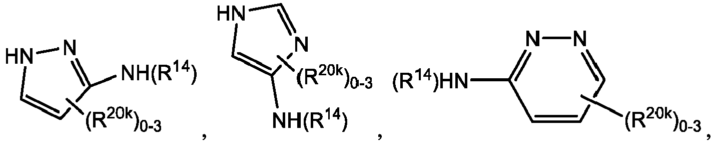 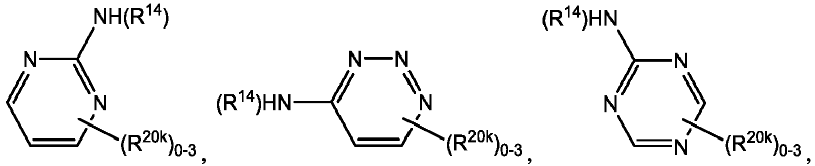 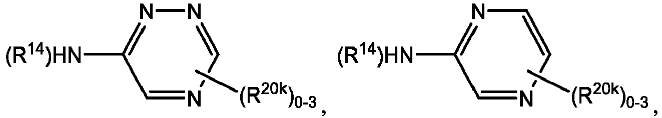 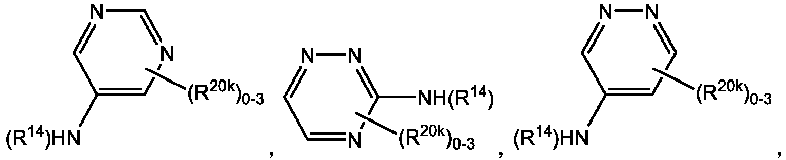 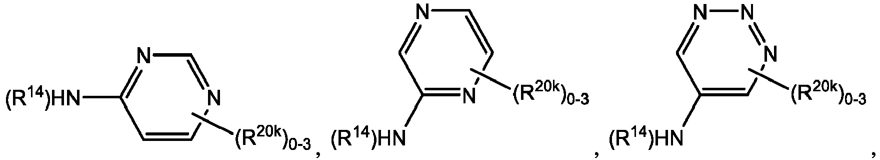 or 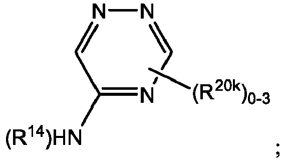
each R¹⁴ is independently selected from hydrogen, C₁₋₆alkyl, and C₁₋₆haloalkyl;
each R^{20k} is independently selected from halogen, -CN, C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and-OC(O)R²⁵; or two R^{20k} bonded to the same or adjacent atoms may optionally be joined to form a C_{3- 6}cycloalkyl, C₂₋₉heterocycloalkyl, C₆₋₁₀aryl, -CH₂-C₆₋₁₀aryl, or C₁₋₉heteroaryl; wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl. and C₁₋₉heteroaryl;
each R²³ is independently selected from Hand C₁₋₆alkyl;
each R²⁴ is independently selected from Hand C₁₋₆alkyl; and
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl.

In an aspect is provided a complex of formula: 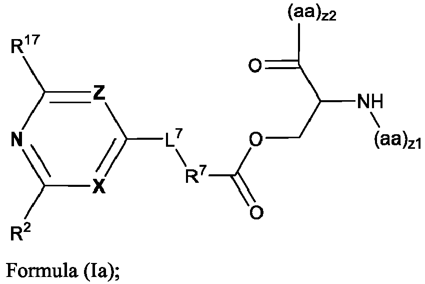 wherein
each aa is independently an amino acid;
z1 is an integer equal to at least 1 or (aa)z1 is hydrogen;
z2 is an integer equal to at least 1 or (aa)z2 is OH;
Z is N or C(R⁸);
X is C(R³) or N;
L⁷ is a bond, -O-, -N(R^{14c})-, -C(O)-, -S-, -S(O)₂-, -S(O)-, or C₁₋₄alkyl, wherein the C₁₋₄alkyl is optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴ and further wherein R⁷ is bonded to C(O) in Formula (Ia) through a ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is selected from -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹;
each R¹¹ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
   R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R^{14c} is independently selected from C₁₋₆alkyl, and C₁₋₆haloalkyl;
   each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f};
   each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from Hand C₁₋₆alkyl;
   each R²⁴ is independently selected from Hand C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   ------ indicates a single or double bond such that all valences are satisfied.

In some embodiments of the complex of Formula (Ia), R⁷ is a 3-12 membered nitrogen containing heterocycloalkyl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴.

In some embodiments of the complex of Formula (Ia),
Z is N and X is C(R³); or Z is C(R⁸) and X is N;
L⁷ is a bond, -O-, -N(R^{14c})-, -C(O)-, -S-, -S(O)₂-, -S(O)-, or C₁₋₄alkyl, wherein the C₁₋₄alkyl is optionally substituted with one, two or three R^{20a};
R⁷ is a 6-12 membered nitrogen containing heterocycloalkyl or 5-12 membered nitrogen containing heteroaryl, wherein the 6-12 membered nitrogen containing heterocycloalkyl or 5-12 membered nitrogen containing heteroaryl are optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₂₋₆alkyl, C₂₋₆alkenyl, C₂₋₆alkynyl, C₃₋₆cycloalkyl, -OR^{12c}, -SR¹², -N(R^{12c})(R^{12c}), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₂₋₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₂₋₆alkyl, C_{2- 6}alkenyl, C₂₋₆alkynyl, and C₃₋₁₀cycloalkyl, are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
   each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R^{14c} is independently selected from C₁₋₆alkyl, and C₁₋₆haloalkyl;
   each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
   each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from Hand C₁₋₆alkyl;
   each R²⁴ is independently selected from Hand C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   ------ indicates a single or double bond such that all valences are satisfied.

In some embodiments of the complex of Formula (Ia),
Z is N and X is C(R³); or Z is C(R⁸) and X is N;
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 7-12 membered nitrogen containing spirocyclic heterocycloalkyl optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
   R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f},
   each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from Hand C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   - - - - - - indicates a single or double bond such that all valences are satisfied.

In some embodiments of the complex of Formula (Ia),
Z is N and X is C(R³); or Z is C(R⁸) and X is N;
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 7-12 membered nitrogen containing fused heterocycloalkyl, 7-12 membered nitrogen containing fused aryl, or 7-12 membered nitrogen containing fused heteroaryl optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, -OR¹², -SR¹², -N(R^{12c})(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, or -(C₃₋₁₀cycloalkyl)-R^{12b} wherein said C₁₋₆alkyl, C₂₋₆alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f},
each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (Ia), R⁷ is a 7-12 membered nitrogen containing fused heterocycloalkyl or 7-12 membered nitrogen containing fused heteroaryl optionally substituted with one or more R¹ and/or one or more R⁴.

In some embodiments of the complex of Formula (Ia),
Z is N and X is C(R³); or Z is C(R⁸) and X is N;
L⁷ is a bond, -C(O)-, -S-, -S(O)₂-, -S(O)-, or C₁₋₄alkyl, wherein the C₁₋₄alkyl is optionally substituted with one, two or three R^{20a};
R⁷ is a -(4-6 membered nitrogen containing saturated heterocycloalkyl)-(4-6 membered nitrogen containing saturated heterocycloalkyl)-, wherein the -(4-6 membered nitrogen containing saturated heterocycloalkyl)-(4-6 membered nitrogen containing saturated heterocycloalkyl)- is optionally substituted with one or more R¹ and/or one or more R⁴; and further wherein the two -(4-6 membered nitrogen containing saturated heterocycloalkyl are joined by a direct bond between a ring C and ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
   R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; wherein one of R² and R¹⁷ is not hydrogen;
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f};
   each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(0)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from Hand C₁₋₆alkyl;
   each R²⁴ is independently selected from Hand C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   ------ indicates a single or double bond such that all valences are satisfied.

In an aspect is provided a complex of formula: 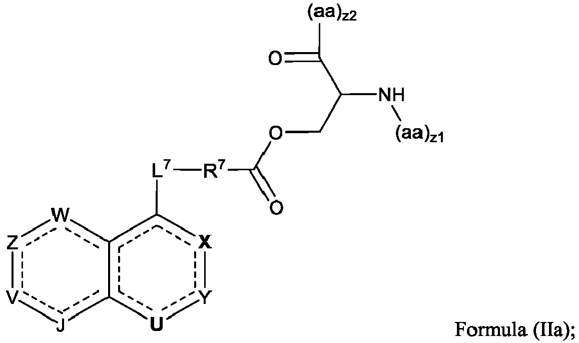 wherein
each aa is independently an amino acid;
z1 is an integer equal to at least 1 or (aa)z1 is hydrogen;
z2 is an integer equal to at least 1 or (aa)z2 is OH;
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴ and further wherein R⁷ is bonded to C(O) in Formula (IIa) through a ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH_{Z}-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is selected from -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{17b} is selected from -L^{1b}-R¹⁹, hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R ¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹;
each R¹¹ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -{C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In some embodiments of the complex of Formula (IIa), R⁷ is a 3-12 membered nitrogen containing heterocycloalkyl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴.

In some embodiments of the complex of Formula (IIa),
W is a bond;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (IIa),
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C₂₋₉ heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20e};
R¹⁸ and R^{18a} are independently selected from hydrogen, halogen, -CN, C₁₋₆alkyl, C₂₋₆alkenyl, C2-6alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (IIa),
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 7-12 membered nitrogen containing spirocyclic heterocycloalkyl optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20e};
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹;
each R¹¹ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C2-6alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20e};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (IIa),
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 7-12 membered nitrogen containing fused heterocycloalkyl, 7-12 membered nitrogen containing fused aryl, or 7-12 membered nitrogen containing fused heteroaryl optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋ioaryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)2N(1²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH2-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (IIa), R⁷ is a 7-12 membered nitrogen containing fused heterocycloalkyl or 7-12 membered nitrogen containing fused heteroaryl optionally substituted with one or more R¹ and/or one or more R⁴.

In some embodiments of the complex of Formula (IIa),
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b});
Y and U are independently selected from N, C(R²), C(R²)(R²), N(R^{2b}), S(O), S(O)₂, and C(O);
L⁷ is a bond, -C(O)-, -S-, -S(O)₂-, -S(O)-, or C₁₋₄alkyl, wherein the C₁₋₄alkyl is optionally substituted with one, two or three R^{20a};
R⁷ is a -(4-6 membered nitrogen containing saturated heterocycloalkyl)-(4-6 membered nitrogen containing saturated heterocycloalkyl)-, wherein the -(4-6 membered nitrogen containing saturated heterocycloalkyl)-(4-6 membered nitrogen containing saturated heterocycloalkyl)- is optionally substituted with one or more R¹ and/or one or more R⁴; and further wherein the two -(4-6 membered nitrogen containing saturated heterocycloalkyl are joined by a direct bond between a ring C and Ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is independently hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀ cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R'^{2b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH2-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R2⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In embodiments of the complex described herein, (aa)zl is MTEYKLVWGA- and (aa)z2 is - GVGKSALTIQLIQNHFVDEYDPTIEDSYRKQWIDGETCLLDILDTAGQEEYSAMRDQYMRTGEGF LCVFAINNTKSFEDIHHYREQIKRVKDSEDVPMVLVGNKCDLPSRTVDTKQAQDLARSYGIPFIETS AKTRQGVDDAFYTLVREIRKHKEKMSKDGKKKKKKSKTKCVIM.

In embodiments of the complex described herein, (aa)zl is MTEYKLVWGA- and (aa)z2 is - GVGKSALTIQLIQNHFVDEYDPTIEDSYRKQWIDGETCLLDILDTAGQEEYSAMRDQYMRTGEGF LCVFAINNTKSFEDIHQYREQIKRVKDSEDVPMVLVGNKCDLPSRTVDTKQAQDLARSYGIPFIETS AKTRQGVDDAFYTLVREIRKHKEKMSKDGKKKKKKSKTKCVIM.

In embodiments of the complex described herein, (aa)zl is MTEYKLVWGA- and (aa)z2 is - GVGKSALTIQLIQNHFVDEYDPTIEDSYRKQWIDGETCLLDILDTAGQEEYSAMRDQYMRTGEGF LCVFAINNTKSFEDIHLYREQIKRVKDSEDVPMVLVGNKCDLPSRTVDTKQAQDLARSYGIPFIETS AKTRQGVDDAFYTLVREIRKHKEKMSKDGKKKKKKSKTKCVIM.

In an aspect is provided a precursor compound of Formula (Ib), or a pharmaceutically acceptable salt or solvate thereof: 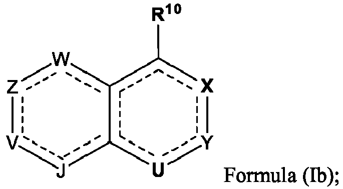 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
   (A) L² is -C(O)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b-}R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O- , N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ.
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), _ N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and-C(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a precursor compound of Formula (Ic), or a pharmaceutically acceptable salt or solvate thereof: 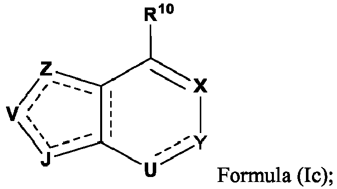 wherein
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
   (A) L² is -C(O)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁-₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a precursor compound of Formula (Id), or a pharmaceutically acceptable salt or solvate thereof: 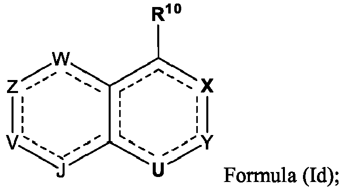 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 7-12 membered nitrogen containing spirocyclic heterocycloalkyl substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵ , -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
   (A) L² is -C(O)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl. and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl. -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In an aspect is provided a precursor compound of Formula (Ie), or a pharmaceutically acceptable salt or solvate thereof: 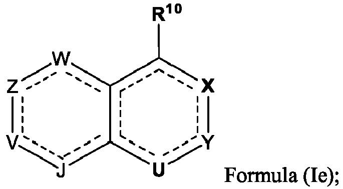 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R²), C(R^{2c})(R^{2c}), N(R^{2b}), S(0), S(O)₂, or C(O);
R¹⁰ is -L⁷-R⁷;
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 7-12 membered nitrogen containing fused heterocycloalkyl, 7-12 membered nitrogen containing fused aryl, or 7-12 membered nitrogen containing fused heteroaryl substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is independently selected from a -C(O)- and -C(O)N(R¹⁴)-;
   (A) L² is -C(O)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (iii) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (iv) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In embodiments (e.g., of a precursor compound of Formula Ie) exactly one of V and J is substituted with R¹⁷ or R^{17b}.

In embodiments, R⁷ is a 7-12 membered nitrogen containing fused heterocycloalkyl or 7-12 membered nitrogen containing fused heteroaryl substituted with one R⁶ directly bonded to a ring N and optionally substituted with one or more R¹ and/or one or more R⁴.

In an aspect is provided a precursor compound of Formula (II), or a pharmaceutically acceptable salt or solvate thereof: 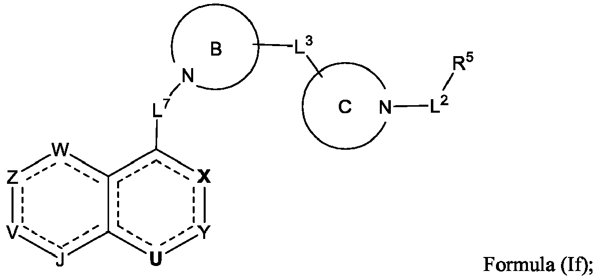 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y and U are independently selected from N, C(R²), C(R²)(R²), N(R^{2b}), S(O), S(O)₂, and C(O);
L⁷ is a bond, -C(O)-, -S-, -S(O)₂-, -S(O)-, or C₁₋₄alkyl, wherein the C₁₋₄alkyl is optionally substituted with one, two or three R^{20a};
Ring B is a 3-12 membered nitrogen containing saturated heterocycloalkyl optionally substituted with one or more R¹ and/or one or more R⁴;
Ring C is a 3-12 membered nitrogen containing saturated heterocycloalkyl optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
   (A) L² is -C(O)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is independently hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In embodiments (e.g., a precursor compound of Formula (If) exactly one of V and J is substituted with R¹⁷ or R^{17b}.

In an aspect is provided a precursor compound of Formula (Ih), or a pharmaceutically acceptable salt or solvate thereof: 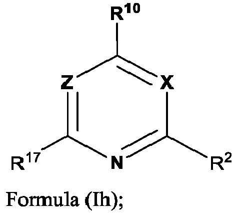 wherein
Z is N and X is C(R³); or Z is C(R⁸) and X is N;
R¹⁰ is -L⁷-R⁷;
L⁷ is a bond, -O-, -N(R^{14c})-, -C(O)-, -S-, -S(O)₂-, -S(O)-, or C₁₋₄alkyl, wherein the C₁₋₄alkyl is optionally substituted with one, two or three R^{20a};
R⁷ is a 6-12 membered nitrogen containing heterocycloalkyl or 5-12 membered nitrogen containing heteroaryl, wherein the 6-12 membered nitrogen containing heterocycloalkyl or 5-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
   (A) L² is -C(0)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₂₋₆alkyl, C₂₋₆alkenyl, C₂₋₆alkynyl, C₃₋₆cycloalkyl, -OR^{12c}, -SR¹², -N(R^{12c})(R^{12c}), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₂₋₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₂₋₆alkyl, C_{2- 6}alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH2-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl. -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from CI-6alkyl, and C₁₋₆haloalkyl;
each R¹⁵ is independently selected C₁₋₆alkyl, C2-6alkenyl, C2-6alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a precursor compound of Formula (Ii), or a pharmaceutically acceptable salt or solvate thereof: 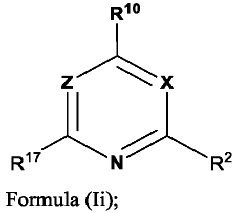 wherein
Z is N and X is C(R³); or Z is C(R⁸) and X is N;
R¹⁰ is -L⁷-R⁷;
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 7-12 membered nitrogen containing spirocyclic heterocycloalkyl substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
   (A) L² is -C(O)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};.
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloalkyl, C₆₋₁₀aryl, -CH₂-C₉₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20b}, R²⁰ⁱ, and R^{20k} is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
= indicates a single or double bond such that all valences are satisfied.

In an aspect is provided a precursor compound of Formula (Ij), or a pharmaceutically acceptable salt or solvate thereof: 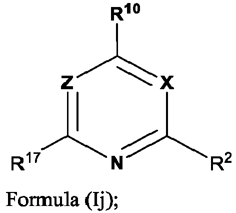 wherein
Z is N and X is C(R³); or Z is C(R⁸) and X is N;
R¹⁰ is -L⁷-R⁷;
L7 is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
R⁷ is a 7-12 membered nitrogen containing fused heterocycloalkyl, fused 7-12 membered nitrogen aryl, or fused 7-12 membered nitrogen heteroaryl substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
   (A) L² is -C(O)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R ¹⁵ , -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹¹;
each R¹¹ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, -OR¹², -SR¹², -N(R^{12c})(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, or -(C₆₋₁₀aryl)-R^{12b}, wherein said C₁₋₆alkyl, C₂₋₆alkenyl, C₂₋₆alkynyl, and C₃₋₁₀cycloalkyl, are optionally substituted with one, two, or three R^{20d};
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂-₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₁-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f},
each R^{20a}, R^{20b}, R^{20c}, R^{20d}, R^{20e}, R^{20f}, R^{20g}, R^{20b}, R²⁰ⁱ, and R^{20k} is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
= indicates a single or double bond such that all valences are satisfied.

In embodiments, R⁷ is a 7-12 membered nitrogen containing fused heterocycloalkyl or fused 7-12 membered nitrogen heteroaryl substituted with one R⁶ directly bonded to a ring N and optionally substituted with one or more R¹ and/or one or more R⁴.

In an aspect is provided a precursor compound of Formula (Ik), or a pharmaceutically acceptable salt or solvate thereof: 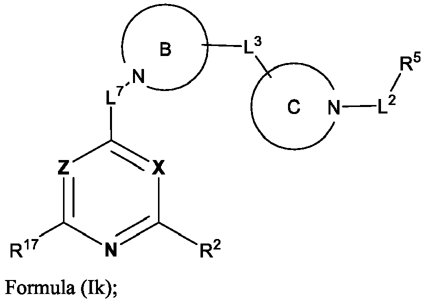 wherein
Z is N and X is C(R³); or Z is C(R⁸) and X is N;
L⁷ is a bond, -C(O)-, -S-, -S(O)₂-, -S(O)-, or C₁₋₄alkyl, wherein the C₁₋₄alkyl is optionally substituted with one, two or three R^{20a};
Ring B is a 4-6 membered nitrogen containing saturated heterocycloalkyl optionally substituted with one or more R¹ and/or one or more R⁴;
Ring C is a 4-6 membered nitrogen containing saturated heterocycloalkyl optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
   (A) L² is -C(O)- or -C(O)N(R¹⁴)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R¹⁴) of L² when L² is -C(O)N(R¹⁴)-; or
   (B) L² is -C(O)- and each R⁵ is selected from:
      (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is (a) bonded through an R⁵ ring nitrogen to L²;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹¹;
each R¹¹ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; wherein one of R² and R¹⁷ is not hydrogen;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloallcyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}; R^{20g}, R^{20f}; and R^{20k} is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
= indicates a single or double bond such that all valences are satisfied.

In some embodiments, R¹⁷ is -L¹-R¹⁹.

In embodiments, R¹⁹ is a bicyclic C₂₋₁₂heteroaryl or a fused ring C₂₋₁₂heteroaryl, wherein the bicyclic C_{2- 12}heteroaryl and fused ring C₂₋₁₂heteroaryl are optionally substituted with one, two, or three R²⁰ⁱ.

In some embodiments, R¹⁹ is selected from: 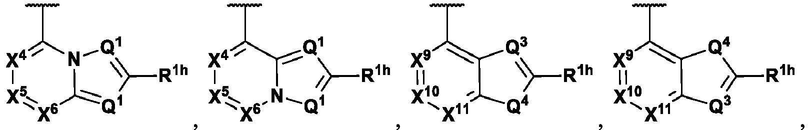 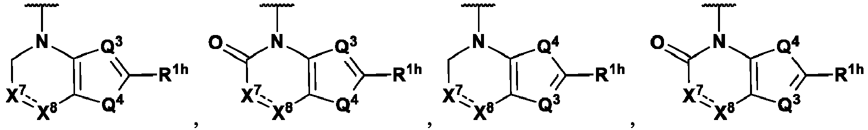 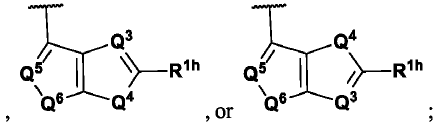
Q¹, Q³, and Q⁵ are independently N or C(R^{1d});
Q⁴ and Q⁶ are independently 0, S, C(R^{1a})(R^{1b}), or N(R^{1c});
X⁴, X⁵, X⁶, X⁹, X¹⁰, and X¹¹ are independently selected from C(R^{1a}) and N;
X⁷ and X⁸ are independently selected from C(R^{1a}), C(R^{1a})(R^{1b}), N, and N(R^{1c});
each R^{1a}, R^{1b}, R^{1a}, R^{1f}, R^{1g}, and R^{1h} are independently selected from hydrogen, halogen, -CN, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1a} and R^{1b} bonded to the same carbon are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; or two R^{1a} bonded to adjacent atoms are joined to form a 4-7 membered heterocycloalkyl ring, a phenyl ring, a 5-6 membered heteroaryl ring, or a 4-7 membered cycloalkyl ring, wherein the 4-7 membered heterocycloalkyl ring, phenyl ring, 5-6 membered heteroaryl ring, or 4-7 membered cycloalkyl ring are optionally substituted with one, two, or three R²⁰ⁱ; or R^{1h} and one of R^{1a}, R^{1b}, R^{1c}, and R^{1d} bonded to adjacent atoms are joined to form a 4-7 membered heterocycloalkyl ring, a phenyl ring, a 5-6 membered heteroaryl ring, or a 4-7 membered cycloalkyl ring, wherein the 4-7 membered heterocycloalkyl ring, phenyl ring, 5-6 membered heteroaryl ring, or 4-7 membered cycloalkyl ring are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; and
each R^{1c} is independently selected from hydrogen, C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ.

In embodiments, R¹⁹ is: 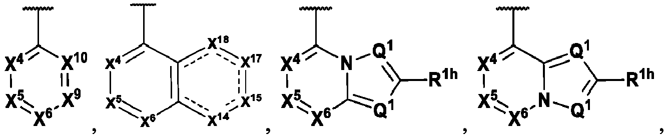 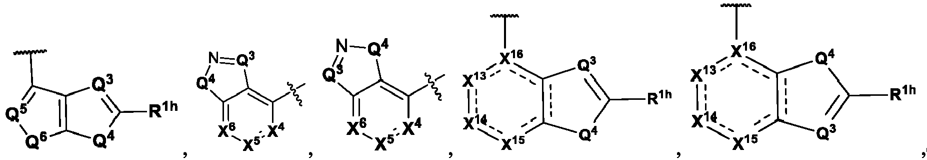 or 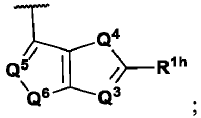
Q¹, Q³, and Q⁵ are independently selected from N and C(R^{1d});
Q⁴ and Q⁶ are independently selected from 0, S, C(R^{1a})(R^{1b}), and N(R^{1c});
X⁴, X⁵, X⁶, X⁹, X¹⁰ are independently selected from C(R^{1a}) and N;
X¹³ is selected from a bond, C(R^{1a}), N, C(O), C(R^{1a})(R^{1b}), C(O)C(R^{1a})(R^{1b}), C(R^{1a})(R^{1b})C(R^{1a})(R^{1b}), C(R^{1a})(R^{1b})N(R^{1c}), and N(R^{1c});
X¹⁴, X¹⁵, X¹⁷, X¹⁸ are independently selected from a C(O), C(R^{1a}), N, C(R^{1a})(R^{1b}), and N(R^{1c});
X¹⁶ are independently selected from C, N, and C(R^{1a});
each R^{1a}, R^{1b}, R^{1a}, and R^{1h} are each independently selected from hydrogen, halogen, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ; or R^{1a} and R^{1b} bonded to the same carbon are joined to form a 3-10 membered heterocycloalkyl ring or a C₃₋₁₀cycloalkyl ring, wherein the 3-10 membered heterocycloalkyl ring or C₃₋₁₀cycloalkyl ring are optionally substituted with one, two, or three R²⁰ⁱ; or two R^{1a} bonded to adjacent atoms are joined to form a 3-10 membered heterocycloalkyl ring, a C₆₋₁₀aryl ring, a 5-12 membered heteroaryl ring, or a C₃₋₁₀cycloalkyl ring, wherein the 3-10 membered heterocycloalkyl ring, C₆₋₁₀aryl ring, 5-12 membered heteroaryl ring, or C₃₋₁₀cycloalkyl ring are optionally substituted with one, two, or three R²⁰ⁱ; or R^{1h} and one of R^{1a}, R^{1b}, R^{1c}, and R^{1d} bonded to adjacent atoms are joined to form a 3-10 membered heterocycloalkyl ring, a C₆₋₁₀aryl ring, a 5-12 membered heteroaryl ring, or a C₃₋₁₀cycloalkyl ring, wherein the 3-10 membered heterocycloalkyl ring, a C₆₋₁₀aryl ring, a 5-12 membered heteroaryl ring, and C_{3- 10}cycloalkyl ring are optionally substituted with one, two, or three R²⁰ⁱ; and
each R^{1c} is independently selected from hydrogen, C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ.

In embodiments, R¹⁹ is selected from: 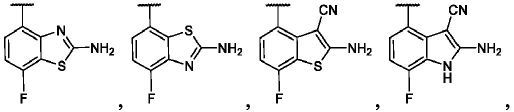 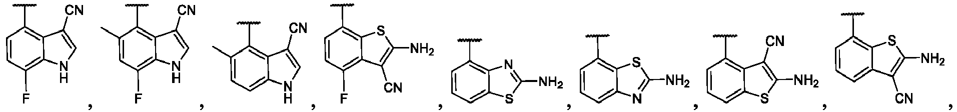 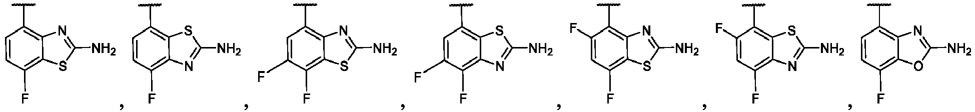 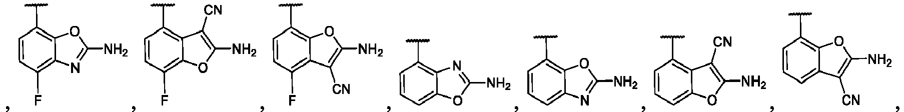 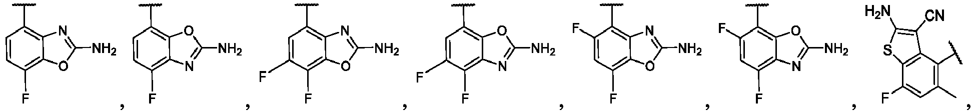 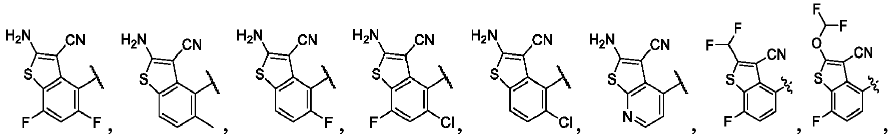 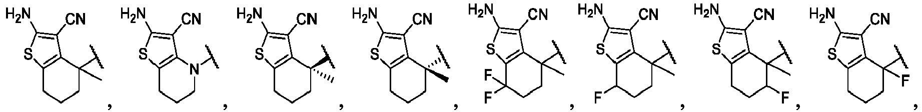 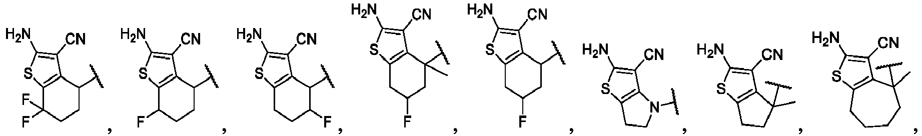 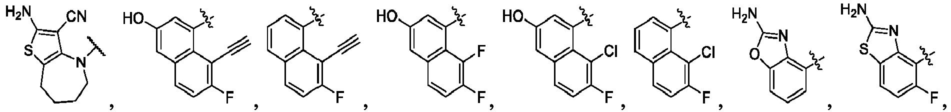 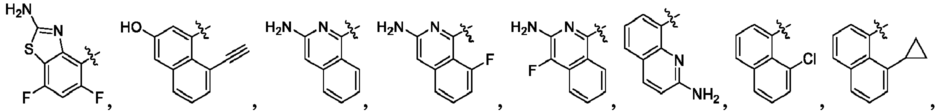 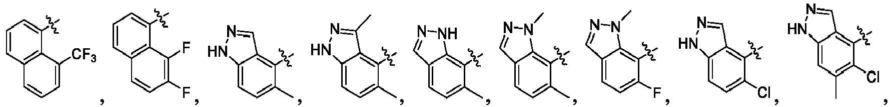 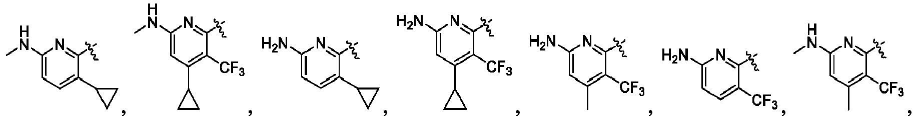 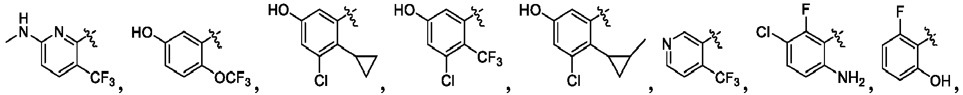 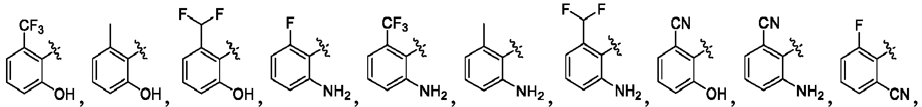 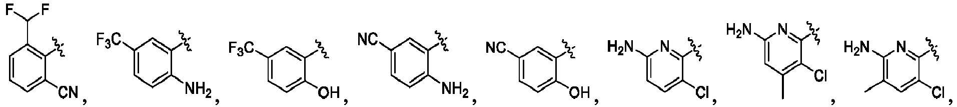 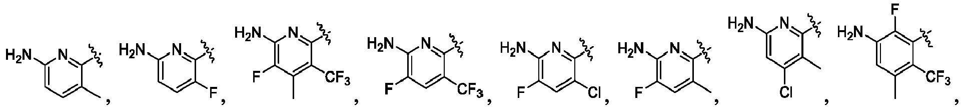 and

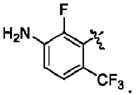

In some embodiments the complex or precursor compound is substituted with one or two independently selected R¹.

In some embodiments, R¹ is independently C₁₋₆alkyl optionally substituted with one, two, or three R^{20a}.

In some embodiments, R¹ is independently unsubstituted C₁₋₆alkyl.

In some embodiments, R¹ is independently -C₁₋₆alkyl-CN.

In some embodiments, the complex or precursor compound is not substituted with R¹.

In some embodiments, the complex or precursor compound is substituted with one or two independently selected R⁴.

In some embodiments, R⁴ is independently halogen or C₁₋₆alkyl optionally substituted with one, two, or three R^{20a}.

In some embodiments, R⁴ is independently unsubstituted C₁₋₆alkyl, C₁₋₆alkyl-CN, or halogen.

In some embodiments, the complex or precursor compound is not substituted with R⁴.

In some embodiments, R² is selected from

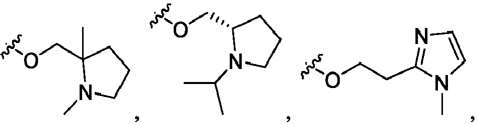

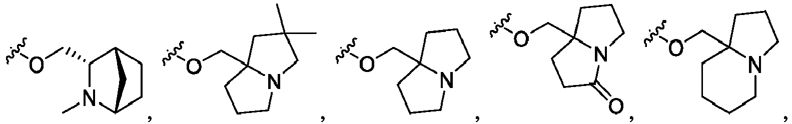

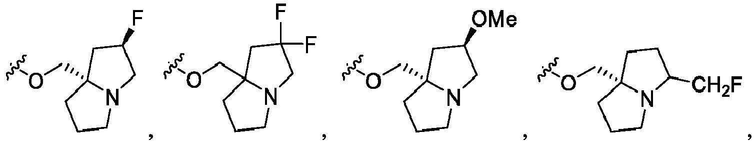

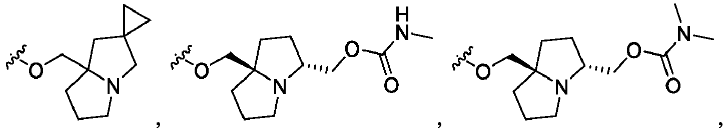

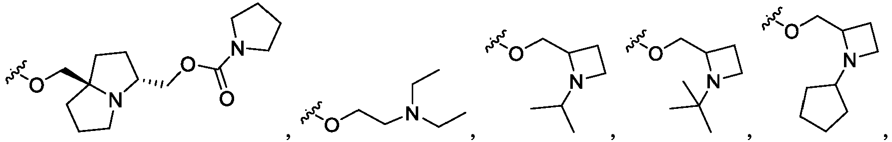

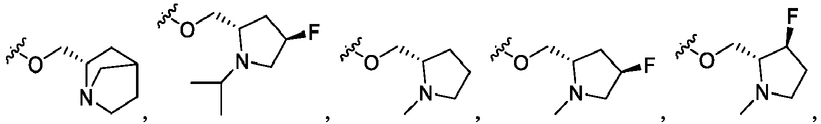

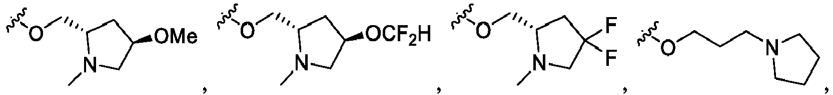

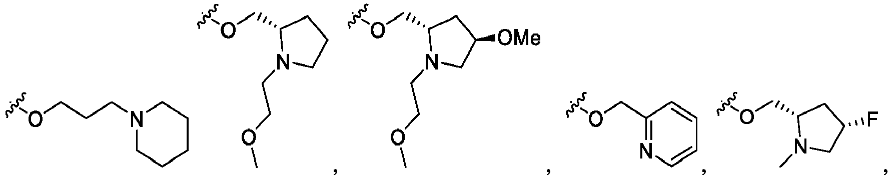

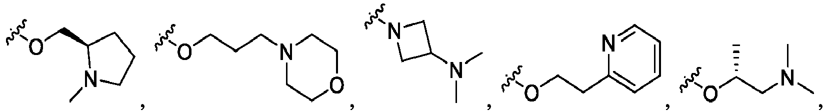

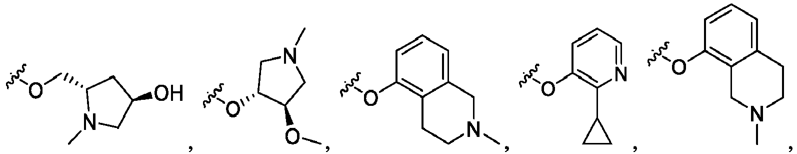

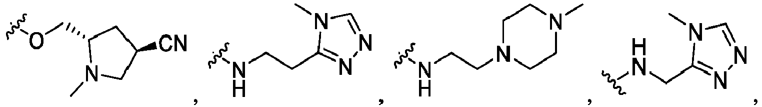

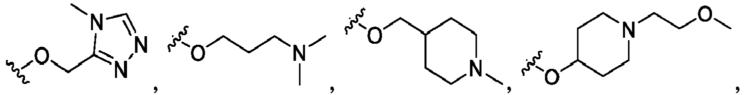

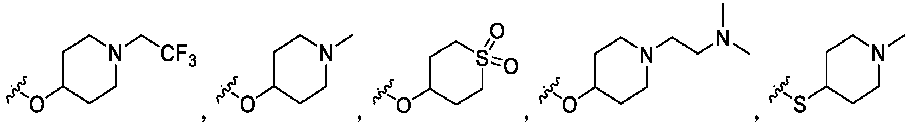

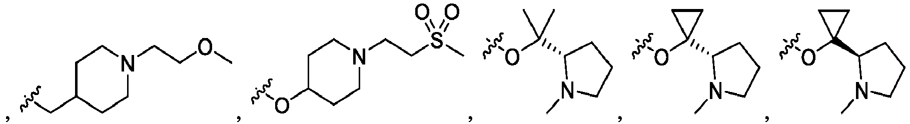

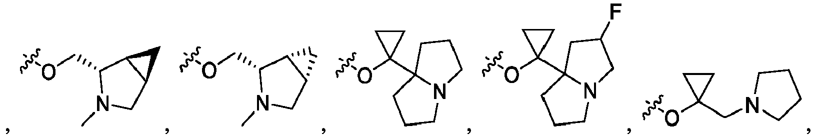

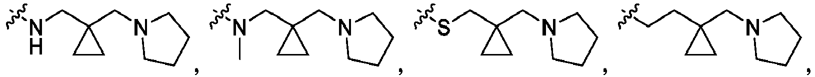

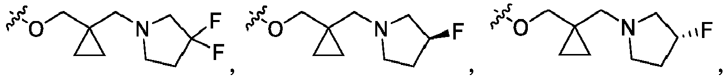

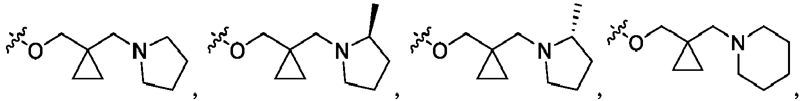

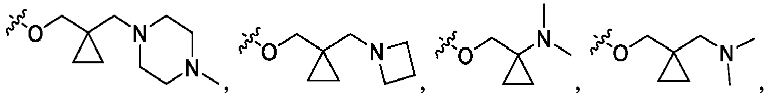

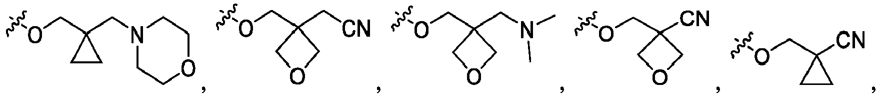

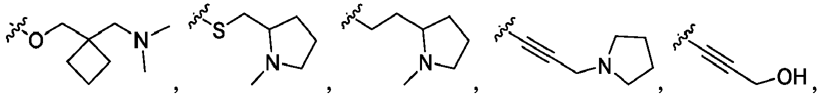

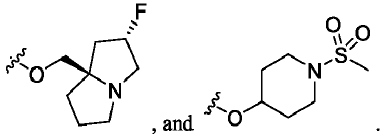

In embodiments, R² is selected from

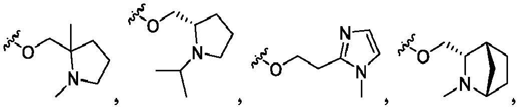

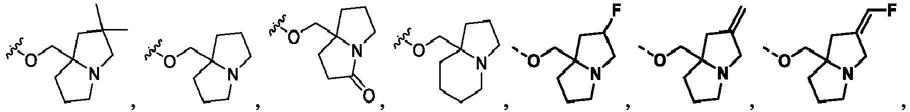

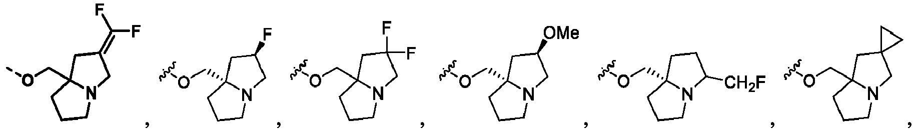

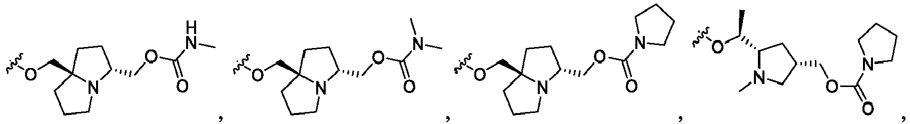

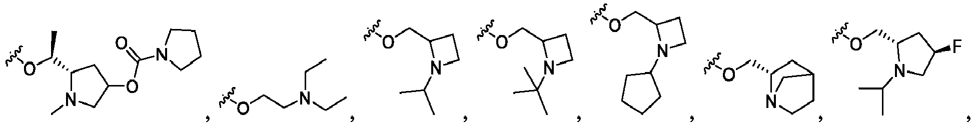

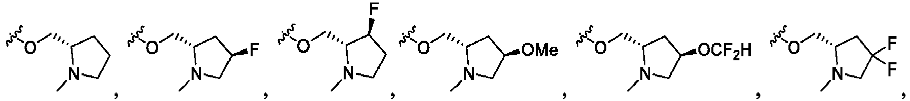

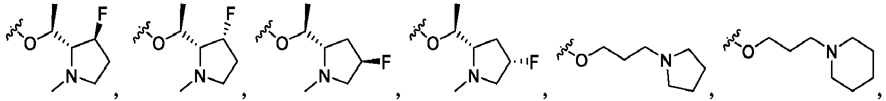

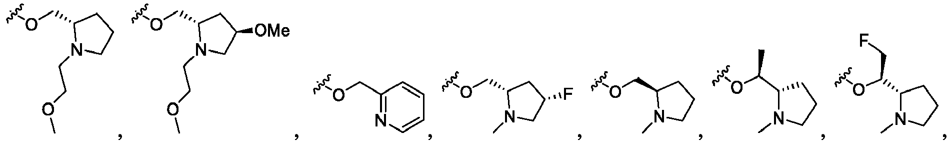

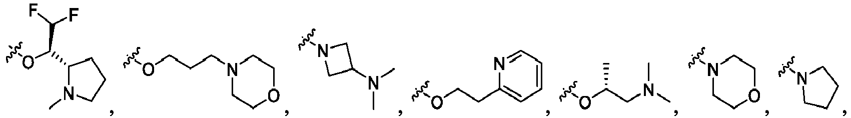

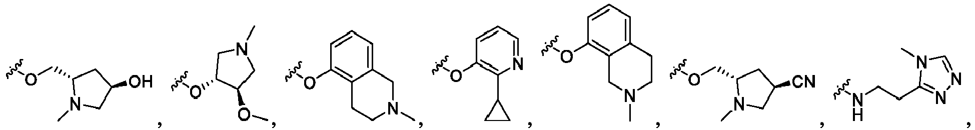

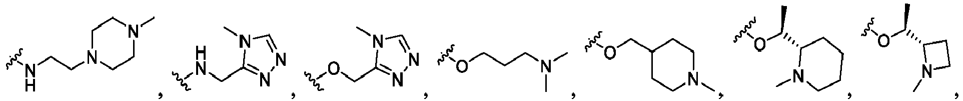

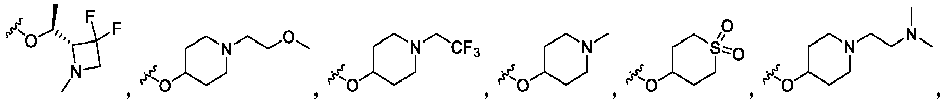

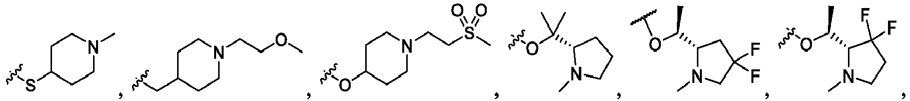

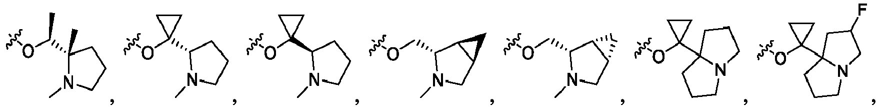

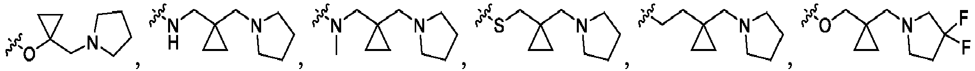

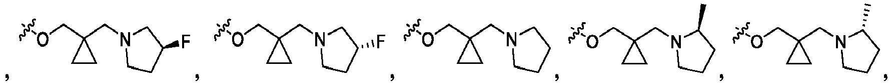

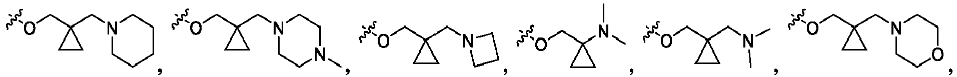

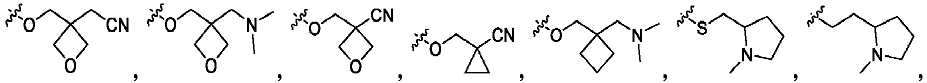

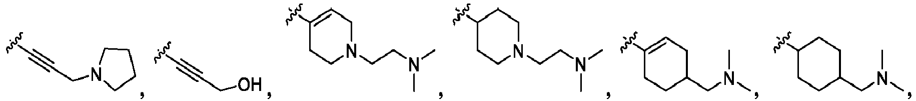

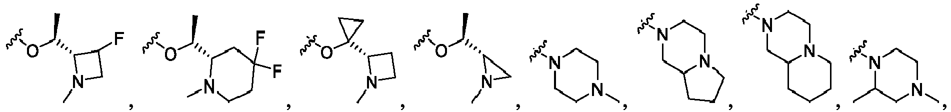

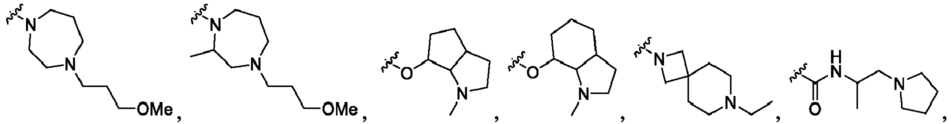

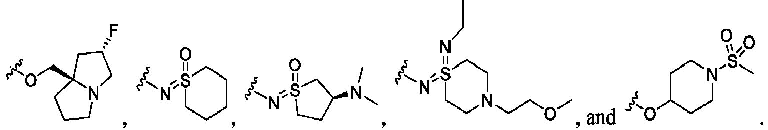

In an aspect is provided a modified Ras mutant protein comprising a compound covalently bonded to its serine residue, wherein the serine residue corresponds to position 12 of SEQ ID No: 1, wherein the covalently bonded compound comprises a staying group having a formula:

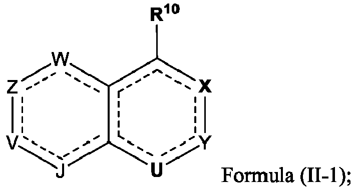

Wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), O, C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C₁₋₃alkyl and C₁₋₄alkyl are optionally substituted with one, two or three R^{20a}; R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are substituted with one R⁶, and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -C₀₋₃alkyl-C(O)-, -C₀₋₃alkyl-N(R¹²)C(O)-, -C₀₋₃alkyl-S(O)-, -C_{0- 3}alkyl-N(R¹²)S(O)-, -C₀₋₃alkyl-S(O)₂-, or -C₀₋₃alkyl-N(R¹²)S(O)₂-; wherein R⁶ is directly bonded to the serine residue corresponding to position 12 of SEQ ID No: 1; R⁸ and R^{8a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, hydrogen, halogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{17b} is -L^{1b}-R¹⁹, hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, - S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹; each R¹¹ is independently selected from halogen, oxo, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; each R² is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; each R^{12d} is independently selected from hydrogen, C₁₋₆alkyl, C₂₋₆alkenyl, C₂₋₆alkynyl, C₃₋₁₀cycloalkyl, -C(R^{12e})₂-C₃₋₁₀cycloalkyl, C₂₋₉heterocycloalkyl, -C(R^{12e})₂-C_{2- 9}heterocycloalkyl, C₆₋₁₀aryl, -C(R^{12e})₂-C₆₋₁₀aryl, -C(R^{12e})₂-C₁₋₉heteroaryl, and C₁₋₉heteroaryl, wherein C₁₋₆alkyl, C_{2- 6}alkenyl, C₂₋₆alkynyl, C₃₋₁₀cycloalkyl, -C(R^{12e})₂-C₃₋₁₀cycloalkyl, C₂₋₉heterocycloalkyl, -C(R^{12e})₂-C_{2- 9}heterocycloalkyl, C₆₋₁₀aryl, -C(R^{12e})₂-C₆₋₁₀aryl, -C(R^{12e})₂-C₁₋₉heteroaryl, and C₁₋₉heteroaryl are optionally substituted with one, two, or three R^{20d}; each R^{12e} is independently selected from hydrogen and R^{20d}; R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloallcyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋ioaryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₁₀cycloallcyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C₂₋₉heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20b}, and R²⁰ⁱ is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, - S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵,-S(O)₂N(R²²)(R²³), and -OC(O)R²⁵; each R²¹ is independently selected from H, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; each R²³ is independently selected from H and C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula Ib-1, or a pharmaceutically acceptable salt or solvate thereof: 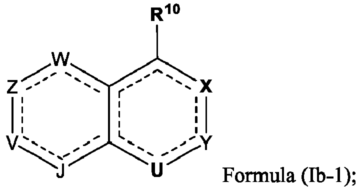 wherein W is N, C(R¹⁸), N(R^{18b}), C(R¹⁸)(R^{18a}), C(O), S(O), or S(O)₂; Z is N, C(R⁸), N(R^{8b}), C(R⁸)(R^{8a}), O, C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, -N(R¹⁴)-C₁₋₃allcyl-, or C_{1- 4}alkyl, wherein the C₁₋₃alkyl and C₁₋₄alkyl are optionally substituted with one, two or three R^{20a}; R⁷ is a C_{3- 12}cycloalkyl, 3-12 membered nitrogen containing heterocycloalkyl, C₆₋₁₂aryl, or 5-12 membered nitrogen containing heteroaryl, wherein the C₃₋₁₂cycloalkyl, 3-12 membered nitrogen containing heterocycloalkyl, C₆₋₁₂aryl, and 5-12 membered nitrogen containing heteroaryl are substituted with one R⁶, and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)O-, -C₀₋₃alkyl-C(O)-, -C₀₋₃alkyl-C(O)N(R¹²)-C_{0- 3}alkyl-, -C₀₋₃alkyl-N(R¹²)C(O)-, -C₀₋₃alkyl-S(O), -C₀₋₃alkyl-S(O)N(R¹²)-C₀₋₃alkyl-, -C₀₋₃alkyl-N(R¹²)S(O), -C₀₋₃alkylS(O)₂-, -C₀₋₃alkyl-S(O)₂N(R¹²)-C₀₋₃alkyl-, or -C₀₋₃alkyl-N(R¹²)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, - S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)_{2N}(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; each R^{12d} is independently selected from hydrogen, C₁₋₆alkyl, C₂₋₆alkenyl, C₂₋₆alkynyl, C₃₋₁₀cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -C(R^{12e})₂-C₃₋₁₀cycloalkyl, C_{2- 9}heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C₁₋₉heteroaryl, and C_{1- 9}heteroaryl are optionally substituted with one, two, or three R^{20d}; each R^{12e} is independently selected from hydrogen and R^{20d}; R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20b}, R²⁰ⁱ, and R^{20k} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³),-OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, - S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵,-S(O)₂N(R²²)(R²³), and -OC(O)R²⁵; each R²¹ is independently selected from H, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; each R²³ is independently selected from H and C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula Id-1, or a pharmaceutically acceptable salt or solvate thereof: 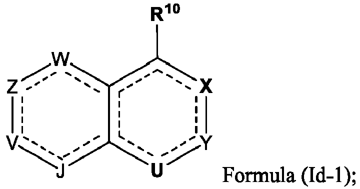 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), O, C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, -N(R¹⁴)-C_{1- 3}alkyl-, or C₁₋₄alkyl, wherein the C₁₋₃alkyl and C₁₋₄alkyl are optionally substituted with one, two or three- R^{20a}; R⁷ is a 7-12 membered nitrogen containing spirocyclic heterocycloalkyl substituted with one R⁶, and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloallcyl, C_{6- 12}aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₁₀cycloalkyl, C₂₋₉heterocycloallcyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)O-, -C₀₋₃alkyl-C(O)-, -C_{0- 3}alkyl-C(O)N(R¹²)-C₀₋₃alkyl-, -C₀₋₃alkyl-N(R¹²)C(O)-, -C₀₋₃alkyl-S(O), -C₀₋₃alkyl-S(O)N(R¹²)-C₀₋₃alkyl-, -C_{0- 3}alkyl-N(R¹²)S(O), -C₀₋₃alkyl-S(O)₂-, -C₀₋₃alkyl-S(O)₂N(R¹²)-C₀₋₃alkyl-, or -C₀₋₃alkyl-N(R¹²)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆allcyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹¹; each R¹¹ is independently selected from halogen, oxo, -CN, C₁₋₆alkyl, C₁₋₆haloalkyl, C₂₋₆alkenyl, C₂₋₆alkynyl, C₃₋₁₀cycloallcyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; each R^{12d} is independently selected from hydrogen, C₁₋₆alkyl, C₂₋₆alkenyl, C₂₋₆alkynyl, C₃₋₁₀cycloalkyl, -C(R^{12e})₂-C₃₋₁₀cycloalkyl, C₂₋₉heterocycloalkyl, - C(R^{12e})₂-C₂₋₉heterocycloalkyl, C₆₋₁₀aryl, -C(R^{12e})₂-C₆₋₁₀aryl, -C(R^{12e})₂-C₁₋₉heteroaryl, and C₁₋₉heteroaryl, wherein C_{1- 6}alkyl, C₂₋₆alkenyl, C₂₋₆alkynyl, C₃₋₁₀cycloalkyl, -C(R^{12e})₂-C₃₋₁₀cycloalkyl, C₂₋₉heterocycloalkyl, -C(R^{12e})₂-C_{2- 9}heterocycloalkyl, C₆₋₁₀aryl, -C(R^{12e})₂-C₆₋₁₀aryl, -C(R^{12e})₂-C₁₋₉heteroaryl, and C₁₋₉heteroaryl are optionally substituted with one, two, or three R^{20d}; each R^{12e} is independently selected from hydrogen and R^{20d}; R^{2c} is hydrogen, halogen, -CN, C₁₋₆allcyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, - S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵; each R²¹ is independently selected from H, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; each R²³ is independently selected from H and C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula Ie-1, or a pharmaceutically acceptable salt or solvate thereof: 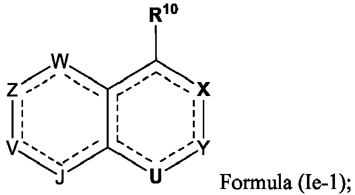 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), O, C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C₁₋₃alkyl and C₁₋₄alkyl are optionally substituted with one, two or three- R^{20a}; R⁷ is a 7-12 membered nitrogen containing fused heterocycloalkyl, 7-12 membered nitrogen containing fused aryl, or 7-12 membered nitrogen containing fused heteroaryl substituted with one R⁶, and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)O-, -C₀₋₃alkyl-C(O)-, -C₀₋₃alkyl-C(O)N(R¹²)-C_{0- 3}alkyl-, -C₀₋₃alkyl-N(R¹²)C(O)-, -C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C₀₋₃alkyl-, or -C₀₋₃alkyl-N(R¹²)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, - S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹; each R¹¹ is independently selected from halogen, oxo, -CN, C₁₋₆alkyl, C₁₋₆haloallcyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆allcyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)2R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; each R^{12d} is independently selected from hydrogen, C₁₋₆alkyl, C₂₋₆alkenyl, C₂₋₆alkynyl, C₃₋₁₀cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -C(R^{12e})₂-C₃₋₁₀cycloalkyl, C_{2- 9}heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C₁₋₉heteroaryl, and C_{1- 9}heteroaryl are optionally substituted with one, two, or three R^{20d}; each R^{12e} is independently selected from hydrogen and R^{20d}; R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or --(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₁₀cycloallcyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)2R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b}; R^{18b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C_{3- 10}cycloallcyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³),-OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, - S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵,-S(O)₂N(R²²)(R²³), and -OC(O)R²⁵; each R²¹ is independently selected from H, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a pharmaceutical composition comprising a precursor compound described herein, or a pharmaceutically acceptable salt or solvate thereof, and a pharmaceutically acceptable excipient.

In an aspect is provided a method of treating cancer in a subject in need thereof, comprising administering to the subject a therapeutically effective amount of a precursor compound described herein, or a pharmaceutically acceptable salt or solvate thereof.

In an aspect is provided a method of treating cancer in a subject comprising a Ras G12S mutant protein (including K-Ras G12S, H-Ras G12S or N-Ras G12S), the method comprising modifying the Ras G12S protein of said subject by administering to said subject a precursor compound described herein, wherein the precursor compound is characterized in that upon contacting a Ras G12S protein, said Ras G12S protein is modified covalently at a serine residue corresponding to reside 12 of SEQ ID No: 1, such that said modified Ras G12S protein exhibits reduced Ras signaling output (e.g., compared to a control such as an unmodified Ras G12S protein not covalently bonded with any compound such as a compound disclosed herein).

In some embodiments, the cancer being treated is a solid tumor, a hematological cancer, a primary cancer, or cancer resulting from metastasis of the primary cancer.In an aspect is provided a method of modulating activity of a Ras protein (e.g., K-Ras G12S, H-Ras G12S, or N-Ras G12S), comprising contacting the Ras protein with an effective amount of a precursor compound described herein, or a pharmaceutically acceptable salt or solvate thereof, thereby modulating the activity of the Ras protein.

In an aspect is provided a method of inhibiting cell growth, comprising administering an effective amount of a precursor compound or compound described herein, or a pharmaceutically acceptable salt or solvate thereof, to a cell expressing a Ras protein, thereby inhibiting growth of said cells.

In practicing any of the aforementioned methods, an additional agent can be used in combination or in conjuction with the precursor compound disclosed herein.

In embodiments, the additional agent comprises (1) an inhibitor of MEK (e.g., MEK1, MEK2) or of mutants thereof (e.g., trametinib, cobimetinib, binimetinib, selumetinib, refametinib); (2) an inhibitor of epidermal growth factor receptor (EGFR) and/or of mutants thereof (e.g., afatinib, erlotinib, gefitinib, lapatinib, cetuximab panitumumab, osimertinib, olmutinib, EGF-816); (3) an immunotherapeutic agent (e.g., checkpoint immune blockade agents, as disclosed herein); (4) a taxane (e.g., paclitaxel, docetaxel); (5) an anti-metabolite (e.g. antifolates such as methotrexate, raltitrexed, pyrimidine analogues such as 5-fluorouracil (5-FU), ribonucleoside and deoxyribonucleoside analogues, capecitabine and gemcitabine, purine and adenosine analogues such as mercaptopurine, thioguanine, cladribine and pentostatin, cytarabine (ara C), fludarabine); (6) an inhibitor of FGFR1 and/or FGFR2 and/or FGFR3 and/or of mutants thereof (e.g., nintedanib); (7) a mitotic kinase inhibitor (e.g., a CDK4/6 inhibitor, such as, for example, palbociclib, ribociclib, abemaciclib); (8) an anti-angiogenic drug (e.g., an anti-VEGF antibody, such as, for example, bevacizumab); (9) a topoisomerase inhibitor (e.g. epipodophyllotoxins such as for example etoposide and etopophos, teniposide, amsacrin, topotecan, irinotecan, mitoxantrone); (10) a platinum-containing compound (e.g. cisplatin, oxaliplatin, carboplatin); (11) an inhibitor of ALK and/or of mutants thereof (e.g. crizotinib, alectinib, entrectinib, brigatinib); (12) an inhibitor of c-MET and/or of mutants thereof (e.g., K252a, SU11274, PHA665752, PF2341066); (13) an inhibitor of BCR-ABL and/or of mutants thereof (e.g., imatinib, dasatinib, nilotinib); (14) an inhibitor of ErbB2 (Her2) and/or of mutants thereof (e.g., afatinib, lapatinib, trastuzumab, pertuzumab); (15) an inhibitor of AXL and/or of mutants thereof (e.g., R428, amuvatinib, XL-880); (16) an inhibitor of NTRK1 and/or of mutants thereof (e.g., Merestinib); (17) an inhibitor of RET and/or of mutants thereof (e.g., BLU-667, Lenvatinib); (18) an inhibitor of A-Raf and/or B-Raf and/or C-Raf and/or of mutants thereof (RAF-709, LY-3009120); (19) an inhibitor of ERK and/or of mutants thereof (e.g., ulixertinib); (20) an MDM2 inhibitor (e.g., HDM-201 , NVP-CGM097, RG-71 12, MK-8242, RG-7388, SAR405838, AMG-232, DS-3032, RG-7775, APG-115); (21) an inhibitor of mTOR (e.g., rapamycin, temsirolimus, everolimus, ridaforolimus); (22) an inhibitor of BET (e.g., I-BET 151, I-BET 762, OTX-015, TEN-010, CPI-203, CPI-0610, olionon, RVX-208, ABBC-744, LY294002, AZD5153, MT-1, MS645); (23) an inhibitor of IGF1/2 and/or of IGF1-R (e.g., xentuzumab, MEDI-573); (24) an inhibitor of CDK9 (e.g., DRB, flavopiridol, CR8, AZD 5438, purvalanol B, AT7519, dinaciclib, SNS-032); (25) an inhibitor of farnesyl transferase (e.g., tipifarnib); (26) an inhibitor of SHIP pathway including SHIP2 inhibitor, as well as SHIP1 inhibitors; (27) an inhibitor of SRC (e.g., dasatinib); (28) an inhibitor of JAK (e.g., tofacitinib); (29) a PARP inhibitor (e.g. Olaparib, Rucaparib, Niraparib, Talazoparib), (30) a BTK inhibitor (e.g. Ibrutinib, Acalabrutinib, Zanubrutinib), (31) a ROS1 inhibitor (e.g., entrectinib), (32) an inhibitor of SHP pathway including SHP2 inhibitor (e.g., 6-(4-amino-4-methylpiperidin-1-yl)-3-(2,3-dichlorophenyl)pyrazin-2-amine, as well as SHP1 inhibitors, or (33) an inhibitor of Src, FLT3, HDAC, VEGFR, PDGFR, LCK, Bcr-Abl or AKT or (34) an inhibitor of KrasG12C mutant (e.g., including but not limited to AMG510, MRTX849, and any covalent inhibitors binding to the cysteine residue 12 of Kras, the structures of these compounds are publically known)( e.g., an inhibitor of Ras G12C as described in US20180334454, US20190144444, US20150239900, US10246424, US20180086753, WO2018143315, WO2018206539, WO20191107519, WO2019141250, WO2019150305, US9862701, US20170197945, US20180086753, US10144724, US20190055211, US20190092767, US20180127396, US20180273523, US10280172, US20180319775, US20180273515, US20180282307, US20180282308, WO2019051291, WO2019213526, WO2019213516, WO2019217691, WO2019241157, WO2019217307, WO2020047192, WO2017087528, WO2018218070, WO2018218069, WO2018218071, WO2020027083, WO2020027084, WO2019215203, WO2019155399, WO2020035031, WO2014160200, WO2018195349, WO2018112240, WO2019204442, WO2019204449, WO2019104505, WO2016179558, WO2016176338, or related patents and applications, each of which is incorporated by reference in its entirety),), (35) a SHC inhibitor (e.g., PP2, AID371185), (36) a GAB inhibitor (e.g., GAB-0001), (37) a GRB inhibitor, (38) a PI-3 kinase inhibitor (e.g., Idelalisib, Copanlisib, Duvelisib, Alpelisib, Taselisib, Perifosine, Buparlisib, Umbralisib, NVP-BEZ235-AN), (39) a MARPK inhibitor, (40) CDK4/6 (e.g., palbociclib, ribociclib, abemaciclib), or (41) MAPK inhibitor (e.g., VX-745, VX-702, RO-4402257, SCIO-469, BIRB-796, SD-0006, PH-797804, AMG-548, LY2228820, SB-681323, GW-856553, RWJ67657, BCT-197), or (42) an inhibitor of SHP pathway including SHP2 inhibitor (e.g., 6-(4-amino-4-methylpiperidin-1-yl)-3-(2,3-dichlorophenyl)pyrazin-2-amine, RMC-4630, TNO155 ( 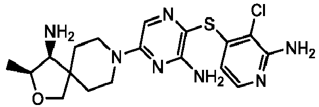 ), JAB-3068 ( 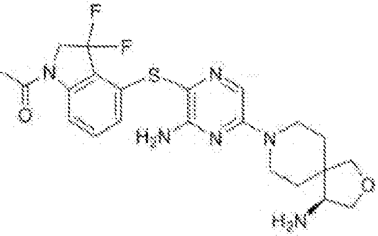 ), IACS-13909/BBP-398 ( 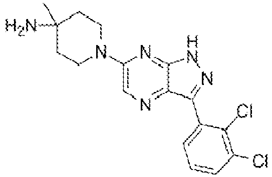 ), SHP099 ( 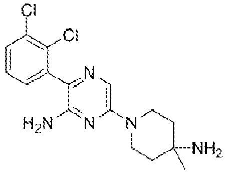 ), ERAS-601, and RMC-4550 ( 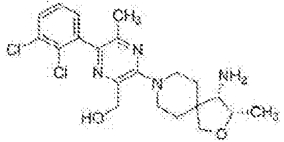 ), or a SHP1 inhibitor; (43) checkpoint immune blockade agents (e.g., anti-PD-1 and/or anti-PD-L1 antibody, anti-CLTA-4 antibody).

In embodiments, the additional agent comprises an inhibitor of SHP2 selected from RMC-4630, TNO155 ( 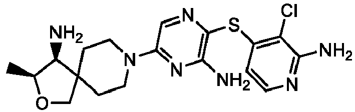 ), JAB-3068 ( 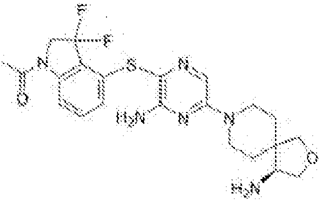 ), IACS-13909/BBP-398 ( 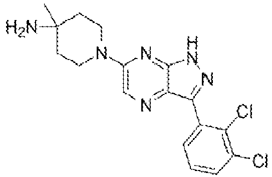 ), SHP099 ( 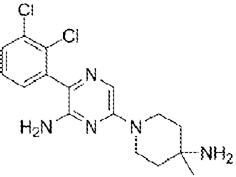 ), ERAS-601, and RMC-4550 ( 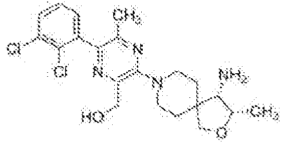 )

In embodiments, the additional agent comprises an inhibitor of SOS selected from RMC-5845, BI-3406 ( 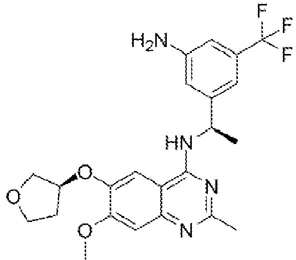 ), BI-1701963, and BAY 293 ( 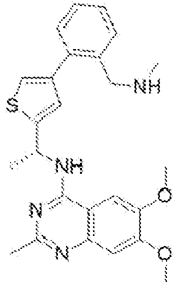 ).

In embodiments, the additional agent comprises an inhibitor of EGFR selected from afatinib, erlotinib, gefitinib, lapatinib, cetuximab panitumumab, osimertinib, olmutinib, and EGF-816.

In embodiments, the additional agent comprises an inhibitor of MEK selected from trametinib, cobimetinib, binimetinib, selumetinib, refametinib, and AZD6244.

In embodiments, the additional agent comprises an inhibitor of ERK selected from ulixertinib, MK-8353, LTT462, AZD0364, SCH772984, BIX02189, LY3214996, and ravoxertinib.

In embodiments, the additional agent comprises an inhibitor of CDK4/6 selected from palbociclib, ribociclib, and abemaciclib.

In embodiments, the additional agent comprises an inhibitor of BRAF selected from Sorafenib, Vemurafenib, Dabrafenib, Encorafenib, regorafenib, and GDC-879.

In an aspect is provided a compound having the formula A-L^{AB}-B wherein
A is a monovalent form of a precursor compound described herein;
L^{AB} is a covalent linker bonded to A and B; and
B is a monovalent form of a degradation enhancer.

In embodiments, the degradation enhancer is capable of binding a protein selected from E3A, mdm2, APC, EDD1, SOCS/BC-box/eloBC/CUL5/RING, LNXp80, CBX4, CBLL1, HACE1, HECTD1, HECTD2, HECTD3, HECTD4, HECW1, HECW2, HERC1, HERC2, HERC3, HERC4, HERS, HERC6, HUWE1, ITCH, NEDD4, NEDD4L, PPIL2, PRPF19, PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UBOX5, UBRS, VHL (von-Hippel-Lindau ubiquitin ligase), WWP1, WWP2, Parkin, MKRN1, CMA (chaperon-mediated autophage), SCFb-TRCP (Skip-Cullin-F box (Beta-TRCP) ubiquitin complex), b-TRCP (b-transducing repeat-containing protein), cIAP1 (cellular inhibitor of apoptosis protein 1), APC/C (anaphase-promoting complex/cyclosome), CRBN (cereblon), CUL4-RBX1-DDB1-CRBN (CRL4^{CRBN}) ubiquitin ligase, XIAP, IAP, KEAP1, DCAF15, RNF114, DCAF16, AhR, SOCS2, KLHL12, UBR2, SPOP, KLHL3, KLHL20, KLHDC2, SPSB1, SPSB2, SPSB4, SOCS6, FBXO4, FBXO31, BTRC, FBW7, CDC20, PML, TRIM21, TRIM24, TRIM33, GID4, avadomide, iberdomide, and CC-885.

In embodiments, the degradation enhancer is capable of binding a protein selected from UBE2A, UBE2B, UBE2C, UBE2D1, UBE2D2, UBE2D3, UBE2DR, UBE2E1, UBE2E2, UBE2E3, UBE2F, UBE2G1, UBE2G2, UBE2H, UBE2I, UBE2J1, UBE2J2, UBE2K, UBE2L3, UBE2L6, UBE2L1, UBE2L2, UBE2L4, UBE2M, UBE2N, UBE2O, UBE2Q1, UBE2Q2, UBE2R1, UBE2R2, UBE2S, UBE2T, UBE2U, UBE2V1, UBE2V2, UBE2W, UBE2Z, ATG3, BIRC6, and UFC1.

In embodiments L^{AB} is -L^{AB}-L^{AB2}-L^{AB3}-L^{AB4}-L^{AB5}-;
L^{AB1}, L^{AB2}, L^{AB3}, L^{AB4}, and L^{AB5} are independently a bond, -O-, -N(R¹⁴)-, -C(O)-, -N(R¹⁴)C(O)-, -C(O)N(R¹⁴)-, -S-, - S(O)₂-, -S(O)-, -S(O)₂N(R¹⁴)-, -S(O)N(R¹⁴)-, -N(R¹⁴)S(O)-, -N(R¹⁴)S(O)₂-, C₁₋₆alkylene, (-O-C₁₋₆alkyl)_{z}-, (-C_{1- 6}alkyl-O)_{z}-, C₂₋₆alkenylene, C₂₋₆alkynylene, C₁₋₆haloalkylene, C₃₋₁₂cycloalkylene, C₁₋₁₁heterocycloallcylene, C_{6- 12}arylene, or C₁₋₁₁heteroarylene, wherein C₁₋₆alkylene, C₂₋₆alkenylene, C₂₋₆alkynylene, C₁₋₆haloalkylene, C_{3- 12}cycloalkylene, C₁₋₁₁heterocycloalkylene, C₆₋₁₂arylene, or C₁₋₁₁heteroarylene,are optionally substituted with one, two, or three R^{20j}; wherein each C₁₋₆alkyl of (-O-C₁₋₆alkyl)_{z}- and (-C₁₋₆alkyl-O)_{z}- is optionally substituted with one, two, or three R^{20j};
z is independently an integer from 0 to 10;
each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f};
each R^{20d}, R^{20e}, R^{20f}, and R^{20j} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(R²²)(R²³), -OCH₂C(O)OR²², and -OC(O)R²⁵, wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloallcyl, C₁₋₆alkoxy, C₁₋₆haloalkoxy, -OR²¹, - SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
each R²¹ is independently selected from H, C₁₋₆alkyl, C₁₋₆haloallcyl, C₂₋₆alkenyl, C₂₋₆alkynyl, C₃₋₆cycloalkyl, C_{2- 9}heterocycloalkyl, C₆₋₁₀aryl, and C₁₋₉heteroaryl;
each R²² is independently selected from H, C₁₋₆alkyl, C₁₋₆haloallcyl, C₂₋₆alkenyl, C₂₋₆alkynyl, C₃₋₆cycloalkyl, C_{2- 9}heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl; and
each R²⁵ is independently selected from C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl.

In embodiments, L^{AB} is -(O-C₂alkyl)_{z}- and z is an integer from 1 to 10.

In embodiments, L^{AB} is -(C₂alkyl-O-)_{z}- and z is an integer from 1 to 10.

In embodiments, L^{AB} is -(CH₂)_{zz1}L^{AB2}(CH₂O)_{zz2}-, wherein L^{AB2} is a bond, a 5 or 6 membered heterocycloalkylene or heteroarylene, phenylene, -(C₂-C₄)alkynylene, -SO₂- or -NH-; and zz1 and zz2 are independently an integer from 0 to 10.

In embodiments, L^{AB} is -(CH₂)_{zz1}(CH₂O)_{zz2}-, wherein zz1 and zz2 are each independently an integer from 0 to 10.

In embodiments, L^{AB} is a PEG linker (e.g., divalent linker of 1 to 10 ethylene glycol subunits).

In embodiments, B is a monovalent form of a compound selected from

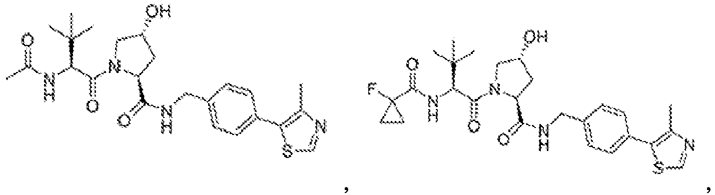

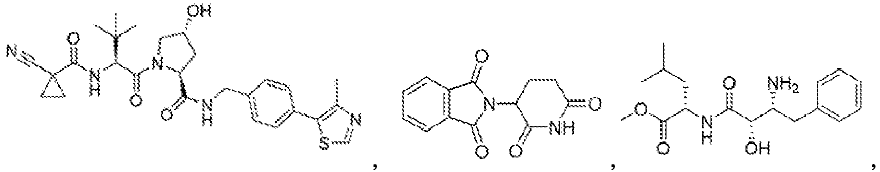

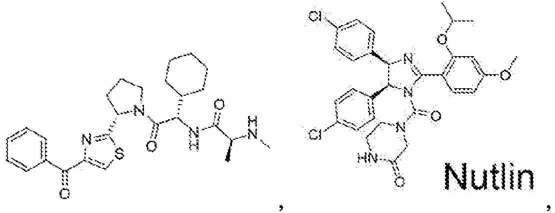

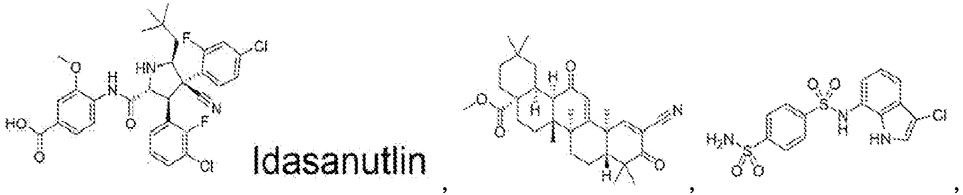

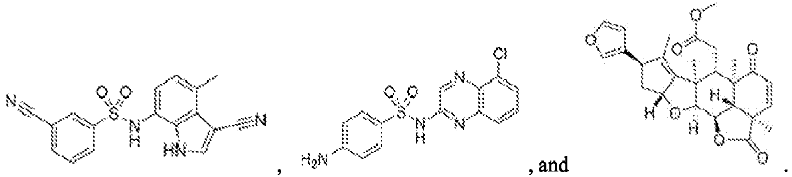

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

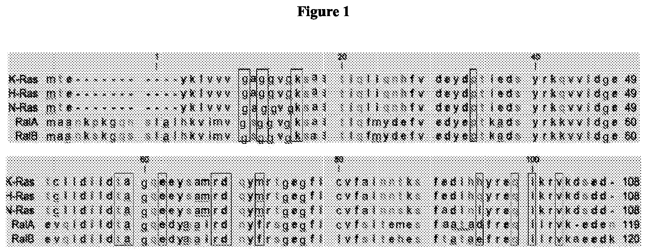

**FIG. 1** depicts a sequence alignment of various wild type Ras proteins including K-Ras, H-Ras, N-Ras, RalA, RalB, from top to bottom.

### DETAILED DESCRIPTION

The practice of some embodiments disclosed herein employ, unless otherwise indicated, conventional techniques of immunology, biochemistry, chemistry, molecular biology, microbiology, cell biology, genomics and recombinant DNA, which are within the skill of the art. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M.J. MacPherson, B.D. Hames and G.R. Taylor eds. (1995)), Harlow and Lane, eds. (1988) Antibodies, A Laboratory Manual, and Culture of Animal Cells: A Manual of Basic Technique and Specialized Applications, 6th Edition (R.I. Freshney, ed. (2010)).

Unless defined otherwise, all technical and scientific terms used herein have the same meaning as is commonly understood to which the claimed subject matter belongs. In the event that there are a plurality of definitions for terms herein, those in this section prevail. All patents, patent applications, publications and published nucleotide and amino acid sequences (*e.g*., sequences available in GenBank or other databases) referred to herein are incorporated by reference. Where reference is made to a URL or other such identifier or address, it is understood that such identifiers can change and particular information on the internet can come and go, but equivalent information can be found by searching the internet. Reference thereto evidences the availability and public dissemination of such information.

It is to be understood that the foregoing general description and the following detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

The section headings used herein are for organizational purposes only and are not to be construed as limiting the subject matter described.

Definition of standard chemistry terms may be found in reference works, including but not limited to, Carey and Sundberg "Advanced Organic Chemistry 4^{th} Ed." Vols. A (2000) and B (2001), Plenum Press, New York. Unless otherwise indicated, conventional methods of mass spectroscopy, NMR, HPLC, protein chemistry, biochemistry, recombinant DNA techniques and pharmacology.

Unless specific definitions are provided, the nomenclature employed in connection with, and the laboratory procedures and techniques of, analytical chemistry, synthetic organic chemistry, and medicinal and pharmaceutical chemistry described herein are those recognized in the field. Standard techniques can be used for chemical syntheses, chemical analyses, pharmaceutical preparation, formulation, and delivery, and treatment of patients. Standard techniques can be used for recombinant DNA, oligonucleotide synthesis, and tissue culture and transformation (e.g., electroporation, lipofection). Reactions and purification techniques can be performed e.g., using kits of manufacturer's specifications or as commonly accomplished in the art or as described herein. The foregoing techniques and procedures can be generally performed of conventional methods and as described in various general and more specific references that are cited and discussed throughout the present specification.

It is to be understood that the methods and compositions described herein are not limited to the particular methodology, protocols, cell lines, constructs, and reagents described herein and as such may vary. It is also to be understood that the terminology used herein is for the purpose of describing particular embodiments only, and is not intended to limit the scope of the methods, compounds, compositions described herein.

As used herein, C₁-Cₓ includes C₁-C₂, C₁-C₃ ... C₁-Cₓ. C₁-Cₓ refers to the number of carbon atoms that make up the moiety to which it designates (excluding optional substituents).

An "alkyl" group refers to a straight or branched hydrocarbon chain radical consisting solely of carbon and hydrogen atoms, containing no unsaturation. In some embodiments, the "alkyl" group may have 1 to 18, 1 to 12, 1 to 10, 1 to 8, or 1 to 6 carbon atoms (whenever it appears herein, a numerical range such as "1 to 6" refers to each integer in the given range; *e.g*., "1 to 6 carbon atoms" means that the alkyl group may consist of 1 carbon atom, 2 carbon atoms, 3 carbon atoms, *etc.,* up to and including 6 carbon atoms, although the present definition also covers the occurrence of the term "alkyl" where no numerical range is designated). The alkyl group of the compounds described herein may be designated as "C₁-C₆alkyl" or similar designations. By way of example only, "C₁-C₆alkyl" indicates that there are one to six carbon atoms in the alkyl chain, *i.e.,* the alkyl chain is selected from the group consisting of methyl, ethyl, n-propyl, iso-propyl, n-butyl, iso-butyl, sec-butyl, t-butyl, n-pentyl, isopentyl, neopentyl, and hexyl. Alkyl groups can be substituted or unsubstituted. Depending on the structure, an alkyl group can be a monoradical or a diradical (i.e., an alkylene group).

An "alkoxy" refers to a "-O-alkyl" group, where alkyl is as defined herein.

The term "alkenyl" refers to a straight or branched hydrocarbon chain radical group consisting solely of carbon and hydrogen atoms, containing at least one carbon-carbon double bond. Non-limiting examples of an alkenyl group include -CH=CH₂, -C(CH₃)=CH₂, -CH=CHCH₃, -CH=C(CH₃)₂ and -C(CH₃)=CHCH₃. In some embodiments, an alkenyl groups may have 2 to 6 carbons. Alkenyl groups can be substituted or unsubstituted. Depending on the structure, an alkenyl group can be a monoradical or a diradical (i.e., an alkenylene group).

The term "alkynyl" refers to a straight or branched hydrocarbon chain radical group consisting solely of carbon and hydrogen atoms, containing at least one carbon-carbon triple bond. Non-limiting examples of an alkynyl group include -C≡CH, -C≡CCH₃, -C≡CCH₂CH₃ and -C≡CCH₂CH₂CH₃. In some embodiments, an alkynyl group can have 2 to 6 carbons. Alkynyl groups can be substituted or unsubstituted. Depending on the structure, an alkynyl group can be a monoradical or a diradical (i.e., an alkynylene group).

"Amino" refers to a -NH₂ group.

The term "alkylamine" or "alkylamino" refers to the -N(alkyl)ₓH_{y} group, where alkyl is as defined herein and x and y are selected from the group x=1, y=1 and x=2, y=0. When x=2, the alkyl groups, taken together with the nitrogen to which they are attached, can optionally form a cyclic ring system. "Dialkylamino" refers to a -N(alkyl)₂ group, where alkyl is as defined herein.

The term "aromatic" refers to a planar ring having a delocalized π-electron system containing 4n+2 π electrons, where n is an integer. Aromatic rings can be formed from five, six, seven, eight, nine, or more than nine atoms. Aromatics can be optionally substituted. The term "aromatic" includes both aryl groups (*e.g.*, phenyl, naphthalenyl) and heteroaryl groups (*e.g*., pyridinyl, quinolinyl).

As used herein, the term "aryl" refers to a monocylic aromatic ring wherein each of the atoms forming the ring is a carbon atom (e.g., phenyl) or a polycyclic ring system (e.g., bicyclic or tricyclic) wherein 1) at least one ring is carbocyclic and aromatic, 2) a bond to the remainder of the compound is directly bonded to a carbocyclic aromatic ring of the aryl ring system, and 3) the carbocyclic aromatic ring of the aryl ring system of 2) is not directly bonded (e.g., fused) to a heteroaryl ring in the polycyclic ring sytem. Aryl rings can be formed by five, six, seven, eight, nine, or more than nine carbon atoms. Aryl groups can be optionally substituted. Examples of aryl groups include, but are not limited to phenyl, and naphthalenyl. Depending on the structure, an aryl group can be a monoradical or a diradical (i.e., an arylene group). As used herein, the aryl radical is a monocyclic, bicyclic, or tricyclic ring system. In embodiments, an aryl is a monocyclic ring. In embodiments, an aryl is a fused ring polycyclic system. In embodiments, an aryl is a bridged ring polycyclic system. In some embodiments the aryl is a "fused ring aryl" wherein the aryl ring is fused with a cycloalkyl or a heterocycloalkyl ring.

"Carboxy" refers to -CO₂H. In some embodiments, carboxy moieties may be replaced with a "carboxylic acid bioisostere", which refers to a functional group or moiety that exhibits similar physical and/or chemical properties as a carboxylic acid moiety. A carboxylic acid bioisostere has similar biological properties to that of a carboxylic acid group. A compound with a carboxylic acid moiety can have the carboxylic acid moiety exchanged with a carboxylic acid bioisostere and have similar physical and/or biological properties when compared to the carboxylic acid-containing compound. For example, in one embodiment, a carboxylic acid bioisostere would ionize at physiological pH to roughly the same extent as a carboxylic acid group. Examples of bioisosteres of a carboxylic acid include, but are not limited to, 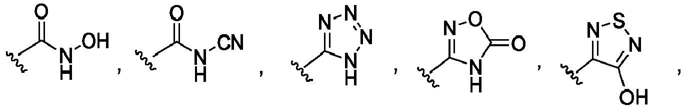 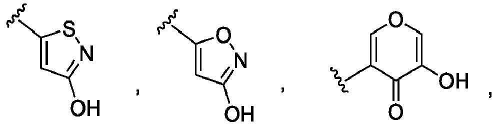 and the like.

The term "cycloalkyl" refers to a monocyclic carbocyclic saturated or partially unsaturated non-aromatic ring or a polycyclic carbocyclic (i.e., does not include heteroatom(s)) ring system (e.g., bicyclic or tricyclic) wherein 1) at least one ring is carbocyclic saturated or partially unsaturated and non-aromatic, 2) a bond to the remainder of the compound is directly bonded to a carbocyclic saturated or partially unsaturated non-aromatic ring of the ring system, and 3) the carbocyclic saturated or partially unsaturated non-aromatic ring of the ring system of 2) is not directly bonded (e.g., fused or spirocyclic) to a heterocycloalkyl ring in the polycyclic ring system. Cycloalkyls may be saturated or partially unsaturated. In some embodiments, a cycloalkyl ring is a spirocyclic cycloalkyl ring. In embodiments, a cycloalkyl is a monocyclic ring. In embodiments, a cycloalkyl is a fused ring polycyclic system. In embodiments, a cycloalkyl is a bridged ring polycyclic system. In embodiments, a cycloalkyl is a spirocyclic polycyclic ring system. In some embodiments, cycloalkyl groups include groups having from 3 to 10 ring atoms. Depending on the structure, a cycloalkyl group can be a monoradical or a diradical (i.e., a cycloalkylene group).

The terms "heteroaryl" or, alternatively, "heteroaromatic" refers to an monocyclic aryl group that includes one or more ring heteroatoms selected from nitrogen, oxygen and sulfur; or a polycyclic ring system (e.g., bicyclic or tricyclic) wherein 1) at least one ring is aromatic and includes one or more heteroatoms selected from nitrogen, oxygen and sulfur and 2) a bond to the remainder of the compound is directly bonded to an aromatic ring including one or more heteroatoms selected from nitrogen, oxygen and sulfur or an aromatic ring directly bonded (e.g., fused) to an aromatic ring including one or more heteroatoms selected from nitrogen, oxygen and sulfur, of the aryl ring sytem. As used herein, the heteroaryl radical may be a monocyclic, bicyclic, or tricyclic ring system, wherein at least one of the rings in the ring system is fully unsaturated (i.e., aromatic) and includes a heteroatom. In embodiments, a heteroaryl is a monocyclic ring. In embodiments, a heteroaryl is a fused ring polycyclic system. In embodiments, a heteroaryl is a bridged ring polycyclic system. In some embodiments is a "fused ring heteroaryl" wherein the heteroaryl ring is fused with a cycloalkyl, aryl, or heterocycloalkyl ring. An N-containing "heteroaromatic" or "heteroaryl" moiety refers to an aromatic group in which at least one of the skeletal atoms of the ring is a nitrogen atom. Depending on the structure, a heteroaryl group can be a monoradical or a diradical (i.e., a heteroarylene group).

A "heterocycloalkyl" group or "heteroalicyclic" group refers to a cycloalkyl group, wherein at least one skeletal ring atom of a saturated or partially unsatured non-aromatic ring is a heteroatom selected from nitrogen, oxygen, phosphorus, and sulfur. A heterocycloalkyl refers to a monocyclic saturated or partially unsaturated non-aromatic ring including one or more heteroatoms or a polycyclic ring system (e.g., bicyclic or tricyclic) wherein 1) at least one ring is saturated or partially unsaturated, non-aromatic, and includes one or more heteroatoms and 2) a bond to the remainder of the compound is directly bonded to a ring of the ring system that is a saturated or partially unsaturated and non-aromatic ring that includes one or more heteroatoms or a non-aromatic ring directly bonded (e.g., fused) to a saturated or partially unsaturated and non-aromatic ring that includes one or more heteroatoms of the ring sytem. Heterocycloalkyls may be saturated or partially unsaturated. The term heterocycloalkyl also includes all ring forms of the carbohydrates, including but not limited to the monosaccharides, the disaccharides and the oligosaccharides. In some embodiments, a heterocycloalkyl ring is a spirocyclic heterocycloalkyl ring. In embodiments, a heterocycloalkyl is a monocyclic ring. In embodiments, a heterocycloalkyl is a fused ring polycyclic system. In embodiments, a heterocycloalkyl is a bridged ring polycyclic system. In embodiments, a heterocycloalkyl is a spirocyclic polycyclic ring system. Unless otherwise noted, heterocycloalkyls have from 2 to 13 carbons in the ring or ring system. It is understood that when referring to the number of carbon atoms in a heterocycloalkyl, the number of carbon atoms in the heterocycloalkyl is not the same as the total number of atoms (including the heteroatoms) that make up the heterocycloalkyl (i.e. skeletal atoms of the heterocycloalkyl ring). Depending on the structure, a heterocycloalkyl group can be a monoradical or a diradical (i.e., a heterocycloalkylene group).

The term "halo" or, alternatively, "halogen" means fluoro, chloro, bromo and iodo.

The abbreviations "Fmoc", "Ac", "Bn", "PMB", "Tr", "Ts", "Boc", and "Cbz" are used in accordance with their well understood common meanings in Chemistry and mean the monovalent chemical substituents fluorenylmethyloxycarbonyl, acetyl, benzyl, p-methoxybenzyl, trityl or triphenylmethyl, tosyl, tert-butyloxycarbonyl, and carbobenzyloxy, respectively.

The term "haloalkyl" refers to an alkyl group that is substituted with one or more halogens. The halogens may the same or they may be different. Non-limiting examples of haloalkyls include -CH₂Cl, -CF₃, -CHF₂, - CH₂CF₃, -CF₂CF₃, and the like.

The terms "fluoroalkyl" and "fluoroalkoxy" include alkyl and alkoxy groups, respectively, that are substituted with one or more fluorine atoms. Non-limiting examples of fluoroalkyls include -CF₃, -CHF₂, -CH₂F, - CH₂CF₃, -CF₂CF₃, -CF₂CF₂CF₃, -CF(CH₃)₃, and the like. Non-limiting examples of fluoroalkoxy groups, include - OCF₃, -OCHF₂, -OCH₂F, -OCH₂CF₃, -OCF₂CF₃, -OCF₂CF₂CF₃, -OCF(CH₃)₂, and the like.

The term "heteroalkyl" refers to an alkyl radical where one or more skeletal chain atoms is selected from an atom other than carbon, *e.g*., oxygen, nitrogen, sulfur, phosphorus, silicon, or combinations thereof. The heteroatom(s) may be placed at any interior position of the heteroalkyl group. Examples include, but are not limited to, -CH₂-O-CH₃, -CH₂-CH₂-O-CH₃, -CH₂-NH-CH₃, -CH₂-CH₂-NH-CH₃, -CH₂-N(CH₃)-CH₃, -CH₂-CH₂-NH-CH₃, - CH₂-CH₂-N(CH₃)-CH₃, -CH₂-S-CH₂-CH₃, -CH₂-CH₂-S(O)-CH₃, -CH₂-CH₂-S(O)₂-CH₃, -CH₂-NH-OCH₃, -CH₂-O-Si(CH₃)₃, -CH₂-CH=N-OCH₃, and -CH=CH-N(CH₃)-CH₃. In addition, up to two heteroatoms may be consecutive, such as, by way of example, -CH₂-NH-OCH₃ and -CH₂-O-Si(CH₃)₃. Excluding the number of heteroatoms, a "heteroalkyl" may have from 1 to 6 carbon atoms.

The term "oxo" refers to the =O radical.

The term "bond" or "single bond" refers to a chemical bond between two atoms, or two moieties when the atoms joined by the bond are considered to be part of larger substructure.

The term "moiety" refers to a specific segment or functional group of a molecule. Chemical moieties are often recognized chemical entities embedded in or appended to a molecule.

The suffix "-di-yl" will be understood to mean the substitutent or linker is a divalent substituent or linker.

As used herein, the substituent "R" appearing by itself and without a number designation refers to a substituent selected from among from alkyl, haloalkyl, heteroalkyl, alkenyl, cycloalkyl, aryl, heteroaryl (bonded through a ring carbon), and heterocycloalkyl.

"Optional" or "optionally" means that a subsequently described event or circumstance may or may not occur and that the description includes instances when the event or circumstance occurs and instances in which it does not.

The term "optionally substituted" or "substituted" means, unless otherwise specified, that the referenced group may be substituted with one or more additional group(s) individually and independently selected from alkyl, cycloalkyl, aryl, heteroaryl, heterocycloalkyl, -OH, alkoxy, aryloxy, alkylthio, arylthio, alkylsulfoxide, arylsulfoxide, alkylsulfone, arylsulfone, -CN, alkyne, C₁-C₆alkylalkyne, halo, acyl, acyloxy, -CO₂H, -CO₂-alkyl, nitro, haloalkyl, fluoroalkyl, and amino, including mono- and di-substituted amino groups (e.g. NH₂, -NHR, -N^{®}₂), and the protected derivatives thereof. By way of example, an optional substituents may be L^{s}R^{s}, wherein each L^{s} is independently selected from a bond, -O-, -C(=O)-, -S-, -S(=O)-, -S(=O)₂-, -NH-, -NHC(O)-, -C(O)NH-, S(=O)₂NH-, -NHS(=O)₂, -OC(O)NH-, -NHC(O)O-, -(C₁-C₆alkyl)-, or -(C₂-C₆alkenyl)-; and each R^{s} is independently selected from among H, (C₁-C₆alkyl), (C₃-C₈cycloalkyl), aryl, heteroaryl, heterocycloalkyl, and C₁-C₆heteroalkyl. The protecting groups that may form the protective derivatives of the above substituents are found in sources such as Greene and Wuts, above.

"Pharmaceutically acceptable salt" includes both acid and base addition salts. A pharmaceutically acceptable salt of any one of the compounds described herein is intended to encompass any and all pharmaceutically suitable salt forms. Preferred pharmaceutically acceptable salts of the compounds described herein are pharmaceutically acceptable acid addition salts and pharmaceutically acceptable base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid, hydroiodic acid, hydrofluoric acid, phosphorous acid, and the like. Also included are salts that are formed with organic acids such as aliphatic mono- and dicarboxylic acids, phenyl-substituted alkanoic acids, hydroxy alkanoic acids, alkanedioic acids, aromatic acids, aliphatic and. aromatic sulfonic acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Exemplary salts thus include sulfates, pyrosulfates, bisulfates, sulfites, bisulfites, nitrates, phosphates, monohydrogenphosphates, dihydrogenphosphates, metaphosphates, pyrophosphates, chlorides, bromides, iodides, acetates, trifluoroacetates, propionates, caprylates, isobutyrates, oxalates, malonates, succinate suberates, sebacates, fumarates, maleates, mandelates, benzoates, chlorobenzoates, methylbenzoates, dinitrobenzoates, phthalates, benzenesulfonates, toluenesulfonates, phenylacetates, citrates, lactates, malates, tartrates, methanesulfonates, and the like. Also contemplated are salts of amino acids, such as arginates, gluconates, and galacturonates (see, for example, Berge S.M. et al., "Pharmaceutical Salts," Journal of Pharmaceutical Science, 66:1 - 19 (1997)). Acid addition salts of basic compounds are, in some embodiments, prepared by contacting the free base forms with a sufficient amount of the desired acid to produce the salt according to methods and techniques with which a skilled artisan is familiar.

"Pharmaceutically acceptable base addition salt" refers to those salts that retain the biological effectiveness and properties of the free acids, which are not biologically or otherwise undesirable. These salts are prepared from addition of an inorganic base or an organic base to the free acid. Pharmaceutically acceptable base addition salts are, in some embodiments, formed with metals or amines, such as alkali and alkaline earth metals or organic amines. Salts derived from inorganic bases include, but are not limited to, sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, for example, isopropylamine, trimethylamine, diethylamine, triethylamine, tripropylamine, ethanolamine, diethanolamine, 2-dimethylaminoethanol, 2-diethylaminoethanol, dicyclohexylamine, lysine, arginine, histidine, caffeine, procaine, *N,N*-dibenzylethylenediamine, chloroprocaine, hydrabamine, choline, betaine, ethylenediamine, ethylenedianiline, *N*-methylglucamine, glucosamine, methylglucamine, theobromine, purines, piperazine, piperidine, *N*-ethylpiperidine, polyamine resins and the like. See Berge et al., *supra.*

The terms "polypeptide", "peptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" refers to either natural and/or unnatural or synthetic amino acids, including glycine and both the D or L optical isomers, and amino acid analogs and peptidomimetics.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or noncoding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. A polynucleotide may comprise one or more modified nucleotides, such as methylated nucleotides and nucleotide analogs, such as peptide nucleic acid (PNA), Morpholino and locked nucleic acid (LNA), glycol nucleic acid (GNA), threose nucleic acid (TNA), 2'-fluoro, 2'-OMe, and phosphorothiolated DNA. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component or other conjugation target.

As used herein, "expression" refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells, and their progeny of a biological entity obtained *in vivo* or cultured *in vitro* are also encompassed.

The terms "agent" or "therapeutic agent", "therapeutic capable agent" or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested. Typically, prophylactic benefit includes reducing the incidence and/or worsening of one or more diseases, conditions, or symptoms under treatment (e.g. as between treated and untreated populations, or between treated and untreated states of a subject).

The term "effective amount" or "therapeutically effective amount" refers to the amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art. An effective amount of an active agent may be administered in a single dose or in multiple doses. A component may be described herein as having at least an effective amount, or at least an amount effective, such as that associated with a particular goal or purpose, such as any described herein. The term "effective amount" also applies to a dose that will provide an image for detection by an appropriate imaging method. The specific dose may vary depending on one or more of: the particular agent chosen, the dosing regimen to be followed, whether it is administered in combination with other compounds, timing of administration, the tissue to be imaged, and the physical delivery system in which it is carried.

The term "*in vivo*" refers to an event that takes place in a subject's body.

The term "*ex vivo*" refers to an event that first takes place outside of the subject's body for a subsequent in vivo application into a subject's body. For example, an ex vivo preparation may involve preparation of cells outside of a subject's body for the purpose of introduction of the prepared cells into the same or a different subject's body.

The term "*in vitro*" refers to an event that takes place outside of a subject's body. For example, an in vitro assay encompasses any assay run outside of a subject's body. In vitro assays encompass cell-based assays in which cells alive or dead are employed. In vitro assays also encompass a cell-free assay in which no intact cells are employed.

The term "Ras" or "RAS" refers to a protein in the Rat sarcoma (Ras) superfamily of small GTPases, such as in the Ras subfamily. The Ras superfamily includes, but is not limited to, the Ras subfamily, Rho subfamily, Rab subfamily, Rap subfamily, Arf subfamily, Ran subfamily, Rheb subfamily, RGK subfamily, Rit subfamily, Miro subfamily, and Unclassified subfamily. In some embodiments, a Ras protein is selected from the group consisting of KRAS (also used interchangeably herein as K-Ras, K-ras, Kras), HRAS (or H-Ras), NRAS (or N-Ras), MRAS (or M-Ras), ERAS (or E-Ras), RRAS2 (or R-Ras2), RALA (or RalA), RALB (or RalB), RIT1, and any combination thereof, such as from KRAS, HRAS, NRAS, RALA, RALB, and any combination thereof. The terms "mutant Ras" and "Ras mutant," as used interchangeably herein, refer to a Ras protein with one or more amino acid mutations, such as with respect to a common reference sequence such as a wild-type (WT) sequence. In some embodiments, a mutant Ras is selected from a mutant KRAS, mutant HRAS, mutant NRAS, mutant MRAS, mutant ERAS, mutant RRAS2, mutant RALA, mutant RALB, mutant RIT1, and any combination thereof, such as from a mutant KRAS, mutant HRAS, mutant NRAS, mutant RALA, mutant RALB, and any combination thereof. In some embodiments, a mutation can be an introduced mutation, a naturally occurring mutation, or a non-naturally occurring mutation. In some embodiments, a mutation can be a substitution (e.g., a substituted amino acid), insertion (e.g., addition of one or more amino acids), or deletion (e.g., removal of one or more amino acids). In some embodiments, two or more mutations can be consecutive, non-consecutive, or a combination thereof. In some embodiments, a mutation can be present at any position of Ras. In some embodiments, a mutation can be present at position 12, 13, 62, 92, 95, or any combination thereof of Ras of SEQ ID No. 2 when optimally aligned. In some embodiments, a mutant Ras may comprise about or at least about 1, 2, 3, 4, 5, 6, 7, 8, 9, 10, 11, 12, 13, 14, 15, 16, 17, 18, 19, 20, 25, 30, 35, 40, 45, 50, or more than 50 mutations. In some embodiments, a mutant Ras may comprise up to about 1, 2, 3, 4, 5, 6, 7, 8, 9, 10, 11, 12, 13, 14, 15, 16, 17, 18, 19, 20, 25, 30, 35, 40, 45, or 50 mutations. In some embodiments, the mutant Ras is about or up to about 500, 400, 300, 250, 240, 233, 230, 220, 219, 210, 208, 206, 204, 200, 195, 190, 189, 188, 187, 186, 185, 180, 175, 174, 173, 172, 171, 170, 169, 168, 167, 166, 165, 160, 155, 150, 125, 100, 90, 80, 70, 60, 50, or fewer than 50 amino acids in length. In some embodiments, an amino acid of a mutation is a proteinogenic, natural, standard, non-standard, non-canonical, essential, non-essential, or non-natural amino acid. In some embodiments, an amino acid of a mutation has a positively charged side chain, a negatively charged side chain, a polar uncharged side chain, a non-polar side chain, a hydrophobic side chain, a hydrophilic side chain, an aliphatic side chain, an aromatic side chain, a cyclic side chain, an acyclic side chain, a basic side chain, or an acidic side chain. In some embodiments, a mutation comprises a reactive moiety. In some embodiments, a substituted amino acid comprises a reactive moiety. In some embodiments, a mutant Ras can be further modified, such as by conjugation with a detectable label. In some embodiments, a mutant Ras is a full-length or truncated polypeptide. For example, a mutant Ras can be a truncated polypeptide comprising residues 1-169 or residues 11-183 (e.g., residues 11-183 of a mutant RALA or mutant RALB).

As used herein, the term "corresponding to" or "corresponds to" as applied to an amino acid residue in a polypeptide sequence refers to the correspondence of such amino acid relative to a reference sequence when optimally aligned (e.g., taking into consideration of gaps, insertions and mismatches). For instance, the serine residue in a Ras G12S mutant refers to the serine corresponding to residue 12 of SEQ ID No. 1, which can serves as a reference sequence. A modified Ras mutant protein disclosed herein may comprise truncations at C-terminus, or truncations at the N-terminal end preceding the serine residue. The serine residue in such N-terminal truncated modified mutant is still considered corresponding to position 12 of SEQ ID No. 1. In addition, serine residue at position 12 of SEQ ID No. 1 finds a corresponding residue in SEQ ID Nos. 3 and 5. "Prodrug" as used herein is meant to indicate a compound that may be converted under physiological conditions or by solvolysis to a biologically active compound described herein. The term "prodrug" refers to a precursor of a biologically active compound that is pharmaceutically acceptable. A prodrug may be inactive when administered to a subject, but is converted *in vivo* to an active compound, for example, by hydrolysis. The prodrug compound may offer advantages of solubility, tissue compatibility and/or delayed release in a mammalian organism (*see, e.g.,* Bundgard, H., Design of Prodrugs (1985), pp. 7-9, 21-24 (Elsevier, Amsterdam). A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987, both of which are incorporated in full by reference herein. A "prodrug" can be any covalently bonded carriers, that release the active compound in vivo when such prodrug is administered to a mammalian subject. Prodrugs of an active compound, as described herein, may be prepared by modifying functional groups present in the active compound in such a way that the modifications are cleaved, either in routine manipulation or in vivo, to the parent active compound.

The terms "leaving group" is used herein in accordance with their well understood meanings in Chemistry and refers to an atom or group of atoms which breaks away from the rest of the molecule, taking with it the electron pair which used to be the bond between the leaving group and the rest of the molecule.

A "degradation enhancer" is a compound capable of binding a ubiquitin ligase protein (e.g., E3 ubiquitin ligase protein) or a compound capable of binding a protein that is capable of binding to a ubiquitin ligase protein to form a protein complex capable of conjugating a ubiquitin protein to a target protein. In embodiments, the degradation enhancer is capable of binding to an E3 ubiquitin ligase protein or a protein complex comprising an E3 ubiquitin ligase protein. In embodiments, the degradation enhancer is capable of binding to an E2 ubiquitin-conjugating enzyme. In embodiments, the degradation enhancer is capable of binding to a protein complex comprising an E2 ubiquitin-conjugating enzyme and an E3 ubiquitin ligase protein.

As used herein, the term "precursor compound" refers to a compound that has not been contacted with a serine residue of a Ras G12S mutant protein. A precursor compound may comprise a moiety capable of or susceptible to reacting with a serine residue, e.g., the serine residue corresponding to position 12 of SEQ ID No: 1. Typically, a precursor compound comprises a staying group and a leaving group, wherein upon contacting a precursor compound with an unmodified protein, such as a Ras G12S mutant protein, the side chain of a serine residue corresponding to position 12 of SEQ ID No: 1 reacts with the precursor compound to form a covalent bond with the staying group while a bond between the leaving group and the staying group breaks, resulting in the separation of the leaving group from the remainder of the precursor compound.

- drawn across a bond or at the end of a bond indicates the location of a bond disconnection or attachment (e.g., location of a bond to another atom) of the depicted chemical formula or atom to a substituent, a further component of a molecule, or an atom. For example, 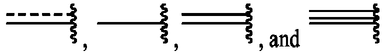 indicate a single or double bond, single bond, double bond, and triple bond, respectively, wherein the 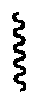 is as defined immediately above. A waved line " 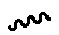 " drawn across a bond or a dashed bond " 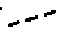 " are used interchangeably herein to denote where a bond disconnection or attachment occurs.

### Modified Mutant Proteins and Compounds

In one aspect, provided is a modified Ras mutant protein comprising a compound covalently bonded to its serine residue, wherein the covalently bonded compound comprises a staying group having a formula: 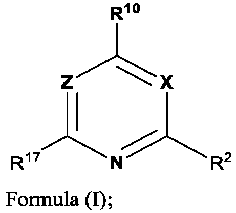 wherein
Z is N and X is C(R³); or Z is C(R⁸) and X is N;
R¹⁰ is -L⁷-R⁷;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -C₀₋₃alkyl-C(O)-, -C₀₋₃alkyl-N(R¹⁴)C(O)-, -C₀₋₃alkyl-S(O)-, -C₀₋₃alkyl-N(R¹⁴)S(O)-, -C₀₋₃alkyl-S(O)₂-, or -C₀₋₃alkyl-N(R¹⁴)S(O)₂-; wherein R⁶ is directly bonded to the serine corresponding to position 12 of SEQ ID No: 1;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³),-N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloallcyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C2-6alkenyl, C2-6alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆allcyl, C₁₋₆haloallcyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

The modified Ras mutant protein may comprise a covalently bonded compound comprising a staying group having a formula: 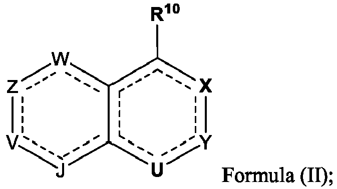 Wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -C₀₋₃alkyl-C(O)-, -C₀₋₃alkyl-N(R¹⁴)C(O)-, -C₀₋₃alkyl-S(O)-, -C₀₋₃alkyl-N(R¹⁴)S(O)-, -C₀₋₃alkyl-S(O)₂-, or -C₀₋₃alkyl-N(R¹⁴)S(O)₂-; wherein R⁶ is directly bonded to the serine residue corresponding to position 12 of SEQ ID No: 1;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{lb} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆allcyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆allcyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆allcyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, and R²⁰¹ are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In an aspect provided is a complex of formula: 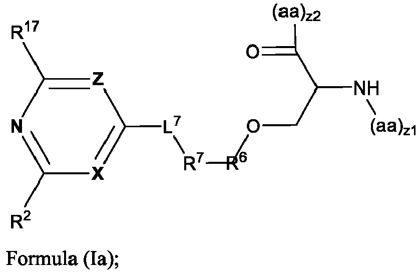 wherein
each aa is independently an amino acid;
z1 is an integer equal to at least 1 or (aa)z1 is hydrogen;
z2 is an integer equal to at least 1 or (aa)z2 is OH;
Z is N or C(R⁸);
X is C(R³) or N;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are optionally substituted with one or more R¹ and/or one or more R⁴;;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -C₀₋₃alkyl-C(O)-, -C₀₋₃alkyl-N(R¹⁴)C(O)-, -C₀₋₃alkyl-S(O)-, -C₀₋₃alkyl-N(R¹⁴)S(O)-, -C₀₋₃alkyl-S(O)₂-, or -C₀₋₃alkyl-N(R¹⁴)S(O)₂-; wherein R⁶ is directly bonded to the serine corresponding to position 12 of SEQ ID No: 1;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In one aspect provided is a complex of formula: 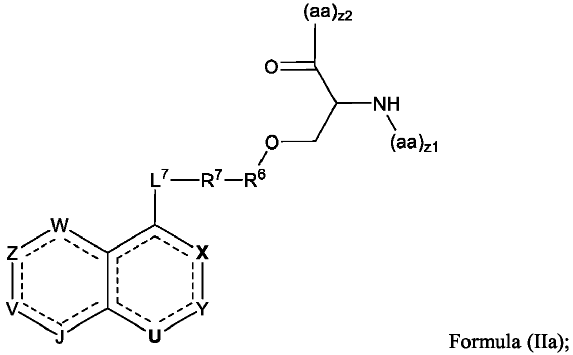 wherein
each aa is independently an amino acid;
z1 is an integer equal to at least 1 or (aa)z1 is hydrogen;
z2 is an integer equal to at least 1 or (aa)z2 is OH;
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloallcyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are optionally substituted with one or more R¹ and/or one or more R⁴;;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -C₀₋₃alkyl-C(O)-, -C₀₋₃alkyl-N(R¹⁴)C(O)-, -C₀₋₃alkyl-S(O)-, -C₀₋₃alkyl-N(R¹⁴)S(O)-, -C₀₋₃alkyl-S(O)₂-, or -C₀₋₃alkyl-N(R¹⁴)S(O)₂-; wherein R⁶ is directly bonded to the serine corresponding to position 12 of SEQ ID No: 1;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆allcyl, C₁₋₆haloallcyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ib), or a pharmaceutically acceptable salt or solvate thereof: 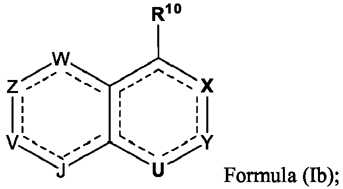 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C_{0- 3}alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C_{0- 3}alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆allcyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{10d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloallcyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ic), or a pharmaceutically acceptable salt or solvate thereof: 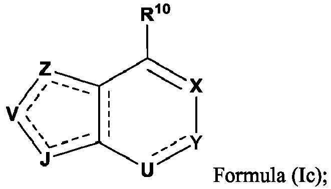 wherein
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R2)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C_{0- 3}alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C_{0- 3}alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆allcyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰¹;
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆allcyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Id), or a pharmaceutically acceptable salt or solvate thereof: 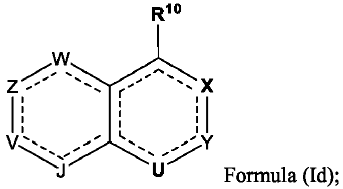 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R²), C(R^{2c})(R^{2c}), N(R^{2b}), S(0), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three- R^{20a};
R⁷ is a 7-12 membered nitrogen containing spirocyclic heterocycloalkyl substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C_{0- 3}alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C_{0- 3}alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)_{2N}(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In an aspect provided is a precursor compound of Formula (Ie), or a pharmaceutically acceptable salt or solvate thereof: 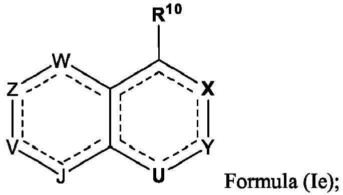 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three- R^{20a};
R⁷ is a 7-12 membered nitrogen containing fused heterocycloalkyl, 7-12 membered nitrogen containing fused aryl, or 7-12 membered nitrogen containing fused heteroaryl substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -Co. ₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C_{0- 3}alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (If), or a pharmaceutically acceptable salt or solvate thereof: 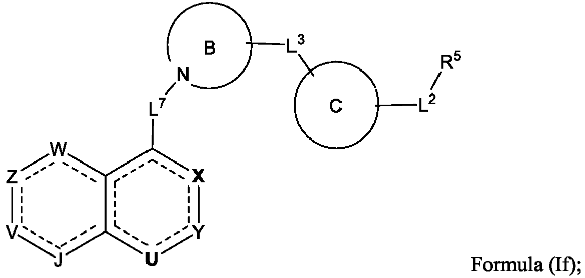 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y and U are independently selected from N, C(R²), C(R²)(R²), N(R^{2b}), S(O), S(O)₂, and C(O);
L⁷ is a bond, -C(O)-, -S-, -S(O)₂-, -S(O)-, -N(R^{14c})-C₁₋₃alkyl-, or C₁₋₄alkyl, wherein the C₁₋₃alkyl and C_{1- 4}alkyl are optionally substituted with one, two or three- R^{20a};
Ring B is a 3-12 membered nitrogen containing saturated heterocycloalkyl optionally substituted with one or more R¹ and/or one or more R⁴;
Ring C is a 3-12 membered nitrogen containing saturated heterocycloalkyl or C₃₋₆cycloalkyl, each optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -Co. ₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R^{1a})-C_{0- 3}alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is independently hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₉alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

The precursor compound described herein, wherein exactly one of V and J is substituted with R¹⁷ or R^{17b}.

In an aspect provided is a precursor compound of Formula (Ih), or a pharmaceutically acceptable salt or solvate thereof: 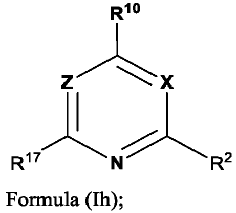 wherein
Z is N and X is C(R³); or Z is C(R⁸) and X is N;
R¹⁰ is -L⁷-R⁷;
L7 is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 5-12 membered nitrogen containing heteroaryl, wherein the 6-12 membered nitrogen containing heterocycloalkyl or 5-12 membered nitrogen containing heteroaryl are substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH2-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -Co. ₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C₀. ₃alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
R² is halogen, -CN, C₂₋₆alkyl, C₂₋₆alkenyl, C₂₋₆alkynyl, C₃₋₆cycloalkyl, -OR^{12c}, -SR¹², -N(R^{12c})(R^{12c}), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₂₋₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₂₋₆alkyl, C_{2- 6}alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In one aspect provided is a precursor compound of Formula (Ii), or a pharmaceutically acceptable salt or solvate thereof: 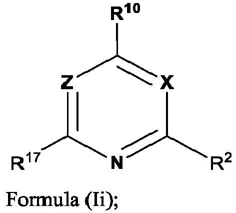 wherein
Z is N and X is C(R³); or Z is C(R⁸) and X is N;
R¹⁰ is -L⁷-R⁷;
L7 is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing spirocyclic heterocycloalkyl substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -Co_ ₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-Co. ₃alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl. -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In one aspect provided is a precursor compound of Formula (Ij), or a pharmaceutically acceptable salt or solvate thereof: 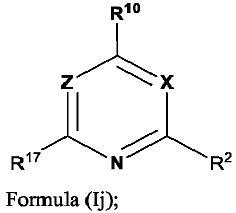 wherein
Z is N and X is C(R³); or Z is C(R⁸) and X is N;
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing fused heterocycloalkyl, fused 7-12 membered nitrogen containing aryl, or fused 7-12 membered nitrogen containing heteroaryl substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -Co. ₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C_{0- 3}alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, -OR¹², -SR¹², -N(R^{12c})(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, or -(C₆₋₁₀aryl)-R^{12b}, wherein said C₁₋₆alkyl, C₂₋₆alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl. -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In one aspect provided is a precursor compound of Formula (Ik), or a pharmaceutically acceptable salt or solvate thereof: 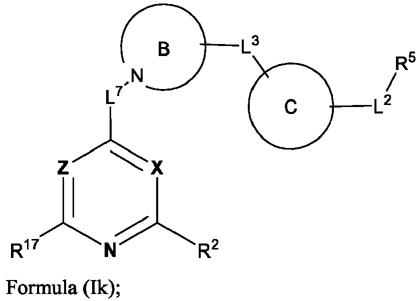 wherein
Z is N and X is C(R³); or Z is C(R⁸) and X is N;
L⁷ is a bond, -C(O)-, -S-, -S(O)₂-, -S(O)-, -N(R^{14c})-C₁₋₃alkyl-, or C₁₋₄alkyl, wherein the C₁₋₃alkyl and C_{1- 4}alkyl are optionally substituted with one, two or three R^{20a};
Ring B is a 4-6 membered nitrogen containing saturated heterocycloalkyl optionally substituted with one or more R¹ and/or one or more R⁴;
Ring C is a 4-6 membered nitrogen containing saturated heterocycloalkyl or C₃₋₆cycloalkyl, each optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -Co_ ₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C_{0- 3}alkyl-, or -C₀₋₃alkyl-N(R¹⁴)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹²,-N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=0)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹;
each R¹¹ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=0)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=0)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; wherein one of R² and R¹⁷ is not hydrogen;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In an aspect provided is a precursor compound of Formula (Ib), or a pharmaceutically acceptable salt or solvate thereof: 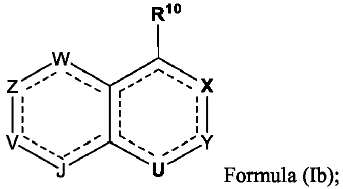 wherein
W is N, C(R¹⁸), N(R^{18b}), C(R¹⁸)(R^{18a}), C(0), S(0), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(W)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14C})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₁-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=0)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), ^{_}CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R¹⁹);
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=0)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=0)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=0)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=0)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=0)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₁₀cycloallcyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆allcyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ic), or a pharmaceutically acceptable salt or solvate thereof: 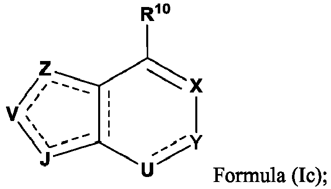 wherein
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(H)(R¹²), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉ heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and Cl-9heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Id), or a pharmaceutically acceptable salt or solvate thereof: 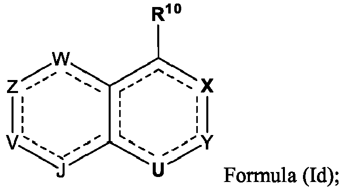 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three- R^{20a};
R⁷ is a 7-12 membered nitrogen containing spirocyclic heterocycloalkyl substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is-C₀₋₃alkyl-C(O)O-, -C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(H)(R¹²), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}.
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}.
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH2-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋9heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ie), or a pharmaceutically acceptable salt or solvate thereof: 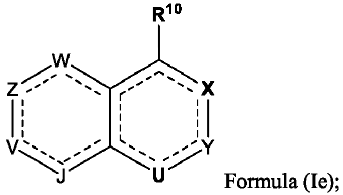 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14C})-C₁₋₃alkyl-, or C₁₋₄alkyl, wherein the C_{1- 3}alkyl and C₁₋₄alkyl are optionally substituted with one, two or three- R^{20a};
R⁷ is a 7-12 membered nitrogen containing fused heterocycloalkyl, 7-12 membered nitrogen containing fused aryl, or 7-12 membered nitrogen containing fused heteroaryl substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}.
R⁶ is -L²-R⁵;
each L² is -C₀₋₃alkyl-C(O)O-,-C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(H)(R¹²), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloallcyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆allcyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
------ indicates a single or double bond such that all valences are satisfied.

In an aspect provided is a precursor compound of Formula (If), or a pharmaceutically acceptable salt or solvate thereof: 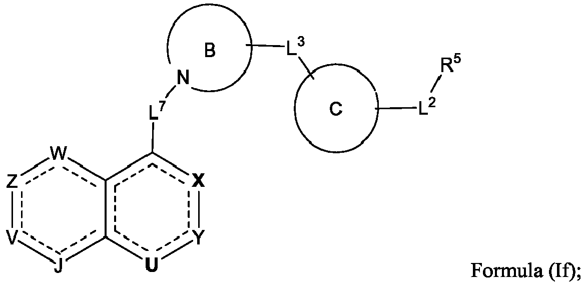 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y and U are independently selected from N, C(R²), C(R²)(R²), N(R^{2b}), S(O), S(O)₂, and C(O);
L⁷ is a bond, -C(O)-, -S-, -S(O)₂-, -S(O)-, -N(R^{14c})-C₁₋₃alkyl-, or C₁₋₄alkyl, wherein the C₁₋₃alkyl and C_{1- 4}alkyl are optionally substituted with one, two or three- R^{20a};
Ring B is a 3-12 membered nitrogen containing saturated heterocycloalkyl optionally substituted with one or more R¹ and/or one or more R⁴;
Ring C is a 3-12 membered nitrogen containing saturated heterocycloalkyl or C₃₋₁₀cycloalkyl, each optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
each L² is -C₀₋₃alkyl-C(O)O-,-C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(H)(R¹²), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloallcyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is independently hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C_{1-g}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R 20h;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

The precursor compound described herein, wherein exactly one of V and J is substituted with R¹⁷ or R^{17b}.

In an aspect provided is a precursor compound of Formula (Ih), or a pharmaceutically acceptable salt or solvate thereof: 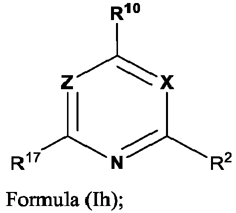 wherein
Z is N and X is C(R³); or Z is C(R⁸) and X is N;
R¹⁰ is -L⁷-R⁷;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 5-12 membered nitrogen containing heteroaryl, wherein the 6-12 membered nitrogen containing heterocycloalkyl or 5-12 membered nitrogen containing heteroaryl are substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
R² is halogen, -CN, C₂₋₆alkyl, C₂₋₆alkenyl, C₂₋₆alkynyl, C₃₋₁₀cycloalkyl, -OR^{12c}, -SR¹², -N(R^{12c})(R^{12c}), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₂₋₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₂₋₆alkyl, C_{2- 6}alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} are each independently selected from halogen, - CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In one aspect provided is a precursor compound of Formula (Ii), or a pharmaceutically acceptable salt or solvate thereof: 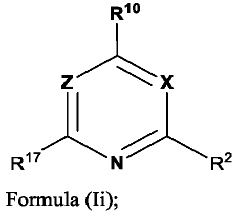 wherein
Z is N and X is C(R³); or Z is C(R⁸) and X is N;
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing spirocyclic heterocycloalkyl substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloallcyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- g}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
------ indicates a single or double bond such that all valences are satisfied.

In one aspect provided is a precursor compound of Formula (Ij), or a pharmaceutically acceptable salt or solvate thereof: 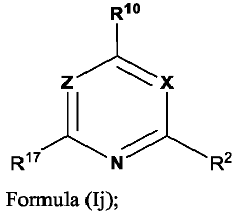 wherein
Z is N and X is C(R³); or Z is C(R⁸) and X is N;
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing fused heterocycloalkyl, fused 7-12 membered nitrogen containing aryl, or fused 7-12 membered nitrogen containing heteroaryl substituted with one R⁶ and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-,-C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₁₀cycloalkyl, -OR¹², -SR¹², -N(R^{12c})(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, or -(C₆₋₁₀aryl)-R^{12b}, wherein said C₁₋₆alkyl, C₂₋₆alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
------ indicates a single or double bond such that all valences are satisfied.

In one aspect provided is a precursor compound of Formula (Ik), or a pharmaceutically acceptable salt or solvate thereof: 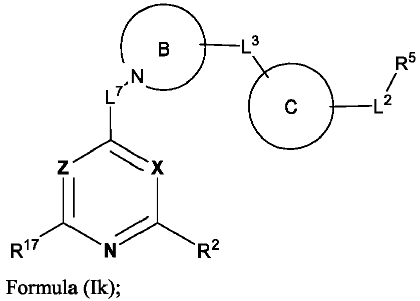 wherein
Z is N and X is C(R³); or Z is C(R⁸) and X is N;
L⁷ is a bond, -C(O)-, -S-, -S(O)₂-, -S(O)-, -N(R^{14c})-C₁₋₃alkyl-, or C₁₋₄alkyl, wherein the C₁₋₃alkyl and C_{1- 4}alkyl are optionally substituted with one, two or three R^{20a};
Ring B is a 4-6 membered nitrogen containing saturated heterocycloalkyl optionally substituted with one or more R¹ and/or one or more R⁴;
Ring C is a 4-6 membered nitrogen containing saturated heterocycloalkyl or C₃₋₁₀cycloalkyl, each optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
each L² is -C₀₋₃alkyl-C(O)O-, -C₀₋₃alkyl-C(O)-, -C₀₋₃alkyl-C(O)N(R¹⁴)-C₀₋₃alkyl-, -C₀₋₃alkyl-N(R¹⁴)C(O)-, - C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{I1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)2N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; wherein one of R² and R¹⁷ is not hydrogen;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} is independently selected from halogen, -CN, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
------ indicates a single or double bond such that all valences are satisfied.

In some aspects, a subject precursor compound exhibits one or more of the following characteristics: it is capable of reacting with a serine residue (including serine residue 12 corresponding to SEQ IN NO. 1, 3, or 5) of a Ras mutant protein and covalently modify such Ras mutant; it comprises a moiety susceptible to reacting with a nucleophilic serine residue corresponding to position 12 of SEQ ID No: 1, 3, or 5; it comprises a staying group and a leaving group, upon contacting an unmodified Ras G12S mutant protein to yield a covalently modified Ras G12S mutant, the leaving group separates from remainder of the precursor compound with an electron pair that previously formed a covalent bond between the leaving group and the remainder of the precursor compound; it selectively labels Ras G12S mutant relatively to other mutants including Ras G12D, Ras G12V and also wildtype Ras proteins. In some embodiments, a subject precursor compound when used to modify a Ras mutant protein, reduces the Ras protein's signaling output. In some embodiments, a subject precursor compound exhibits an IC50 (against K-Ras 12S, as ascertained by reduction of K-Ras::SOS1 interaction) less than 500 nM, less than 100 nM, less than 50 nM, 10 nM, 5 nM, 1nM, 500 pM, 50 pM, 10 pM or less.

In embodiments, L² is -C₀₋₃alkyl-C(O)-. In embodiments, L² is -C₀₋₃alkyl-C(O)N(R¹⁴)-C₀₋₃alkyl-. In embodiments, L² is -C₀₋₃alkyl-N(R¹⁴)C(O)-. In embodiments, L² is -C₀₋₃alkyl-S(O)-. In embodiments, L² is -C₀₋₃alkyl-S(O)N(R¹⁴)-C₀₋₃alkyl-. In embodiments, L² is -C₀₋₃alkyl-N(R¹⁴)S(O)-. In embodiments, L² is - C₀₋₃alkyl-S(O)₂-. In embodiments, L² is -C₀₋₃alkyl-S(O)₂N(R¹⁴)-C₀₋₃alkyl-. In embodiments, L² is -C_{0- 3}alkyl-N(R¹⁴)S(O)₂-.

In embodiments, L² is -C₀₋₃alkyl-C(O)-. In embodiments, L² is -C₀₋₃alkyl-C(O)N(R¹⁴)-. In embodiments, L² is -C₀₋₃alkyl-N(R¹⁴)C(O)-. In embodiments, L² is -C₀₋₃alkyl-S(O)-. In embodiments, L² is -C_{0- 3}alkyl-S(O)N(R¹⁴)-. In embodiments, L² is -C₀₋₃alkyl-N(R¹⁴)S(O)-. In embodiments, L² is -C₀₋₃alkyl-S(O)₂-. In embodiments, L² is -C₀₋₃alkyl-S(O)₂N(R¹⁴)-. In embodiments, L² is -C₀₋₃alkyl-N(R¹⁴)S(O)₂-.

In embodiments, L² is -C(O)N(R¹⁴)-C₀₋₃alkyl-. In embodiments, L² is -S(O)N(R¹⁴)-C₀₋₃alkyl-. In embodiments, L² is -S(O)₂-C₀₋₃alkyl-. In embodiments, L² is -S(O)₂N(R¹⁴)-C₀₋₃alkyl-.

In embodiments, L² is -CH₂-C(O)-. In embodiments, L² is -CH₂-C(O)N(R¹⁴)-. In embodiments, L² is - CH₂-N(R¹⁴)C(O)-. In embodiments, L² is -CH₂-S(O)-. In embodiments, L² is -CH₂-S(O)N(R¹⁴)-. In embodiments, L² is -CH₂-N(R¹⁴)S(O)-. In embodiments, L² is -CH₂-S(O)₂-. In embodiments, L² is - CH₂-S(O)₂N(R¹⁴)-. In embodiments, L² is -CH₂-N(R¹⁴)S(O)₂-.

In embodiments, L² is -C(O)N(R¹⁴)-CH₂-. In embodiments, L² is -S(O)N(R¹⁴)-CH₂-. In embodiments, L² is -S(O)₂N(R¹⁴)-CH₂-.

In some embodiments, a modified Ras mutant protein disclosed herein exhibits a reduced Ras signaling output. A reduction of signaling output can be ascertained by a wide variety of methods known in the art. For example, phosphorylation of a substrate or a specific amino acid residue thereof can be detected and/or quantified one or more techniques, such as kinase activity assays, phospho-specific antibodies, Western blot, enzyme-linked immunosorbent assays (ELISA), cell-based ELISA, intracellular flow cytometry, mass spectrometry, and multi-analyte profiling. A host of readout can evidence a reduction of Ras signaling output including without limitation: (i) an increase in steady state level of GDP-bound modified protein; (ii) a reduction of phosphorylated AKTs473, (iii) a reduction of phosphorylated ERK T202/Y204, (iv) a reduction of phosphorylated S6 S235/236, and (v) reduction of cell growth of a tumor cell expressing a Ras G12S mutant protein, and (vi) reduction in Ras interaction with a Ras-pathway signaling protein.. In some embodiments, a reduction is evidenced by 2, 3, 4 or more of items (i)- (vi). In some embodiments, the reduction in Ras signaling output can be evidenced by any one of (i) - (vi) as compared to control unmodified corresponding Ras proteins that is not covalently bonded to any compound disclosed herein. For example, a control Ras protein, as described herein, can be a Ras protein (e.g., wildtype or mutated) that is not complexed with any subject compound of the present disclosure. The increase in item (i) or reduction in items (ii) through (vi) can be at least about 0.1-fold, 0.2-fold, 0.3-fold, 0.4-fold, 0.5-fold, 0.6-fold, 0.7-fold, 0.8-fold, 0.9-fold, 1-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, 200-fold, 300-fold, 400-fold, 500-fold, 600-fold, 700-fold, 800-fold, 900-fold, 1000-fold, 2000-fold, 3000-fold, 4000-fold, 5000-fold, or more as compared to the control Ras proteins. In some embodiments, a reduction in Ras interaction with a Ras-pathway signaling protein is established by a reduced interaction with SOS (including SOS 1 and SOS2), RAF, SHC, SHP (including SHP1 and SHP2), MEK, MAPK, ERK, GRB, RASA1, and GNAQ.

In embodiments the modified Ras mutant protein described herein is formed by contacting a precursor compound with the serine residue of an unmodified Ras G12S mutant protein, wherein the precursor compound comprises a moiety susceptible to reacting with a nucleophilic serine residue corresponding to position 12 of SEQ ID No: 1. Non-limiting examples of a moiety susceptible to reaction with a nucleophilic serine residue of a K-Ras G12S protein comprise an optionally substitituted azetidin-2-one, optionally substitituted 1,2-thiazetidine 1,1-dioxide, optionally substituted lactam, or optionally substituted amide.

In embodiments the modified K-Ras G12S protein described herein that is formed by contacting a precursor compound with the serine residue of an unmodified Ras G12S mutant protein, wherein the precursor compound comprises a staying group and a leaving group, and upon contacting the precursor compound with an unmodified Ras G12S mutant protein, said leaving group separates from remainder of the precursor compound with an electron pair that previously formed a covalent bond between the leaving group and the remainder of the precursor compound.

In embodiments the modified K-Ras G12S protein described herein that is formed by contacting a precursor compound with the serine residue of an unmodified Ras G12S mutant protein, wherein the precursor compound comprises a staying group and a leaving group, and wherein said contacting results in release of the leaving group and formation of said modified protein.

Release of the leaving group can be ascertained by a variety of methods known in the art including without limitation mass spectroscopy. In particular, the molecular weight of the leaving group can be determined by the following formula: $L = \left[U+P\right] - \left[M\right]$ wherein:
L is the molecular weight of the leaving group; U is the molecular weight of an unmodified Ras G12S mutant; P is the molecular weight of a subject precursor compound used to modify the unmodified Ras G12S mutant; M is the molecular weight of the modified Ras G12S mutant covalently bond to the precursor excluding the leaving group. The molecular weight of the modified Ras G12S Mutant can be ascertained by mass spectroscopy.

In some embodiments, a subject precursor compound upon contacting an unmodified Ras G12S mutant protein (e.g., K-Ras G12S, H-Ras G12S, or N-Ras G12S) yields a leaving group of a molecular weight less than about 150, 140, 130, 120, 110, 100, 90, 80, 70, 60, 50, 40, 30, 20 or less. One or more exemplified compounds herein (including compounds in Table 3) yields, upon contacting K-Ras G12S, a leaving group of molecular weight less than 100 (e.g., leaving groups of compounds 101, 102, 103, 104, 105 are respectively, 68, 69, 68, 68, and 92).

Signaling output measured in terms of IC50 values can be obtained, a ratio of IC50 against one mutant relative to another mutant can be calculated. For instance, a selective reduction of K-Ras G12S signaling output can be evidenced by a ratio greater than one. In particular, a selective reduction of K-Ras G12S signaling relative to K-Ras G12D signaling is evidenced as the ratio of IC50 (against K-Ras G12D) to IC50 (against K-Ras G12S) is greater than 1. As is summarized below in Table 4, one or more subject precursor compounds disclosed herein (including compounds shown in Table 3) exhibit selective inhibition of K-Rras G12S relative to K-Ras G12D by at least 1-fold, and in some instances greater than 2-, 3-, 4- or 5-fold. A subject precursor compounds including the compounds disclosed in Table 3 exhibit an IC50 against K-Ras G12S less than 500 nM, less than 100 nM, 50 nM, 10 nM or even less.

Applying the method exemplified herein, subject precursor compounds exhibit selective labeling of Ras G12S mutant relative to Ras G12D or wildtype mutant. Table 4 disclosed herein summarizes the results that exemplary precursor compounds including those shown in Table 3 can covalently label K-Ras G12S mutant by at least 1%, 10%, 20%, 50% or more, wheras no detectable labeling was registered for K-Ras G12D or wildtype when tested under comparable or same conditions.

In embodiments of the modified K-Ras G12S protein described herein, the precursor compound selectively labels the serine residue as compared to (i) an aspartate residue of a K-Ras G12D mutant protein, said aspartate corresponding to residue 12 of SEQ ID NO: 7, and/or (ii) a valine residue of a K-Ras G12V mutant protein, said valine corresponding to residue 12 of SEQ ID NO: 8.

It will be understood that when a precursor compound selectively labels the serine residue of a K-Ras G12S protein compared to another K-Ras protein(s) (e.g., WT, G12D, G12V), the precursor compound labels the K-Ras G12S protein with greater speed or to a greater degree or by any other quantifiable measurement compared to the other K-Ras protein (e.g., WT, G12D, G12V), under similar or identical reaction conditions for the proteins being compared. In some embodiments, the greater labeling of K-Ras G12S can be 0.1-fold, 0.2-fold, 0.3-fold, 0.4-fold, 0.5-fold, 0.6-fold, 0.7-fold, 0.8-fold, 0.9-fold, 1-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, 200-fold, 300-fold, 400-fold, 500-fold, 600-fold, 700-fold, 800-fold, 900-fold, 1000-fold, 2000-fold, 3000-fold, 4000-fold, 5000-fold, or more as compared to another K-Ras protein (e.g., WT, G12D, G12V).

In embodiments of the modified K-Ras G12S protein described herein, the precursor compound selectively labels the serine residue as compared to (i) an aspartate residue of a K-Ras G12D mutant protein, said aspartate corresponding to residue 12 of SEQ ID NO: 7, and/or (ii) a valine residue of a K-Ras G12V mutant protein, said valine corresponding to residue 12 of SEQ ID NO: 8, by at least 0.1, 0.2, 0.3, 0.4, 0.5, 1, 2, 3, 4, 5, 10 folds or more, when assayed under comparable conditions.

In embodiments of the modified K-Ras G12S protein described herein, the precursor compound contacts the serine residue of an unmodified Ras G12S protein corresponding to position 12 of SEQ ID No: 1 in vitro.

In embodiments of the modified K-Ras G12S protein described herein, the precursor compound contacts the serine residue of an unmodified Ras G12S protein corresponding to position 12 of SEQ ID No: 1 in vivo.

In some embodiments of the modified Ras mutant protein described herein, the covalently bonded compound comprises a staying group having a formula: 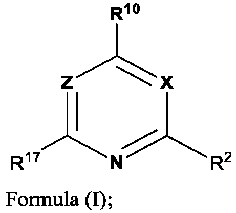 wherein
Z is N and X is C(R³); or Z is C(R⁸) and X is N;
R¹⁰ is -L⁷-R⁷;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁-₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20a};
R⁶ is -C(O)-; wherein R⁶ is directly bonded to the serine corresponding to position 12 of SEQ ID No: 1;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;R¹⁹ is selected from a C_{3- 12}cycloalkyl, C₂₋₁₁heterocycloalkyl, C₆₋₁₂aryl, and C₂₋₁₂heteroaryl, wherein the C₃₋₁₂cycloalkyl, C_{2- 11}heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C_{1- 6}alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In embodiments a fused ring is a bridged ring system.

In some embodiments of the modified Ras mutant protein described herein, the covalently bonded compound comprises a staying group having a formula: 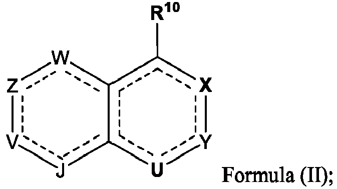 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20a};
R⁶ is -C(O)-; wherein R⁶ is directly bonded to the serine corresponding to position 12 of SEQ ID No: 1;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -{C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰¹;
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20b}, and R²⁰ⁱ are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C_{1- 6}alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³),-C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In embodiments of the modified Ras mutant protein described herein, the leaving group has a formula:
A) 5 membered partially unsaturated heterocycloalkyl or a 5 membered heteroaryl, each optionally substituted with one, two or three R^{20k}, wherein the partially unsaturated heterocycloalkyl or heteroaryl comprises two or three ring nitrogen atoms; or
B) 5 or 6 membered partially unsaturated heterocycloalkyl or a 5 or 6 membered heteroaryl, each optionally substituted with one, two or three R^{20k}, wherein the partially unsaturated heterocycloalkyl or heteroaryl comprises two or three ring nitrogen atoms; and further wherein the partially unsaturated heterocycloalkyl or heteroaryl is substituted with a -NH(R¹⁴) bonded to a ring carbon of the partially unsaturated heterocycloalkyl or heteroaryl;

each R^{20k} is independently selected from halogen, -CN, C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and-OC(O)R²⁵; or two R^{20k} bonded to the same or adjacent atoms may optionally be joined to form a C_{3- 6}cycloalkyl, C₂₋₉heterocycloalkyl, C₆₋₁₀aryl, -CH₂-C₆₋₁₀aryl, or C₁₋₉heteroaryl; wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from H and C₁₋₆alkyl; and
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl.

In embodiments of the modified Ras mutant protein described herein, the leaving group has a formula: 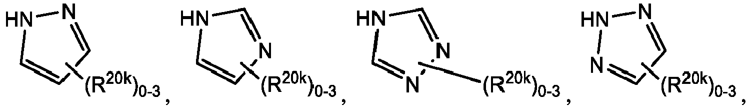 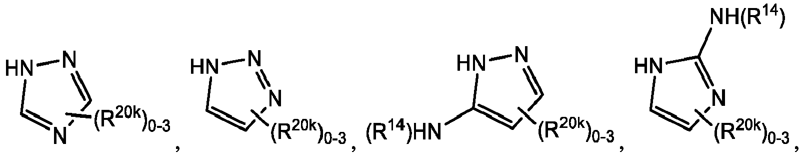 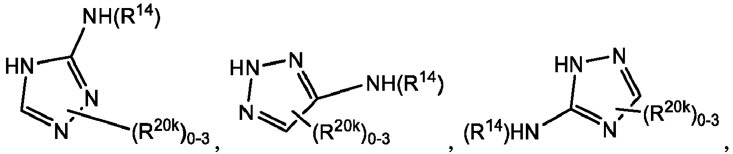 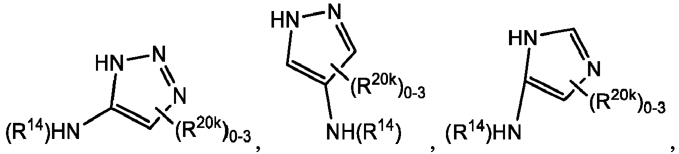 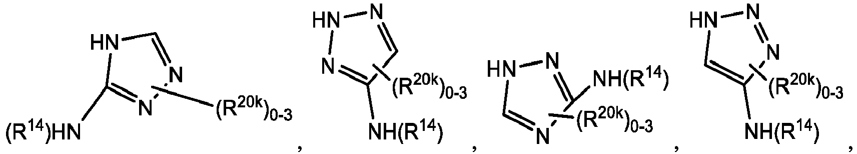 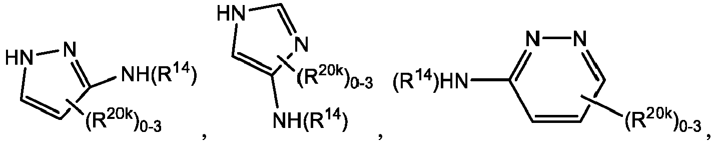 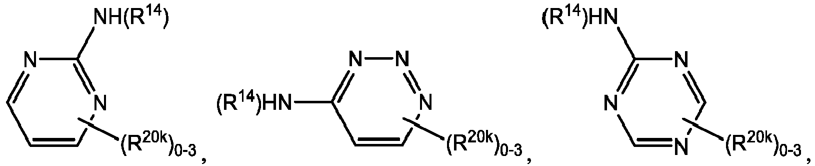 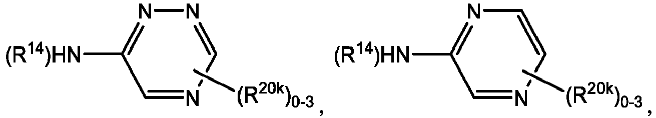 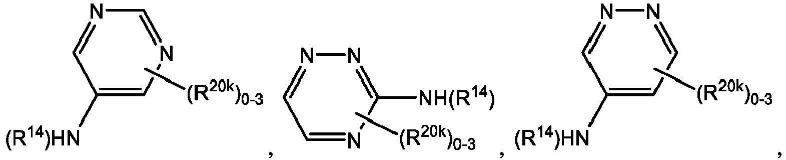 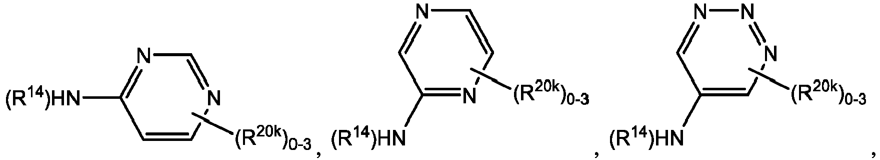 or 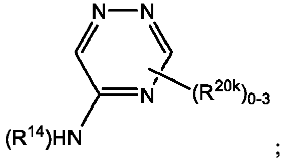
each R¹⁴ is independently selected from hydrogen, C₁₋₆alkyl, and C₁₋₆haloalkyl;
each R^{20k} is independently selected from halogen, -CN, C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and-OC(O)R²⁵; or two R^{20k} bonded to the same or adjacent atoms may optionally be joined to form a C_{3- 6}cycloalkyl, C₂₋₉heterocycloalkyl, C₆₋₁₀aryl, -CH₂-C₆₋₁₀aryl, or C₁₋₉heteroaryl; wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉ heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from H and C₁₋₆alkyl; and
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl.

In an aspect is provided a complex of formula: 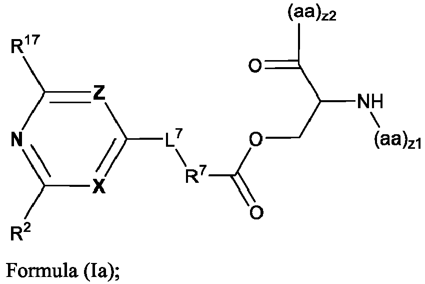 wherein
each aa is independently an amino acid;
z1 is an integer equal to at least 1 or (aa)z1 is hydrogen;
z2 is an integer equal to at least 1 or (aa)z2 is OH;
Z is N or C(R⁸);
X is C(R³) or N;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴; and further wherein R⁷ is bonded to C(O) in Formula (Ia) through a ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)2N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (Ia), R⁷ is a 3-12 membered nitrogen containing heterocycloalkyl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴.

In some embodiments of the complex of Formula (Ia),
Z is N and X is C(R³); or Z is C(R⁸) and X is N;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 5-12 membered nitrogen containing heteroaryl, wherein the 6-12 membered nitrogen containing heterocycloalkyl or 5-12 membered nitrogen containing heteroaryl are optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₂₋₆alkyl, C₂₋₆alkenyl, C₂₋₆alkynyl, C₃₋₆cycloalkyl, -OR^{12c}, -SR¹², -N(R^{12c})(R^{12c}), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₂₋₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₂₋₆alkyl, C_{2- 6}alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (Ia),
Z is N and X is C(R³); or Z is C(R⁸) and X is N;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing spirocyclic heterocycloalkyl optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (Ia),
Z is N and X is C(R³); or Z is C(R⁸) and X is N;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing fused heterocycloalkyl, 7-12 membered nitrogen containing fused aryl, or 7-12 membered nitrogen containing fused heteroaryl optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, -OR¹², -SR¹², -N(R^{12c})(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₁-C₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₁₋₆alkyl, C_{2- 6}alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)2N(R²²)(R²³), -OCH₂C(O)OR²², and -OC(O)R²⁵; wherein C₁₋₆alkyl, C2-6alkenyl, C2-6alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)2N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (Ia), R⁷ is a 7-12 membered nitrogen containing fused heterocycloalkyl or 7-12 membered nitrogen containing fused heteroaryl optionally substituted with one or more R¹ and/or one or more R⁴.

In some embodiments of the complex of Formula (Ia),
Z is N and X is C(R³); or Z is C(R⁸) and X is N;
L⁷ is a bond, -C(O)-, -S-, -S(O)₂-, -S(O)-, -N(R^{14C})-C₁₋₃alkyl-, or C₁₋₄alkyl, wherein the C₁₋₃alkyl and C_{1- 4}alkyl are optionally substituted with one, two or three R^{20a};
R⁷ is a -(4-6 membered nitrogen containing saturated heterocycloalkyl)-(4-6 membered nitrogen containing saturated heterocycloalkyl)-, wherein the -(4-6 membered nitrogen containing saturated heterocycloalkyl)-(4-6 membered nitrogen containing saturated heterocycloalkyl)- is optionally substituted with one or more R¹ and/or one or more R⁴; and further wherein the two -(4-6 membered nitrogen containing saturated heterocycloalkyl are joined by a direct bond between a ring C and ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁-₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁-₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; wherein one of R² and R¹⁷ is not hydrogen;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloallcyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)2N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)2N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a complex of formula: 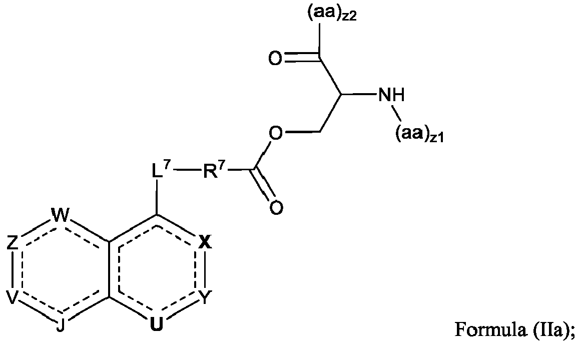 wherein
each aa is independently an amino acid;
z1 is an integer equal to at least 1 or (aa)z1 is hydrogen;
z2 is an integer equal to at least 1 or (aa)z2 is OH;
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R2^{c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴; and further wherein R⁷ is bonded to C(O) in Formula (IIa) through a ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁-₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵,-N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R²⁰¹;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl;
each R²⁵ is independently selected from C₁₋₆alkyl, C₁₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (IIa), R⁷ is a 3-12 membered nitrogen containing heterocycloalkyl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴.

In some embodiments of the complex of Formula (IIa),
W is a bond;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₁₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₁₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₁₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₁₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₁₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₁₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₁₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₁₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₁₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₁₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₁₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₁₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₁₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₁₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₁₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₁₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₁₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₁₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.
R^{2c} is hydrogen, halogen, -CN, C₁₋₆alkyl, C₁₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₁₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is selected from hydrogen, -CN, C₁₋₆alkyl, C₁₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₁₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₁₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₁₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₁₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₁₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₁₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In some embodiments of the complex of Formula (IIa),
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein CI-6alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)2N(R²²)(R²³), -OCH₂C(O)OR²², and -OC(O)R²⁵; wherein Ci.6alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, CI-6alkyl, CI-6haloalkyl, CI-6alkoxy, CI-6haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In some embodiments of the complex of Formula (IIa),
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(0), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing spirocyclic heterocycloalkyl optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁-₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (IIa),
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing fused heterocycloalkyl, 7-12 membered nitrogen containing fused aryl, or 7-12 membered nitrogen containing fused heteroaryl optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In some embodiments of the complex of Formula (IIa), R⁷ is a 7-12 membered nitrogen containing fused heterocycloalkyl or 7-12 membered nitrogen containing fused heteroaryl optionally substituted with one or more R¹ and/or one or more R⁴.

In some embodiments of the complex of Formula (IIa),
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b});
Y and U are independently selected from N, C(R²), C(R²)(R²), N(R^{2b}), S(O), S(O)₂, and C(O);
L⁷ is a bond, -C(O)-, -S-, -S(O)₂-, -S(O)-, -N(R^{14c})-C₁₋₃alkyl-, or C₁₋₄alkyl, wherein the C₁₋₃alkyl and C_{1- 4}alkyl are optionally substituted with one, two or three R^{20a};
R⁷ is a -(4-6 membered nitrogen containing saturated heterocycloalkyl)-(4-6 membered nitrogen containing saturated heterocycloalkyl)-, wherein the -(4-6 membered nitrogen containing saturated heterocycloalkyl)-(4-6 membered nitrogen containing saturated heterocycloalkyl)- is optionally substituted with one or more R¹ and/or one or more R⁴; and further wherein the two -(4-6 membered nitrogen containing saturated heterocycloalkyl are joined by a direct bond between a ring C and Ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ,
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is independently hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In embodiments of the complex described herein, (aa)zl is MTEYKLVWGA- and (aa)z2 is - GVGKSALTIQLIQNHFVDEYDPTIEDSYRKQWIDGETCLLDILDTAGQEEYSAMRDQYMRTGEGF LCVFAINNTKSFEDIHHYREQIKRVKDSEDVPMVLVGNKCDLPSRTVDTKQAQDLARSYGIPFIETS AKTRQGVDDAFYTLVREIRKHKEKMSKDGKKKKKKSKTKCVIM wherein the backbone C(O) of (aa)zl A11 is directly bonded to the NH of a formulae described herein including (aa)zl and the NH of (aa)z2 G13 is directly bonded to the C(O) of a formulae described herein including (aa)z2.

In embodiments of the complex described herein, (aa)zl is MTEYKLVWGA- and (aa)z2 is - GVGKSALTIQLIQNHFVDEYDPTIEDSYRKQWIDGETCLLDILDTAGQEEYSAMRDQYMRTGEGF LCVFAINNTKSFEDIHQYREQIKRVKDSEDVPMVLVGNKCDLPSRTVDTKQAQDLARSYGIPFIETS AKTRQGVDDAFYTLVREIRKHKEKMSKDGKKKKKKSKTKCVIM.

In embodiments of the complex described herein, (aa)zl is MTEYKLVWGA- and (aa)z2 is - GVGKSALTIQLIQNHFVDEYDPTIEDSYRKQWIDGETCLLDILDTAGQEEYSAMRDQYMRTGEGF LCVFAINNTKSFEDIHLYREQIKRVKDSEDVPMVLVGNKCDLPSRTVDTKQAQDLARSYGIPFIETS AKTRQGVDDAFYTLVREIRKHKEKMSKDGKKKKKKSKTKCVIM.

In an aspect is provided a precursor compound of Formula (Ib), or a pharmaceutically acceptable salt or solvate thereof: 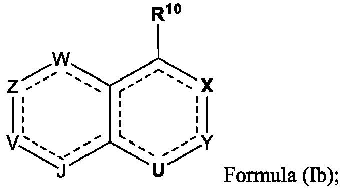 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C(O)- or -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl. -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein CI-6alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - - indicates a single or double bond such that all valences are satisfied.

In an aspect is provided a precursor compound of Formula (Ic), or a pharmaceutically acceptable salt or solvate thereof: 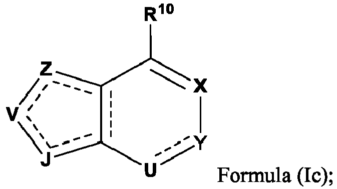 wherein
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is independently selected from a -C(O)- and -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -{C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉ heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)2R²⁵, -C(O)R²⁵, -S(O)2R²⁵, -S(O)2N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a precursor compound of Formula (Id), or a pharmaceutically acceptable salt or solvate thereof: 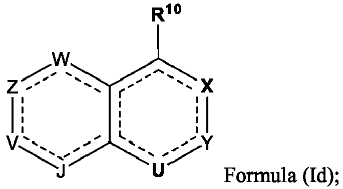 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing spirocyclic heterocycloalkyl substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C(O)- or -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a precursor compound of Formula (Ie), or a pharmaceutically acceptable salt or solvate thereof: 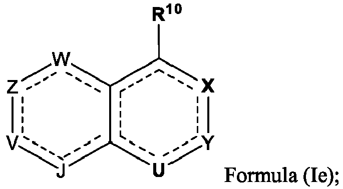 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing fused heterocycloalkyl, 7-12 membered nitrogen containing fused aryl, or 7-12 membered nitrogen containing fused heteroaryl substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is independently selected from a -C(O)- and -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}), -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In embodiments (e.g., of a precursor compound of Formula Ie) exactly one of V and J is substituted with R¹⁷ or R^{17b}.

In embodiments, R⁷ is a 7-12 membered nitrogen containing fused heterocycloalkyl or 7-12 membered nitrogen containing fused heteroaryl substituted with one R⁶ directly bonded to a ring N and optionally substituted with one or more R¹ and/or one or more R⁴.

In an aspect is provided a precursor compound of Formula (II), or a pharmaceutically acceptable salt or solvate thereof: 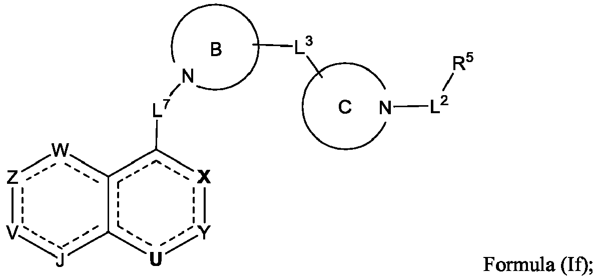 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y and U are independently selected from N, C(R²), C(R²)(R²), N(R^{2b}), S(O), S(O)₂, and C(O);
L⁷ is a bond, -C(O)-, -S-, -S(O)₂-, -S(O)-, -N(R^{14c})-C₁₋₃alkyl-, or C₁₋₄alkyl, wherein the C₁₋₃alkyl and C_{1- 4}alkyl are optionally substituted with one, two or three R^{20a};
Ring B is a 3-12 membered nitrogen containing saturated heterocycloalkyl optionally substituted with one or more R¹ and/or one or more R⁴;
Ring C is a 3-12 membered nitrogen containing saturated heterocycloalkyl optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
each L² is -C(O)- or -C(O)N(R^{1a})-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is independently hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b},-(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl₉ -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In embodiments (e.g., a precursor compound of Formula (If) exactly one of V and J is substituted with R¹⁷ or R^{17b.}

In embodiments, R⁷ is a 3-12 membered nitrogen containing heterocycloalkyl or 5 to 12 membered nitrogen containing heteroaryl substituted with one R⁶ directly bonded to a ring N and optionally substituted with one or more R¹ and/or one or more R⁴.

In an aspect is provided a precursor compound of Formula (Ih), or a pharmaceutically acceptable salt or solvate thereof: 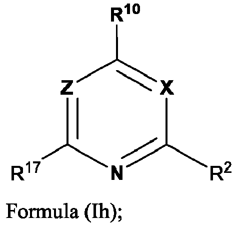 wherein
Z is N and X is C(R³); or Z is C(R⁸) and X is N;
R¹⁰ is -L⁷-R⁷;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 6-12 membered nitrogen containing heterocycloalkyl or 5-12 membered nitrogen containing heteroaryl, wherein the 6-12 membered nitrogen containing heterocycloalkyl or 5-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is independently selected from a -C(O)- and -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₂₋₆alkyl, C₂₋₆alkenyl, C₂₋₆alkynyl, C₃₋₆cycloalkyl, -OR^{12c}, -SR¹², -N(R^{12c})(R^{12c}), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₂₋₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₂₋₆alkyl, C_{2- 6}alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋9heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In an aspect is provided a precursor compound of Formula (Ii), or a pharmaceutically acceptable salt or solvate thereof: 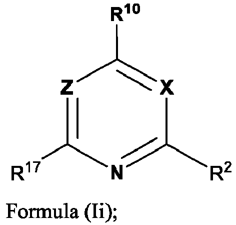 wherein
Z is N and X is C(R³); or Z is C(R⁸) and X is N;
R¹⁰ is -L⁷-R⁷;
L7 is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing spirocyclic heterocycloalkyl substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is independently selected from a -C(O)- and -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O), -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl. -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In an aspect is provided a precursor compound of Formula (Ij), or a pharmaceutically acceptable salt or solvate thereof: 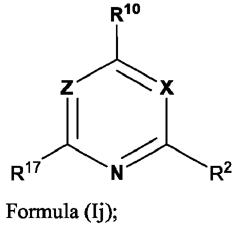 wherein
Z is N and X is C(R³); or Z is C(R⁸) and X is N;
R¹⁰ is -L⁷-R⁷;
L7 is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing fused heterocycloalkyl, fused 7-12 membered nitrogen aryl, or fused 7-12 membered nitrogen heteroaryl substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is independently selected from a -C(O)- and -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, -OR¹², -SR¹², -N(R^{12c})(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), - (C₁-C₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₁₋₆alkyl, C_{2- 6}alkenyl, C₂₋₆alkynyl, and C₃₋₁₀cycloalkyl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), _ N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, \C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In embodiments, R⁷ is a 7-12 membered nitrogen containing fused heterocycloalkyl or fused 7-12 membered nitrogen heteroaryl substituted with one R⁶ directly bonded to a ring N and optionally substituted with one or more R¹ and/or one or more R⁴.

In an aspect is provided a precursor compound of Formula (Ik), or a pharmaceutically acceptable salt or solvate thereof: 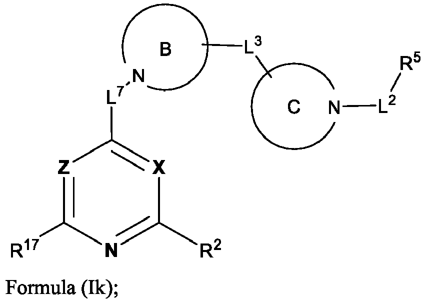 wherein
Z is N and X is C(R³); or Z is C(R⁸) and X is N;
L⁷ is a bond, -C(O)-, -S-, -S(O)₂-, -S(O)-, -N(R^{14c})-C₁₋₃alkyl-, or C₁₋₄alkyl, wherein the C₁₋₃alkyl and C_{1- 4}alkyl are optionally substituted with one, two or three R^{20a};
Ring B is a 4-6 membered nitrogen containing saturated heterocycloalkyl optionally substituted with one or more R¹ and/or one or more R⁴;
Ring C is a 4-6 membered nitrogen containing saturated heterocycloalkyl optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
each L² is independently selected from a -C(O)- and -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; wherein one of R² and R¹⁷ is not hydrogen;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the modified K-Ras G12S protein described herein, the covalently bonded compound comprises a staying group having a formula: 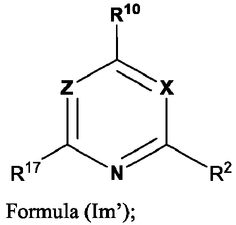 wherein
Z is N and X is C(R³) or Z is C(R⁸) and X is N;
R¹⁰ is -L⁷-R⁷;
L7 is a bond, -O-, -N(R^{14c})-, _C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 3-12 membered nitrogen containing heterocycloalkyl or monocyclic 5-12 membered nitrogen containing heteroaryl, wherein the monocyclic 3-12 membered nitrogen containing heterocycloalkyl or monocyclic 5-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloallcyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and Ci-nheteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20a};
R⁶ -C(O)-; wherein R6 is directly bonded to the K-Ras G12S serine corresponding to position 12 of SEQ ID No: 1;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloallcyl, C₆₋₁₀aryl. -CH₂-C₆₋₁₀aryl, -CH₂-C₁₋₉heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C_{1- 6}alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the modified K-Ras G12S protein described herein, the covalently bonded compound comprises a staying group having a formula: 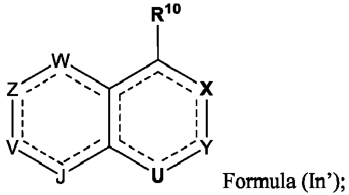 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 3-12 membered nitrogen containing heterocycloalkyl or monocyclic 5-12 membered nitrogen containing heteroaryl, wherein the monocyclic 3-12 membered nitrogen containing heterocycloalkyl or monocyclic 5-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or Ci-nheteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloallcyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20a};
R⁶ -C(O)-; wherein R⁶ is directly bonded to the K-Ras G12S serine corresponding to position 12 of SEQ ID No: 1;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, and R²⁰ⁱ are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C_{1- 6}alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a complex of formula: 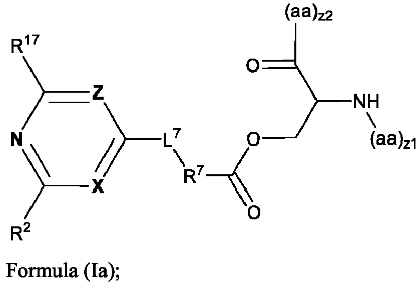 wherein
each aa is independently an amino acid;
zl is an integer equal to at least 1 or (aa)zl is hydrogen;
z2 is an integer equal to at least 1 or (aa)z2 is OH;
Z is N or C(R⁸);
X is C(R³) or N;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 3-12 membered nitrogen containing heterocycloalkyl or monocyclic 5-12 membered nitrogen containing heteroaryl, wherein the monocyclic 3-12 membered nitrogen containing heterocycloalkyl or monocyclic 5-12 membered nitrogen containing heteroaryl are substituted with one R⁶ directly bonded to a ring N and optionally substituted with one or more R¹ and/or one or more R⁴; and further wherein R⁷ is bonded to C(O) in Formula (Ia) through a ring N;;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (Ia),
Z is N and X is C(R³) or Z is C(R⁸) and X is N;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 6-12 membered nitrogen containing heterocycloalkyl or monocyclic 5-12 membered nitrogen containing heteroaryl, wherein the monocyclic 6-12 membered nitrogen containing heterocycloalkyl or monocyclic 5-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁ₒaryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³),-CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl. and C₁₋₉heteroaryl are optionally substituted with one, two, or three R²⁰ⁱ;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₂₋₆alkyl, C₂₋₆alkenyl, C₂₋₆alkynyl, C₃₋₆cycloalkyl, C₆₋₁₀aryl, -OR^{12c}, -SR¹², - N(R^{12c})(R^{12c}), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₂₋₆alkyl)-R^{12b}, -(C₂₋₆alkenyl)-R^{12b}, -(C₂₋₆alkynyl)-R^{12b}, -(C₃₋₁₀cycloalkyl)-R^{12b}, or -(C₆₋₁₀aryl)-R^{12b}, wherein said C₂₋₆alkyl, C₂₋₆alkenyl, C₂₋₆alkynyl, C₃₋₁₀cycloalkyl, and C₆₋₁₀aryl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl,C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)2N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a complex of formula: 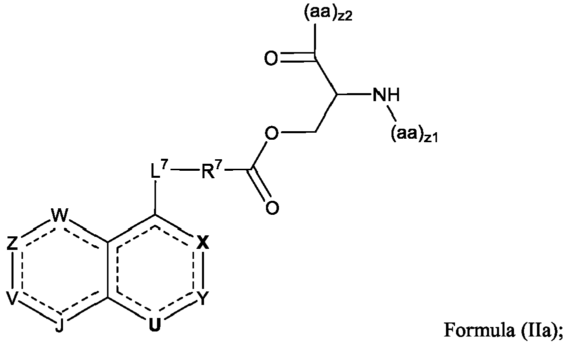 wherein
each aa is independently an amino acid;
zl is an integer equal to at least 1 or (aa)zl is hydrogen;
z2 is an integer equal to at least 1 or (aa)z2 is OH;
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L7 is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 3-12 membered nitrogen containing heterocycloalkyl or monocyclic 5-12 membered nitrogen containing heteroaryl, wherein the monocyclic 3-12 membered nitrogen containing heterocycloalkyl or monocyclic 5-12 membered nitrogen containing heteroaryl are optionally substituted with one or more R¹ and/or one or more R⁴; and further wherein R⁷ is bonded to C(O) in Formula (Ia) through a ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵ , -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl,C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl. -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)2N(R¹²)(R¹³), wherein CI-6alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (IIa),
W is a bond;
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 6-12 membered nitrogen containing heterocycloalkyl or monocyclic 6-12 membered nitrogen containing heteroaryl, wherein the monocyclic 6-12 membered nitrogen containing heterocycloalkyl or monocyclic 6-12 membered nitrogen containing heteroaryl are optionally substituted with one or more R¹ and/or one or more R⁴; and further wherein R⁷ is bonded to C(O) in Formula (Ia) through a ring N;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20b}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), _ N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In some embodiments of the complex of Formula (IIa),
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(0)2-, -S(O)-, -N(R^{14c})-C₁₋₃alkyl-, or Ci-4alkyl, wherein the C_{1- 3}alkyl and C₁₋₄alkyl are optionally substituted with one, two or three R^{20a};
R⁷ is a monocyclic 6-12 membered nitrogen containing heterocycloalkyl or monocyclic 6-12 membered nitrogen containing heteroaryl, wherein the monocyclic 6-12 membered nitrogen containing heterocycloalkyl or monocyclic 6-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ,
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
------ indicates a single or double bond such that all valences are satisfied.

In an aspect is provided a precursor compound of Formula (Ib), or a pharmaceutically acceptable salt or solvate thereof: 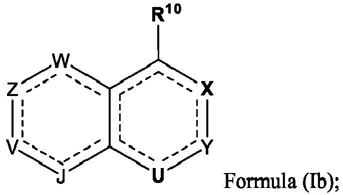 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 6-12 membered nitrogen containing heterocycloalkyl or monocyclic 6-12 membered nitrogen containing heteroaryl, wherein the monocyclic 6-12 membered nitrogen containing heterocycloalkyl or monocyclic 6-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C(O)- or -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -{C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁-₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -{C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a precursor compound of Formula (Ic), or a pharmaceutically acceptable salt or solvate thereof: 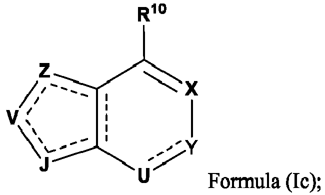 wherein
Z is N, C(R⁸), N(R^{8b}), C(R⁸)(R^{8a}), C(O), S(O), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 6-12 membered nitrogen containing heterocycloalkyl or monocyclic 6-12 membered nitrogen containing heteroaryl, wherein the monocyclic 6-12 membered nitrogen containing heterocycloalkyl or monocyclic 6-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is independently selected from a -C(O)- and -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In embodiments, R⁷ is a 3-12 membered nitrogen containing heterocycloalkyl or 5 to 12 membered nitrogen containing heteroaryl substituted with one R⁶ directly bonded to a ring N and optionally substituted with one or more R¹ and/or one or more R⁴.

In an aspect is provided a precursor compound of Formula (Ih), or a pharmaceutically acceptable salt or solvate thereof: 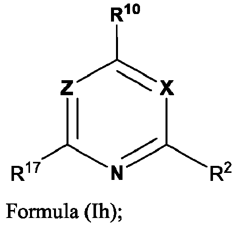 wherein
(i) Z is N and X is C(R³) or Z is C(R⁸) and X is N;

R¹⁰ is -L⁷-R⁷;
L7 is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a monocyclic 6-12 membered nitrogen containing heterocycloalkyl or monocyclic 5-12 membered nitrogen containing heteroaryl, wherein the monocyclic 6-12 membered nitrogen containing heterocycloalkyl or monocyclic 5-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is independently selected from a -C(O)- and -C(O)N(R¹⁴)-;
each R⁵ is selected from:
   (i) a 5-6 membered partially unsaturated heterocycloalkyl comprising one, two, or three ring nitrogen atoms that is optionally substituted with one, two or three R^{20k}, wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-; and
   (ii) a 5-6 membered heteroaryl comprising one, two, or three ring nitrogen atoms that is optionally substituted with one, two or three R^{20k}; wherein R⁵ is (a) bonded through an R⁵ ring nitrogen to L² when L² is -C(O)-, or (b) bonded through an R⁵ ring carbon to the N(R^{4d}) of L² when L² is -C(O)N(R^{4d})-;
R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b};
R² is halogen, -CN, C₂₋₆alkyl, C₂₋₆alkenyl, C₂₋₆alkynyl, C₃₋₆cycloalkyl, C₆₋₁₀aryl, -OR^{12c}, -SR¹², - N(R^{12C})(R^{12C}), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₂₋₆alkyl)-R^{12b}, -(C₂₋₆alkenyl)-R^{12b}, -(C₂₋₆alkynyl)-R^{12b}, -(C₃₋₁₀cycloalkyl)-R^{12b}, or -( C₆₋₁₀aryl)-R^{12b}, wherein said C₂₋₆alkyl, C₂₋₆alkenyl, C₂₋₆alkynyl, C₃₋₁₀cycloalkyl, and C₆₋₁₀aryl, are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl; -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₁-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} are each independently selected from halogen, - CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₁₀aryl, and C₁₋₉heteroaryl; and
- - - - - - indicates a single or double bond such that all valences are satisfied.
   .........................................................................

The modified Ras mutant protein may comprise a covalently bonded compound comprising a staying group having a formula: 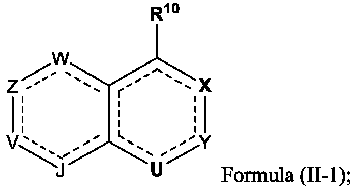 Wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -C₀₋₃alkyl-C(O)-, -C₀₋₃alkyl-N(R¹²)C(O)-, -C₀₋₃alkyl-S(O)-, -C₀₋₃alkyl-N(R¹²)S(O)-, -C₀₋₃alkyl-S(O)₂-, or -C₀₋₃alkyl-N(R¹²)S(O)₂-; wherein R⁶ is directly bonded to the serine residue corresponding to position 12 of SEQ ID No: 1;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R^{17b} is -L^{1b}-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂₋C₁₋₉heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁-₉heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁. ₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, and R²⁰ⁱ is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a complex of formula: 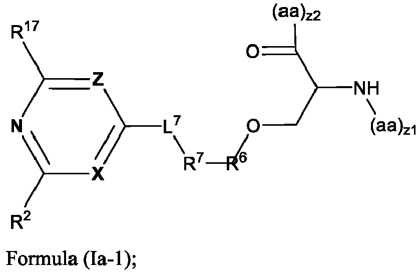 wherein
each aa is independently an amino acid;
zl is an integer equal to at least 1 or (aa)zl is hydrogen;
z2 is an integer equal to at least 1 or (aa)z2 is OH;
Z is N or C(R⁸);
X is C(R³) or N;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -C₀₋₃alkyl-C(O)-, -C₀₋₃alkyl-N(R¹²)C(O)-, -C₀₋₃alkyl-S(O)-, -C₀₋₃alkyl-N(R¹²)S(O)-, -Co-₃alkyl-S(0)₂-, or -C₀₋₃alkyl-N(R¹²)S(O)₂-; wherein R⁶ is directly bonded to the serine corresponding to position 12 of SEQ ID No: 1;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹¹ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NMN(R¹²)(R¹³)₉ -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂₋C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃_ₗₒcycloallcyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, and R²⁰ⁱ is independently selected from halogen, -CN, oxo, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH2-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), _ N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆allcyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), _ N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In one aspect provided is a complex of formula: 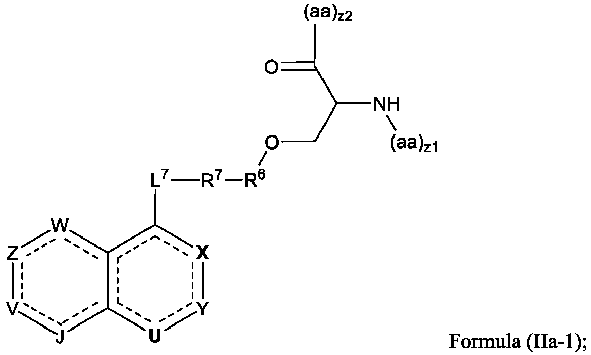 wherein
each aa is independently an amino acid;
zl is an integer equal to at least 1 or (aa)zl is hydrogen;
z2 is an integer equal to at least 1 or (aa)z2 is OH;
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₁₀cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₁₀cycloalkyl)- are optionally substituted with one or more R¹ and/or one or more R⁴;;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -C₀₋₃alkyl-C(O)-, -C₀₋₃alkyl-N(R¹⁴)C(O)-, -C₀₋₃alkyl-S(O)-, -C₀₋₃alkyl-N(R¹⁴)S(O)-, -C₀₋₃alkyl-S(O)₂-, or -C₀₋₃alkyl-N(R¹⁴)S(O)₂-; wherein R⁶ is directly bonded to the serine corresponding to position 12 of SEQ ID No: 1;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R^{17b} is -L^{1b}-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloallcyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20g};
each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁-₉heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloallcyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloallcyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, and R²⁰ⁱ is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ib-1), or a pharmaceutically acceptable salt or solvate thereof: 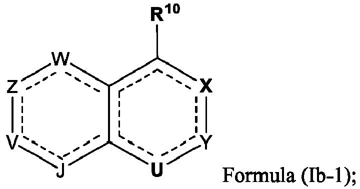 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L7 is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three R^{20a};
R⁷ is a C₃₋₁₂cycloalkyl, 3-12 membered nitrogen containing heterocycloalkyl, C₆₋₁₂aryl, or 5-12 membered nitrogen containing heteroaryl, wherein the a C₃₋₁₂cycloalkyl, 3-12 membered nitrogen containing heterocycloalkyl, C₆₋₁₂aryl, and 5-12 membered nitrogen containing heteroaryl are substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵,-S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloallcyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloallcyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R20h.
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20b}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆allcyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ib-2), or a pharmaceutically acceptable salt or solvate thereof: 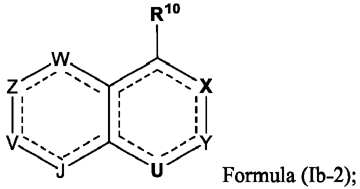 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(W)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L7 is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three R^{20a};
R⁷ is a C₃₋₁₂cycloalkyl, 3-12 membered nitrogen containing heterocycloalkyl, C₆₋₁₂aryl, or 5-12 membered nitrogen containing heteroaryl, wherein the a C₃₋₁₂cycloalkyl, 3-12 membered nitrogen containing heterocycloalkyl, C₆₋₁₂aryl, and 5-12 membered nitrogen containing heteroaryl are substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5-12 membered heteroaryl optionally substituted with one, two, three, four, or five R^{20k}, wherein the 5-12 membered heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloallcyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆allcyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloallcyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ib-1), or a pharmaceutically acceptable salt or solvate thereof: 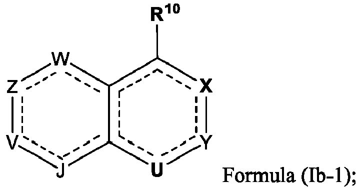 wherein
W is N, C(R¹⁸), N(R^{18b}), C(R¹⁸)(R^{18a}), C(0), S(0), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three R^{20a};
R⁷ is a C₃₋₁₂cycloalkyl, 5-12 membered nitrogen containing heterocycloalkyl, C₆₋₁₂aryl, or 5-12 membered nitrogen containing heteroaryl, wherein the a C₃₋₁₂cycloalkyl, 5-12 membered nitrogen containing heterocycloalkyl, C₆₋₁₂aryl, and 5-12 membered nitrogen containing heteroaryl are substituted with one R⁶, and optionally substituted with one or more R¹ and/or one or more R⁴;
each R¹ is independently selected from C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and Ci-nheteroaryl are optionally substituted with one, two, or three R^{20a};
each R⁴ is independently selected from halogen, oxo, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆allcyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹²)-C₀₋₃alkyl-, -C₀₋₃alkyl-N(R¹²)C(O)-, -C₀₋₃alkyl-S(O), -Co. ₃alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C_{0- 3}alkyl-, or -C₀₋₃alkyl-N(R¹²)S(O)₂-;
each R⁵ is a 5-12 membered heteroaryl optionally substituted with one, two, three, four, or five R^{20k}, wherein the 5-12 membered heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20b}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Id-1), or a pharmaceutically acceptable salt or solvate thereof: 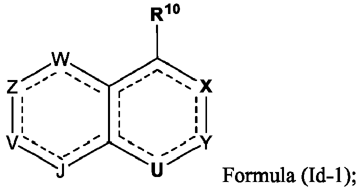 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three- R^{20a};
R⁷ is a 7-12 membered nitrogen containing spirocyclic heterocycloalkyl substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloallcyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloallcyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloallcyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R^{20a}, R^{20b}, R^{20c}, R^{20d}, R^{20e}, R^{20f}, R^{20g}, R^{20b}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloallcyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and C₁₋₉heteroaryl;
each R²³ is independently selected from H and C₁₋₆allcyl;
each R²⁴ is independently selected from H and C₁₋₆allcyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ie-1), or a pharmaceutically acceptable salt or solvate thereof: 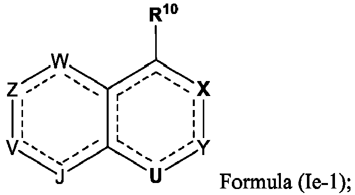 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three- R^{20a};
R⁷ is a 7-12 membered nitrogen containing fused heterocycloalkyl, 7-12 membered nitrogen containing fused aryl, or 7-12 membered nitrogen containing fused heteroaryl substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆allcyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl. and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂₋C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀ cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆allcyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloallcyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆allcyl, C₁₋₆haloallcyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (If-1), or a pharmaceutically acceptable salt or solvate thereof: 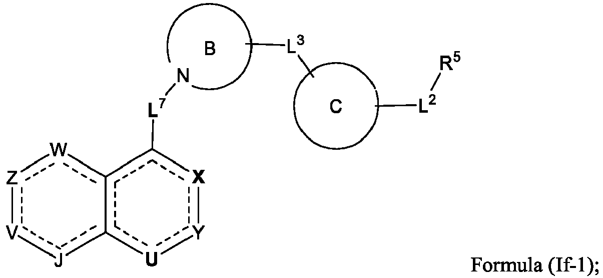 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y and U are independently selected from N, C(R²), C(R²)(R²), N(R^{2b}), S(O), S(O)₂, and C(O);
L⁷ is a bond, -C(O)-, -S-, -S(O)₂-, -S(O)-, -N(R¹⁴)-C₁₋₃alkyl-, or C₁₋₄alkyl, wherein the C₁₋₃alkyl and C_{1- 4}alkyl are optionally substituted with one, two or three- R^{20a};
Ring B is a 3-12 membered nitrogen containing saturated heterocycloalkyl optionally substituted with one or more R¹ and/or one or more R⁴;
Ring C is a 3-12 membered nitrogen containing saturated heterocycloalkyl or C₃₋₁₀cycloalkyl, each optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₁-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁-₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R 20h;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH2S(0)2R15, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R24)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ih-1), or a pharmaceutically acceptable salt or solvate thereof: 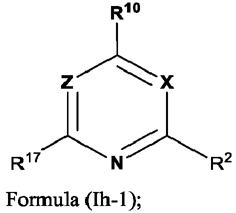 wherein
Z is N and X is C(R³); or Z is C(R⁸) and X is N;
R¹⁰ is -L⁷-R⁷;
L7 is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 5-12 membered nitrogen containing heteroaryl, wherein the 3-12 membered nitrogen containing heterocycloalkyl or 5-12 membered nitrogen containing heteroaryl are substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloallcyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl,-C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl,-C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl,-CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹,-N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆allcyl, C₁₋₆haloalkyl, C₁₋₆alkoxy, C₁₋₆haloalkoxy, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In one aspect provided is a precursor compound of Formula (Ii-1), or a pharmaceutically acceptable salt or solvate thereof: 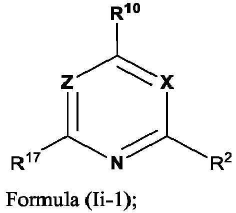 wherein
Z is N and X is C(R³); or Z is C(R⁸) and X is N;
R¹⁰ is -L⁷-R⁷;
L7 is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing spirocyclic heterocycloalkyl substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵,-S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵,-S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵,-CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-,-C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-,-C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-,-OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵,-N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³),-N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³),-CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl,-C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl,-C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl,-CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(OR²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In one aspect provided is a precursor compound of Formula (Ij-1), or a pharmaceutically acceptable salt or solvate thereof: 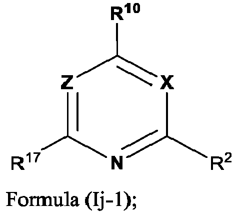 wherein
Z is N and X is C(R³); or Z is C(R⁸) and X is N;
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted with one, two or three R^{20a};
R⁷ is a 7-12 membered nitrogen containing fused heterocycloalkyl, fused 7-12 membered nitrogen containing aryl, or fused 7-12 membered nitrogen containing heteroaryl substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵,-S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵,-S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵,-CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-,-C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloallcyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20h};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-,-C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-,-OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³),-N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³),-CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵,-C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵,-CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R^{12c} is independently selected from C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and - OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
*- - - - - -* indicates a single or double bond such that all valences are satisfied.

In one aspect provided is a precursor compound of Formula (Ik-1), or a pharmaceutically acceptable salt or solvate thereof: 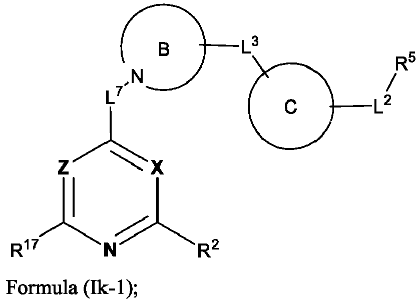 wherein
Z is N and X is C(R³); or Z is C(R⁸) and X is N;
L⁷ is a bond, -C(O)-, -S-, -S(O)₂-, -S(O)-, -N(R¹⁴)-C₁₋₃alkyl-, or C₁₋₄alkyl, wherein the C₁₋₃alkyl and C_{1- 4}alkyl are optionally substituted with one, two or three R^{20a};
Ring B is a 4-6 membered nitrogen containing saturated heterocycloalkyl optionally substituted with one or more R¹ and/or one or more R⁴;
Ring C is a 4-6 membered nitrogen containing saturated heterocycloalkyl or C₃₋₁₀cycloalkyl, each optionally substituted with one or more R¹ and/or one or more R⁴;
L³ is an N-C bond joining Ring B and Ring C;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}.
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -Co_ ₃alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20c};
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵ , -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20g};
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20b};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted with one, two, or three R^{20d};
each R^{12e} is independently selected from hydrogen and R^{20d};
wherein one of R² and R¹⁷ is not hydrogen;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20f};
each R^{20a}, R^{20b}, R^{20c}, R^{20d}, R^{20e}, R^{20f}, R^{20g}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₁-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and-OC(O)R²⁵;;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a precursor compound of Formula (1-2), or a pharmaceutically acceptable salt or solvate thereof: 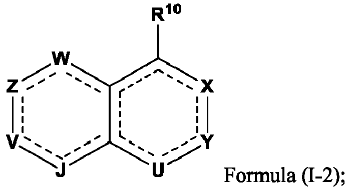 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, C₁₋₄alkyl, or 2-4 membered heteroalkyl linker, wherein the C₁₋₄alkyl and 2-4 membered heteroalkyl linker are each optionally substituted with one, two or three R^{20a};
R⁷ is 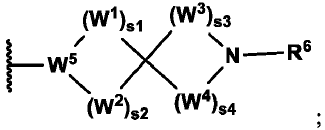
W¹, W², W³, and W⁴ are independently selected from N(R¹), N(R⁴), C(R¹)(R¹), C(R^{l})(R⁴), C(R⁴)(R⁴), C(O), S, O, S(O), and S(O)₂;
W⁵ is selected from N, C(R¹), and C(R⁴);
s1 is an integer from 1 to 6;
s2 is an integer from 0 to 3;
s3 is an integer from 1 to 3;
s4 is an integer from 1 to 3;
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹²)-C₀₋₃alkyl-, -C₀₋₃alkyl-N(R¹²)C(O)-, -C₀₋₃alkyl-S(O), -C_{0- 3}alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C₀₋₃alkyl-, or - C₀₋₃alkyl-N(R¹²)S(O)₂-;
each R⁵ is a 5-12 membered heteroaryl optionally substituted with one, two, three, four, or five R^{20k}, wherein the heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋ioaryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c}.
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R'^{2b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloallcyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloallcyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R'^{2b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R'^{2b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three p20h.
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C_{3- 10}cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl; and
- - - - - - indicates a single or double bond such that all valences are satisfied.

In an aspect is provided a precursor compound of Formula (1-1), or a pharmaceutically acceptable salt or solvate thereof: 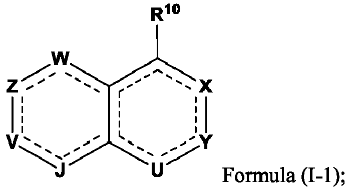 Wherein
wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, C₁₋₄alkyl, or 2-4 membered heteroalkyl linker, wherein the C₁₋₃alkyl, C₁₋₄alkyl, and 2-4 membered heteroalkyl linker are each optionally substituted with one, two or three R^{20a};
R⁷ is 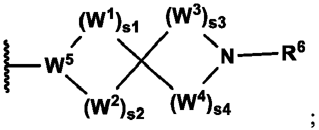
W¹, W², W³, and W⁴ are independently selected from N(R¹), N(R⁴), C(R¹)(R¹), C(R¹)(R⁴), C(R⁴)(R⁴), C(O), S, O, S(O), and S(O)₂;
W⁵ is selected from N, C(R¹), and C(R⁴);
s1 is an integer from 1 to 6;
s2 is an integer from 0 to 3;
s3 is an integer from 1 to 3;
s4 is an integer from 1 to 3;
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
L² is selected from -C(O)- and -C(O)N(R¹²)-;
R⁵ is a 5-6 membered heteroaryl, optionally substituted with one, two, or three R^{20k}, wherein the 5-6 membered heteroaryl comprises one, two, or three ring nitrogen atoms and further wherein when R⁵ is directly bonded to a C(O) of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond;
L^{1b} is selected from a bond;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C₂₋₉heterocycloalkyl, C_{6- 10}aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋iocycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C₂₋₉heterocycloalkyl, C_{6- 10}aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵,-OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and Cl-9heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C_{3- 10}cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, - SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl; and
- - - - - - indicates a single or double bond such that all valences are satisfied.

In an aspect provided is a precursor compound of Formula (Ib-3), or a pharmaceutically acceptable salt or solvate thereof: 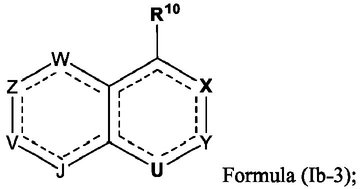 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L7 is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a C₃₋₁₂cycloalkyl, 3-12 membered nitrogen containing heterocycloalkyl, C₆₋₁₂aryl,, or 5-12 membered nitrogen containing heteroaryl, wherein the a C₃₋₁₂cycloalkyl, 3-12 membered nitrogen containing heterocycloalkyl, C₆₋₁₂aryl,, and 5-12 membered nitrogen containing heteroaryl are substituted with one R⁶, and optionally further substituted, wherein two substituents that are bonded to the same or adjacent atoms are optionally joined to form a C₃₋₁₂cycloalkyl, C₁₋₁₁heterocycloalkyl, C₆₋₁₂aryl, or C_{1- 11}heteroaryl, wherein the C₃₋₁₂cycloalkyl, C₁₋₁₁heterocycloalkyl, C₆₋₁₂aryl, or Ci-nheteroaryl are optionally substituted;
R⁶ is -L²-R⁵;
each L² is -C₀₋₃alkyl-C(O)-, -C₀₋₃alkyl-C(O)N(R¹²)-C₀₋₃alkyl-, -C₀₋₃alkyl-N(R¹²)C(O)-, -C₀₋₃alkyl-S(O), -Co. ₃alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C_{0- 3}alkyl-, or -C₀₋₃alkyl-N(R¹²)S(O)₂-;
each R⁵ is an optionally substituted 5-12 membered heteroaryl, wherein the 5-12 membered heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C₃₋iocycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋iocycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

The modified Ras mutant protein may comprise a covalently bonded compound comprising a staying group having a formula: 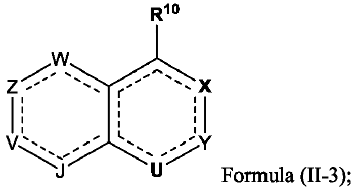 Wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are substituted with one R⁶, and optionally further substituted, wherein two substituents that are bonded to the same or adjacent atoms are optionally joined to form a C_{3- 12}cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or Ci-nheteroaryl are optionally substituted;
R⁶ is -C₀₋₃alkyl-C(O)-, -C₀₋₃alkyl-N(R¹²)C(O)-, -C₀₋₃alkyl-S(O)-, -C₀₋₃alkyl-N(R¹²)S(O)-, -C₀₋₃alkyl-S(O)₂-, or -C₀₋₃alkyl-N(R¹²)S(O)₂-; wherein R⁶ is directly bonded to the serine residue corresponding to position 12 of SEQ ID No: 1;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{17b} is -L^{1b}-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R² is independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, _ CH₂S(O)₂R¹⁵, and -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁-₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a complex of formula: 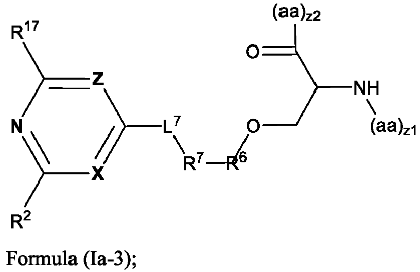 wherein
each aa is independently an amino acid;
zl is an integer equal to at least 1 or (aa)zl is hydrogen;
z2 is an integer equal to at least 1 or (aa)z2 is OH;
Z is N or C(R⁸);
X is C(R³) or N;
L⁷ is a bond, -O-, -N(R^{14c})-, _C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted;
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are optionally further substituted, wherein two substituents that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted;
R⁶ is -C₀₋₃alkyl-C(O)-, -C₀₋₃alkyl-N(R¹²)C(O)-, -C₀₋₃alkyl-S(O)-, -C₀₋₃alkyl-N(R¹²)S(O)-, -C₀₋₃alkyl-S(O)₂-, or -C₀₋₃alkyl-N(R¹²)S(O)₂-; wherein R⁶ is directly bonded to the serine corresponding to position 12 of SEQ ID No: 1;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted;
R¹⁷ is -L¹-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted; R¹⁹ is selected from a C_{3- 12}cycloalkyl, C₂₋₁₁heterocycloalkyl, C₆₋₁₂aryl, and C₂₋₁₂heteroaryl, wherein the C₃₋₁₂cycloalkyl, C_{2- 11}heterocycloalkyl, C₆₋₁₂aryl, and C₂₋₁₂heteroaryl are optionally substituted;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl; and
- - - - - - indicates a single or double bond such that all valences are satisfied.

In one aspect provided is a complex of formula: 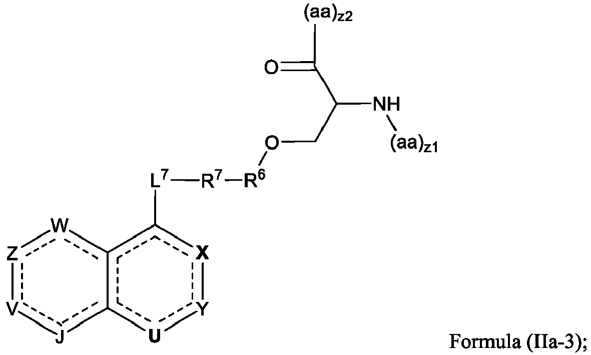 wherein
each aa is independently an amino acid;
zl is an integer equal to at least 1 or (aa)zl is hydrogen;
z2 is an integer equal to at least 1 or (aa)z2 is OH;
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₁₀cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₁₀cycloalkyl)- are optionally further substituted, wherein two substituents that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted;
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and Ci-nheteroaryl are optionally substituted;
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R⁶ is -C₀₋₃alkyl-C(O)-, -C₀₋₃alkyl-N(R¹²)C(O)-, -C₀₋₃alkyl-S(O)-, -C₀₋₃alkyl-N(R¹²)S(O)-, -C₀₋₃alkyl-S(O)₂-, or -C₀₋₃alkyl-N(R¹²)S(O)₂-; wherein R⁶ is directly bonded to the serine corresponding to position 12 of SEQ ID No: 1;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted;
R¹⁷ is -L¹-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted;
R^{17b} is -L^{1b}-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted; R¹⁹ is selected from a C_{3- 12}cycloalkyl, C₂₋₁₁heterocycloalkyl, C₆₋₁₂aryl, and C₂₋₁₂heteroaryl, wherein the C₃₋₁₂cycloalkyl, C_{2- 11}heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, _ CH₂S(O)₂R¹⁵, and -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloallcyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ib-3), or a pharmaceutically acceptable salt or solvate thereof: 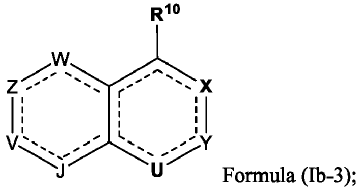 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L7 is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a C₃₋₁₂cycloalkyl, 3-12 membered nitrogen containing heterocycloalkyl, C₆₋₁₂aryl, or 5-12 membered nitrogen containing heteroaryl, wherein the a C₃₋₁₂cycloalkyl, 3-12 membered nitrogen containing heterocycloalkyl, C₆₋₁₂aryl,, and 5-12 membered nitrogen containing heteroaryl are substituted with one R⁶, and optionally further substituted, wherein two substituents that are bonded to the same or adjacent atoms are optionally joined to form a C₃₋₁₂cycloalkyl, C₁₋₁₁heterocycloalkyl, C₆₋₁₂aryl, or C_{1- 11}heteroaryl, wherein the C₃₋₁₂cycloalkyl, C₁₋₁₁heterocycloalkyl, C₆₋₁₂aryl, or Ci-nheteroaryl are optionally substituted;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted;
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and Ci-nheteroaryl are optionally substituted;
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ib-4), or a pharmaceutically acceptable salt or solvate thereof: 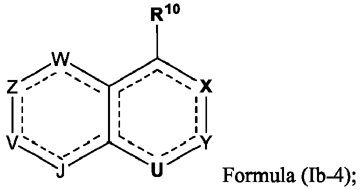 wherein
W is N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L7 is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a C₃₋₁₂cycloalkyl, 3-12 membered nitrogen containing heterocycloalkyl, C₆₋₁₂aryl,, or 5-12 membered nitrogen containing heteroaryl, wherein the a C₃₋₁₂cycloalkyl, 3-12 membered nitrogen containing heterocycloalkyl, C₆₋₁₂aryl,, and 5-12 membered nitrogen containing heteroaryl are substituted with one R⁶, and optionally further substituted, wherein two substituents that are bonded to the same or adjacent atoms are optionally joined to form a C₃₋₁₂cycloalkyl, C₁₋₁₁heterocycloalkyl, C₆₋₁₂aryl, or C_{1- 11}heteroaryl, wherein the C₃₋₁₂cycloalkyl, C₁₋₁₁heterocycloalkyl, C₆₋₁₂aryl, or Ci-nheteroaryl are optionally substituted;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted;
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and Ci-nheteroaryl are optionally substituted;
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5-12 membered heteroaryl optionally substituted, wherein the 5-12 membered heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloallcyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH_{Z}-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
------ indicates a single or double bond such that all valences are satisfied.

In an aspect provided is a precursor compound of Formula (Id-3), or a pharmaceutically acceptable salt or solvate thereof: 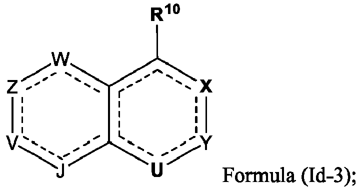 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a 7-12 membered nitrogen containing spirocyclic heterocycloalkyl substituted with one R⁶, and optionally further substituted, wherein two substituents that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted;;
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloallcyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloallcyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³),-OCH₂C(O)OR²², and -OC(O)R²⁵; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, and Cl-9heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ie-3), or a pharmaceutically acceptable salt or solvate thereof: 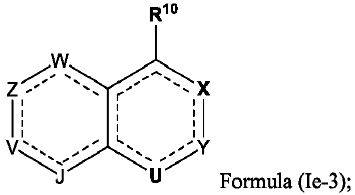 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a 7-12 membered nitrogen containing fused heterocycloalkyl, 7-12 membered nitrogen containing fused aryl, or 7-12 membered nitrogen containing fused heteroaryl substituted with one R⁶, and optionally further substituted, wherein two substituents that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloallcyl, C₆₋₁₂aryl, or Ci-nheteroaryl are optionally substituted;;
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₂₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloallcyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C2-6alkenyl, C2-6alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; wherein C₁₋₆alkyl, C₂₋₆alkenyl, C₂₋₆alkynyl, C₃₋₁₀cycloalkyl, -CH₂-C_{3- 10}cycloallcyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋iocycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (If-3), or a pharmaceutically acceptable salt or solvate thereof: 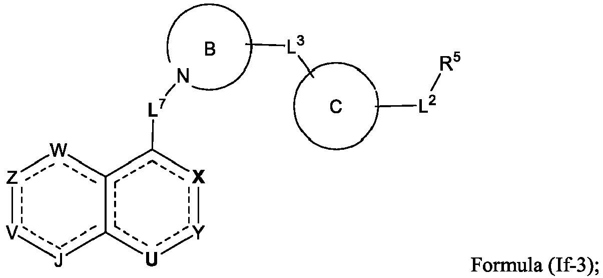 wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), O, C(O), S(O), or S(O)₂;
V and J are independently selected from C(R¹⁷), C(R¹⁷)(R^{16a}), C(R¹⁶), C(R¹⁶)(R^{16a}), N, N(R^{17b}), and N(R^{16b});
Y and U are independently selected from N, C(R²), C(R²)(R²), N(R^{2b}), S(O), S(O)₂, and C(O);
L⁷ is a bond, -C(O)-, -S-, -S(O)₂-, -S(O)-, -N(R¹⁴)-C₁₋₃alkyl-, or C₁₋₄alkyl, wherein the C₁₋₃alkyl and C_{1- 4}alkyl are optionally substituted;
Ring B is an optionally substituted 3-12 membered nitrogen containing saturated heterocycloalkyl, fuwherein two substituents that are bonded to the same or adjacent atoms are optionally joined to form a C₃₋₁₂cycloalkyl, C₁₋₁₁heterocycloallcyl, C₆₋₁₂aryl, or Ci-nheteroaryl, wherein the C₃₋₁₂cycloalkyl, C_{1- 11}heterocycloalkyl, C₆₋₁₂aryl, or Ci-nheteroaryl are optionally substituted;
Ring C is a 3-12 membered nitrogen containing saturated heterocycloalkyl or C₃₋₁₀cycloalkyl, each optionally substituted, wherein two substituents that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted;
L³ is an N-C bond joining Ring B and Ring C;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂₋C₆₋₁₀aryl, -C(R^{12e})₂₋C₁₋₉heteroaryl, and C₁₋₉heteroaryl are optionally substituted;
each R^{12e} is independently selected from hydrogen and R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²²,-C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³),-OCH₂C(O)OR²², and -OC(O)R²⁵; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ih-3), or a pharmaceutically acceptable salt or solvate thereof: 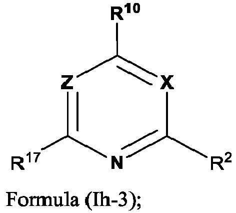 wherein
Z is N and X is C(R³); or Z is C(R⁸) and X is N;
R¹⁰ is -L⁷-R⁷;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted;
R⁷ is a 3-12 membered nitrogen containing heterocycloalkyl or 5-12 membered nitrogen containing heteroaryl, wherein the 3-12 membered nitrogen containing heterocycloalkyl or 5-12 membered nitrogen containing heteroaryl are substituted with one R⁶, and optionally further substituted, wherein two substituents that are bonded to the same or adjacent atoms are optionally joined to form a C_{3- 12}cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or Ci-nheteroaryl are optionally substituted;
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-,-C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(0), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R^{12c} is independently selected from C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³),-OCH₂C(O)OR²², and -OC(O)R²⁵; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉ heteroaryl, and C₁₋₉heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In one aspect provided is a precursor compound of Formula (Ii-3), or a pharmaceutically acceptable salt or solvate thereof: 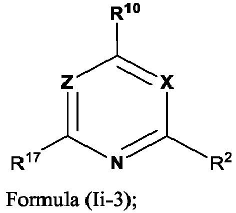 wherein
Z is N and X is C(R³); or Z is C(R⁸) and X is N;
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a 7-12 membered nitrogen containing spirocyclic heterocycloalkyl substituted with one R⁶, and optionally further substituted; wherein two substituents that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or Ci-nheteroaryl are optionally substituted;
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - indicates a single or double bond such that all valences are satisfied.

In one aspect provided is a precursor compound of Formula (Ij-3), or a pharmaceutically acceptable salt or solvate thereof: 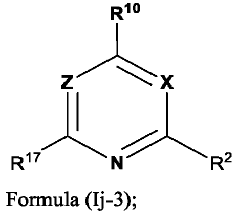 wherein
Z is N and X is C(R³); or Z is C(R⁸) and X is N;
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a 7-12 membered nitrogen containing fused heterocycloalkyl, fused 7-12 membered nitrogen containing aryl, or fused 7-12 membered nitrogen containing heteroaryl substituted with one R⁶, and optionally further substituted, wherein two substituents that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloallcyl, C₆₋₁₂aryl, or Ci-nheteroaryl are optionally substituted;
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R^{12c} is independently selected from C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³),-OCH₂C(O)OR²², and -OC(O)R²⁵; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - indicates a single or double bond such that all valences are satisfied.

In one aspect provided is a precursor compound of Formula (Ik-3), or a pharmaceutically acceptable salt or solvate thereof: 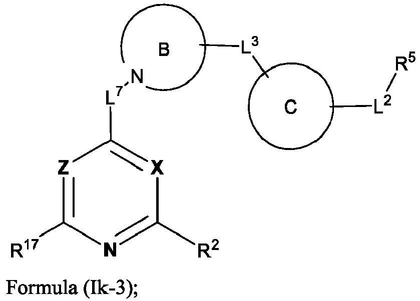 wherein
Z is N and X is C(R³); or Z is C(R⁸) and X is N;
L⁷ is a bond, -C(O)-, -S-, -S(O)₂-, -S(O)-, -N(R¹⁴)-C₁₋₃alkyl-, or C₁₋₄alkyl, wherein the C₁₋₃alkyl and C_{1- 4}alkyl are optionally substituted;
Ring B is an optionally substituted 4-6 membered nitrogen containing saturated heterocycloalkyl; wherein two substituents that are bonded to the same or adjacent atoms are optionally joined to form a C_{3- 12}cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or Ci-nheteroaryl are optionally substituted;
Ring C is a 4-6 membered nitrogen containing saturated heterocycloalkyl or C₃₋₁₀cycloalkyl, each optionally substituted; wherein two substituents that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted;
L³ is an N-C bond joining Ring B and Ring C;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is selected from -L¹-R¹⁹, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R³ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
wherein one of R² and R¹⁷ is not hydrogen;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³),-OCH₂C(O)OR²², and -OC(O)R²⁵; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect provided is a precursor compound of Formula (Ic), or a pharmaceutically acceptable salt or solvate thereof: 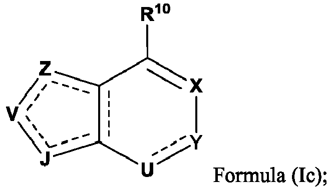 wherein
Z is N, C(R⁸), N(R^{8b}), C(R⁸)(R^{8a}), C(0), S(0), or S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C_{1- 3}alkyl and C₁₋₄alkyl are optionally substituted;
R⁷ is a 3-12 membered nitrogen containing heterocycloalkyl or 5-12 membered nitrogen containing heteroaryl, wherein the 3-12 membered nitrogen containing heterocycloalkyl or 5-12 membered nitrogen containing heteroaryl are substituted with one R⁶, and optionally further substituted;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted;
each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and Ci-nheteroaryl are optionally substituted;
each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R⁶ is -L²-R⁵;
each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, - C₀₋₃alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C_{0- 3}alkyl-S(O)₂N(R¹²)-C₀₋₃alkyl-, or -C₀₋₃alkyl-N(R¹²)S(O)₂-;
each R⁵ is a 5 to 12 membered partially unsaturated heterocycloalkyl or a 5 to 12 membered heteroaryl, each optionally substituted, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(H)(R¹²), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R^{8b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{16b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋ioaryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉ heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
X is C(R³), C(R³)(R³), N(R³), or N;
each R³ is independently selected from hydrogen, halogen, -CN, C₁₋₆alkyl, C₂₋₆alkenyl, C₂₋₆alkynyl, C₃₋₁₀ cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R¹² is independently selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted;
R^{18b} is selected from hydrogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In one aspect, provided is a modified Ras mutant protein comprising a compound covalently bonded to its serine residue, wherein the covalently bonded compound comprises a staying group having a formula: 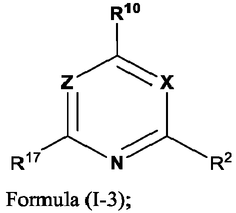 wherein
Z is N and X is C(R³); or Z is C(R⁸) and X is N;
R¹⁰ is -L⁷-R⁷;
L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C_{1- 3}alkyl and C₁₋₄alkyl are optionally substituted with one, two or three R^{20a};
R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are substituted with one R⁶, and optionally further substituted, wherein two substituents that are bonded to the same or adjacent atoms are optionally joined to form a C_{3- 12}cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or Ci-nheteroaryl are optionally substituted;
R⁶ is -C₀₋₃alkyl-C(O)-, -C₀₋₃alkyl-N(R¹²)C(O)-, -C₀₋₃alkyl-S(O)-, -C₀₋₃alkyl-N(R¹²)S(O)-, -C₀₋₃alkyl-S(O)₂-, or -C₀₋₃alkyl-N(R¹²)S(O)₂-; wherein R⁶ is directly bonded to the serine residue corresponding to position 12 of SEQ ID No: 1;
R⁸ is selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁷ is -L¹-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloallcyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted;
R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted;
R³ is selected from hydrogen, halogen, -CN, C₁₋₆alkyl, C₂₋₆alkenyl, C₂₋₆alkynyl, C₃₋₁₀cycloallcyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R² is independently selected from halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted;
each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C_{6- 10}aryl, -C(R^{12e})₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -C(R^{12e})₂-C₃₋₁₀cycloalkyl, C₂₋₉heterocycloalkyl, -C(R^{12e})₂-C₂₋₉heterocycloalkyl, C₆₋₁₀aryl, - C(R^{12e})₂-C₆₋₁₀aryl, -C(R^{12e})₂-C₁₋₉heteroaryl, and C₁₋₉heteroaryl are optionally substituted;
each R^{12e} is independently selected from hydrogen and R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R¹² is independently selected from hydrogen, C₁₋₆alkyl, C₂₋₆alkenyl, C₂₋₆alkynyl, C₃₋₁₀cycloallcyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted;
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted;
each R¹⁴ is independently selected from hydrogen, C₁₋₆alkyl, and C₁₋₆haloalkyl;
each R^{14c} is independently selected from C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted;
each R^{20d} is independently selected from halogen, -CN, oxo, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), - OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
------ indicates a single or double bond such that all valences are satisfied.

In an aspect is provided a compound of the formula (X) 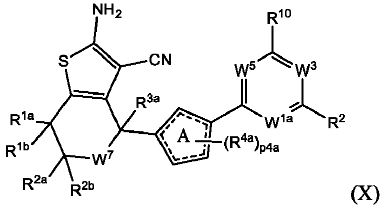 wherein
R^{1a} and R^{1b} are both independently selected from the group consisting of hydrogen, C₁₋₄alkyl, C₁₋₄haloalkyl, C_{1- 4}alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocycloalkyl;
R^{2a} and R^{2b} are both independently selected from the group consisting of hydrogen, C₁₋₄alkyl, C₁₋₄haloalkyl, C_{1- 4}alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocycloalkyl; and/or,
optionally, one of R^{1a} or R^{1b} and one of R^{2a} or R^{2b} together with the carbon atoms they are attached form a cyclopropane ring;
W⁷ is -(CR^{w7a}R^{w7b})_{w7z}-;
each R^{w7a} and R^{w7b} is independently selected from the group consisting of hydrogen, C₁₋₄alkyl, C₁₋₄haloalkyl, C_{1- 4}alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocycloalkyl;
w7z is selected from the group consisting 0, 1 and 2;
R^{3a} is selected from the group consisting of hydrogen, C₁₋₆alkyl, C₁₋₆haloalkyl, C₁₋₆alkoxy, C₁₋₆haloalkoxy, cyano-C_{1- 6}alkyl, halogen, -OH, -NH₂, -NH(C₁₋₄alkyl), -N(C₁₋₄alkyl)₂, -CN, C₃₋₅cycloalkyl and 3-5 membered heterocycloalkyl;
ring A is a ring selected from the group consisting of oxadiazole, thiadiazole, pyrrole, furan, thiophene, imidazole, pyrazole, oxazole, isoxazole, thiazole, isothiazole and triazole;
each R^{4a}, if present, is independently selected from the group consisting of C₁₋₆alkyl, C₁₋₆haloalkyl, C₁₋₆alkoxy, C_{1- 6}haloalkoxy, cyano-C₁₋₆alkyl, halogen, -OH, -NH₂, -NH(C₁₋₄alkyl), -N(C₁₋₄alkyl)₂, -CN, C₃₋₅cycloalkyl and 3-5 membered heterocycloalkyl; p4a is selected from the group consisting 0, 1, 2 and 3;
W³ is selected from the group consisting of nitrogen (=N-) and carbon substituted with R^{A} (=C(R³)-);
W⁵ is selected from the group consisting of nitrogen (=N-) and carbon substituted with R^{B} (=C(R⁵)-);
W^{1a} is selected from the group consisting of nitrogen (=N-) and carbon substituted with R^{c} (=C(R^{1a})-);
R^{1a}, R³ and R⁵ is each independently selected from the group consisting of hydrogen, C₁₋₆haloalkyl, C₂₋₆alkynyl optionally substituted with C₃₋₅cycloalkyl, C₁₋₆alkoxy, C₁₋₆haloalkoxy, halogen, -CN, -OH, -NH₂, -NH(C_{1- 4}alkyl), -N(C₁₋₄alkyl)₂, -C(=O)NH₂, -C(=O)NH(C₁₋₄alkyl), -C(=O)N(C₁₋₄alkyl)₂, -S-S₁₋₆alkyl, -S(=O)₂-C₁₋₆alkyl, C₃₋₅cycloalkyl, 3-5 membered heterocycloalkyl and C₁₋₆alkyl optionally substituted with a substituent selected from the group consisting of C₁₋₆alkoxy, -CN, -OH, -NH₂, -NH(C₁₋₄alkyl), -N(C₁₋₄alkyl)₂, -C(=O)NH₂, - C(=O)NH(C₁₋₄alkyl) and -C(=O)N(C₁₋₄alkyl)₂;
R² is selected from the group consisting of R^{a1} and R^{b1};
R^{a1} is selected from the group consisting of C₁₋₆alkyl, C₁₋₆haloalkyl, C₂₋₆alkenyl, C₂₋₆alkynyl, C₃₋₁₀cycloalkyl, C_{4- 10}cycloalkenyl, 3-11 membered heterocycloalkyl, C₆₋₁₀aryl and 5-10 membered heteroaryl, wherein the C_{1- 6}alkyl, C₁₋₆haloalkyl, C₂₋₆alkenyl, C₂₋₆alkynyl, C₃₋₁₀cycloalkyl, C₄₋₁₀cycloalkenyl, 3-11 membered heterocycloalkyl, C₆₋₁₀aryl and 5-10 membered heteroaryl are all optionally substituted with one or more, identical or different R^{b1} and/or R^{c1};
each R^{b1} is independently selected from the group consisting of -OR^{c1}, -NR^{c1}R^{c1}, halogen, -CN, -C(=O)R^{c1}, - C(=O)OR^{c1}, -C(=O)NR^{c1}R^{c1}, -S(=O)2R^{c1}, -S(=O)2NR^{c1}R^{c1}, -NHC(=O)R^{c1}, -N(C₁₋₄alkyl)C(=O)R^{c1}, - NHS(=O)₂R^{c1}, -N(C₁₋₄alkyl)S(=O)₂R^{c1}, -NHC(=O)OR^{c1}, -N(C₁₋₄alkyl)C(=O)OR^{c1} and the bivalent substituent =0;
each R^{c1} is independently selected from the group consisting of hydrogen, C₁₋₆alkyl, C₁₋₆haloalkyl, C₂₋₆alkenyl, C_{2- 6}alkynyl, C₃₋₁₀cycloalkyl, C₄₋₁₀cycloalkenyl, 3-11 membered heterocycloalkyl, C₆₋₁₀aryl and 5-10 membered heteroaryl, wherein the C₁₋₆alkyl, C₁₋₆haloalkyl, C₂₋₆alkenyl, C₂₋₆alkynyl, C₃₋₁₀cycloalkyl, C₄₋₁₀cycloalkenyl, 3-11 membered heterocycloalkyl, C₆₋₁₀aryl and 5-10 membered heteroaryl are all optionally substituted with one or more, identical or different R^{d1} and/or R^{e1},
each R^{d1} is independently selected from the group consisting of -OR^{e1}, -NR^{e1}R^{e1}, halogen, -CN, -C(=O)R^{e1}, - C(=O)OR^{e1}, -C(=O)NR^{e1}R^{e1}, -S(=O)₂R^{e1}, -S(=O)₂NR^{e1}R^{e1}, -NHC(=O)R^{e1}, -N(C₁₋₄alkyl)C(=O)R^{e1}, - NHS(=O)₂R^{c1}, -N(C₁₋₄alkyl)S(=O)₂R^{c1}, -NHC(=O)OR^{e1}, -N(C₁₋₄alkyl)C(=O)OR^{e1} and the bivalent substituent =0;
each R^{e1} is independently selected from the group consisting of hydrogen, C₁₋₆alkyl, C₁₋₆haloalkyl, C₂₋₆alkenyl, C_{2- 6}alkynyl, C₃₋₁₀cycloalkyl, C₄₋₁₀cycloalkenyl, 3-11 membered heterocycloalkyl, C₆₋₁₀aryl and 5-10 membered heteroaryl,
wherein the C₁₋₆alkyl, C₁₋₆haloalkyl, C₂₋₆alkenyl, C₂₋₆alkynyl, C₃₋₁₀cycloalkyl, C₄₋₁₀cycloalkenyl, 3-11 membered heterocycloalkyl, C₆₋₁₀aryl and 5-10 membered heteroaryl are all optionally substituted with one or more, identical or different substituent(s) selected from the group consisting of C₁₋₆alkyl, C₁₋₆haloalkyl, C_{3- 10}cycloalkyl, 3-11 membered heterocycloalkyl optionally substituted with one or more, identical or different C_{1- 4}alkyl, C₆₋₁₀aryl, 5-10 membered heteroaryl, -OH, C₁₋₆alkoxy, C₁₋₄alkoxy-C₁₋₄alkyl, hydroxy-C₁₋₄alkyl, halogen, -CN, -NH₂, -C(=O)C₁₋₄alkyl, -NH(C₁₋₄alkyl), -N(C₁₋₄alkyl)₂ and the bivalent substituent =0;
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C₁₋₃alkyl and C₁₋₄alkyl are optionally substituted with one, two or three R^{20a};
R⁷ is a 5-12 membered nitrogen containing heterocycloalkyl or 5-12 membered nitrogen containing heteroaryl, wherein the 5-12 membered nitrogen containing heterocycloalkyl or 5-12 membered nitrogen containing heteroaryl are substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹²)-C₀₋₃alkyl-, -C₀₋₃alkyl-N(R¹²)C(O)-, -C₀₋₃alkyl-S(O), -C_{0- 3}alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C₀₋₃alkyl-, or - C₀₋₃alkyl-N(R¹²)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloallcyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f};
each R^{20a}, R^{20d}, R^{20e}, R^{20f}, and R^{20k} are each independently selected from halogen, -CN, oxo, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloallcyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R2³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl; and
------ indicates a single or double bond such that all valences are satisfied.

In an aspect is provided a compound of the formula (XI) 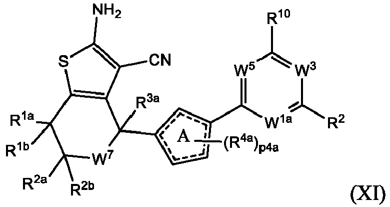 wherein
R^{1a} and R^{1b} are both independently selected from the group consisting of hydrogen, C₁₋₄alkyl, C₁₋₄haloalkyl, C_{1- 4}alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocycloalkyl;
R^{2a} and R^{2b} are both independently selected from the group consisting of hydrogen, C₁₋₄alkyl, C₁₋₄haloalkyl, C_{1- 4}alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocycloalkyl; and/or,
optionally, one of R^{1a} or R^{1b} and one of R^{2a} or R^{2b} together with the carbon atoms they are attached form a cyclopropane ring;
W⁷ is -(CR^{w7a}R^{w7b})_{w7z}-;
each R^{w7a} and R^{w7b} is independently selected from the group consisting of hydrogen, C₁₋₄alkyl, C₁₋₄haloalkyl, C_{1- 4}alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocycloalkyl;
w7z is selected from the group consisting 0, 1 and 2;
R^{3a} is selected from the group consisting of hydrogen, C₁₋₆alkyl, C₁₋₆haloalkyl, C₁₋₆alkoxy, C₁₋₆haloalkoxy, cyano-C_{1- 6}alkyl, halogen, -OH, -NH₂, -NH(C₁₋₄alkyl), -N(C₁₋₄alkyl)₂, -CN, C₃₋₅cycloalkyl and 3-5 membered heterocycloalkyl;
ring A is a ring selected from the group consisting of oxadiazole, thiadiazole, pyrrole, furan, thiophene, imidazole, pyrazole, oxazole, isoxazole, thiazole, isothiazole and triazole;
each R^{4a}, if present, is independently selected from the group consisting of C₁₋₆alkyl, C₁₋₆haloalkyl, C₁₋₆alkoxy, C_{1- 6}haloalkoxy, cyano-C₁₋₆allcyl, halogen, -OH, -NH₂, -NH(C₁₋₄alkyl), -N(C₁₋₄alkyl)₂, -CN, C₃₋₅cycloalkyl and 3-5 membered heterocycloalkyl; p4a is selected from the group consisting 0, 1, 2 and 3;
W³ is selected from the group consisting of nitrogen (=N-) and carbon substituted with R^{A} (=C(R³)-);
W⁵ is selected from the group consisting of nitrogen (=N-) and carbon substituted with R^{B} (=C(R⁵)-);
W^{1a} is selected from the group consisting of nitrogen (=N-) and carbon substituted with R^{c} (=C(R^{1a})-);
R^{1a}, R³ and R⁵ is each independently selected from the group consisting of hydrogen, C₁₋₆haloalkyl, C₂₋₆alkynyl optionally substituted with C₃₋₅cycloalkyl, C₁₋₆alkoxy, C₁₋₆haloalkoxy, halogen, -CN, -OH, -NH₂, -NH(C_{1- 4}alkyl), -N(C₁₋₄alkyl)₂, -C(=O)NH₂, -C(=O)NH(C₁₋₄alkyl), -C(=O)N(C₁₋₄alkyl)₂, -S-C₁₋₆allcyl, -S(=O)₂-C₁₋₆alkyl, C₃₋₅cycloalkyl, 3-5 membered heterocycloalkyl and C₁₋₆alkyl optionally substituted with a substituent selected from the group consisting of C₁₋₆alkoxy, -CN, -OH, -NH₂, -NH(C₁₋₄alkyl), -N(C₁₋₄alkyl)₂, -C(=O)NH₂, - C(=O)NH(C₁₋₄alkyl) and -C(=O)N(C₁₋₄alkyl)₂;
wherein the C₁₋₆alkyl, C₁₋₆haloalkyl, C₂₋₆alkenyl, C₂₋₆alkynyl, C₃₋₁₀cycloalkyl, C₄₋₁₀cycloalkenyl, 3-11 membered heterocycloalkyl, C₆₋₁₀aryl and 5-10 membered heteroaryl are all optionally substituted with one or more, identical or different substituent(s) selected from the group consisting of C₁₋₆alkyl, C₁₋₆haloalkyl, C_{3- 10}cycloalkyl, 3-11 membered heterocycloalkyl optionally substituted with one or more, identical or different C_{1- 4}alkyl, C₆₋₁₀aryl, 5-10 membered heteroaryl, -OH, C₁₋₆alkoxy, C₁₋₄alkoxy-C₁₋₄alkyl, hydroxy-C₁₋₄alkyl, halogen, -CN, -NH₂, -C(=O)C₁₋₄alkyl, -NH(C₁₋₄alkyl), -N(C₁₋₄alkyl)₂ and the bivalent substituent =0;
R¹⁰ is -L⁷-R⁷;
L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C₁₋₃alkyl and C₁₋₄alkyl are optionally substituted with one, two or three R^{20a};
R⁷ is a 5-12 membered nitrogen containing heterocycloalkyl or 5-12 membered nitrogen containing heteroaryl, wherein the 5-12 membered nitrogen containing heterocycloalkyl or 5-12 membered nitrogen containing heteroaryl are substituted with one R⁶, and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloallcyl, C₆₋₁₂aryl, or Ci-nheteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20a};
R⁶ is -L²-R⁵;
each L² is -C₀₋₃alkyl-C(O)-, -C₀₋₃alkyl-C(O)N(R¹²)-C₀₋₃alkyl-, -C₀₋₃alkyl-N(R¹²)C(O)-, -C₀₋₃alkyl-S(O), -C_{0- 3}alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C₀₋₃alkyl-, or - C₀₋₃alkyl-N(R¹²)S(O)₂-;
each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
each R² is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³),-S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C_{2- 9}heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C_{2- 6}alkynyl, C₃₋₁₀cycloalkyl, C₂₋₉heterocycloalkyl, C₆₋₁₀aryl, and C₁₋₉heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
each R^{20a}, R^{20b}, R^{20d}, R^{20e}, R^{20f}, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, - N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋ioaryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆allcyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²¹, -N(R²⁴)S(O)₂R²⁵, -C(O)R²¹, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloallcyl, C₂₋₉heterocycloalkyl, C_{6- 10}aryl, and C₁₋₉heteroaryl; and
- - - - - - indicates a single or double bond such that all valences are satisfied.

In an aspect is provided a precursor compound of Formula (I-la), or a pharmaceutically acceptable salt or solvate thereof: 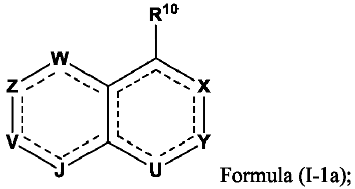 Wherein
W is a C(R¹⁸) or C(O); Z is N, C(R⁸), or N(R^{8b}); V is C(R¹⁷); J is C(R¹⁶); Y is C(R²); U is N; X is N;
R¹⁰ is -L⁷-R⁷;
L⁷ is a bond;
R⁷ is 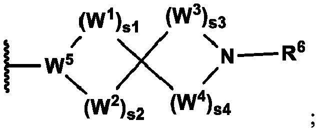
W¹, W², W³, and W⁴ are independently selected from C(R¹)(R¹), C(R¹)(R⁴), C(R⁴)(R⁴), and O;
W⁵ is selected from N and CH;
si is an integer from 1 to 3; s2 is an integer from 1 to 3; s3 is an integer from 1 to 3; s4 is an integer from 1 to 3; each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH2-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
L² is selected from -C(O)-;
R⁵ is a 5-6 membered heteroaryl, optionally substituted with one, two, or three R^{20k}, wherein the 5-6 membered heteroaryl comprises one, two, or three ring nitrogen atoms and further wherein when R⁵ is directly bonded to a C(O) of L², L² is directly bonded to an N atom of R⁵;
R⁸ is independently selected from hydrogen, halogen, C₁₋₆alkyl, C₂₋₆alkenyl, C₂₋₆alkynyl, C₃₋₁₀cycloalkyl, C_{2- 9}heterocycloalkyl, -OR¹², -SR¹², and -N(R¹²)(R¹³), wherein C₁₋₆alkyl, C₂₋₆alkenyl, C₂₋₆alkynyl, C₃₋₁₀cycloallcyl, and C₂₋₉heterocycloalkyl are optionally substituted with one, two, or three R^{20c};
R^{8b} is selected from hydrogen, C₁₋₆alkyl, C₂₋₆alkenyl, C₂₋₆alkynyl, C₃₋₁₀cycloalkyl, and C₂₋₉heterocycloalkyl, wherein C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20c};
R¹⁷ is -L¹-R¹⁹;
L¹ is selected from a bond;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ;
R¹⁶ is independently selected from hydrogen, halogen, C₁₋₆allcyl, C₂₋₆alkenyl, C₂₋₆alkynyl, C₃₋₁₀cycloalkyl, C_{2- 9}heterocycloalkyl, -OR¹², -SR¹², and -N(R¹²)(R¹³), wherein C₁₋₆allcyl, C₂₋₆alkenyl, C₂₋₆alkynyl, C₃₋₁₀cycloallcyl, and C₂₋₉heterocycloalkyl are optionally substituted with one, two, or three R^{20g};
R² is C₁₋₆alkyl, C₂₋₆alkenyl, C₂₋₆alkynyl, C₃₋₁₀cycloalkyl, C₂₋₉heterocycloalkyl, -OR^{12d}, -SR^{12d}, or -N(R^{12d})(R¹³), wherein said C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20d};
each R^{12d} is independently selected from C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R¹²e)₂-C_{1- 9}heteroaryl, and C₁₋₉heteroaryl are optionally substituted with one, two, or three R^{20d};
each R^{12e} is independently selected from hydrogen and R^{20d};
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
R¹⁸ is independently selected from hydrogen, halogen, C₁₋₆allcyl, C₂₋₆alkenyl, C₂₋₆alkynyl, C₃₋₁₀cycloallcyl, and C_{2- 9}heterocycloalkyl, wherein C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C_{3- 10}cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloallcyl, C₂₋₉heterocycloalkyl, C_{6- 10}aryl, and C₁₋₉heteroaryl; and
- - - - - - indicates a single or double bond such that all valences are satisfied.

In an aspect is provided a precursor compound of Formula (I-lb), or a pharmaceutically acceptable salt or solvate thereof: 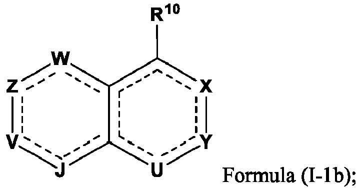 Wherein
W is a CH; Z is N or C(R⁸); V is C(R¹⁷); J is C(R¹⁶); Y is C(R²); U is N; X is N;
R¹⁰ is -L⁷-R⁷;
L⁷ is a bond;
R⁷ is 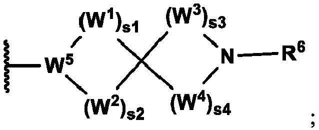
W¹, W², W³, and W⁴ are independently selected from C(R¹)(R¹), C(R¹)(R⁴), C(R⁴)(R⁴), and O;
W⁵ is selected from N and CH;
si is an integer from 1 to 3; s2 is an integer from 1 to 3; s3 is an integer from 1 to 3; s4 is an integer from 1 to 3;
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
L² is selected from -C(O)-;
R⁵ is a 5 membered heteroaryl, optionally substituted with one, two, or three R^{20k}, wherein the 5 membered heteroaryl comprises one, two, or three ring nitrogen atoms and further wherein when R⁵ is directly bonded to a C(O) of L², L² is directly bonded to an N atom of R⁵;
R⁸ is independently selected from hydrogen and halogen;
R¹⁷ is -L¹-R¹⁹;
L¹ is selected from a bond;
R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C₂₋₆alkynyl, -OR¹², -N(R¹²)(R¹³), wherein C₁₋₆alkyl, C₂₋₆alkenyl, and C₂₋₆alkynyl are optionally substituted with one, two, or three R²⁰ⁱ;
R¹⁶ is independently selected from hydrogen and halogen;
R² is -OR^{12d};
each R^{12d} is independently selected from -C(R^{12e})₂-C₂₋₉heterocycloalkyl, wherein -C(R^{12e})₂-C₂₋₉heterocycloalkyl is optionally substituted with one, two, or three R^{20d};
each R^{12e} is independently selected from hydrogen and C₁₋₆alkyl;
each R¹² is independently selected from hydrogen, C₁₋₆alkyl, C₂₋₆alkenyl, C₂₋₆alkynyl, C₃₋₁₀cycloallcyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
each R^{20a}, R^{20d}, R^{20e}, R^{20f}, R^{20f}; and R^{20k} is independently selected from halogen, -CN, oxo, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, - N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋ioaryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C_{3- 10}cycloallcyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²¹, -N(R²⁴)S(O)₂R²⁵, -C(O)R²¹, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl; and
- - - - - - indicates a single or double bond such that all valences are satisfied.

In an aspect is provided a precursor compound of Formula (I-1d), or a pharmaceutically acceptable salt or solvate thereof: 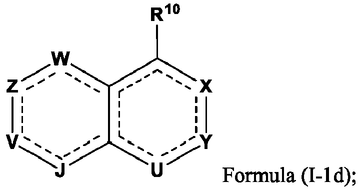 Wherein
W is a bond, N, C(R¹⁸), N(R^{18b}), C(R¹⁸)(R^{18a}), C(O), S(O), or S(O)₂;
Z is N, C(R⁸), N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is N, C(R²), C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, C₁₋₄alkyl, or 2-4 membered heteroalkyl linker, wherein the C₁₋₃alkyl, C₁₋₄alkyl, and 2-4 membered heteroalkyl linker are each optionally substituted with one, two or three R^{20a};
R⁷ is 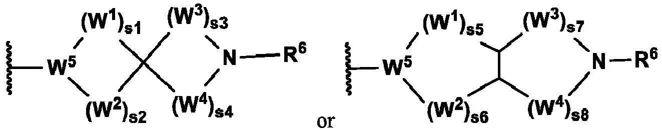
W¹, W², W³, and W⁴ are independently selected from N(R¹), N(R⁴), C(R¹)(R¹), C(R¹)(R⁴), C(R⁴)(R⁴), C(O), S, O, S(O), and S(O)₂;
W⁵ is selected from N, C(R¹), and C(R⁴);
s1 is an integer from 1 to 6; s2 is an integer from 0 to 3; s3 is an integer from 1 to 3; s4 is an integer from 1 to 3;
s5 is an integer from 0 to 6; s6 is an integer from 0 to 3; s7 is an integer from 0 to 3; s8 is an integer from 0 to 3;
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂Cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
L² is selected from -C(O)- and -C(O)N(R¹²)-;
R⁵ is a 5-6 membered heteroaryl, optionally substituted with one, two, or three R^{20k}, wherein the 5-6 membered heteroaryl comprises one, two, or three ring nitrogen atoms and further wherein when R⁵ is directly bonded to a C(O) of L², L² is directly bonded to an N atom of R⁵;
R⁸ and R^{8a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond;
L^{1b} is selected from a bond;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³),-CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20g};
R² is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
each R^{12d} is independently selected from hydrogen, C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl are optionally substituted with one, two, or three R^{20d};
each R^{12e} is independently selected from hydrogen and R^{20d};
R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀ary, -CH₂-C₁₋₉heteroaryl, and C₁₋₉heteroaryl are optionally substituted with one, two, or three R^{20d};
X is C(R³), C(R³)(R³), N(R³), or N;
each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloallcyl, C₂₋₉heterocycloalkyl, C_{6- 10}aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloallcyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and Cl-9heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three p20h.
each R^{20a}, R^{20b}, R^{20c}, R^{20d}, R^{20e}, R^{20f}, R^{20g}, R^{20b}, R²⁰ⁱ, and R^{20k} is independently selected from halogen, -CN, oxo, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C_{3- 10}cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, - SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloallcyl, C₂₋₉heterocycloalkyl, C_{6- 10}aryl, and C₁₋₉heteroaryl; and
- - - - - - indicates a single or double bond such that all valences are satisfied.

In an aspect is provided a precursor compound of Formula (I-le), or a pharmaceutically acceptable salt or solvate thereof: 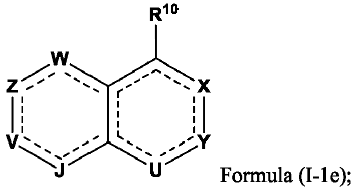 Wherein
W is a C(R¹⁸) or C(O); Z is N, C(R⁸), or N(R^{8b}); V is C(R¹⁷); J is C(R¹⁶); Y is C(R²); U is N; X is N;
R¹⁰ is -L⁷-R⁷;
L⁷ is a bond;
R⁷ is 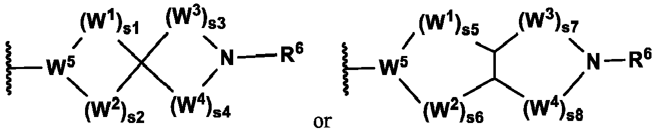
W¹, W², W³, and W⁴ are independently selected from C(R¹)(R¹), C(R¹)(R⁴), C(R⁴)(R⁴), and 0;
W⁵ is selected from N and CH;
s1 is an integer from 1 to 6; s2 is an integer from 0 to 3; s3 is an integer from 1 to 3; s4 is an integer from 1 to 3;
s5 is an integer from 0 to 6; s6 is an integer from 0 to 3; s7 is an integer from 0 to 3; s8 is an integer from 0 to 3;
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂Cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂Cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
L² is selected from -C(O)-;
R⁵ is a 5-6 membered heteroaryl, optionally substituted with one, two, or three R^{20k}, wherein the 5-6 membered heteroaryl comprises one, two, or three ring nitrogen atoms and further wherein when R⁵ is directly bonded to a C(O) of L², L² is directly bonded to an N atom of R⁵;
R⁸ is independently selected from hydrogen, halogen, C₁₋₆allcyl, C₂₋₆alkenyl, C₂₋₆alkynyl, C₃₋₁₀cycloalkyl, C_{2- 9}heterocycloalkyl, -OR¹², -SR¹², and -N(R¹²)(R¹³), wherein C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20c};
R^{8b} is selected from hydrogen, C₁₋₆alkyl, C₂₋₆alkenyl, C₂₋₆alkynyl, C₃₋₁₀cycloalkyl, and C₂₋₉heterocycloalkyl, wherein C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20c};
R¹⁷ is -L¹-R¹⁹;
L¹ is selected from a bond;
R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C₂₋₆alkynyl, C_{3- 10}cycloallcyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ;
R¹⁶ is independently selected from hydrogen, halogen, C₁₋₆allcyl, C₂₋₆alkenyl, C₂₋₆alkynyl, C₃₋₁₀cycloalkyl, C_{2- 9}heterocycloalkyl, -OR¹², -SR¹², and -N(R¹²)(R¹³), wherein C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20g};
R² is C₁₋₆alkyl, C₂₋₆alkenyl, C₂₋₆alkynyl, C₃₋₁₀cycloalkyl, C₂₋₉heterocycloalkyl, -OR^{12d}, -SR^{12d}, or -N(R^{12d})(R¹³), wherein said C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20d};
each R^{12d} is independently selected from C₁₋₆alkyl, C₂₋₆alkenyl, C₂₋₆alkynyl, C₃₋₁₀cycloalkyl, -C(R^{12e})₂-C₃. ₁₀cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C₁. ₉heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C₁. ₉heteroaryl, and C₁₋₉heteroaryl are optionally substituted with one, two, or three R^{20d};
each R^{12e} is independently selected from hydrogen and R^{20d};
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
R¹⁸ is independently selected from hydrogen, halogen, C₁₋₆alkyl, C₂₋₆alkenyl, C₂₋₆alkynyl, C₃₋₁₀cycloalkyl, and C_{2- 9}heterocycloalkyl, wherein C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C_{3- 10}cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl; and
- - - - - - indicates a single or double bond such that all valences are satisfied.

In an aspect is provided a precursor compound of Formula (I-I f), or a pharmaceutically acceptable salt or solvate thereof: 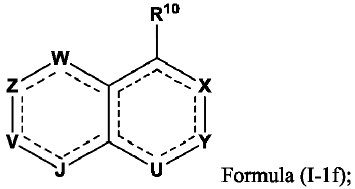 Wherein
W is a CH; Z is N or C(R⁸); V is C(R¹⁷); J is C(R¹⁶); Y is C(R²); U is N; X is N;
R¹⁰ is -L⁷-R⁷;
L⁷ is a bond;
R⁷ is 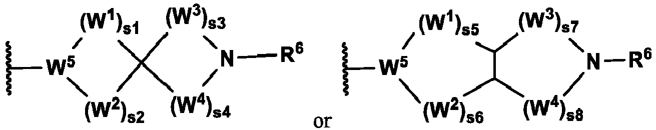
W¹, W², W³, and W⁴ are independently selected from C(R¹)(R¹), C(R¹)(R⁴), C(R⁴)(R⁴), and 0;
W⁵ is selected from N and CH;
s1 is an integer from 1 to 6; s2 is an integer from 0 to 3; s3 is an integer from 1 to 3; s4 is an integer from 1 to 3;
s5 is an integer from 0 to 6; s6 is an integer from 0 to 3; s7 is an integer from 0 to 3; s8 is an integer from 0 to 3;
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
L² is selected from -C(O)-;
R⁵ is a 5 membered heteroaryl, optionally substituted with one, two, or three R^{20k}, wherein the 5 membered heteroaryl comprises one, two, or three ring nitrogen atoms and further wherein when R⁵ is directly bonded to a C(O) of L², L² is directly bonded to an N atom of R⁵;
R⁸ is independently selected from hydrogen and halogen;
R¹⁷ is -L¹-R¹⁹;
L¹ is selected from a bond;
R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C₂₋₆alkynyl, -OR¹², -N(R¹²)(R¹³), wherein C₁₋₆alkyl, C₂₋₆alkenyl, and C₂₋₆alkynyl are optionally substituted with one, two, or three R²⁰ⁱ;
R¹⁶ is independently selected from hydrogen and halogen;
R² is -OR^{12d};
each R^{12d} is independently selected from -C(R^{12e})₂-C₂₋₉heterocycloalkyl, wherein -C(R^{12e})₂-C₂₋₉heterocycloalkyl is optionally substituted with one, two, or three R^{20d};
each R^{12e} is independently selected from hydrogen and C₁₋₆alkyl;
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
each R^{20a}, R^{20d}, R^{20e}, R^{20f}; R²⁰ⁱ, and R^{20k} is independently selected from halogen, -CN, oxo, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, - N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C_{6- 10}aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²¹, -N(R²⁴)S(O)₂R²⁵, -C(O)R²¹, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl; and
- - - - - - indicates a single or double bond such that all valences are satisfied.

In an aspect is provided a precursor compound of Formula (I-1g), or a pharmaceutically acceptable salt or solvate thereof: 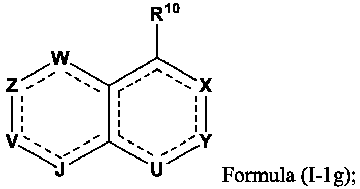 Wherein
W is a CH; Z is N or C(R⁸); V is C(R¹⁷); J is C(R¹⁶); Y is C(R²); U is N; X is N;
R¹⁰ is -L⁷-R⁷;
L⁷ is a bond;
R⁷ is 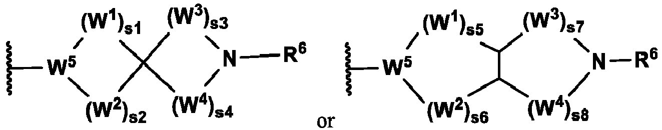
W¹, W², W³, and W⁴ are independently selected from C(R¹)(R¹), C(R¹)(R⁴), C(R⁴)(R⁴), and 0;
W⁵ is selected from N and CH;
s1 is an integer from 1 to 6; s2 is an integer from 0 to 3; s3 is an integer from 1 to 3; s4 is an integer from 1 to 3;
s5 is an integer from 0 to 6; s6 is an integer from 0 to 3; s7 is an integer from 0 to 3; s8 is an integer from 0 to 3;
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
L² is -C(O)-;
R⁵ is selected from 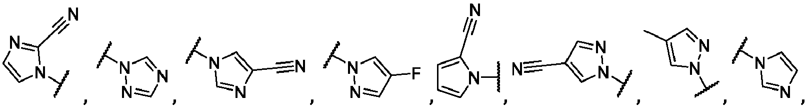 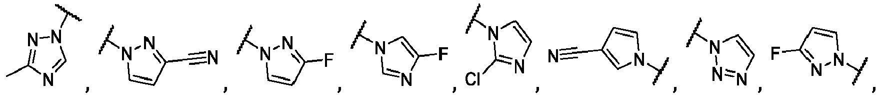 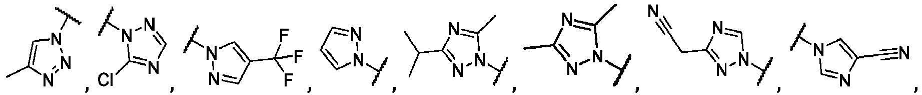 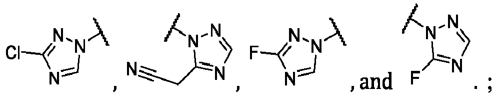
R⁸ is independently selected from hydrogen and halogen;
R¹⁷ is selected from 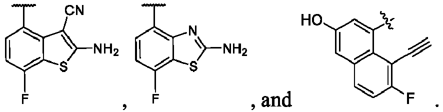
R¹⁶ is independently selected from hydrogen and halogen;
R² is selected from 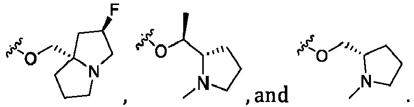
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
each R^{20a} is independently selected from halogen, -CN, oxo, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, - S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, - N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl; and
- - - - - - indicates a single or double bond such that all valences are satisfied.

In an aspect is provided a precursor compound of Formula (I-Ih), or a pharmaceutically acceptable salt or solvate thereof: 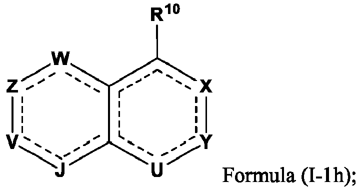 Wherein
W is a CH; Z is N or C(R⁸); V is C(R¹⁷); J is C(R¹⁶); Y is C(R²); U is N; X is N;
R¹⁰ is -L⁷-R⁷;
L⁷ is a bond;
R⁷ is 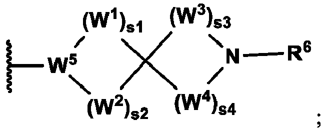
W¹, W², W³, and W⁴ are independently selected from C(R¹)(R¹), C(R¹)(R⁴), C(R⁴)(R⁴), and 0;
W⁵ is selected from N and CH;
s1 is an integer from 1 to 6; s2 is an integer from 0 to 3; s3 is an integer from 1 to 3; s4 is an integer from 1 to 3;
each R¹ is independently selected from hydrogen, C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20a};
R⁶ is -L²-R⁵;
L² is -C(O)-;
R⁵ is selected from 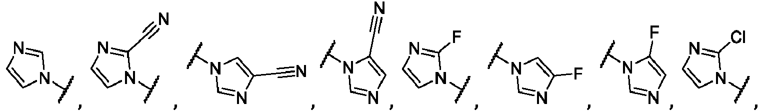 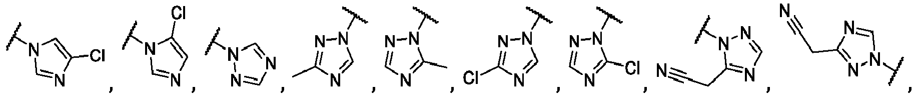 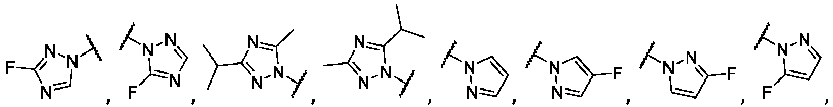 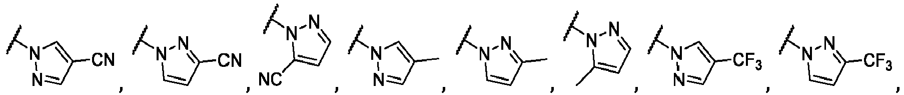 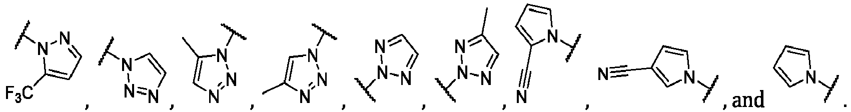
R⁸ is independently selected from hydrogen and halogen;
R¹⁷ is selected from 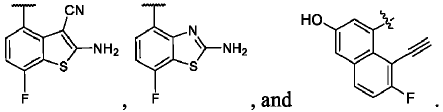
R¹⁶ is independently selected from hydrogen and halogen;
R² is selected from 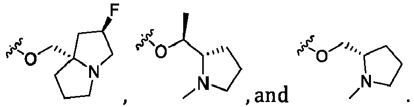
each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
each R^{20a} is independently selected from halogen, -CN, oxo, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, - S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 10}cycloallcyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
each R²³ is independently selected from H and C₁₋₆allcyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl; and
- - - - - - indicates a single or double bond such that all valences are satisfied.

In embodiments, the precursor compound, or a pharmaceutically acceptable salt or solvate thereof, has the formula (I-li): 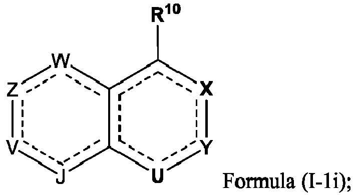 wherein
L⁷ is a bond, -O-, or -NH-;
R⁷ is a 6-12 membered nitrogen containing heterocycloalkyl having a formula selected from: 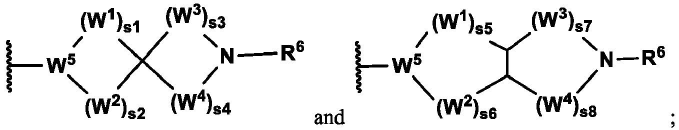
W¹, W², W³, and W⁴ are independently selected from N(R¹), N(R⁴), C(R¹)(R¹), C(R¹)(R⁴), C(R⁴)(R⁴), C(O), S, O, S(O), and S(O)₂;
W⁵ is selected from N, C(R¹), and C(R⁴);
s1 is an integer from 1 to 6; s2 is an integer from 0 to 3; s3 is an integer from 1 to 3; s4 is an integer from 1 to 3;
s5 is an integer from 0 to 6; s6 is an integer from 0 to 3; s7 is an integer from 0 to 3; s8 is an integer from 0 to 3; R⁶ is -L²-R⁵;
each L² is -C(O)-;
R⁵ is a 5 membered heteroaryl, optionally substituted with one, two, or three R^{20k}, wherein the 5 membered heteroaryl comprises one, two, or three ring nitrogen atoms and further wherein when R⁵ is directly bonded to a C(O) of L², L² is directly bonded to an N atom of R⁵;
L¹ is a bond, L^{1b} is a bond; and
R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ.

In embodiments, W is C(R¹⁸) or C(O); Z is N, C(R⁸), or N(R^{8b}); V is C(R¹⁷); J is C(R¹⁶); Y is C(R²); U is N; and X is N.

In embodiments, W is CH; Z is C(R⁸) or N(R^{8b}); V is C(R¹⁷); J is C(R¹⁶); Y is C(R²); U is N; and X is N.

In an aspect is provided a precursor compound, or a pharmaceutically acceptable salt or solvate thereof, having the formula (I-lk): 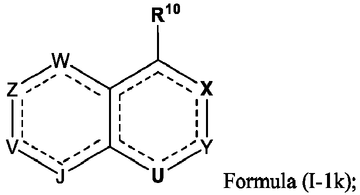 wherein
W is CH; Z is C(R⁸), N, or N(R^{8b}); V is C(R¹⁷); J is C(R¹⁶) or N; Y is C(R²) or C(O); U is N or N(R^{2b}); X is N;
R⁸ is hydrogen or halogen;
R^{8b} is selected from hydrogen, C₁₋₆alkyl, C₃₋₁₀cycloalkyl, or C₂₋₉heterocycloalkyl, wherein C₁₋₆alkyl, C_{3- 10}cycloalkyl, or C₂₋₉heterocycloalkyl are optionally substituted with one, two, or three R^{20c};
R¹⁶ is hydrogen or halogen;
R^{2b} is phenyl or pyridyl, wherein said phenyl or pyridyl are optionally substituted with one, two, or three R^{20d};
L7 is a bond, -O-, or -NHCH₂CH₂-;
R⁷ is a 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl, wherein the 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl is each substituted with one R⁶, and optionally substituted with one, two, or three R⁴;
each R⁴ is independently selected from halogen, oxo, -CN, C₁₋₆alkyl, C₂₋₆alkenyl, C₂₋₆alkynyl, C_{3- 6}cycloalkyl, C₂₋₅heterocycloalkyl, -OR¹², and -N(R¹²)(R¹³); wherein C₁₋₆alkyl, C₂₋₆alkenyl, C₂₋₆alkynyl, C₃₋₆cycloalkyl, and C₂₋₅heterocycloalkyl are optionally substituted with one, two, or three R^{20a};
R⁶ is -L²-R⁵;
each L² is -C(O)-, -N(R¹²)CO-, or C(O)N(R¹²)-;
R⁵ is a 5-6 membered heteroaryl, optionally substituted with one, two, or three R^{20k}, wherein the 5-6 membered heteroaryl comprises one, two, or three ring nitrogen atoms and further wherein when R⁵ is directly bonded to a C(O) of L², L² is directly bonded to an N atom of R⁵;
L¹ is a bond;
R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C_{2- 6}alkynyl, -OR¹², -N(R¹²)(R¹³), wherein C₁₋₆alkyl, C₂₋₆alkenyl, and C₂₋₆alkynyl are optionally substituted with one, two, or three R²⁰ⁱ;
R² is -O-C(R^{12e})₂-C₂₋₉heterocycloalkyl optionally substituted with one, two, or three R^{20d};
each R^{12e} is independently selected from hydrogen and C₁₋₆alkyl;
each R¹² is independently selected from hydrogen, C₁₋₆alkyl, C₂₋₆alkenyl, C₂₋₆alkynyl, C₃₋₁₀cycloalkyl, and C₂₋₉heterocycloalkyl, wherein C₁₋₆alkyl, C₂₋₆alkenyl, C₂₋₆alkynyl, C₃₋₁₀cycloalkyl, and C_{2- 9}heterocycloalkyl are optionally substituted with one, two, or three R^{20d};
each R¹³ is independently selected from hydrogen, C₁₋₆alkyl, and C₁₋₆haloalkyl;
each R¹⁴ is independently selected from hydrogen, C₁₋₆alkyl, and C₁₋₆haloalkyl;
each R^{20a}, R^{20c}, R^{20d}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, C₂₋₉heterocycloalkyl, -OR²¹, -SR²¹, -N(R²²)(R²³), -C(O)OR²², - C(O)N(R²²)(R²³), -N(R²⁴)C(O)R²¹, and -C(O)R²¹, wherein Cl-6alkyl, C2-6alkenyl, C2-6alkynyl, C_{3- 10}cycloalkyl, and C₂₋₉heterocycloalkyl, are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.

In an aspect is provided a precursor compound, or a pharmaceutically acceptable salt or solvate thereof, having the formula (I-1m): 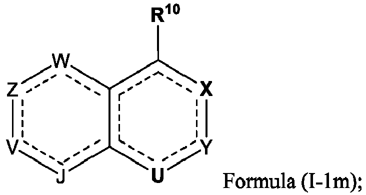 wherein
W is CH; Z is C(R⁸) or N; V is C(R¹⁷); J is C(R¹⁶); Y is C(R²); U is N; X is N;
R⁸ is hydrogen or halogen;
R¹⁶ is hydrogen or halogen;
L7 is a bond, -O-, or -NHCH₂CH₂-;
R⁷ is a 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl, wherein the 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl is each substituted with one R⁶, and optionally substituted with one, two, or three R⁴;
each R⁴ is independently selected from halogen, C₁₋₄alkyl, C₂₋₄alkenyl, C₂₋₄alkynyl, and -OH, wherein C_{1- 4}alkyl, C₂₋₄alkenyl, and C₂₋₄alkynyl are optionally substituted with one, two, or three R^{20a};
R⁶ is -L²-R⁵;
each L² is -C(0)- or -N(R¹²)CO-;
R⁵ is a 5 membered heteroaryl, optionally substituted with one, two, or three R^{20k}, wherein the 5 membered heteroaryl comprises one, two, or three ring nitrogen atoms and further wherein when R⁵ is directly bonded to a C(O) of L², L² is directly bonded to an N atom of R⁵;
L¹ is a bond;
R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ;
each R¹ⁱ is independently selected from halogen, -CN, C₁₋₄alkyl, C₂₋₄alkynyl, -OH, and -NH₂, wherein C_{1- 6}alkyl and C₂₋₄alkynyl are optionally substituted with one, two, or three R²⁰ⁱ;
R² is -O-C(R^{12e})₂-C₂₋₉heterocycloalkyl optionally substituted with one, two, or three R^{20d};
each R^{12e} is independently selected from hydrogen and methyl;
each R¹² is independently selected from hydrogen and C₁₋₆alkyl, wherein C₁₋₆alkyl is optionally substituted with one, two, or three R^{20d};
each R¹³ is independently selected from hydrogen, C₁₋₆alkyl, and C₁₋₆haloalkyl;
each R¹⁴ is independently selected from hydrogen, C₁₋₆alkyl, and C₁₋₆haloalkyl;
each R^{20a}, R^{20c}, R^{20d}, R²⁰ⁱ, and R^{20k} is independently selected from halogen, -CN, oxo, C₁₋₄alkyl, C₂₋₄alkenyl, C₂₋₄alkynyl, C₃₋₆cycloalkyl, C₂₋₅heterocycloalkyl, -OR²¹, -SR²¹, -N(R²²)(R²³), -C(O)OR²², - C(O)N(R²²)(R²³), -N(R²⁴)C(O)R²¹, and -C(O)R²¹, wherein C₁₋₄alkyl, C₂₋₄alkenyl, C₂₋₄alkynyl, C_{3- 6}cycloalkyl, and C₂₋₅heterocycloalkyl" are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵,-S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
- - - - - - indicates a single or double bond such that all valences are satisfied.\

In an aspect is provided a precursor compound, or a pharmaceutically acceptable salt or solvate thereof, having the formula (I-In): 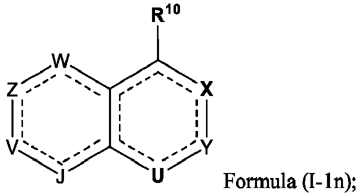 wherein
W is CH; Z is C(R⁸) or N; V is C(R¹⁷); J is C(R¹⁶); Y is C(R²); U is N; X is N;
R⁸ is hydrogen or Cl;
R¹⁶ is F;
L7 is a bond;
R⁷ is a 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl, wherein the 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl is each substituted with one R⁶, and optionally substituted with one, two, or three R⁴;
each R⁴ is independently selected from halogen, C₁₋₄alkyl, and -OH, wherein C₁₋₄alkyl is optionally substituted with one, two, or three R^{20a};
R⁶ is -L²-R⁵;
each L² is -C(O)-;
R⁵ is a 5 membered heteroaryl, optionally substituted with one, two, or three R^{20k}, wherein the 5 membered heteroaryl comprises one, two, or three ring nitrogen atoms and further wherein when R⁵ is directly bonded to a C(O) of L², L² is directly bonded to an N atom of R⁵;
L¹ is a bond;
R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ;
each R¹ⁱ is independently selected from halogen, -CN, C₁₋₄alkyl, C₂₋₄alkynyl, -OH, and -NH₂, wherein C_{1- 6}alkyl and C₂₋₄alkynyl are optionally substituted with one, two, or three R²⁰ⁱ;
R² is -O-C(R^{12e})₂-C₂₋₉heterocycloalkyl optionally substituted with one, two, or three R^{20d};
each R^{12e} is independently selected from hydrogen and methyl;
each R^{20d} is independently selected from halogen, C₁₋₄alkyl, C₂₋₄alkenyl, and C₂₋₄alkynyl wherein C₁₋₄alkyl, C₂₋₄alkenyl, and C₂₋₄alkynyl are optionally substituted with one, two, or three groups independently selected from halogen, oxo, -OH, and -NH₂;
each R²⁰ⁱ is independently selected from halogen;
each R^{20k} is independently selected from halogen, -CN, and C₁₋₄alkyl, wherein C₁₋₄alkyl is optionally substituted with one, two, or three groups independently selected from halogen, -CN, -OH, and -NH₂; and
- - - - - - indicates a single or double bond such that all valences are satisfied.

In embodiments, R⁷ is a 7-12 membered spirocyclic bicyclic nitrogen containing heterocycloalkyl or 7-12 membered fused bicyclic nitrogen containing heterocycloalkyl, wherein the 7-12 membered spirocyclic bicyclic nitrogen containing heterocycloalkyl or 7-12 membered fused bicyclic nitrogen containing heterocycloalkyl is each substituted with one R⁶, and optionally substituted with one, two, or three R⁴.

In embodiments of the precursor compound, or a pharmaceutically acceptable salt or solvate thereof, the precursor compound has a formula selected from: 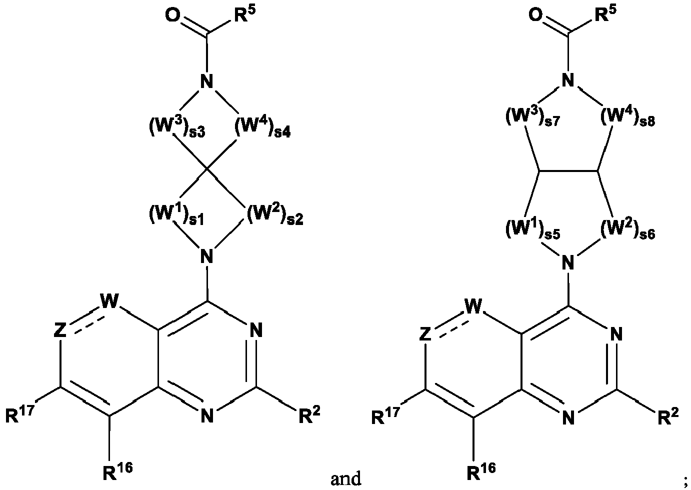 wherein
W¹, W², W³, and W⁴ are independently selected from N(R⁴), CH₂, CH(R⁴), C(R⁴)(R⁴), C(O), S, O, S(O), and S(O)₂;
W⁵ is selected from N, C(R¹), and C(R⁴);
s1 is an integer from 1 to 6; s2 is an integer from 0 to 3; s3 is an integer from 1 to 3; s4 is an integer from 1 to 3; s5 is an integer from 0 to 6; s6 is an integer from 0 to 3; s7 is an integer from 0 to 3; and s8 is an integer from 0 to 3.
R², R⁵, R¹⁶, R¹⁷, Z, and W are as described herein.

In embodiments of the precursor compound, or a pharmaceutically acceptable salt or solvate thereof,
W¹, W², W³, and W⁴ are independently selected from CH₂, CH(R⁴), C(R⁴)(R⁴), and 0;
W⁵ is selected from N, CH, and C(R⁴);
s1 is an integer from 1 to 6; s2 is an integer from 0 to 3; s3 is an integer from 1 to 3; s4 is an integer from 1 to 3; s5 is an integer from 0 to 6; s6 is an integer from 0 to 3; s7 is an integer from 0 to 3; and s8 is an integer from 0 to 3.

In embodiments, s1 is 0. In embodiments, s1 is 1. In embodiments, s1 is 2. In embodiments, s1 is 3. In embodiments, s1 is 4. In embodiments, s1 is 5. In embodiments, s1 is 6. In embodiments, s2 is 0. In embodiments, s2 is 1. In embodiments, s2 is 2. In embodiments, s2 is 3. In embodiments, s3 is 1. In embodiments, s3 is 2. In embodiments, s3 is 3. In embodiments, s4 is 1. In embodiments, s4 is 2. In embodiments, s4 is 3. In embodiments, s5 is 0. In embodiments, s5 is 1. In embodiments, s5 is 2. In embodiments, s5 is 3. In embodiments, s6 is 0. In embodiments, s6 is 1. In embodiments, s6 is 2. In embodiments, s6 is 3. In embodiments, s7 is 0. In embodiments, s7 is 1. In embodiments, s7 is 2. In embodiments, s7 is 3. In embodiments, s8 is 0. In embodiments, s8 is 1. In embodiments, s8 is 2. In embodiments, s8 is 3.

In some embodiments, W¹ is independently N(R¹). In some embodiments, W¹ is independently N(R⁴). In some embodiments, W¹ is independently C(R¹)(R¹). In some embodiments, W¹ is independently C(R¹)(R⁴). In some embodiments, W¹ is independently C(R⁴)(R⁴). In some embodiments, W¹ is independently C(O). In some embodiments, W¹ is independently S. In some embodiments, W¹ is independently O. In some embodiments, W¹ is independently S(O). In some embodiments, W¹ is independently S(O)₂. In some embodiments, W¹ is independently NH. In some embodiments, W¹ is independently CH₂. In some embodiments, W¹ is independently CH(R⁴).

In some embodiments, W² is independently N(R¹). In some embodiments, W² is independently N(R⁴). In some embodiments, W² is independently C(R¹)(R¹). In some embodiments, W² is independently C(R¹)(R⁴). In some embodiments, W² is independently C(R⁴)(R⁴). In some embodiments, W² is independently C(O). In some embodiments, W² is independently S. In some embodiments, W² is independently O. In some embodiments, W² is independently S(O). In some embodiments, W² is independently S(O)₂. In some embodiments, W² is independently NH. In some embodiments, W² is independently CH₂. In some embodiments, W² is independently CH(R⁴).

In some embodiments, W³ is independently N(R¹). In some embodiments, W³ is independently N(R⁴). In some embodiments, W³ is independently C(R¹)(R¹). In some embodiments, W³ is independently C(R¹)(R⁴). In some embodiments, W³ is independently C(R⁴)(R⁴). In some embodiments, W³ is independently C(O). In some embodiments, W³ is independently S. In some embodiments, W³ is independently O. In some embodiments, W³ is independently S(O). In some embodiments, W³ is independently S(O)₂. In some embodiments, W³ is independently NH. In some embodiments, W³ is independently CH₂. In some embodiments, W³ is independently CH(R⁴).

In some embodiments, W⁴ is independently N(R¹). In some embodiments, W⁴ is independently N(R⁴). In some embodiments, W⁴ is independently C(R¹)(R¹). In some embodiments, W⁴ is independently C(R¹)(R⁴). In some embodiments, W⁴ is independently C(R⁴)(R⁴). In some embodiments, W⁴ is independently C(O). In some embodiments, W⁴ is independently S. In some embodiments, W⁴ is independently O. In some embodiments, W⁴ is independently S(O). In some embodiments, W⁴ is independently S(O)₂. In some embodiments, W⁴ is independently NH. In some embodiments, W⁴ is independently CH₂. In some embodiments, W⁴ is independently CH(R⁴).

In some embodiments, W⁵ is independently N. In some embodiments, W⁵ is independently C(R¹). In some embodiments, W⁵ is independently C(R⁴). In some embodiments, W⁵ is independently CH.

In embodiments, the precursor compound or compound has a formula selected from: 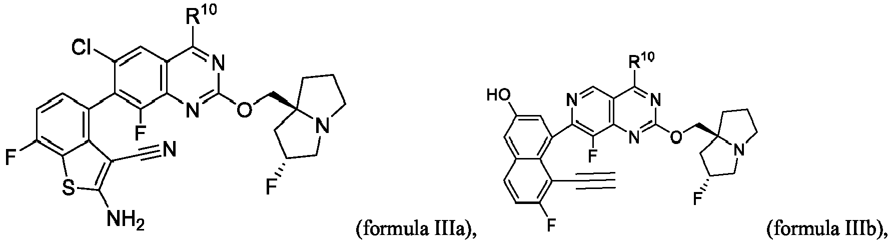 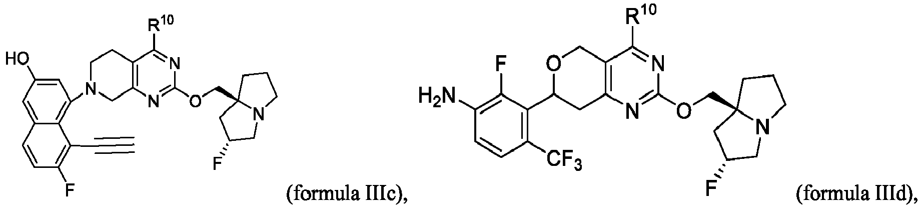 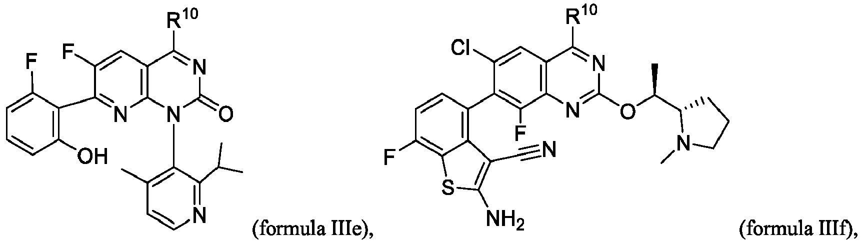 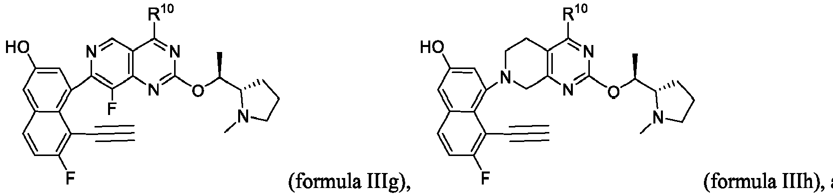 and 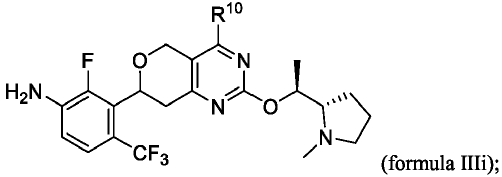 wherein RIO is as described herein, including in any aspect or embodiment (e.g., any single cell of Table 2).

The modified Ras mutant protein may comprise a covalently bonded compound comprising a staying group having a formula:

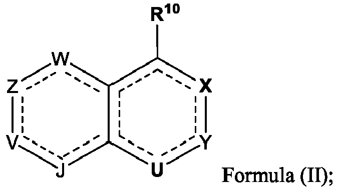

Wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C_{3- 6}cycloalkyl)- are substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or Ci-nheteroaryl, wherein the C₃₋₁₂cycloalkyl, C_{1- 11}heterocycloalkyl, C₆₋₁₂aryl, or Ci-nheteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and Ci-nheteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -C₀₋₃alkyl-C(O)-, -C_{0- 3}alkyl-N(R¹⁴)C(O)-, -C₀₋₃alkyl-S(O)-, -C₀₋₃alkyl-N(R¹⁴)S(O)-, -C₀₋₃alkyl-S(O)₂-, or -C₀₋₃alkyl-N(R¹⁴)S(O)₂-; wherein R⁶ is directly bonded to the serine residue corresponding to position 12 of SEQ ID No: 1; R⁸ and R^{8a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{17b} is -L^{1b}-R¹⁹, hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -0-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, - N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆lialoalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20b}, and R²⁰ⁱ are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), _ OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and -- - indicates a single or double bond such that all valences are satisfied.

In one aspect provided is a complex of formula: 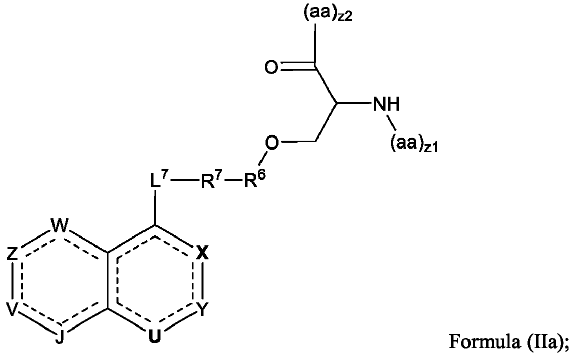 wherein each aa is independently an amino acid; z1 is an integer equal to at least 1 or (aa)z1 is hydrogen; z2 is an integer equal to at least 1 or (aa)z2 is OH; W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O)-, or C_{1- 4}alkyl, wherein the C₁₋₄alkyl is optionally substituted with one, two or three R^{20a}; R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C_{3- 6}cycloalkyl)- are optionally substituted with one or more R¹ and/or one or more R⁴;; wherein two substituents selected from R¹ and R⁴ that are bonded to the same or adjacent atoms are optionally joined to form a C_{3- 12}cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -C₀₋₃alkyl-C(O)-, -C_{0- 3}alkyl-N(R¹⁴)C(O)-, -C₀₋₃alkyl-S(O)-, -C₀₋₃alkyl-N(R¹⁴)S(O)-, -C₀₋₃alkyl-S(O)₂-, or -C₀₋₃alkyl-N(R¹⁴)S(O)₂-; wherein R⁶ is directly bonded to the serine corresponding to position 12 of SEQ ID No: 1; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)2N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)2N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20g}; R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹¹; each R¹¹ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), _ CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂-₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³),-C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20b}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³),-OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, _ S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect provided is a precursor compound of Formula (Ib), or a pharmaceutically acceptable salt or solvate thereof: 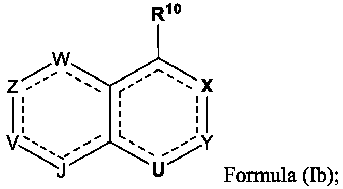 wherein W is N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C_{1- 4}alkyl is optionally substituted with one, two or three R^{20a}; R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C_{6- 12}aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C_{0- 3}alkyl-S(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁₋C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, - S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloallcyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloallcyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C2-₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In an aspect provided is a precursor compound of Formula (Ic), or a pharmaceutically acceptable salt or solvate thereof: 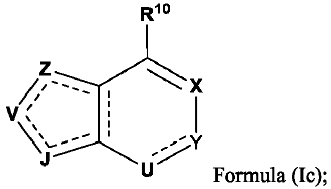 wherein Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloallcyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C₀₋₃alkyl-, or -C_{0- 3}alkyl-N(R¹⁴)S(O)₂-; each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f)}(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹; each R¹¹ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³),-N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C_{2- 9}heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect provided is a precursor compound of Formula (Id), or a pharmaceutically acceptable salt or solvate thereof: 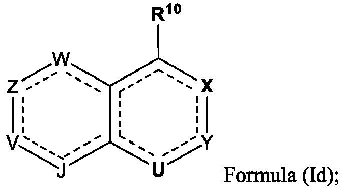 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C_{1- 4}alkyl is optionally substituted with one, two or three- R^{20a}; R⁷ is a 7-12 membered nitrogen containing spirocyclic heterocycloalkyl substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)-, - C₀₋₃alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C_{0- 3}alkyl-N(R¹⁴)S(O), -C₀₋₃alkyl-S(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², _ C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)2N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³),-CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C2-₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C2-6alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In an aspect provided is a precursor compound of Formula (Ie), or a pharmaceutically acceptable salt or solvate thereof: 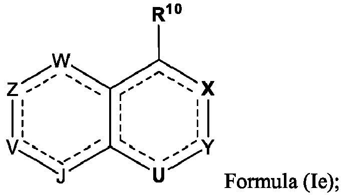 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three- R^{20a}; R⁷ is a 7-12 membered nitrogen containing fused heterocycloalkyl, 7-12 membered nitrogen containing fused aryl, or 7-12 membered nitrogen containing fused heteroaryl substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)-, - C₀₋₃alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C_{0- 3}alkyl-N(R¹⁴)S(O), -C₀₋₃alkyl-S(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², _ C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)2N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), _ N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In an aspect provided is a precursor compound of Formula (If), or a pharmaceutically acceptable salt or solvate thereof: 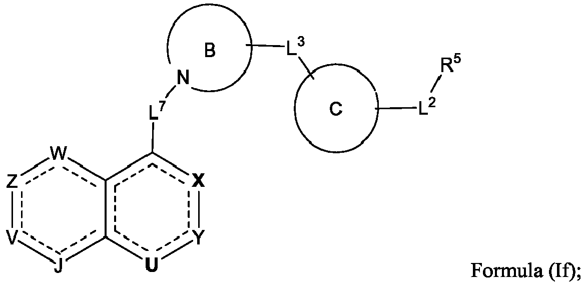 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y and U are independently selected from N, C(R²), C(R²)(R²), N(R^{2b}), S(O), S(O)₂, and C(O); L⁷ is a bond, -C(O)-, -S-, -S(O)₂-, -S(O)-, or C₁₋₄alkyl, wherein the C₁₋₄alkyl is optionally substituted with one, two or three- R^{20a}; Ring B is a 3-12 membered nitrogen containing saturated heterocycloalkyl optionally substituted with one or more R¹ and/or one or more R⁴; Ring C is a 3-12 membered nitrogen containing saturated heterocycloalkyl or C₃₋₆cycloalkyl, each optionally substituted with one or more R¹ and/or one or more R⁴; L³ is an N-C bond joining Ring B and Ring C; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C_{0- 3}alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C₀₋₃alkyl-, or -C_{0- 3}alkyl-N(R¹⁴)S(O)₂-; each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is independently hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C_{2- 9}heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In one aspect provided is a precursor compound of Formula (Ii), or a pharmaceutically acceptable salt or solvate thereof: 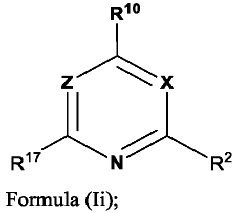 wherein Z is N and X is C(R³); or Z is C(R⁸) and X is N; R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 7-12 membered nitrogen containing spirocyclic heterocycloalkyl substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C_{1- 11}heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C_{2- 6}alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C_{6- 12}aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)-, -C₀₋₃alkyl-C(O)N(R¹⁴)-C₀₋₃alkyl-, -C_{0- 3}alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-; each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; L¹ is selected from a bond, C₁-C₆alkyl, C₂-C₆alkenyl, C₂-C₆alkynyl, -O-, -N(R¹⁴)-, - C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C_{2- 12}heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, - CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; R² is halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²²,-C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²² is independently selected from H, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl; each R²³ is independently selected from H and C_{1- 6}alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and = indicates a single or double bond such that all valences are satisfied.

In one aspect provided is a precursor compound of Formula (Ij), or a pharmaceutically acceptable salt or solvate thereof: 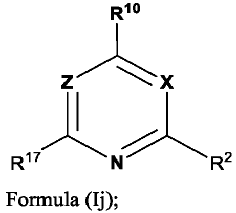 wherein Z is N and X is C(R³); or Z is C(R⁸) and X is N; R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 7-12 membered nitrogen containing fused heterocycloalkyl, fused 7-12 membered nitrogen containing aryl, or fused 7-12 membered nitrogen containing heteroaryl substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloallcyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C₀₋₃alkyl-, or -C_{0- 3}alkyl-N(R¹⁴)S(O)₂-; each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R¹⁷ is -L¹-R¹⁹, halogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)2R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, - S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R¹⁹ is selected from a C_{3- 12}cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R² is halogen, -CN, C₁₋₆alkyl, C₂₋₆alkenyl, C₂₋₆alkynyl, C₃₋₆cydoalkyl, -OR¹², -SR¹², - N(R^{12c})(R¹³), -C(O)OR¹², -OC(O)N(R¹²)(R¹³), -N(R¹⁴)C(O)N(R¹²)(R¹³), -N(R¹⁴)C(O)OR¹³, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)2R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, or -(C_{6- 10}aryl)-R^{12b}, wherein said C₁₋₆alkyl, C₂₋₆alkenyl, C₂₋₆alkynyl, and C₃₋₁₀cycloalkyl, are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R^{12c} is independently selected from C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In one aspect provided is a precursor compound of Formula (Ik), or a pharmaceutically acceptable salt or solvate thereof: 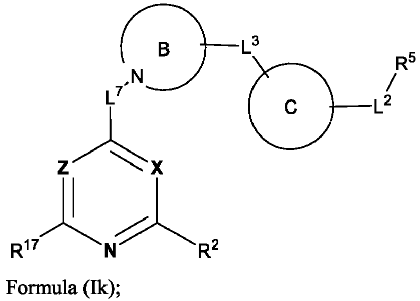 wherein Z is N and X is C(R³); or Z is C(R⁸) and X is N; L⁷ is a bond, -C(O)-, -S-, -S(O)₂-, -S(O)-, or C₁₋₄alkyl, wherein the C₁₋₄alkyl is optionally substituted with one, two or three R^{20a}; Ring B is a 4-6 membered nitrogen containing saturated heterocycloalkyl optionally substituted with one or more R¹ and/or one or more R⁴; Ring C is a 4-6 membered nitrogen containing saturated heterocycloalkyl or C₃₋₆cycloalkyl, each optionally substituted with one or more R¹ and/or one or more R⁴; L³ is an N-C bond joining Ring B and Ring C; wherein two substituents selected from R¹ and R⁴ that are bonded to the same or adjacent atoms are optionally joined to form a C₃₋₁₂cycloalkyl, C_{1- 11}heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C_{1- 11}heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; each L² is -C₀₋₃alkyl-C(O)-, -C₀₋₃alkyl-C(O)N(R¹⁴)-C₀₋₃alkyl-, - C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, - C₀₋₃alkyl-S(O)₂N(R¹⁴)-C₀₋₃alkyl-, or -C₀₋₃alkyl-N(R¹⁴)S(O)₂-; each R⁵ is a 5 or 12 membered partially unsaturated heterocycloalkyl or a 5 or 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³),-CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, _ C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C_{2- 12}heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, - CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵,-S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; R² is halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; wherein one of R² and R¹⁷ is not hydrogen; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₁₀aryl, -CH₂-C₁₋₉heteroaryl, and C₁₋₉heteroaryl, wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substitutedwith one, two, or three R^{20f}; each R^{20a}, R^{20b}, R^{20c}, R^{20d}, R^{20e}, R^{20f}, R^{20g}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂(R²²)(R²³), -OCH₂C(O)OR²², and -OC(O)R²⁵; or two R^{20k} bonded to the same or adjacent atoms may optionally be joined to form a C₃₋₆cycloalkyl, C_{2- 9}heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect provided is a precursor compound of Formula (Ib), or a pharmaceutically acceptable salt or solvate thereof: 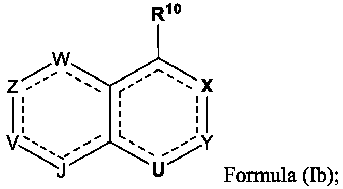 wherein W is N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C_{1- 4}alkyl is optionally substituted with one, two or three R^{20a}; R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C_{6- 12}aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)O-, -C₀₋₃alkyl-C(O)-, -C_{0- 3}alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C_{0- 3}alkyl-N(R¹⁴)S(O), -C₀₋₃alkyl-S(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², _ C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹¹; each R¹¹ is independently selected from halogen, oxo, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)2N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³),-N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³),-CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), _ N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and - - - - - - indicates a single or double bond such that all valences are satisfied.

In an aspect provided is a precursor compound of Formula (Ic), or a pharmaceutically acceptable salt or solvate thereof: 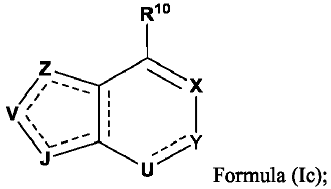 wherein Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloallcyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C_{2- 9}heterocycloallcyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)O-, -C₀₋₃alkyl-C(O)-, -C₀₋₃alkyl-C(O)N(R¹⁴)-C_{0- 3}alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkylS(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(H)(R¹²), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, - S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹; each R¹¹ is independently selected from halogen, oxo, -CN, C₁₋₆alkyl, C₁₋₆haloallcyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} are each independently selected from halogen, - CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl; and - - - - - - indicates a single or double bond such that all valences are satisfied.

In an aspect provided is a precursor compound of Formula (Id), or a pharmaceutically acceptable salt or solvate thereof: 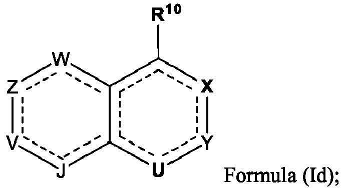 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C_{1- 4}alkyl is optionally substituted with one, two or three- R^{20a}; R⁷ is a 7-12 membered nitrogen containing spirocyclic heterocycloalkyl substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is-C₀₋₃alkyl-C(O)O-, - C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, - N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, - N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, - C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵,-C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C2-6alkenyl, C2-6alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C2-₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloallcyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), _ N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₁-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C2-6alkynyl, C₃₋₁₀cycloalkyl, C2-9heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and = indicates a single or double bond such that all valences are satisfied.

In an aspect provided is a precursor compound of Formula (Ie), or a pharmaceutically acceptable salt or solvate thereof: 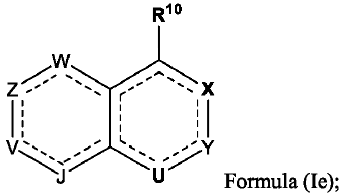 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three- R^{20a}; R⁷ is a 7-12 membered nitrogen containing fused heterocycloalkyl, 7-12 membered nitrogen containing fused aryl, or 7-12 membered nitrogen containing fused heteroaryl substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)O-,-C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, - N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, - N(R¹⁴)S(O)₂-, -OCON(R¹⁴)-, -N(R¹⁴)C(O)O-, N(R^{1e}), C(0)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, - C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², _ C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b}; R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C2-6alkynyl, C₃₋₁₀cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and - - - - - indicates a single or double bond such that all valences are satisfied.

In an aspect provided is a precursor compound of Formula (If), or a pharmaceutically acceptable salt or solvate thereof: 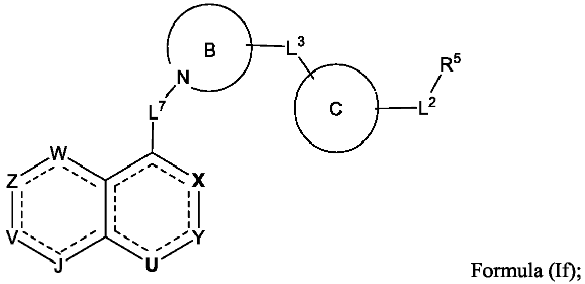 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y and U are independently selected from N, C(R²), C(R²)(R²), N(R^{2b}), S(O), S(O)₂, and C(O); L⁷ is a bond, -C(O)-, -S-, -S(O)₂-, -S(O)-, or C₁₋₄alkyl, wherein the C₁₋₄alkyl is optionally substituted with one, two or three- R^{20a}; Ring B is a 3-12 membered nitrogen containing saturated heterocycloalkyl optionally substituted with one or more R¹ and/or one or more R⁴; Ring C is a 3-12 membered nitrogen containing saturated heterocycloalkyl or C₃₋₁₀cycloalkyl, each optionally substituted with one or more R¹ and/or one or more R⁴; L³ is an N-C bond joining Ring B and Ring C; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; each L² is -C₀₋₃alkyl-C(O)O-,-C₀₋₃alkyl-C(O)-, -C₀₋₃alkyl-C(O)N(R¹⁴)-C₀₋₃alkyl-, -C_{0- 3}alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C_{0- 3}alkyl-S(O)₂N(R¹⁴)-C₀₋₃alkyl-, or -C₀₋₃alkyl-N(R¹⁴)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ and R^{8a} are independently selected from hydrogen, halogen, -CN, C₁₋₆alkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; R² is independently hydrogen, halogen, - CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C_{2- 9}heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and - - - - - indicates a single or double bond such that all valences are satisfied.

In one aspect provided is a precursor compound of Formula (Ii), or a pharmaceutically acceptable salt or solvate thereof: 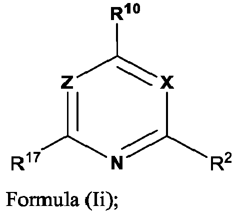 wherein Z is N and X is C(R³); or Z is C(R⁸) and X is N; R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 7-12 membered nitrogen containing spirocyclic heterocycloalkyl substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C_{1- 11}heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C_{2- 6}alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C_{6- 12}aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)O-, -C₀₋₃alkyl-C(O)-, -C₀₋₃alkyl-C(O)N(R¹⁴)-C_{0- 3}alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkylS(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ is selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is selected from -L¹-R¹⁹, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; L¹ is selected from a bond, C₁-C₆alkyl, C₂-C₆alkenyl, C₂-C₆alkynyl, -O-, -N(R¹⁴)-, - C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C_{1- 6}alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C_{1- 6}alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, - CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b}; R² is halogen, -CN, C₁₋₆alkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} is independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C2-9heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C_{1- 6}haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl; each R²² is independently selected from H, C₁₋₆alkyl, C₁₋₆haloalkyl, C₂₋₆alkenyl, C₂₋₆alkynyl, C_{3- 10}cycloallcyl, C2-9heterocycloalkyl, C₆₋₁₀aryl, and C₁₋₉heteroaryl; each R²³ is independently selected from H and C_{1- 6}alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl; and - - - - - - indicates a single or double bond such that all valences are satisfied.

In one aspect provided is a precursor compound of Formula (Ij), or a pharmaceutically acceptable salt or solvate thereof: 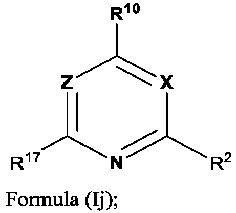 wherein Z is N and X is C(R³); or Z is C(R⁸) and X is N; R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 7-12 membered nitrogen containing fused heterocycloalkyl, fused 7-12 membered nitrogen containing aryl, or fused 7-12 membered nitrogen containing heteroaryl substituted with one R⁶ and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloallcyl, C₃₋₁₂cycloalkyl, - CH₃-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C₀₋₃alkyl-C(O)O-,-C₀₋₃alkyl-C(O)-, -C₀₋₃alkyl-C(O)N(R¹⁴)-C_{0- 3}alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkylS(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ is selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)2N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is selected from -L¹-R¹⁹, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)2N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h}; L¹ is selected from a bond, C₁-C₆alkyl, C₂-C₆alkenyl, C₂-C₆alkynyl, -O-, -N(R¹⁴)-, - C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C_{1- 6}alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C_{1- 6}alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, - CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20b}; R² is halogen, -CN, C₁₋₆alkyl, C_{2- 6}alkenyl, C₂₋₆alkynyl, C₃₋₁₀cycloalkyl, -OR¹², -SR¹², -N(R^{12c})(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹³, -S(O)R¹³, -OC(O)R¹³, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, or -(C₆₋₁₀aryl)-R^{12b}, wherein said C₁₋₆alkyl, C_{2- 6}alkenyl, C₂₋₆alkynyl, and C₃₋₁₀cycloalkyl, are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R^{12c} is independently selected from C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20b}, R²⁰ⁱ, and R^{20k} is independently selected from halogen, -CN, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), _ C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C_{1- 6}haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl; each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl; each R²³ is independently selected from Hand C_{1- 6}alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl; and - - - - - - indicates a single or double bond such that all valences are satisfied.

In one aspect provided is a precursor compound of Formula (Ik), or a pharmaceutically acceptable salt or solvate thereof: 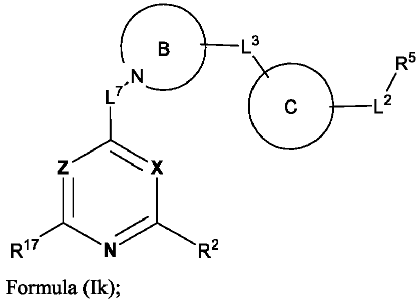 wherein Z is N and X is C(R³); or Z is C(R⁸) and X is N; L⁷ is a bond, -C(O)-, -S-, -S(O)₂-, -S(O)-, or C₁₋₄alkyl, wherein the C₁₋₄alkyl is optionally substituted with one, two or three R^{20a}; Ring B is a 4-6 membered nitrogen containing saturated heterocycloalkyl optionally substituted with one or more R¹ and/or one or more R⁴; Ring C is a 4-6 membered nitrogen containing saturated heterocycloalkyl or C₃₋₁₀cycloalkyl, each optionally substituted with one or more R¹ and/or one or more R⁴; L³ is an N-C bond joining Ring B and Ring C; wherein two substituents selected from R¹ and R⁴ that are bonded to the same or adjacent atoms are optionally joined to form a C_{3- 12}cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a}; each L² is -C₀₋₃alkyl-C(O)O-, -C₀₋₃alkyl-C(O)-, -C₀₋₃alkyl-C(O)N(R¹⁴)-C₀₋₃alkyl-, -C₀₋₃alkyl-N(R¹⁴)C(O)-, -C₀₋₃alkyl-S(O), -C₀₋₃alkyl-S(O)N(R¹⁴)-C₀₋₃alkyl-, -C₀₋₃alkyl-N(R¹⁴)S(O), -C₀₋₃alkyl-S(O)₂-, -C₀₋₃alkyl-S(O)₂N(R¹⁴)-C₀₋₃alkyl-, or -C₀₋₃alkyl-N(R¹⁴)S(O)₂-; each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵; R⁸ is selected from hydrogen, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c}; R¹⁷ is selected from -L¹-R¹⁹, halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)_{2N}(R¹⁴)-,-S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C_{2- 11}heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b}; R² is halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; wherein one of R² and R¹⁷ is not hydrogen; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R²⁰ⁱ, and R^{20k} is independently selected from halogen, -CN, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, C₁₋₉heteroaryl, -OR²¹, -SR²¹, -N(R²²)(R²³), -C(O)OR²², -C(O)N(R²²)(R²³),-C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(W²)(W³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C_{1- 6}haloalkyl, C₁₋₆alkoxy, C₁₋₆haloalkoxy, -OR²¹, -SR²¹, -N(R²²)(R²³), -C(O)OR²², -C(O)N(R²²)(R²³),-C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵,-N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl; each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl; each R²³ is independently selected from Hand C_{1- 6}alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl; and - - - - - - indicates a single or double bond such that all valences are satisfied.

In embodiments, L² is -C₀₋₃alkyl-C(O)-. In embodiments, L² is -C₀₋₃alkyl-C(O)N(R¹⁴)-C₀₋₃alkyl-. In embodiments, L² is -C₀₋₃alkyl-N(R¹⁴)C(O)-. In embodiments, L² is -C₀₋₃alkyl-S(O)-. In embodiments, L² is -C₀₋₃alkyl-S(O)N(R¹⁴)-C₀₋₃alkyl-. In embodiments, L² is -C₀₋₃alkyl-N(R¹⁴)S(O)-. In embodiments, L² is - C₀₋₃alkyl-S(O)₂-. In embodiments, L² is -C₀₋₃alkyl-S(O)₂N(R¹⁴)-C₀₋₃alkyl-. In embodiments, L² is -C₀-₃alkyl-N(R¹⁴)S(O)₂-.

In some embodiments of the modified Ras mutant protein described herein, the covalently bonded compound comprises a staying group having a formula: 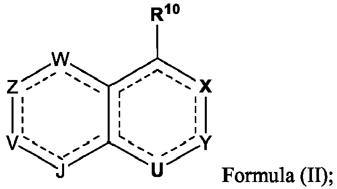 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloallcyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -C(O)-; wherein R⁶ is directly bonded to the serine corresponding to position 12 of SEQ ID No: 1; R⁸ and R^{8a} are independently selected from hydrogen, halogen, -CN, C₁₋₆alkyl, C₂₋₆alkenyl, C₂₋₆alkynyl, C₃₋₆cycloallcyl, C₂₋₉heterocycloalkyl, C₆₋₁₀aryl, C₁₋₉heteroaryl, - OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, hydrogen, halogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, - S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵,-S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20h}, and R²⁰ⁱ are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), _ OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), -OCH₂C(O)OR²² , and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and = indicates a single or double bond such that all valences are satisfied.

In an aspect is provided a complex of formula: 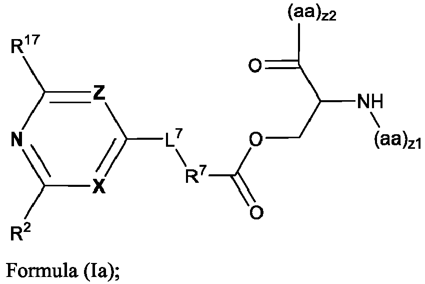 wherein each aa is independently an amino acid; z1 is an integer equal to at least 1 or (aa)z1 is hydrogen; z2 is an integer equal to at least 1 or (aa)z2 is OH; Z is N or C(R⁸); X is C(R³) or N; L⁷ is a bond, -O-, -N(R^{14c})-, -C(O)-, -S-, -S(O)₂-, -S(O)-, - or C₁₋₄alkyl, wherein the C₁₋₄alkyl is optionally substituted with one, two or three R^{20a}; R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴; and further wherein R⁷ is bonded to C(O) in Formula (Ia) through a ring N; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C_{1- 11}heteroaryl, wherein the C₃₋₁₂cycloalkyl, C₁₋₁₁heterocycloallcyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆ alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C_{6- 12}aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉ heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ is selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆ alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-,-C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C_{2- 12}heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, - CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹³, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; R² is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C_{2- 9}heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R^{14c} is independently selected from C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, - OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In some embodiments of the complex of Formula (Ia),
Z is N and X is C(R³); or Z is C(R⁸) and X is N; L⁷ is a bond, -O-, -N(R¹⁴)-, -C(O)-, -S-, -S(O)₂-, -S(O)-, or C_{1- 4}alkyl, wherein the C₁₋₄alkyl is optionally substituted with one, two or three R^{20a}; R⁷ is a 7-12 membered nitrogen containing spirocyclic heterocycloalkyl optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹²,-SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ is selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, - N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R¹⁹ is selected from a C_{3- 12}cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹¹; each R¹¹ is independently selected from halogen, oxo, -CN, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋scycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹³, -S(O)R¹³, -OC(O)R¹³, - C(O)N(R¹²)(R¹³), -C(O)C(O)N(R¹²)(R¹³), -N(R¹⁴)C(O)R¹³, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R² is halogen, -CN, C₁₋₆alkyl, C₂₋₆alkenyl, C₂₋₆alkynyl, C₃₋₆cycloalkyl, C_{2- 9}heterocycloallcyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R'^{2b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl are optionally substituted with one, two, or three R^{20f}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C_{1- 9}heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In some embodiments of the complex of Formula (Ia),
Z is N and X is C(R³); or Z is C(R⁸) and X is N; L⁷ is a bond, -O-, -N(R¹⁴)-, -C(O)-, -S-, -S(O)₂-, -S(O)-, or C_{1- 4}alkyl, wherein the C₁₋₄alkyl is optionally substituted with one, two or three R^{20a}; R⁷ is a 7-12 membered nitrogen containing fused heterocycloalkyl, 7-12 membered nitrogen containing fused aryl, or 7-12 membered nitrogen containing fused heteroaryl optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloallcyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ is selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹²,-N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, - N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R¹⁹ is selected from a C_{3- 12}cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋scycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹³, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹³, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R² is halogen, -CN, C₁₋₆alkyl, C₂₋₆alkenyl, C₂₋₆alkynyl, C₃₋₆cydoalkyl, - OR¹², -SR¹², -N(R^{12c})(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₁₋₆alkyl, C₂₋₆alkenyl, C₂₋₆alkynyl, and C₃₋₁₀cycloalkyl, are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R^{12c} is independently selected from C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³),-OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵; each R²¹ is independently selected from H, C₁₋₆alkyl, C₁₋₆haloallcyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In some embodiments of the complex of Formula (Ia),
Z is N and X is C(R³); or Z is C(R⁸) and X is N; L⁷ is a bond, -C(O)-, -S-, -S(O)₂-, -S(O)-, or C₁₋₄alkyl, wherein the C₁₋₄alkyl is optionally substituted with one, two or three R^{20a}; R⁷ is a -(4-6 membered nitrogen containing saturated heterocycloalkyl)-(4-6 membered nitrogen containing saturated heterocycloalkyl)-, wherein the -(4-6 membered nitrogen containing saturated heterocycloalkyl)-(4-6 membered nitrogen containing saturated heterocycloalkyl)- is optionally substituted with one or more R¹ and/or one or more R⁴; and further wherein the two -(4-6 membered nitrogen containing saturated heterocycloalkyl are joined by a direct bond between a ring C and ring N; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloallcyl, C₃₋₁₂cycloalkyl, - CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁸ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; each R³ is independently selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹³, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R² is halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; wherein one of R² and R¹⁷ is not hydrogen; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a complex of formula: 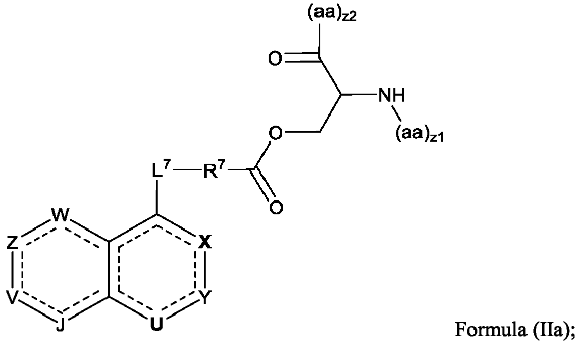 wherein each aa is independently an amino acid; z1 is an integer equal to at least 1 or (aa)z1 is hydrogen; z2 is an integer equal to at least 1 or (aa)z2 is OH; W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are optionally substituted with one or more R¹ and/or one or more R⁴; and further wherein R⁷ is bonded to C(O) in Formula (IIa) through a ring N; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, hydrogen, halogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{17b} is -L^{1b}-R¹⁹, hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, - N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C2-6alkenyl, C2-6alkynyl, C₃₋₆cycloalkyl, C2-9heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C_{1- 9}heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and -- - indicates a single or double bond such that all valences are satisfied.

In some embodiments of the complex of Formula (IIa),
W is a bond; Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵,-S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², _ C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,--N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, _ S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and -- - indicates a single or double bond such that all valences are satisfied.

In some embodiments of the complex of Formula (IIa),
W is N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), _CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl,-CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³),-N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- g}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- g}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In some embodiments of the complex of Formula (IIa),
W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O or C₁₋₄alkyl, wherein the C₁₋₄alkyl is optionally substituted with one, two or three R^{20a}; R⁷ is a 7-12 membered nitrogen containing spirocyclic heterocycloalkyl optionally substituted with one or more R¹ and/or one or more R⁴; wherein two substituents selected from R¹ and R⁴ that are bonded to the same or adjacent atoms are optionally joined to form a C_{3- 12}cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹²,-C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³),-CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³),-CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³),-N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³),-N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl,-CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- g}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³),-N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In some embodiments of the complex of Formula (IIa),
W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 7-12 membered nitrogen containing fused heterocycloalkyl, 7-12 membered nitrogen containing fused aryl, or 7-12 membered nitrogen containing fused heteroaryl optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl,-CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵,-OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, - S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f)}(R^{1g})N(R^{1c}), and C(R^{1f)}(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -{C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl; and ------ indicates a single or double bond such that all valences are satisfied.

In some embodiments of the complex of Formula (IIa),
W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); Y and U are independently selected from N, C(R²), C(R²)(R²), N(R^{2b}), S(O), S(O)₂, and C(O); L⁷ is a bond, -C(O)-, -S-, - S(O)₂-, -S(O)-, or C₁₋₄alkyl, wherein the C₁₋₄alkyl is optionally substituted with one, two or three R^{20a}; R⁷ is a -(4-6 membered nitrogen containing saturated heterocycloalkyl)-(4-6 membered nitrogen containing saturated heterocycloalkyl)-, wherein the -(4-6 membered nitrogen containing saturated heterocycloalkyl)-(4-6 membered nitrogen containing saturated heterocycloalkyl)- is optionally substituted with one or more R¹ and/or one or more R⁴; and further wherein the two -(4-6 membered nitrogen containing saturated heterocycloalkyl are joined by a direct bond between a ring C and Ring N; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C_{1- 11}heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C_{2- 6}alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C_{6- 12}aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, - S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloallcyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is independently hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -{C₂₋₆alkynyl)-R^{12b}, -(C₃₋₁₀cycloalkyl)-R^{12b}, -(C_{2- 9}heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20b}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula (Ib), or a pharmaceutically acceptable salt or solvate thereof: 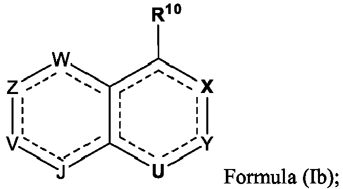 wherein W is N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C_{1- 4}alkyl is optionally substituted with one, two or three R^{20a}; R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C_{1- 11}heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C_{1- 11}heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)2R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁-₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²(R¹³), -C(O)C(O)N(R¹²)(R¹³), -N(R¹⁴)C(O)R¹⁵, -S(O)2R¹⁵, -S(O)₂N(R¹²(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloallcyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In an aspect is provided a precursor compound of Formula (Ic), or a pharmaceutically acceptable salt or solvate thereof: 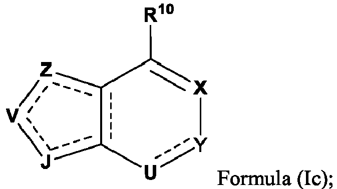 wherein Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C_{6- 12}aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵,-OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C_{2- 9}heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula (Id), or a pharmaceutically acceptable salt or solvate thereof: 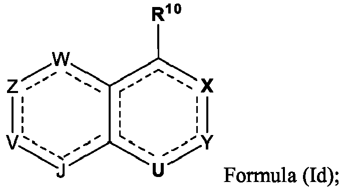 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C_{1- 4}alkyl is optionally substituted with one, two or three R^{20a}; R⁷ is a 7-12 membered nitrogen containing spirocyclic heterocycloalkyl substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ and R^{8a} are independently selected from hydrogen, halogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ;R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀ary, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In an aspect is provided a precursor compound of Formula (Ie), or a pharmaceutically acceptable salt or solvate thereof: 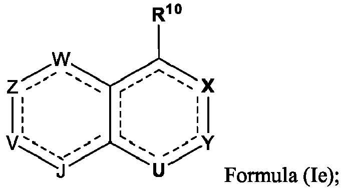 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a 7-12 membered nitrogen containing fused heterocycloalkyl, 7-12 membered nitrogen containing fused aryl, or 7-12 membered nitrogen containing fused heteroaryl substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ and R^{8a} are independently selected from hydrogen, halogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), _ C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In an aspect is provided a precursor compound of Formula (If), or a pharmaceutically acceptable salt or solvate thereof: 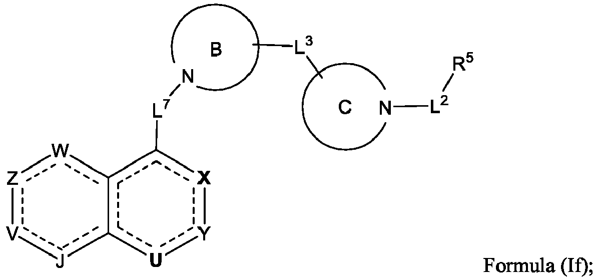 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are independently selected from C(R¹⁷), C(R¹⁷)(R^{16a}), C(R¹⁶), C(R¹⁶)(R^{16a}), N, N(R^{17b}), and N(R^{16b}); Y and U are independently selected from N, C(R²), C(R²)(R²), N(R^{2b}), S(O), S(O)₂, and C(O); L⁷ is a bond, -C(O)-, -S-, -S(O)₂-, -S(O)-, or C₁₋₄alkyl, wherein the C₁₋₄alkyl is optionally substituted with one, two or three R^{20a}; Ring B is a 3-12 membered nitrogen containing saturated heterocycloalkyl optionally substituted with one or more R¹ and/or one or more R⁴; Ring C is a 3-12 membered nitrogen containing saturated heterocycloalkyl optionally substituted with one or more R¹ and/or one or more R⁴; L³ is an N-C bond joining Ring B and Ring C; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵,-S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is independently hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, - C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C_{2- 9}heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), _ OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In an aspect is provided a precursor compound of Formula (Ih), or a pharmaceutically acceptable salt or solvate thereof: 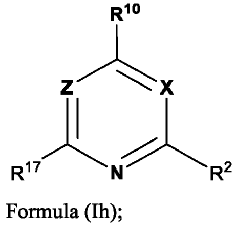 wherein Z is N and X is C(R³); or Z is C(R⁸) and X is N; R¹⁰ is -L⁷-R⁷; L⁷ is a bond, -O-, -N(R^{14c})-, -C(O)-, -S-, - S(O)₂-, -S(O)-, -N(R^{14c})-C₁₋₃alkyl-, or C₁₋₄alkyl, wherein the C₁₋₃alkyl and C₁₋₄alkyl are optionally substituted with one, two or three R^{20a}; R⁷ is a 6-12 membered nitrogen containing heterocycloalkyl or 5-12 membered nitrogen containing heteroaryl, wherein the 6-12 membered nitrogen containing heterocycloalkyl or 5-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, - N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R² is halogen, -CN, C₂₋₆alkyl, C₂₋₆alkenyl, C₂₋₆alkynyl, C₃₋₆cycloalkyl, - OR^{12c}, -SR¹², -N(R^{12c})(R^{12c}), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₂₋₆alkyl)-R^{12b}, -(C₂₋₆alkenyl)-R^{12b}, -(C₂₋₆alkynyl)-R^{12b}, or -(C₃₋₁₀cycloalkyl)-R^{12b}, wherein said C₂₋₆alkyl, C₂₋₆alkenyl, C₂₋₆alkynyl, and C₃₋₁₀cycloalkyl, are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R^{12c} is independently selected from C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R^{14c} is independently selected from C_{1- 6}alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), _ C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C_{1- 6}haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²² is independently selected from H, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl; each R²³ is independently selected from Hand C_{1- 6}alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula (Ii), or a pharmaceutically acceptable salt or solvate thereof: 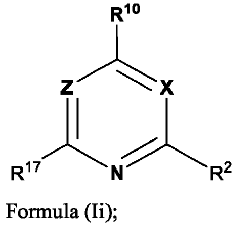 wherein Z is N and X is C(R³); or Z is C(R⁸) and X is N; R¹⁰ is -L⁷-R⁷; L⁷ is a bond, -O-, -N(R¹⁴)-, -C(O)-, -S-, - S(O)₂-, -S(O)-, or C₁₋₄alkyl, wherein the C₁₋₄alkyl is optionally substituted with one, two or three R^{20a}; R⁷ is a 7-12 membered nitrogen containing spirocyclic heterocycloalkyl substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; L¹ is selected from a bond, C₁-C₆alkyl, C₂-C₆alkenyl, C₂-C₆alkynyl, -O-, -N(R¹⁴)-, - C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C_{2- 12}heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, - CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²² is independently selected from H, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl; each R²³ is independently selected from Hand C_{1- 6}alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula (Ij), or a pharmaceutically acceptable salt or solvate thereof: 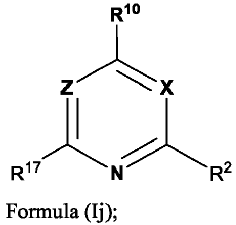 wherein Z is N and X is C(R³); or Z is C(R⁸) and X is N; R¹⁰ is -L⁷-R⁷; L⁷ is a bond, -O-, -N(R¹⁴)-, -C(O)-, -S-, - S(O)₂-, -S(O)-, or C₁₋₄alkyl, wherein the C₁₋₄alkyl is optionally substituted with one, two or three R^{20a}; R⁷ is a 7-12 membered nitrogen containing fused heterocycloalkyl, fused 7-12 membered nitrogen aryl, or fused 7-12 membered nitrogen heteroaryl substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ is selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(H)(R¹²), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; L¹ is selected from a bond, C₁-C₆alkyl, C₂-C₆alkenyl, C₂-C₆alkynyl, -O-, -N(R¹⁴)-, - C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C_{2- 12}heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, - CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵,-N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R³ is selected from hydrogen, halogen, -CN, Ci-salkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)aR¹⁵, -C(O)R¹³, -S(O)R¹³, -OC(O)R¹³, -C(O)N(R¹²)(R¹³), -C(O)C(O)N(R¹²)(R¹³), -N(R¹⁴)C(O)R¹³, - S(O)₂R¹⁵, -S(O)aN(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein Ci-salkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; R² is halogen, -CN, C₁₋₆alkyl, C₂₋₆alkenyl, C₂₋₆alkynyl, C₃₋₆cycloalkyl, -OR¹², -SR¹², -N(R^{12c})(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³),-CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R¹²b, -(C₂₋₆alkynyl)-R¹²b, or -(C₃₋₁₀cycloalkyl)-R^{12b}, wherein said C₁₋₆alkyl, C₂₋₆alkenyl, C₂₋₆alkynyl, and C₃₋₁₀cycloalkyl, are optionally substituted with one, two, or three R^{20d}; R^{12b} is selected from hydrogen, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R^{12c} is independently selected from C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- g}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, _ N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³),-C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, _ N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- g}heteroaryl; each R²² is independently selected from H, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl; each R²³ is independently selected from H and C_{1- 6}alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula (Ik), or a pharmaceutically acceptable salt or solvate thereof:

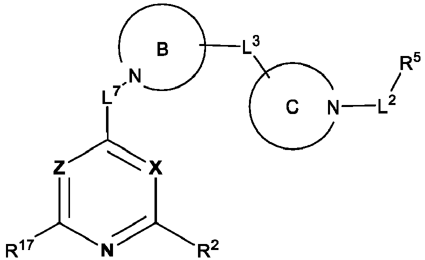

Formula (Ik); wherein Z is N and X is C(R³); or Z is C(R⁸) and X is N; L⁷ is a bond, -C(O)-, -S-, -S(O)₂-, -S(O)-, or C₁₋₄alkyl, wherein the C₁₋₄alkyl is optionally substituted with one, two or three R^{20a}; Ring B is a 4-6 membered nitrogen containing saturated heterocycloalkyl optionally substituted with one or more R¹ and/or one or more R⁴; Ring C is a 4-6 membered nitrogen containing saturated heterocycloalkyl optionally substituted with one or more R¹ and/or one or more R⁴; L³ is an N-C bond joining Ring B and Ring C; wherein two substituents selected from R¹ and R⁴ that are bonded to the same or adjacent atoms are optionally joined to form a C₃₋₁₂cycloalkyl, C_{1- 11}heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C_{1- 11}heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, _ C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁹ is selected from a C₃₋₁₂cycloalkyl, C₂₋₁₁heterocycloalkyl, C₆₋₁₂aryl, and C_{2- 12}heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, - CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ; R³ is selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; wherein one of R² and R¹⁷ is not hydrogen; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), _ N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C_{2- 9}heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), _ N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In some embodiments of the modified K-Ras G12S protein described herein, the covalently bonded compound comprises a staying group having a formula: 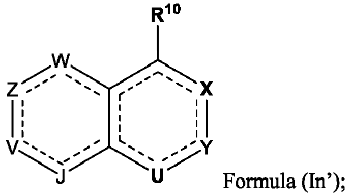 wherein W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a monocyclic 3-12 membered nitrogen containing heterocycloalkyl or monocyclic 5-12 membered nitrogen containing heteroaryl, wherein the monocyclic 3-12 membered nitrogen containing heterocycloalkyl or monocyclic 5-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁶ -C(O)-; wherein R⁶ is directly bonded to the K-Ras G12S serine corresponding to position 12 of SEQ ID No: 1; R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³),-CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵,-C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{17b} is -L^{1b}-R¹⁹, hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-,-N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰¹ R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; each R^{20a}, R^{20b}, R^{20c}, R^{20d}, R^{20e}, R^{20f}, R^{20g}, R^{20h}, and R²⁰ⁱ are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³),-OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵,-S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- g}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In some embodiments of the complex of Formula (Ia),
Z is N and X is C(R³) or Z is C(R⁸) and X is N; L⁷ is a bond, -O-, -N(R^{14c})-, -C(O)-, -S-, -S(O)₂-, -S(O)-, or C_{1- 4}alkyl, wherein the C₁₋₄alkyl is optionally substituted with one, two or three R^{20a}; R⁷ is a monocyclic 6-12 membered nitrogen containing heterocycloalkyl or monocyclic 5-12 membered nitrogen containing heteroaryl, wherein the monocyclic 6-12 membered nitrogen containing heterocycloalkyl or monocyclic 5-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C_{1- 11}heterocycloalkyl, C₆₋₁₂aryl, or C₁₋₁₁heteroaryl, wherein the C₃₋₁₂cycloalkyl, C₁₋₁₁heterocycloallcyl, C₆₋₁₂aryl, or C_{1- 11}heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C₁₋₁₁heterocycloallcyl, -CH₂-C₁₋₁₁heterocycloalkyl, C₆₋₁₂aryl, -CH₂-C₆₋₁₂aryl, -CH₂-C₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ is selected from hydrogen, halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵,-OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, - S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R²⁰ⁱ R¹⁹ is selected from a C_{3- 12}cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³),-N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; each R³ is independently selected from hydrogen, halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹³, -N(R¹⁴)S(O)₂R¹³, -C(O)R¹³, -S(O)R¹³, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=OX=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R² is halogen, -CN, C₂₋₆alkyl, C₂₋₆alkenyl, C₂₋₆alkynyl, C₃₋ecycloalkyl, C₆₋₁₀aryl, -OR^{12c}, -SR¹², -N(R^{12c})(R^{12c}), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹³, -S(O)₂N(R¹²)(R¹³)-, S(=O)(=NH)N(R¹²)(R¹³), -CH₂C(O)N(R¹²)(R¹³), - CH₂N(R¹⁴)C(O)R¹³, -CH₂S(O)₂R¹³, -CH₂S(O)₂N(R¹²)(R¹³), -(C₂-₆alkyl)-R^{12b}, -(C₂₋₆alkenyl)-R^{12b}, -(C_{2- 6}alkynyl)-R^{12b}, -(C₃₋₁₀cycloalkyl)-R^{12b}, or -(C₆₋₁₀aryl)-R^{12b}, wherein said C₂₋₆alkyl, C₂₋₆alkenyl, C₂₋₆alkynyl, C_{3- 10}cycloalkyl, and C₆₋₁₀aryl, are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R^{12c} is independently selected from C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R^{14c} is independently selected from C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, - OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²²,-C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and -- - indicates a single or double bond such that all valences are satisfied.

In an aspect is provided a complex of formula: 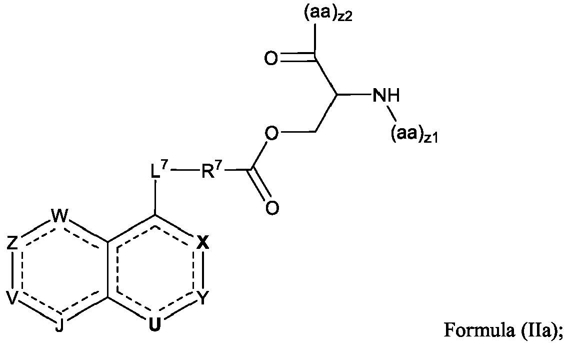 wherein each aa is independently an amino acid; zl is an integer equal to at least 1 or (aa)zl is hydrogen; z2 is an integer equal to at least 1 or (aa)z2 is OH; W is a bond, N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a monocyclic 3-12 membered nitrogen containing heterocycloalkyl or monocyclic 5-12 membered nitrogen containing heteroaryl, wherein the monocyclic 3-12 membered nitrogen containing heterocycloalkyl or monocyclic 5-12 membered nitrogen containing heteroaryl are optionally substituted with one or more R¹ and/or one or more R⁴; and further wherein R⁷ is bonded to C(O) in Formula (Ia) through a ring N; wherein two substituents selected from R¹ and R⁴ that are bonded to the same or adjacent atoms are optionally joined to form a C₃₋₁₂cycloalkyl, C₁₋₁₁heterocycloalkyl, C_{6- 12}aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C_{2- 6}alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C_{1- 11}heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹, hydrogen, halogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹²,-N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵,-C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵,-S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and-CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{17b} is -L^{1b}-R¹⁹, hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², _ C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-,-S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f}(R^{1g})O, C(R^{1f})(R^{1g})N(R^{1c}), and C(R^{1f}(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloallcyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; R¹⁸ and R^{18a} are independently selected from hydrogen, halogen, - CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C_{1- 9}heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), ^{_}OCH₂C(O)OR²², and -OC(O)R²⁵; wherein C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²²,-C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In some embodiments of the complex of Formula (IIa),
W is a bond; Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a monocyclic 6-12 membered nitrogen containing heterocycloalkyl or monocyclic 6-12 membered nitrogen containing heteroaryl, wherein the monocyclic 6-12 membered nitrogen containing heterocycloalkyl or monocyclic 6-12 membered nitrogen containing heteroaryl are optionally substituted with one or more R¹ and/or one or more R⁴; and further wherein R⁷ is bonded to C(O) in Formula (Ia) through a ring N; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁ heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-,-S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵,-C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³),-C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In some embodiments of the complex of Formula (IIa),
W is N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a monocyclic 6-12 membered nitrogen containing heterocycloalkyl or monocyclic 6-12 membered nitrogen containing heteroaryl, wherein the monocyclic 6-12 membered nitrogen containing heterocycloalkyl or monocyclic 6-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C_{3- 12}cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², - C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, - S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C_{1- 6}alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ R¹⁹ is selected from a C₃₋₁₂cycloalkyl, C₂₋₁₁heterocycloalkyl, C_{6- 12}aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20h}, and R²⁰ⁱ is independently selected from halogen, -CN, C₁₋₆alkyl, C₂₋₆alkenyl, C₂₋₆alkynyl, C₃₋₆cycloallcyl, -CH₂-C₃₋₆cycloalkyl, C₂₋₉heterocycloalkyl, -CH₂-C₂₋₉heterocycloalkyl, C₆₋₁₀ary1, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²³ is independently selected from Hand C₁₋₆alkyl; each R²⁴ is independently selected from Hand C₁₋₆alkyl; each R²⁵ is independently selected from C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl; and - - - - - - indicates a single or double bond such that all valences are satisfied.

In an aspect is provided a precursor compound of Formula (Ib), or a pharmaceutically acceptable salt or solvate thereof: 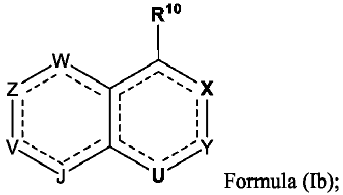 wherein W is N, C(R¹⁸), N(R^{18b}), C(R¹⁸)(R^{18a}), C(O), S(O), or S(O)₂; Z is N, C(R⁸), N(R^{8b}), C(R⁸)(R^{8a}), C(O), S(O), or S(O)₂; wherein W and Z are not both selected from C(O), S(O), and S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is N, C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C_{1- 4}alkyl is optionally substituted with one, two or three R^{20a}; R⁷ is a monocyclic 6-12 membered nitrogen containing heterocycloalkyl or monocyclic 6-12 membered nitrogen containing heteroaryl, wherein the monocyclic 6-12 membered nitrogen containing heterocycloalkyl or monocyclic 6-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ and R^{8a} are independently selected from hydrogen, halogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, - CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C₂₋₆alkynyl, C₃₋₁₀cycloalkyl, - CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C_{1- 6}haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f}; R¹⁸ and R^{18a} are independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h}; R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R^{20a}, R^{20b}, R^{20c}, R^{20d}, R^{20e}, R^{20f}, R^{20g}, R^{20b}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, - SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and - - - - - - indicates a single or double bond such that all valences are satisfied.

In an aspect is provided a precursor compound of Formula (Ic), or a pharmaceutically acceptable salt or solvate thereof: 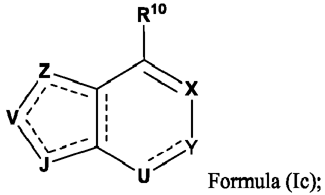 wherein Z is N, C(R⁸), N(R^{8b}), C(R⁸)(R^{8a}), C(O), S(O), or S(O)₂; V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b}; Y is C(R²), C(R²)(R^{2c}), N(R^{2b}), S(O), S(O)₂, or C(O); U is N, C(R^{2c}), C(R^{2c})(R^{2c}), N(R^{2b}), S(O), S(O)₂, or C(O); R¹⁰ is -L⁷-R⁷; L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; R⁷ is a monocyclic 6-12 membered nitrogen containing heterocycloalkyl or monocyclic 6-12 membered nitrogen containing heteroaryl, wherein the monocyclic 6-12 membered nitrogen containing heterocycloalkyl or monocyclic 6-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C_{3- 12}cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloallcyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ and R^{8a} are independently selected from hydrogen, halogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R^{8b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), _ CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}; R¹⁷ is -L¹-R¹⁹; R^{17b} is -L^{1b}-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R^{16b} is selected from hydrogen, -CN, C_{1- 6}alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}; R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d}; R^{2b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), _ CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}; R^{12b} is selected from hydrogen, C₁₋₆alkyl, C₂₋₆alkenyl, C_{2- 6}alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; X is C(R³), C(R³)(R³), N(R³), or N; each R³ is independently selected from hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C_{2- 9}heterocycloalkyl ring optionally substituted with one, two, or three R^{20e}; each R¹⁴ is independently selected from hydrogen, C₁₋₆alkyl, and C₁₋₆haloalkyl; each R¹⁵ is independently selected C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C_{2- 9}heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; each R²² is independently selected from H, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²³ is independently selected from H and C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and -- - indicates a single or double bond such that all valences are satisfied.

In an aspect is provided a precursor compound of Formula (Ih), or a pharmaceutically acceptable salt or solvate thereof: 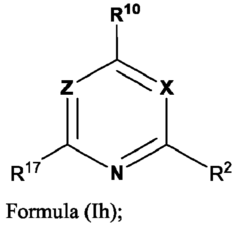 wherein Z is N and X is C(R³) or Z is C(R⁸) and X is N; R¹⁰ is -L⁷-R⁷; L⁷ is a bond, -O-, -N(R^{14c})-, -C(O)-, -S-, - S(O)₂-, -S(O)-, -N(R^{14c})-C₁₋₃alkyl-, or C₁₋₄alkyl, wherein the C₁₋₃alkyl and C₁₋₄alkyl are optionally substituted with one, two or three R^{20a}; R⁷ is a monocyclic 6-12 membered nitrogen containing heterocycloalkyl or monocyclic 5-12 membered nitrogen containing heteroaryl, wherein the monocyclic 6-12 membered nitrogen containing heterocycloalkyl or monocyclic 5-12 membered nitrogen containing heteroaryl are substituted with one R⁶ directly bonded to a ring N and optionally substituted with one or more R¹ and/or one or more R⁴; wherein two substituents selected from R¹ and R⁴ that are bonded to the same or adjacent atoms are optionally joined to form a C_{3- 12}cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}; each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C_{1- 11}heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a}; each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵; each L² is -C(O)-; each R⁵ is selected from: a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and a 5-6 membered heteroaryl comprising one, two, or three ring nitrogen atoms that is optionally substituted with one, two or three R^{20k}; wherein R⁵ is bonded through an R⁵ ring nitrogen to L²; R⁸ is selected from hydrogen, halogen, -CN, C₁₋₆alkyl, C₂₋₆alkenyl, C₂₋₆alkynyl, C₃₋₆cycloallcyl, C₂₋₉heterocycloalkyl, C₆₋₁₀aryl, C₁₋₉heteroaryl, - OR¹², -SR¹², -N(H)(R¹²), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl are optionally substituted with one, two, or three R^{20c}; R¹⁷ is -L¹-R¹⁹; L¹ is selected from a bond, C₁-C₆alkyl, C₂-C₆alkenyl, C₂-C₆alkynyl, -O-, -N(R¹⁴)-, -C(O)-, -N(R¹⁴)C(O)-, -C(O)N(R¹⁴)-, -S-, -S(O)₂-, - S(O)-, -S(O)₂N(R¹⁴)-, -S(O)N(R¹⁴)-, -N(R¹⁴)S(O)-, -N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g}); R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R²⁰ⁱ R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ; each R¹ⁱ is independently selected from halogen, oxo, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b}; R² is halogen, -CN, C₂₋₆alkyl, C₂₋₆alkenyl, C₂₋₆alkynyl, C₃₋₆cycloalkyl, C₆₋₁₀aryl, -OR^{12c}, -SR¹², -N(R^{12c})(R^{12c}), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₂₋₆alkyl)-R^{12b}, -(C_{2- 6}alkenyl)-R^{12b}, -(C₂₋₆alkynyl)-R^{12b}, -(C₃₋₁₀cycloalkyl)-R^{12b}, or -( C₆₋₁₀aryl)-R^{12b}, wherein said C₂₋₆alkyl, C₂₋₆alkenyl, C2-6alkynyl, C₃₋₁₀cycloalkyl, and C₆₋₁₀aryl, are optionally substituted with one, two, or three R^{20d}; R^{12b} is selected from hydrogen, C₁₋₆alkyl, C₂₋₆alkenyl, C₂₋₆alkynyl, C₃₋₁₀cycloallcyl, -CH₂-C₃₋₁₀cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C_{2- 6}alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C_{6- 10}aryl, -CH₂-C₆₋₁₀aryl, -CH₂-C₁₋₉heteroaryl, and C₁₋₉heteroaryl are optionally substituted with one, two, or three R^{20d}; each R^{12c} is independently selected from C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹² is independently selected from hydrogen, C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d}; each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e}; each R¹⁴ is independently selected from hydrogen, C₁₋₆alkyl, and C₁₋₆haloalkyl; each R^{14c} is independently selected from C₁₋₆alkyl, and C₁₋₆haloalkyl; each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f}; each R^{20a}, R^{20b}, R^{20c}, R^{20d}, R^{20e}, R^{20f}, R^{20g}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, - OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵; each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C_{1- 9}heteroaryl; each R²³ is independently selected from Hand C₁₋₆alkyl; each R²⁴ is independently selected from H and C₁₋₆alkyl; each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and = indicates a single or double bond such that all valences are satisfied.

In embodiments, the leaving group is a 6 membered partially unsaturated heterocycloalkyl or a 6 membered heteroaryl, each optionally substituted with one, two or three R^{20k}, wherein the partially unsaturated heterocycloalkyl or heteroaryl comprises two or three ring nitrogen atoms.

In embodiments of a compound or precursor compound (e.g., compound of formula (Ib), (Id), (Ie), (Ib-1), (Ib-2). (Ib-3), (Ib-4), (Id-1), (Id-3), (Ie-1), (Ie-3), (I-1), (I-2), (I-la), (I-lb), (I-ld), (I-le), (I-If), ((I-lg), (I-lh), (I-li), (I-lk), (I-lm), (I-ln), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In')), R⁶ is -L²-R⁵; each L² is independently selected from a -C(O)- and -C(O)N(R¹⁴)-; each R⁵ is a 5 or 6 membered partially unsaturated heterocycloalkyl or a heteroaryl optionally substituted with one, two or three R^{20k}, wherein the partially unsaturated heterocycloalkyl or heteroaryl comprises one, two, or three ring nitrogen atoms; and when L² is -C(O)-, R⁵ is a 5 membered partially unsaturated heterocycloalkyl or heteroaryl, optionally substituted with one, two or three R^{20k}, bonded to L² through a ring nitrogen and when L² is -C(O)N(R¹⁴)- wherein the N of L² is directly bonded to R⁵, R⁵ is a 5 or 6 membered partially unsaturated heterocycloalkyl or heteroaryl, optionally substituted with one, two or three R^{20k}, bonded to L² through a ring carbon.

In some embodiments of a complex or compound (e.g., precursor compound) described herein having a monocyclic ring including Z and X, Z is N.

In some embodiments of a complex or compound (e.g., precursor compound) described herein having a monocyclic ring including Z and X, Z is C(R⁸).

In some embodiments of a complex or compound (e.g., precursor compound) described herein having a monocyclic ring including Z and X, X is N.

In some embodiments of a complex or compound (e.g., precursor compound) described herein having a monocyclic ring including Z and X, X is C(R³).

In some embodiments of a complex or compound (e.g., precursor compound) described herein W is a bond. In some embodiments of a complex or compound (e.g., precursor compound) described herein W is a N. In some embodiments of a complex or compound (e.g., precursor compound) described herein W is a C(R¹⁸). In some embodiments of a complex or compound (e.g., precursor compound) described herein W is a N(R^{18b}). In some embodiments of a complex or compound (e.g., precursor compound) described herein W is a C(R¹⁸)(R^{18a}). In some embodiments of a complex or compound (e.g., precursor compound) described herein W is a C(O). In some embodiments of a complex or compound (e.g., precursor compound) described herein W is a S(O). In some embodiments of a complex or compound (e.g., precursor compound) described herein W is a S(O)₂.

In some embodiments of a complex or compound (e.g., precursor compound) described herein Z is N. In some embodiments of a complex or compound (e.g., precursor compound) described herein Z is C(R⁸). In some embodiments of a complex or compound (e.g., precursor compound) described herein Z is N(R^{8b}). In some embodiments of a complex or compound (e.g., precursor compound) described herein Z is C(R⁸)(R^{8a}). In some embodiments of a complex or compound (e.g., precursor compound) described herein Z is C(O). In some embodiments of a complex or compound (e.g., precursor compound) described herein Z is S(O). In some embodiments of a complex or compound (e.g., precursor compound) described herein Z is S(O)₂.

In some embodiments of a complex or compound (e.g., precursor compound) described herein V is C(R¹⁷). In some embodiments of a complex or compound (e.g., precursor compound) described herein V is C(R¹⁷)(R^{16a}). In some embodiments of a complex or compound (e.g., precursor compound) described herein V is C(R¹⁶). In some embodiments of a complex or compound (e.g., precursor compound) described herein V is C(R¹⁶)(R^{16a}). In some embodiments of a complex or compound (e.g., precursor compound) described herein V is N. In some embodiments of a complex or compound (e.g., precursor compound) described herein V is N(R^{17b}). In some embodiments of a complex or compound (e.g., precursor compound) described herein V is and N(R^{16b}).

In some embodiments of a complex or compound (e.g., precursor compound) described herein J is C(R¹⁷). In some embodiments of a complex or compound (e.g., precursor compound) described herein J is C(R¹⁷)(R^{16a}). In some embodiments of a complex or compound (e.g., precursor compound) described herein J is C(R¹⁶). In some embodiments of a complex or compound (e.g., precursor compound) described herein J is C(R¹⁶)(R^{16a}). In some embodiments of a complex or compound (e.g., precursor compound) described herein J is N. In some embodiments of a complex or compound (e.g., precursor compound) described herein J is N(R^{17b}). In some embodiments of a complex or compound (e.g., precursor compound) described herein J is and N(R^{16b}).

In some embodiments of a complex or compound (e.g., precursor compound) described herein Y is N. In some embodiments of a complex or compound (e.g., precursor compound) described herein Y is C(R²). In some embodiments of a complex or compound (e.g., precursor compound) described herein Y is C(R²)(R^{2c}). In some embodiments of a complex or compound (e.g., precursor compound) described herein Y is N(R^{2b}). In some embodiments of a complex or compound (e.g., precursor compound) described herein Y is S(O). In some embodiments of a complex or compound (e.g., precursor compound) described herein Y is S(O)₂. In some embodiments of a complex or compound (e.g., precursor compound) described herein Y is C(O).

In some embodiments of a complex or compound (e.g., precursor compound) described herein U is N. In some embodiments of a complex or compound (e.g., precursor compound) described herein U is C(R^{2c}). In some embodiments of a complex or compound (e.g., precursor compound) described herein U is C(R^{2c})(R^{2c}). In some embodiments of a complex or compound (e.g., precursor compound) described herein U is N(R^{2b}). In some embodiments of a complex or compound (e.g., precursor compound) described herein U is S(O). In some embodiments of a complex or compound (e.g., precursor compound) described herein U is S(O)₂. In some embodiments of a complex or compound (e.g., precursor compound) described herein U is C(O).

In some embodiments of a complex or compound (e.g., precursor compound) described herein X is C(R³). In some embodiments of a complex or compound (e.g., precursor compound) described herein X is C(R³)(R³). In some embodiments of a complex or compound (e.g., precursor compound) described herein X is N(R³). In some embodiments of a complex or compound (e.g., precursor compound) described herein X is N.

The embodiments of L⁷ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-la), (I-lb), (I-ld), (I-le), (I-lf), ((I-1g), (I-lh), (I-li), (I-lk), (I-lm), (I-ln), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. The embodiments of R⁷ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-la), (I-lb), (I-ld), (I-le), (I-lf), ((I-1g), (I-lh), (I-li), (I-lk), (I-lm), (I-ln), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following.

In embodiments, L⁷ is a bond, -O-, -N(R^{14c})-, -C(O)-, -S-, -S(O)₂-, -S(O)-, -N(R^{14c})-C₁₋₃alkyl-, or C₁₋₄alkyl, wherein the C₁₋₃alkyl and C₁₋₄alkyl are optionally substituted with one, two or three R^{20a}; and each R^{14c} is independently selected from C₁₋₆alkyl, and C₁₋₆haloalkyl. In embodiments, L⁷ is a bond, -O-, -N(R¹⁴)-, -C(O)-, -S-, - S(O)₂-, -S(O)-, -N(R^{14c})-C₁₋₃alkyl-, or C₁₋₄alkyl, wherein the C₁₋₃alkyl and C₁₋₄alkyl are optionally substituted with one, two or three R^{20a}; and each R^{14c} is independently selected from C₁₋₆alkyl, and C₁₋₆haloalkyl.

In some embodiments, R⁷ is a 7-12 membered nitrogen containing spirocyclic heterocycloalkyl substituted with one R⁶ directly bonded to a ring N and optionally substituted with one or more R¹ and/or one or more R⁴. In some embodiments, R⁷ is a 7-12 membered nitrogen containing spirocyclic heterocycloalkyl substituted with one R⁶ directly bonded to a ring N.

In some embodiments, R⁷ is a 7 membered nitrogen containing spirocyclic heterocycloalkyl substituted with one R⁶ directly bonded to a ring N and optionally substituted with one or more R¹ and/or one or more R⁴. In some embodiments, R⁷ is a 7 membered nitrogen containing spirocyclic heterocycloalkyl substituted with one R⁶ directly bonded to a ring N. In some embodiments, R⁷ is a 8 membered nitrogen containing spirocyclic heterocycloalkyl substituted with one R⁶ directly bonded to a ring N and optionally substituted with one or more R¹ and/or one or more R⁴. In some embodiments, R⁷ is a 8 membered nitrogen containing spirocyclic heterocycloalkyl substituted with one R⁶ directly bonded to a ring N. In some embodiments, R⁷ is a 9 membered nitrogen containing spirocyclic heterocycloalkyl substituted with one R⁶ directly bonded to a ring N and optionally substituted with one or more R¹ and/or one or more R⁴. In some embodiments, R⁷ is a 9 membered nitrogen containing spirocyclic heterocycloalkyl substituted with one R⁶ directly bonded to a ring N. In some embodiments, R⁷ is a 10 membered nitrogen containing spirocyclic heterocycloalkyl substituted with one R⁶ directly bonded to a ring N and optionally substituted with one or more R¹ and/or one or more R⁴. In some embodiments, R⁷ is a 10 membered nitrogen containing spirocyclic heterocycloalkyl substituted with one R⁶ directly bonded to a ring N. In some embodiments, R⁷ is a 11 membered nitrogen containing spirocyclic heterocycloalkyl substituted with one R⁶ directly bonded to a ring N and optionally substituted with one or more R¹ and/or one or more R⁴. In some embodiments, R⁷ is a 11 membered nitrogen containing spirocyclic heterocycloalkyl substituted with one R⁶ directly bonded to a ring N. In some embodiments, R⁷ is a 12 membered nitrogen containing spirocyclic heterocycloalkyl substituted with one R⁶ directly bonded to a ring N and optionally substituted with one or more R¹ and/or one or more R⁴. In some embodiments, R⁷ is a 12 membered nitrogen containing spirocyclic heterocycloalkyl substituted with one R⁶ directly bonded to a ring N.

In some embodiments, R⁷ is a 7-12 membered nitrogen containing fused heterocycloalkyl substituted with one R⁶ directly bonded to a ring N and optionally substituted with one or more R¹ and/or one or more R⁴. In some embodiments, R⁷ is a 7-12 membered nitrogen containing fused aryl substituted with one R⁶ directly bonded to a ring N and optionally substituted with one or more R¹ and/or one or more R⁴. In some embodiments, R⁷ is a 7-12 membered nitrogen containing fused heteroaryl heterocycloalkyl substituted with one R⁶ directly bonded to a ring N and optionally substituted with one or more R¹ and/or one or more R⁴. In some embodiments, R⁷ is a 7-12 membered nitrogen containing fused heterocycloalkyl substituted with one R⁶ directly bonded to a ring N. In some embodiments, R⁷ is a 7-12 membered nitrogen containing fused aryl substituted with one R⁶ directly bonded to a ring N. In some embodiments, R⁷ is a 7-12 membered nitrogen containing fused heteroaryl substituted with one R⁶ directly bonded to a ring N. In some embodiments, R⁷ is a 7-12 membered nitrogen containing fused heterocycloalkyl substituted with one R⁶ directly bonded to a ring N.

In some embodiments, R⁷ is a 7 membered nitrogen containing fused heterocycloalkyl substituted with one R⁶ directly bonded to a ring N and optionally substituted with one or more R¹ and/or one or more R⁴. In some embodiments, R⁷ is a 7 membered nitrogen containing fused aryl substituted with one R⁶ directly bonded to a ring N and optionally substituted with one or more R¹ and/or one or more R⁴. In some embodiments, R⁷ is a 7 membered nitrogen containing fused heteroaryl heterocycloalkyl substituted with one R⁶ directly bonded to a ring N and optionally substituted with one or more R¹ and/or one or more R⁴. In some embodiments, R⁷ is a 7 membered nitrogen containing fused heterocycloalkyl substituted with one R⁶ directly bonded to a ring N. In some embodiments, R⁷ is a 7 membered nitrogen containing fused aryl substituted with one R⁶ directly bonded to a ring N. In some embodiments, R⁷ is a 7 membered nitrogen containing fused heteroaryl substituted with one R⁶ directly bonded to a ring N. In some embodiments, R⁷ is a 7 membered nitrogen containing fused heterocycloalkyl substituted with one R⁶ directly bonded to a ring N.

In some embodiments, R⁷ is a 8 membered nitrogen containing fused heterocycloalkyl substituted with one R⁶ directly bonded to a ring N and optionally substituted with one or more R¹ and/or one or more R⁴. In some embodiments, R⁷ is a 8 membered nitrogen containing fused aryl substituted with one R⁶ directly bonded to a ring N and optionally substituted with one or more R¹ and/or one or more R⁴. In some embodiments, R⁷ is a 8 membered nitrogen containing fused heteroaryl heterocycloalkyl substituted with one R⁶ directly bonded to a ring N and optionally substituted with one or more R¹ and/or one or more R⁴. In some embodiments, R⁷ is a 8 membered nitrogen containing fused heterocycloalkyl substituted with one R⁶ directly bonded to a ring N. In some embodiments, R⁷ is a 8 membered nitrogen containing fused aryl substituted with one R⁶ directly bonded to a ring N. In some embodiments, R⁷ is a 8 membered nitrogen containing fused heteroaryl substituted with one R⁶ directly bonded to a ring N. In some embodiments, R⁷ is a 8 membered nitrogen containing fused heterocycloalkyl substituted with one R⁶ directly bonded to a ring N.

In some embodiments, R⁷ is a 9 membered nitrogen containing fused heterocycloalkyl substituted with one R⁶ directly bonded to a ring N and optionally substituted with one or more R¹ and/or one or more R⁴. In some embodiments, R⁷ is a 9 membered nitrogen containing fused aryl substituted with one R⁶ directly bonded to a ring N and optionally substituted with one or more R¹ and/or one or more R⁴. In some embodiments, R⁷ is a 9 membered nitrogen containing fused heteroaryl heterocycloalkyl substituted with one R⁶ directly bonded to a ring N and optionally substituted with one or more R¹ and/or one or more R⁴. In some embodiments, R⁷ is a 9 membered nitrogen containing fused heterocycloalkyl substituted with one R⁶ directly bonded to a ring N. In some embodiments, R⁷ is a 9 membered nitrogen containing fused aryl substituted with one R⁶ directly bonded to a ring N. In some embodiments, R⁷ is a 9 membered nitrogen containing fused heteroaryl substituted with one R⁶ directly bonded to a ring N. In some embodiments, R⁷ is a 9 membered nitrogen containing fused heterocycloalkyl substituted with one R⁶ directly bonded to a ring N.

In some embodiments, R⁷ is a 10 membered nitrogen containing fused heterocycloalkyl substituted with one R⁶ directly bonded to a ring N and optionally substituted with one or more R¹ and/or one or more R⁴. In some embodiments, R⁷ is a 10 membered nitrogen containing fused aryl substituted with one R⁶ directly bonded to a ring N and optionally substituted with one or more R¹ and/or one or more R⁴. In some embodiments, R⁷ is a 10 membered nitrogen containing fused heteroaryl heterocycloalkyl substituted with one R⁶ directly bonded to a ring N and optionally substituted with one or more R¹ and/or one or more R⁴. In some embodiments, R⁷ is a 10 membered nitrogen containing fused heterocycloalkyl substituted with one R⁶ directly bonded to a ring N. In some embodiments, R⁷ is a 10 membered nitrogen containing fused aryl substituted with one R⁶ directly bonded to a ring N. In some embodiments, R⁷ is a 10 membered nitrogen containing fused heteroaryl substituted with one R⁶ directly bonded to a ring N. In some embodiments, R⁷ is a 10 membered nitrogen containing fused heterocycloalkyl substituted with one R⁶ directly bonded to a ring N.

In some embodiments, R⁷ is a 11 membered nitrogen containing fused heterocycloalkyl substituted with one R⁶ directly bonded to a ring N and optionally substituted with one or more R¹ and/or one or more R⁴. In some embodiments, R⁷ is a 11 membered nitrogen containing fused aryl substituted with one R⁶ directly bonded to a ring N and optionally substituted with one or more R¹ and/or one or more R⁴. In some embodiments, R⁷ is a 11 membered nitrogen containing fused heteroaryl heterocycloalkyl substituted with one R⁶ directly bonded to a ring N and optionally substituted with one or more R¹ and/or one or more R⁴. In some embodiments, R⁷ is a 11 membered nitrogen containing fused heterocycloalkyl substituted with one R⁶ directly bonded to a ring N. In some embodiments, R⁷ is a 11 membered nitrogen containing fused aryl substituted with one R⁶ directly bonded to a ring N. In some embodiments, R⁷ is a 11 membered nitrogen containing fused heteroaryl substituted with one R⁶ directly bonded to a ring N. In some embodiments, R⁷ is a 11 membered nitrogen containing fused heterocycloalkyl substituted with one R⁶ directly bonded to a ring N.

In some embodiments, R⁷ is a 12 membered nitrogen containing fused heterocycloalkyl substituted with one R⁶ directly bonded to a ring N and optionally substituted with one or more R¹ and/or one or more R⁴. In some embodiments, R⁷ is a 12 membered nitrogen containing fused aryl substituted with one R⁶ directly bonded to a ring N and optionally substituted with one or more R¹ and/or one or more R⁴. In some embodiments, R⁷ is a 12 membered nitrogen containing fused heteroaryl heterocycloalkyl substituted with one R⁶ directly bonded to a ring N and optionally substituted with one or more R¹ and/or one or more R⁴. In some embodiments, R⁷ is a 12 membered nitrogen containing fused heterocycloalkyl substituted with one R⁶ directly bonded to a ring N. In some embodiments, R⁷ is a 12 membered nitrogen containing fused aryl substituted with one R⁶ directly bonded to a ring N. In some embodiments, R⁷ is a 12 membered nitrogen containing fused heteroaryl substituted with one R⁶ directly bonded to a ring N. In some embodiments, R⁷ is a 12 membered nitrogen containing fused heterocycloalkyl substituted with one R⁶ directly bonded to a ring N.

In embodiments, R⁷ is a 3-12 membered nitrogen containing heterocycloalkyl or 5 to 12 membered nitrogen containing heteroaryl substituted with one R⁶ directly bonded to a ring N and optionally substituted with one or more R¹ and/or one or more R⁴.

The embodiments of R¹⁷ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-la), (I-lb), (I-ld), (I-le), (I-lf), ((I-1g), (I-lh), (I-li), (I-lk), (I-lm), (I-ln), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. The embodiments of R¹⁹ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-la), (I-lb), (I-ld), (I-le), (I-lf), ((I-1g), (I-lh), (I-li), (I-lk), (I-lm), (I-ln), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following.

In some embodiments, R² is independently halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.

In some embodiments, R¹⁷ is -L¹-R¹⁹, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), _ N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g}.

In some embodiments, R¹⁷ is -L¹-R¹⁹.

In embodiments, R¹⁹ is a bicyclic C₂₋₁₂heteroaryl or a fused ring C₂₋₁₂heteroaryl, wherein the bicyclic C_{2- 12}heteroaryl and fused ring C₂₋₁₂heteroaryl are optionally substituted with one, two, or three R²⁰ⁱ.

In some embodiments, R¹⁹ is selected from: 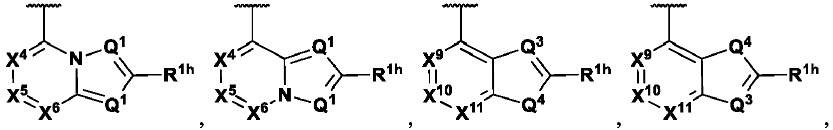 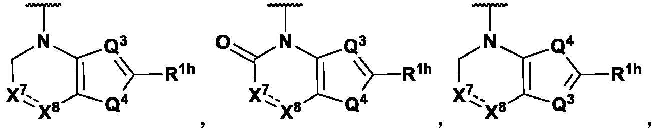 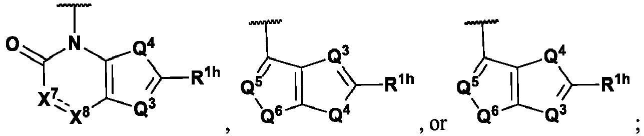
Q¹, Q³, and Q⁵ are independently N or C(R^{1d});
Q⁴ and Q⁶ are independently O, S, C(R^{1a})(R^{1b}), or N(R^{1c});
X⁴, X⁵, X⁶, X⁹, X¹⁰, and X¹¹ are independently selected from C(R^{1a}) or N;
X⁷ and X⁸ are independently selected from C(R^{1a}), C(R^{1a})(R^{1b}), N, or N(R^{1c});
each R^{1a}, R^{1b}, R^{1d}, R^{1f}, R^{1g}, and R^{1h} are independently selected from hydrogen, halogen, -CN, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³),-N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)2R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1a} and R^{1b} bonded to the same carbon are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; or two R^{1a} bonded to adjacent atoms are joined to form a 4-7 membered heterocycloalkyl ring, a phenyl ring, a 5-6 membered heteroaryl ring, or a 4-7 membered cycloalkyl ring, wherein the 4-7 membered heterocycloalkyl ring, phenyl ring, 5-6 membered heteroaryl ring, or 4-7 membered cycloalkyl ring are optionally substituted with one, two, or three R²⁰ⁱ; or R^{1h} and one of R^{1a}, R^{1b}, R^{1c}, and R^{1d} bonded to adjacent atoms are joined to form a 4-7 membered heterocycloalkyl ring, a phenyl ring, a 5-6 membered heteroaryl ring, or a 4-7 membered cycloalkyl ring, wherein the 4-7 membered heterocycloalkyl ring, phenyl ring, 5-6 membered heteroaryl ring, or 4-7 membered cycloalkyl ring are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; and
each R^{1c} is independently selected from hydrogen, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ.

In embodiments, R² is independently selected from hydrogen, halogen, -CN, C₁₋₆alkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -N=(R¹⁵),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵,-OC(O)R¹⁵, -C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³),-S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.

The embodiments of R² as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following.

In embodiments, R² is selected from

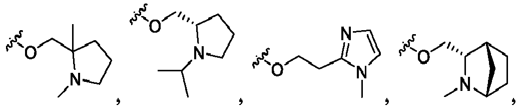

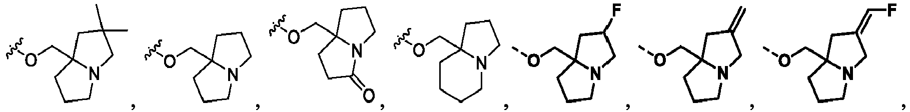

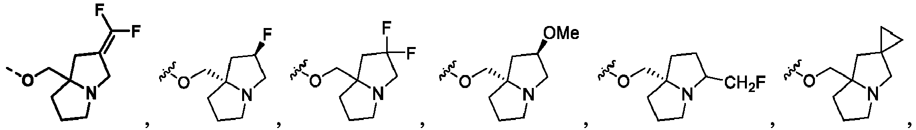

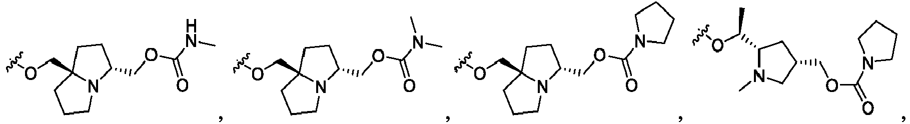

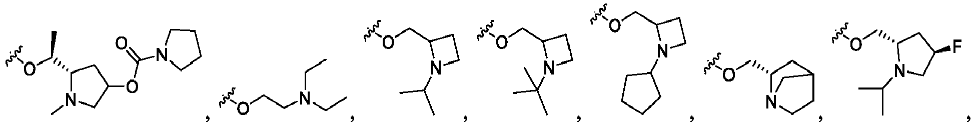

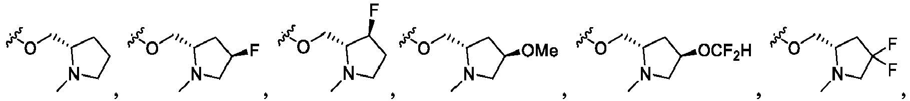

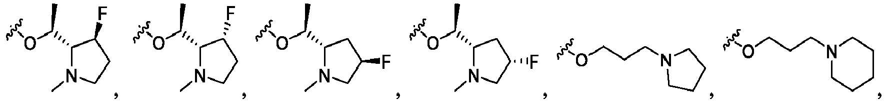

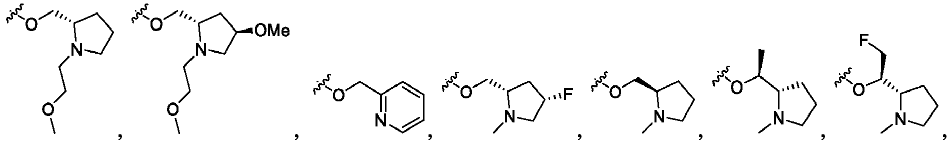

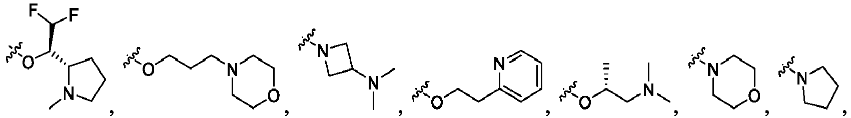

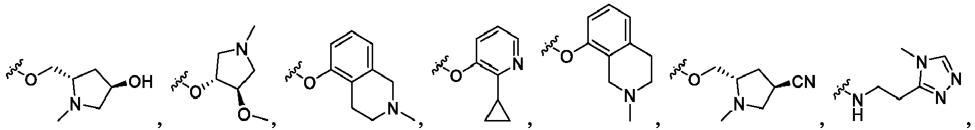

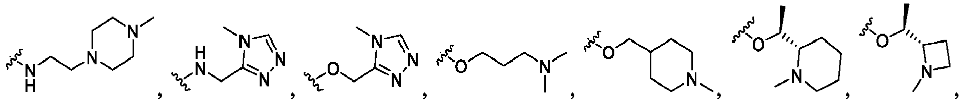

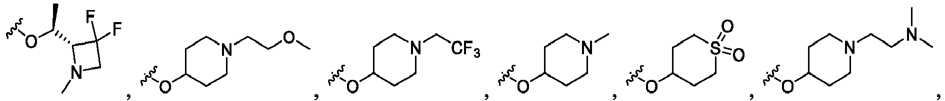

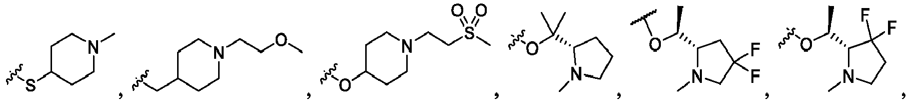

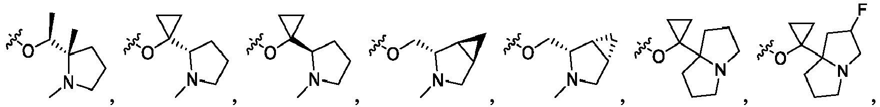

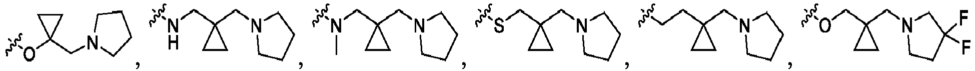

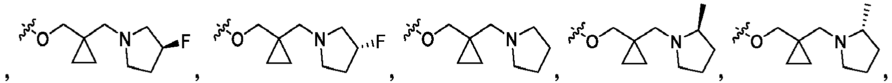

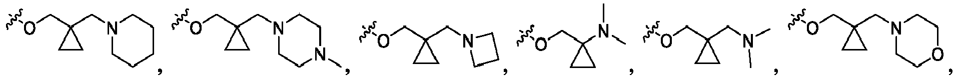

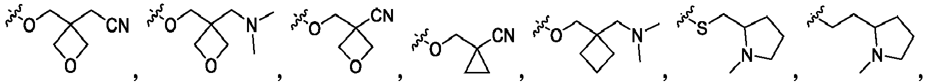

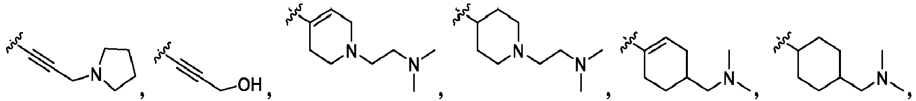

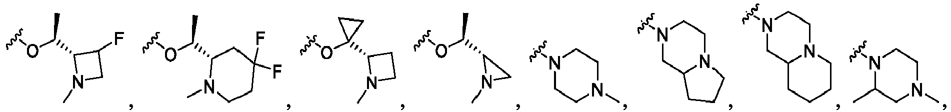

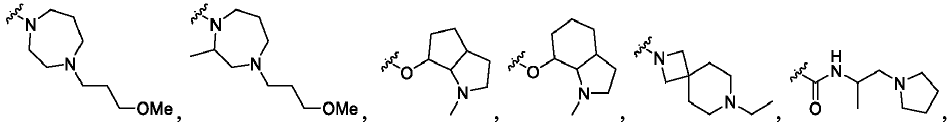

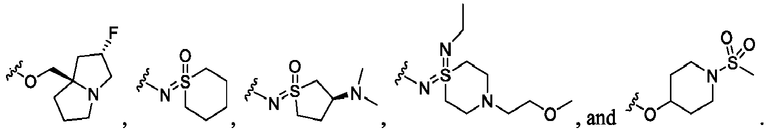

In some embodiments R² is selected from

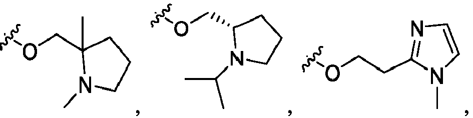

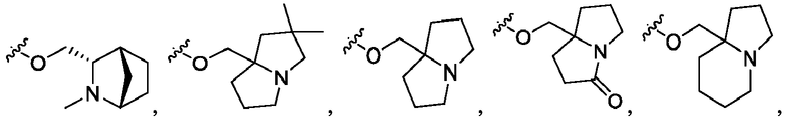

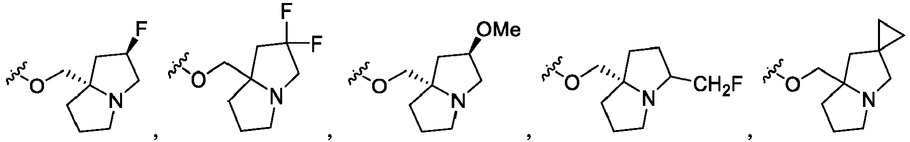

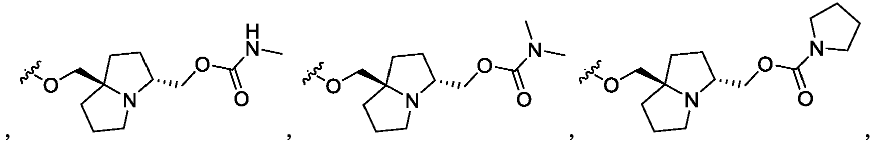

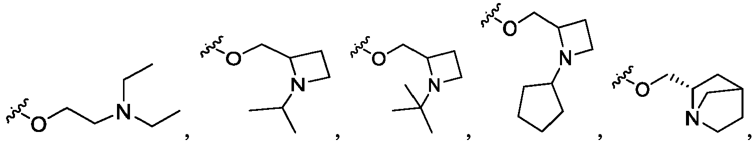

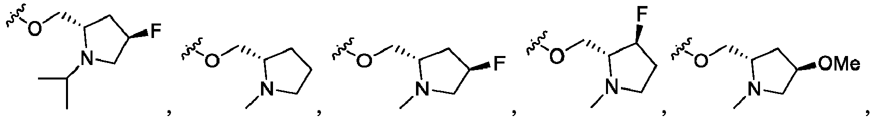

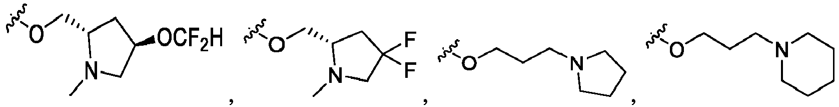

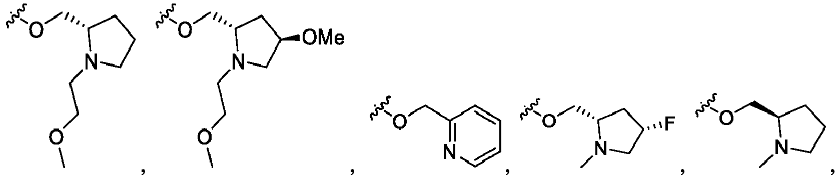

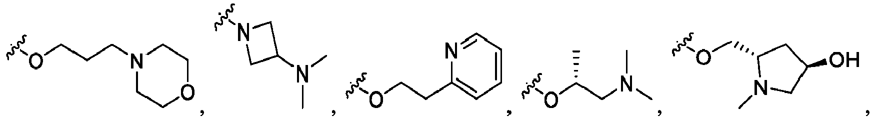

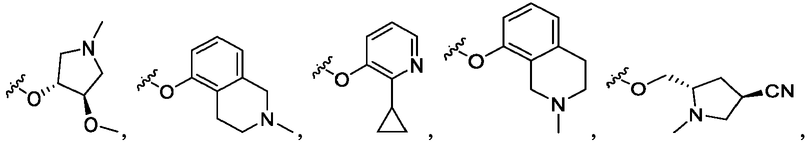

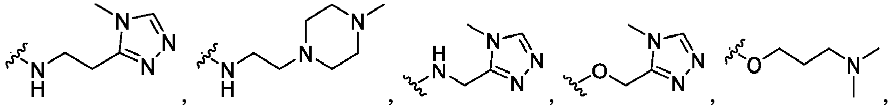

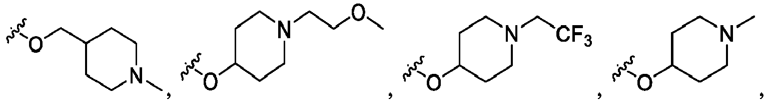

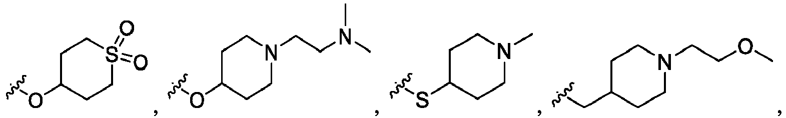

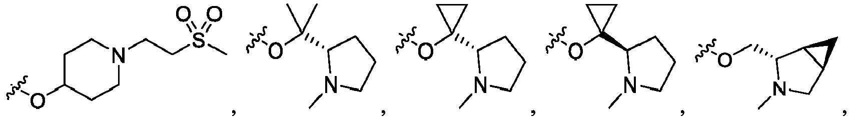

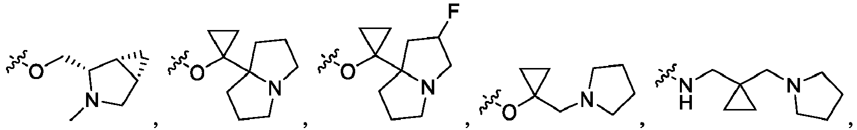

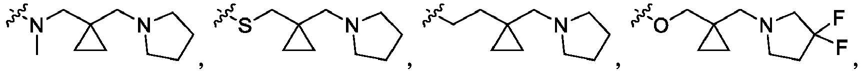

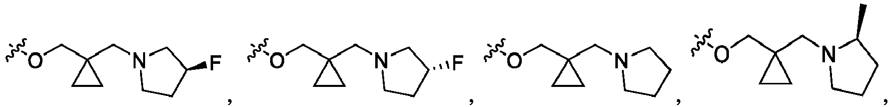

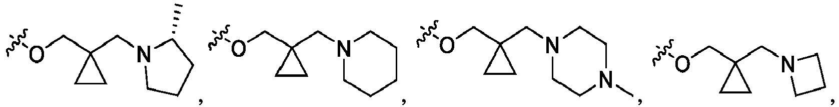

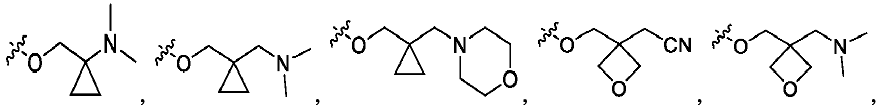

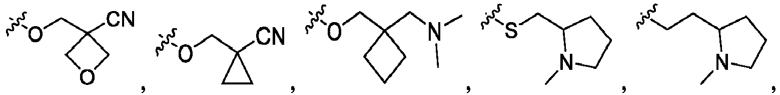

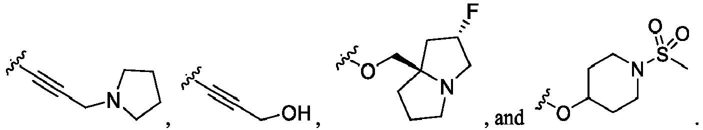

In embodiments, R² is independently selected from hydrogen, halogen, -CN, C₁₋₆alkyl, C₂₋₆alkenyl, C_{2- 6}alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -N=(R¹⁵),-C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵,-OC(O)R¹⁵, -C(O)N(R¹²)(R¹³), -C(O)C(O)N(R¹²)(R¹³), -N(R¹⁴)C(O)R¹⁵, -S(O)2R¹⁵, -S(O)₂N(R¹²)(R¹³),-S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d}.

In embodiments, R² is selected from

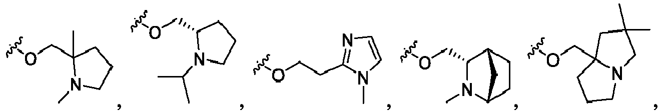

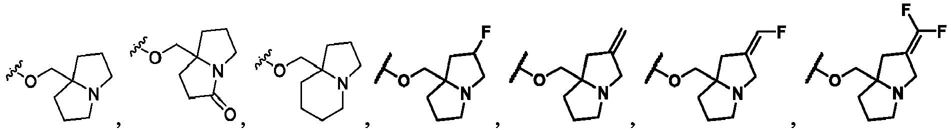

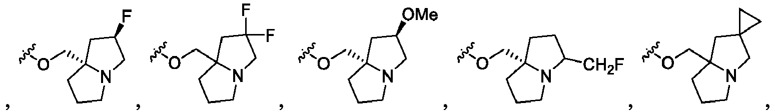

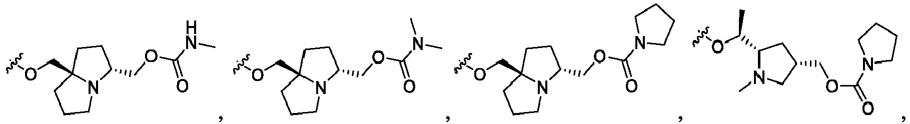

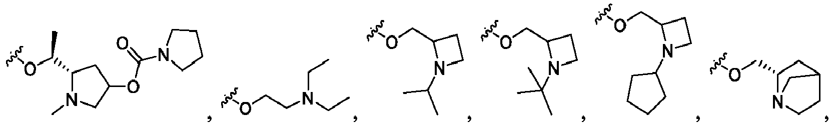

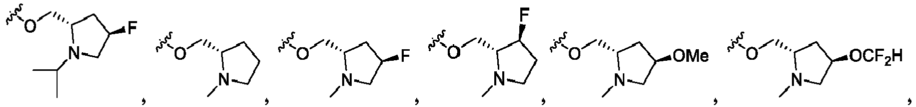

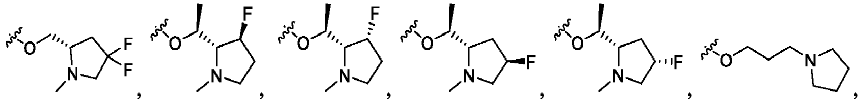

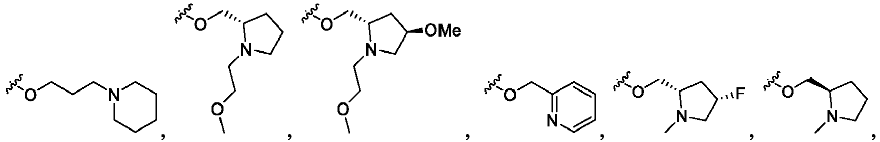

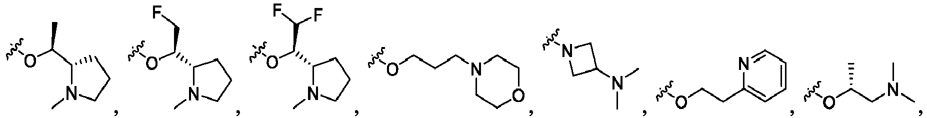

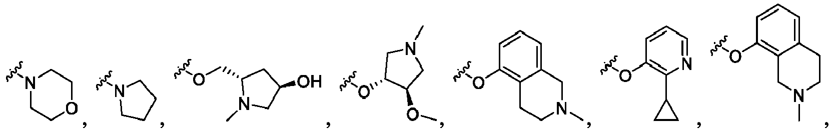

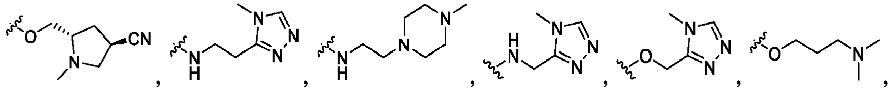

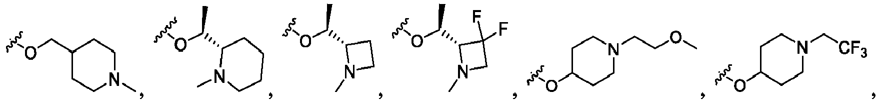

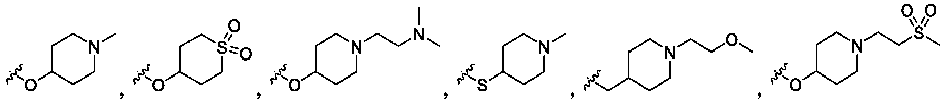

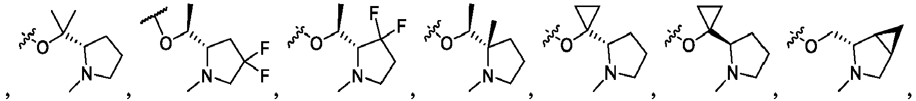

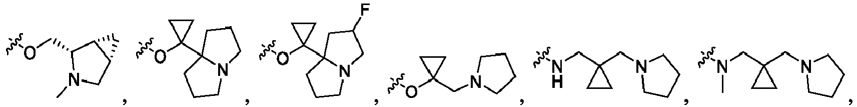

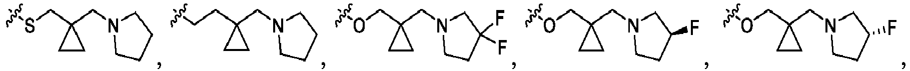

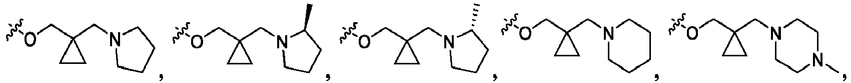

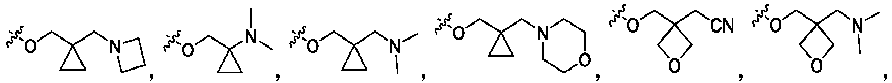

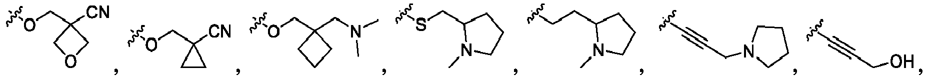

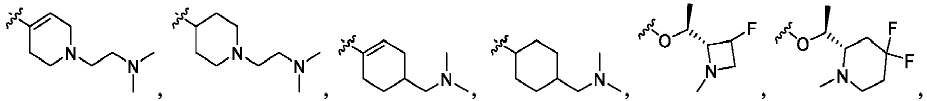

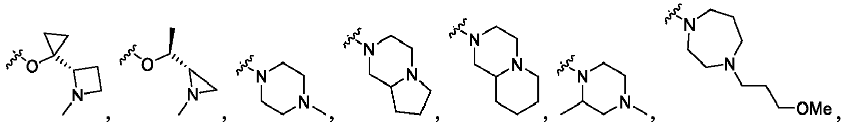

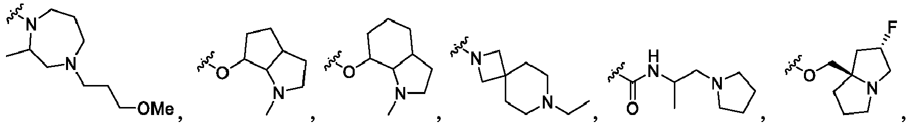

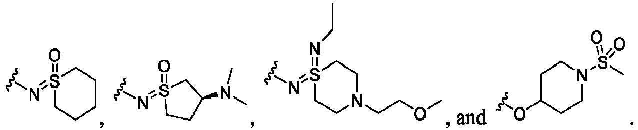

The embodiments of R¹ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. The embodiments of R⁴ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following.

In some embodiments of a complex or compound (e.g., precursor compound) described herein, or a pharmaceutically acceptable salt or solvate thereof, the complex or compound (e.g., precursor compound) is substituted with one or two independently selected R¹.

In some embodiments of a complex or compound (e.g., precursor compound) described herein, or a pharmaceutically acceptable salt or solvate thereof, R¹ is independently C₁₋₆alkyl optionally substituted with one, two, or three R^{20a}.

In some embodiments of a complex or compound (e.g., precursor compound) described herein, or a pharmaceutically acceptable salt or solvate thereof, R¹ is independently unsubstituted C₁₋₆alkyl.

In some embodiments of a complex or compound (e.g., precursor compound) described herein, or a pharmaceutically acceptable salt or solvate thereof, R¹ is independently -C₁₋₆alkyl-CN.

In some embodiments of a complex or compound (e.g., precursor compound) described herein, or a pharmaceutically acceptable salt or solvate thereof, the complex or compound (e.g., precursor compound) is not substituted with R¹.

In some embodiments of a complex or compound (e.g., precursor compound) described herein, or a pharmaceutically acceptable salt or solvate thereof, the complex or compound (e.g., precursor compound) is substituted with one or two independently selected R⁴.

In some embodiments of a complex or compound (e.g., precursor compound) described herein, or a pharmaceutically acceptable salt or solvate thereof, R⁴ is independently halogen or C₁₋₆alkyl optionally substituted with one, two, or three R^{20a}.

In some embodiments of a complex or compound (e.g., precursor compound) described herein, or a pharmaceutically acceptable salt or solvate thereof, R⁴ is independently unsubstituted C₁₋₆alkyl, C₁₋₆alkyl-CN, or halogen.

In some embodiments of a complex or compound (e.g., precursor compound) described herein, or a pharmaceutically acceptable salt or solvate thereof, the complex or compound (e.g., precursor compound) is not substituted with R⁴.

In additional embodiments of the subject complex or compound (e.g., precursor compound), R⁴ is independently selected from hydrogen, halogen, oxo, -CN, C₁₋₆alkyl, C₂₋₆alkenyl, C₂₋₆alkynyl, -OR¹², -N(R¹²)(R¹³), - C(O)OR¹², -OC(O)N(R¹²)(R¹³), and -C(O)R¹⁵, wherein C₁₋₆alkyl, C₂₋₆alkenyl, and C₂₋₆alkynyl, are optionally substituted with one, two, or three R^{20a}. In embodiments of the subject complex or compound (e.g., precursor compound), R⁴ is independently hydrogen. In further embodiments of the subject complex or compound (e.g., precursor compound), R⁴ is independently halogen. In some embodiments of the subject complex or compound (e.g., precursor compound), R⁴ is independently oxo. In some embodiments of the subject complex or compound (e.g., precursor compound), R⁴ is independently -CN. In additional embodiments of the subject complex or compound (e.g., precursor compound), R⁴ is independently C₁₋₆alkyl. In embodiments of the subject complex or compound (e.g., precursor compound), R⁴ is independently C₂₋₆alkenyl. In some embodiments of the subject complex or compound (e.g., precursor compound), R⁴ is independently C₂₋₆alkynyl. In further embodiments of the subject complex or compound (e.g., precursor compound), R⁴ is independently -OR¹². In select embodiments of the subject complex or compound (e.g., precursor compound), R⁴ is independently -N(R¹²)(R¹³). In additional embodiments of the subject complex or compound (e.g., precursor compound), R⁴ is independently -C(O)OR¹². In embodiments of the subject complex or compound (e.g., precursor compound), R⁴ is independently - OC(O)N(R¹²)(R¹³). In some embodiments of the subject complex or compound (e.g., precursor compound), R⁴ is independently -C(O)R¹⁵. In select embodiments of the subject complex or compound (e.g., precursor compound), R⁴ is independently -NH₂. In further embodiments of the subject complex or compound (e.g., precursor compound), R⁴ is independently -C(O)OH. In additional embodiments of the subject complex or compound (e.g., precursor compound), R⁴ is independently -OC(O)NH₂. In embodiments of the subject complex or compound (e.g., precursor compound), R⁴ is independently -C(O)CH₃.

In embodiments of the subject complex or compound (e.g., precursor compound), R¹ is independently C_{1- 6}alkyl optionally substituted with one, two, or three R^{20a}.

In embodiments of the subject complex or compound (e.g., precursor compound), R¹ is independently C_{1- 6}alkyl optionally substituted with one, two, or three R^{20a}. In embodiments of the subject complex or compound (e.g., precursor compound), R¹ is independently methyl optionally substituted with one, two, or three R^{20a}. In embodiments of the subject complex or compound (e.g., precursor compound), R¹ is independently ethyl optionally substituted with one, two, or three R^{20a}. In embodiments of the subject complex or compound (e.g., precursor compound), R¹ is independently propyl optionally substituted with one, two, or three R^{20a}. In embodiments of the subject complex or compound (e.g., precursor compound), R¹ is independently butyl optionally substituted with one, two, or three R^{20a}. In embodiments of the subject complex or compound (e.g., precursor compound), R¹ is independently pentyl optionally substituted with one, two, or three R^{20a}. In embodiments of the subject complex or compound (e.g., precursor compound), R¹ is independently hexyl optionally substituted with one, two, or three R^{20a}.

In embodiments of the subject complex or compound (e.g., precursor compound), R¹ is independently C_{2- 6}alkenyl optionally substituted with one, two, or three R^{20a}.
In embodiments of the subject complex or compound (e.g., precursor compound), R¹ is independently C₂₋₆alkenyl optionally substituted with one, two, or three R^{20a}. In embodiments of the subject complex or compound (e.g., precursor compound), R¹ is independently ethenyl optionally substituted with one, two, or three R^{20a}. In embodiments of the subject complex or compound (e.g., precursor compound), R¹ is independently propenyl optionally substituted with one, two, or three R^{20a}. In embodiments of the subject complex or compound (e.g., precursor compound), R¹ is independently butenyl optionally substituted with one, two, or three R^{20a}. In embodiments of the subject complex or compound (e.g., precursor compound), R¹ is independently pentenyl optionally substituted with one, two, or three R^{20a}. In embodiments of the subject complex or compound (e.g., precursor compound), R¹ is independently hexenyl optionally substituted with one, two, or three R^{20a}.
In embodiments of the subject complex or compound (e.g., precursor compound), R¹ is independently C₂₋₆alkynyl optionally substituted with one, two, or three R^{20a}.
In embodiments of the subject complex or compound (e.g., precursor compound), R¹ is independently C₂₋₆alkynyl optionally substituted with one, two, or three R^{20a}. In embodiments of the subject complex or compound (e.g., precursor compound), R¹ is independently ethynyl optionally substituted with one, two, or three R^{20a}. In embodiments of the subject complex or compound (e.g., precursor compound), R¹ is independently propynyl optionally substituted with one, two, or three R^{20a}. In embodiments of the subject complex or compound (e.g., precursor compound), R¹ is independently butynyl optionally substituted with one, two, or three R^{20a}. In embodiments of the subject complex or compound (e.g., precursor compound), R¹ is independently pentynyl optionally substituted with one, two, or three R^{20a}. In embodiments of the subject complex or compound (e.g., precursor compound), R¹ is independently hexynyl optionally substituted with one, two, or three R^{20a}.
In embodiments of the subject complex or compound (e.g., precursor compound), R¹ is independently C₁₋₆haloalkyl optionally substituted with one, two, or three R^{20a}.
In embodiments of the subject complex or compound (e.g., precursor compound), R¹ is independently C₁₋₆haloalkyl optionally substituted with one, two, or three R^{20a}. In embodiments of the subject complex or compound (e.g., precursor compound), R¹ is independently C₁haloalkyl optionally substituted with one, two, or three R^{20a}. In embodiments of the subject complex or compound (e.g., precursor compound), R¹ is independently C₂haloalkyl optionally substituted with one, two, or three R^{20a}. In embodiments of the subject complex or compound (e.g., precursor compound), R¹ is independently C₃haloalkyl optionally substituted with one, two, or three R^{20a}. In embodiments of the subject complex or compound (e.g., precursor compound), R¹ is independently C₄haloalkyl optionally substituted with one, two, or three R^{20a}. In embodiments of the subject complex or compound (e.g., precursor compound), R¹ is independently C₅haloalkyl optionally substituted with one, two, or three R^{20a}. In embodiments of the subject complex or compound (e.g., precursor compound), R¹ is independently C₆haloalkyl optionally substituted with one, two, or three R^{20a}.
In embodiments of the subject complex or compound (e.g., precursor compound), R¹ is independently C_{3- 12}cycloalkyl optionally substituted with one, two, or three R^{20a}.
In embodiments of the subject complex or compound (e.g., precursor compound), R¹ is independently C₃cycloalkyl optionally substituted with one, two, or three R^{20a}. In embodiments of the subject complex or compound (e.g., precursor compound), R¹ is independently C₄cycloalkyl optionally substituted with one, two, or three R^{20a}. In embodiments of the subject complex or compound (e.g., precursor compound), R¹ is independently C₅cycloalkyl optionally substituted with one, two, or three R^{20a}. In embodiments of the subject complex or compound (e.g., precursor compound), R¹ is independently C₆cycloalkyl optionally substituted with one, two, or three R^{20a}. In embodiments of the subject complex or compound (e.g., precursor compound), R¹ is independently C₇cycloalkyl optionally substituted with one, two, or three R^{20a}. In embodiments of the subject complex or compound (e.g., precursor compound), R¹ is independently C₈cycloalkyl optionally substituted with one, two, or three R^{20a}. In embodiments of the subject complex or compound (e.g., precursor compound), R¹ is independently C₉cycloalkyl optionally substituted with one, two, or three R^{20a}. In embodiments of the subject complex or compound (e.g., precursor compound), R¹ is independently C₁₀cycloalkyl optionally substituted with one, two, or three R^{20a}. In embodiments of the subject complex or compound (e.g., precursor compound), R¹ is independently C₁₁cycloalkyl optionally substituted with one, two, or three R^{20a}. In embodiments of the subject complex or compound (e.g., precursor compound), R¹ is independently C₁₂cycloalkyl optionally substituted with one, two, or three R^{20a}.
In embodiments of the subject complex or compound (e.g., precursor compound), R¹ is independently -CH₂-C_{3- 12}cycloalkyl optionally substituted with one, two, or three R^{20a}.

In embodiments of the subject complex or compound (e.g., precursor compound), R¹ is independently - CH₂-C₃cycloalkyl optionally substituted with one, two, or three R^{20a}. In embodiments of the subject complex or compound (e.g., precursor compound), R¹ is independently -CH₂-C₄cycloalkyl optionally substituted with one, two, or three R^{20a}. In embodiments of the subject complex or compound (e.g., precursor compound), R¹ is independently -CH₂-C₅cycloalkyl optionally substituted with one, two, or three R^{20a}. In embodiments of the subject complex or compound (e.g., precursor compound), R¹ is independently -CH₂-C₆cycloalkyl optionally substituted with one, two, or three R^{20a}. In embodiments of the subject complex or compound (e.g., precursor compound), R¹ is independently -CH₂-C₇cycloalkyl optionally substituted with one, two, or three R^{20a}. In embodiments of the subject complex or compound (e.g., precursor compound), R¹ is independently -CH₂-C₈cycloalkyl optionally substituted with one, two, or three R^{20a}. In embodiments of the subject complex or compound (e.g., precursor compound), R¹ is independently -CH₂-C₉cycloalkyl optionally substituted with one, two, or three R^{20a}. In embodiments of the subject complex or compound (e.g., precursor compound), R¹ is independently -CH₂-C₁₀cycloalkyl optionally substituted with one, two, or three R^{20a}. In embodiments of the subject complex or compound (e.g., precursor compound), R¹ is independently -CH₂-C₁₁cycloalkyl optionally substituted with one, two, or three R^{20a}. In embodiments of the subject complex or compound (e.g., precursor compound), R¹ is independently -CH₂-C₁₂cycloalkyl optionally substituted with one, two, or three R^{20a}.

In embodiments of the subject complex or compound (e.g., precursor compound), R¹ is independently C_{1- 11}heterocycloalkyl optionally substituted with one, two, or three R^{20a}.

In embodiments of the subject complex or compound (e.g., precursor compound), R¹ is independently C₁heterocycloalkyl optionally substituted with one, two, or three R^{20a}. In embodiments of the subject complex or compound (e.g., precursor compound), R¹ is independently C₂heterocycloalkyl optionally substituted with one, two, or three R^{20a}. In embodiments of the subject complex or compound (e.g., precursor compound), R¹ is independently C₃heterocycloalkyl optionally substituted with one, two, or three R^{20a}. In embodiments of the subject complex or compound (e.g., precursor compound), R¹ is independently C₄heterocycloalkyl optionally substituted with one, two, or three R^{20a}. In embodiments of the subject complex or compound (e.g., precursor compound), R¹ is independently C₅heterocycloalkyl optionally substituted with one, two, or three R^{20a}. In embodiments of the subject complex or compound (e.g., precursor compound), R¹ is independently C₆heterocycloalkyl optionally substituted with one, two, or three R^{20a}. In embodiments of the subject complex or compound (e.g., precursor compound), R¹ is independently C₇heterocycloalkyl optionally substituted with one, two, or three R^{20a}. In embodiments of the subject complex or compound (e.g., precursor compound), R¹ is independently C₈heterocycloalkyl optionally substituted with one, two, or three R^{20a}. In embodiments of the subject complex or compound (e.g., precursor compound), R¹ is independently C₉heterocycloalkyl optionally substituted with one, two, or three R^{20a}. In embodiments of the subject complex or compound (e.g., precursor compound), R¹ is independently C₁₀heterocycloalkyl optionally substituted with one, two, or three R^{20a}. In embodiments of the subject complex or compound (e.g., precursor compound), R¹ is independently C₁₁heterocycloalkyl optionally substituted with one, two, or three R^{20a}.

In embodiments of the subject complex or compound (e.g., precursor compound), R¹ is independently - CH₂-C₁₋₁₁heterocycloalkyl optionally substituted with one, two, or three R^{20a}.

In embodiments of the subject complex or compound (e.g., precursor compound), R¹ is independently - CH₂-C₁heterocycloalkyl optionally substituted with one, two, or three R^{20a}. In embodiments of the subject complex or compound (e.g., precursor compound), R¹ is independently -CH₂-C₂heterocycloalkyl optionally substituted with one, two, or three R^{20a}. In embodiments of the subject complex or compound (e.g., precursor compound), R¹ is independently -CH₂-C₃heterocycloalkyl optionally substituted with one, two, or three R^{20a}. In embodiments of the subject complex or compound (e.g., precursor compound), R¹ is independently -CH₂-C₄heterocycloalkyl optionally substituted with one, two, or three R^{20a}. In embodiments of the subject complex or compound (e.g., precursor compound), R¹ is independently -CH₂-C₅heterocycloalkyl optionally substituted with one, two, or three R^{20a}. In embodiments of the subject complex or compound (e.g., precursor compound), R¹ is independently -CH₂-C₆heterocycloalkyl optionally substituted with one, two, or three R^{20a}. In embodiments of the subject complex or compound (e.g., precursor compound), R¹ is independently -CH₂-C₇heterocycloalkyl optionally substituted with one, two, or three R^{20a}. In embodiments of the subject complex or compound (e.g., precursor compound), R¹ is independently -CH₂-C₈heterocycloalkyl optionally substituted with one, two, or three R^{20a}. In embodiments of the subject complex or compound (e.g., precursor compound), R¹ is independently -CH₂-C₉heterocycloalkyl optionally substituted with one, two, or three R^{20a}. In embodiments of the subject complex or compound (e.g., precursor compound), R¹ is independently -CH₂-C₁₀heterocycloalkyl optionally substituted with one, two, or three R^{20a}. In embodiments of the subject complex or compound (e.g., precursor compound), R¹ is independently -CH₂-C₁₁heterocycloalkyl optionally substituted with one, two, or three R^{20a}.

In embodiments of the subject complex or compound (e.g., precursor compound), R¹ is independently C_{6- 12}aryl optionally substituted with one, two, or three R^{20a}.

In embodiments of the subject complex or compound (e.g., precursor compound), R¹ is independently C₆aryl optionally substituted with one, two, or three R^{20a}. In embodiments of the subject complex or compound (e.g., precursor compound), R¹ is independently C₇aryl optionally substituted with one, two, or three R^{20a}. In embodiments of the subject complex or compound (e.g., precursor compound), R¹ is independently C₈aryl optionally substituted with one, two, or three R^{20a}. In embodiments of the subject complex or compound (e.g., precursor compound), R¹ is independently C₉aryl optionally substituted with one, two, or three R^{20a}. In embodiments of the subject complex or compound (e.g., precursor compound), R¹ is independently C₁₀aryl optionally substituted with one, two, or three R^{20a}. In embodiments of the subject complex or compound (e.g., precursor compound), R¹ is independently C₁₁aryl optionally substituted with one, two, or three R^{20a}. In embodiments of the subject complex or compound (e.g., precursor compound), R¹ is independently C₁₂aryl optionally substituted with one, two, or three R^{20a}.

In embodiments of the subject complex or compound (e.g., precursor compound), R¹ is independently - CH₂-C₆₋₁₂aryl optionally substituted with one, two, or three R^{20a}.

In embodiments of the subject complex or compound (e.g., precursor compound), R¹ is independently - CH₂-C₆aryl optionally substituted with one, two, or three R^{20a}. In embodiments of the subject complex or compound (e.g., precursor compound), R¹ is independently -CH₂-C₇aryl optionally substituted with one, two, or three R^{20a}. In embodiments of the subject complex or compound (e.g., precursor compound), R¹ is independently -CH₂-C₈aryl optionally substituted with one, two, or three R^{20a}. In embodiments of the subject complex or compound (e.g., precursor compound), R¹ is independently -CH₂-C₉aryl optionally substituted with one, two, or three R^{20a}. In embodiments of the subject complex or compound (e.g., precursor compound), R¹ is independently -CH₂-C₁₀aryl optionally substituted with one, two, or three R^{20a}. In embodiments of the subject complex or compound (e.g., precursor compound), R¹ is independently -CH₂-C₁₁aryl optionally substituted with one, two, or three R^{20a}. In embodiments of the subject complex or compound (e.g., precursor compound), R¹ is independently -CH₂-C₁₂aryl optionally substituted with one, two, or three R^{20a}.

In embodiments of the subject complex or compound (e.g., precursor compound), R¹ is independently C_{1- 11}heteroaryl optionally substituted with one, two, or three R^{20a}.

In embodiments of the subject complex or compound (e.g., precursor compound), R¹ is independently C₁heteroaryl optionally substituted with one, two, or three R^{20a}. In embodiments of the subject complex or compound (e.g., precursor compound), R¹ is independently C₂heteroaryl optionally substituted with one, two, or three R^{20a}. In embodiments of the subject complex or compound (e.g., precursor compound), R¹ is independently C₃heteroaryl optionally substituted with one, two, or three R^{20a}. In embodiments of the subject complex or compound (e.g., precursor compound), R¹ is independently C₄heteroaryl optionally substituted with one, two, or three R^{20a}. In embodiments of the subject complex or compound (e.g., precursor compound), R¹ is independently C₅heteroaryl optionally substituted with one, two, or three R^{20a}. In embodiments of the subject complex or compound (e.g., precursor compound), R¹ is independently C₆heteroaryl optionally substituted with one, two, or three R^{20a}. In embodiments of the subject complex or compound (e.g., precursor compound), R¹ is independently C₇heteroaryl optionally substituted with one, two, or three R^{20a}. In embodiments of the subject complex or compound (e.g., precursor compound), R¹ is independently C₈heteroaryl optionally substituted with one, two, or three R^{20a}. In embodiments of the subject complex or compound (e.g., precursor compound), R¹ is independently C₉heteroaryl optionally substituted with one, two, or three R^{20a}. In embodiments of the subject complex or compound (e.g., precursor compound), R¹ is independently C₁₀heteroaryl optionally substituted with one, two, or three R^{20a}. In embodiments of the subject complex or compound (e.g., precursor compound), R¹ is independently C₁₁heteroaryl optionally substituted with one, two, or three R^{20a}.

In embodiments of the subject complex or compound (e.g., precursor compound), R¹ is independently - CH₂-C₁₋₁₁heteroaryl optionally substituted with one, two, or three R^{20a}.

In embodiments of the subject complex or compound (e.g., precursor compound), R¹ is independently - CH₂-C₁heteroaryl optionally substituted with one, two, or three R^{20a}. In embodiments of the subject complex or compound (e.g., precursor compound), R¹ is independently -CH₂-C₂heteroaryl optionally substituted with one, two, or three R^{20a}. In embodiments of the subject complex or compound (e.g., precursor compound), R¹ is independently -CH₂-C₃heteroaryl optionally substituted with one, two, or three R^{20a}. In embodiments of the subject complex or compound (e.g., precursor compound), R¹ is independently -CH₂-C₄heteroaryl optionally substituted with one, two, or three R^{20a}. In embodiments of the subject complex or compound (e.g., precursor compound), R¹ is independently -CH₂-C₅heteroaryl optionally substituted with one, two, or three R^{20a}. In embodiments of the subject complex or compound (e.g., precursor compound), R¹ is independently -CH₂-C₆heteroaryl optionally substituted with one, two, or three R^{20a}. In embodiments of the subject complex or compound (e.g., precursor compound), R¹ is independently -CH₂-C₇heteroaryl optionally substituted with one, two, or three R^{20a}. In embodiments of the subject complex or compound (e.g., precursor compound), R¹ is independently -CH₂-C₈heteroaryl optionally substituted with one, two, or three R^{20a}. In embodiments of the subject complex or compound (e.g., precursor compound), R¹ is independently -CH₂-C₉heteroaryl optionally substituted with one, two, or three R^{20a}. In embodiments of the subject complex or compound (e.g., precursor compound), R¹ is independently -CH₂-C₁₀heteroaryl optionally substituted with one, two, or three R^{20a}. In embodiments of the subject complex or compound (e.g., precursor compound), R¹ is independently -CH₂-C₁₁heteroaryl optionally substituted with one, two, or three R^{20a}.

In embodiments of the subject complex or compound (e.g., precursor compound), R⁴ is independently C_{1- 6}alkyl optionally substituted with one, two, or three R^{20a}. In embodiments of the subject complex or compound (e.g., precursor compound), R⁴ is independently methyl optionally substituted with one, two, or three R^{20a}. In embodiments of the subject complex or compound (e.g., precursor compound), R⁴ is independently ethyl optionally substituted with one, two, or three R^{20a}. In embodiments of the subject complex or compound (e.g., precursor compound), R⁴ is independently propyl optionally substituted with one, two, or three R^{20a}. In embodiments of the subject complex or compound (e.g., precursor compound), R⁴ is independently butyl optionally substituted with one, two, or three R^{20a}. In embodiments of the subject complex or compound (e.g., precursor compound), R⁴ is independently pentyl optionally substituted with one, two, or three R^{20a}. In embodiments of the subject complex or compound (e.g., precursor compound), R⁴ is independently hexyl optionally substituted with one, two, or three R^{20a}. In embodiments of the subject complex or compound (e.g., precursor compound), R⁴ is independently C₂₋₆alkenyl optionally substituted with one, two, or three R^{20a}. In embodiments of the subject complex or compound (e.g., precursor compound), R⁴ is independently ethenyl optionally substituted with one, two, or three R^{20a}. In embodiments of the subject complex or compound (e.g., precursor compound), R⁴ is independently propenyl optionally substituted with one, two, or three R^{20a}. In embodiments of the subject complex or compound (e.g., precursor compound), R⁴ is independently butenyl optionally substituted with one, two, or three R^{20a}. In embodiments of the subject complex or compound (e.g., precursor compound), R⁴ is independently pentenyl optionally substituted with one, two, or three R^{20a}. In embodiments of the subject complex or compound (e.g., precursor compound), R⁴ is independently hexenyl optionally substituted with one, two, or three R^{20a}. In embodiments of the subject complex or compound (e.g., precursor compound), R⁴ is independently C₂₋₆alkynyl optionally substituted with one, two, or three R^{20a}. In embodiments of the subject complex or compound (e.g., precursor compound), R⁴ is independently ethynyl optionally substituted with one, two, or three R^{20a}. In embodiments of the subject complex or compound (e.g., precursor compound), R⁴ is independently propynyl optionally substituted with one, two, or three R^{20a}. In embodiments of the subject complex or compound (e.g., precursor compound), R⁴ is independently butynyl optionally substituted with one, two, or three R^{20a}. In embodiments of the subject complex or compound (e.g., precursor compound), R⁴ is independently pentynyl optionally substituted with one, two, or three R^{20a}. In embodiments of the subject complex or compound (e.g., precursor compound), R⁴ is independently hexynyl optionally substituted with one, two, or three R^{20a}. In embodiments of the subject complex or compound (e.g., precursor compound), R⁴ is independently C₃₋₆cycloalkyl optionally substituted with one, two, or three R^{20a}. In embodiments of the subject complex or compound (e.g., precursor compound), R⁴ is independently C₃cycloalkyl optionally substituted with one, two, or three R^{20a}. In embodiments of the subject complex or compound (e.g., precursor compound), R⁴ is independently C₄cycloalkyl optionally substituted with one, two, or three R^{20a}. In embodiments of the subject complex or compound (e.g., precursor compound), R⁴ is independently C₅cycloalkyl optionally substituted with one, two, or three R^{20a}. In embodiments of the subject complex or compound (e.g., precursor compound), R⁴ is independently C₆cycloalkyl optionally substituted with one, two, or three R^{20a}.

In embodiments of the subject complex or compound (e.g., precursor compound), R⁴ is independently C_{1- 9}heterocycloalkyl (e.g., 3, 4, 5, 6, 7, 8, 9, 10, 11, or 12 membered heterocycloalkyl) optionally substituted with one, two, or three R^{20a}. In embodiments of the subject complex or compound (e.g., precursor compound), R⁴ is independently C₂₋₉heterocycloalkyl (e.g., 3, 4, 5, 6, 7, 8, 9, 10, 11, or 12 membered heterocycloalkyl) optionally substituted with one, two, or three R^{20a}. In embodiments of the subject complex or compound (e.g., precursor compound), R⁴ is independently C₁heterocycloalkyl (e.g., 3, 4, 5, 6, 7, 8, 9, 10, 11, or 12 membered heterocycloalkyl) optionally substituted with one, two, or three R^{20a}. In embodiments of the subject complex or compound (e.g., precursor compound), R⁴ is independently C₂heterocycloalkyl (e.g., 3, 4, 5, 6, 7, 8, 9, 10, 11, or 12 membered heterocycloalkyl) optionally substituted with one, two, or three R^{20a}. In embodiments of the subject complex or compound (e.g., precursor compound), R⁴ is independently C₃heterocycloalkyl (e.g., 4, 5, 6, 7, 8, 9, 10, 11, or 12 membered heterocycloalkyl) optionally substituted with one, two, or three R^{20a}. In embodiments of the subject complex or compound (e.g., precursor compound), R⁴ is independently C₄heterocycloalkyl (e.g., 5, 6, 7, 8, 9, 10, 11, or 12 membered heterocycloalkyl) optionally substituted with one, two, or three R^{20a}. In embodiments of the subject complex or compound (e.g., precursor compound), R⁴ is independently C₅heterocycloalkyl (e.g., 6, 7, 8, 9, 10, 11, or 12 membered heterocycloalkyl) optionally substituted with one, two, or three R^{20a}. In embodiments of the subject complex or compound (e.g., precursor compound), R⁴ is independently C₆heterocycloalkyl (e.g., 7, 8, 9, 10, 11, or 12 membered heterocycloalkyl) optionally substituted with one, two, or three R^{20a}. In embodiments of the subject complex or compound (e.g., precursor compound), R⁴ is independently C₇heterocycloalkyl (e.g., 8, 9, 10, 11, or 12 membered heterocycloalkyl) optionally substituted with one, two, or three R^{20a}. In embodiments of the subject complex or compound (e.g., precursor compound), R⁴ is independently C₈heterocycloalkyl (e.g., 9, 10, 11, or 12 membered heterocycloalkyl) optionally substituted with one, two, or three R^{20a}. In embodiments of the subject complex or compound (e.g., precursor compound), R⁴ is independently C₉heterocycloalkyl (e.g., 10, 11 or 12 membered heterocycloalkyl) optionally substituted with one, two, or three R^{20a}.

In embodiments of the subject complex or compound (e.g., precursor compound), R⁴ is independently C_{6- 10}aryl optionally substituted with one, two, or three R^{20a}. In embodiments of the subject complex or compound (e.g., precursor compound), R⁴ is independently C₆aryl optionally substituted with one, two, or three R^{20a}. In embodiments of the subject complex or compound (e.g., precursor compound), R⁴ is independently C₇aryl optionally substituted with one, two, or three R^{20a}. In embodiments of the subject complex or compound (e.g., precursor compound), R⁴ is independently C₈aryl optionally substituted with one, two, or three R^{20a}. In embodiments of the subject complex or compound (e.g., precursor compound), R⁴ is independently C₉aryl optionally substituted with one, two, or three R^{20a}. In embodiments of the subject complex or compound (e.g., precursor compound), R⁴ is independently C₁₀aryl optionally substituted with one, two, or three R^{20a}. In embodiments of the subject complex or compound (e.g., precursor compound), R⁴ is independently C₁₋₉heteroaryl (e.g., 5 or 6 membered heteroaryl) optionally substituted with one, two, or three R^{20a}. In embodiments of the subject complex or compound (e.g., precursor compound), R⁴ is independently C₁heteroaryl (e.g., 5 or 6 membered heteroaryl) optionally substituted with one, two, or three R^{20a}. In embodiments of the subject complex or compound (e.g., precursor compound), R⁴ is independently C₂heteroaryl (e.g., 5 or 6 membered heteroaryl) optionally substituted with one, two, or three R^{20a}. In embodiments of the subject complex or compound (e.g., precursor compound), R⁴ is independently C₃heteroaryl (e.g., 5, 6, 7, 8, or 9 membered heteroaryl) optionally substituted with one, two, or three R^{20a}. In embodiments of the subject complex or compound (e.g., precursor compound), R⁴ is independently C₄heteroaryl (e.g., 5, 6, 7, 8, 9, 10, 11, or 12 membered heteroaryl) optionally substituted with one, two, or three R^{20a}. In embodiments of the subject complex or compound (e.g., precursor compound), R⁴ is independently C₅heteroaryl (e.g., 6, 7, 8, 9, 10, 11, or 12 membered heteroaryl) optionally substituted with one, two, or three R^{20a}. In embodiments of the subject complex or compound (e.g., precursor compound), R⁴ is independently C₆heteroaryl (e.g., 7, 8, 9, 10, 11, or 12 membered heteroaryl) optionally substituted with one, two, or three R^{20a}. In embodiments of the subject complex or compound (e.g., precursor compound), R⁴ is independently C₇heteroaryl (e.g., 8, 9, 10, 11, or 12 membered heteroaryl) optionally substituted with one, two, or three R^{20a}. In embodiments of the subject complex or compound (e.g., precursor compound), R⁴ is independently C₈heteroaryl (e.g., 9, 10, 11, or 12 membered heteroaryl) optionally substituted with one, two, or three R^{20a}. In embodiments of the subject complex or compound (e.g., precursor compound), R⁴ is independently C₉heteroaryl (e.g., 10, 11, or 12 membered heteroaryl) optionally substituted with one, two, or three R^{20a}.

In embodiments of the subject complex or compound (e.g., precursor compound), R⁴ is independently - SR¹². In embodiments of the subject complex or compound (e.g., precursor compound), R⁴ is independently - N(R¹⁴)C(O)N(R¹²)(R¹³). In embodiments of the subject complex or compound (e.g., precursor compound), R⁴ is independently -N(R¹⁴)C(O)OR¹⁵. In embodiments of the subject complex or compound (e.g., precursor compound), R⁴ is independently -N(R¹⁴)S(O)₂R¹⁵. In embodiments of the subject complex or compound (e.g., precursor compound), R⁴ is independently -C(O)R¹². In embodiments of the subject complex or compound (e.g., precursor compound), R⁴ is independently -S(O)R¹⁵. In embodiments of the subject complex or compound (e.g., precursor compound), R⁴ is independently -OC(O)R¹⁵. In embodiments of the subject complex or compound (e.g., precursor compound), R⁴ is independently -C(O)N(R¹²)(R¹³). In embodiments of the subject complex or compound (e.g., precursor compound), R⁴ is independently -C(O)C(O)N(R¹²)(R¹³). In embodiments of the subject complex or compound (e.g., precursor compound), R⁴ is independently -N(R¹⁴)C(O)R¹². In embodiments of the subject complex or compound (e.g., precursor compound), R⁴ is independently -S(O)₂R¹⁵. In embodiments of the subject complex or compound (e.g., precursor compound), R⁴ is independently -S(O)₂N(R¹²)(R¹³)-. In embodiments of the subject complex or compound (e.g., precursor compound), R⁴ is independently S(=O)(=NH)N(R¹²)(R¹³). In embodiments of the subject complex or compound (e.g., precursor compound), R⁴ is independently - CH₂C(O)N(R¹²)(R¹³). In embodiments of the subject complex or compound (e.g., precursor compound), R⁴ is independently -CH₂N(R¹⁴)C(O)R¹⁵. In embodiments of the subject complex or compound (e.g., precursor compound), R⁴ is independently -CH₂S(O)₂R¹⁵. In embodiments of the subject complex or compound (e.g., precursor compound), R⁴ is independently -CH₂S(O)₂N(R¹²)(R¹³).

The embodiments of R⁵ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. The embodiments of R⁷ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. The embodiments of R¹⁰ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following.

In embodiments, R⁷ is

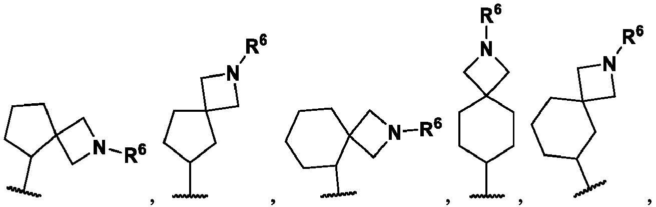

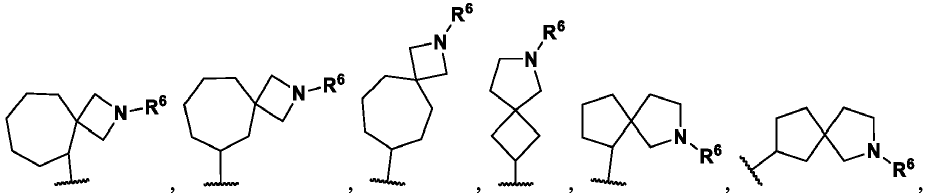

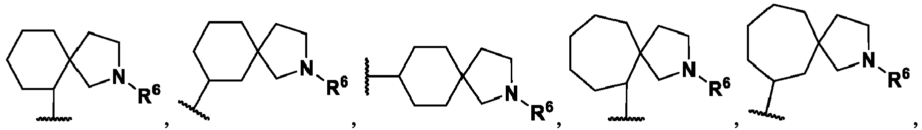

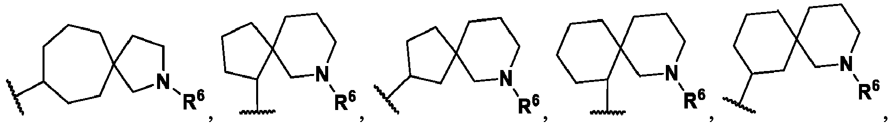

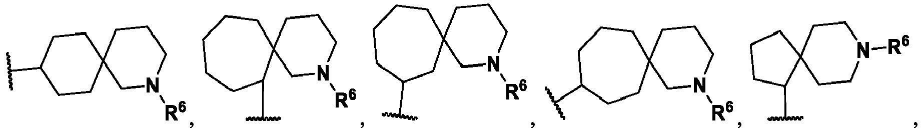

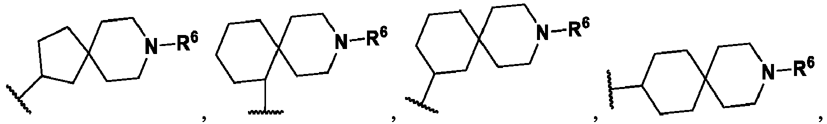

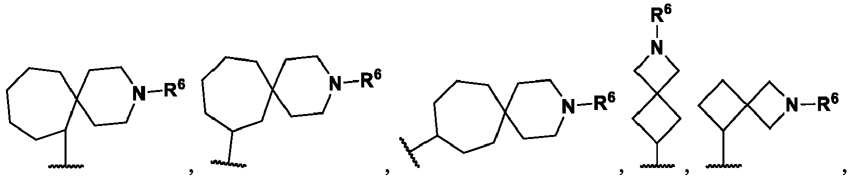

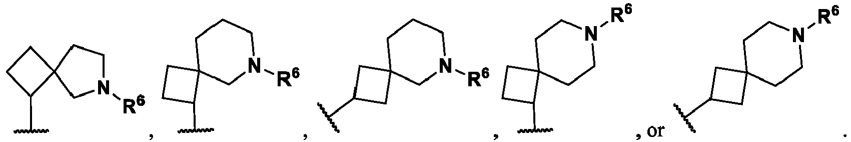

In embodiments, R⁷ is

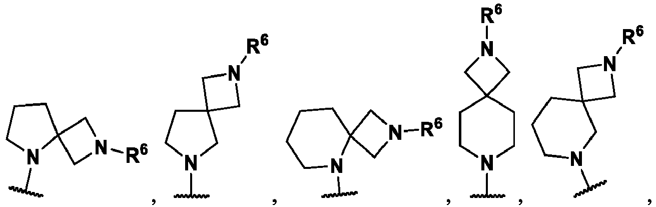

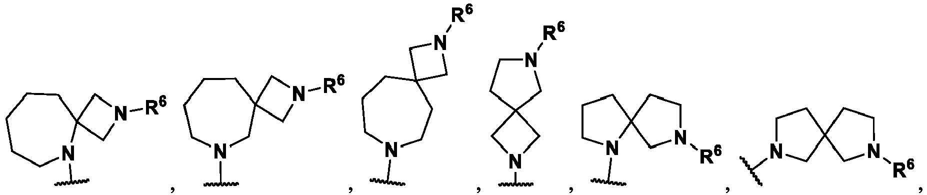

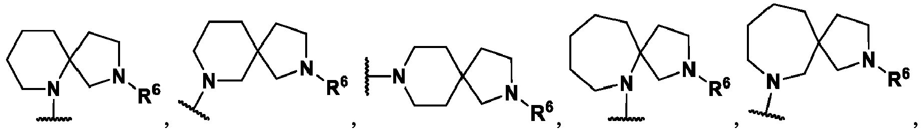

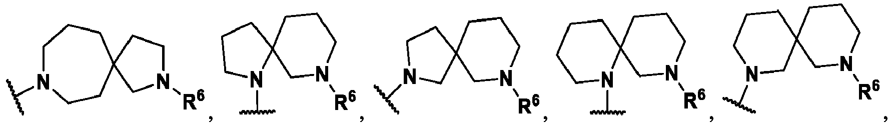

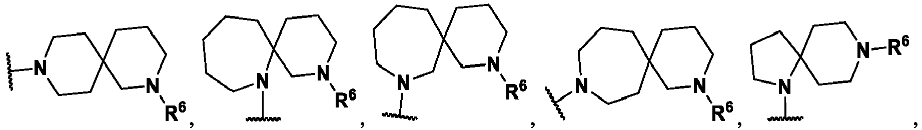

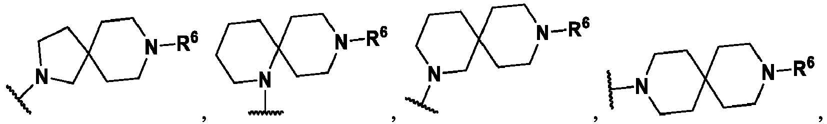

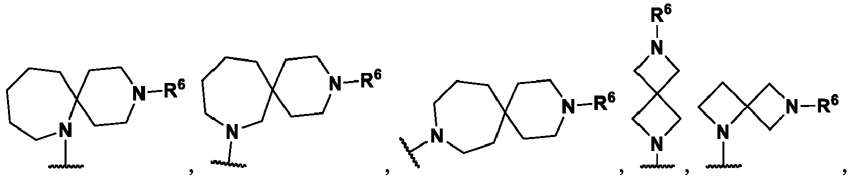

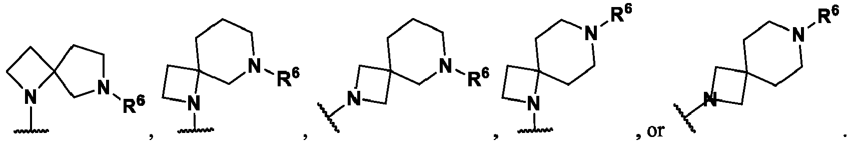

In embodiments, R⁷ is

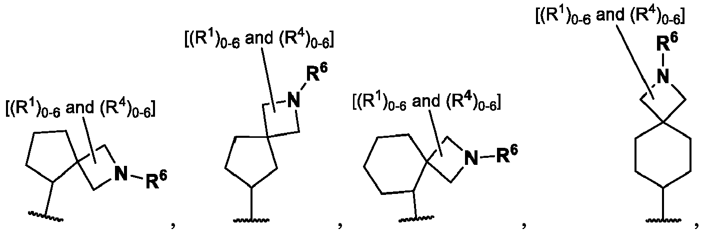

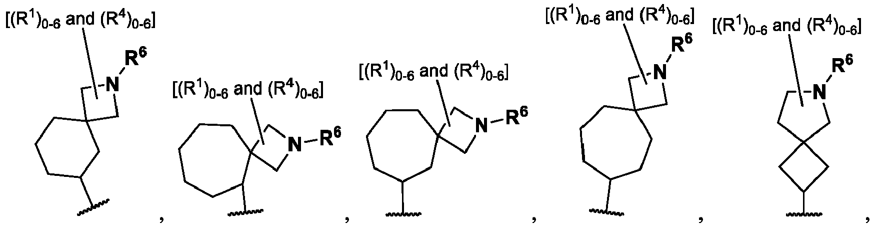

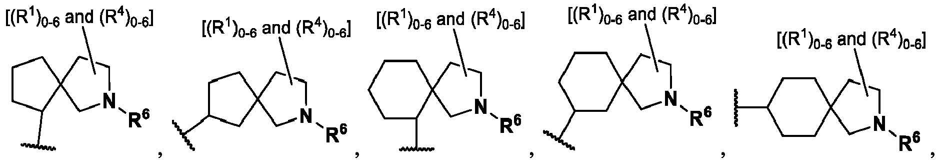

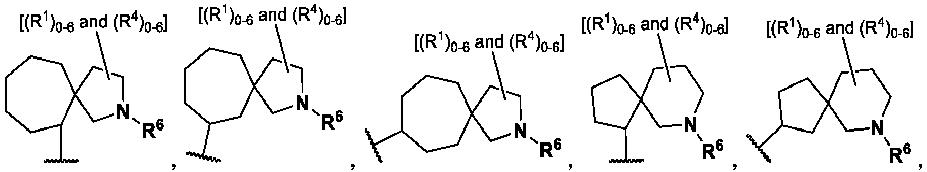

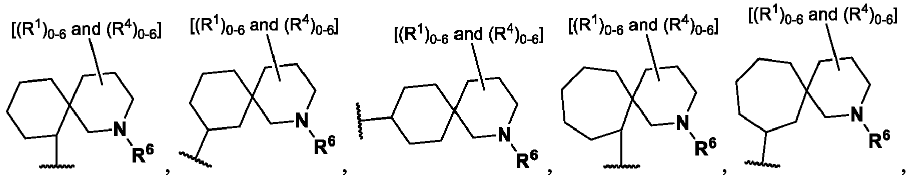

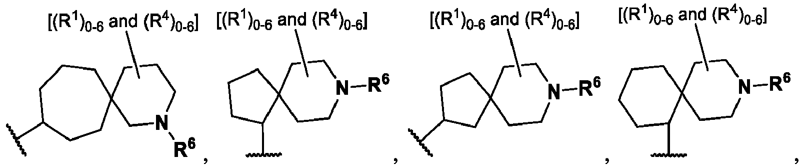

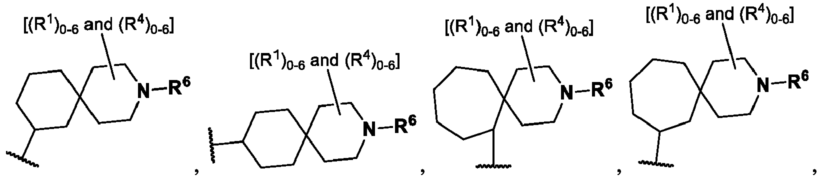

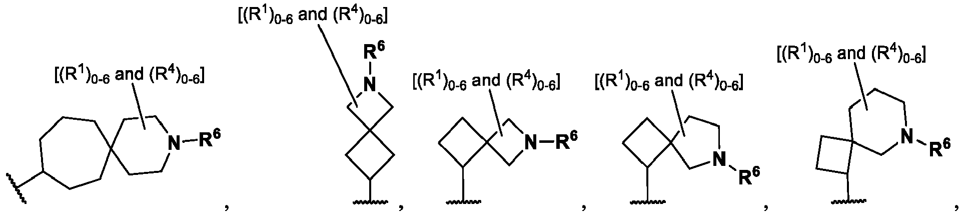

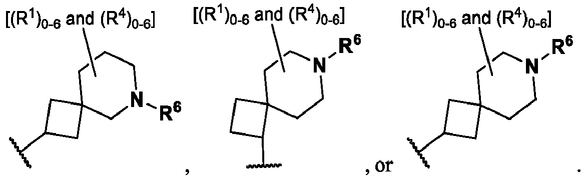

In embodiments, R⁷ is

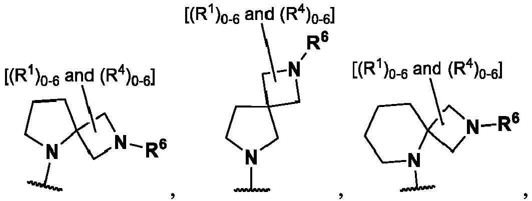

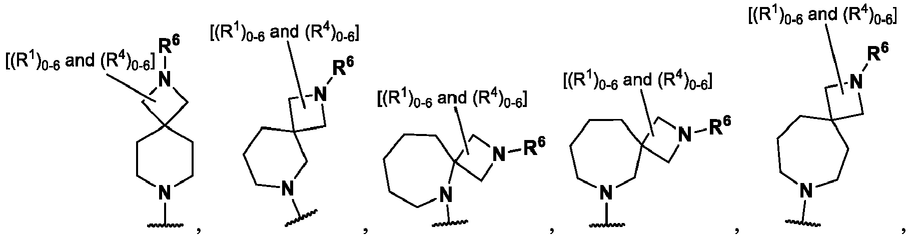

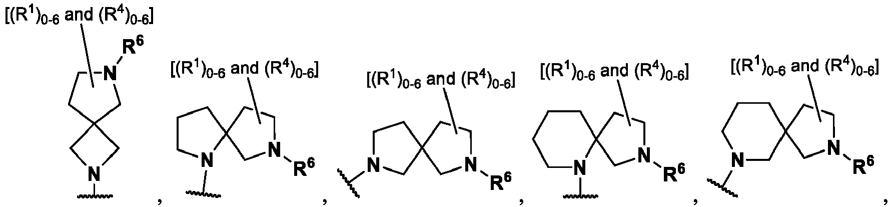

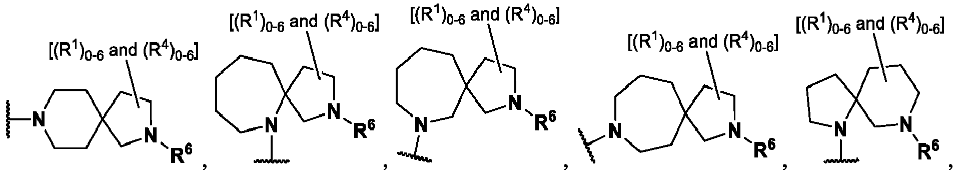

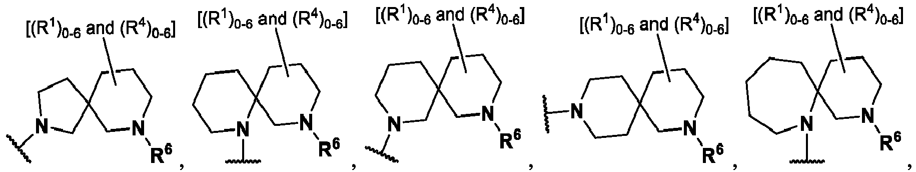

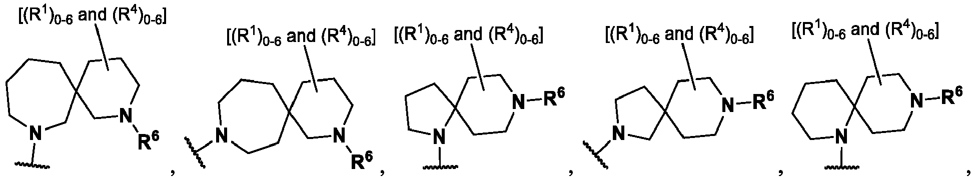

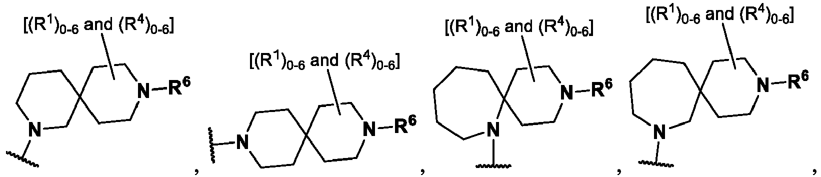

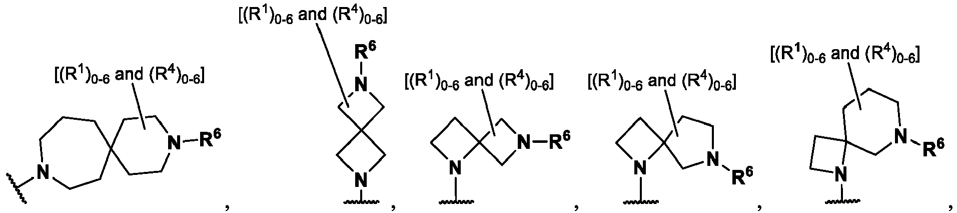

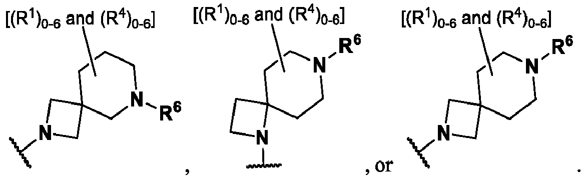

It will be understood that when one or more floating substituent(s) is/are shown extending from one ring in a polycyclic ring system (e.g., fused ring system, bridged ring system, or spirocyclic ring system), the one or more floating substituent(s), may be bonded to the ring from which the one or more floating substituents are shown extending or may be bonded to any other ring in the polycyclic ring system and when multiple substituents are represented by the floating substituents, each substituent may be bonded to the same or different rings in the polycyclic ring system, unless indicated otherwise.

In embodiments, R⁷ is selected from

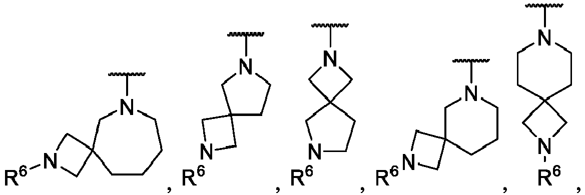

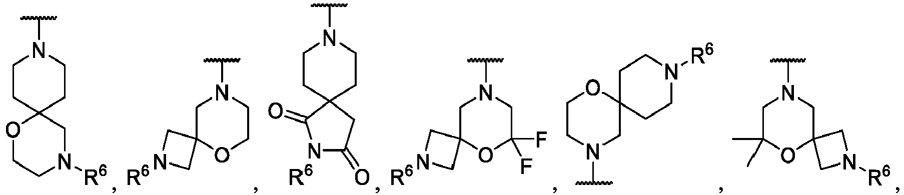

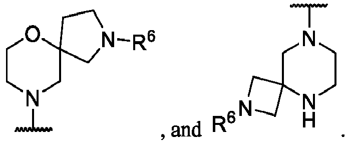

In embodiments, R⁷ is selected from

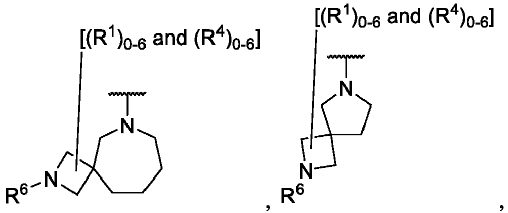

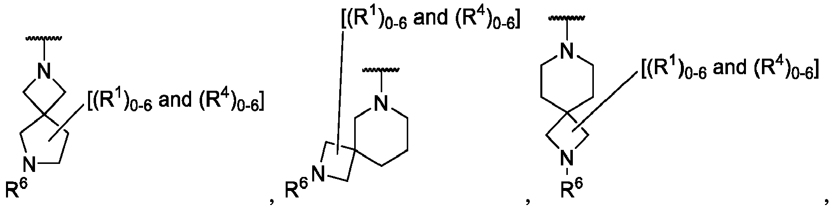

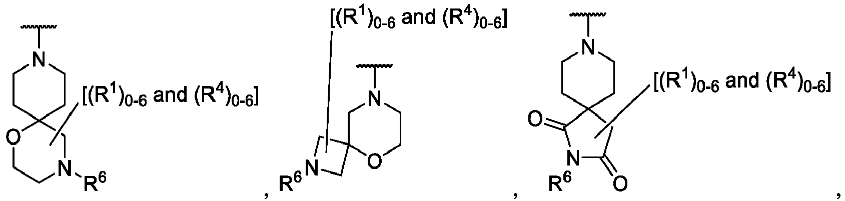

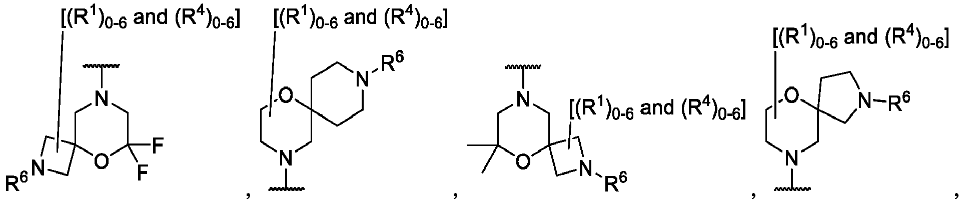

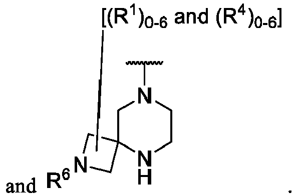

In embodiments, R⁷ is selected from 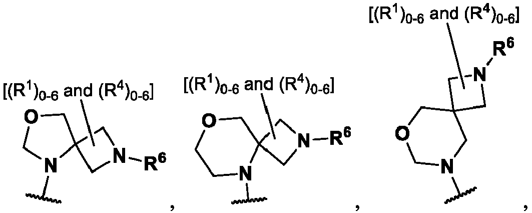 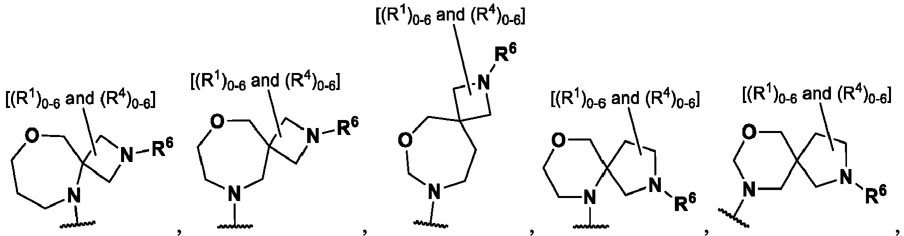 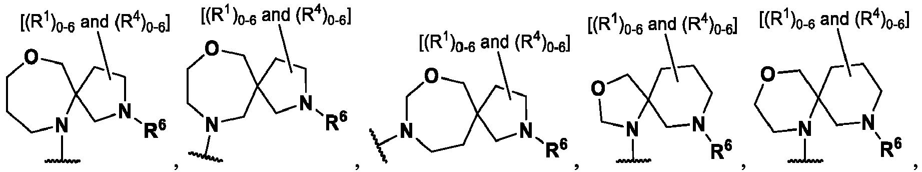 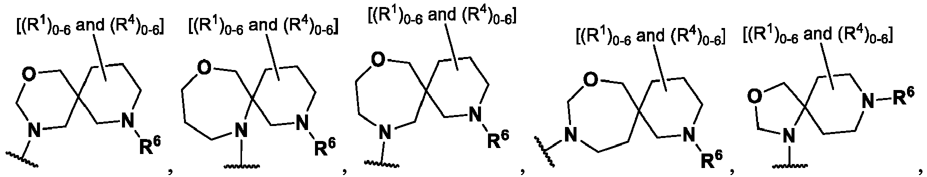 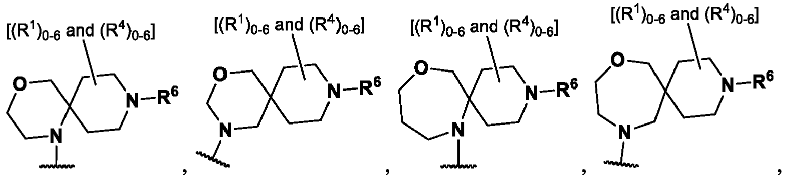 and

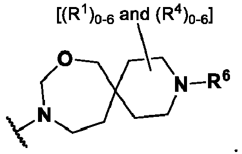

In embodiments, R⁷ is selected from 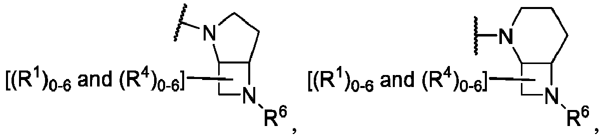 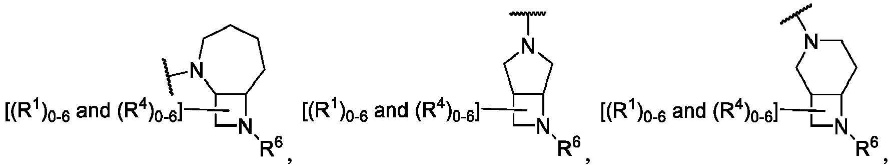 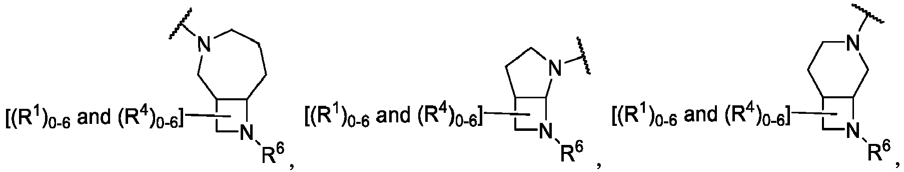 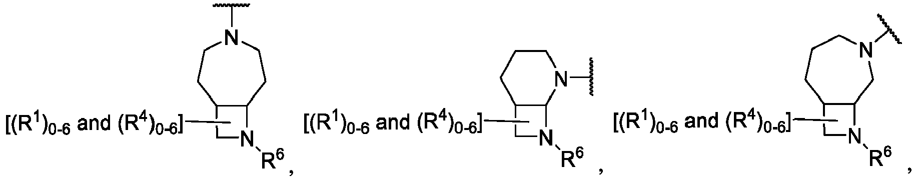 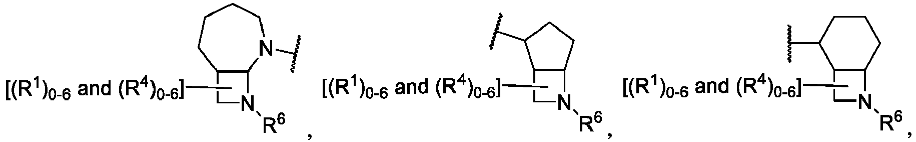 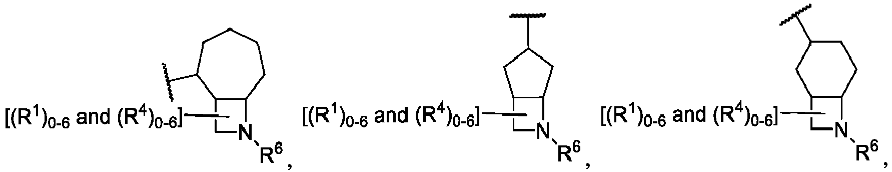 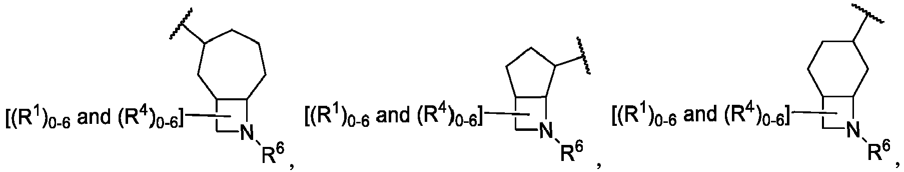 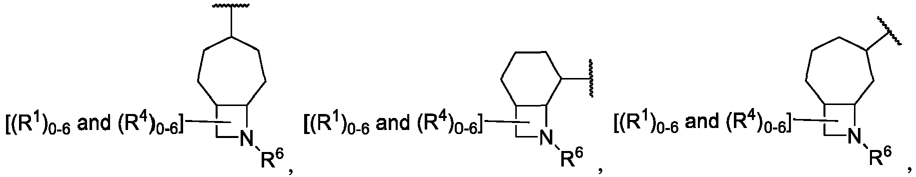 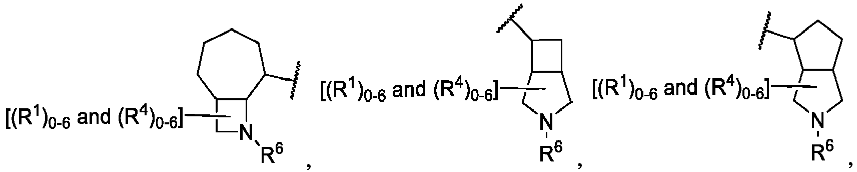 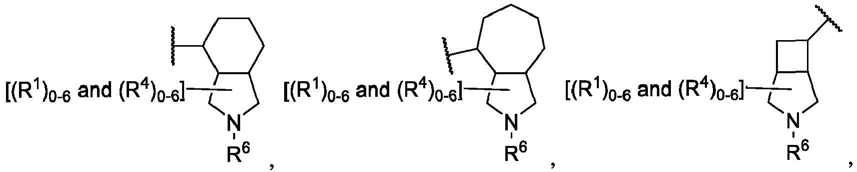 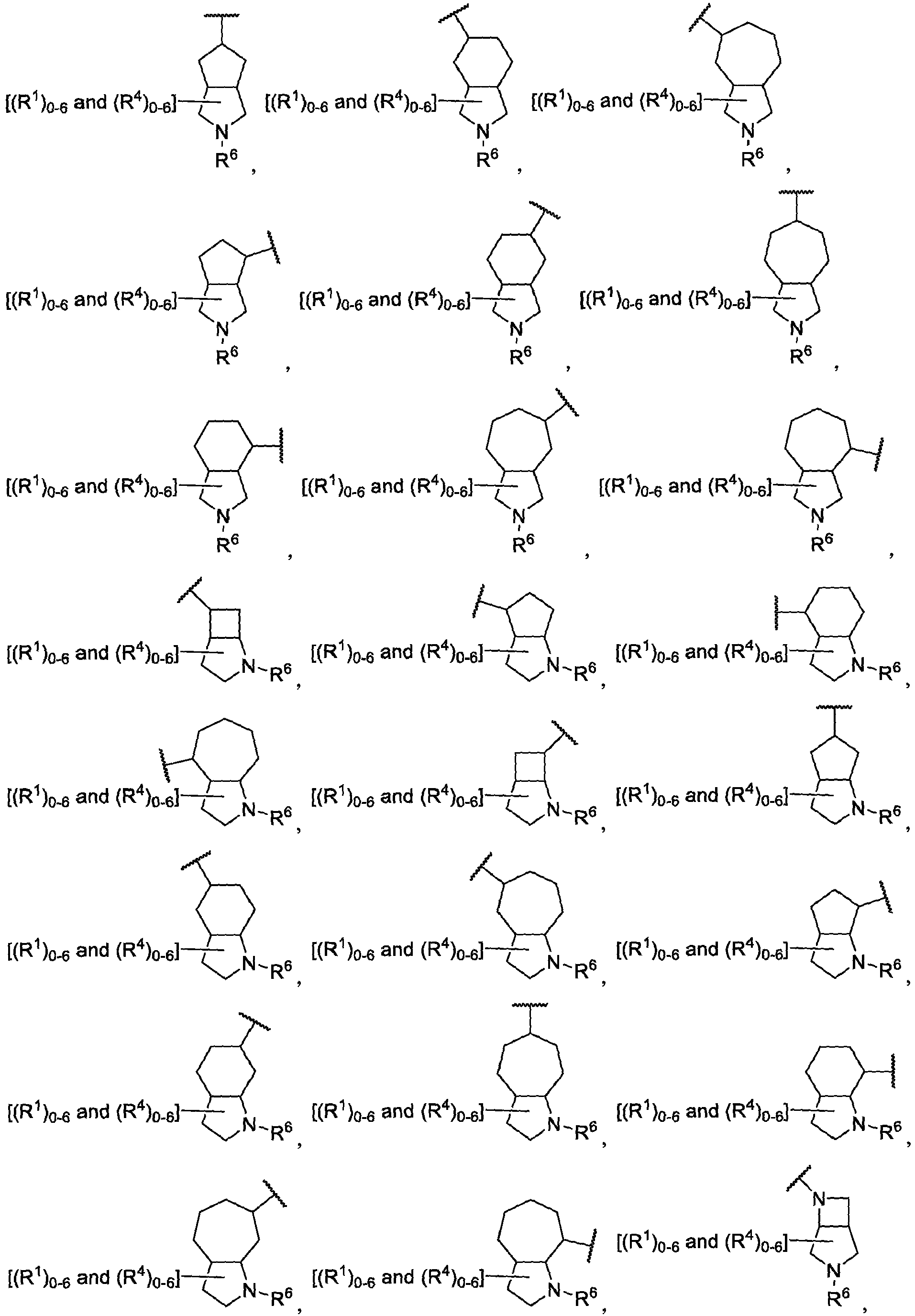 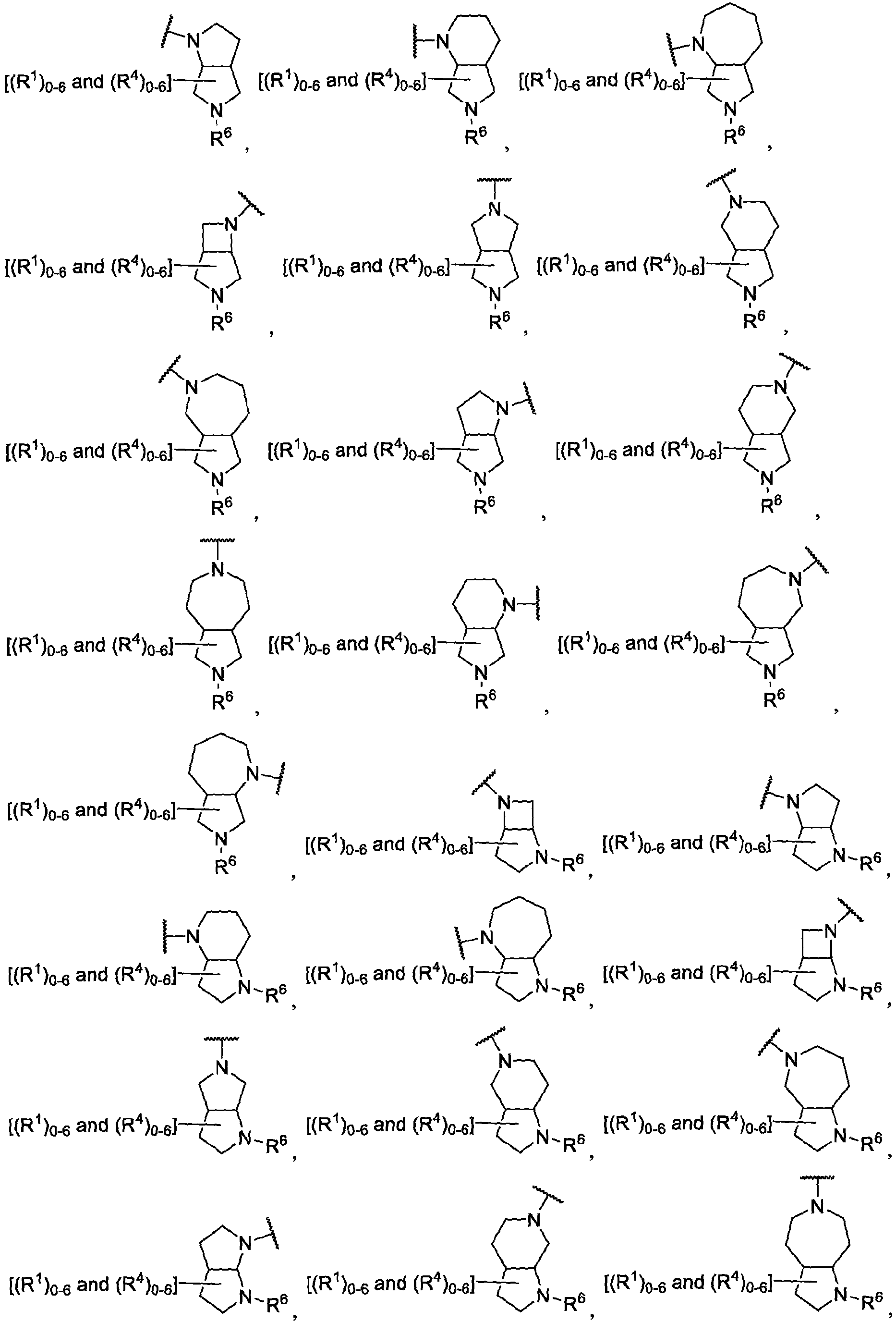 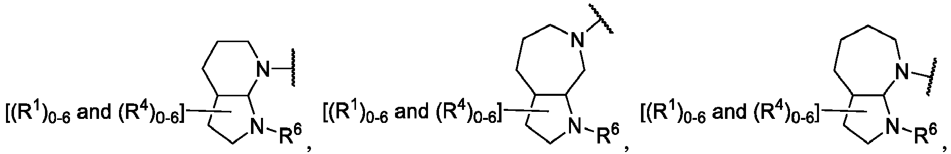 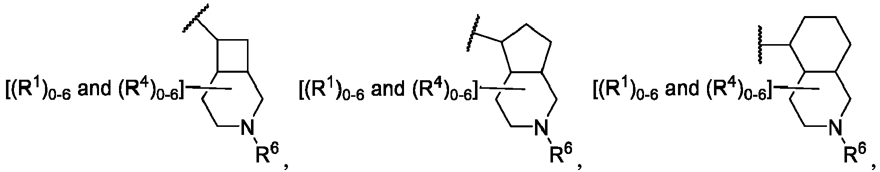 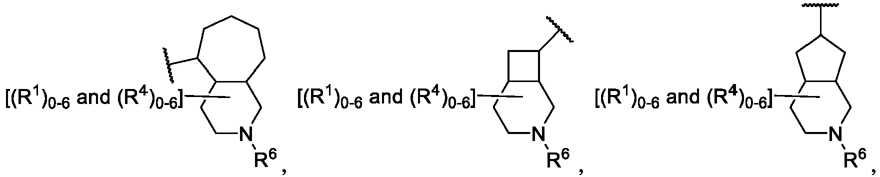 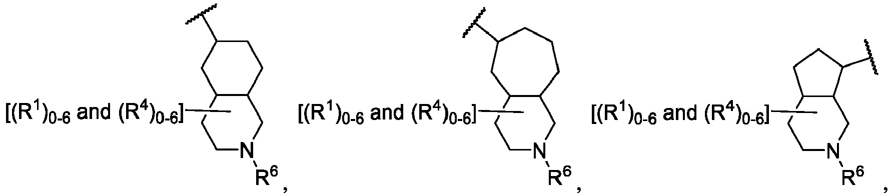 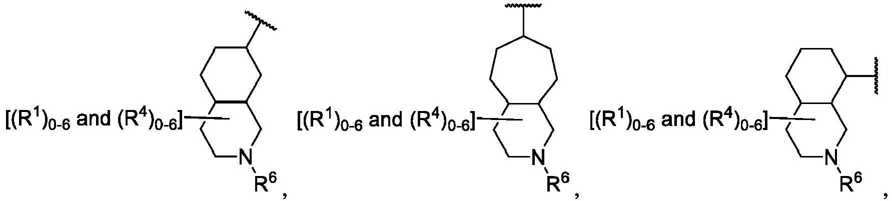 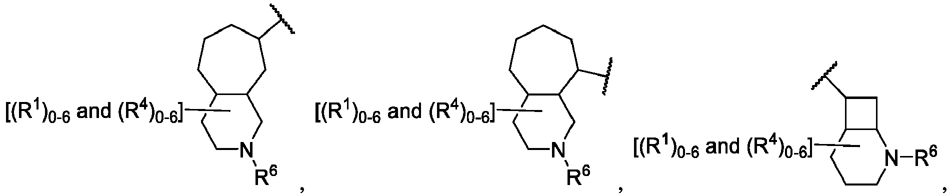 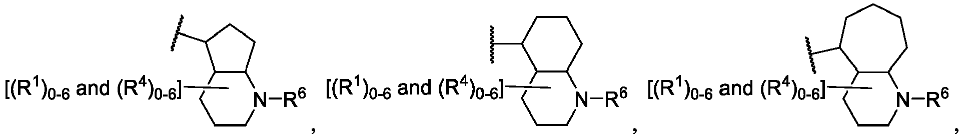 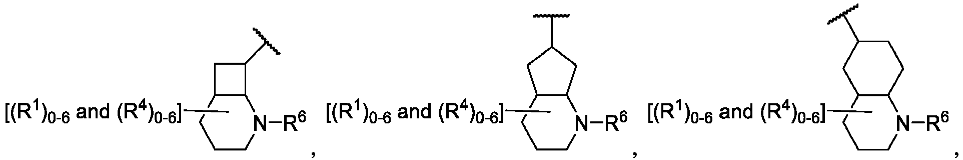 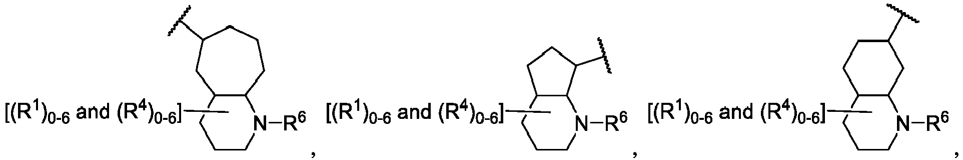 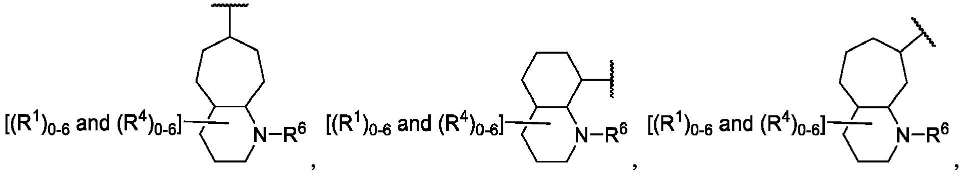 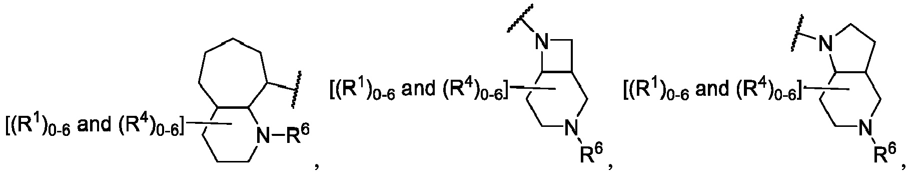 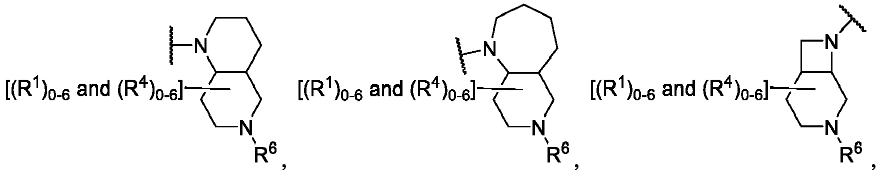 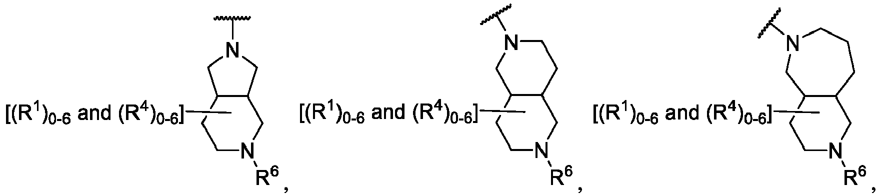 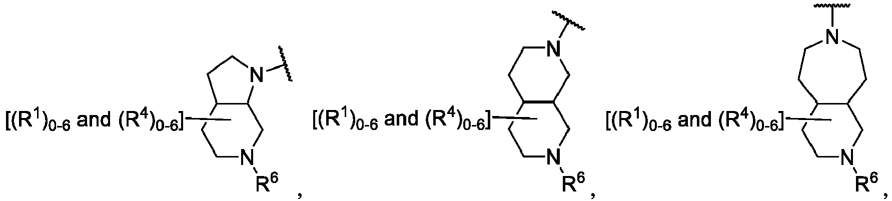 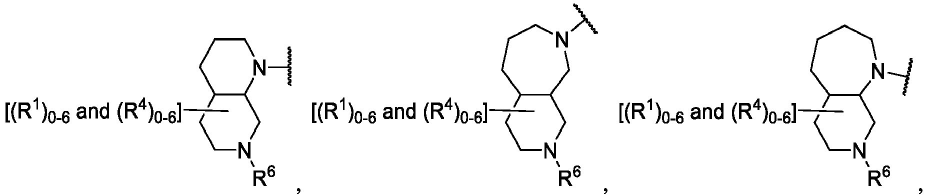 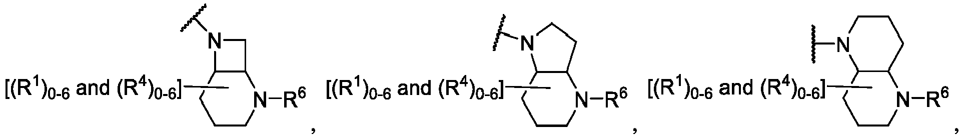 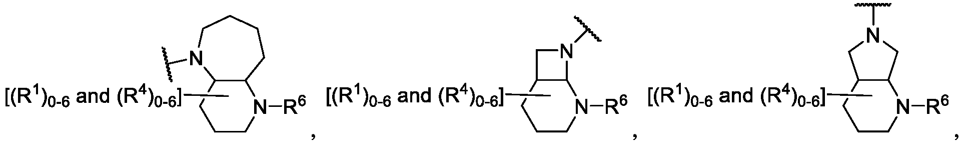 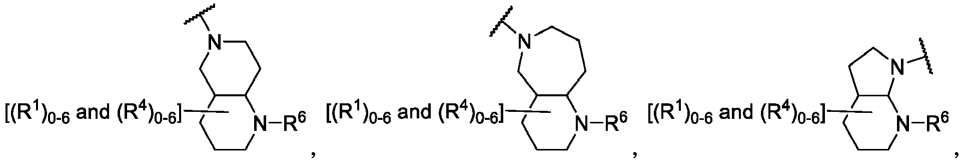 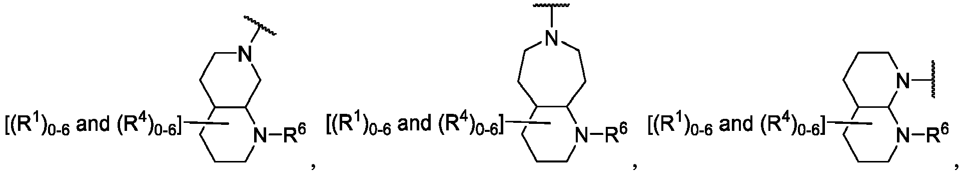 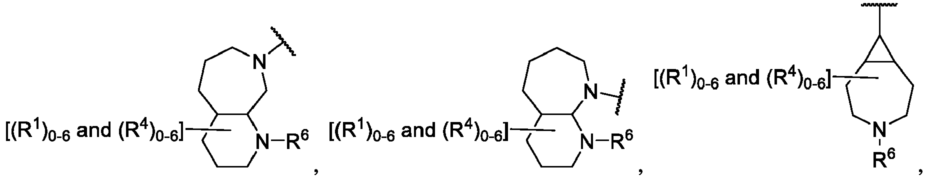 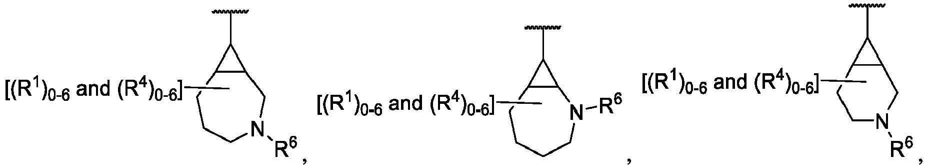 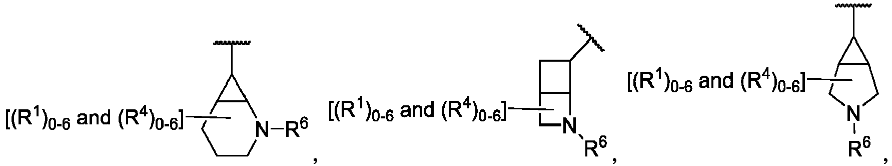 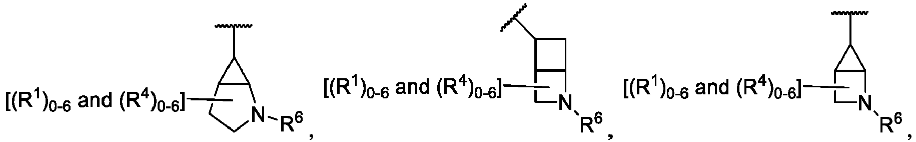 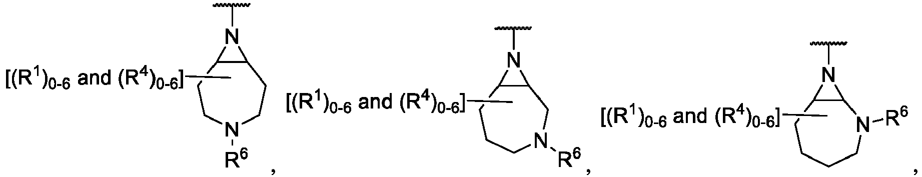 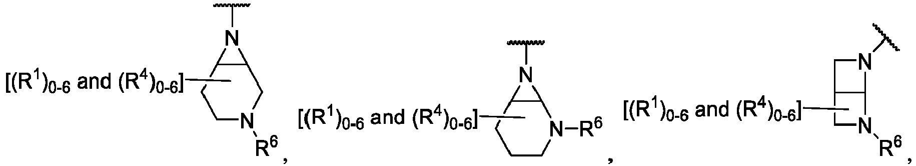 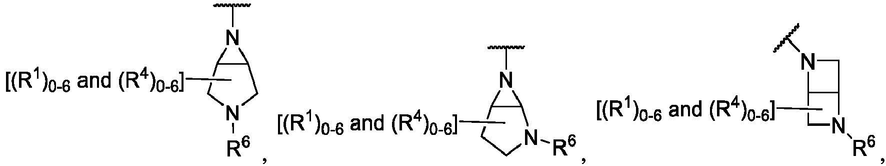 and 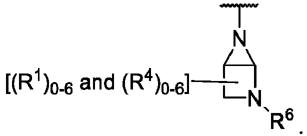 In embodiments, R⁷ is selected from 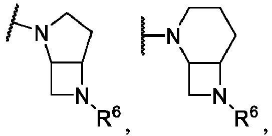 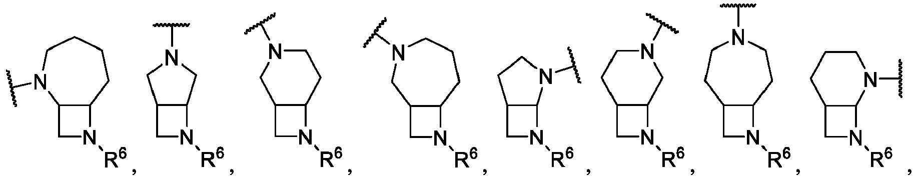 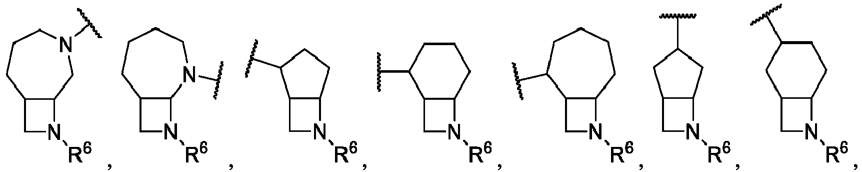 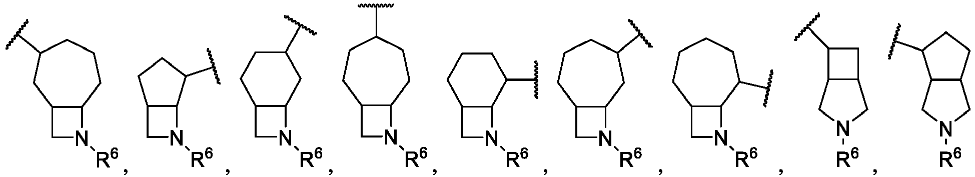 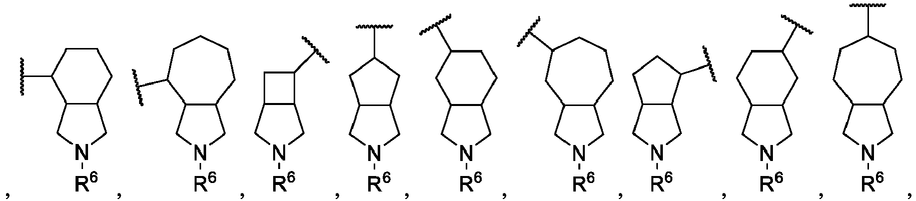 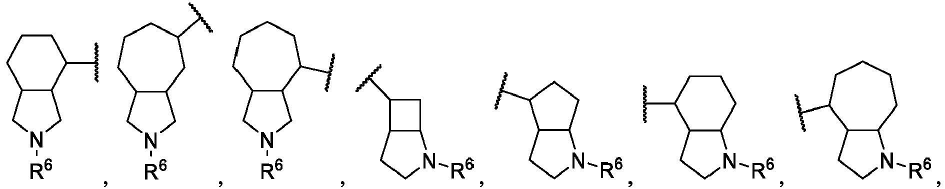 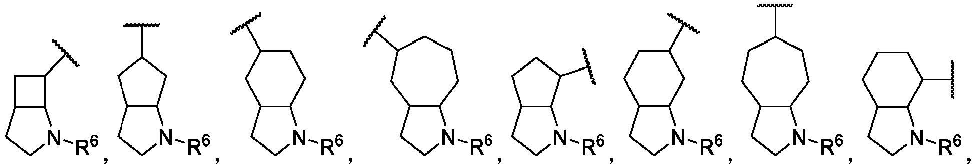 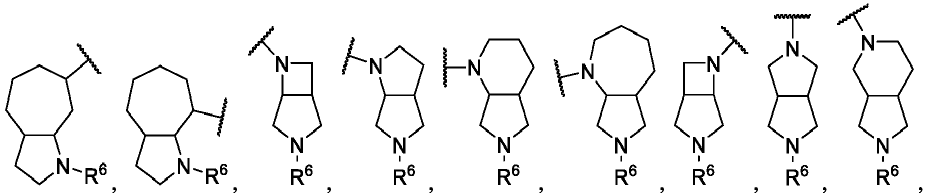 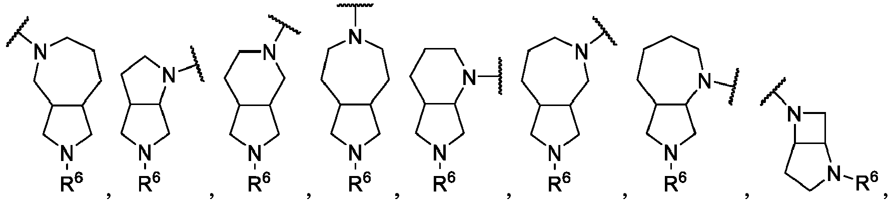 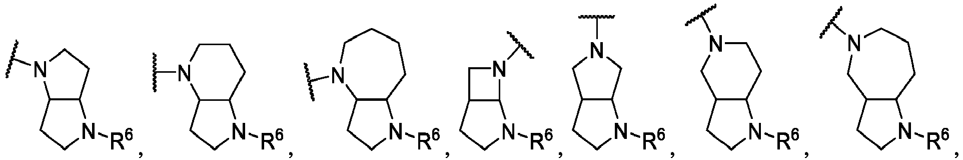 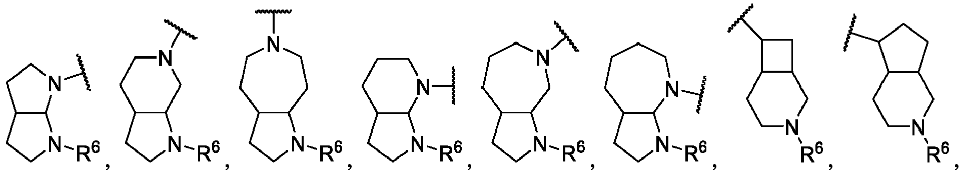 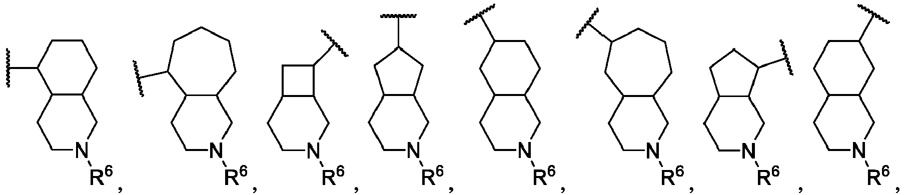 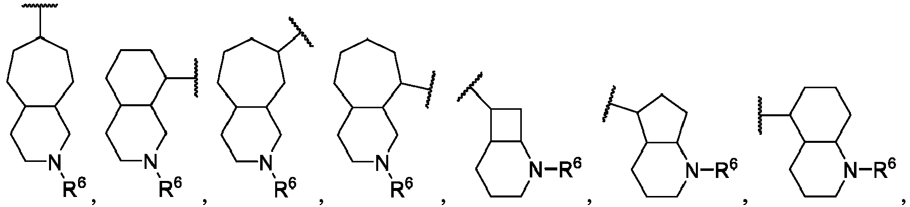 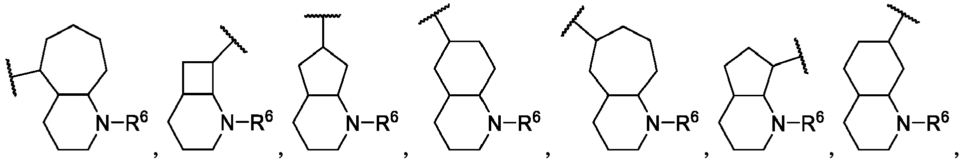 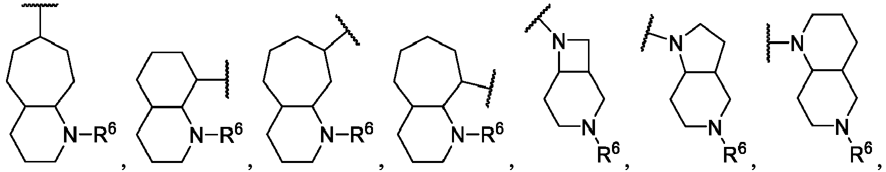 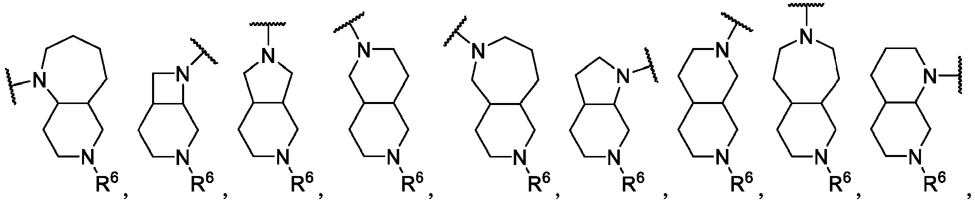 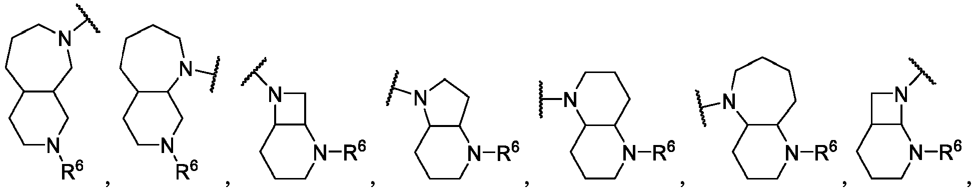 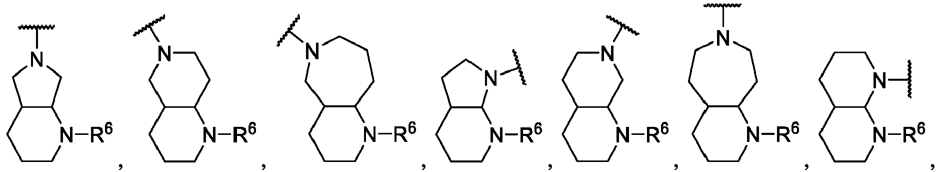 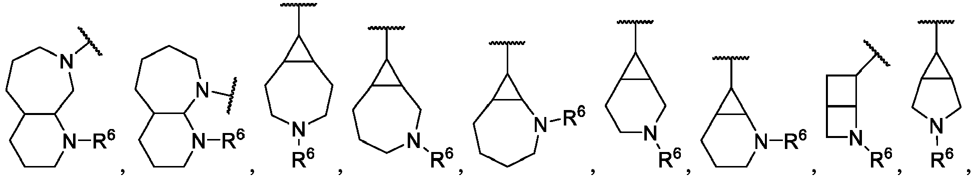 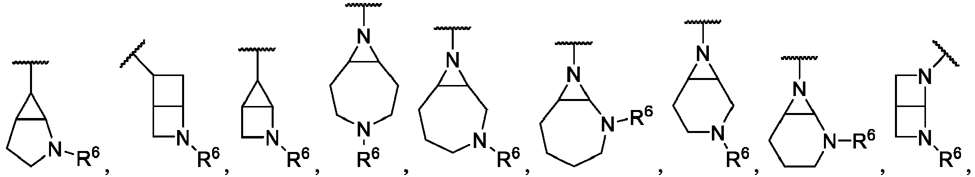 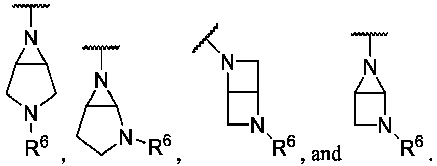 In embodiments, R⁷ is selected from 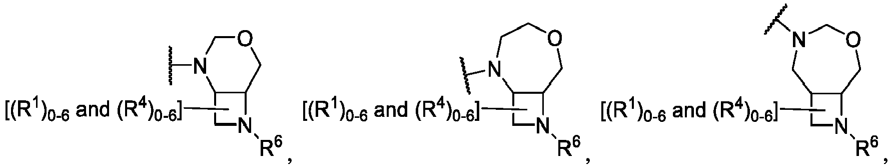 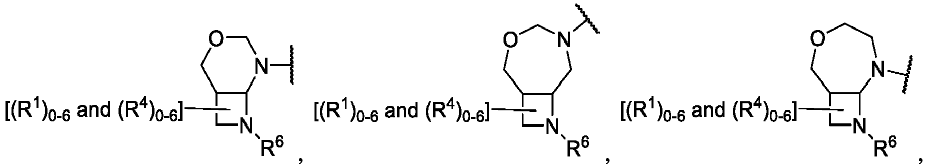 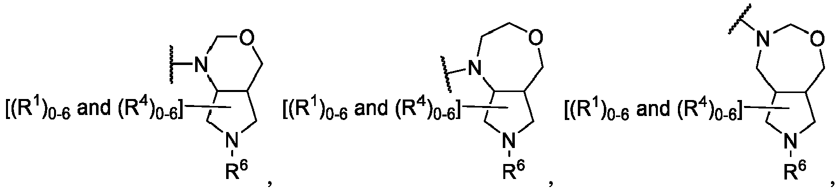 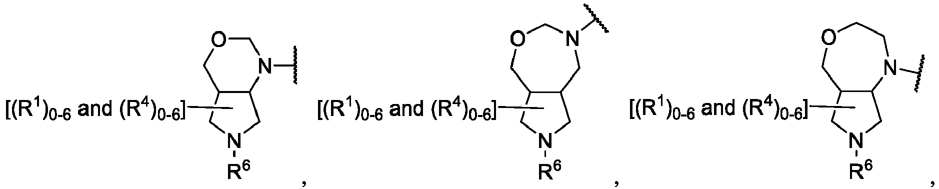 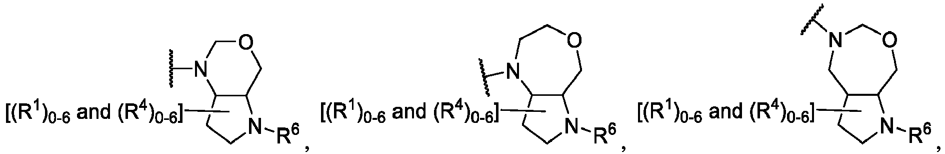 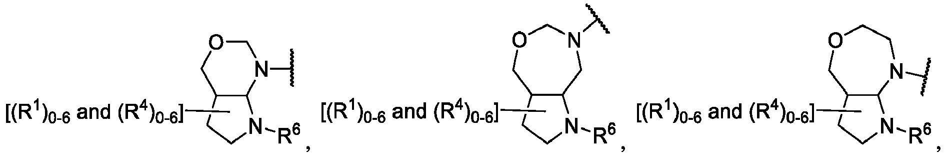 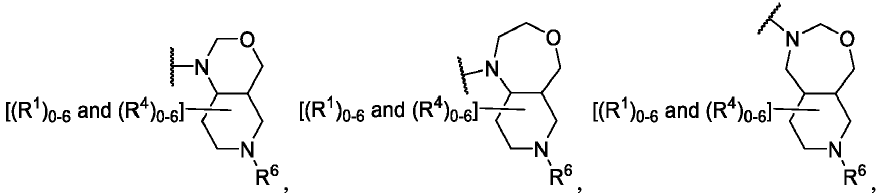 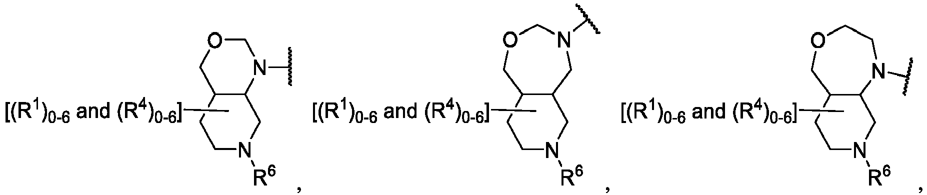 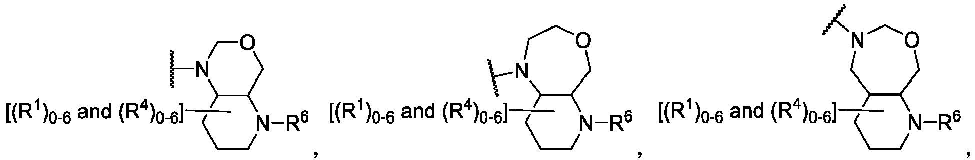 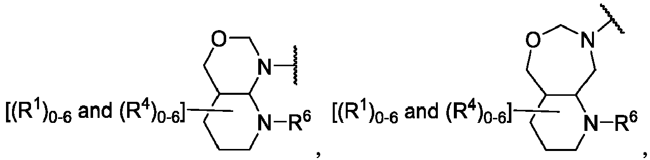 and 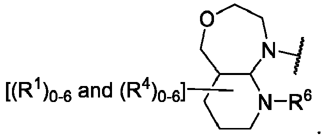 In embodiments, R⁷ is selected from 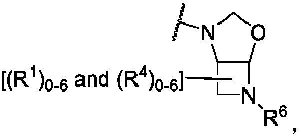 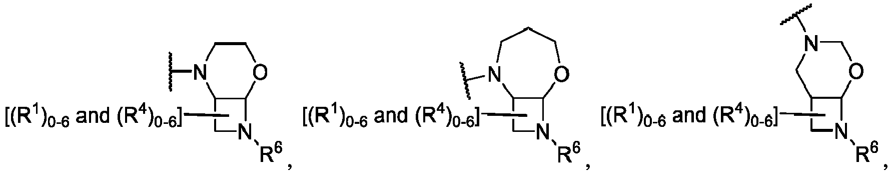 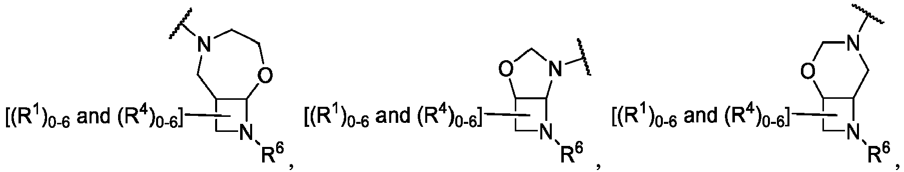 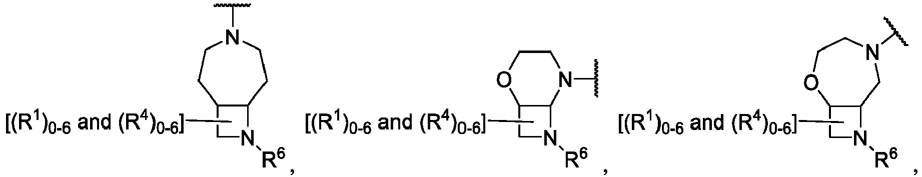 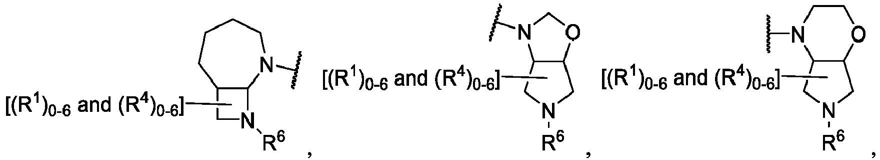 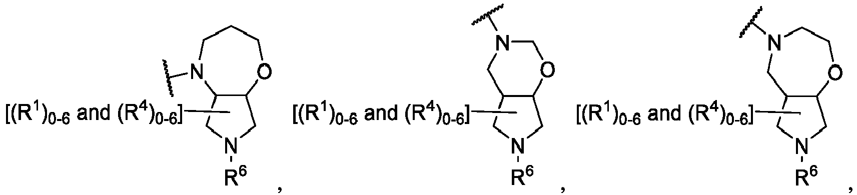 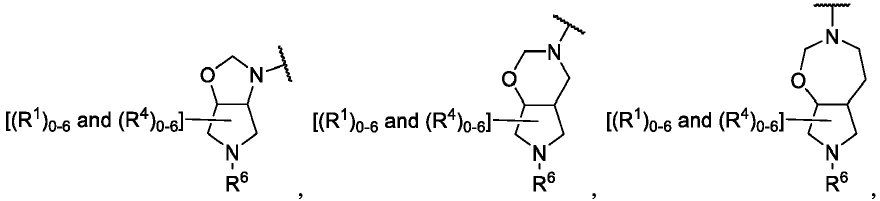 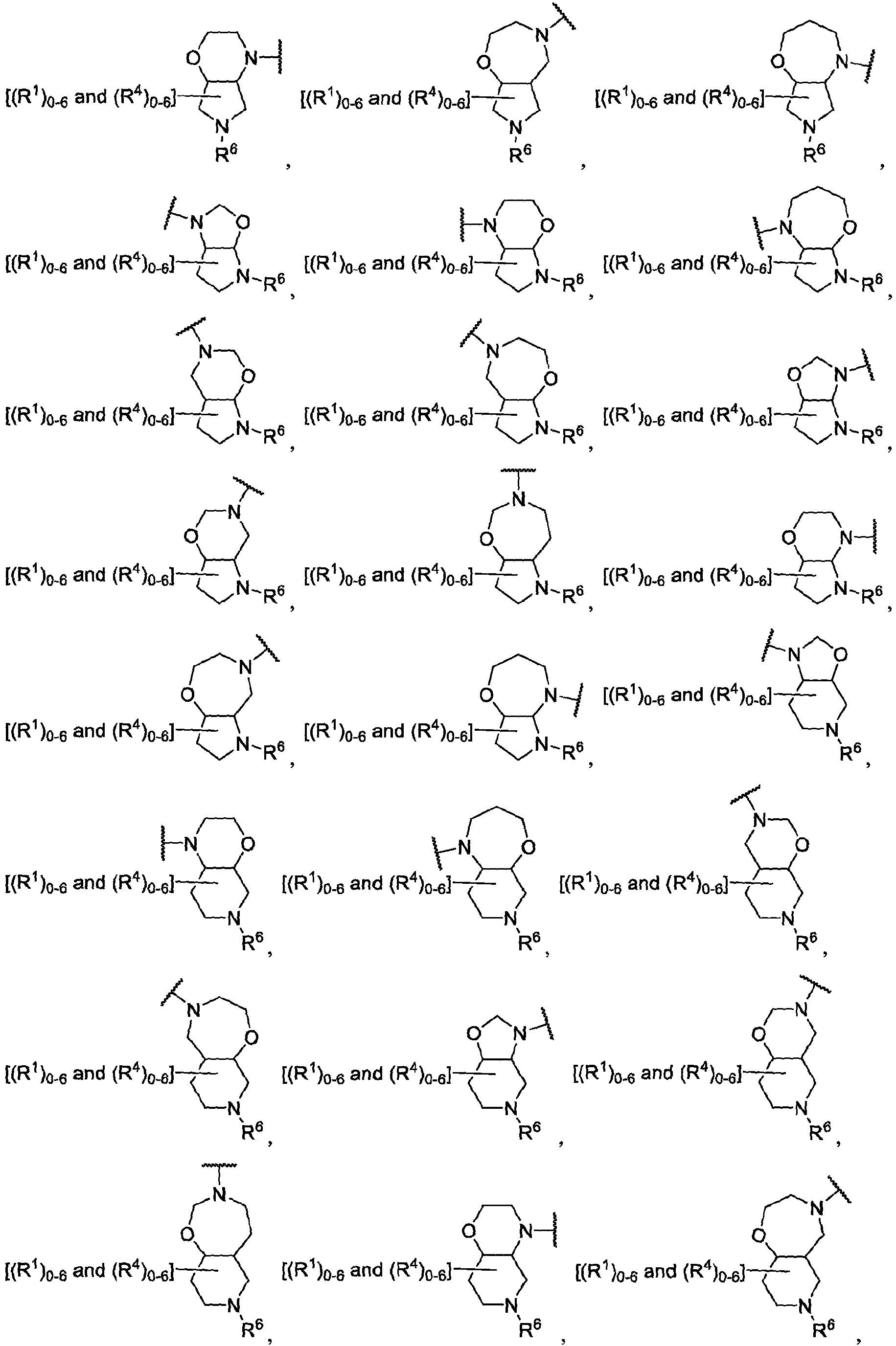 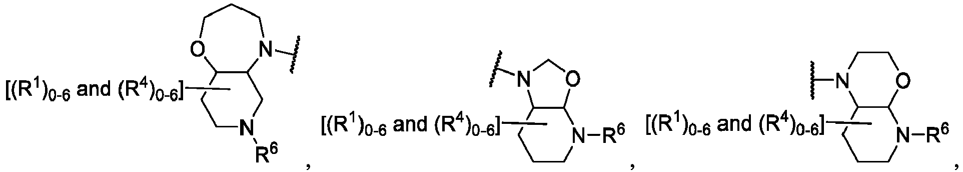 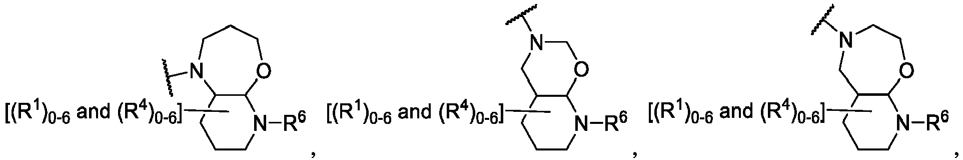 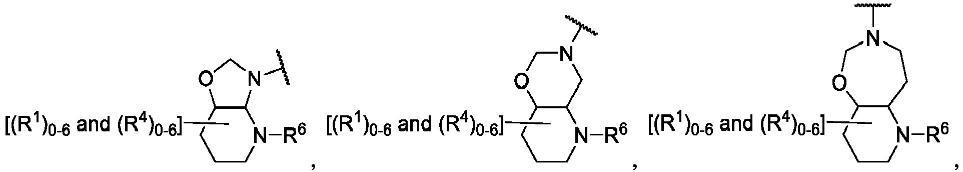 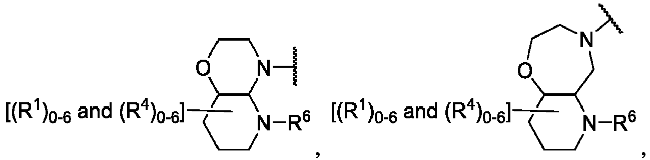 and 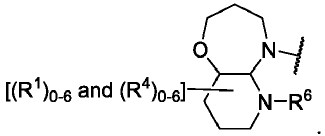 In embodiments, R⁷ is selected from

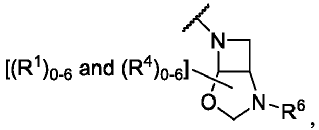

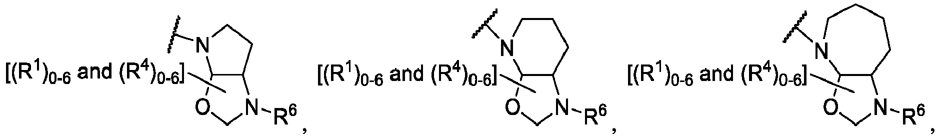

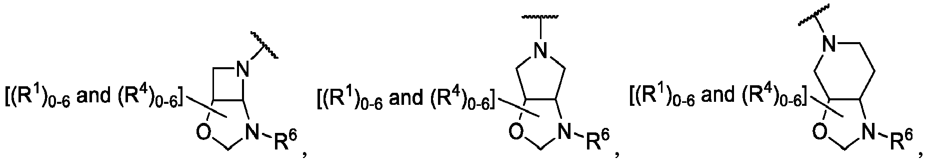

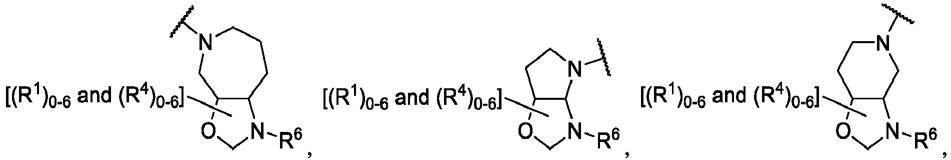

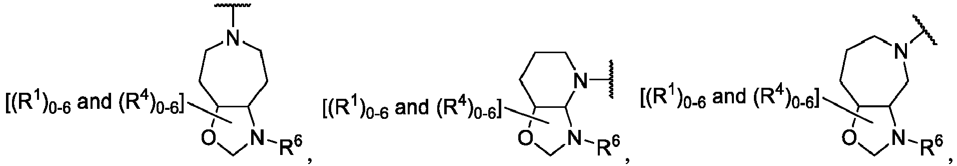

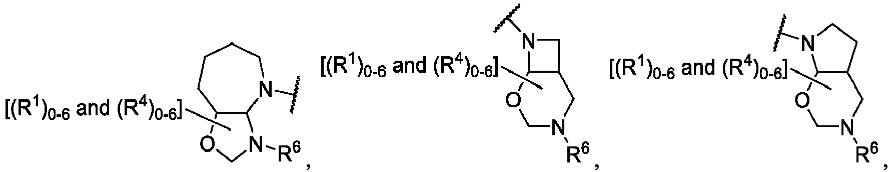

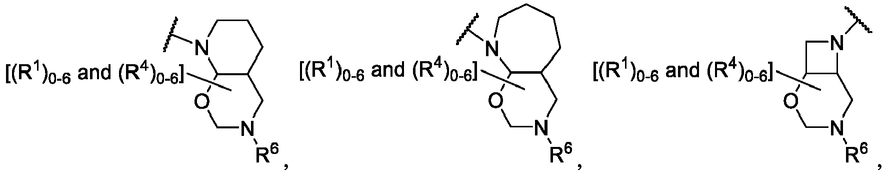

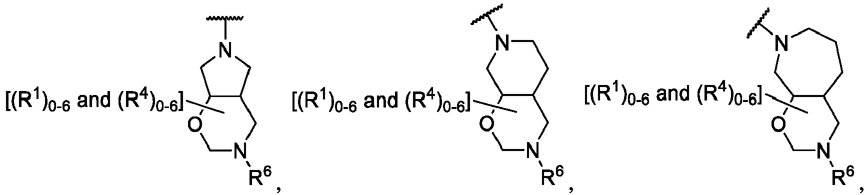

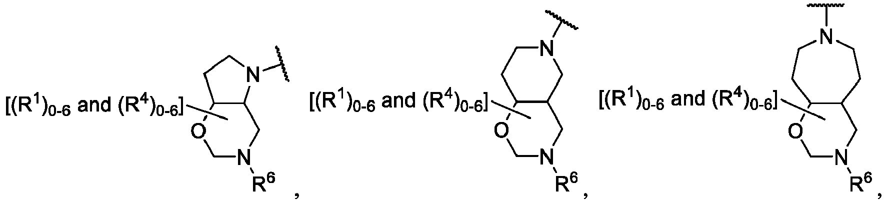

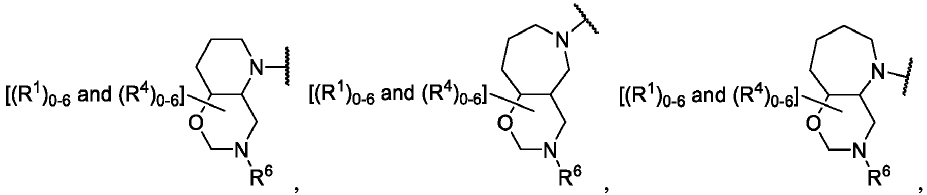

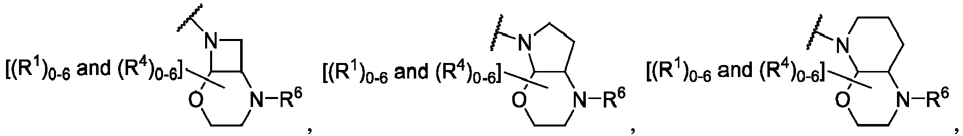

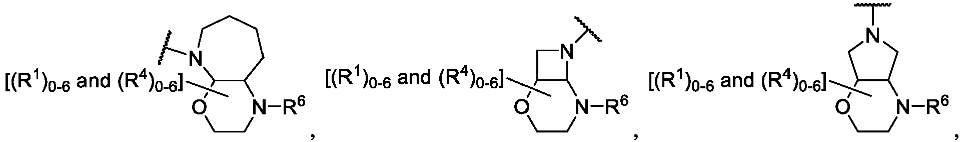

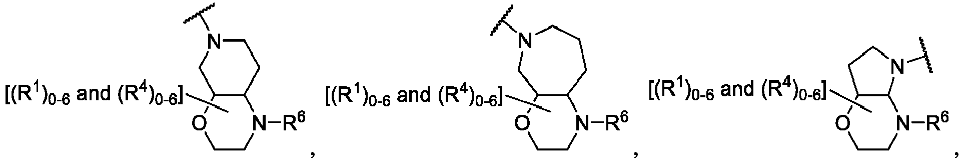

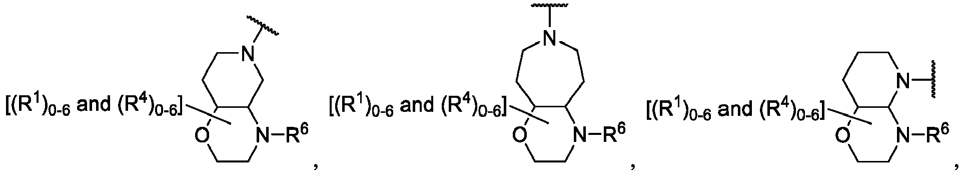

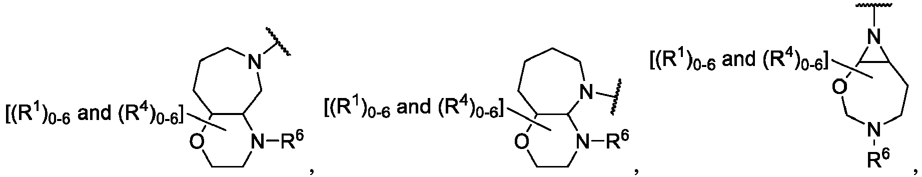

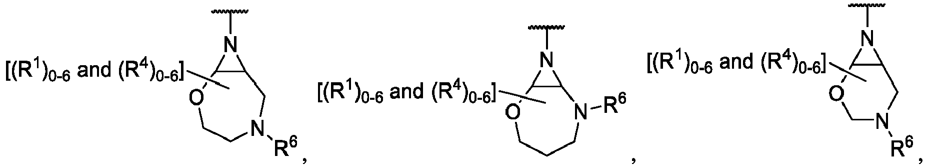

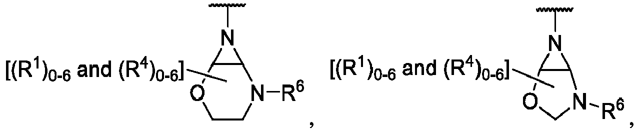

In some embodiments, R⁶ is 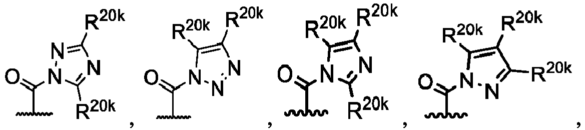 or 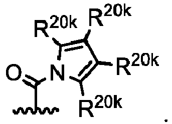 In some embodiments, R⁶ is 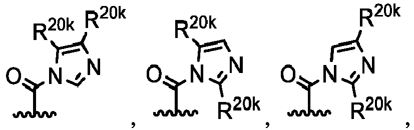 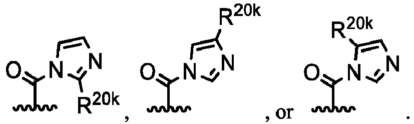 In some embodiments, R⁶ is 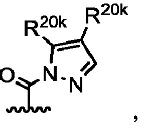 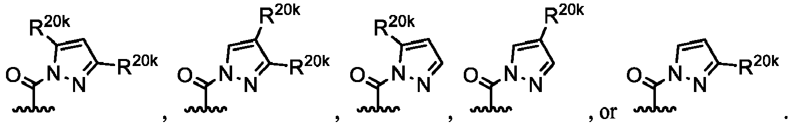 In some embodiments, R⁶ is 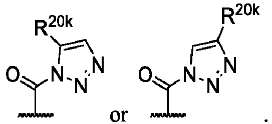 In some embodiments, R⁶ is 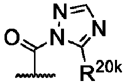 or 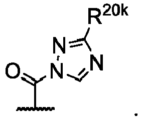 In some embodiments, R⁶ is 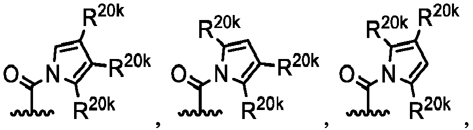 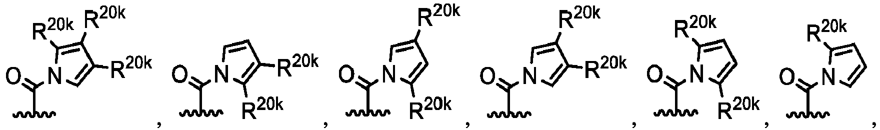 or

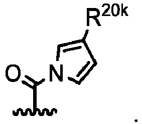

In embodiments, each R^{20k} is independently halogen. In embodiments, each R^{20k} is independently -CN. In embodiments, each R^{20k} is independently oxo. In embodiments, each R^{20k} is independently C₁₋₆alkyl. In embodiments, each R^{20k} is independently C₂₋₆alkenyl. In embodiments, each R^{20k} is independently C₂₋₆alkynyl. In embodiments, each R^{20k} is independently C₃₋₁₀cycloalkyl. In embodiments, each R^{20k} is independently -CH₂-C_{3- 10}cycloalkyl. In embodiments, each R^{20k} is independently C₂₋₉heterocycloalkyl. In embodiments, each R^{20k} is independently -CH₂-C₂₋₉heterocycloalkyl. In embodiments, each R^{20k} is independently C₆₋₁₀aryl. In embodiments, each R^{20k} is independently -CH₂-C₆₋₁₀aryl. In embodiments, each R^{20k} is independently -CH₂-C₁₋₉heteroaryl. In embodiments, each R^{20k} is independently C₁₋₉heteroaryl. In embodiments, each R^{20k} is independently C₁₋₆alkyl optionally substituted with one, two, or three groups independently selected from halogen, oxo, -CN, C₁₋₆allcyl, C_{1- 6}haloalkyl, C₁₋₆alkoxy, C₁₋₆haloalkoxy, -OR²¹, -SR²¹, -N(R²²)(R²³), -C(O)OR²², -C(O)N(R²²)(R²³),-C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³), and -OC(O)R²⁵.In embodiments, each R^{20k} is independently C₂₋₆alkenyl optionally substituted with one, two, or three groups independently selected from halogen, oxo, -CN, C₁₋₆allcyl, C₁₋₆haloalkyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵.In embodiments, each R^{20k} is independently C₂₋₆alkynyl optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵. In embodiments, each R^{20k} is independently C₃₋₁₀cycloalkyl optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵.In embodiments, each R^{20k} is independently -CH₂-C₃₋₁₀cycloalkyl optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, - N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵. In embodiments, each R^{20k} is independently C₂₋₉heterocycloalkyl optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, - N(R²²)(R²³), -C(O)OR²², -C(O)N(R²²)(R²³), ^{_}C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵.In embodiments, each R^{20k} is independently -CH₂-C₂₋₉heterocycloalkyl optionally substituted with one, two, or three groups independently selected from halogen, oxo, -CN, C₁₋₆alkyl, C₁₋₆haloallcyl, C₁₋₆alkoxy, C₁₋₆haloalkoxy, -OR²¹, - SR²¹, -N(R²²)(R²³), -C(O)OR²², -C(O)N(R²²)(R²³), -C(O)C(O)N(R²²)(R²³), -OC(O)N(R²²)(R²³), _ N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, _ S(O)₂N(R²²)(R²³), and -OC(O)R²⁵. In embodiments, each R^{20k} is independently C₆₋₁₀aryl optionally substituted with one, two, or three groups independently selected from halogen, oxo, -CN, C₁₋₆alkyl, C₁₋₆haloalkyl, C₁₋₆alkoxy, C_{1- 6}haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), and -OC(O)R²⁵. In embodiments, each R^{20k} is independently -CH₂-C₆₋₁₀aryl optionally substituted with one, two, or three groups independently selected from halogen, oxo, -CN, C₁₋₆alkyl, C₁₋₆haloallcyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, - S(O)₂R²⁵, -S(O)₂N(R²²)(R²³), and -OC(O)R²⁵.In embodiments, each R^{20k} is independently -CH₂-C₁₋₉heteroaryl optionally substituted with one, two, or three groups independently selected from halogen, oxo, -CN, C₁₋₆alkyl, C_{1- 6}haloalkyl, C₁₋₆alkoxy, C₁₋₆haloalkoxy, -OR²¹, -SR²¹, -N(R²²)(R²³), -C(O)OR²², -C(O)N(R²²)(R²³),-C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³), and -OC(O)R²⁵. In embodiments, each R^{20k} is independently C₁₋₉heteroaryl optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵.In embodiments, each R^{20k} is independently -OR²¹. In embodiments, each R^{20k} is independently -SR²¹. In embodiments, each R^{20k} is independently -N(R²²)(R²³). In embodiments, each R^{20k} is independently -C(O)OR²². In embodiments, each R^{20k} is independently -C(O)N(R²²)(R²³). In embodiments, each R^{20k} is independently -C(O)C(O)N(R²²)(R²³). In embodiments, each R^{20k} is independently -OC(O)N(R²²)(R²³). In embodiments, each R^{20k} is independently - N(R²⁴)C(O)N(R²²)(R²³). In embodiments, each R^{20k} is independently -N(R²⁴)C(O)OR²⁵. In embodiments, each R^{20k} is independently -N(R²⁴)C(O)R²⁵. In embodiments, each R^{20k} is independently -N(R²⁴)S(O)₂R²⁵. In embodiments, each R^{20k} is independently -C(O)R²⁵. In embodiments, each R^{20k} is independently -S(O)₂R²⁵. In embodiments, each R^{20k} is independently -S(O)₂N(R²²)(R²³). In embodiments, each R^{20k} is independently -OCH₂C(O)OR²². In embodiments, each R^{20k} is independently -OC(O)R²⁵. In embodiments, two R^{20k} bonded to the same or adjacent atoms are joined to form a C₃₋₁₀cycloalkyl optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), _ N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵.In embodiments, two R^{20k} bonded to the same or adjacent atoms are joined to form a C₂₋₉heterocycloalkyl optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵,-S(O)₂R²⁵, -S(O)₂N(R²²)(R²³), and -OC(O)R²⁵.In embodiments, two R^{20k} bonded to the same or adjacent atoms are joined to form a C₆₋₁₀aryl optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵,-N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵.In embodiments, two R^{20k} bonded to the same or adjacent atoms are joined to form a C₁₋₉heteroaryl optionally substituted with one, two, or three groups independently selected from halogen, oxo, -CN, C₁₋₆alkyl, C₁₋₆haloallcyl, C₁₋₆alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵. In embodiments, two R^{20k} bonded to the same or adjacent atoms are joined to form a C₃₋₁₀cycloalkyl. In embodiments, two R^{20k} bonded to the same or adjacent atoms are joined to form a C_{2- 9}heterocycloalkyl. In embodiments, two R^{20k} bonded to the same or adjacent atoms are joined to form a C₆₋₁₀aryl. In embodiments, two R^{20k} bonded to the same or adjacent atoms are joined to form a ntly -CH₂-C₆₋₁₀aryl. In embodiments, two R^{20k} bonded to the same or adjacent atoms are joined to form a C₁₋₉heteroaryl. In embodiments, each R^{20k} is independently -OH. In embodiments, each R^{20k} is independently -SH. In embodiments, each R^{20k} is independently -NH₂. In embodiments, each R^{20k} is independently -C(O)OH. In embodiments, each R^{20k} is independently -C(O)NH₂. In embodiments, each R^{20k} is independently -C(O)C(O)NH₂. In embodiments, each R^{20k} is independently -OC(O)NH₂. In embodiments, each R^{20k} is independently -NHC(O)NH₂. In embodiments, each R^{20k} is independently -NHC(O)OH. In embodiments, each R^{20k} is independently -NHC(O)H. In embodiments, each R^{20k} is independently -NHS(O)₂CH₃. In embodiments, each R^{20k} is independently -C(O)H. In embodiments, each R^{20k} is independently -S(O)₂CH₃. In embodiments, each R^{20k} is independently -S(O)₂N(R²². In embodiments, each R^{20k} is independently -OCH₂C(O)OR²². In embodiments, each R^{20k} is independently -OC(O)R²⁵.

In some embodiments R⁷ is 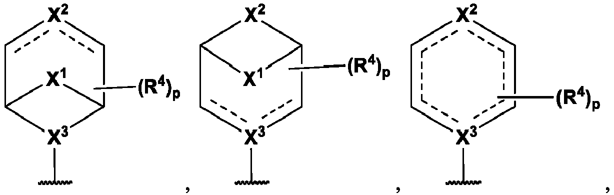 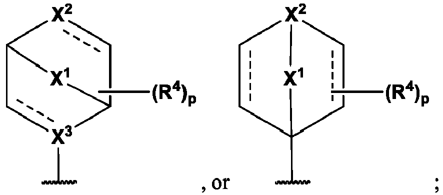
p is an integer from 0 to 12;
X¹ is selected from CH₂, C=N-OR⁴, C=NN(R⁴)(R⁶), C(O)N(R⁴), N(R⁴), N(R⁶), O, S, S(O), S(=O)(=NR⁴), S(O)₂N(R⁴), N(R⁴)S(O)N(R⁴), N(R⁴)S(O)₂N(R⁴), S(O)N(R⁴), OC(O)N(R⁴), N(R⁴)C(O)N(R⁴), S(O)₂, CH₂C=NN(R⁴)(R⁶), C(R⁴)(R⁴), ON-OR4, ONN(R⁴)(R⁴), CH₂C(R⁴)(R⁴), CH₂C(R⁴)(R⁴)CH₂, C(R⁴)(R⁴)C(R⁴)(R⁴)C(R⁴)(R⁴), C(R⁴)(R4)C=N-OR4, CH₂ONN(R⁴)(R4), C(R⁴)(R⁴)C(O)N(R⁴), C(R⁴)(R⁴)N(R⁴), C(R⁴)(R⁴)N(R⁶), C(R⁴)(R⁴)O, C(R⁴)(R⁴)OC(R⁴)(R⁴), C(R⁴)(R⁴)S, C(R⁴)(R⁴)SC(R⁴)(R⁴), C(R⁴)(R⁴)S(O), C(R⁴)(R⁴)S(O)C(R⁴)(R⁴), C(R⁴)(R⁴)S(O)₂C(R⁴)(R⁴), C(R⁴)(R⁴)S(=O)(=NR⁴), C(R⁴)(R⁴)S(O)₂N(R⁴), C(R⁴)(R⁴)N(R⁴)S(O)N(R⁴), C(R⁴)(R⁴)N(R⁴)S(O)₂N(R⁴), C(R⁴)(R⁴)S(O)N(R⁴), C(R⁴)(R⁴)OC(O)N(R⁴), C(R⁴)(R⁴)N(R⁴)C(O)N(R⁴), C(R⁴)(R⁴)S(O)₂, C=NN(R⁴)(R⁴)C(R⁴)(R⁴), C(O)N(R⁴)C(R⁴)(R⁴), S(O)₂N(R⁴)C(R⁴)(R⁴), S(O)N(R⁴)C(R⁴)(R⁴), and OC(O)N(R⁴)C(R⁴)(R⁴);
X² is selected from N, C, C(R⁶), C(R⁴), CH, N(R¹), N(R⁴), N(R⁶), O, S, S(O), C(R⁴)₂, CH₂, S(=O)(=NR⁴), S(O)₂; and
X³ is selected from N, C, and C(R⁴).

In embodiments R⁷ is

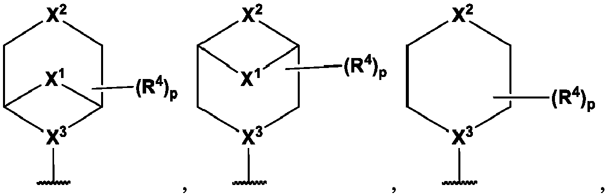

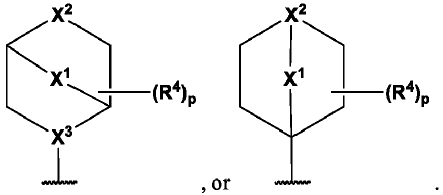

In embodiments R⁷ is 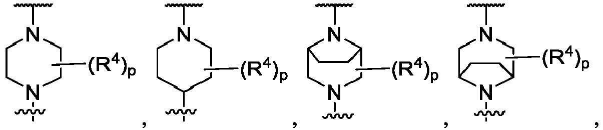 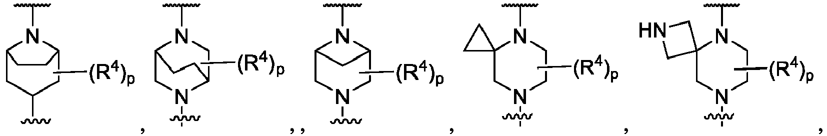 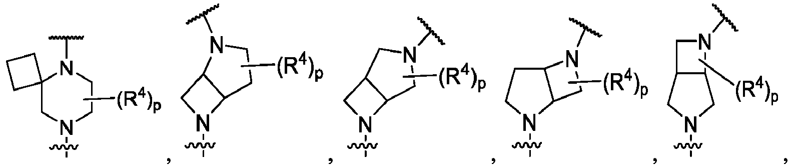 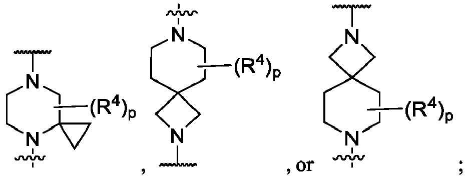 and p is an integer from 0 to 12.

In embodiments R⁷ is 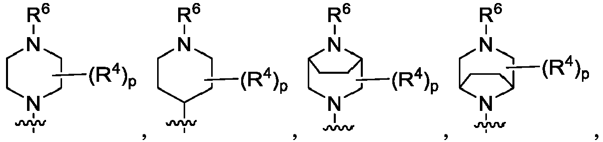 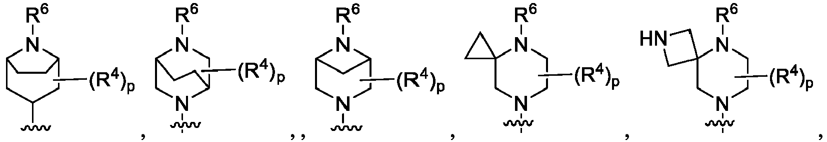 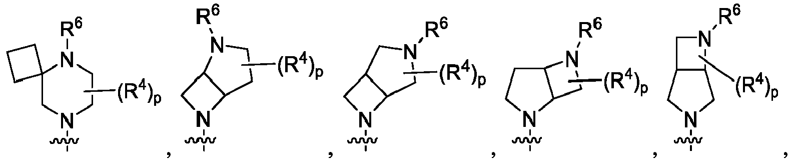 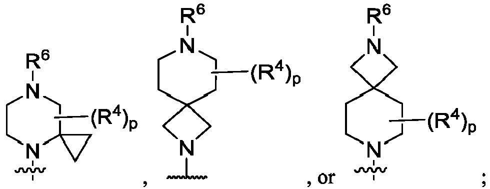 and p is an integer from 0 to 12.

In embodiments, R⁷ is

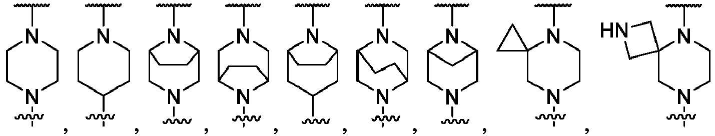

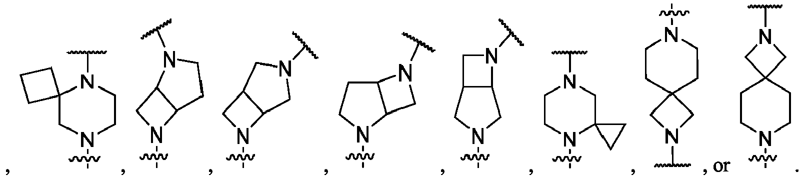

In some embodiments, R⁷ is 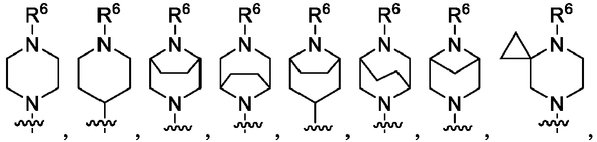 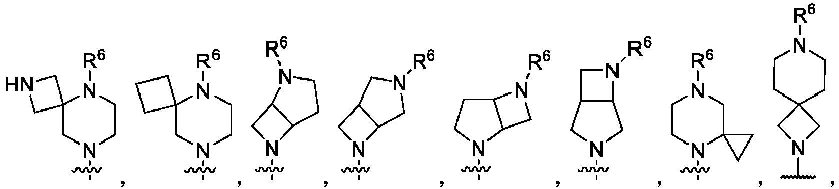 or

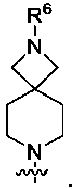

In some embodiments, R⁷ is

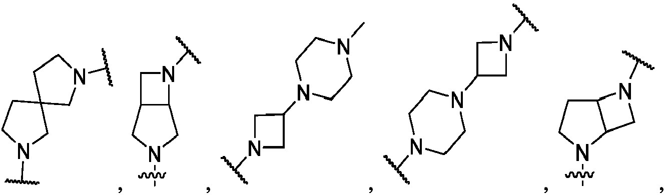

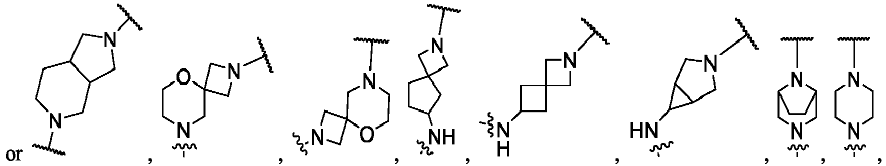

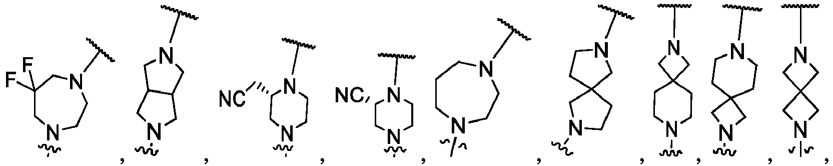

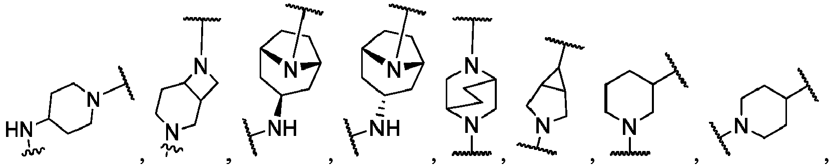

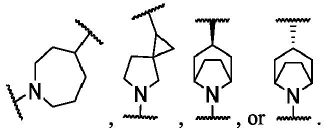

In some embodiments, R⁷ is 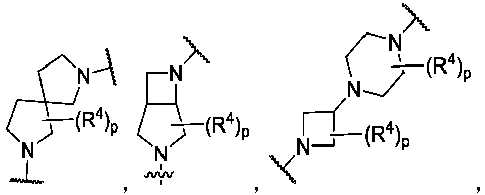 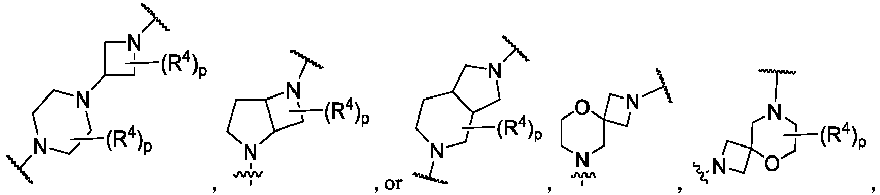 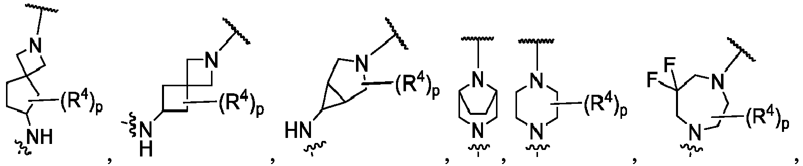 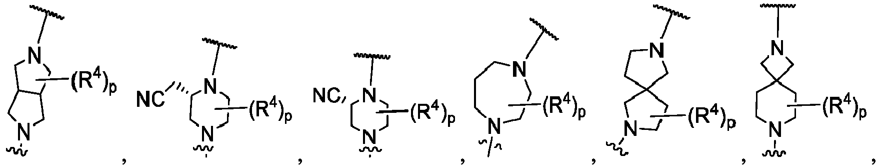 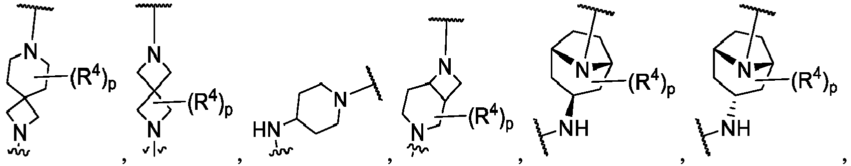 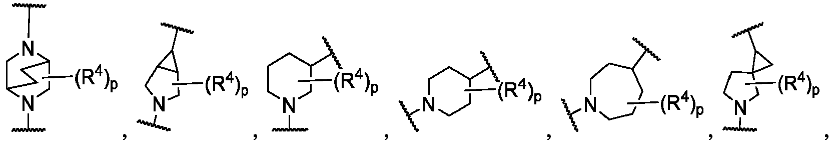 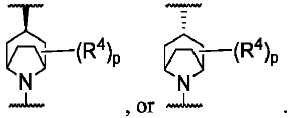 and p is independently an integer from 0 to 12.

In some embodiments R⁷ is 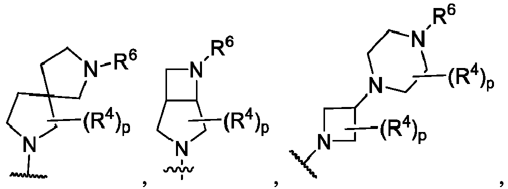 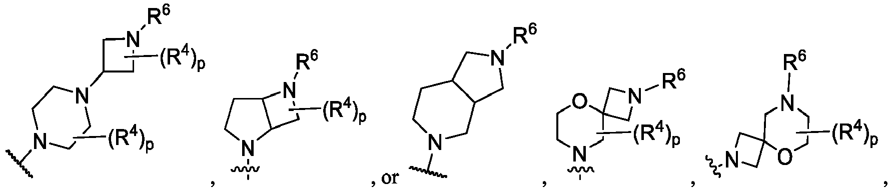 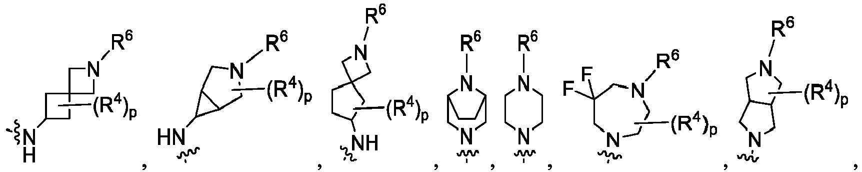 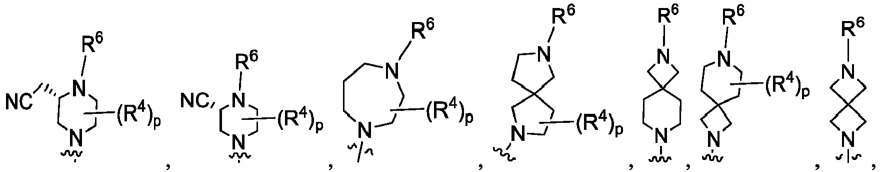 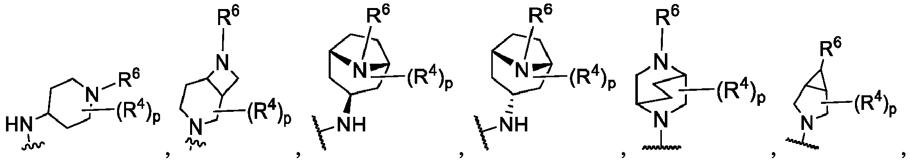 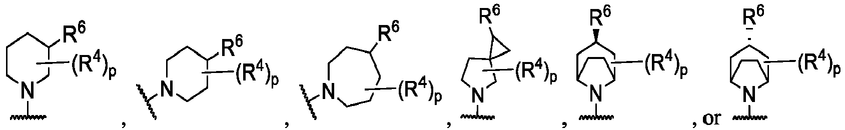 and p is independently an integer from 0 to 12.

In some embodiments R⁷ is

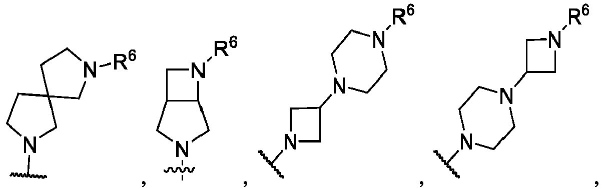

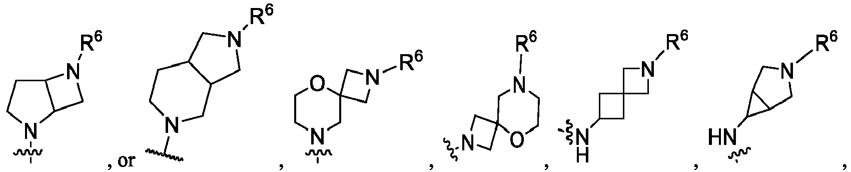

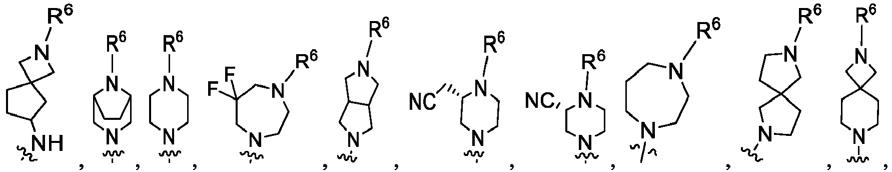

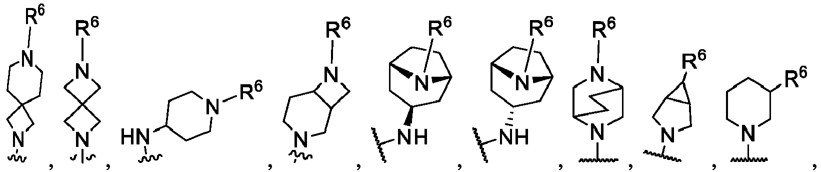

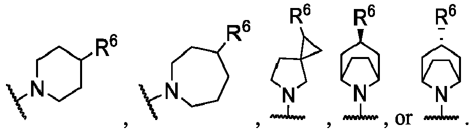

In some embodiments, R⁷ is

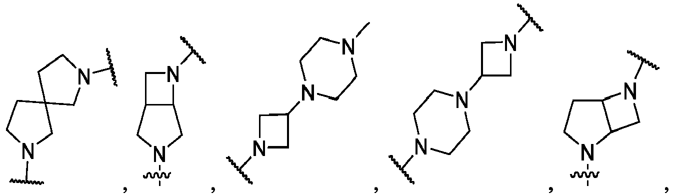

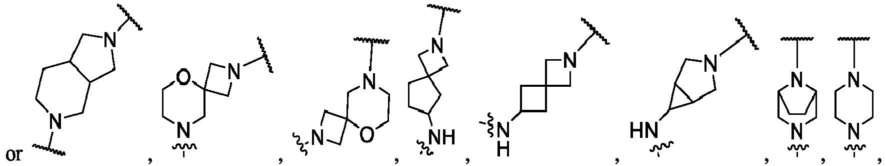

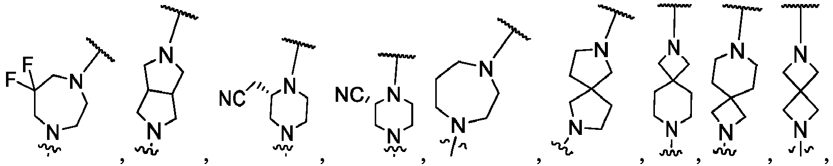

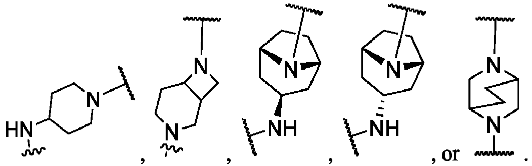

In some embodiments, R⁷ is 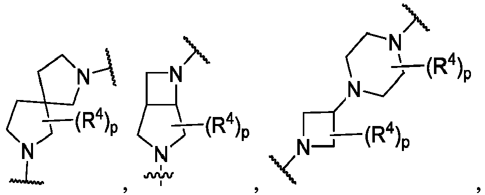 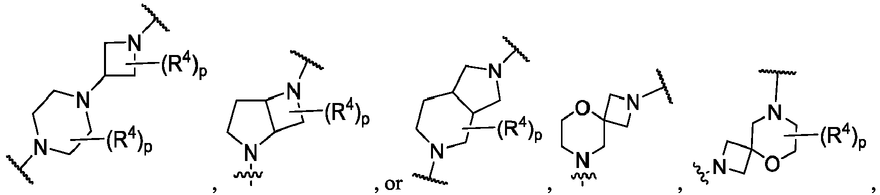 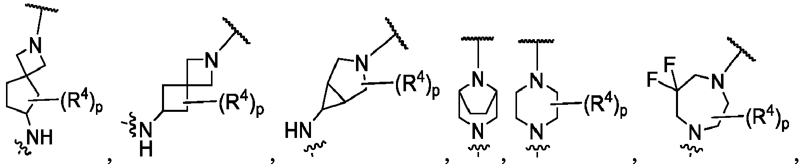 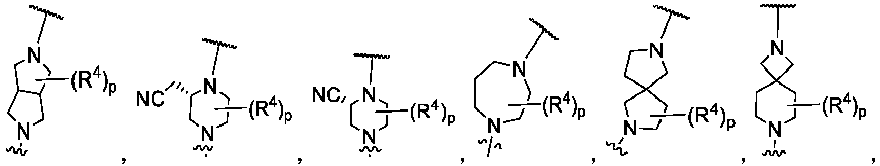 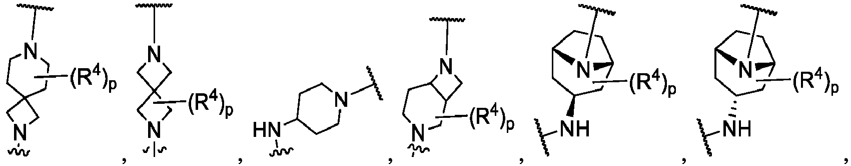 or 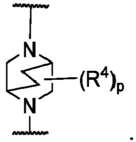 and p is independently an integer from 0 to 12.

In some embodiments R⁷ is 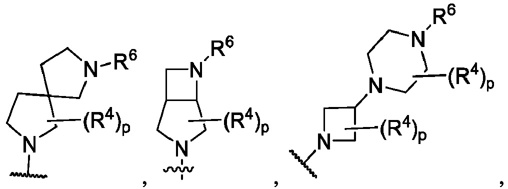 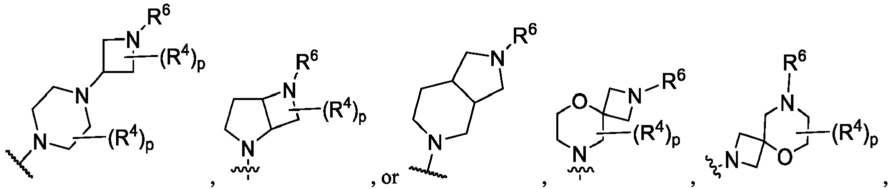 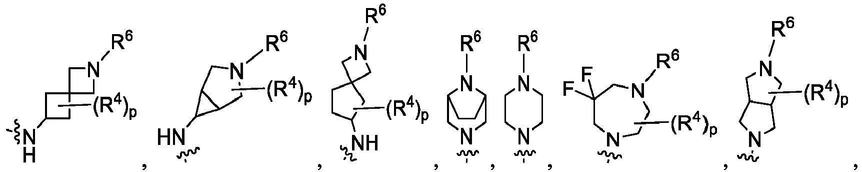 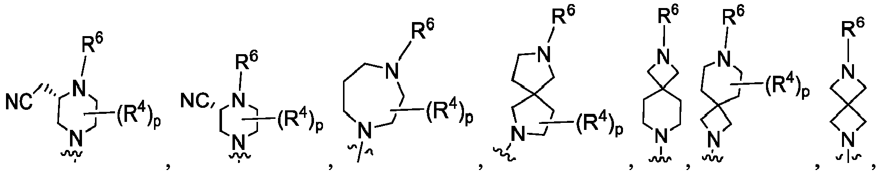 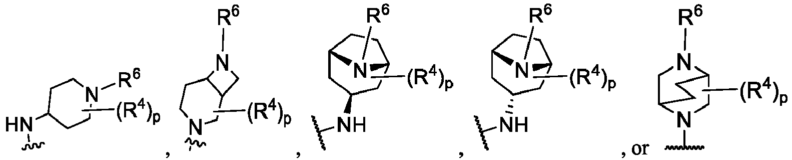 and p is independently an integer from 0 to 12.

In some embodiments R⁷ is

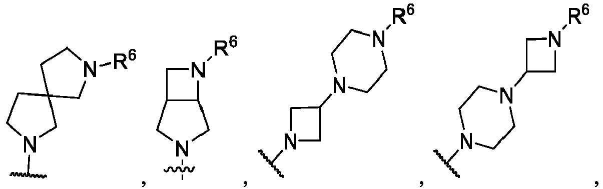

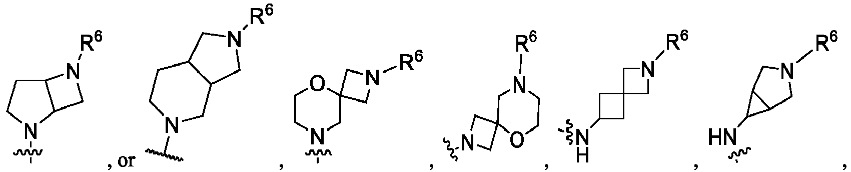

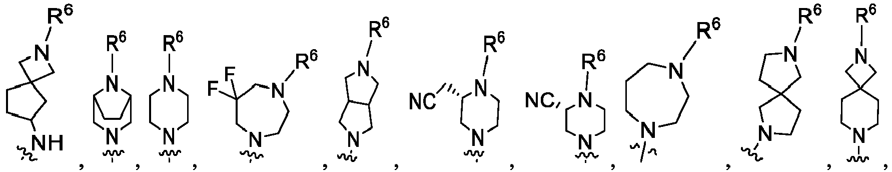

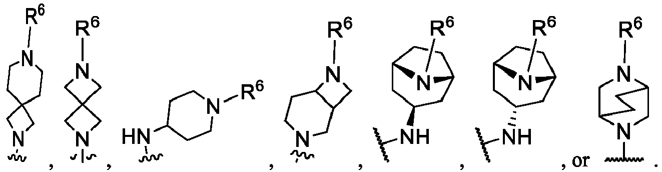

In some embodiments R⁶ is

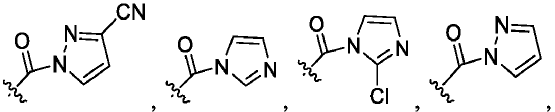

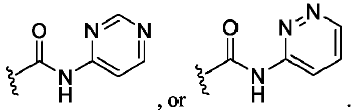

In some embodiments R⁶ is 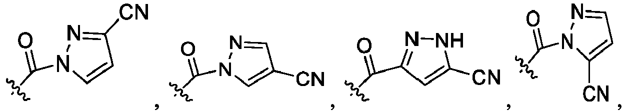 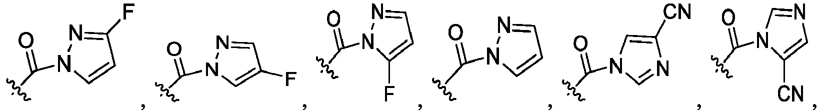 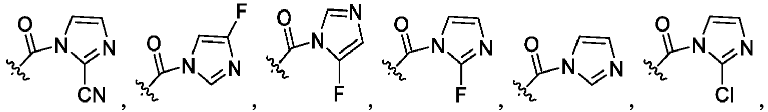 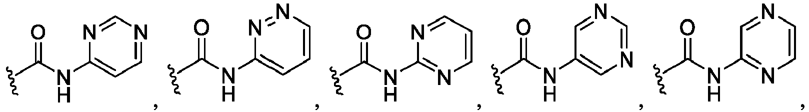 or

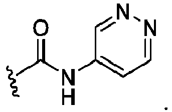

In some embodiments, R⁶ is

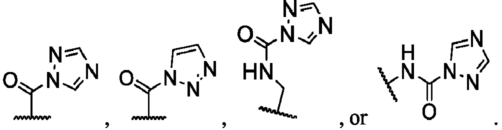

In some embodiments, R¹⁰ is

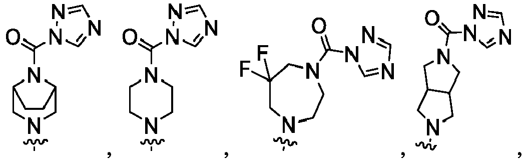

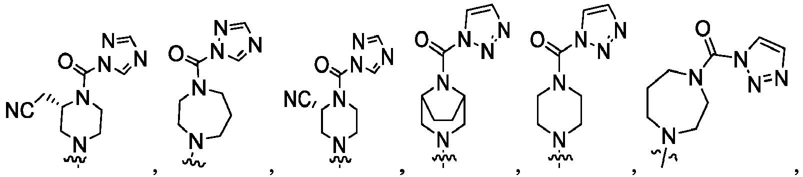

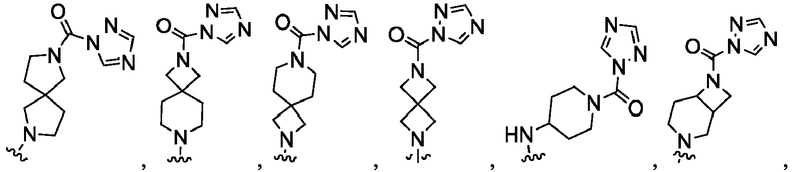

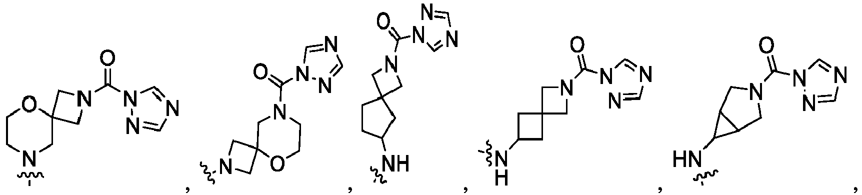

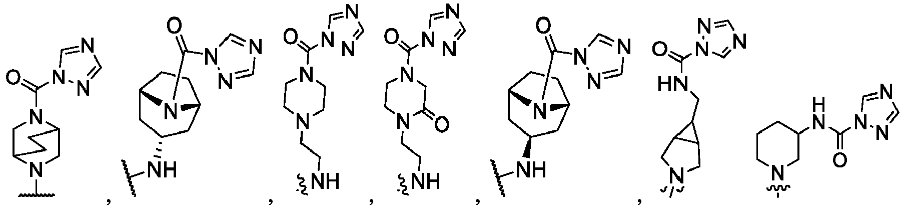

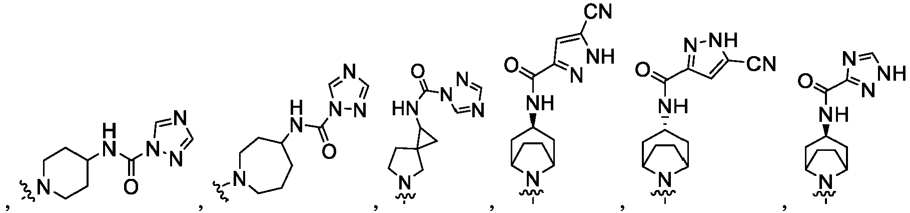

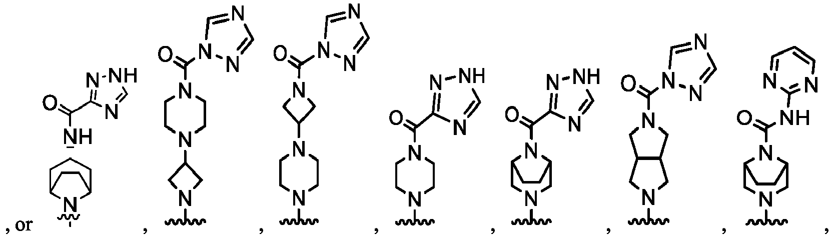

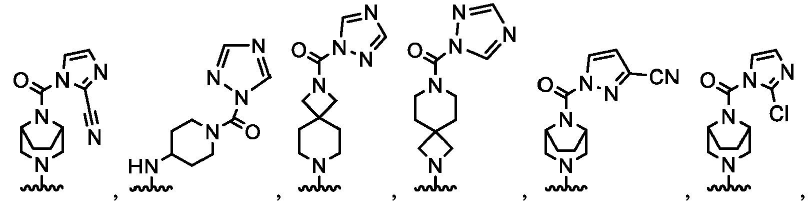

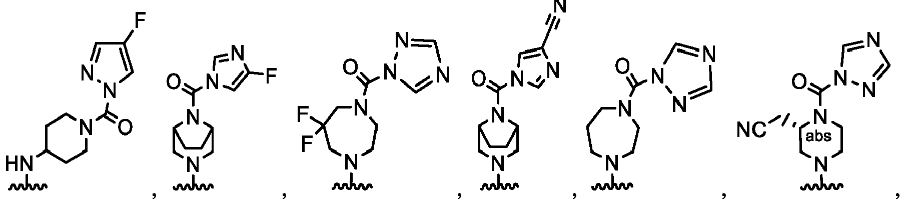

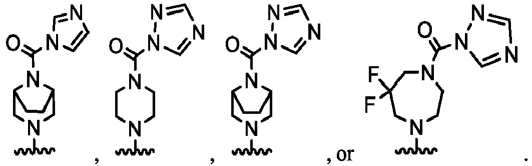

In some embodiments, R¹⁰ is

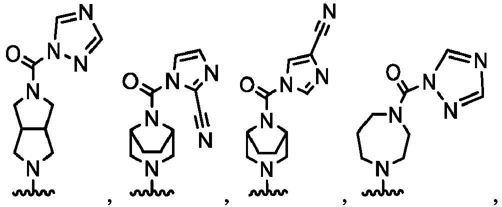

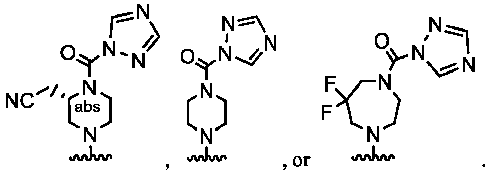

In some embodiments, R⁶ is

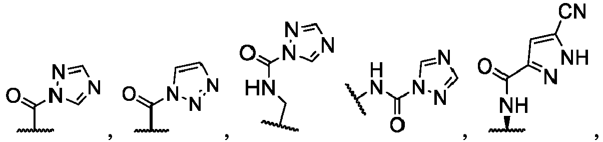

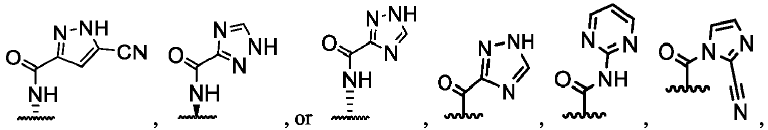

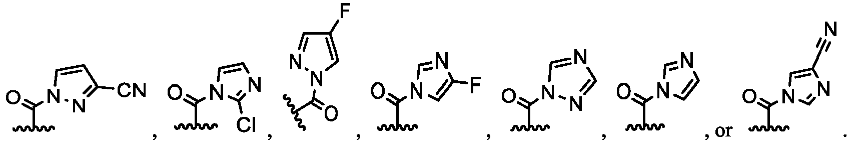

In some embodiments, R⁷ is

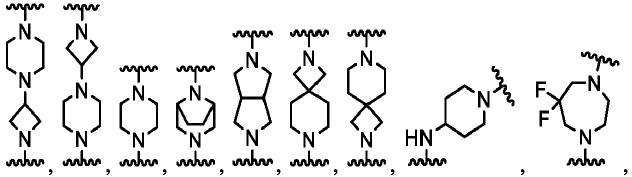

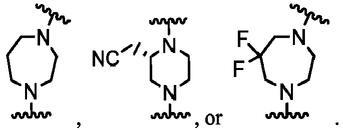

In some embodiments, R⁷ is

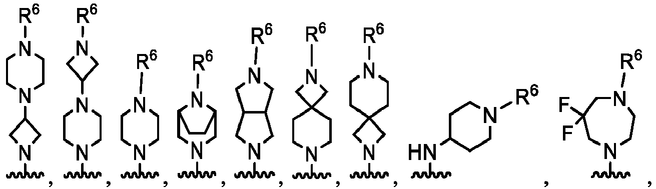

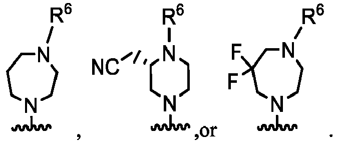

In some embodiments, R¹⁰ is

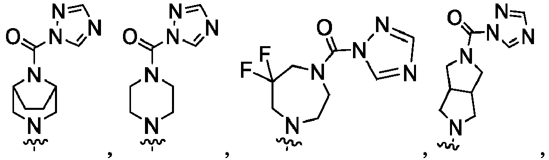

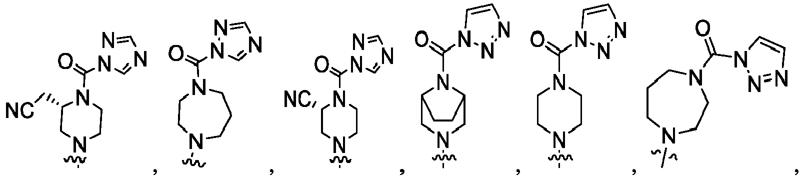

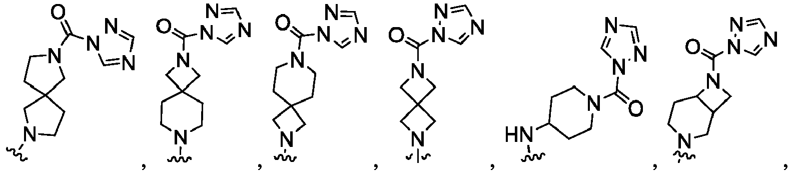

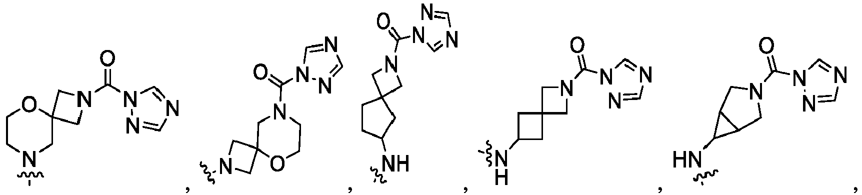

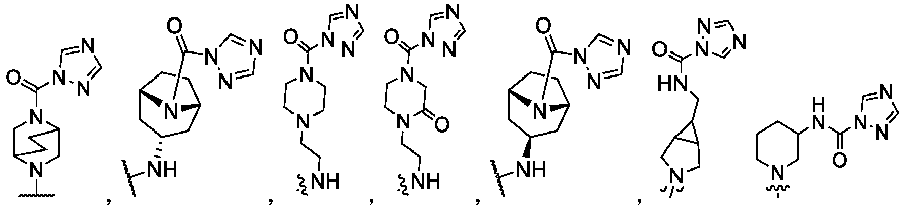

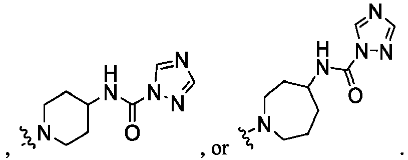

In embodiments, R¹⁰ is selected from 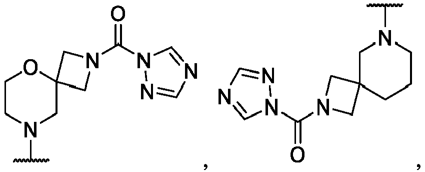 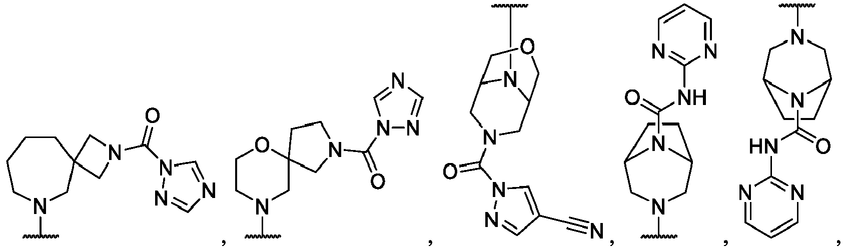 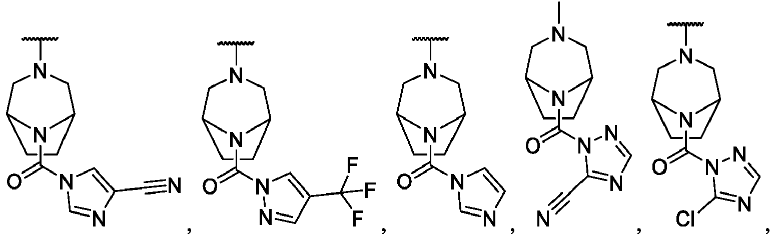 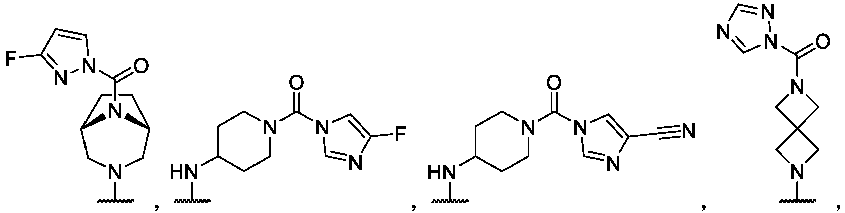 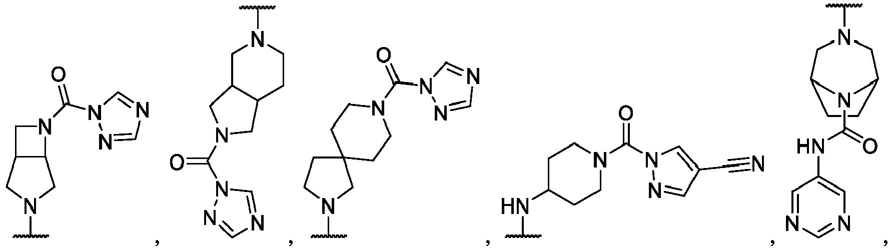 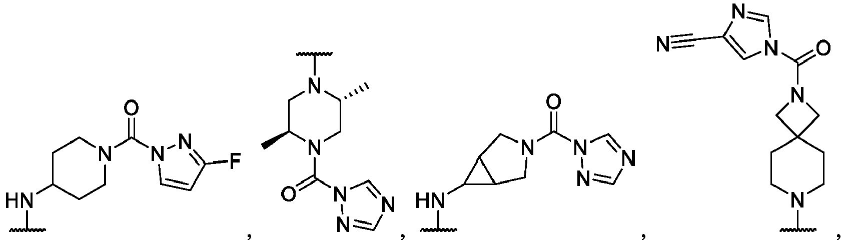 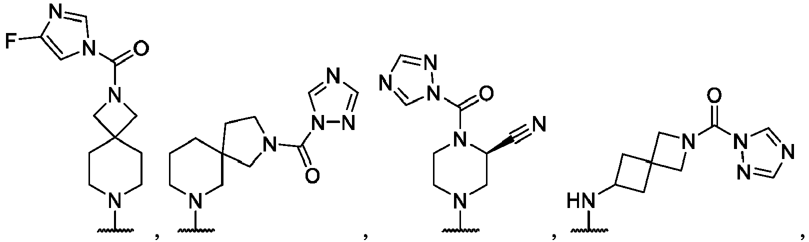 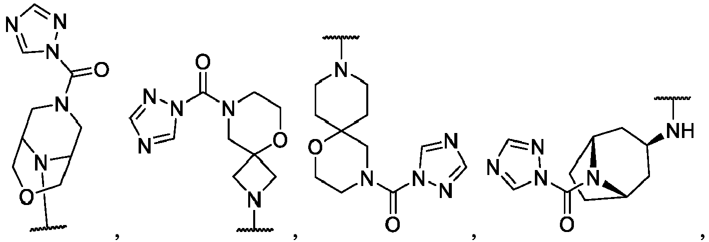 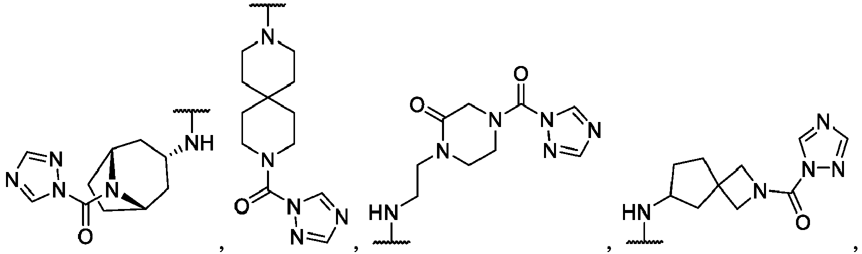 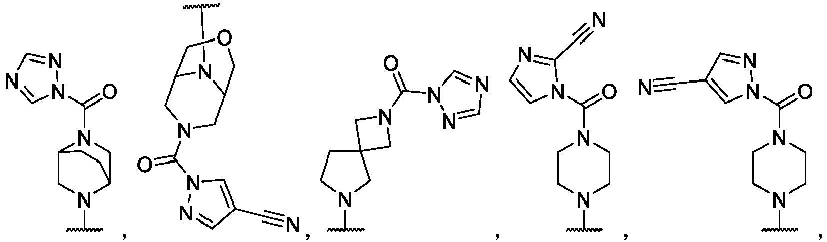 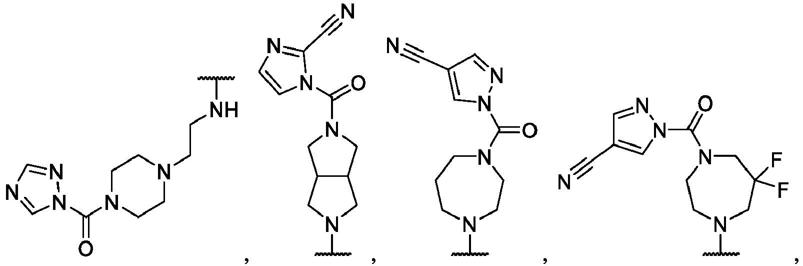 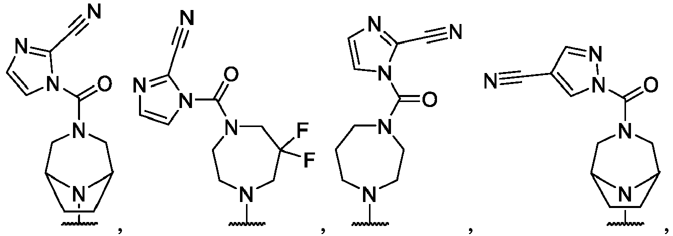 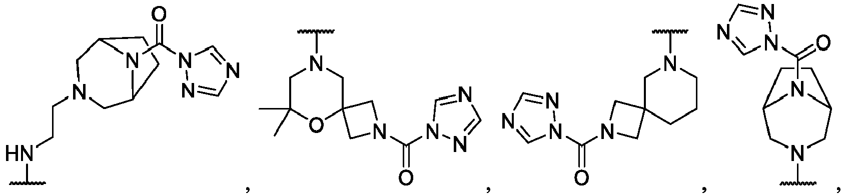 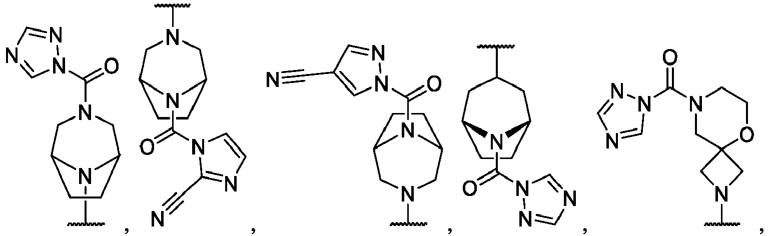 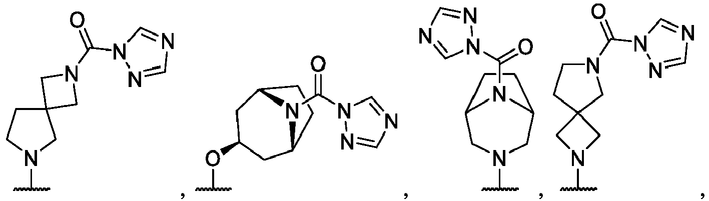 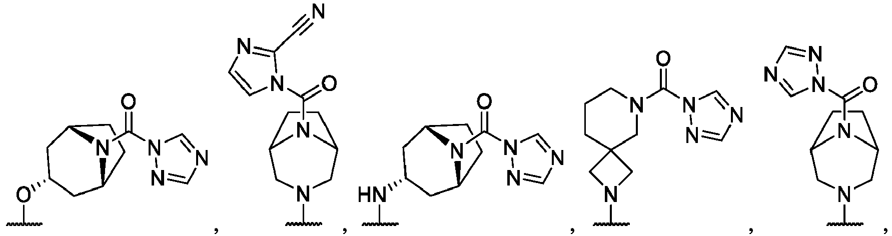 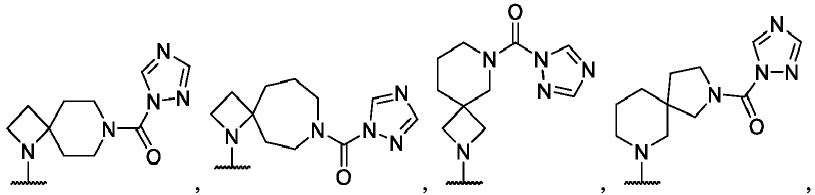 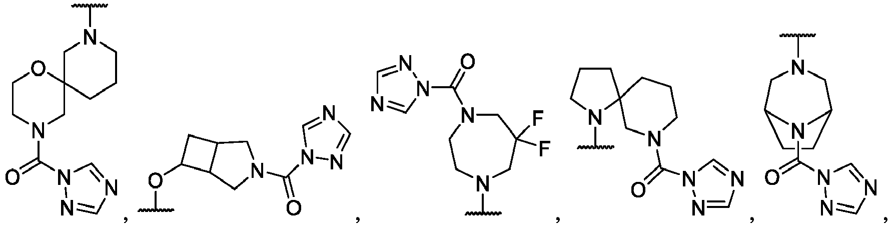 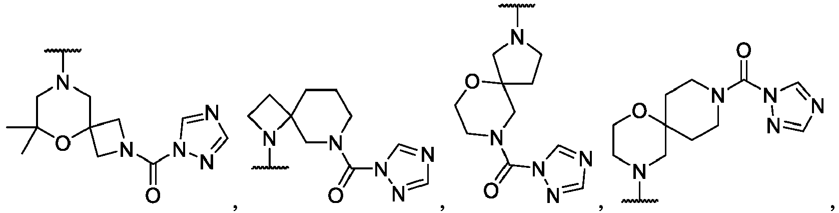 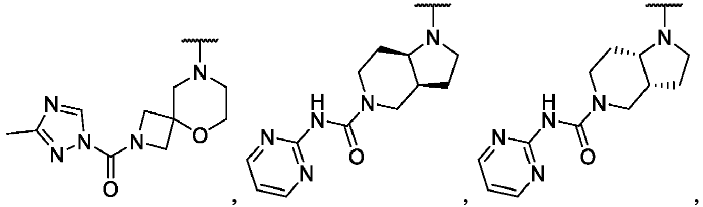 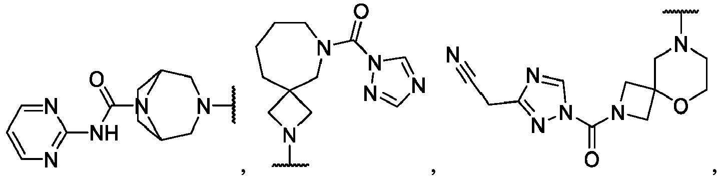 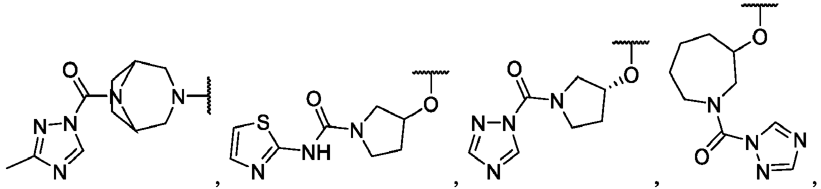 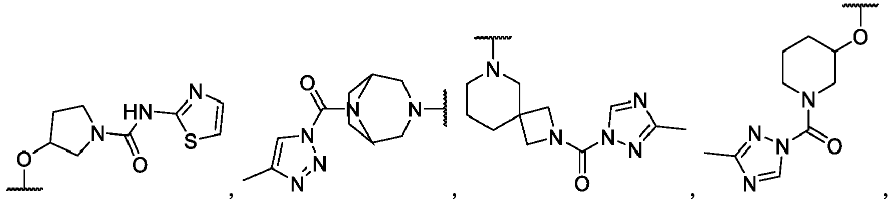 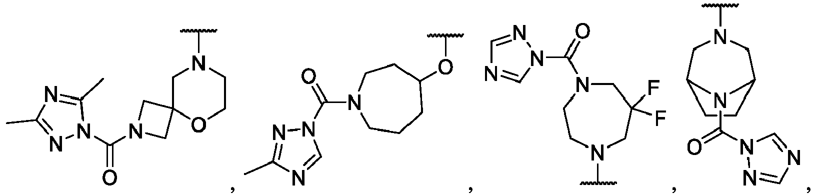 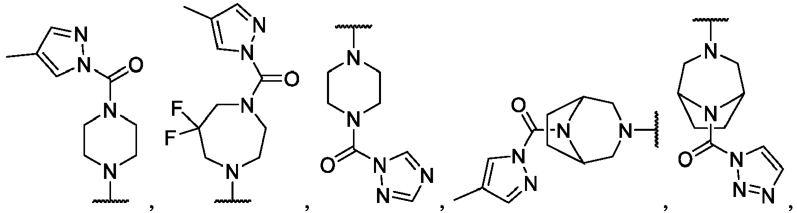 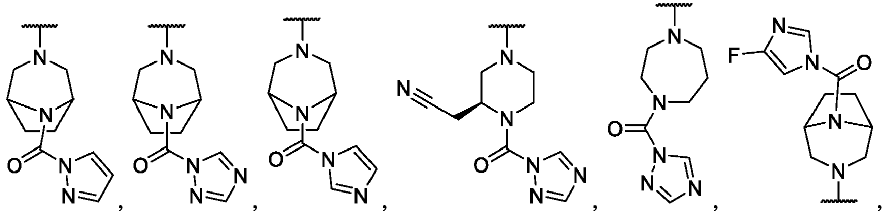 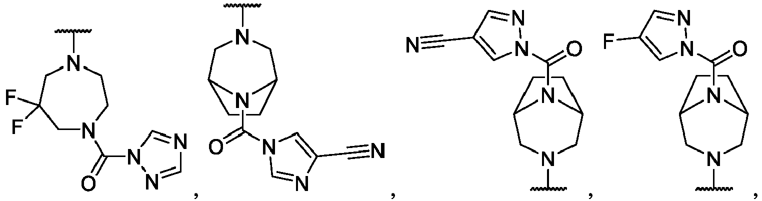 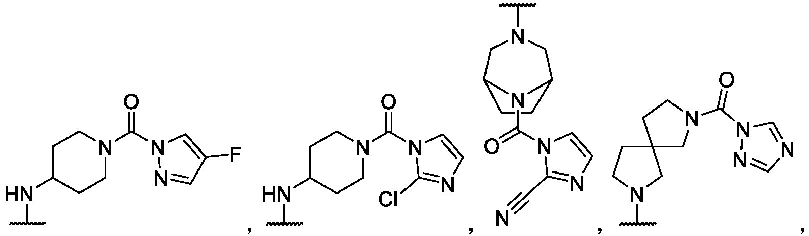 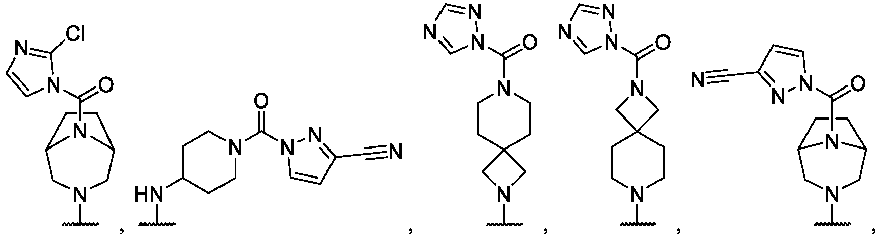 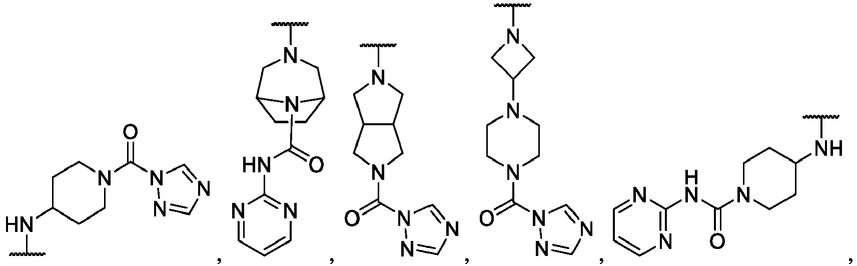 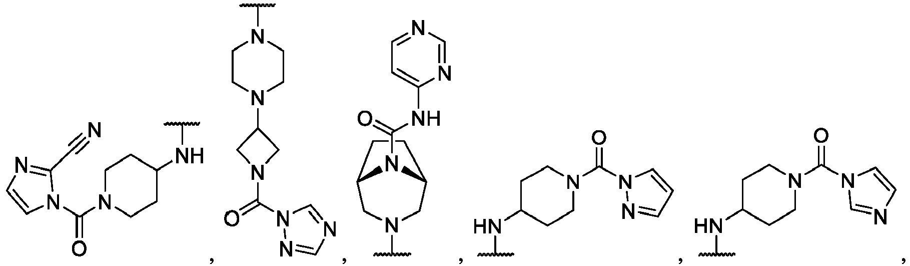 and

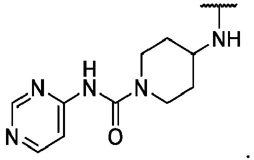

In embodiments of the modified Ras protein, complex, or compound (e.g., precursor compound), L² is selected from -C(O)-, -N(R¹²)C(O)-, -C(O)N(R¹²)-, -CH₂N(R¹²)C(O)-, -CH₂CH₂N(R¹²)C(O)-, and - CH₂CH₂CH₂N(R¹²)C(O)-. In embodiments of a modified Ras protein, complex, or compound (e.g., precursor compound), L² is selected from a bond, -C(O)NH-, -NHC(O)-, and -C(O)-.

In embodiments of the compound (e.g., precursor compound), L² is selected from a -C(O)-, -N(R¹²)C(O)-, - C(O)N(R¹²)-, -CH₂N(R¹²)C(O)-, -CH₂CH₂N(R¹²)C(O)-, and -CH₂CH₂CH₂N(R¹²)C(O)-. In embodiments of the compound (e.g., precursor compound), L² is selected from a bond, -C(O)NH-, -NHC(O)-, and -C(O)-. In embodiments of the compound (e.g., precursor compound), R⁵ is a 5-6 membered heteroaryl optionally substituted with one, two, three, or four R^{20k}, wherein the heteroaryl comprises one, two, three, or four, ring nitrogen atoms and further wherein when R⁵ is directly bonded to a C(O), S(O), or S(O)₂ of L², L² is directly bonded to an N atom of R⁵. In embodiments of the compound (e.g., precursor compound), R⁵ is a 5 membered heteroaryl optionally substituted with one, two, three, or four R^{20k}, wherein the heteroaryl comprises one, two, or three ring nitrogen atoms and further wherein when R⁵ is directly bonded to a C(O), S(O), or S(O)₂ of L², L² is directly bonded to an N atom of R⁵. In embodiments of the compound (e.g., precursor compound), R⁵ is a pyrrolyl, pyrazolyl, imidazolyl, or triazolyl optionally substituted with one, two, three, or four R^{20k}, wherein when R⁵ is directly bonded to a C(O), S(O), or S(O)₂ of L², L² is directly bonded to an N atom of R⁵. In embodiments of the compound (e.g., precursor compound), R⁵ is a triazolyl optionally substituted with one, two, three, or four R^{20k}, wherein when R⁵ is directly bonded to a C(O), S(O), or S(O)₂ of L², L² is directly bonded to an N atom of R⁵. In embodiments of the compound (e.g., precursor compound), R⁵ is a pyrrolyl, pyrazolyl, imidazolyl, oxazolyl, isoxazolyl, thiazolyl, isothiazolyl, pyridyl, pyridazinyl, pyrimidinyl, pyrazinyl, triazinyl, oxadiazolyl, thiadiazolyl, or triazolyl optionally substituted with one, two, three, or four R^{20k}, wherein when R⁵ is directly bonded to a C(O), S(O), or S(O)₂ of L², L² is directly bonded to an N atom of R⁵.

In embodiments of the compound (e.g., precursor compound), L² is a -C(O)-, -N(R¹²)C(O)-, or -C(O)N(R¹²)-; and R⁵ is a 5-6 membered heteroaryl optionally substituted with one, two, three, or four R^{20k}, wherein the heteroaryl comprises one, two, three, or four, ring nitrogen atoms and further wherein when R⁵ is directly bonded to a C(O) of L², L² is directly bonded to an N atom of R⁵. In embodiments of the compound (e.g., precursor compound), L² is a - C(O)-, -N(R¹²)C(O)-, or -C(O)N(R¹²)-; and R⁵ is a 5 membered heteroaryl optionally substituted with one, two, three, or four R^{20k}, wherein the heteroaryl comprises one, two, or three ring nitrogen atoms and further wherein when R⁵ is directly bonded to a C(O) of L², L² is directly bonded to an N atom of R⁵. In embodiments of the compound (e.g., precursor compound), L² is a -C(O)-, -N(R¹²)C(O)-, or -C(O)N(R¹²)-; and R⁵ is a pyrrolyl, pyrazolyl, imidazolyl, or triazolyl optionally substituted with one, two, three, or four R^{20k}, wherein when R⁵ is directly bonded to a C(O) of L², L² is directly bonded to an N atom of R⁵. In embodiments of the compound (e.g., precursor compound), L² is a -C(O)-, -N(R¹²)C(O)-, or -C(O)N(R¹²)-; and R⁵ is a triazolyl optionally substituted with one, two, three, or four R^{20k}, wherein when R⁵ is directly bonded to a C(O) of L², L² is directly bonded to an N atom of R⁵. In embodiments of the compound (e.g., precursor compound), L² is a -C(O)-, -N(R¹²)C(O)-, or - C(O)N(R¹²)-; and R⁵ is a pyrrolyl, pyrazolyl, imidazolyl, oxazolyl, isoxazolyl, thiazolyl, isothiazolyl, pyridyl, pyridazinyl, pyrimidinyl, pyrazinyl, triazinyl, oxadiazolyl, thiadiazolyl, or triazolyl optionally substituted with one, two, three, or four R^{20k}, wherein when R⁵ is directly bonded to a C(O) of L², L² is directly bonded to an N atom of R5.

In embodiments, R⁵ is selected from 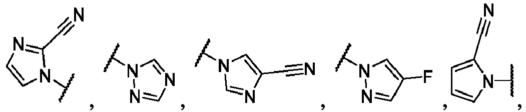 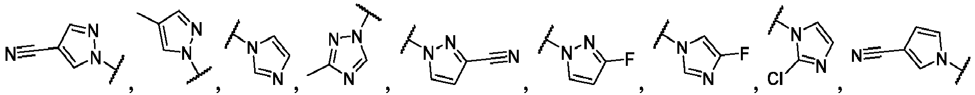 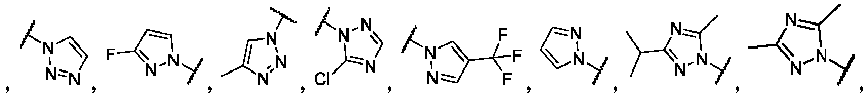 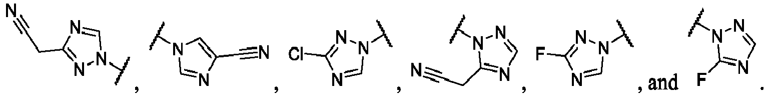 In embodiments, R⁵ is selected from

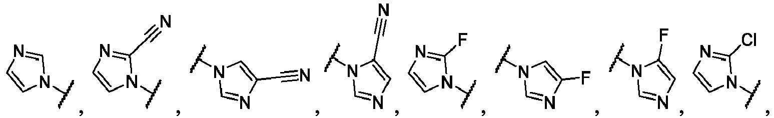

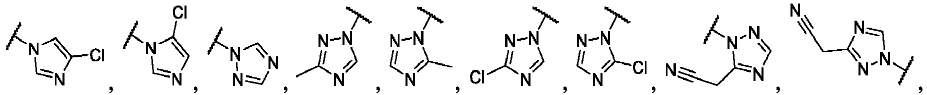

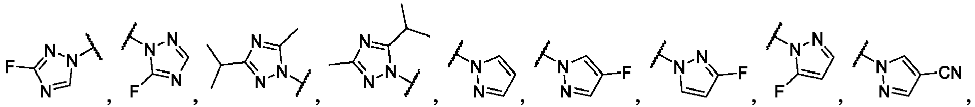

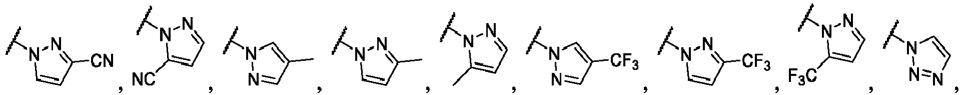

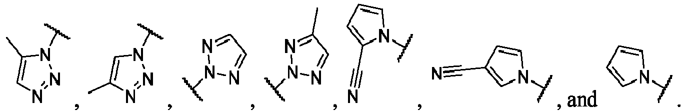

In embodiments, R¹⁰ is selected from

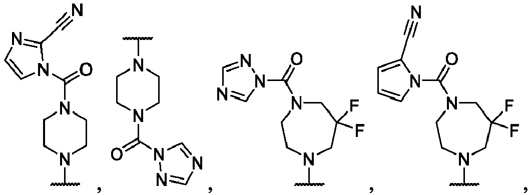

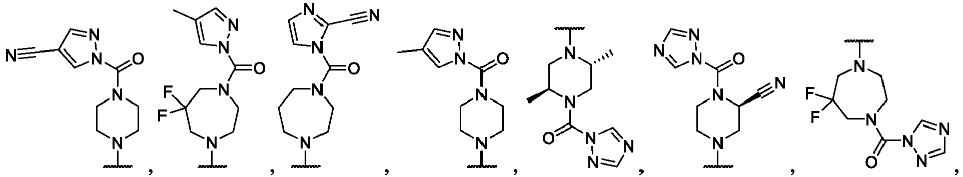

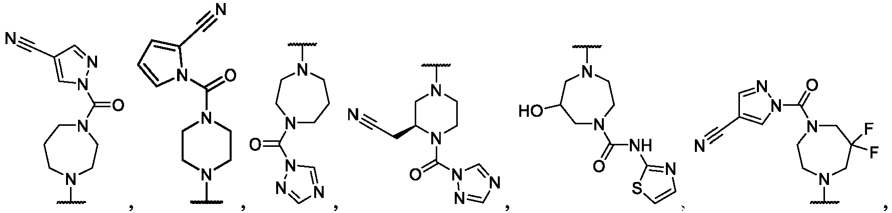

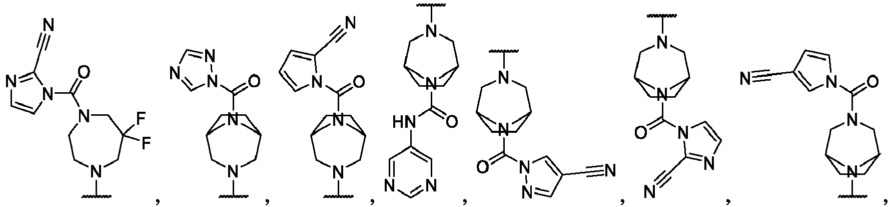

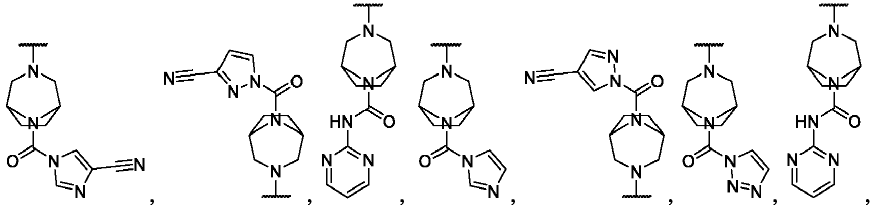

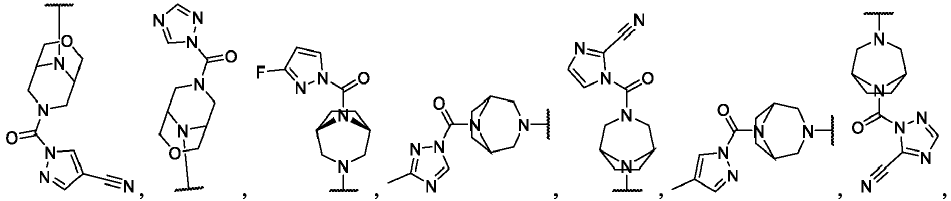

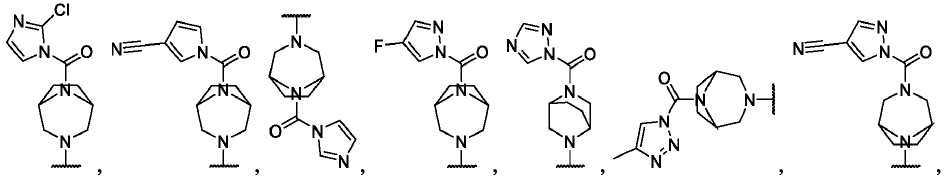

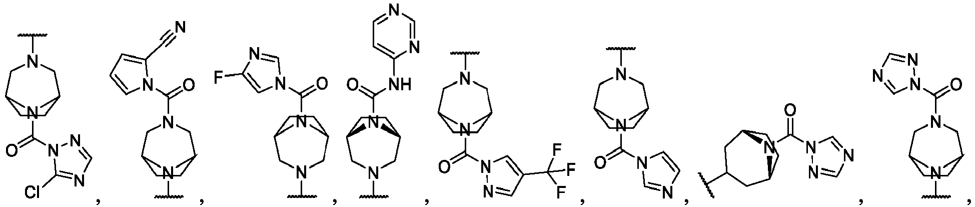

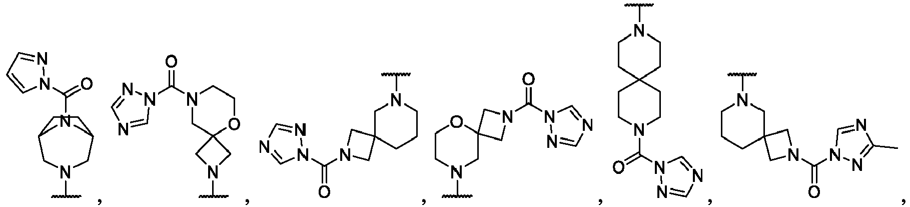

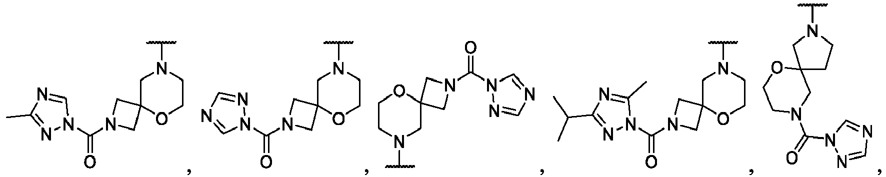

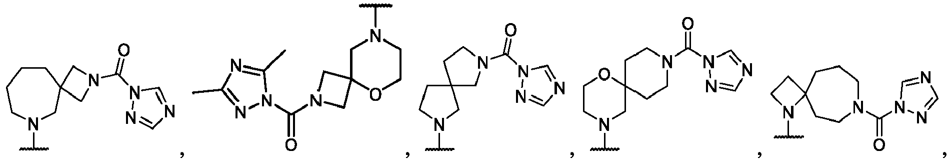

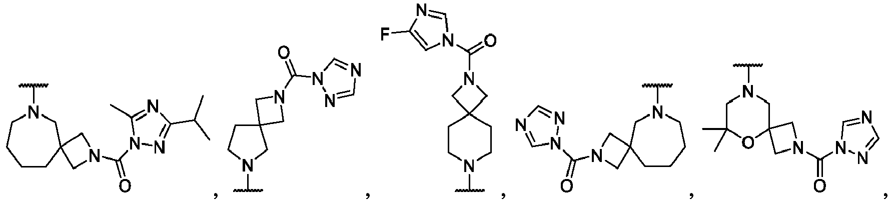

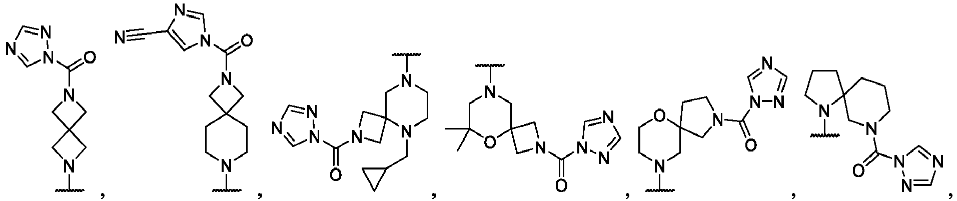

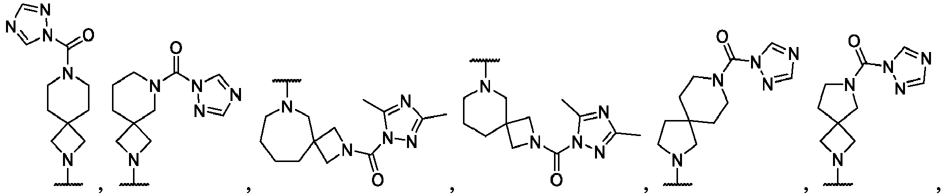

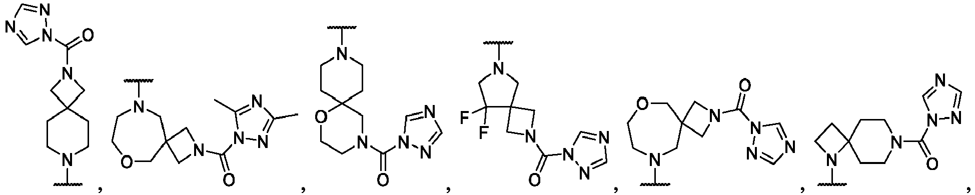

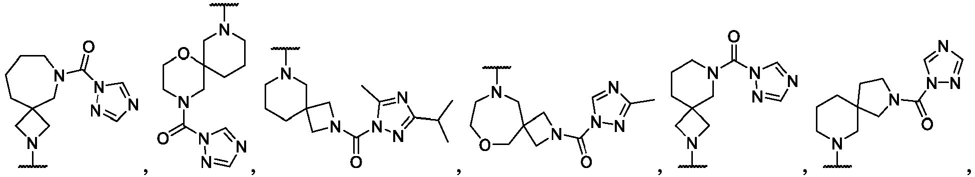

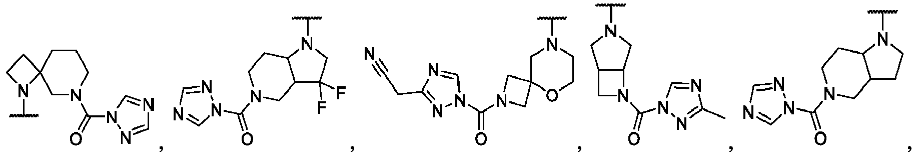

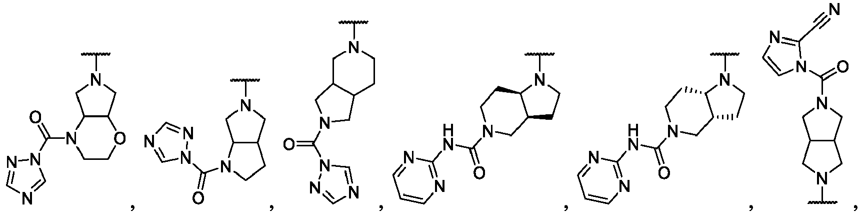

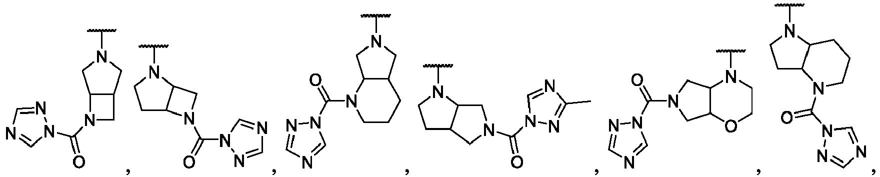

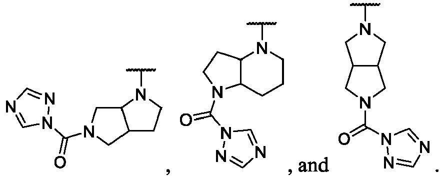

In embodiments, R⁷ is selected from

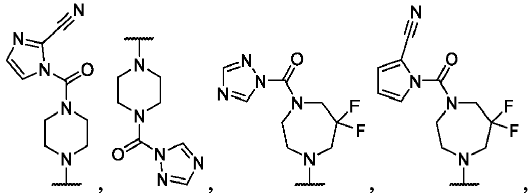

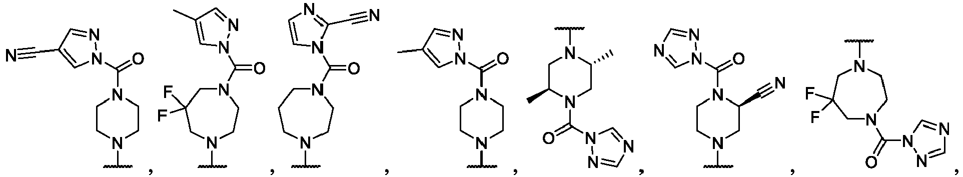

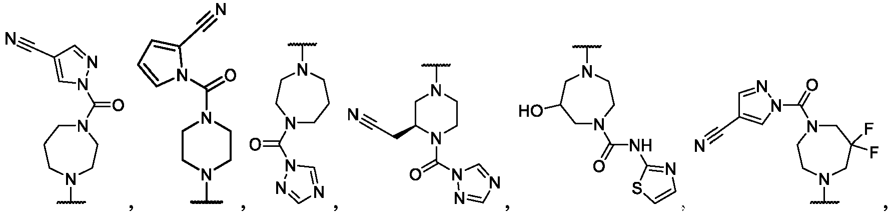

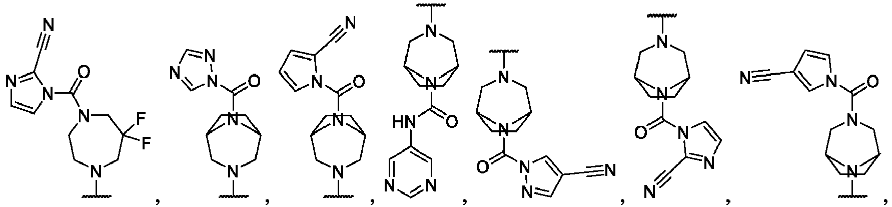

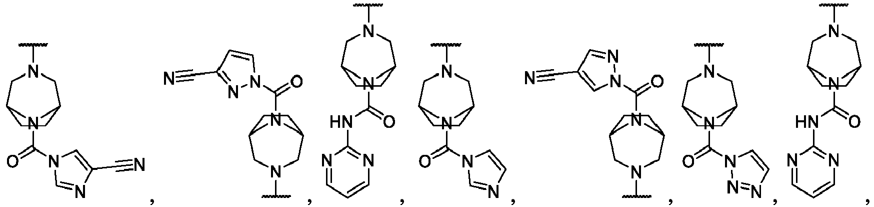

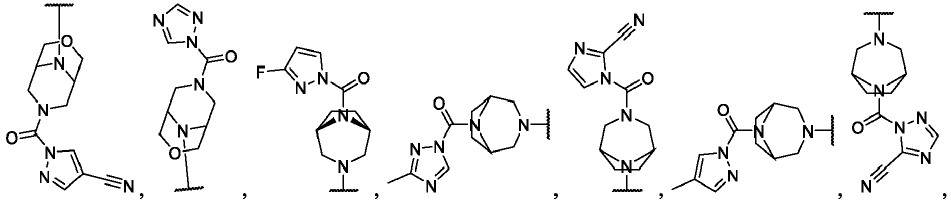

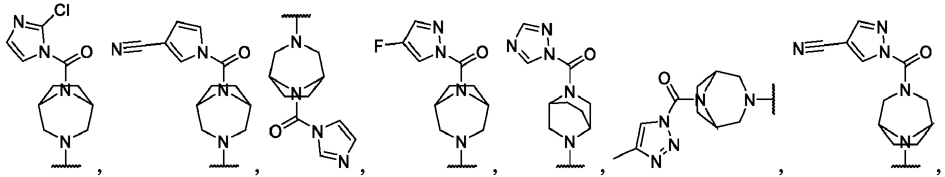

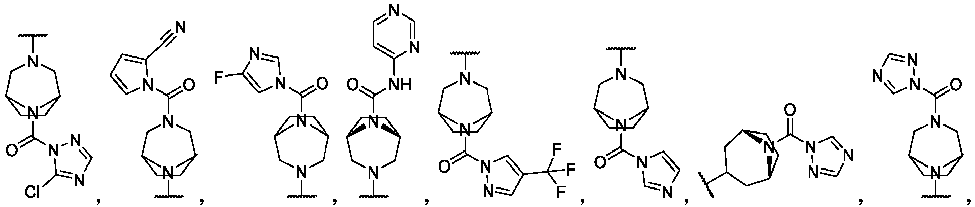

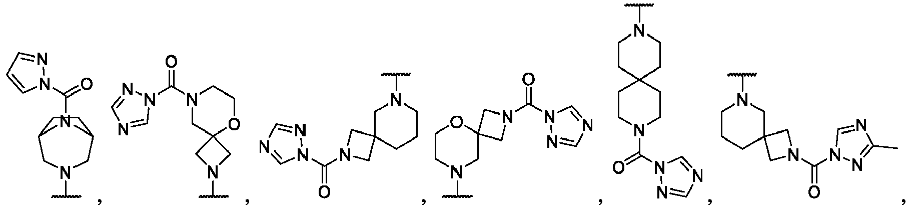

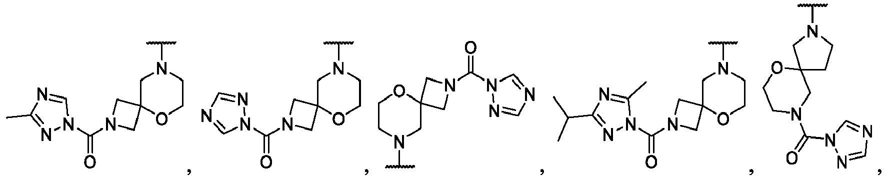

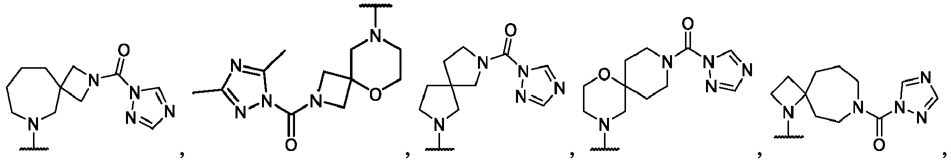

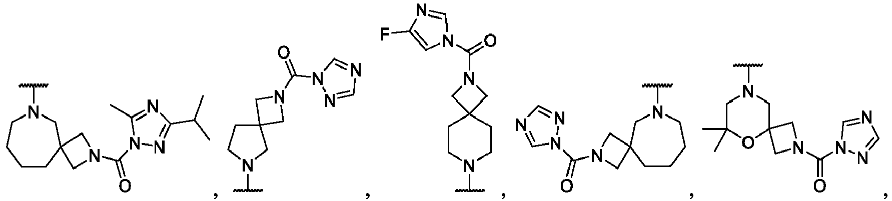

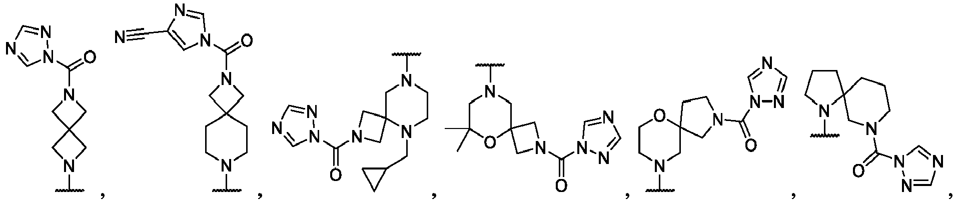

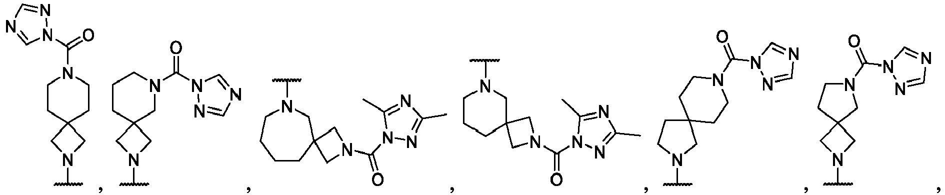

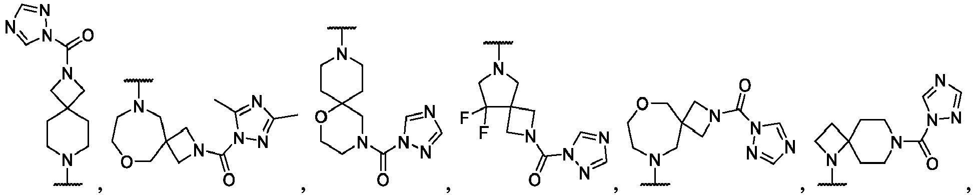

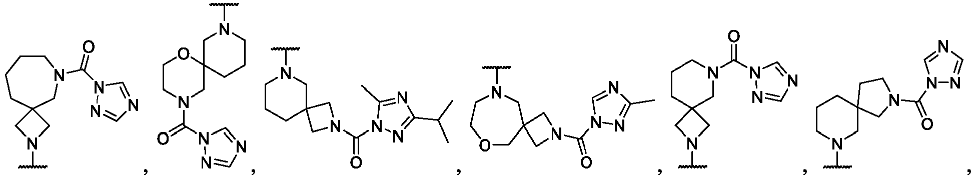

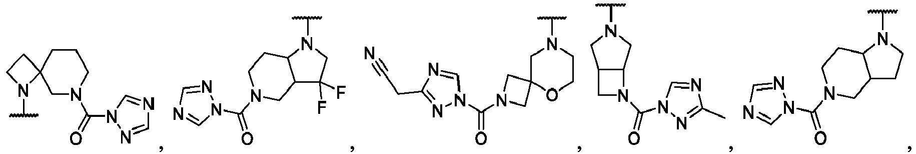

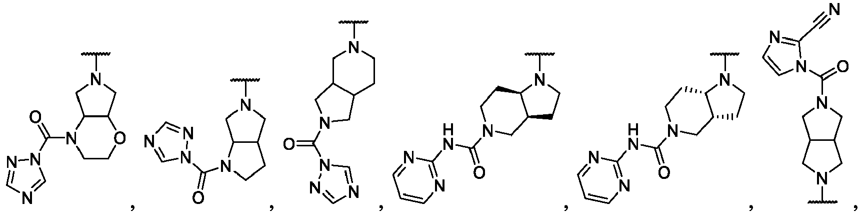

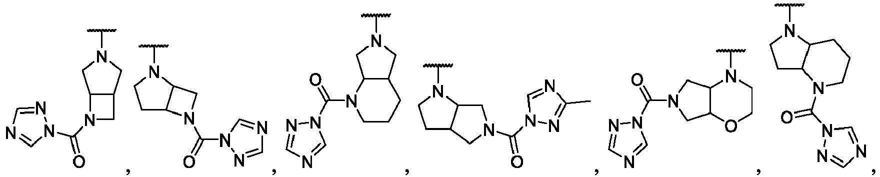

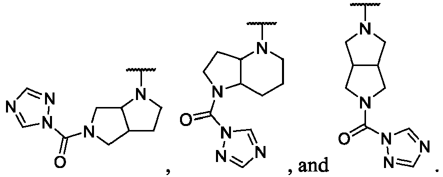

The embodiments of R¹⁶, R^{16a}, R^{16b}, R⁸, R^{8a}, and R^{8b} as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following.

In additional embodiments of the subject complex or compound (e.g., precursor compound), or a pharmaceutically acceptable salt or solvate thereof, R¹⁶ is independently selected from hydrogen, halogen, -CN, C_{1- 6}alkyl, C₂₋₆alkenyl, C₂₋₆alkynyl, and C₃₋₆cycloalkyl, wherein C₁₋₆alkyl, C₂₋₆alkenyl, C₂₋₆alkynyl, and C₃₋₆cycloalkyl are optionally substituted with one, two, or three R^{20g}. In some embodiments of the subject complex or compound (e.g., precursor compound), or a pharmaceutically acceptable salt or solvate thereof, R¹⁶ is independently selected from hydrogen and halogen. In embodiments of the subject complex or compound (e.g., precursor compound), or a pharmaceutically acceptable salt or solvate thereof, R¹⁶ is independently selected from hydrogen and fluoro. In embodiments, R¹⁶ is independently -OR¹².

In additional embodiments of the subject complex or compound (e.g., precursor compound), or a pharmaceutically acceptable salt or solvate thereof, R^{16a} is independently selected from hydrogen, halogen, -CN, C_{1- 6}alkyl, C₂₋₆alkenyl, C₂₋₆alkynyl, and C₃₋₆cycloalkyl, wherein C₁₋₆alkyl, C₂₋₆alkenyl, C₂₋₆alkynyl, and C₃₋₆cycloalkyl are optionally substituted with one, two, or three R^{20g}. In some embodiments of the subject complex or compound (e.g., precursor compound), or a pharmaceutically acceptable salt or solvate thereof, R^{16a} is independently selected from hydrogen and halogen. In embodiments of the subject complex or compound (e.g., precursor compound), or a pharmaceutically acceptable salt or solvate thereof, R^{16a} is independently selected from hydrogen and fluoro.

In additional embodiments of the subject complex or compound (e.g., precursor compound), or a pharmaceutically acceptable salt or solvate thereof, R^{16b} is independently selected from hydrogen, halogen, -CN, C_{1- 6}alkyl, C₂₋₆alkenyl, C₂₋₆alkynyl, and C₃₋₆cycloalkyl, wherein C₁₋₆alkyl, C₂₋₆alkenyl, C₂₋₆alkynyl, and C₃₋₆cycloalkyl are optionally substituted with one, two, or three R^{20g}. In some embodiments of the subject complex or compound (e.g., precursor compound), or a pharmaceutically acceptable salt or solvate thereof, R^{16b} is independently selected from hydrogen and halogen. In embodiments of the subject complex or compound (e.g., precursor compound), or a pharmaceutically acceptable salt or solvate thereof, R^{16b} is independently selected from hydrogen and fluoro.

In further embodiments of the subject complex or compound (e.g., precursor compound), or a pharmaceutically acceptable salt or solvate thereof, R⁸ is selected from hydrogen, C₁₋₆alkyl, C₂₋₆alkenyl, C₂₋₆alkynyl, C₃₋₁₀cycloalkyl, and C₂₋₉heterocycloalkyl, wherein C₁₋₆alkyl, C₂₋₆alkenyl, C₂₋₆alkynyl, C₃₋₁₀cycloalkyl, and C_{2- 9}heterocycloalkyl are optionally substituted with one, two, or three R^{20c}. In additional embodiments of the subject complex or compound (e.g., precursor compound), or a pharmaceutically acceptable salt or solvate thereof, R⁸ is selected from hydrogen, C₁₋₆alkyl, C₃₋₁₀cycloalkyl, and C₂₋₉heterocycloalkyl, wherein C₁₋₆allcyl, C₃₋₁₀cycloalkyl, and C₂₋₉heterocycloalkyl are optionally substituted with one, two, or three R^{20c} independently selected from halogen, C_{1- 6}alkyl, C₂₋₆alkenyl, C₂₋₆alkynyl, C₃₋₁₀cycloalkyl, and C₂₋₉heterocycloalkyl. In some embodiments of the subject complex or compound (e.g., precursor compound), or a pharmaceutically acceptable salt or solvate thereof, R⁸ is selected from hydrogen, methyl, cyclopropyl, cyclobutyl, and oxetanyl, wherein said methyl, cyclopropyl, cyclobutyl, and oxetanyl are optionally substituted with one, two, or three R^{20c} independently selected from fluoro, methyl, cyclopropyl, cyclobutyl, and oxetanyl. In embodiments of the subject complex or compound (e.g., precursor compound), or a pharmaceutically acceptable salt or solvate thereof, R⁸ is selected from hydrogen, methyl, cyclopropyl, cyclobutyl, and oxetanyl. In embodiments, R⁸ is selected from halogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}. In embodiments of the subject complex or compound (e.g., precursor compound), or a pharmaceutically acceptable salt or solvate thereof, R⁸ is selected from C₁₋₆alkyl, C₃₋₁₀cycloalkyl, and C_{2- 9}heterocycloalkyl, wherein C₁₋₆alkyl, C₃₋₁₀cycloalkyl, and C₂₋₉heterocycloalkyl are optionally substituted with one, two, or three R^{20c} independently selected from halogen, C₁₋₆allcyl, C₂₋₆alkenyl, C₂₋₆alkynyl, C₃₋₁₀cycloalkyl, and C_{2- 9}heterocycloalkyl. In further embodiments of the subject complex or compound (e.g., precursor compound), or a pharmaceutically acceptable salt or solvate thereof, R⁸ is selected from methyl, cyclopropyl, cyclobutyl, and oxetanyl, wherein said methyl, cyclopropyl, cyclobutyl, and oxetanyl are optionally substituted with one, two, or three R^{20c} independently selected from fluoro, methyl, cyclopropyl, cyclobutyl, and oxetanyl. In some embodiments of the subject complex or compound (e.g., precursor compound), or a pharmaceutically acceptable salt or solvate thereof, R⁸ is selected from methyl, cyclopropyl, cyclobutyl, and oxetanyl. In embodiments of the subject complex or compound (e.g., precursor compound), R⁸ is hydrogen. In some embodiments of the subject complex or compound (e.g., precursor compound), R⁸ is C₁₋₆alkyl optionally substituted with one, two, or three R^{20c}. In select embodiments of the subject complex or compound (e.g., precursor compound), R⁸ is C₂₋₆alkenyl optionally substituted with one, two, or three R^{20c}. In additional embodiments of the subject complex or compound (e.g., precursor compound), R⁸ is C₂₋₆alkynyl optionally substituted with one, two, or three R^{20c}. In some embodiments of the subject complex or compound (e.g., precursor compound), R⁸ is C₃₋₆cycloalkyl optionally substituted with one, two, or three R^{20c}. In embodiments of the subject complex or compound (e.g., precursor compound), R⁸ is C_{2- 9}heterocycloalkyl optionally substituted with one, two, or three R^{20c}. In further embodiments of the subject complex or compound (e.g., precursor compound), R⁸ is C₁₋₆alkyl. In select embodiments of the subject complex or compound (e.g., precursor compound), R⁸ is C₂₋₆alkenyl. In additional embodiments of the subject complex or compound (e.g., precursor compound), R⁸ is C₂₋₆alkynyl. In some embodiments of the subject complex or compound (e.g., precursor compound), R⁸ is C₃₋₆cycloalkyl. In embodiments of the subject complex or compound (e.g., precursor compound), R⁸ is C₂₋₉heterocycloalkyl.

In further embodiments of the subject complex or compound (e.g., precursor compound), or a pharmaceutically acceptable salt or solvate thereof, R^{8a} is selected from hydrogen, C₁₋₆alkyl, C₂₋₆alkenyl, C_{2- 6}alkynyl, C₃₋₁₀cycloalkyl, and C₂₋₉heterocycloalkyl, wherein C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20c}. In additional embodiments of the subject complex or compound (e.g., precursor compound), or a pharmaceutically acceptable salt or solvate thereof, R^{8a} is selected from hydrogen, C₁₋₆alkyl, C₃₋₁₀cycloalkyl, and C₂₋₉heterocycloalkyl, wherein C₁₋₆alkyl, C_{3- 10}cycloalkyl, and C₂₋₉heterocycloalkyl are optionally substituted with one, two, or three R^{20c} independently selected from halogen, C₁₋₆alkyl, C₂₋₆alkenyl, C₂₋₆alkynyl, C₃₋₁₀cycloalkyl, and C₂₋₉heterocycloalkyl. In some embodiments of the subject complex or compound (e.g., precursor compound), or a pharmaceutically acceptable salt or solvate thereof, R^{8a} is selected from hydrogen, methyl, cyclopropyl, cyclobutyl, and oxetanyl, wherein said methyl, cyclopropyl, cyclobutyl, and oxetanyl are optionally substituted with one, two, or three R^{20c} independently selected from fluoro, methyl, cyclopropyl, cyclobutyl, and oxetanyl. In embodiments of the subject complex or compound (e.g., precursor compound), or a pharmaceutically acceptable salt or solvate thereof, R^{8a} is selected from hydrogen, methyl, cyclopropyl, cyclobutyl, and oxetanyl. In embodiments, R^{8a} is selected from halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}. In embodiments of the subject complex or compound (e.g., precursor compound), or a pharmaceutically acceptable salt or solvate thereof, R^{8a} is selected from C₁₋₆alkyl, C_{3- 10}cycloalkyl, and C₂₋₉heterocycloalkyl, wherein C₁₋₆alkyl, C₃₋₁₀cycloalkyl, and C₂₋₉heterocycloalkyl are optionally substituted with one, two, or three R^{20c} independently selected from halogen, C₁₋₆alkyl, C₂₋₆alkenyl, C₂₋₆alkynyl, C_{3- 10}cycloalkyl, and C₂₋₉heterocycloalkyl. In further embodiments of the subject complex or compound (e.g., precursor compound), or a pharmaceutically acceptable salt or solvate thereof, R^{8a} is selected from methyl, cyclopropyl, cyclobutyl, and oxetanyl, wherein said methyl, cyclopropyl, cyclobutyl, and oxetanyl are optionally substituted with one, two, or three R^{20c} independently selected from fluoro, methyl, cyclopropyl, cyclobutyl, and oxetanyl. In some embodiments of the subject complex or compound (e.g., precursor compound), or a pharmaceutically acceptable salt or solvate thereof, R^{8a} is selected from methyl, cyclopropyl, cyclobutyl, and oxetanyl.

In further embodiments of the subject complex or compound (e.g., precursor compound), or a pharmaceutically acceptable salt or solvate thereof, R^{8b} is selected from hydrogen, C₁₋₆alkyl, C₂₋₆alkenyl, C_{2- 6}alkynyl, C₃₋₁₀cycloalkyl, and C₂₋₉heterocycloalkyl, wherein C₁₋₆alkyl, C₂₋₆alkenyl, C₂₋₆alkynyl, C₃₋₁₀cycloalkyl, and C₂₋₉heterocycloalkyl are optionally substituted with one, two, or three R^{20c}. In additional embodiments of the subject complex or compound (e.g., precursor compound), or a pharmaceutically acceptable salt or solvate thereof, R^{8b} is selected from hydrogen, C₁₋₆alkyl, C₃₋₁₀cycloalkyl, and C₂₋₉heterocycloalkyl, wherein C₁₋₆alkyl, C_{3- 10}cycloalkyl, and C₂₋₉heterocycloalkyl are optionally substituted with one, two, or three R^{20c} independently selected from halogen, C₁₋₆alkyl, C₂₋₆alkenyl, C₂₋₆alkynyl, C₃₋₁₀cycloalkyl, and C₂₋₉heterocycloalkyl. In some embodiments of the subject complex or compound (e.g., precursor compound), or a pharmaceutically acceptable salt or solvate thereof, R^{8b} is selected from hydrogen, methyl, cyclopropyl, cyclobutyl, and oxetanyl, wherein said methyl, cyclopropyl, cyclobutyl, and oxetanyl are optionally substituted with one, two, or three R^{20c} independently selected from fluoro, methyl, cyclopropyl, cyclobutyl, and oxetanyl. In embodiments of the subject complex or compound (e.g., precursor compound), or a pharmaceutically acceptable salt or solvate thereof, R^{8b} is selected from hydrogen, methyl, cyclopropyl, cyclobutyl, and oxetanyl. In embodiments, R^{8b} is selected from halogen, -CN, C₁₋₆alkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, - C(O)N(R¹²)(R¹³), -C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c}. In embodiments of the subject complex or compound (e.g., precursor compound), or a pharmaceutically acceptable salt or solvate thereof, R^{8b} is selected from C₁₋₆alkyl, C₃₋₁₀cycloalkyl, and C₂₋₉heterocycloalkyl, wherein C₁₋₆alkyl, C₃₋₁₀cycloalkyl, and C₂₋₉heterocycloalkyl are optionally substituted with one, two, or three R^{20c} independently selected from halogen, C₁₋₆alkyl, C₂₋₆alkenyl, C_{2- 6}alkynyl, C₃₋₁₀cycloalkyl, and C₂₋₉heterocycloalkyl. In further embodiments of the subject complex or compound (e.g., precursor compound), or a pharmaceutically acceptable salt or solvate thereof, R^{8b} is selected from methyl, cyclopropyl, cyclobutyl, and oxetanyl, wherein said methyl, cyclopropyl, cyclobutyl, and oxetanyl are optionally substituted with one, two, or three R^{20c} independently selected from fluoro, methyl, cyclopropyl, cyclobutyl, and oxetanyl. In some embodiments of the subject complex or compound (e.g., precursor compound), or a pharmaceutically acceptable salt or solvate thereof, R^{8b} is selected from methyl, cyclopropyl, cyclobutyl, and oxetanyl.

In further embodiments of the subject complex or compound (e.g., precursor compound), or a pharmaceutically acceptable salt or solvate thereof, R³ is independently hydrogen or CN. In additional embodiments of the subject complex or compound (e.g., precursor compound), or a pharmaceutically acceptable salt or solvate thereof, R³ is independently hydrogen. In some embodiments of the subject complex or compound (e.g., precursor compound), R³ is independently hydrogen. In embodiments of the subject complex or compound (e.g., precursor compound), R³ is independently -CN. In embodiments of the subject complex or compound (e.g., precursor compound), R³ is independently C₁₋₆alkyl. In additional embodiments of the subject complex or compound (e.g., precursor compound), R³ is independently methyl.

In some embodiments of the subject complex or compound (e.g., precursor compound), or a pharmaceutically acceptable salt or solvate thereof, L¹ is a bond. In some embodiments of the subject complex or compound (e.g., precursor compound), or a pharmaceutically acceptable salt or solvate thereof, L¹ is selected from a C₁-C₆alkyl, C₂-C₆alkenyl, C₂-C₆alkynyl, -C(O)-, -NHC(O)-, -C(O)NH-, CH₂O, CH₂NH, and CH₂. In some embodiments of the subject complex or compound (e.g., precursor compound), or a pharmaceutically acceptable salt or solvate thereof, L¹ is C₁-C₆alkyl. In some embodiments of the subject complex or compound (e.g., precursor compound), or a pharmaceutically acceptable salt or solvate thereof, L¹ is C₂-C₆alkenyl. In some embodiments of the subject complex or compound (e.g., precursor compound), or a pharmaceutically acceptable salt or solvate thereof, L¹ is C₂-C₆alkynyl. In some embodiments of the subject complex or compound (e.g., precursor compound), or a pharmaceutically acceptable salt or solvate thereof, L¹ is -C(O)-. In some embodiments of the subject complex or compound (e.g., precursor compound), or a pharmaceutically acceptable salt or solvate thereof, L¹ is -NHC(O)-. In some embodiments of the subject complex or compound (e.g., precursor compound), or a pharmaceutically acceptable salt or solvate thereof, L¹ is -C(O)NH-. In some embodiments of the subject complex or compound (e.g., precursor compound), or a pharmaceutically acceptable salt or solvate thereof, L¹ is CH₂O. In some embodiments of the subject complex or compound (e.g., precursor compound), or a pharmaceutically acceptable salt or solvate thereof, L¹ is CH₂NH. In some embodiments of the subject complex or compound (e.g., precursor compound), or a pharmaceutically acceptable salt or solvate thereof, L¹ is CH₂.

In some embodiments of the subject complex or compound (e.g., precursor compound), or a pharmaceutically acceptable salt or solvate thereof, L^{1b} is a bond. In some embodiments of the subject complex or compound (e.g., precursor compound), or a pharmaceutically acceptable salt or solvate thereof, L^{1b} is selected from a C₁-C₆alkyl, C₂-C₆alkenyl, C₂-C₆alkynyl, -C(O)-, -NHC(O)-, -C(O)NH-, CH₂O, CH₂NH, and CH₂. In some embodiments of the subject complex or compound (e.g., precursor compound), or a pharmaceutically acceptable salt or solvate thereof, L^{1b} is C₁-C₆alkyl. In some embodiments of the subject complex or compound (e.g., precursor compound), or a pharmaceutically acceptable salt or solvate thereof, L^{1b} is C₂-C₆alkenyl. In some embodiments of the subject complex or compound (e.g., precursor compound), or a pharmaceutically acceptable salt or solvate thereof, L^{1b} is C₂-C₆alkynyl. In some embodiments of the subject complex or compound (e.g., precursor compound), or a pharmaceutically acceptable salt or solvate thereof, L^{1b} is -C(O)-. In some embodiments of the subject complex or compound (e.g., precursor compound), or a pharmaceutically acceptable salt or solvate thereof, L^{1b} is -NHC(O)-. In some embodiments of the subject complex or compound (e.g., precursor compound), or a pharmaceutically acceptable salt or solvate thereof, L^{1b} is -C(O)NH-. In some embodiments of the subject complex or compound (e.g., precursor compound), or a pharmaceutically acceptable salt or solvate thereof, L^{1b} is CH₂O. In some embodiments of the subject complex or compound (e.g., precursor compound), or a pharmaceutically acceptable salt or solvate thereof, L^{1b} is CH₂NH. In some embodiments of the subject complex or compound (e.g., precursor compound), or a pharmaceutically acceptable salt or solvate thereof, L^{1b} is CH₂.

The embodiments of R¹⁹ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. In some embodiments of the subject complex or compound (e.g., precursor compound), or a pharmaceutically acceptable salt or solvate thereof, R¹⁹ is a monocyclic ring. In some embodiments of the subject complex or compound (e.g., precursor compound), or a pharmaceutically acceptable salt or solvate thereof, R¹⁹ is a bicyclic ring system. In some embodiments of the subject complex or compound (e.g., precursor compound), or a pharmaceutically acceptable salt or solvate thereof, R¹⁹ is a polycyclic ring system.In embodiments of the subject complex or compound (e.g., precursor compound), or a pharmaceutically acceptable salt or solvate thereof, R¹⁹ is: 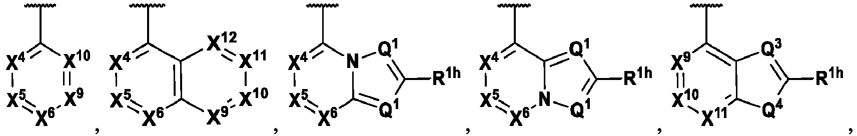 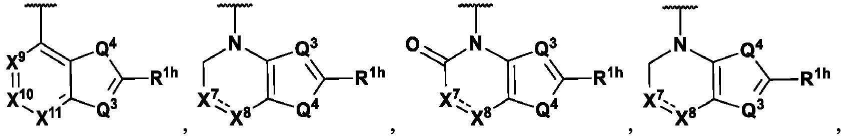 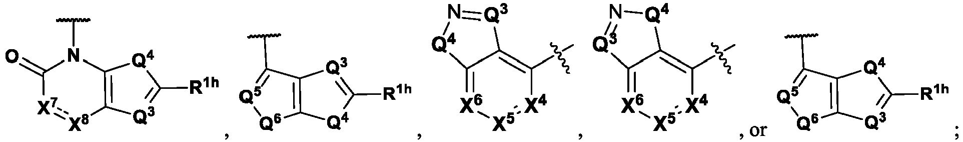
Q¹, Q³, and Q⁵ are independently N or C(R^{1d});
Q⁴ and Q⁶ are independently O, S, C(R^{1a})(R^{1b}), or N(R^{1c});
X⁴, X⁵, X⁶, X⁹, X¹⁰, X¹¹, and X¹² are independently selected from C(R^{1a}) or N;
X⁷ and X⁸ are independently selected from C(R^{1a}), C(R^{1a})(R^{1b}), N, or N(R^{1c});
each R^{1a}, R^{1b}, R^{1a}, and R^{1h} are each independently selected from hydrogen, halogen, -CN, C₁₋₆alkyl, C_{1- 6}haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ; or R^{1a} and R^{1b} bonded to the same carbon are joined to form a 3-10 membered heterocycloalkyl ring or a C₃₋₁₀cycloalkyl ring, wherein the 3-10 membered heterocycloalkyl ring or C₃₋₁₀cycloalkyl ring are optionally substituted with one, two, or three R²⁰ⁱ; or two R^{1a} bonded to adjacent atoms are joined to form a 3-10 membered heterocycloalkyl ring, a C₆₋₁₀aryl ring, a 5-12 membered heteroaryl ring, or a C₃₋₁₀cycloalkyl ring, wherein the 3-10 membered heterocycloalkyl ring, C₆₋₁₀aryl ring, 5-12 membered heteroaryl ring, or C₃₋₁₀cycloalkyl ring are optionally substituted with one, two, or three R²⁰ⁱ; or R^{1h} and one of R^{1a}, R^{1b}, R^{1c}, and R^{1d} bonded to adjacent atoms are joined to form a 3-10 membered heterocycloalkyl ring, a C₆₋₁₀aryl ring, a 5-12 membered heteroaryl ring, or a C₃₋₁₀cycloalkyl ring, wherein the 3-10 membered heterocycloalkyl ring, a C₆₋₁₀aryl ring, a 5-12 membered heteroaryl ring, and C₃₋₁₀cycloalkyl ring are optionally substituted with one, two, or three R²⁰ⁱ; and
each R^{1c} is independently selected from hydrogen, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ.

In embodiments, R^{1h} is selected from hydrogen, C₁₋₆alkyl, C₁₋₆haloalkyl, C₂₋₆alkenyl, C₂₋₆alkynyl, - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -C(O)R¹⁵, - OC(O)R¹⁵, -C(O)N(R¹²)(R¹³), -C(O)C(O)N(R¹²)(R¹³), -N(R¹⁴)C(O)R¹⁵, -CH₂C(O)N(R¹²)(R¹³), and - CH₂N(R¹⁴)C(O)R¹⁵, wherein C₁₋₆alkyl, C₂₋₆alkenyl, and C₂₋₆alkynyl are optionally substituted with one, two, or three R²⁰ⁱ.

In embodiments, R^{1h} is selected from hydrogen and -N(R¹²)(R¹³).

In embodiments, R^{1d} is independently selected from hydrogen, halogen, -CN, C₁₋₆alkyl, C₁₋₆haloallcyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}. In embodiments, R^{1d} is independently selected from hydrogen, -CN, C₁₋₆alkyl, and C_{1- 6}haloalkyl, wherein C₁₋₆alkyl is optionally substituted with one, two, or three R²⁰ⁱ.

In embodiments, the formula 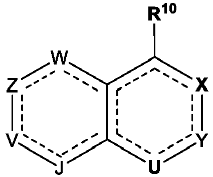 is selected from 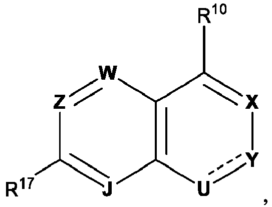 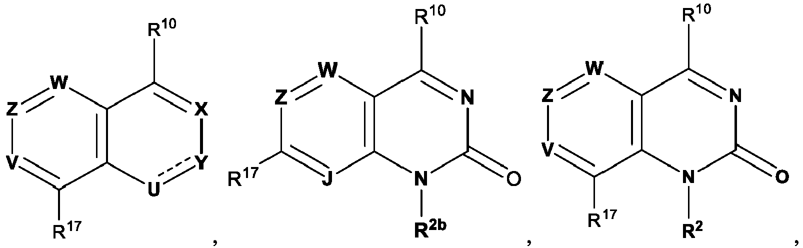 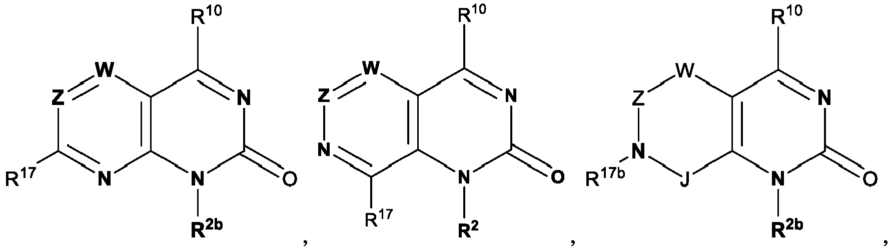 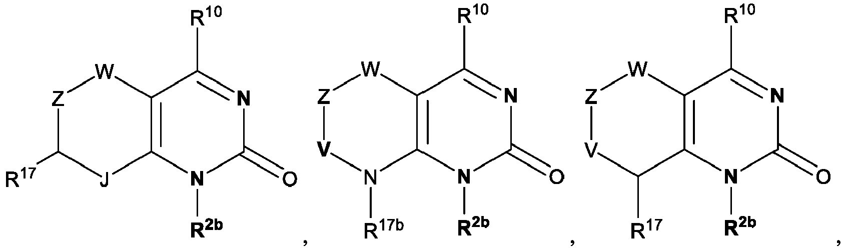 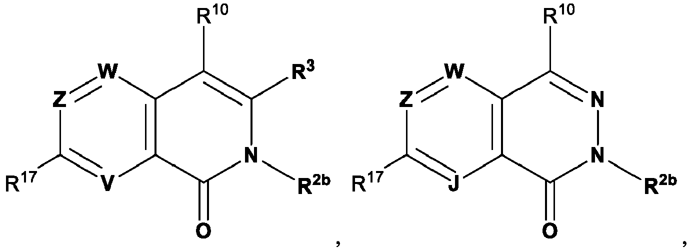 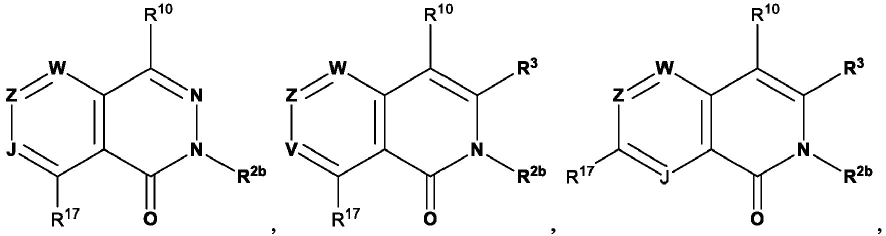 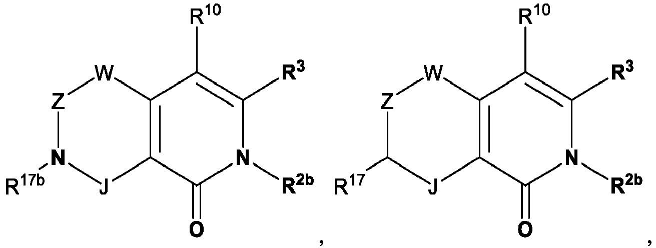 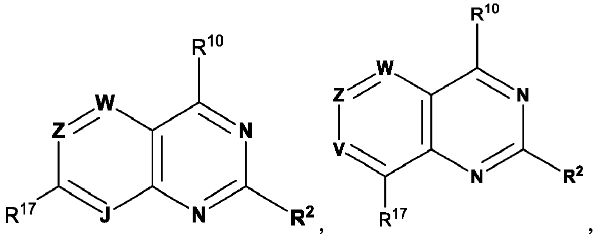 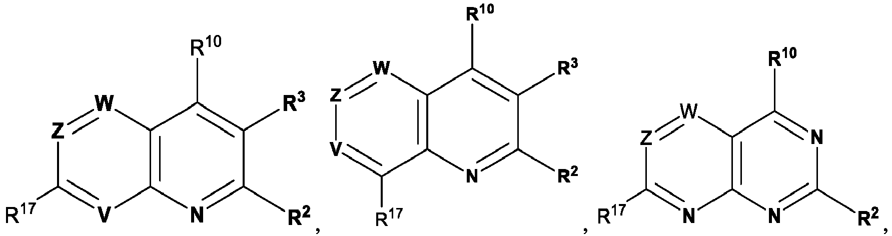 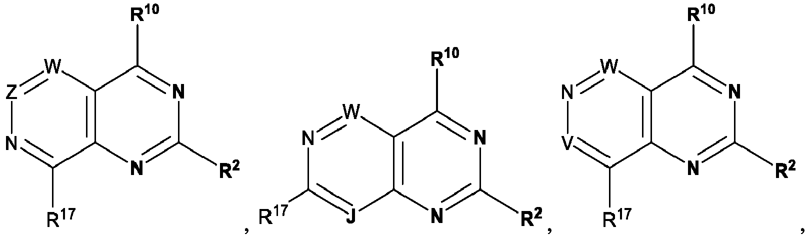 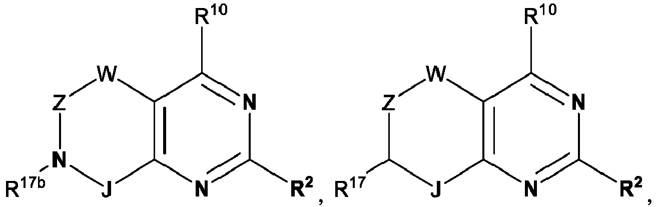 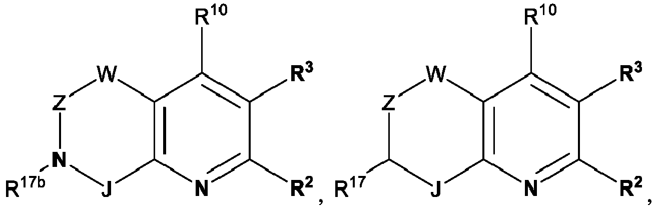 and 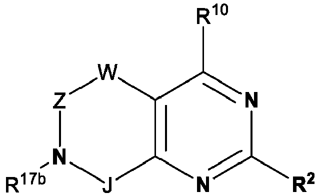 wherein X, Y, U, W, Z, V, J, R², R^{2b}, R³, R¹⁰, R¹⁷, and R^{17b} are as described herein. In embodiments, the formula 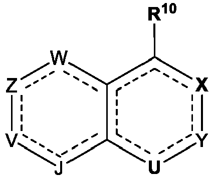 is selected from 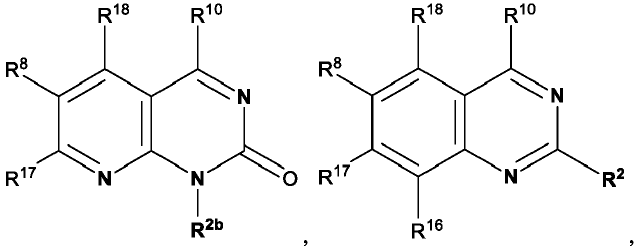 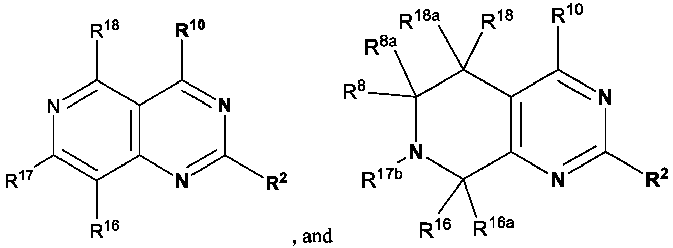 wherein X, Y, U, W, Z, V, J, R², R^{2b}, R¹⁰, R⁸, R^{8a}, R¹⁶, R^{16a}, R¹⁸, R^{18a}, R¹⁷, and R^{17b} are as described herein.

In embodiments, the formula 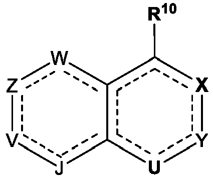 is 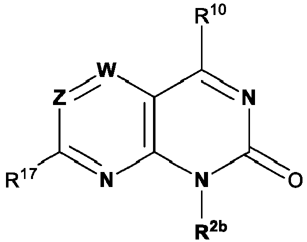 wherein W, Z, R^{2b}, R¹⁰, and R¹⁷ are as described herein.

In embodiments, the formula 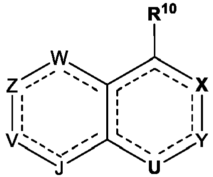 is 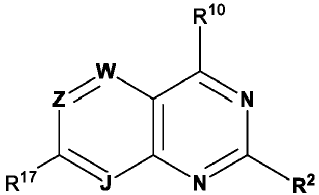 wherein W, Z, J, R², R¹⁰, and R¹⁷ are as described herein.

In embodiments, the formula 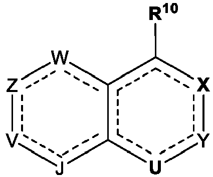 is 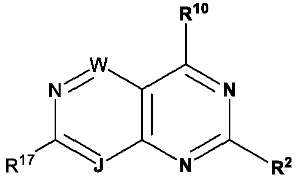 wherein W, J, R², R¹⁰, and R¹⁷ are as described herein.

In embodiments, the formula 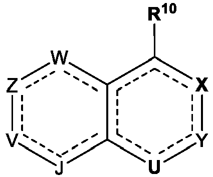 is 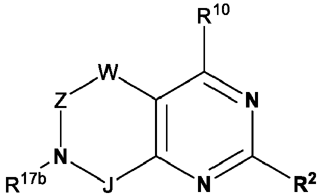 wherein W, Z, J, R², R¹⁰, and R^{17b} are as described herein.

In embodiments, the formula 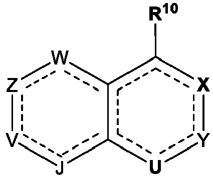 is 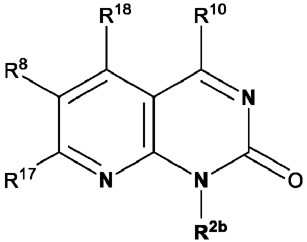 wherein R^{2b}, R¹⁰, R⁸, R¹⁸, and R¹⁷ are as described herein.

In embodiments, the formula 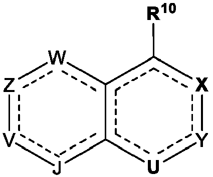 is 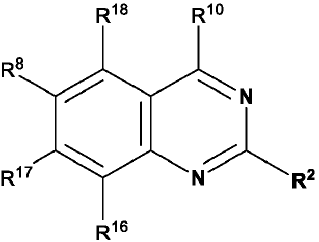 wherein R², R¹⁰, R⁸, R¹⁶, R¹⁸, and R¹⁷ are as described herein.

In embodiments, the formula 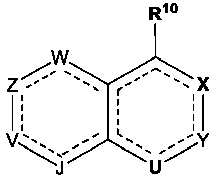 is 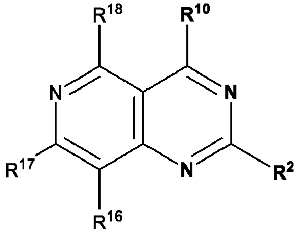 wherein R², R¹⁰, R¹⁶, R¹⁸, and R¹⁷ are as described herein.

In embodiments, the formula 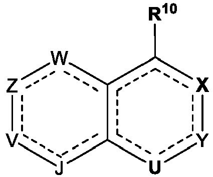 is 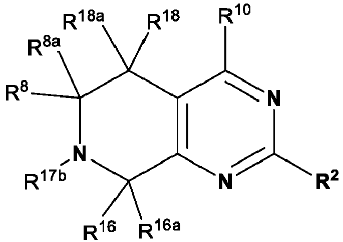 wherein R², R¹⁰, R⁸, R^{8a}, R¹⁶, R^{16a}, R¹⁸, R^{18a}, and R^{17b} are as described herein.

**(1) Table 1.**

| | A | B | C |
|---|---|---|---|
| 1 | 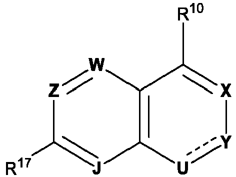 | 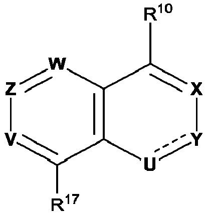 | 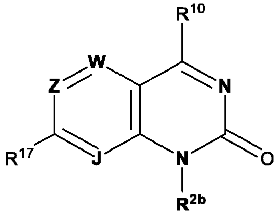 |
| 2 | 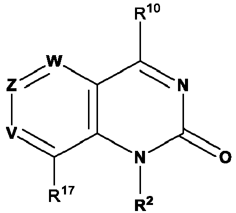 | 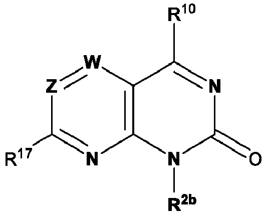 | 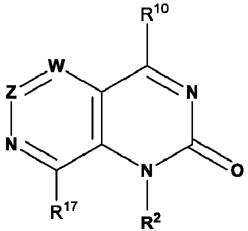 |
| 3 | 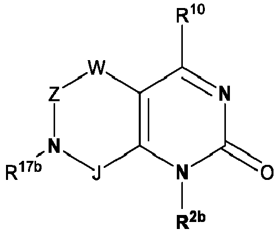 | 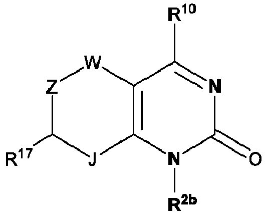 | 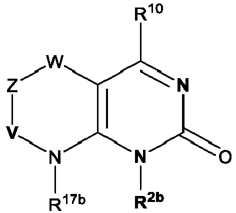 |
| 4 | 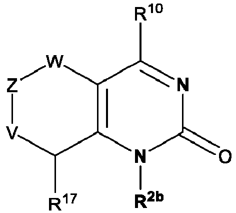 | 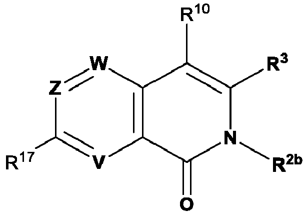 | 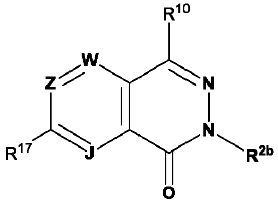 |
| 5 | 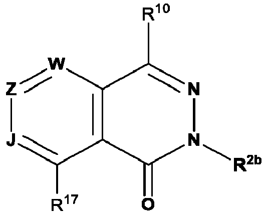 | 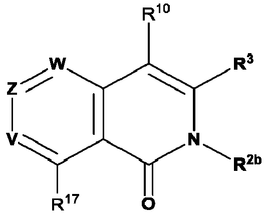 | 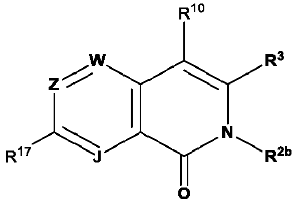 |
| 6 | 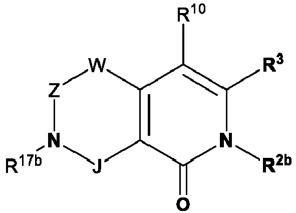 | 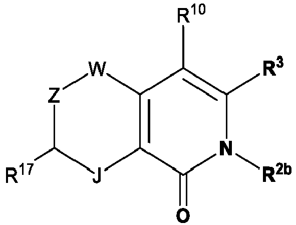 | 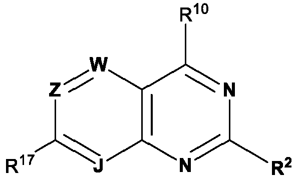 |
| 7 | 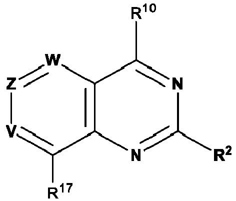 | 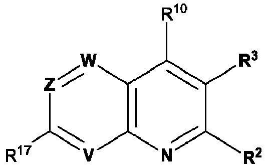 | 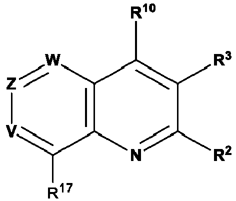 |
| 8 | 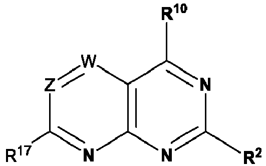 | 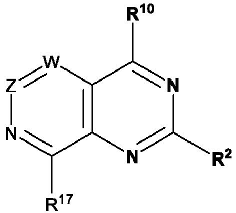 | 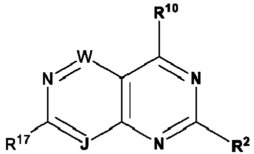 |
| 9 | 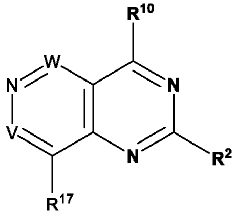 | 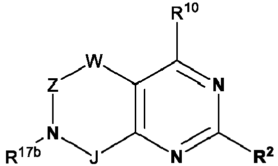 | 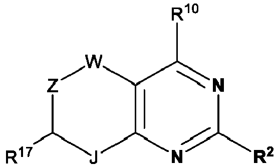 |
| 10 | 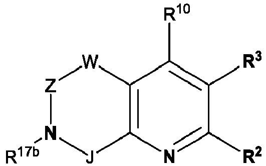 | 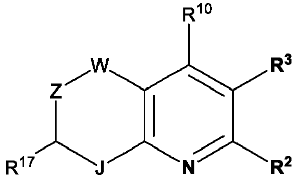 | 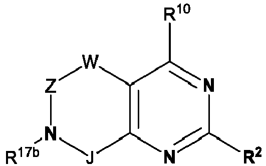 |
| 11 | 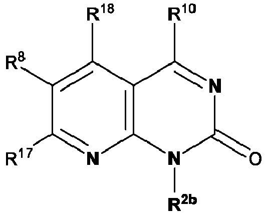 | 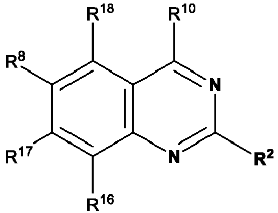 | 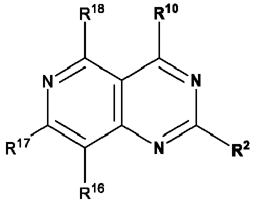 |
| 12 | 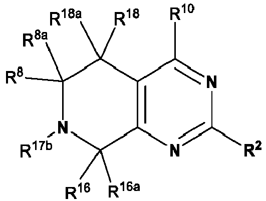 | 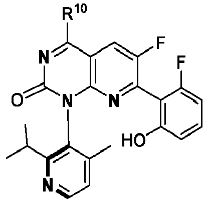 | 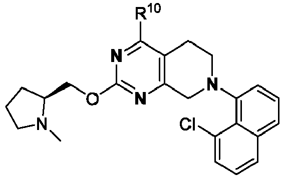 |
| 13 | 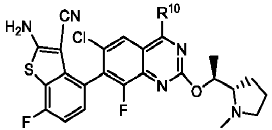 | 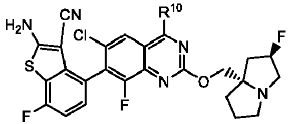 | 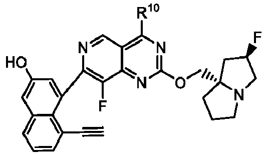 |

| | | | |
|---|---|---|---|
| ***X, Y, U, W, Z, V, J, R², R^{2b}, R³, R¹⁰, R⁸, R^{8a}, R¹⁶, R^{16a}, R¹⁸, R^{18a}, R¹⁷, and R^{17b} are as described herein.*** | | | |

**(2) Table 2 R¹⁰**

| | D | E | | F | G |
|---|---|---|---|---|---|
| 1 | 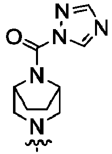 | 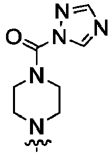 | | 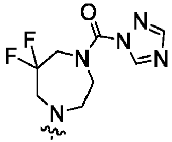 | 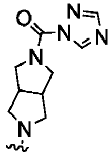 |
| 2 | 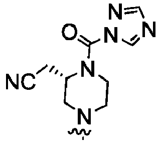 | 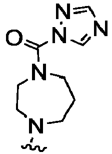 | | 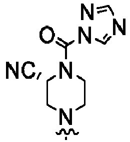 | 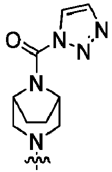 |
| 3 | 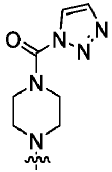 | 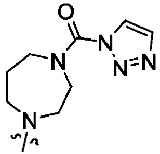 | | 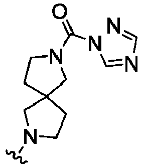 | 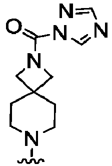 |
| 4 | 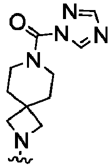 | 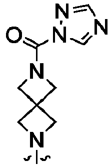 | | 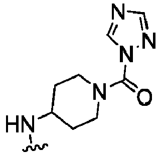 | 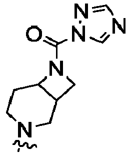 |
| 5 | 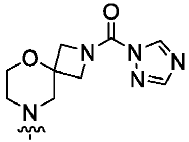 | 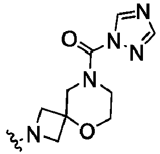 | | 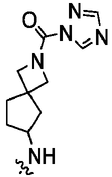 | 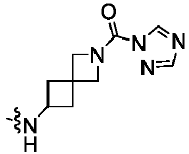 |
| 6 | 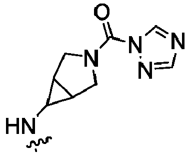 | 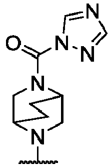 | | 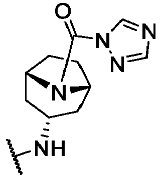 | 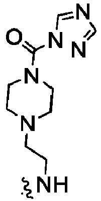 |
| 7 | 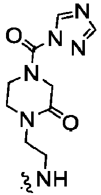 | 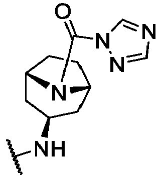 | | 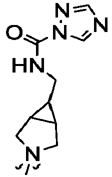 | 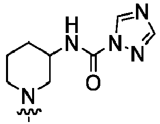 |
| 8 | 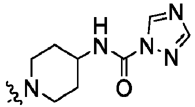 | 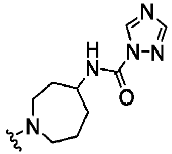 | | 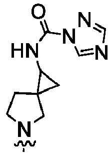 | 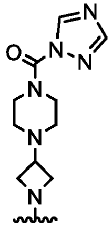 |
| 9 | 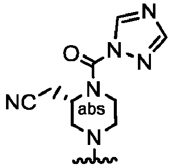 | 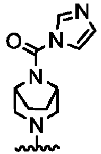 | | 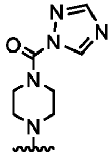 | 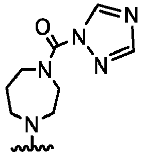 |
| 10 | 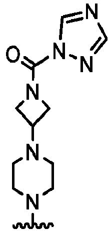 | 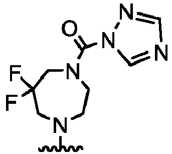 | | 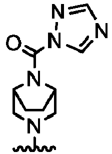 | 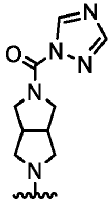 |
| 11 | 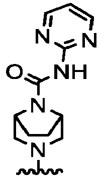 | 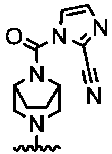 | | 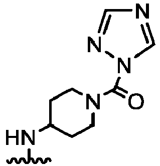 | 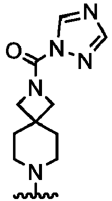 |
| 12 | 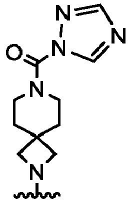 | 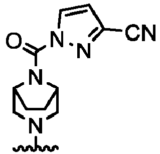 | | 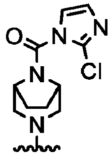 | 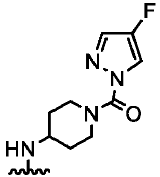 |
| 13 | 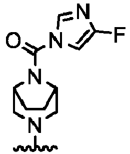 | 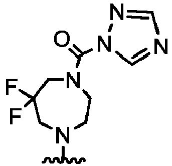 | | 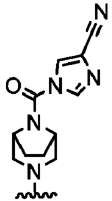 | 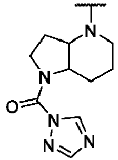 |
| 14 | 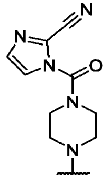 | 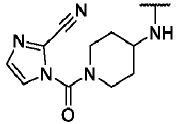 | | 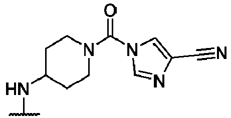 | 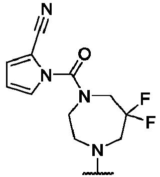 |
| 15 | 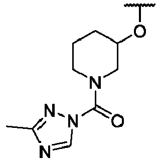 | 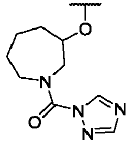 | | 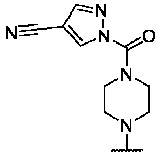 | 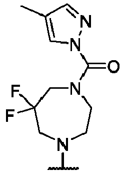 |
| 16 | 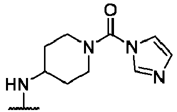 | 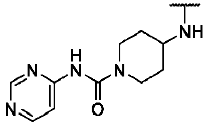 | | 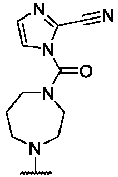 | 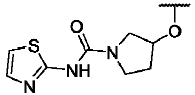 |
| 17 | 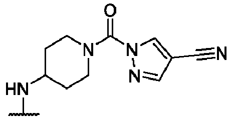 | 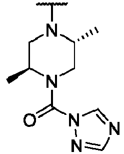 | | 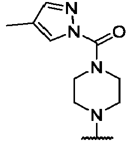 | 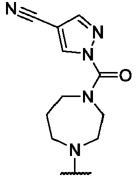 |
| 18 | 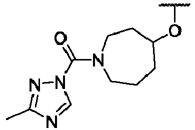 | 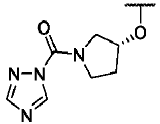 | | 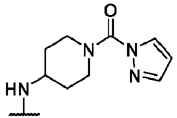 | 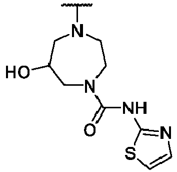 |
| 19 | 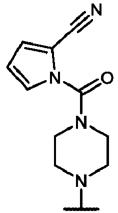 | 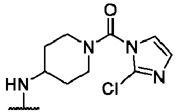 | | 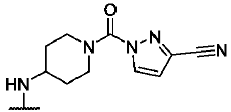 | 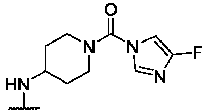 |
| 20 | 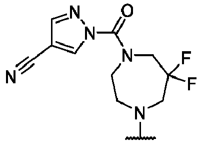 | 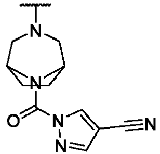 | | 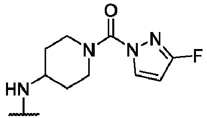 | 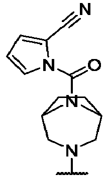 |
| 21 | 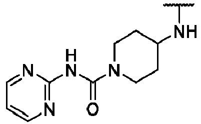 | 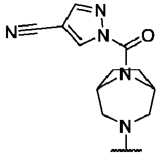 | | 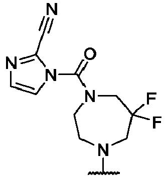 | 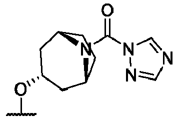 |
| 22 | 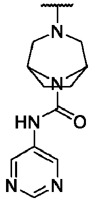 | 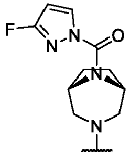 | | 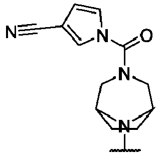 | 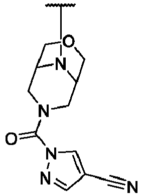 |
| 23 | 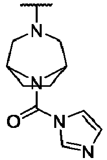 | 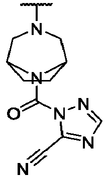 | | 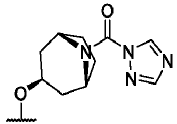 | 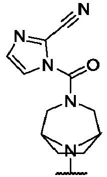 |
| 24 | 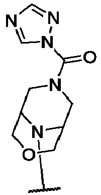 | 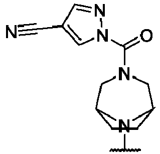 | | 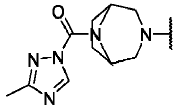 | 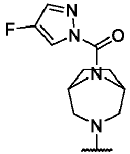 |
| 25 | 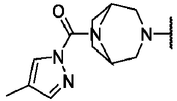 | 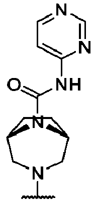 | | 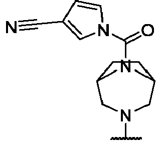 | 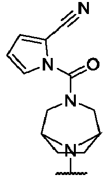 |
| 26 | 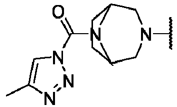 | 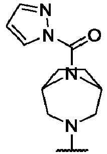 | | 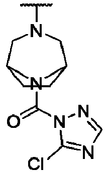 | 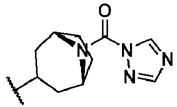 |
| 27 | 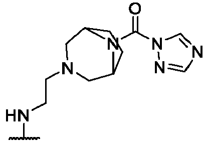 | 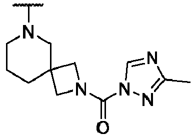 | | 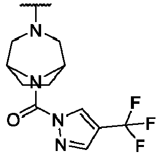 | 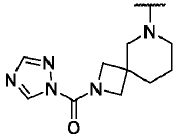 |
| 28 | 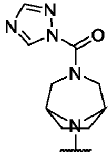 | 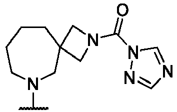 | | 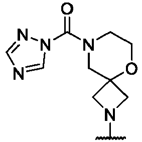 | 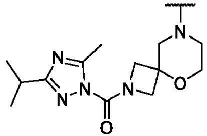 |
| 29 | 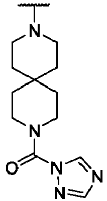 | 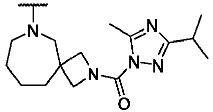 | | 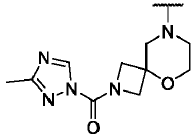 | 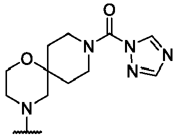 |
| 30 | 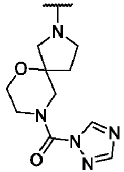 | 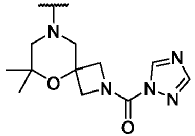 | | 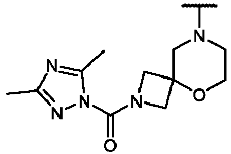 | 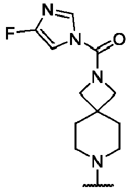 |
| 31 | 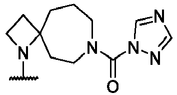 | 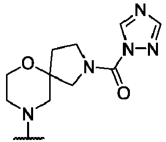 | | 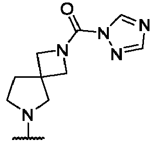 | 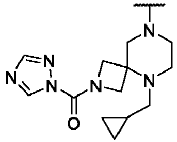 |
| 32 | 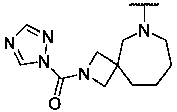 | 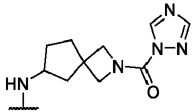 | | 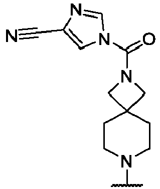 | 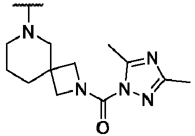 |
| 33 | 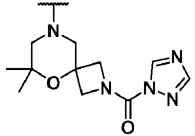 | 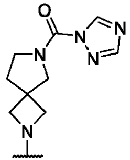 | | 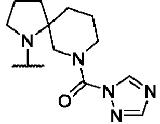 | 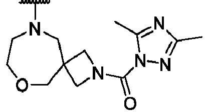 |
| 34 | 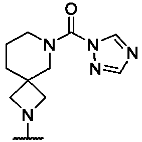 | 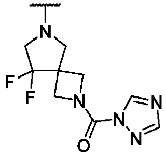 | | 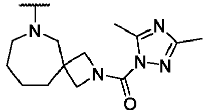 | 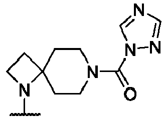 |
| 35 | 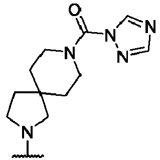 | 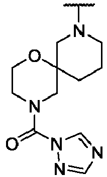 | | 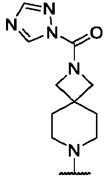 | 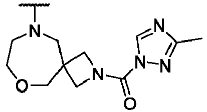 |
| 36 | 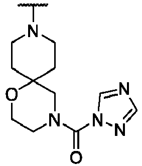 | 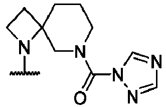 | | 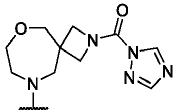 | 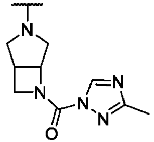 |
| 37 | 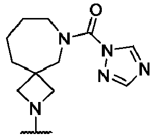 | 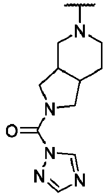 | | 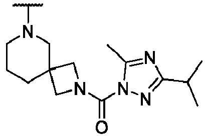 | 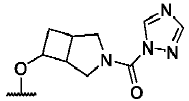 |
| 38 | 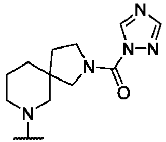 | 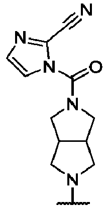 | | 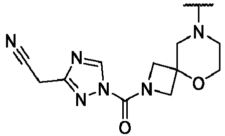 | 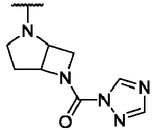 |
| 39 | 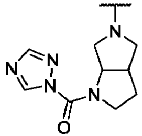 | 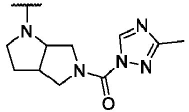 | | 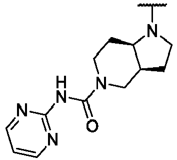 | 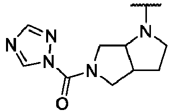 |
| 40 | 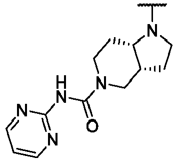 | 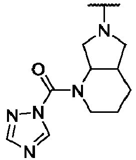 | | 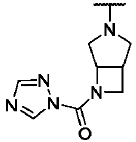 | 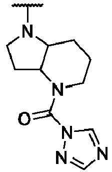 |

In embodiments, any Compound formula of Table 1 may be combined with any R¹⁰ of Table 2. For example, the compound formula of Table 1 A1 may be combined with the R¹⁰ of Table 2 D1 to form the compound formula A1D1 wherein X, Y, U, W, Z, V, J, R², R^{2b}, R³, R¹⁰, R⁸, R^{8a}, R¹⁶, R^{16a}, R¹⁸, R^{18a}, R¹⁷, and R^{17b} are as described herein. In further embodiments, the compound may be a compound of formula A1D1, A2D1, A3D1, A4D1, A5D1, A6D1, A7D1, A8D1, A9D1, A10D1, A11D1, A12D1, A1D2, A2D2, A3D2, A4D2, A5D2, A6D2, A7D2, A8D2, A9D2, A10D2, A11D2, A12D2, A1D3, A2D3, A3D3, A4D3, A5D3, A6D3, A7D3, A8D3, A9D3, A10D3, A11D3, A12D3, A1D4, A2D4, A3D4, A4D4, A5D4, A6D4, A7D4, A8D4, A9D4, A10D4, A11D4, A12D4, A1D5, A2D5, A3D5, A4D5, A5D5, A6D5, A7D5, A8D5, A9D5, A10D5, A11D5, A12D5, A1D6, A2D6, A3D6, A4D6, A5D6, A6D6, A7D6, A8D6, A9D6, A10D6, A11D6, A12D6, A1D7, A2D7, A3D7, A4D7, A5D7, A6D7, A7D7, A8D7, A9D7, A10D7, A11D7, A12D7, A1D8, A2D8, A3D8, A4D8, A5D8, A6D8, A7D8, A8D8, A9D8, A10D8, A11D8, A12D8, A1D9, A2D9, A3D9, A4D9, A5D9, A6D9, A7D9, A8D9, A9D9, A10D9, A11D9, A12D9, A1D10, A2D10, A3D10, A4D10, A5D10, A6D10, A7D10, A8D10, A9D10, A10D10, A11D10, A12D10, A1D11, A2D11, A3D11, A4D11, A5D11, A6D11, A7D11, A8D11, A9D11, A10D11, A11D11, A12D11, A1D12, A2D12, A3D12, A4D12, A5D12, A6D12, A7D12, A8D12, A9D12, A10D12, A11D12, A12D12, A1D13, A2D13, A3D13, A4D13, A5D13, A6D13, A7D13, A8D13, A9D13, A10D13, A11D13, A12D13, A1E1, A2E1, A3E1, A4E1, A5E1, A6E1, A7E1, A8E1, A9E1, A10E1, A11E1, A12E1, A1E2, A2E2, A3E2, A4E2, A5E2, A6E2, A7E2, A8E2, A9E2, A10E2, A11E2, A12E2, A1E3, A2E3, A3E3, A4E3, A5E3, A6E3, A7E3, A8E3, A9E3, A10E3, A11E3, A12E3, A1E4, A2E4, A3E4, A4E4, A5E4, A6E4, A7E4, A8E4, A9E4, A10E4, A11E4, A12E4, A1E5, A2E5, A3E5, A4E5, A5E5, A6E5, A7E5, A8E5, A9E5, A10E5, A11E5, A12E5, A1E6, A2E6, A3E6, A4E6, A5E6, A6E6, A7E6, A8E6, A9E6, A10E6, A11E6, A12E6, A1E7, A2E7, A3E7, A4E7, A5E7, A6E7, A7E7, A8E7, A9E7, A10E7, A11E7, A12E7, A1E8, A2E8, A3E8, A4E8, A5E8, A6E8, A7E8, A8E8, A9E8, A10E8, A11E8, A12E8, A1E9, A2E9, A3E9, A4E9, A5E9, A6E9, A7E9, A8E9, A9E9, A10E9, A11E9, A12E9, A1E10, A2E10, A3E10, A4E10, A5E10, A6E10, A7E10, A8E10, A9E10, A10E10, A11E10, A12E10, A1E11, A2E11, A3E11, A4E11, A5E11, A6E11, A7E11, A8E11, A9E11, A10E11, A11E11, A12E11, A1E12, A2E12, A3E12, A4E12, A5E12, A6E12, A7E12, A8E12, A9E12, A10E12, A11E12, A12E12, A1E13, A2E13, A3E13, A4E13, A5E13, A6E13, A7E13, A8E13, A9E13, A10E13, A11E13, A12E13, A1F1, A2F1, A3F1, A4F1, A5F1, A6F1, A7F1, A8F1, A9F1, A10F1, A11F1, A12F1, A1F2, A2F2, A3F2, A4F2, A5F2, A6F2, A7F2, A8F2, A9F2, A10F2, A11F2, A12F2, A1F3, A2F3, A3F3, A4F3, A5F3, A6F3, A7F3, A8F3, A9F3, A10F3, A11F3, A12F3, A1F4, A2F4, A3F4, A4F4, A5F4, A6F4, A7F4, A8F4, A9F4, A10F4, A11F4, A12F4, A1F5, A2F5, A3F5, A4F5, A5F5, A6F5, A7F5, A8F5, A9F5, A10F5, A11F5, A12F5, A1F6, A2F6, A3F6, A4F6, A5F6, A6F6, A7F6, A8F6, A9F6, A10F6, A11F6, A12F6, A1F7, A2F7, A3F7, A4F7, A5F7, A6F7, A7F7, A8F7, A9F7, A10F7, A11F7, A12F7, A1F8, A2F8, A3F8, A4F8, A5F8, A6F8, A7F8, A8F8, A9F8, A10F8, A11F8, A12F8, A1F9, A2F9, A3F9, A4F9, A5F9, A6F9, A7F9, A8F9, A9F9, A10F9, A11F9, A12F9, A1F10, A2F10, A3F10, A4F10, A5F10, A6F10, A7F10, A8F10, A9F10, A10F10, A11F10, A12F10, A1F11, A2F11, A3F11, A4F11, A5F11, A6F11, A7F11, A8F11, A9F11, A10F11, A11F11, A12F11, A1F12, A2F12, A3F12, A4F12, A5F12, A6F12, A7F12, A8F12, A9F12, A10F12, A11F12, A12F12, A1F13, A2F13, A3F13, A4F13, A5F13, A6F13, A7F13, A8F13, A9F13, A10F13, A11F13, A12F13, A1G1, A2G1, A3G1, A4G1, A5G1, A6G1, A7G1, A8G1, A9G1, A10G1, A11G1, A12G1, A1G2, A2G2, A3G2, A4G2, A5G2, A6G2, A7G2, A8G2, A9G2, A10G2, A11G2, A12G2, A1G3, A2G3, A3G3, A4G3, A5G3, A6G3, A7G3, A8G3, A9G3, A10G3, A11G3, A12G3, A1G4, A2G4, A3G4, A4G4, A5G4, A6G4, A7G4, A8G4, A9G4, A10G4, A11G4, A12G4, A1G5, A2G5, A3G5, A4G5, A5G5, A6G5, A7G5, A8G5, A9G5, A10G5, A11G5, A12G5, A1G6, A2G6, A3G6, A4G6, A5G6, A6G6, A7G6, A8G6, A9G6, A10G6, A11G6, A12G6, A1G7, A2G7, A3G7, A4G7, A5G7, A6G7, A7G7, A8G7, A9G7, A10G7, A11G7, A12G7, A1G8, A2G8, A3G8, A4G8, A5G8, A6G8, A7G8, A8G8, A9G8, A10G8, A11G8, A12G8, A1G9, A2G9, A3G9, A4G9, A5G9, A6G9, A7G9, A8G9, A9G9, A10G9, A11G9, A12G9, A1G10, A2G10, A3G10, A4G10, A5G10, A6G10, A7G10, A8G10, A9G10, A10G10, A11G10, A12G10, A1G11, A2G11, A3G11, A4G11, A5G11, A6G11, A7G11, A8G11, A9G11, A10G11, A11G11, A12G11, A1G12, A2G12, A3G12, A4G12, A5G12, A6G12, A7G12, A8G12, A9G12, A10G12, A11G12, A12G12, , B1D1, B2D1, B3D1, B4D1, B5D1, B6D1, B7D1, B8D1, B9D1, B10D1, B11D1, B1D2, B2D2, B3D2, B4D2, B5D2, B6D2, B7D2, B8D2, B9D2, B10D2, B11D2, B1D3, B2D3, B3D3, B4D3, B5D3, B6D3, B7D3, B8D3, B9D3, B10D3, B11D3, B1D4, B2D4, B3D4, B4D4, B5D4, B6D4, B7D4, B8D4, B9D4, B10D4, B11D4, B1D5, B2D5, B3D5, B4D5, B5D5, B6D5, B7D5, B8D5, B9D5, B10D5, B11D5, B1D6, B2D6, B3D6, B4D6, B5D6, B6D6, B7D6, B8D6, B9D6, B10D6, B11D6, B1D7, B2D7, B3D7, B4D7, B5D7, B6D7, B7D7, B8D7, B9D7, B10D7, B11D7, B1D8, B2D8, B3D8, B4D8, B5D8, B6D8, B7D8, B8D8, B9D8, B10D8, B11D8, B1D9, B2D9, B3D9, B4D9, B5D9, B6D9, B7D9, B8D9, B9D9, B10D9, B11D9, B1D10, B2D10, B3D10, B4D10, B5D10, B6D10, B7D10, B8D10, B9D10, B10D10, B11D10, B1D11, B2D11, B3D11, B4D11, BSD11, B6D11, B7D11, B8D11, B9D11, B10D11, B11D11, B1D12, B2D12, B3D12, B4D12, B5D12, B6D12, B7D12, B8D12, B9D12, B10D12, B11D12, B1D13, B2D13, B3D13, B4D13, B5D13, B6D13, B7D13, B8D13, B9D13, B10D13, B11D13, B1E1, B2E1, B3E1, B4E1, B5E1, B6E1, B7E1, B8E1, B9E1, B10E1, B11E1, B1E2, B2E2, B3E2, B4E2, B5E2, B6E2, B7E2, B8E2, B9E2, B10E2, B11E2, B1E3, B2E3, B3E3, B4E3, B5E3, B6E3, B7E3, B8E3, B9E3, B10E3, B11E3, B1E4, B2E4, B3E4, B4E4, B5E4, B6E4, B7E4, B8E4, B9E4, B10E4, B11E4, B1E5, B2E5, B3E5, B4E5, B5E5, B6E5, B7E5, B8E5, B9E5, B10E5, B1 1E5, B1E6, B2E6, B3E6, B4E6, B5E6, B6E6, B7E6, B8E6, B9E6, B10E6, B11E6, B1E7, B2E7, B3E7, B4E7, B5E7, B6E7, B7E7, B8E7, B9E7, B10E7, B11E7, B1E8, B2E8, B3E8, B4E8, B5E8, B6E8, B7E8, B8E8, B9E8, B10E8, B11E8, B1E9, B2E9, B3E9, B4E9, B5E9, B6E9, B7E9, B8E9, B9E9, B10E9, B11E9, B1E10, B2E10, B3E10, B4E10, B5E10, B6E10, B7E10, B8E10, B9E10, B10E10, B11E10, B1E11, B2E11, B3E11, B4E11, B5E11, B6E11, B7E11, B8E11, B9E11, B10E11, B11E11, B1E12, B2E12, B3E12, B4E12, BSE12, B6E12, B7E12, B8E12, B9E12, B10E12, B11E12, B1E13, B2E13, B3E13, B4E13, BSE13, B6E13, B7E13, B8E13, B9E13, B10E13, B11E13, B1F1, B2F1, B3F1, B4F1, B5F1, B6F1, B7F1, B8F1, B9F1, B10F1, B11F1, B1F2, B2F2, B3F2, B4F2, B5F2, B6F2, B7F2, B8F2, B9F2, B10F2, B11F2, B1F3, B2F3, B3F3, B4F3, B5F3, B6F3, B7F3, B8F3, B9F3, B10F3, B11F3, B1F4, B2F4, B3F4, B4F4, B5F4, B6F4, B7F4, B8F4, B9F4, B10F4, B11F4, B1F5, B2F5, B3F5, B4F5, B5F5, B6F5, B7F5, B8F5, B9F5, B10F5, B11F5, B1F6, B2F6, B3F6, B4F6, B5F6, B6F6, B7F6, B8F6, B9F6, B10F6, B11F6, B1F7, B2F7, B3F7, B4F7, B5F7, B6F7, B7F7, B8F7, B9F7, B10F7, B11F7, B1F8, B2F8, B3F8, B4F8, B5F8, B6F8, B7F8, B8F8, B9F8, B10F8, B11F8, B1F9, B2F9, B3F9, B4F9, B5F9, B6F9, B7F9, B8F9, B9F9, B10F9, B11F9, B1F10, B2F10, B3F10, B4F10, BSF10, B6F10, B7F10, B8F10, B9F10, B10F10, B11F10, B1F11, B2F11, B3F11, B4F11, BSF11, B6F11, B7F11, B8F11, B9F11, B10F11, B11F11, B1F12, B2F12, B3F12, B4F12, BSF12, B6F12, B7F12, B8F12, B9F12, B10F12, B11F12, B1F13, B2F13, B3F13, B4F13, B5F13, B6F13, B7F13, B8F13, B9F13, B10F13, B11F13, B1G1, B2G1, B3G1, B4G1, B5G1, B6G1, B7G1, B8G1, B9G1, B10G1, B11G1, B1G2, B2G2, B3G2, B4G2, B5G2, B6G2, B7G2, B8G2, B9G2, B10G2, B11G2, B1G3, B2G3, B3G3, B4G3, B5G3, B6G3, B7G3, B8G3, B9G3, B10G3, B11G3, B1G4, B2G4, B3G4, B4G4, BSG4, B6G4, B7G4, B8G4, B9G4, B10G4, B11G4, B1G5, B2G5, B3G5, B4G5, B5G5, B6G5, B7G5, B8G5, B9G5, B10G5, B11G5, B1G6, B2G6, B3G6, B4G6, B5G6, B6G6, B7G6, B8G6, B9G6, B10G6, B11G6, B1G7, B2G7, B3G7, B4G7, B5G7, B6G7, B7G7, B8G7, B9G7, B10G7, B11G7, B1G8, B2G8, B3G8, B4G8, B5G8, B6G8, B7G8, B8G8, B9G8, B10G8, B11G8, B1G9, B2G9, B3G9, B4G9, B5G9, B6G9, B7G9, B8G9, B9G9, B10G9, B11G9, B1G10, B2G10, B3G10, B4G10, B5G10, B6G10, B7G10, B8G10, B9G10, B10G10, B11G10, B1G11, B2G11, B3G11, B4G11, B5G11, B6G11, B7G11, B8G11, B9G11, B10G11, B11G11, B1G12, B2G12, B3G12, B4G12, B5G12, B6G12, B7G12, B8G12, B9G12, B10G12, B11G12, C1D1, C2D1, C3D1, C4D1, C5D1, C6D1, C7D1, C8D1, C9D1, C10D1, C11D1, C1D2, C2D2, C3D2, C4D2, C5D2, C6D2, C7D2, C8D2, C9D2, C10D2, C11D2, C1D3, C2D3, C3D3, C4D3, CSD3, C6D3, C7D3, C8D3, C9D3, C10D3, C11D3, C1D4, C2D4, C3D4, C4D4, C5D4, C6D4, C7D4, C8D4, C9D4, C10D4, C11D4, C1D5, C2D5, C3D5, C4D5, C5D5, C6D5, C7D5, C8D5, C9D5, C10D5, C11D5, C1D6, C2D6, C3D6, C4D6, C5D6, C6D6, C7D6, C8D6, C9D6, C10D6, C11D6, C1D7, C2D7, C3D7, C4D7, C5D7, C6D7, C7D7, C8D7, C9D7, C10D7, C11D7, C1D8, C2D8, C3D8, C4D8, C5D8, C6D8, C7D8, C8D8, C9D8, C10D8, C11D8, C1D9, C2D9, C3D9, C4D9, C5D9, C6D9, C7D9, C8D9, C9D9, C10D9, C11D9, C1D10, C2D10, C3D10, C4D10, C5D10, C6D10, C7D10, C8D10, C9D10, C10D10, C11D10, C1D11, C2D11, C3D11, C4D11, C5D11, C6D11, C7D11, C8D11, C9D11, C10D11, C11D11, C1D12, C2D12, C3D12, C4D12, C5D12, C6D12, C7D12, C8D12, C9D12, C10D12, C11D12, C1D13, C2D13, C3D13, C4D13, C5D13, C6D13, C7D13, C8D13, C9D13, C10D13, C11D13, C1E1, C2E1, C3E1, C4E1, C5E1, C6E1, C7E1, C8E1, C9E1, C10E1, C11E1, C1E2, C2E2, C3E2, C4E2, C5E2, C6E2, C7E2, C8E2, C9E2, C10E2, C11E2, C1E3, C2E3, C3E3, C4E3, C5E3, C6E3, C7E3, C8E3, C9E3, C10E3, C11E3, C1E4, C2E4, C3E4, C4E4, C5E4, C6E4, C7E4, C8E4, C9E4, C10E4, C11E4, C1E5, C2E5, C3E5, C4E5, C5E5, C6E5, C7E5, C8E5, C9E5, C10E5, C11E5, C1E6, C2E6, C3E6, C4E6, C5E6, C6E6, C7E6, C8E6, C9E6, C10E6, C11E6, C1E7, C2E7, C3E7, C4E7, C5E7, C6E7, C7E7, C8E7, C9E7, C10E7, C11E7, C1E8, C2E8, C3E8, C4E8, C5E8, C6E8, C7E8, C8E8, C9E8, C10E8, C11E8, C1E9, C2E9, C3E9, C4E9, C5E9, C6E9, C7E9, C8E9, C9E9, C10E9, C11E9, C1E10, C2E10, C3E10, C4E10, C5E10, C6E10, C7E10, C8E10, C9E10, C10E10, C11E10, C1E11, C2E11, C3E11, C4E11, C5E11, C6E11, C7E11, C8E11, C9E11, C10E11, C11E11, C1E12, C2E12, C3E12, C4E12, C5E12, C6E12, C7E12, C8E12, C9E12, C10E12, C11E12, C1E13, C2E13, C3E13, C4E13, C5E13, C6E13, C7E13, C8E13, C9E13, C10E13, C11E13, C1F1, C2F1, C3F1, C4F1, C5F1, C6F1, C7F1, C8F1, C9F1, C10F1, C11F1, C1F2, C2F2, C3F2, C4F2, C5F2, C6F2, C7F2, C8F2, C9F2, C10F2, C11F2, C1F3, C2F3, C3F3, C4F3, C5F3, C6F3, C7F3, C8F3, C9F3, C10F3, C11F3, C1F4, C2F4, C3F4, C4F4, C5F4, C6F4, C7F4, C8F4, C9F4, C10F4, C11F4, C1F5, C2F5, C3F5, C4F5, C5F5, C6F5, C7F5, C8F5, C9F5, C10F5, C11F5, C1F6, C2F6, C3F6, C4F6, C5F6, C6F6, C7F6, C8F6, C9F6, C10F6, C11F6, C1F7, C2F7, C3F7, C4F7, C5F7, C6F7, C7F7, C8F7, C9F7, C10F7, C11F7, C1F8, C2F8, C3F8, C4F8, C5F8, C6F8, C7F8, C8F8, C9F8, C10F8, C11F8, C1F9, C2F9, C3F9, C4F9, C5F9, C6F9, C7F9, C8F9, C9F9, C10F9, C11F9, C1F10, C2F10, C3F10, C4F10, CSF10, C6F10, C7F10, C8F10, C9F10, C10F10, C11F10, C1F11, C2F11, C3F11, C4F11, CSF11, C6F11, C7F11, C8F11, C9F11, C10F11, C11F11, C1F12, C2F12, C3F12, C4F12, CSF12, C6F12, C7F12, C8F12, C9F12, C10F12, C11F12, C1F13, C2F13, C3F13, C4F13, C5F13, C6F13, C7F13, C8F13, C9F13, C10F13, C11F13, C1G1, C2G1, C3G1, C4G1, C5G1, C6G1, C7G1, C8G1, C9G1, C10G1, C11G1, C1G2, C2G2, C3G2, C4G2, CSG2, C6G2, C7G2, C8G2, C9G2, C10G2, C11G2, C1G3, C2G3, C3G3, C4G3, CSG3, C6G3, C7G3, C8G3, C9G3, C10G3, C11G3, C1G4, C2G4, C3G4, C4G4, CSG4, C6G4, C7G4, C8G4, C9G4, C10G4, C11G4, C1G5, C2G5, C3G5, C4G5, C5G5, C6G5, C7G5, C8G5, C9G5, C10G5, C11G5, C1G6, C2G6, C3G6, C4G6, C5G6, C6G6, C7G6, C8G6, C9G6, C10G6, C11G6, C1G7, C2G7, C3G7, C4G7, C5G7, C6G7, C7G7, C8G7, C9G7, C10G7, C11G7, C1G8, C2G8, C3G8, C4G8, C5G8, C6G8, C7G8, C8G8, C9G8, C10G8, C11G8, C1G9, C2G9, C3G9, C4G9, C5G9, C6G9, C7G9, C8G9, C9G9, C10G9, C11G9, C1G10, C2G10, C3G10, C4G10, C5G10, C6G10, C7G10, C8G10, C9G10, C10G10, C11G10, C1G11, C2G11, C3G11, C4G11, C5G11, C6G11, C7G11, C8G11, C9G11, C10G11, C11G11, C1G12, C2G12, C3G12, C4G12, C5G12, C6G12, C7G12, C8G12, C9G12, C10G12, C11G12, B12D1, B12D2, B12D3, B12D4, B12D5, B12D6, B12D7, B12D8, B12D9, B12D10, B12D11, B12D12, B12D13, B12E1, B12E2, B12E3, B12E4, B12E5, B12E6, B12E7, B12E8, B12E9, B12E10, B12E11, B12E12, B12E13, B12F1, B12F2, B12F3, B12F4, B12F5, B12F6, B12F7, B12F8, B12F9, B12F10, B12F11, B12F12, B12F13, B12G1, B12G2, B12G3, B12G4, B12G5, B12G6, B12G7, B12G8, B12G9, B12G10, B12G11, B12G12, C12D1, C12D2, C12D3, C12D4, C12D5, C12D6, C12D7, C12D8, C12D9, C12D10, C12D11, C12D12, C12D13, C12E1, C12E2, C12E3, C12E4, C12E5, C12E6, C12E7, C12E8, C12E9, C12E10, C12E11, C12E12, C12E13, C12F1, C12F2, C12F3, C12F4, C12F5, C12F6, C12F7, C12F8, C12F9, C12F10, C12F11, C12F12, C12F13, C12G1, C12G2, C12G3, C12G4, C12G5, C12G6, C12G7, C12G8, C12G9, C12G10, C12G11, or C12G12. In further embodiments, the compound may be a compound of formula A1G13, A1D14, A1E14, A1F14, A1G14, A1D15, A1E15, A1F15, A1G15, A1D16, A1E16, A1F16, A1G16, A1D17, A1E17, A1F17, A1G17, A1D18, A1E18, A1F18, A1G18, A1D19, A1E19, A1F19, A1G19, A1D20, A1E20, A1F20, A1G20, A1D21, A1E21, A1F21, A1G21, A1D22, A1E22, A1F22, A1G22, A1D23, A1E23, A1F23, A1G23, A1D24, A1E24, A1F24, A1G24, A1D25, A1E25, A1F25, A1G25, A1D26, A1E26, A1F26, A1G26, A1D27, A1E27, A1F27, A1G27, A1D28, A1E28, A1F28, A1G28, A1D29, A1E29, A1F29, A1G29, A1D30, A1E30, A1F30, A1G30, A1D31, A1E31, A1F31, A1G31, A1D32, A1E32, A1F32, A1G32, A1D33, A1E33, A1F33, A1G33, A1D34, A1E34, A1F34, A1G34, A1D35, A1E35, A1F35, A1G35, A1D36, A1E36, A1F36, A1G36, A1D37, A1E37, A1F37, A1G37, A1D38, A1E38, A1F38, A1G38, A1D39, A1E39, A1F39, A1G39, A1D40, A1E40, A1F40, A1G40, A2G13, A2D14, A2E14, A2F14, A2G14, A2D15, A2E15, A2F15, A2G15, A2D16, A2E16, A2F16, A2G16, A2D17, A2E17, A2F17, A2G17, A2D18, A2E18, A2F18, A2G18, A2D19, A2E19, A2F19, A2G19, A2D20, A2E20, A2F20, A2G20, A2D21, A2E21, A2F21, A2G21, A2D22, A2E22, A2F22, A2G22, A2D23, A2E23, A2F23, A2G23, A2D24, A2E24, A2F24, A2G24, A2D25, A2E25, A2F25, A2G25, A2D26, A2E26, A2F26, A2G26, A2D27, A2E27, A2F27, A2G27, A2D28, A2E28, A2F28, A2G28, A2D29, A2E29, A2F29, A2G29, A2D30, A2E30, A2F30, A2G30, A2D31, A2E31, A2F31, A2G31, A2D32, A2E32, A2F32, A2G32, A2D33, A2E33, A2F33, A2G33, A2D34, A2E34, A2F34, A2G34, A2D35, A2E35, A2F35, A2G35, A2D36, A2E36, A2F36, A2G36, A2D37, A2E37, A2F37, A2G37, A2D38, A2E38, A2F38, A2G38, A2D39, A2E39, A2F39, A2G39, A2D40, A2E40, A2F40, A2G40, A3G13, A3D14, A3E14, A3F14, A3G14, A3D15, A3E15, A3F15, A3G15, A3D16, A3E16, A3F16, A3G16, A3D17, A3E17, A3F17, A3G17, A3D18, A3E18, A3F18, A3G18, A3D19, A3E19, A3F19, A3G19, A3D20, A3E20, A3F20, A3G20, A3D21, A3E21, A3F21, A3G21, A3D22, A3E22, A3F22, A3G22, A3D23, A3E23, A3F23, A3G23, A3D24, A3E24, A3F24, A3G24, A3D25, A3E25, A3F25, A3G25, A3D26, A3E26, A3F26, A3G26, A3D27, A3E27, A3F27, A3G27, A3D28, A3E28, A3F28, A3G28, A3D29, A3E29, A3F29, A3G29, A3D30, A3E30, A3F30, A3G30, A3D31, A3E31, A3F31, A3G31, A3D32, A3E32, A3F32, A3G32, A3D33, A3E33, A3F33, A3G33, A3D34, A3E34, A3F34, A3G34, A3D35, A3E35, A3F35, A3G35, A3D36, A3E36, A3F36, A3G36, A3D37, A3E37, A3F37, A3G37, A3D38, A3E38, A3F38, A3G38, A3D39, A3E39, A3F39, A3G39, A3D40, A3E40, A3F40, A3G40, A4G13, A4D14, A4E14, A4F14, A4G14, A4D15, A4E15, A4F15, A4G15, A4D16, A4E16, A4F16, A4G16, A4D17, A4E17, A4F17, A4G17, A4D18, A4E18, A4F18, A4G18, A4D19, A4E19, A4F19, A4G19, A4D20, A4E20, A4F20, A4G20, A4D21, A4E21, A4F21, A4G21, A4D22, A4E22, A4F22, A4G22, A4D23, A4E23, A4F23, A4G23, A4D24, A4E24, A4F24, A4G24, A4D25, A4E25, A4F25, A4G25, A4D26, A4E26, A4F26, A4G26, A4D27, A4E27, A4F27, A4G27, A4D28, A4E28, A4F28, A4G28, A4D29, A4E29, A4F29, A4G29, A4D30, A4E30, A4F30, A4G30, A4D31, A4E31, A4F31, A4G31, A4D32, A4E32, A4F32, A4G32, A4D33, A4E33, A4F33, A4G33, A4D34, A4E34, A4F34, A4G34, A4D35, A4E35, A4F35, A4G35, A4D36, A4E36, A4F36, A4G36, A4D37, A4E37, A4F37, A4G37, A4D38, A4E38, A4F38, A4G38, A4D39, A4E39, A4F39, A4G39, A4D40, A4E40, A4F40, A4G40, A5G13, A5D14, A5E14, A5F14, A5G14, A5D15, A5E15, A5F15, A5G15, A5D16, A5E16, A5F16, A5G16, A5D17, A5E17, A5F17, A5G17, A5D18, A5E18, A5F18, A5G18, A5D19, A5E19, A5F19, A5G19, A5D20, A5E20, A5F20, A5G20, A5D21, A5E21, A5F21, A5G21, A5D22, A5E22, A5F22, A5G22, A5D23, A5E23, A5F23, A5G23, A5D24, A5E24, A5F24, A5G24, A5D25, A5E25, A5F25, A5G25, A5D26, A5E26, A5F26, A5G26, A5D27, A5E27, A5F27, A5G27, A5D28, A5E28, A5F28, A5G28, A5D29, A5E29, A5F29, A5G29, A5D30, A5E30, A5F30, A5G30, ASD31, A5E31, A5F31, A5G31, A5D32, A5E32, A5F32, A5G32, A5D33, A5E33, A5F33, A5G33, A5D34, A5E34, A5F34, A5G34, A5D35, A5E35, A5F35, A5G35, A5D36, A5E36, A5F36, A5G36, A5D37, A5E37, A5F37, A5G37, A5D38, A5E38, A5F38, A5G38, A5D39, A5E39, A5F39, A5G39, A5D40, A5E40, A5F40, A5G40, A6G13, A6D14, A6E14, A6F14, A6G14, A6D15, A6E15, A6F15, A6G15, A6D16, A6E16, A6F16, A6G16, A6D17, A6E17, A6F17, A6G17, A6D18, A6E18, A6F18, A6G18, A6D19, A6E19, A6F19, A6G19, A6D20, A6E20, A6F20, A6G20, A6D21, A6E21, A6F21, A6G21, A6D22, A6E22, A6F22, A6G22, A6D23, A6E23, A6F23, A6G23, A6D24, A6E24, A6F24, A6G24, A6D25, A6E25, A6F25, A6G25, A6D26, A6E26, A6F26, A6G26, A6D27, A6E27, A6F27, A6G27, A6D28, A6E28, A6F28, A6G28, A6D29, A6E29, A6F29, A6G29, A6D30, A6E30, A6F30, A6G30, A6D31, A6E31, A6F31, A6G31, A6D32, A6E32, A6F32, A6G32, A6D33, A6E33, A6F33, A6G33, A6D34, A6E34, A6F34, A6G34, A6D35, A6E35, A6F35, A6G35, A6D36, A6E36, A6F36, A6G36, A6D37, A6E37, A6F37, A6G37, A6D38, A6E38, A6F38, A6G38, A6D39, A6E39, A6F39, A6G39, A6D40, A6E40, A6F40, A6G40, A7G13, A7D14, A7E14, A7F14, A7G14, A7D15, A7E15, A7F15, A7G15, A7D16, A7E16, A7F16, A7G16, A7D17, A7E17, A7F17, A7G17, A7D18, A7E18, A7F18, A7G18, A7D19, A7E19, A7F19, A7G19, A7D20, A7E20, A7F20, A7G20, A7D21, A7E21, A7F21, A7G21, A7D22, A7E22, A7F22, A7G22, A7D23, A7E23, A7F23, A7G23, A7D24, A7E24, A7F24, A7G24, A7D25, A7E25, A7F25, A7G25, A7D26, A7E26, A7F26, A7G26, A7D27, A7E27, A7F27, A7G27, A7D28, A7E28, A7F28, A7G28, A7D29, A7E29, A7F29, A7G29, A7D30, A7E30, A7F30, A7G30, A7D31, A7E31, A7F31, A7G31, A7D32, A7E32, A7F32, A7G32, A7D33, A7E33, A7F33, A7G33, A7D34, A7E34, A7F34, A7G34, A7D35, A7E35, A7F35, A7G35, A7D36, A7E36, A7F36, A7G36, A7D37, A7E37, A7F37, A7G37, A7D38, A7E38, A7F38, A7G38, A7D39, A7E39, A7F39, A7G39, A7D40, A7E40, A7F40, A7G40, A8G13, A8D14, A8E14, A8F14, A8G14, A8D15, A8E15, A8F15, A8G15, A8D16, A8E16, A8F16, A8G16, A8D17, A8E17, A8F17, A8G17, A8D18, A8E18, A8F18, A8G18, A8D19, A8E19, A8F19, A8G19, A8D20, A8E20, A8F20, A8G20, A8D21, A8E21, A8F21, A8G21, A8D22, A8E22, A8F22, A8G22, A8D23, A8E23, A8F23, A8G23, A8D24, A8E24, A8F24, A8G24, A8D25, A8E25, A8F25, A8G25, A8D26, A8E26, A8F26, A8G26, A8D27, A8E27, A8F27, A8G27, A8D28, A8E28, A8F28, A8G28, A8D29, A8E29, A8F29, A8G29, A8D30, A8E30, A8F30, A8G30, A8D31, A8E31, A8F31, A8G31, A8D32, A8E32, A8F32, A8G32, A8D33, A8E33, A8F33, A8G33, A8D34, A8E34, A8F34, A8G34, A8D35, A8E35, A8F35, A8G35, A8D36, A8E36, A8F36, A8G36, A8D37, A8E37, A8F37, A8G37, A8D38, A8E38, A8F38, A8G38, A8D39, A8E39, A8F39, A8G39, A8D40, A8E40, A8F40, A8G40, A9G13, A9D14, A9E14, A9F14, A9G14, A9D15, A9E15, A9F15, A9G15, A9D16, A9E16, A9F16, A9G16, A9D17, A9E17, A9F17, A9G17, A9D18, A9E18, A9F18, A9G18, A9D19, A9E19, A9F19, A9G19, A9D20, A9E20, A9F20, A9G20, A9D21, A9E21, A9F21, A9G21, A9D22, A9E22, A9F22, A9G22, A9D23, A9E23, A9F23, A9G23, A9D24, A9E24, A9F24, A9G24, A9D25, A9E25, A9F25, A9G25, A9D26, A9E26, A9F26, A9G26, A9D27, A9E27, A9F27, A9G27, A9D28, A9E28, A9F28, A9G28, A9D29, A9E29, A9F29, A9G29, A9D30, A9E30, A9F30, A9G30, A9D31, A9E31, A9F31, A9G31, A9D32, A9E32, A9F32, A9G32, A9D33, A9E33, A9F33, A9G33, A9D34, A9E34, A9F34, A9G34, A9D35, A9E35, A9F35, A9G35, A9D36, A9E36, A9F36, A9G36, A9D37, A9E37, A9F37, A9G37, A9D38, A9E38, A9F38, A9G38, A9D39, A9E39, A9F39, A9G39, A9D40, A9E40, A9F40, A9G40, A10G13, A10D14, A10E14, A10F14, A10G14, A10D15, A10E15, A10F15, A10G15, A10D16, A10E16, A10F16, A10G16, A10D17, A10E17, A10F17, A10G17, A10D18, A10E18, A10F18, A10G18, A10D19, A10E19, A10F19, A10G19, A10D20, A10E20, A10F20, A10G20, A10D21, A10E21, A10F21, A10G21, A10D22, A10E22, A10F22, A10G22, A10D23, A10E23, A10F23, A10G23, A10D24, A10E24, A10F24, A10G24, A10D25, A10E25, A10F25, A10G25, A10D26, A10E26, A10F26, A10G26, A10D27, A10E27, A10F27, A10G27, A10D28, A10E28, A10F28, A10G28, A10D29, A10E29, A10F29, A10G29, A10D30, A10E30, A10F30, A10G30, A10D31, A10E31, A10F31, A10G31, A10D32, A10E32, A10F32, A10G32, A10D33, A10E33, A10F33, A10G33, A10D34, A10E34, A10F34, A10G34, A10D35, A10E35, A10F35, A10G35, A10D36, A10E36, A10F36, A10G36, A10D37, A10E37, A10F37, A10G37, A10D38, A10E38, A10F38, A10G38, A10D39, A10E39, A10F39, A10G39, A10D40, A10E40, A10F40, A10G40, A11G13, A11D14, A11E14, A11F14, A11G14, A11D15, A11E15, A11F15, A11G15, A11D16, A11E16, A11F16, A11G16, A11D17, A11E17, A11F17, A11G17, A11D18, A11E18, A11F18, A11G18, A11D19, A11E19, A11F19, A11G19, A11D20, A11E20, A11F20, A11G20, A11D21, A11E21, A11F21, A11G21, A11D22, A11E22, A11F22, A11G22, A11D23, A11E23, A11F23, A11G23, A11D24, A11E24, A11F24, A11G24, A11D25, A11E25, A11F25, A11G25, A11D26, A11E26, A11F26, A11G26, A11D27, A11E27, A11F27, A11G27, A11D28, A11E28, A11F28, A11G28, A11D29, A11E29, A11F29, A11G29, A11D30, A11E30, A11F30, A11G30, A11D31, A11E31, A11F31, A11G31, A11D32, A11E32, A11F32, A11G32, A11D33, A11E33, A11F33, A11G33, A11D34, A11E34, A11F34, A11G34, A11D35, A11E35, A11F35, A11G35, A11D36, A11E36, A11F36, A11G36, A11D37, A11E37, A11F37, A11G37, A11D38, A11E38, A11F38, A11G38, A11D39, A11E39, A11F39, A11G39, A11D40, A11E40, A11F40, A11G40, A12G13, A12D14, A12E14, A12F14, A12G14, A12D15, A12E15, A12F15, A12G15, A12D16, A12E16, A12F16, A12G16, A12D17, A12E17, A12F17, A12G17, A12D18, A12E18, A12F18, A12G18, A12D19, A12E19, A12F19, A12G19, A12D20, A12E20, A12F20, A12G20, A12D21, A12E21, A12F21, A12G21, A12D22, A12E22, A12F22, A12G22, A12D23, A12E23, A12F23, A12G23, A12D24, A12E24, A12F24, A12G24, A12D25, A12E25, A12F25, A12G25, A12D26, A12E26, A12F26, A12G26, A12D27, A12E27, A12F27, A12G27, A12D28, A12E28, A12F28, A12G28, A12D29, A12E29, A12F29, A12G29, A12D30, A12E30, A12F30, A12G30, A12D31, A12E31, A12F31, A12G31, A12D32, A12E32, A12F32, A12G32, A12D33, A12E33, A12F33, A12G33, A12D34, A12E34, A12F34, A12G34, A12D35, A12E35, A12F35, A12G35, A12D36, A12E36, A12F36, A12G36, A12D37, A12E37, A12F37, A12G37, A12D38, A12E38, A12F38, A12G38, A12D39, A12E39, A12F39, A12G39, A12D40, A12E40, A12F40, A12G40, B1G13, B1D14, B1E14, B1F14, B1G14, B1D15, B1E15, B1F15, B1G15, B1D16, B1E16, B1F16, B1G16, B1D17, B1E17, B1F17, B1G17, B1D18, B1E18, B1F18, B1G18, B1D19, B1E19, B1F19, B1G19, B1D20, B1E20, B1F20, B1G20, B1D21, B1E21, B1F21, B1G21, B1D22, B1E22, B1F22, B1G22, B1D23, B1E23, B1F23, B1G23, B1D24, B1E24, B1F24, B1G24, B1D25, B1E25, B1F25, B1G25, B1D26, B1E26, B1F26, B1G26, B1D27, B1E27, B1F27, B1G27, B1D28, B1E28, B1F28, B1G28, B1D29, B1E29, B1F29, B1G29, B1D30, B1E30, B1F30, B1G30, B1D31, B1E31, B1F31, B1G31, B1D32, B1E32, B1F32, B1G32, B1D33, B1E33, B1F33, B1G33, B1D34, B1E34, B1F34, B1G34, B1D35, B1E35, B1F35, B1G35, B1D36, B1E36, B1F36, B1G36, B1D37, B1E37, B1F37, B1G37, B1D38, B1E38, B1F38, B1G38, B1D39, B1E39, B1F39, B1G39, B1D40, B1E40, B1F40, B1G40, B2G13, B2D14, B2E14, B2F14, B2G14, B2D15, B2E15, B2F15, B2G15, B2D16, B2E16, B2F16, B2G16, B2D17, B2E17, B2F17, B2G17, B2D18, B2E18, B2F18, B2G18, B2D19, B2E19, B2F19, B2G19, B2D20, B2E20, B2F20, B2G20, B2D21, B2E21, B2F21, B2G21, B2D22, B2E22, B2F22, B2G22, B2D23, B2E23, B2F23, B2G23, B2D24, B2E24, B2F24, B2G24, B2D25, B2E25, B2F25, B2G25, B2D26, B2E26, B2F26, B2G26, B2D27, B2E27, B2F27, B2G27, B2D28, B2E28, B2F28, B2G28, B2D29, B2E29, B2F29, B2G29, B2D30, B2E30, B2F30, B2G30, B2D31, B2E31, B2F31, B2G31, B2D32, B2E32, B2F32, B2G32, B2D33, B2E33, B2F33, B2G33, B2D34, B2E34, B2F34, B2G34, B2D35, B2E35, B2F35, B2G35, B2D36, B2E36, B2F36, B2G36, B2D37, B2E37, B2F37, B2G37, B2D38, B2E38, B2F38, B2G38, B2D39, B2E39, B2F39, B2G39, B2D40, B2E40, B2F40, B2G40, B3G13, B3D14, B3E14, B3F14, B3G14, B3D15, B3E15, B3F15, B3G15, B3D16, B3E16, B3F16, B3G16, B3D17, B3E17, B3F17, B3G17, B3D18, B3E18, B3F18, B3G18, B3D19, B3E19, B3F19, B3G19, B3D20, B3E20, B3F20, B3G20, B3D21, B3E21, B3F21, B3G21, B3D22, B3E22, B3F22, B3G22, B3D23, B3E23, B3F23, B3G23, B3D24, B3E24, B3F24, B3G24, B3D25, B3E25, B3F25, B3G25, B3D26, B3E26, B3F26, B3G26, B3D27, B3E27, B3F27, B3G27, B3D28, B3E28, B3F28, B3G28, B3D29, B3E29, B3F29, B3G29, B3D30, B3E30, B3F30, B3G30, B3D31, B3E31, B3F31, B3G31, B3D32, B3E32, B3F32, B3G32, B3D33, B3E33, B3F33, B3G33, B3D34, B3E34, B3F34, B3G34, B3D35, B3E35, B3F35, B3G35, B3D36, B3E36, B3F36, B3G36, B3D37, B3E37, B3F37, B3G37, B3D38, B3E38, B3F38, B3G38, B3D39, B3E39, B3F39, B3G39, B3D40, B3E40, B3F40, B3G40, B4G13, B4D14, B4E14, B4F14, B4G14, B4D15, B4E15, B4F15, B4G15, B4D16, B4E16, B4F16, B4G16, B4D17, B4E17, B4F17, B4G17, B4D18, B4E18, B4F18, B4G18, B4D19, B4E19, B4F19, B4G19, B4D20, B4E20, B4F20, B4G20, B4D21, B4E21, B4F21, B4G21, B4D22, B4E22, B4F22, B4G22, B4D23, B4E23, B4F23, B4G23, B4D24, B4E24, B4F24, B4G24, B4D25, B4E25, B4F25, B4G25, B4D26, B4E26, B4F26, B4G26, B4D27, B4E27, B4F27, B4G27, B4D28, B4E28, B4F28, B4G28, B4D29, B4E29, B4F29, B4G29, B4D30, B4E30, B4F30, B4G30, B4D31, B4E31, B4F31, B4G31, B4D32, B4E32, B4F32, B4G32, B4D33, B4E33, B4F33, B4G33, B4D34, B4E34, B4F34, B4G34, B4D35, B4E35, B4F35, B4G35, B4D36, B4E36, B4F36, B4G36, B4D37, B4E37, B4F37, B4G37, B4D38, B4E38, B4F38, B4G38, B4D39, B4E39, B4F39, B4G39, B4D40, B4E40, B4F40, B4G40, B5G13, B5D14, B5E14, B5F14, B5G14, B5D15, B5E15, B5F15, B5G15, B5D16, B5E16, B5F16, B5G16, B5D17, B5E17, B5F17, B5G17, B5D18, B5E18, B5F18, B5G18, B5D19, B5E19, B5F19, B5G19, B5D20, B5E20, BSF20, BSG20, BSD21, BSE21, BSF21, BSG21, BSD22, BSE22, BSF22, BSG22, BSD23, BSE23, BSF23, BSG23, BSD24, B5E24, BSF24, BSG24, BSD25, BSE25, BSF25, BSG25, BSD26, BSE26, BSF26, BSG26, BSD27, BSE27, BSF27, BSG27, BSD28, BSE28, BSF28, BSG28, BSD29, BSE29, BSF29, BSG29, BSD30, BSE30, BSF30, BSG30, BSD31, BSE31, BSF31, BSG31, B5D32, B5E32, B5F32, B5G32, B5D33, B5E33, B5F33, B5G33, B5D34, B5E34, B5F34, B5G34, B5D35, B5E35, B5F35, B5G35, B5D36, B5E36, B5F36, B5G36, B5D37, B5E37, B5F37, B5G37, BSD38, BSE38, BSF38, BSG38, BSD39, BSE39, BSF39, BSG39, BSD40, BSE40, BSF40, BSG40, B6G13, B6D14, B6E14, B6F14, B6G14, B6D15, B6E15, B6F15, B6G15, B6D16, B6E16, B6F16, B6G16, B6D17, B6E17, B6F17, B6G17, B6D18, B6E18, B6F18, B6G18, B6D19, B6E19, B6F19, B6G19, B6D20, B6E20, B6F20, B6G20, B6D21, B6E21, B6F21, B6G21, B6D22, B6E22, B6F22, B6G22, B6D23, B6E23, B6F23, B6G23, B6D24, B6E24, B6F24, B6G24, B6D25, B6E25, B6F25, B6G25, B6D26, B6E26, B6F26, B6G26, B6D27, B6E27, B6F27, B6G27, B6D28, B6E28, B6F28, B6G28, B6D29, B6E29, B6F29, B6G29, B6D30, B6E30, B6F30, B6G30, B6D31, B6E31, B6F31, B6G31, B6D32, B6E32, B6F32, B6G32, B6D33, B6E33, B6F33, B6G33, B6D34, B6E34, B6F34, B6G34, B6D35, B6E35, B6F35, B6G35, B6D36, B6E36, B6F36, B6G36, B6D37, B6E37, B6F37, B6G37, B6D38, B6E38, B6F38, B6G38, B6D39, B6E39, B6F39, B6G39, B6D40, B6E40, B6F40, B6G40, B7G13, B7D14, B7E14, B7F14, B7G14, B7D15, B7E15, B7F15, B7G15, B7D16, B7E16, B7F16, B7G16, B7D17, B7E17, B7F17, B7G17, B7D18, B7E18, B7F18, B7G18, B7D19, B7E19, B7F19, B7G19, B7D20, B7E20, B7F20, B7G20, B7D21, B7E21, B7F21, B7G21, B7D22, B7E22, B7F22, B7G22, B7D23, B7E23, B7F23, B7G23, B7D24, B7E24, B7F24, B7G24, B7D25, B7E25, B7F25, B7G25, B7D26, B7E26, B7F26, B7G26, B7D27, B7E27, B7F27, B7G27, B7D28, B7E28, B7F28, B7G28, B7D29, B7E29, B7F29, B7G29, B7D30, B7E30, B7F30, B7G30, B7D31, B7E31, B7F31, B7G31, B7D32, B7E32, B7F32, B7G32, B7D33, B7E33, B7F33, B7G33, B7D34, B7E34, B7F34, B7G34, B7D35, B7E35, B7F35, B7G35, B7D36, B7E36, B7F36, B7G36, B7D37, B7E37, B7F37, B7G37, B7D38, B7E38, B7F38, B7G38, B7D39, B7E39, B7F39, B7G39, B7D40, B7E40, B7F40, B7G40, B8G13, B8D14, B8E14, B8F14, B8G14, B8D15, B8E15, B8F15, B8G15, B8D16, B8E16, B8F16, B8G16, B8D17, B8E17, B8F17, B8G17, B8D18, B8E18, B8F18, B8G18, B8D19, B8E19, B8F19, B8G19, B8D20, B8E20, B8F20, B8G20, B8D21, B8E21, B8F21, B8G21, B8D22, B8E22, B8F22, B8G22, B8D23, B8E23, B8F23, B8G23, B8D24, B8E24, B8F24, B8G24, B8D25, B8E25, B8F25, B8G25, B8D26, B8E26, B8F26, B8G26, B8D27, B8E27, B8F27, B8G27, B8D28, B8E28, B8F28, B8G28, B8D29, B8E29, B8F29, B8G29, B8D30, B8E30, B8F30, B8G30, B8D31, B8E31, B8F31, B8G31, B8D32, B8E32, B8F32, B8G32, B8D33, B8E33, B8F33, B8G33, B8D34, B8E34, B8F34, B8G34, B8D35, B8E35, B8F35, B8G35, B8D36, B8E36, B8F36, B8G36, B8D37, B8E37, B8F37, B8G37, B8D38, B8E38, B8F38, B8G38, B8D39, B8E39, B8F39, B8G39, B8D40, B8E40, B8F40, B8G40, B9G13, B9D14, B9E14, B9F14, B9G14, B9D15, B9E15, B9F15, B9G15, B9D16, B9E16, B9F16, B9G16, B9D17, B9E17, B9F17, B9G17, B9D18, B9E18, B9F18, B9G18, B9D19, B9E19, B9F19, B9G19, B9D20, B9E20, B9F20, B9G20, B9D21, B9E21, B9F21, B9G21, B9D22, B9E22, B9F22, B9G22, B9D23, B9E23, B9F23, B9G23, B9D24, B9E24, B9F24, B9G24, B9D25, B9E25, B9F25, B9G25, B9D26, B9E26, B9F26, B9G26, B9D27, B9E27, B9F27, B9G27, B9D28, B9E28, B9F28, B9G28, B9D29, B9E29, B9F29, B9G29, B9D30, B9E30, B9F30, B9G30, B9D31, B9E31, B9F31, B9G31, B9D32, B9E32, B9F32, B9G32, B9D33, B9E33, B9F33, B9G33, B9D34, B9E34, B9F34, B9G34, B9D35, B9E35, B9F35, B9G35, B9D36, B9E36, B9F36, B9G36, B9D37, B9E37, B9F37, B9G37, B9D38, B9E38, B9F38, B9G38, B9D39, B9E39, B9F39, B9G39, B9D40, B9E40, B9F40, B9G40, B10G13, B10D14, B10E14, B10F14, B10G14, B10D15, B10E15, B10F15, B10G15, B10D16, B10E16, B10F16, B10G16, B10D17, B10E17, B10F17, B10G17, B10D18, B10E18, B10F18, B10G18, B10D19, B10E19, B10F19, B10G19, B10D20, B10E20, B10F20, B10G20, B10D21, B10E21, B10F21, B10G21, B10D22, B10E22, B10F22, B10G22, B10D23, B10E23, B10F23, B10G23, B10D24, B10E24, B10F24, B10G24, B10D25, B10E25, B10F25, B10G25, B10D26, B10E26, B10F26, B10G26, B10D27, B10E27, B10F27, B10G27, B10D28, B10E28, B10F28, B10G28, B10D29, B10E29, B10F29, B10G29, B10D30, B10E30, B10F30, B10G30, B10D31, B10E31, B10F31, B10G31, B10D32, B10E32, B10F32, B10G32, B10D33, B10E33, B10F33, B10G33, B10D34, B10E34, B10F34, B10G34, B10D35, B10E35, B10F35, B10G35, B10D36, B10E36, B10F36, B10G36, B10D37, B10E37, B10F37, B10G37, B10D38, B10E38, B10F38, B10G38, B10D39, B10E39, B10F39, B10G39, B10D40, B10E40, B10F40, B10G40, B11G13, B11D14, B11E14, B11F14, B11G14, B11D15, B11E15, B11F15, B11G15, B11D16, B11E16, B11F16, B11G16, B11D17, B11E17, B11F17, B11G17, B11D18, B11E18, B11F18, B11G18, B11D19, B11E19, B11F19, B11G19, B11D20, B11E20, B11F20, B11G20, B11D21, B11E21, B11F21, B11G21, B11D22, B11E22, B11F22, B11G22, B11D23, B11E23, B11F23, B11G23, B11D24, B11E24, B11F24, B11G24, B11D25, B11E25, B11F25, B11G25, B11D26, B11E26, B11F26, B11G26, B11D27, B11E27, B11F27, B11G27, B11D28, B11E28, B11F28, B11G28, B11D29, B11E29, B11F29, B11G29, B11D30, B11E30, B11F30, B11G30, B11D31, B11E31, B11F31, B11G31, B11D32, B11E32, B11F32, B11G32, B11D33, B11E33, B11F33, B11G33, B11D34, B11E34, B11F34, B11G34, B11D35, B11E35, B11F35, B11G35, B11D36, B11E36, B11F36, B11G36, B11D37, B11E37, B11F37, B11G37, B11D38, B11E38, B11F38, B11G38, B11D39, B11E39, B11F39, B11G39, B11D40, B11E40, B11F40, B11G40, B12G13, B12D14, B12E14, B12F14, B12G14, B12D15, B12E15, B12F15, B12G15, B12D16, B12E16, B12F16, B12G16, B12D17, B12E17, B12F17, B12G17, B12D18, B12E18, B12F18, B12G18, B12D19, B12E19, B12F19, B12G19, B12D20, B12E20, B12F20, B12G20, B12D21, B12E21, B12F21, B12G21, B12D22, B12E22, B12F22, B12G22, B12D23, B12E23, B12F23, B12G23, B12D24, B12E24, B12F24, B12G24, B12D25, B12E25, B12F25, B12G25, B12D26, B12E26, B12F26, B12G26, B12D27, B12E27, B12F27, B12G27, B12D28, B12E28, B12F28, B12G28, B12D29, B12E29, B12F29, B12G29, B12D30, B12E30, B12F30, B12G30, B12D31, B12E31, B12F31, B12G31, B12D32, B12E32, B12F32, B12G32, B12D33, B12E33, B12F33, B12G33, B12D34, B12E34, B12F34, B12G34, B12D35, B12E35, B12F35, B12G35, B12D36, B12E36, B12F36, B12G36, B12D37, B12E37, B12F37, B12G37, B12D38, B12E38, B12F38, B12G38, B12D39, B12E39, B12F39, B12G39, B12D40, B12E40, B12F40, B12G40, C1G13, C1D14, C1E14, C1F14, C1G14, C1D15, C1E15, C1F15, C1G15, C1D16, C1E16, C1F16, C1G16, C1D17, C1E17, C1F17, C1G17, C1D18, C1E18, C1F18, C1G18, C1D19, C1E19, C1F19, C1G19, C1D20, C1E20, C1F20, C1G20, C1D21, C1E21, C1F21, C1G21, C1D22, C1E22, C1F22, C1G22, C1D23, C1E23, C1F23, C1G23, C1D24, C1E24, C1F24, C1G24, C1D25, C1E25, C1F25, C1G25, C1D26, C1E26, C1F26, C1G26, C1D27, C1E27, C1F27, C1G27, C1D28, C1E28, C1F28, C1G28, C1D29, C1E29, C1F29, C1G29, C1D30, C1E30, C1F30, C1G30, C1D31, C1E31, C1F31, C1G31, C1D32, C1E32, C1F32, C1G32, C1D33, C1E33, C1F33, C1G33, C1D34, C1E34, C1F34, C1G34, C1D35, C1E35, C1F35, C1G35, C1D36, C1E36, C1F36, C1G36, C1D37, C1E37, C1F37, C1G37, C1D38, C1E38, C1F38, C1G38, C1D39, C1E39, C1F39, C1G39, C1D40, C1E40, C1F40, C1G40, C2G13, C2D14, C2E14, C2F14, C2G14, C2D15, C2E15, C2F15, C2G15, C2D16, C2E16, C2F16, C2G16, C2D17, C2E17, C2F17, C2G17, C2D18, C2E18, C2F18, C2G18, C2D19, C2E19, C2F19, C2G19, C2D20, C2E20, C2F20, C2G20, C2D21, C2E21, C2F21, C2G21, C2D22, C2E22, C2F22, C2G22, C2D23, C2E23, C2F23, C2G23, C2D24, C2E24, C2F24, C2G24, C2D25, C2E25, C2F25, C2G25, C2D26, C2E26, C2F26, C2G26, C2D27, C2E27, C2F27, C2G27, C2D28, C2E28, C2F28, C2G28, C2D29, C2E29, C2F29, C2G29, C2D30, C2E30, C2F30, C2G30, C2D31, C2E31, C2F31, C2G31, C2D32, C2E32, C2F32, C2G32, C2D33, C2E33, C2F33, C2G33, C2D34, C2E34, C2F34, C2G34, C2D35, C2E35, C2F35, C2G35, C2D36, C2E36, C2F36, C2G36, C2D37, C2E37, C2F37, C2G37, C2D38, C2E38, C2F38, C2G38, C2D39, C2E39, C2F39, C2G39, C2D40, C2E40, C2F40, C2G40, C3G13, C3D14, C3E14, C3F14, C3G14, C3D15, C3E15, C3F15, C3G15, C3D16, C3E16, C3F16, C3G16, C3D17, C3E17, C3F17, C3G17, C3D18, C3E18, C3F18, C3G18, C3D19, C3E19, C3F19, C3G19, C3D20, C3E20, C3F20, C3G20, C3D21, C3E21, C3F21, C3G21, C3D22, C3E22, C3F22, C3G22, C3D23, C3E23, C3F23, C3G23, C3D24, C3E24, C3F24, C3G24, C3D25, C3E25, C3F25, C3G25, C3D26, C3E26, C3F26, C3G26, C3D27, C3E27, C3F27, C3G27, C3D28, C3E28, C3F28, C3G28, C3D29, C3E29, C3F29, C3G29, C3D30, C3E30, C3F30, C3G30, C3D31, C3E31, C3F31, C3G31, C3D32, C3E32, C3F32, C3G32, C3D33, C3E33, C3F33, C3G33, C3D34, C3E34, C3F34, C3G34, C3D35, C3E35, C3F35, C3G35, C3D36, C3E36, C3F36, C3G36, C3D37, C3E37, C3F37, C3G37, C3D38, C3E38, C3F38, C3G38, C3D39, C3E39, C3F39, C3G39, C3D40, C3E40, C3F40, C3G40, C4G13, C4D14, C4E14, C4F14, C4G14, C4D15, C4E15, C4F15, C4G15, C4D16, C4E16, C4F16, C4G16, C4D17, C4E17, C4F17, C4G17, C4D18, C4E18, C4F18, C4G18, C4D19, C4E19, C4F19, C4G19, C4D20, C4E20, C4F20, C4G20, C4D21, C4E21, C4F21, C4G21, C4D22, C4E22, C4F22, C4G22, C4D23, C4E23, C4F23, C4G23, C4D24, C4E24, C4F24, C4G24, C4D25, C4E25, C4F25, C4G25, C4D26, C4E26, C4F26, C4G26, C4D27, C4E27, C4F27, C4G27, C4D28, C4E28, C4F28, C4G28, C4D29, C4E29, C4F29, C4G29, C4D30, C4E30, C4F30, C4G30, C4D31, C4E31, C4F31, C4G31, C4D32, C4E32, C4F32, C4G32, C4D33, C4E33, C4F33, C4G33, C4D34, C4E34, C4F34, C4G34, C4D35, C4E35, C4F35, C4G35, C4D36, C4E36, C4F36, C4G36, C4D37, C4E37, C4F37, C4G37, C4D38, C4E38, C4F38, C4G38, C4D39, C4E39, C4F39, C4G39, C4D40, C4E40, C4F40, C4G40, CSG13, CSD14, CSE14, CSF14, CSG14, CSD15, CSE15, CSF15, C5G15, C5D16, C5E16, C5F16, C5G16, C5D17, C5E17, C5F17, C5G17, C5D18, C5E18, C5F18, C5G18, C5D19, CSE19, CSF19, CSG19, CSD20, CSE20, CSF20, C5G20, CSD21, CSE21, CSF21, C5G21, CSD22, CSE22, CSF22, C5G22, CSD23, CSE23, CSF23, CSG23, CSD24, C5E24, CSF24, C5G24, C5D25, C5E25, CSF25, CSG25, CSD26, C5E26, CSF26, CSG26, C5D27, C5E27, CSF27, CSG27, CSD28, C5E28, CSF28, CSG28, C5D29, C5E29, CSF29, CSG29, CSD30, C5E30, CSF30, CSG30, CSD31, CSE31, CSF31, CSG31, CSD32, CSE32, CSF32, CSG32, CSD33, C5E33, CSF33, CSG33, CSD34, C5E34, CSF34, CSG34, CSD35, C5E35, CSF35, CSG35, CSD36, C5E36, CSF36, CSG36, CSD37, C5E37, CSF37, CSG37, CSD38, C5E38, CSF38, CSG38, CSD39, C5E39, CSF39, CSG39, CSD40, C5E40, C5F40, C5G40, C6G13, C6D14, C6E14, C6F14, C6G14, C6D15, C6E15, C6F15, C6G15, C6D16, C6E16, C6F16, C6G16, C6D17, C6E17, C6F17, C6G17, C6D18, C6E18, C6F18, C6G18, C6D19, C6E19, C6F19, C6G19, C6D20, C6E20, C6F20, C6G20, C6D21, C6E21, C6F21, C6G21, C6D22, C6E22, C6F22, C6G22, C6D23, C6E23, C6F23, C6G23, C6D24, C6E24, C6F24, C6G24, C6D25, C6E25, C6F25, C6G25, C6D26, C6E26, C6F26, C6G26, C6D27, C6E27, C6F27, C6G27, C6D28, C6E28, C6F28, C6G28, C6D29, C6E29, C6F29, C6G29, C6D30, C6E30, C6F30, C6G30, C6D31, C6E31, C6F31, C6G31, C6D32, C6E32, C6F32, C6G32, C6D33, C6E33, C6F33, C6G33, C6D34, C6E34, C6F34, C6G34, C6D35, C6E35, C6F35, C6G35, C6D36, C6E36, C6F36, C6G36, C6D37, C6E37, C6F37, C6G37, C6D38, C6E38, C6F38, C6G38, C6D39, C6E39, C6F39, C6G39, C6D40, C6E40, C6F40, C6G40, C7G13, C7D14, C7E14, C7F14, C7G14, C7D15, C7E15, C7F15, C7G15, C7D16, C7E16, C7F16, C7G16, C7D17, C7E17, C7F17, C7G17, C7D18, C7E18, C7F18, C7G18, C7D19, C7E19, C7F19, C7G19, C7D20, C7E20, C7F20, C7G20, C7D21, C7E21, C7F21, C7G21, C7D22, C7E22, C7F22, C7G22, C7D23, C7E23, C7F23, C7G23, C7D24, C7E24, C7F24, C7G24, C7D25, C7E25, C7F25, C7G25, C7D26, C7E26, C7F26, C7G26, C7D27, C7E27, C7F27, C7G27, C7D28, C7E28, C7F28, C7G28, C7D29, C7E29, C7F29, C7G29, C7D30, C7E30, C7F30, C7G30, C7D31, C7E31, C7F31, C7G31, C7D32, C7E32, C7F32, C7G32, C7D33, C7E33, C7F33, C7G33, C7D34, C7E34, C7F34, C7G34, C7D35, C7E35, C7F35, C7G35, C7D36, C7E36, C7F36, C7G36, C7D37, C7E37, C7F37, C7G37, C7D38, C7E38, C7F38, C7G38, C7D39, C7E39, C7F39, C7G39, C7D40, C7E40, C7F40, C7G40, C8G13, C8D14, C8E14, C8F14, C8G14, C8D15, C8E15, C8F15, C8G15, C8D16, C8E16, C8F16, C8G16, C8D17, C8E17, C8F17, C8G17, C8D18, C8E18, C8F18, C8G18, C8D19, C8E19, C8F19, C8G19, C8D20, C8E20, C8F20, C8G20, C8D21, C8E21, C8F21, C8G21, C8D22, C8E22, C8F22, C8G22, C8D23, C8E23, C8F23, C8G23, C8D24, C8E24, C8F24, C8G24, C8D25, C8E25, C8F25, C8G25, C8D26, C8E26, C8F26, C8G26, C8D27, C8E27, C8F27, C8G27, C8D28, C8E28, C8F28, C8G28, C8D29, C8E29, C8F29, C8G29, C8D30, C8E30, C8F30, C8G30, C8D31, C8E31, C8F31, C8G31, C8D32, C8E32, C8F32, C8G32, C8D33, C8E33, C8F33, C8G33, C8D34, C8E34, C8F34, C8G34, C8D35, C8E35, C8F35, C8G35, C8D36, C8E36, C8F36, C8G36, C8D37, C8E37, C8F37, C8G37, C8D38, C8E38, C8F38, C8G38, C8D39, C8E39, C8F39, C8G39, C8D40, C8E40, C8F40, C8G40, C9G13, C9D14, C9E14, C9F14, C9G14, C9D15, C9E15, C9F15, C9G15, C9D16, C9E16, C9F16, C9G16, C9D17, C9E17, C9F17, C9G17, C9D18, C9E18, C9F18, C9G18, C9D19, C9E19, C9F19, C9G19, C9D20, C9E20, C9F20, C9G20, C9D21, C9E21, C9F21, C9G21, C9D22, C9E22, C9F22, C9G22, C9D23, C9E23, C9F23, C9G23, C9D24, C9E24, C9F24, C9G24, C9D25, C9E25, C9F25, C9G25, C9D26, C9E26, C9F26, C9G26, C9D27, C9E27, C9F27, C9G27, C9D28, C9E28, C9F28, C9G28, C9D29, C9E29, C9F29, C9G29, C9D30, C9E30, C9F30, C9G30, C9D31, C9E31, C9F31, C9G31, C9D32, C9E32, C9F32, C9G32, C9D33, C9E33, C9F33, C9G33, C9D34, C9E34, C9F34, C9G34, C9D35, C9E35, C9F35, C9G35, C9D36, C9E36, C9F36, C9G36, C9D37, C9E37, C9F37, C9G37, C9D38, C9E38, C9F38, C9G38, C9D39, C9E39, C9F39, C9G39, C9D40, C9E40, C9F40, C9G40, C10G13, C10D14, C10E14, C10F14, C10G14, C10D15, C10E15, C10F15, C10G15, C10D16, C10E16, C10F16, C10G16, C10D17, C10E17, C10F17, C10G17, C10D18, C10E18, C10F18, C10G18, C10D19, C10E19, C10F19, C10G19, C10D20, C10E20, C10F20, C10G20, C10D21, C10E21, C10F21, C10G21, C10D22, C10E22, C10F22, C10G22, C10D23, C10E23, C10F23, C10G23, C10D24, C10E24, C10F24, C10G24, C10D25, C10E25, C10F25, C10G25, C10D26, C10E26, C10F26, C10G26, C10D27, C10E27, C10F27, C10G27, C10D28, C10E28, C10F28, C10G28, C10D29, C10E29, C10F29, C10G29, C10D30, C10E30, C10F30, C10G30, C10D31, C10E31, C10F31, C10G31, C10D32, C10E32, C10F32, C10G32, C10D33, C10E33, C10F33, C10G33, C10D34, C10E34, C10F34, C10G34, C10D35, C10E35, C10F35, C10G35, C10D36, C10E36, C10F36, C10G36, C10D37, C10E37, C10F37, C10G37, C10D38, C10E38, C10F38, C10G38, C10D39, C10E39, C10F39, C10G39, C10D40, C10E40, C10F40, C10G40, C11G13, C11D14, C11E14, C11F14, C11G14, C11D15, C11E15, C11F15, C11G15, C11D16, C11E16, C11F16, C11G16, C11D17, C11E17, C11F17, C11G17, C11D18, C11E18, C11F18, C11G18, C11D19, C11E19, C11F19, C11G19, C11D20, C11E20, C11F20, C11G20, C11D21, C11E21, C11F21, C11G21, C11D22, C11E22, C11F22, C11G22, C11D23, C11E23, C11F23, C11G23, C11D24, C11E24, C11F24, C11G24, C11D25, C11E25, C11F25, C11G25, C11D26, C11E26, C11F26, C11G26, C11D27, C11E27, C11F27, C11G27, C11D28, C11E28, C11F28, C11G28, C11D29, C11E29, C11F29, C11G29, C11D30, C11E30, C11F30, C11G30, C11D31, C11E31, C11F31, C11G31, C11D32, C11E32, C11F32, C11G32, C11D33, C11E33, C11F33, C11G33, C11D34, C11E34, C11F34, C11G34, C11D35, C11E35, C11F35, C11G35, C11D36, C11E36, C11F36, C11G36, C11D37, C11E37, C11F37, C11G37, C11D38, C11E38, C11F38, C11G38, C11D39, C11E39, C11F39, C11G39, C11D40, C11E40, C11F40, C11G40, C12G13, C12D14, C12E14, C12F14, C12G14, C12D15, C12E15, C12F15, C12G15, C12D16, C12E16, C12F16, C12G16, C12D17, C12E17, C12F17, C12G17, C12D18, C12E18, C12F18, C12G18, C12D19, C12E19, C12F19, C12G19, C12D20, C12E20, C12F20, C12G20, C12D21, C12E21, C12F21, C12G21, C12D22, C12E22, C12F22, C12G22, C12D23, C12E23, C12F23, C12G23, C12D24, C12E24, C12F24, C12G24, C12D25, C12E25, C12F25, C12G25, C12D26, C12E26, C12F26, C12G26, C12D27, C12E27, C12F27, C12G27, C12D28, C12E28, C12F28, C12G28, C12D29, C12E29, C12F29, C12G29, C12D30, C12E30, C12F30, C12G30, C12D31, C12E31, C12F31, C12G31, C12D32, C12E32, C12F32, C12G32, C12D33, C12E33, C12F33, C12G33, C12D34, C12E34, C12F34, C12G34, C12D35, C12E35, C12F35, C12G35, C12D36, C12E36, C12F36, C12G36, C12D37, C12E37, C12F37, C12G37, C12D38, C12E38, C12F38, C12G38, C12D39, C12E39, C12F39, C12G39, C12D40, C12E40, C12F40, C12G40, A13D1, A13E1, A13F1, A13G1, A13D2, A13E2, A13F2, A13G2, A13D3, A13E3, A13F3, A13G3, A13D4, A13E4, A13F4, A13G4, A13D5, A13E5, A13F5, A13G5, A13D6, A13E6, A13F6, A13G6, A13D7, A13E7, A13F7, A13G7, A13D8, A13E8, A13F8, A13G8, A13D9, A13E9, A13F9, A13G9, A13D10, A13E10, A13F10, A13G10, A13D11, A13E11, A13F11, A13G11, A13D12, A13E12, A13F12, A13G12, A13D13, A13E13, A13F13, A13G13, A13D14, A13E14, A13F14, A13G14, A13D15, A13E15, A13F15, A13G15, A13D16, A13E16, A13F16, A13G16, A13D17, A13E17, A13F17, A13G17, A13D18, A13E18, A13F18, A13G18, A13D19, A13E19, A13F19, A13G19, A13D20, A13E20, A13F20, A13G20, A13D21, A13E21, A13F21, A13G21, A13D22, A13E22, A13F22, A13G22, A13D23, A13E23, A13F23, A13G23, A13D24, A13E24, A13F24, A13G24, A13D25, A13E25, A13F25, A13G25, A13D26, A13E26, A13F26, A13G26, A13D27, A13E27, A13F27, A13G27, A13D28, A13E28, A13F28, A13G28, A13D29, A13E29, A13F29, A13G29, A13D30, A13E30, A13F30, A13G30, A13D31, A13E31, A13F31, A13G31, A13D32, A13E32, A13F32, A13G32, A13D33, A13E33, A13F33, A13G33, A13D34, A13E34, A13F34, A13G34, A13D35, A13E35, A13F35, A13G35, A13D36, A13E36, A13F36, A13G36, A13D37, A13E37, A13F37, A13G37, A13D38, A13E38, A13F38, A13G38, A13D39, A13E39, A13F39, A13G39, A13D40, A13E40, A13F40, A13G40, B13D1, B13E1, B13F1, B13G1, B13D2, B13E2, B13F2, B13G2, B13D3, B13E3, B13F3, B13G3, B13D4, B13E4, B13F4, B13G4, B13D5, B13E5, B13F5, B13G5, B13D6, B13E6, B13F6, B13G6, B13D7, B13E7, B13F7, B13G7, B13D8, B13E8, B13F8, B13G8, B13D9, B13E9, B13F9, B13G9, B13D10, B13E10, B13F10, B13G10, B13D11, B13E11, B13F11, B13G11, B13D12, B13E12, B13F12, B13G12, B13D13, B13E13, B13F13, B13G13, B13D14, B13E14, B13F14, B13G14, B13D15, B13E15, B13F15, B13G15, B13D16, B13E16, B13F16, B13G16, B13D17, B13E17, B13F17, B13G17, B13D18, B13E18, B13F18, B13G18, B13D19, B13E19, B13F19, B13G19, B13D20, B13E20, B13F20, B13G20, B13D21, B13E21, B13F21, B13G21, B13D22, B13E22, B13F22, B13G22, B13D23, B13E23, B13F23, B13G23, B13D24, B13E24, B13F24, B13G24, B13D25, B13E25, B13F25, B13G25, B13D26, B13E26, B13F26, B13G26, B13D27, B13E27, B13F27, B13G27, B13D28, B13E28, B13F28, B13G28, B13D29, B13E29, B13F29, B13G29, B13D30, B13E30, B13F30, B13G30, B13D31, B13E31, B13F31, B13G31, B13D32, B13E32, B13F32, B13G32, B13D33, B13E33, B13F33, B13G33, B13D34, B13E34, B13F34, B13G34, B13D35, B13E35, B13F35, B13G35, B13D36, B13E36, B13F36, B13G36, B13D37, B13E37, B13F37, B13G37, B13D38, B13E38, B13F38, B13G38, B13D39, B13E39, B13F39, B13G39, B13D40, B13E40, B13F40, B13G40, C13D1, C13E1, C13F1, C13G1, C13D2, C13E2, C13F2, C13G2, C13D3, C13E3, C13F3, C13G3, C13D4, C13E4, C13F4, C13G4, C13D5, C13E5, C13F5, C13G5, C13D6, C13E6, C13F6, C13G6, C13D7, C13E7, C13F7, C13G7, C13D8, C13E8, C13F8, C13G8, C13D9, C13E9, C13F9, C13G9, C13D10, C13E10, C13F10, C13G10, C13D11, C13E11, C13F11, C13G11, C13D12, C13E12, C13F12, C13G12, C13D13, C13E13, C13F13, C13G13, C13D14, C13E14, C13F14, C13G14, C13D15, C13E15, C13F15, C13G15, C13D16, C13E16, C13F16, C13G16, C13D17, C13E17, C13F17, C13G17, C13D18, C13E18, C13F18, C13G18, C13D19, C13E19, C13F19, C13G19, C13D20, C13E20, C13F20, C13G20, C13D21, C13E21, C13F21, C13G21, C13D22, C13E22, C13F22, C13G22, C13D23, C13E23, C13F23, C13G23, C13D24, C13E24, C13F24, C13G24, C13D25, C13E25, C13F25, C13G25, C13D26, C13E26, C13F26, C13G26, C13D27, C13E27, C13F27, C13G27, C13D28, C13E28, C13F28, C13G28, C13D29, C13E29, C13F29, C13G29, C13D30, C13E30, C13F30, C13G30, C13D31, C13E31, C13F31, C13G31, C13D32, C13E32, C13F32, C13G32, C13D33, C13E33, C13F33, C13G33, C13D34, C13E34, C13F34, C13G34, C13D35, C13E35, C13F35, C13G35, C13D36, C13E36, C13F36, C13G36, C13D37, C13E37, C13F37, C13G37, C13D38, C13E38, C13F38, C13G38, C13D39, C13E39, C13F39, C13G39, C13D40, C13E40, C13F40, or C13G40.

The embodiments of R¹⁹ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. In some embodiments of the subject complex or compound (e.g., precursor compound), or a pharmaceutically acceptable salt or solvate thereof, R¹⁹ is:

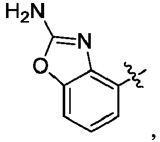

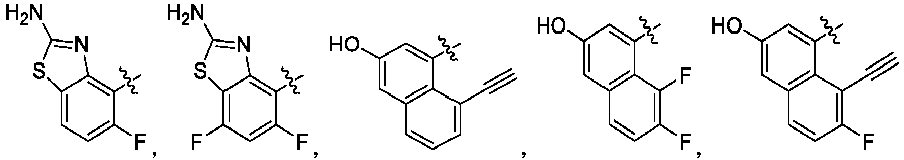

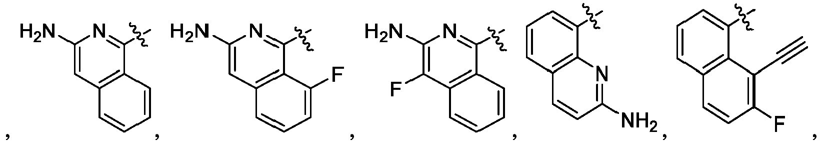

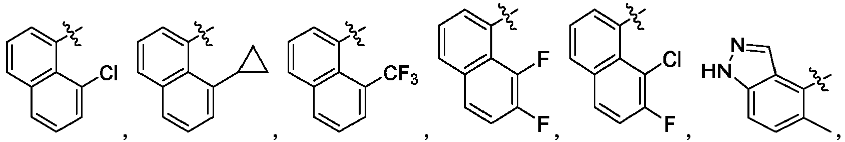

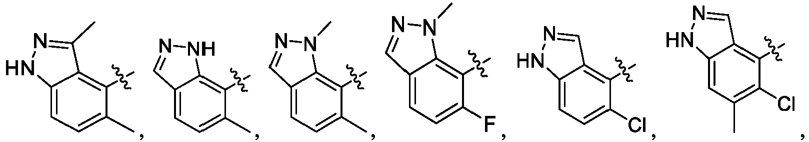

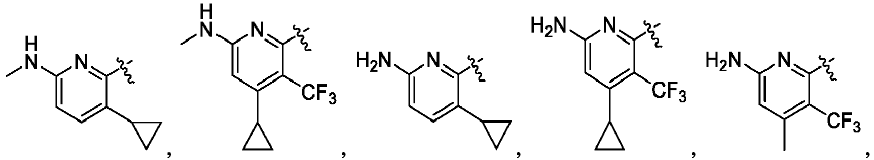

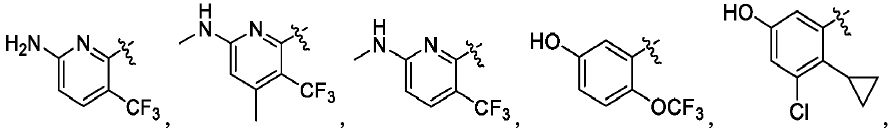

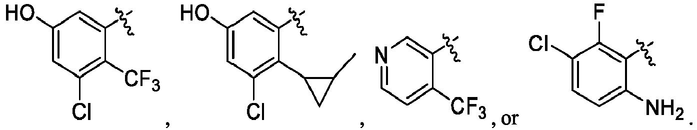

In embodiments R¹⁹ is selected from -CH=CH₂,

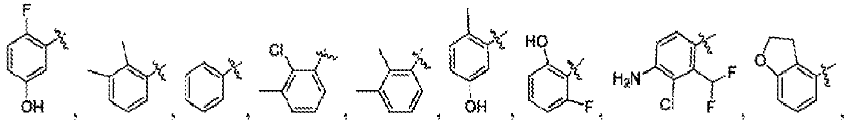

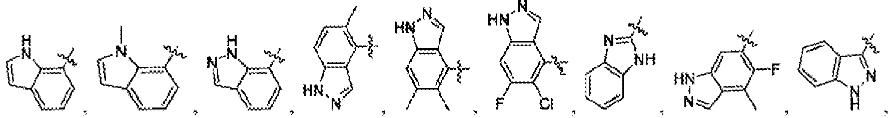

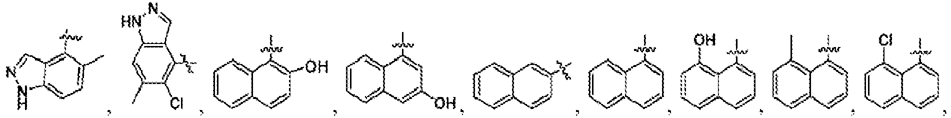

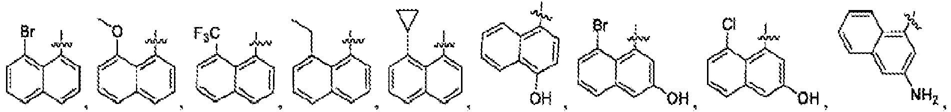

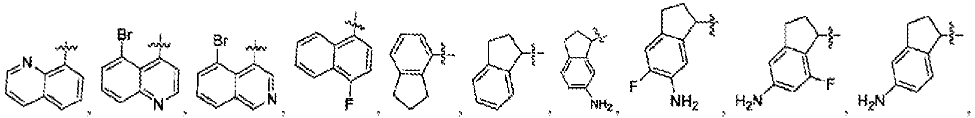

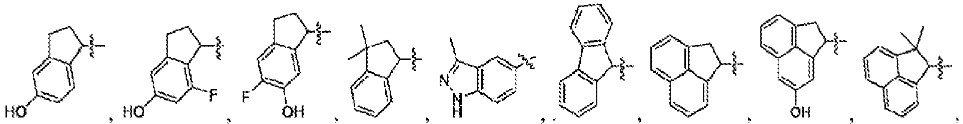

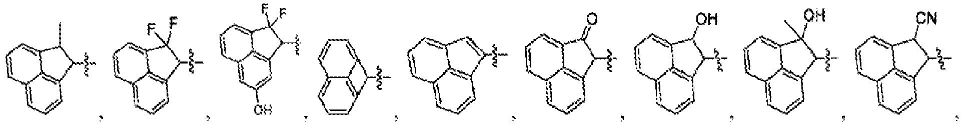

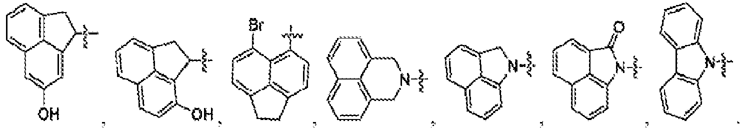

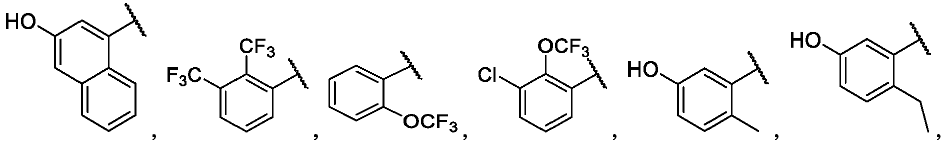

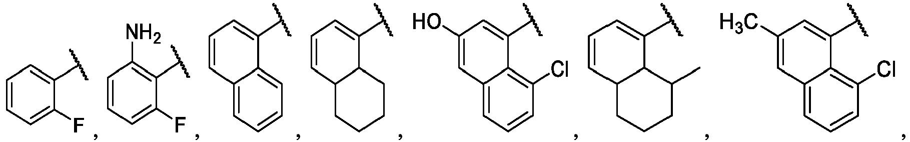

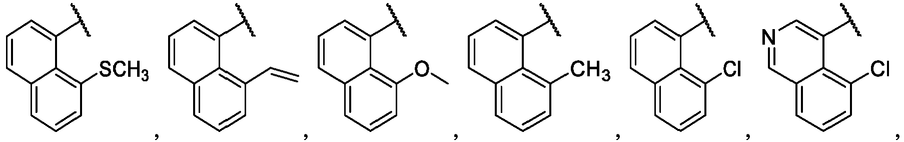

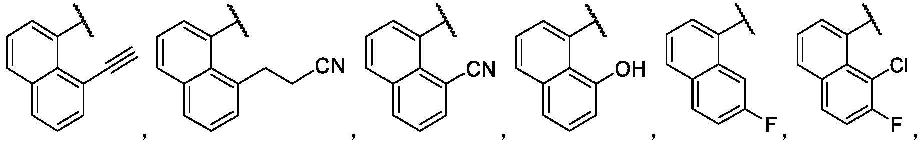

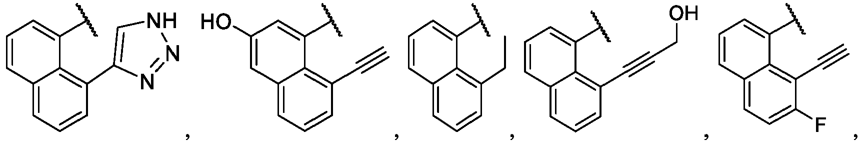

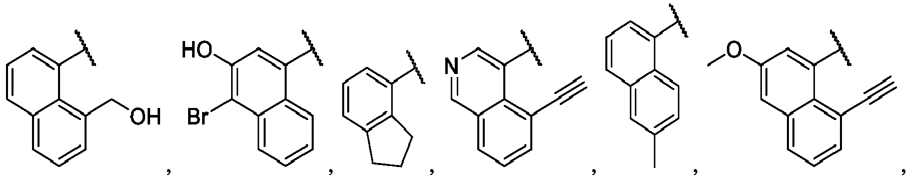

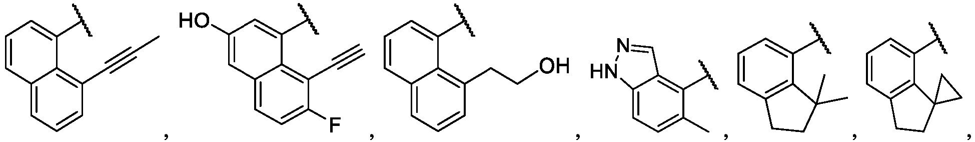

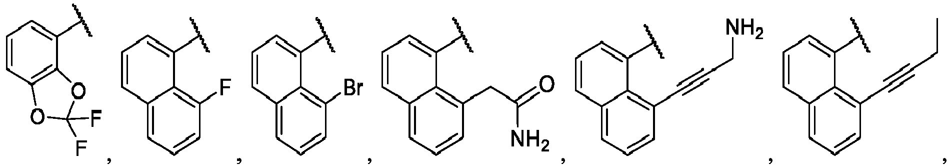

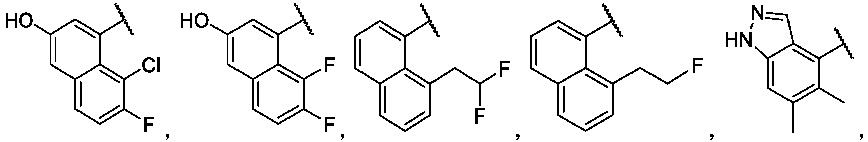

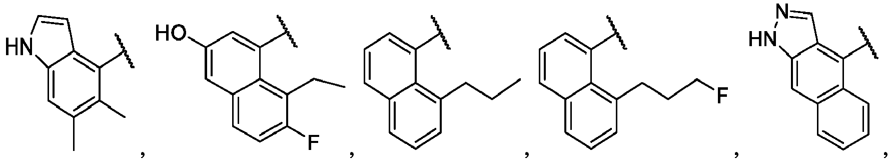

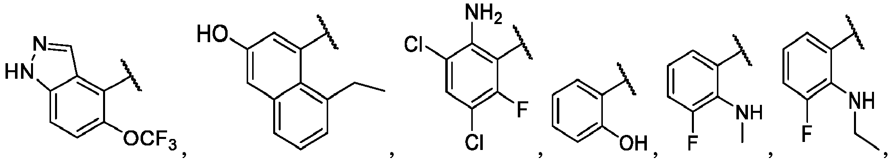

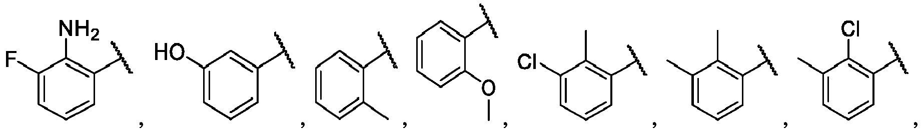

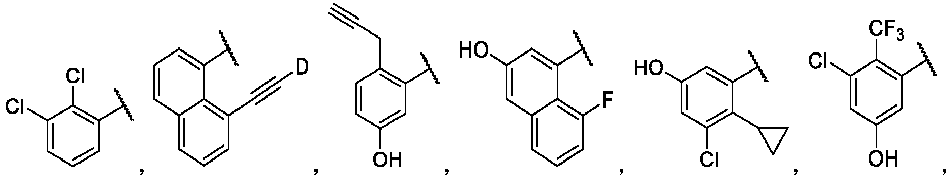

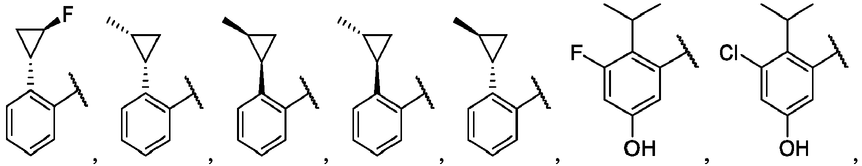

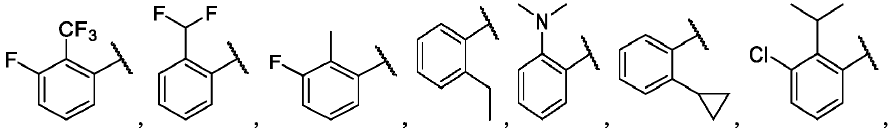

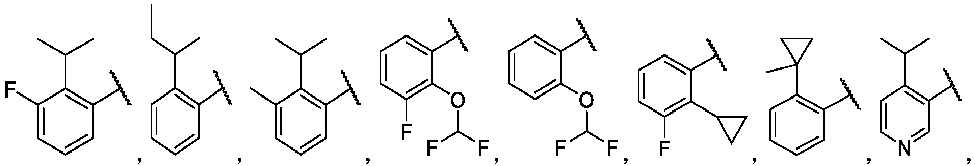

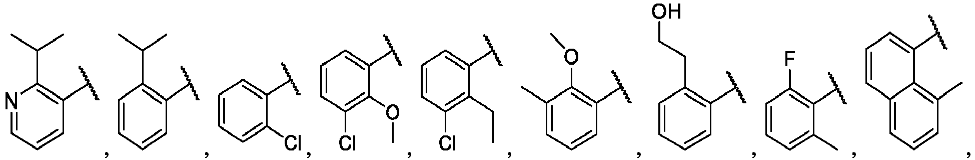

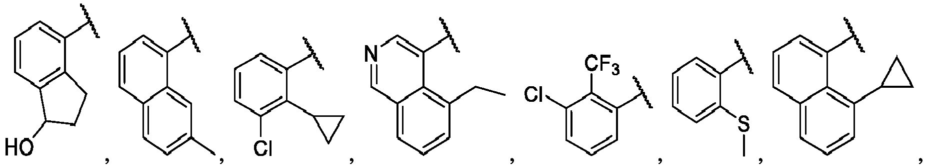

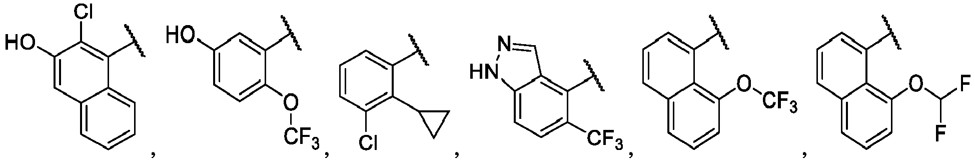

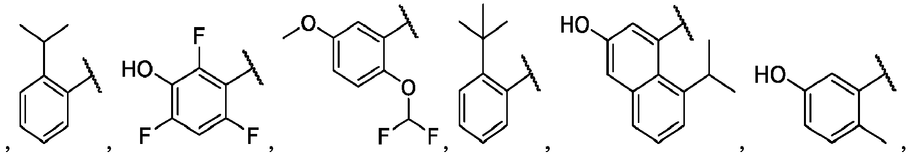

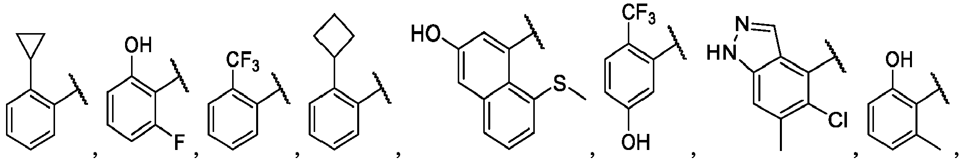

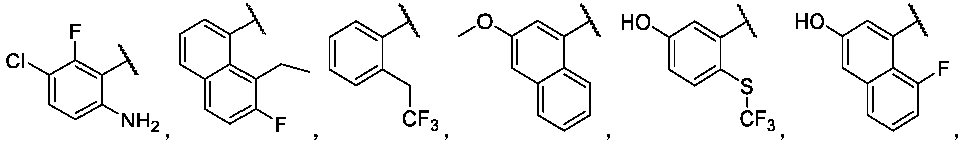

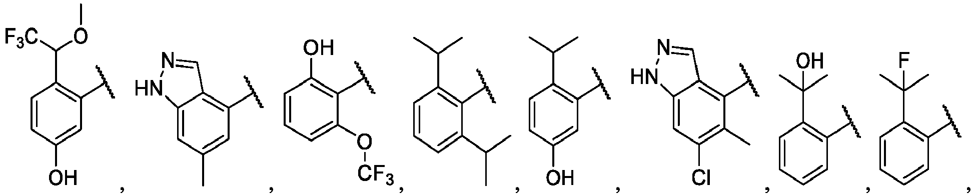

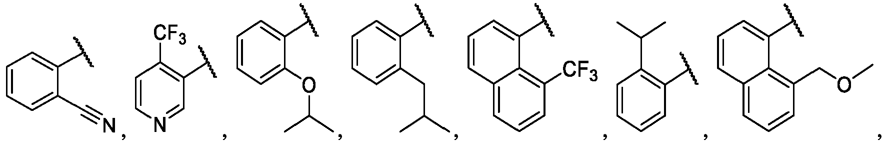

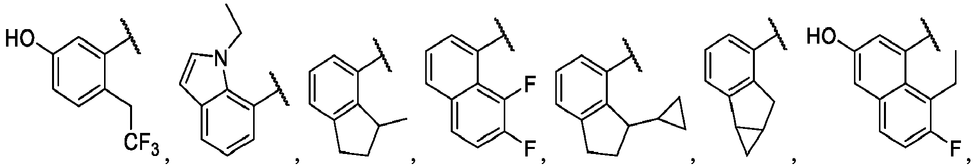

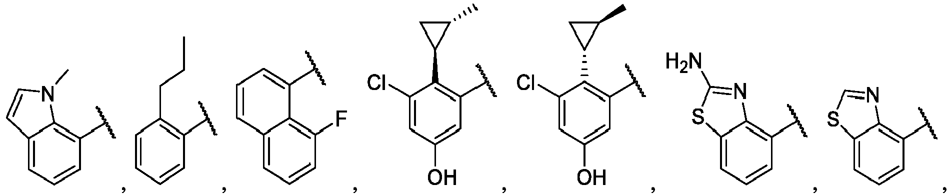

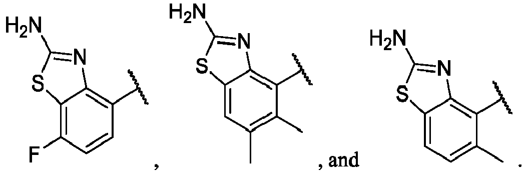

In embodiments, R¹⁹ is 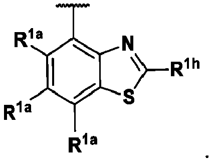 In embodiments, R¹⁹ is 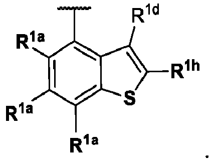 In embodiments, R¹⁹ is 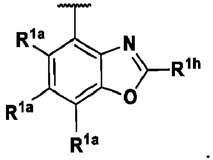 In embodiments, R¹⁹ is 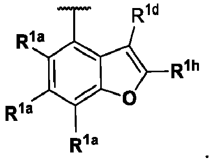 In embodiments, R¹⁹ is 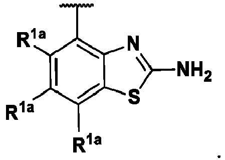 In embodiments, R¹⁹ is 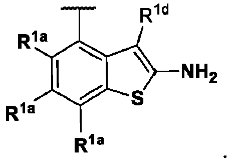 In embodiments, R¹⁹ is 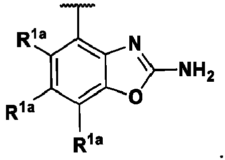 In embodiments, R¹⁹ is 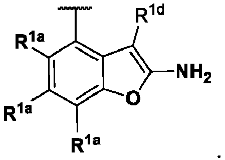 In embodiments, R¹⁹ is 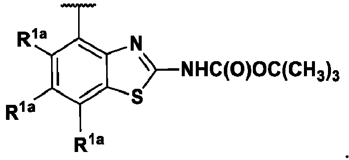 In embodiments, R¹⁹ is 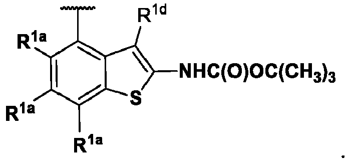 In embodiments, R¹⁹ is 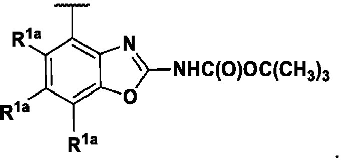 In embodiments, R¹⁹ is 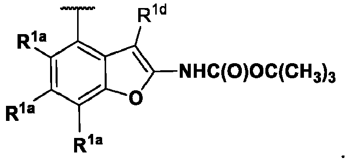 In embodiments, R¹⁹ is 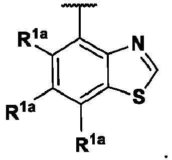 In embodiments, R¹⁹ is 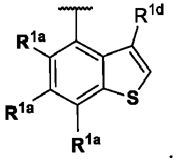 In embodiments, R¹⁹ is 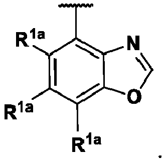 In embodiments, R¹⁹ is 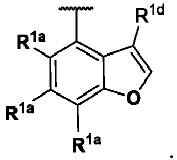 In embodiments, R¹⁹ is 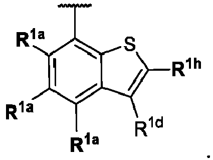 In embodiments, R¹⁹ is 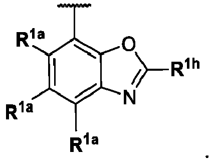 In embodiments, R¹⁹ is 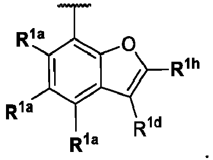 In embodiments, R¹⁹ is 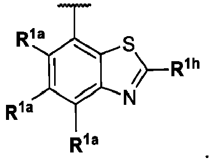 In embodiments, R¹⁹ is 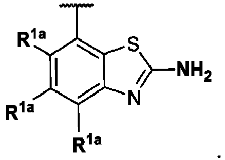 In embodiments, R¹⁹ is 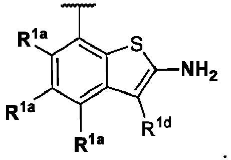 In embodiments, R¹⁹ is 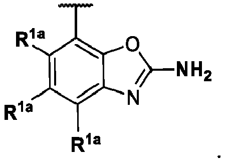 In embodiments, R¹⁹ is 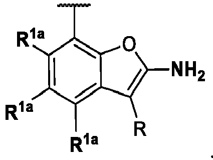 In embodiments, R¹⁹ is 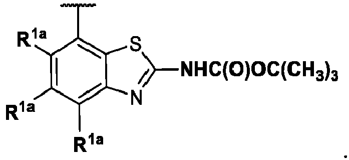 In embodiments, R¹⁹ is 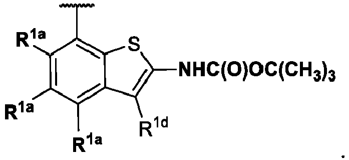 In embodiments, R¹⁹ is 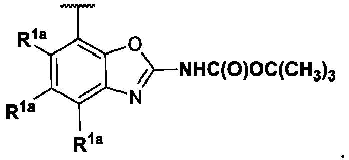 In embodiments, R¹⁹ is 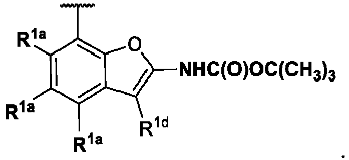 In embodiments, R¹⁹ is 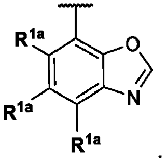 In embodiments, R¹⁹ is 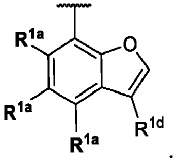 In embodiments, R¹⁹ is 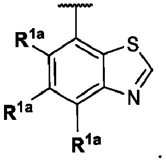 In embodiments, R¹⁹ is

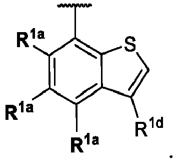

In some embodiments, R¹⁹ is 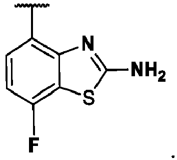 In some embodiments, R¹⁹ is 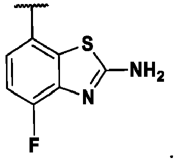 In some embodiments, R¹⁹ is 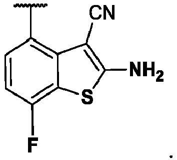 In some embodiments, R¹⁹ is 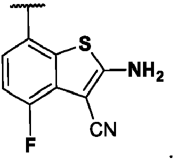 In some embodiments, R¹⁹ is 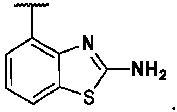 In some embodiments, R¹⁹ is 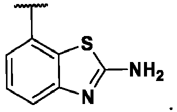 In some embodiments, R¹⁹ is 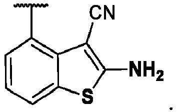 In some embodiments, R¹⁹ is 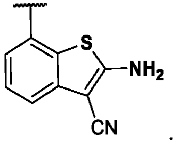 In some embodiments, R¹⁹ is 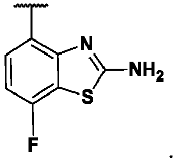 In some embodiments, R¹⁹ is 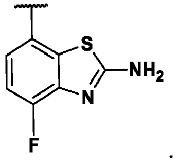 In some embodiments, R¹⁹ is 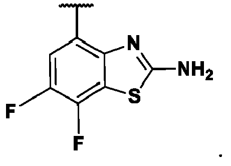 In some embodiments, R¹⁹ is 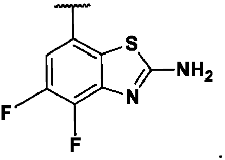 In some embodiments, R¹⁹ is 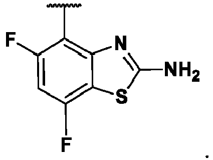 In some embodiments, R¹⁹ is 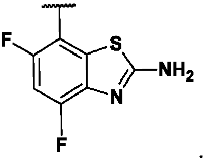 In some embodiments, R¹⁹ is 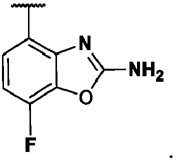 In some embodiments, R¹⁹ is 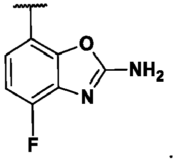 In some embodiments, R¹⁹ is 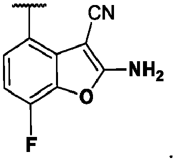 In some embodiments, R¹⁹ is 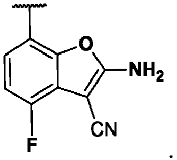 In some embodiments, R¹⁹ is 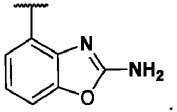 In some embodiments, R¹⁹ is 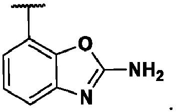 In some embodiments, R¹⁹ is 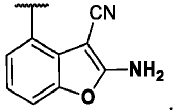 In some embodiments, R¹⁹ is 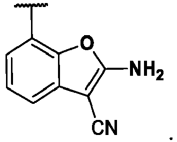 In some embodiments, R¹⁹ is 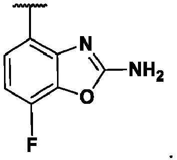 In some embodiments, R¹⁹ is 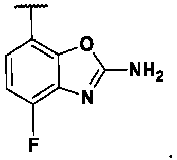 In some embodiments, R¹⁹ is 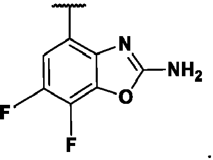 In some embodiments, R¹⁹ is 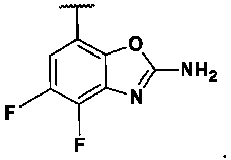 In some embodiments, R¹⁹ is 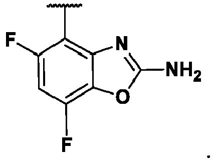 In some embodiments, R¹⁹ is

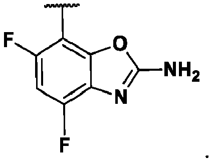

The embodiments of R² as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. In embodiments of the subject complex or compound (e.g., precursor compound), or a pharmaceutically acceptable salt or solvate thereof, R² is -OR¹². In embodiments of the subject complex or compound (e.g., precursor compound), or a pharmaceutically acceptable salt or solvate thereof, R² is - OR^{12c}. In some embodiments (e.g., of monocyclic formulae of precursor compounds disclosed herein), R² is halogen, -CN, C₂₋₆alkyl, C₂₋₆alkenyl, C₂₋₆alkynyl, C₃₋₆cycloalkyl, -OR^{12c}, -SR¹², -N(R^{12c})(R^{12c}), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₂₋₆alkyl)-R^{12b}, -(C₂₋₆alkenyl)-R^{12b}, -(C₂₋₆alkynyl)-R^{12b}, or -(C_{3- 10}cycloalkyl)-R^{12b}, wherein said C₂₋₆alkyl, C₂₋₆alkenyl, C₂₋₆alkynyl, and C₃₋₁₀cycloalkyl, are optionally substituted with one, two, or three R^{20d}.

In embodiments of the subject complex or compound (e.g., precursor compound), or a pharmaceutically acceptable salt or solvate thereof, R² is selected from

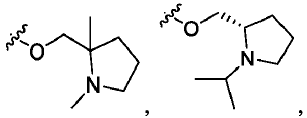

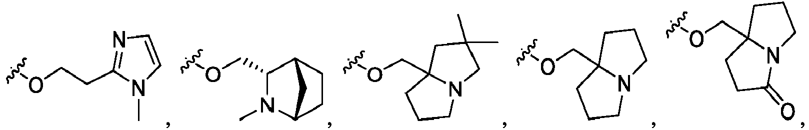

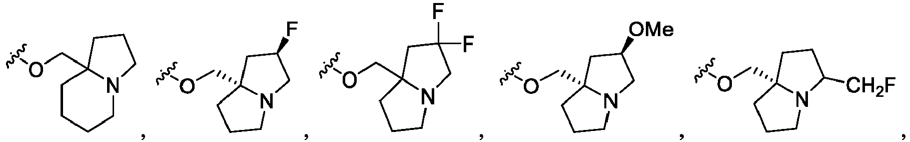

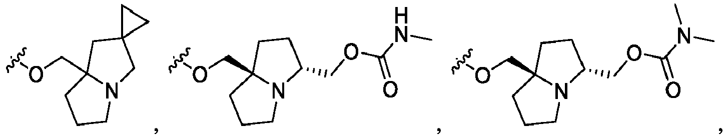

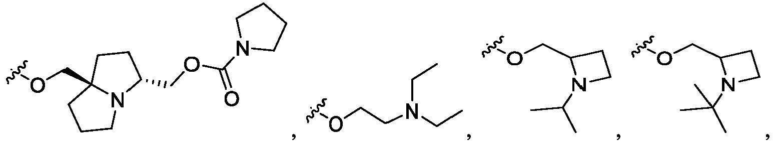

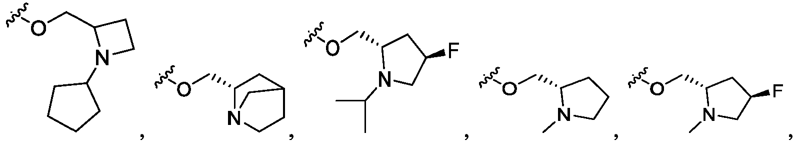

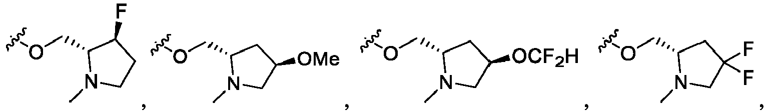

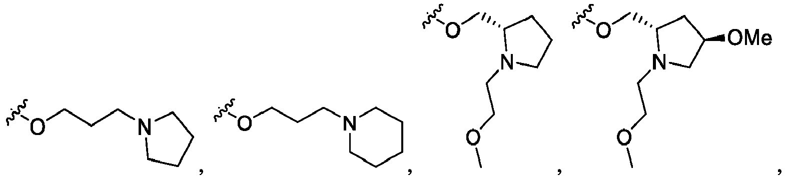

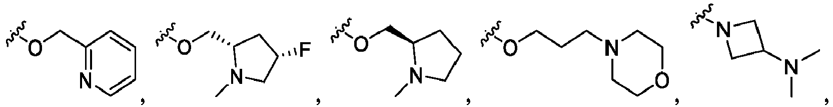

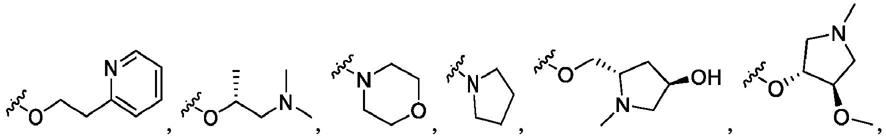

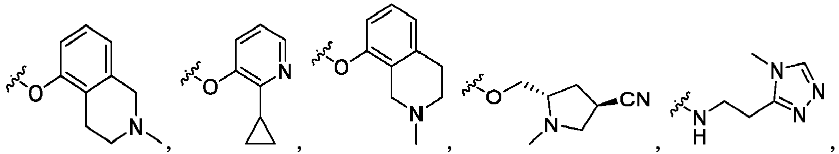

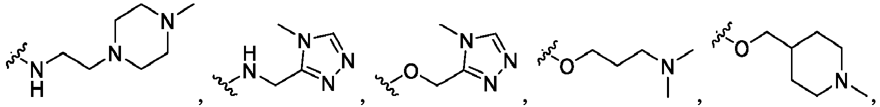

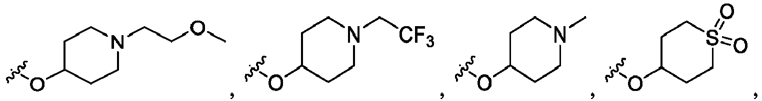

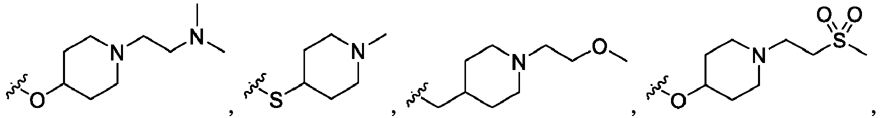

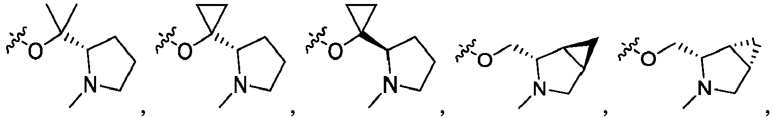

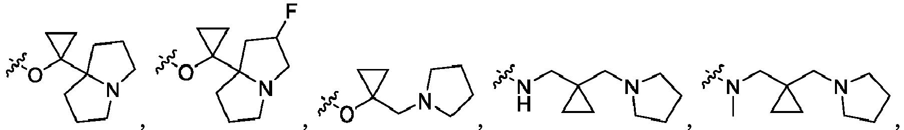

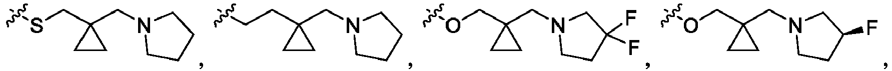

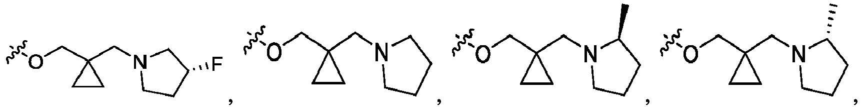

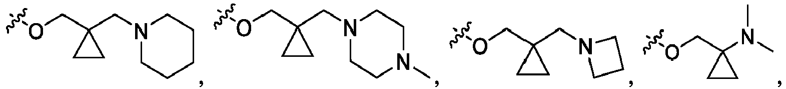

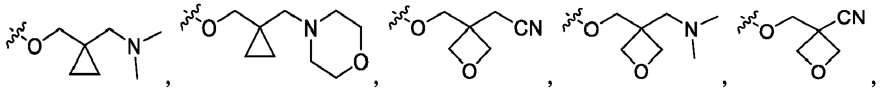

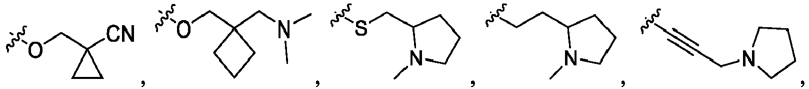

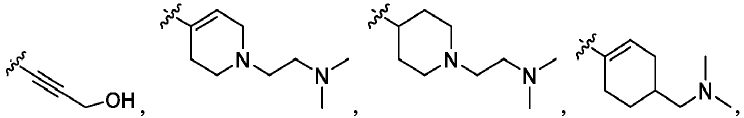

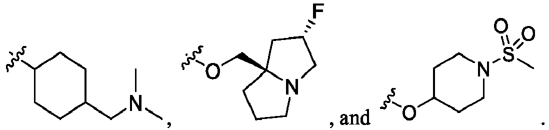

In embodiments, R² is -O-CH₂-C₂₋₉heterocycloalkyl optionally substituted with one, two, or three R^{20d} wherein R^{20d} is independently halogen (e.g., F). In embodiments, R² is -O-CH₂-C₂₋₉heterocycloalkyl optionally substituted with one, two, or three R^{20d} wherein R^{20d} is independently C₁₋₆alkyl (e.g., methyl). In embodiments, R² is 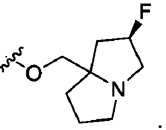 In embodiments, R² is 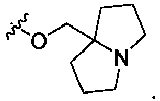 In embodiments, R² is 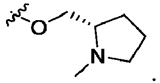 In embodiments, R² is 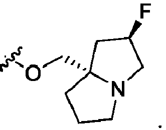 In embodiments, R² is

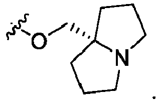

In some embodiments of the subject complex or compound (e.g., precursor compound), L⁷ is a bond. In embodiments of the subject complex or compound (e.g., precursor compound), L⁷ is -N(R¹⁴)-. In embodiments of the subject complex or compound (e.g., precursor compound), L⁷ is -NH-. In additional embodiments of the subject complex or compound (e.g., precursor compound), L⁷ is methylene. In embodiments of the subject complex or compound (e.g., precursor compound), L⁷ is -C(O)-. In embodiments of the subject complex or compound (e.g., precursor compound), L⁷ is -S-. In embodiments of the subject complex or compound (e.g., precursor compound), L⁷ is -S(O)₂-. In embodiments of the subject complex or compound (e.g., precursor compound), L⁷ is -S(O)-. In embodiments of the subject complex or compound (e.g., precursor compound), L⁷ is -N(R^{14c})-C₁₋₃alkyl- wherein the C₁₋₃alkyl is optionally substituted with one, two or three R^{20a}. In embodiments of the subject complex or compound (e.g., precursor compound), L⁷ is C₁₋₄alkyl, wherein the C₁₋₄alkyl is optionally substituted with one, two or three R^{20a}.

In some embodiments, R⁷ is a 3-12 membered nitrogen containing heterocycloalkyl substituted with one R⁶ directly bonded to a ring N and optionally substituted with one or more R¹ and/or one or more R⁴. In some embodiments, R⁷ is a 7-12 membered nitrogen containing fused aryl substituted with one R⁶ directly bonded to a ring N and optionally substituted with one or more R¹ and/or one or more R⁴. In some embodiments, R⁷ is a 5-12 membered nitrogen containing heteroaryl substituted with one R⁶ directly bonded to a ring N and optionally substituted with one or more R¹ and/or one or more R⁴. In some embodiments, R⁷ is a -(4-6 membered nitrogen containing saturated heterocycloalkyl)-(4-6 membered nitrogen containing saturated heterocycloalkyl)- substituted with one R⁶ directly bonded to a ring N and optionally substituted with one or more R¹ and/or one or more R⁴.

In some embodiments, R⁷ is a 3-12 membered nitrogen containing heterocycloalkyl substituted with one R⁶ directly bonded to a ring N. In some embodiments, R⁷ is a 7-12 membered nitrogen containing fused aryl substituted with one R⁶ directly bonded to a ring N. In some embodiments, R⁷ is a 5-12 membered nitrogen containing heteroaryl substituted with one R⁶ directly bonded to a ring N. In some embodiments, R⁷ is a - (4-6 membered nitrogen containing saturated heterocycloalkyl)-(4-6 membered nitrogen containing saturated heterocycloalkyl)- substituted with one R⁶ directly bonded to a ring N

. In some embodiments, R⁷ is a 6-12 membered nitrogen containing heterocycloalkyl substituted with one R⁶ directly bonded to a ring N and optionally substituted with one or more R¹ and/or one or more R⁴. In some embodiments, R⁷ is a 5-12 membered nitrogen containing heteroaryl, substituted with one R⁶ directly bonded to a ring N and optionally substituted with one or more R¹ and/or one or more R⁴. In some embodiments, R⁷ is a 6-12 membered nitrogen containing heterocycloalkyl substituted with one R⁶ directly bonded to a ring N. In some embodiments, R⁷ is a 5-12 membered nitrogen containing heteroaryl, substituted with one R⁶ directly bonded to a ring N. In some embodiments, R⁷ is a 6-12 membered nitrogen containing heteroaryl, substituted with one R⁶ directly bonded to a ring N and optionally substituted with one or more R¹ and/or one or more R⁴. In some embodiments, R⁷ is a 6-12 membered nitrogen containing heteroaryl, substituted with one R⁶ directly bonded to a ring N.

In some embodiments, R⁷ is a 7-12 membered nitrogen containing spirocyclic heterocycloalkyl substituted with one R⁶ directly bonded to a ring N and optionally substituted with one or more R¹ and/or one or more R⁴. In some embodiments, R⁷ is a 7-12 membered nitrogen containing spirocyclic heterocycloalkyl substituted with one R⁶ directly bonded to a ring N.

In some embodiments, R⁷ is a 7-12 membered nitrogen containing fused heterocycloalkyl substituted with one R⁶ directly bonded to a ring N and optionally substituted with one or more R¹ and/or one or more R⁴. In some embodiments, R⁷ is a 7-12 membered nitrogen containing fused aryl substituted with one R⁶ directly bonded to a ring N and optionally substituted with one or more R¹ and/or one or more R⁴. In some embodiments, R⁷ is a 7-12 membered nitrogen containing fused heteroaryl heterocycloalkyl substituted with one R⁶ directly bonded to a ring N and optionally substituted with one or more R¹ and/or one or more R⁴. In some embodiments, R⁷ is a 7-12 membered nitrogen containing fused heterocycloalkyl substituted with one R⁶ directly bonded to a ring N. In some embodiments, R⁷ is a 7-12 membered nitrogen containing fused aryl substituted with one R⁶ directly bonded to a ring N. In some embodiments, R⁷ is a 7-12 membered nitrogen containing fused heteroaryl heterocycloalkyl substituted with one R⁶ directly bonded to a ring N.

In some embodiments, R⁷ is a -(4-6 membered nitrogen containing saturated heterocycloalkyl)-(4-6 membered nitrogen containing saturated heterocycloalkyl)- substituted with one R⁶ directly bonded to a ring N and optionally substituted with one or more R¹ and/or one or more R⁴ and further wherein the two - (4-6 membered nitrogen containing saturated heterocycloalkyl are joined by a direct bond between a ring C and ring N. In some embodiments, R⁷ is a -(4-6 membered nitrogen containing saturated heterocycloalkyl)-(4-6 membered nitrogen containing saturated heterocycloalkyl)- substituted with one R⁶ directly bonded to a ring N and further wherein the two -(4-6 membered nitrogen containing saturated heterocycloalkyl are joined by a direct bond between a ring C and ring N.

In some embodiments, R⁵ is 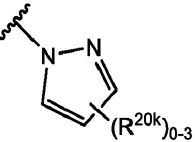 and R^{20k} are as described herein. In some embodiments, R⁵ is 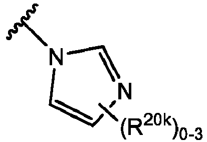 and R^{20k} is as described herein. In some embodiments, R⁵ is 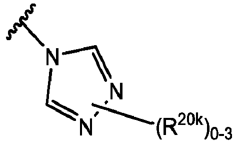 and R^{20k} is as described herein. In some embodiments, R⁵ is 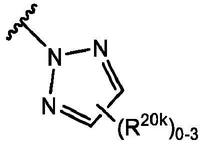 and R^{20k} is as described herein. In some embodiments, R⁵ is 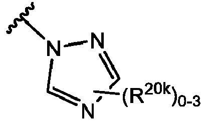 and R^{20k} is as described herein. In some embodiments, R⁵ is 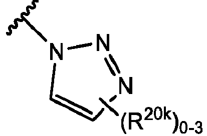 and R^{20k} is as described herein. In some embodiments, R⁵ is 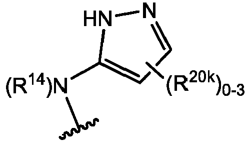 and R¹⁴ and R^{20k} are as described herein. In some embodiments, R⁵ is 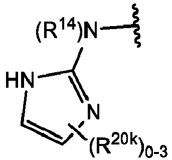 and R¹⁴ and R^{20k} are as described herein. In some embodiments, R⁵ is 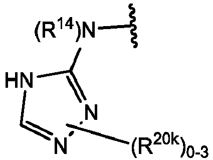 and R¹⁴ and R^{20k} are as described herein. In some embodiments, R⁵ is 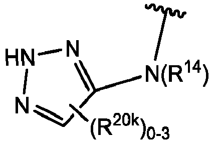 and R¹⁴ and R^{20k} are as described herein. In some embodiments, R⁵ is 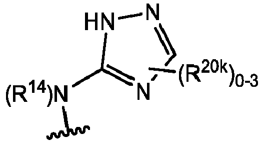 and R¹⁴ and R^{20k} are as described herein. In some embodiments, R⁵ is 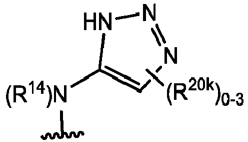 and R¹⁴ and R^{20k} are as described herein. In some embodiments, R⁵ is 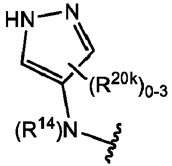 and R¹⁴ and R^{20k} are as described herein. In some embodiments, R⁵ is 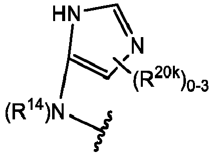 and R¹⁴ and R^{20k} are as described herein. In some embodiments, R⁵ is 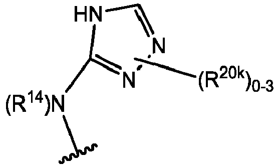 and R¹⁴ and R^{20k} are as described herein. In some embodiments, R⁵ is 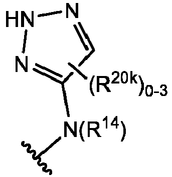 and R¹⁴ and R^{20k} are as described herein. In some embodiments, R⁵ is 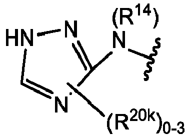 and R¹⁴ and R^{20k} are as described herein. In some embodiments, R⁵ is 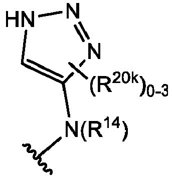 and R¹⁴ and R^{20k} are as described herein. In some embodiments, R⁵ is 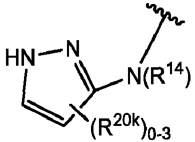 and R¹⁴ and R^{20k} are as described herein. In some embodiments, R⁵ is 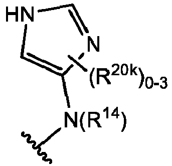 and R¹⁴ and R^{20k} are as described herein. In some embodiments, R⁵ is 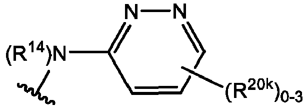 and R¹⁴ and R^{20k} are as described herein. In some embodiments, R⁵ is 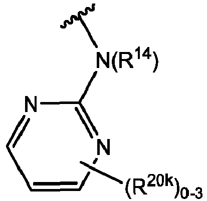 and R¹⁴ and R^{20k} are as described herein. In some embodiments, R⁵ is 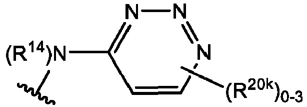 and R¹⁴ and R^{20k} are as described herein. In some embodiments, R⁵ is 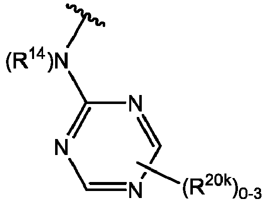 and R¹⁴ and R^{20k} are as described herein. In some embodiments, R⁵ is 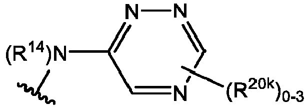 and R¹⁴ and R^{20k} are as described herein. In some embodiments, R⁵ is 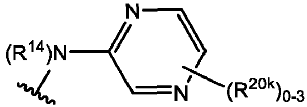 and R¹⁴ and R^{20k} are as described herein. In some embodiments, R⁵ is 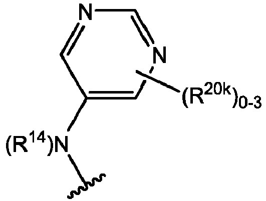 and R¹⁴ and R^{20k} are as described herein. In some embodiments, R⁵ is 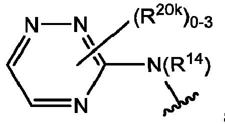 and R¹⁴ and R^{20k} are as described herein. In some embodiments, R⁵ is 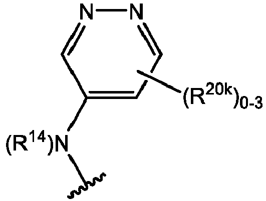 and R¹⁴ and R^{20k} are as described herein. In some embodiments, R⁵ is 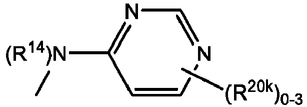 and R¹⁴ and R^{20k} are as described herein. In some embodiments, R⁵ is 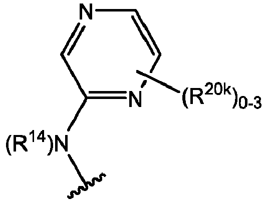 and R¹⁴ and R^{20k} are as described herein. In some embodiments, R⁵ is 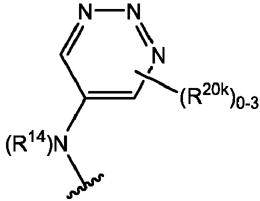 and R¹⁴ and R^{20k} are as described herein. In some embodiments, R⁵ is 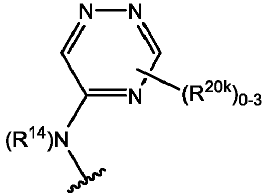 and R¹⁴ and R^{20k} are as described herein. In embodiments, R¹⁴ is hydrogen. In embodiments, R^{20k} is independently halogen. In embodiments, R^{20k} is independently F. In embodiments, R^{20k} is independently -CN. In embodiments, R^{20k} is independently C₁₋₆alkyl. In embodiments, R^{20k} is independently C₃₋₆cycloalkyl.

The embodiments of R¹ and R⁴ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. In additional embodiments of the subject complex or compound (e.g., precursor compound), two substituents selected from R¹ and R⁴ that are bonded to the same atom are joined to form a C₃₋₁₂cycloalkyl or C₁₋₁₁heterocycloalkyl, wherein the C₃₋₁₂cycloalkyl and C₁₋₁₁heterocycloalkyl are optionally substituted with one, two, or three R^{20a}. In additional embodiments of the subject complex or compound (e.g., precursor compound), two substituents selected from R¹ and R⁴ that are bonded to the same atom are joined to form a C₃₋₁₂cycloalkyl or C₁₋₁₁heterocycloalkyl. In additional embodiments of the subject complex or compound (e.g., precursor compound), two substituents selected from R¹ and R⁴ that are bonded to adjacent atoms are joined to form a C₃₋₁₂cycloalkyl, C₁₋₁₁heterocycloalkyl, C₆₋₁₂aryl, or C_{1- 11}heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a}. In additional embodiments of the subject complex or compound (e.g., precursor compound), two substituents selected from R¹ and R⁴ that are bonded to adjacent atoms are joined to form a C₃₋₁₂cycloalkyl, C₁₋₁₁heterocycloalkyl, C₆₋₁₂aryl, or C₁₋₁₁heteroaryl. In some embodiments of the subject complex or compound (e.g., precursor compound), two substituents selected from R¹ and R⁴ that are bonded to adjacent atoms are joined to form a C₃₋₁₂cycloalkyl, wherein the C₃₋₁₂cycloalkyl is optionally substituted with one, two, or three R^{20a}. In additional embodiments of the subject complex or compound (e.g., precursor compound), two substituents selected from R¹ and R⁴ that are bonded to adjacent atoms are joined to form a C₃₋₁₂cycloalkyl. In further embodiments of the subject complex or compound (e.g., precursor compound), two substituents selected from R¹ and R⁴ that are bonded to adjacent atoms are joined to form a C₁₋₁₁heterocycloalkyl, wherein the C₁₋₁₁heterocycloalkyl is optionally substituted with one, two, or three R^{20a}. In some embodiments of the subject complex or compound (e.g., precursor compound), two substituents selected from R¹ and R⁴ that are bonded to adjacent atoms are joined to form a C_{1- 11}heterocycloalkyl. In additional embodiments of the subject complex or compound (e.g., precursor compound), two substituents selected from R¹ and R⁴ that are bonded to adjacent atoms are joined to form a C₆₋₁₂aryl, wherein the C₆₋₁₂aryl is optionally substituted with one, two, or three R^{20a}. In embodiments of the subject complex or compound (e.g., precursor compound), two substituents selected from R¹ and R⁴ that are bonded to adjacent atoms are joined to form a C₆₋₁₂aryl. In additional embodiments of the subject complex or compound (e.g., precursor compound), two substituents selected from R¹ and R⁴ that are bonded to adjacent atoms are joined to form a C₁₋₁₁heteroaryl, wherein the C₁₋₁₁heteroaryl is optionally substituted with one, two, or three R^{20a}. In additional embodiments of the subject complex or compound (e.g., precursor compound), two substituents selected from R¹ and R⁴ that are bonded to adjacent atoms are joined to form a C₁₋₁₁heteroaryl.

In additional embodiments of the subject complex or compound (e.g., precursor compound), R⁴ is independently selected from hydrogen, halogen, oxo, -CN, C₁₋₆alkyl, C₂₋₆alkenyl, C₂₋₆alkynyl, -OR¹², - N(R¹²)(R¹³), -C(O)OR¹², -OC(O)N(R¹²)(R¹³), and -C(O)R¹⁵, wherein C₁₋₆alkyl, C₂₋₆alkenyl, and C_{2- 6}alkynyl, are optionally substituted with one, two, or three R^{20a}. In embodiments of the subject complex or compound (e.g., precursor compound), R⁴ is independently hydrogen. In further embodiments of the subject complex or compound (e.g., precursor compound), R⁴ is independently halogen. In some embodiments of the subject complex or compound (e.g., precursor compound), R⁴ is independently oxo. In some embodiments of the subject complex or compound (e.g., precursor compound), R⁴ is independently - CN. In additional embodiments of the subject complex or compound (e.g., precursor compound), R⁴ is independently C₁₋₆alkyl. In embodiments of the subject complex or compound (e.g., precursor compound), R⁴ is independently C₂₋₆alkenyl. In some embodiments of the subject complex or compound (e.g., precursor compound), R⁴ is independently C₂₋₆alkynyl. In further embodiments of the subject complex or compound (e.g., precursor compound), R⁴ is independently -OR¹². In select embodiments of the subject complex or compound (e.g., precursor compound), R⁴ is independently -N(R¹²)(R¹³). In additional embodiments of the subject complex or compound (e.g., precursor compound), R⁴ is independently - C(O)OR¹². In embodiments of the subject complex or compound (e.g., precursor compound), R⁴ is independently -OC(O)N(R¹²)(R¹³). In some embodiments of the subject complex or compound (e.g., precursor compound), R⁴ is independently -C(O)R¹⁵. In select embodiments of the subject complex or compound (e.g., precursor compound), R⁴ is independently -NH₂. In further embodiments of the subject complex or compound (e.g., precursor compound), R⁴ is independently -C(O)OH. In additional embodiments of the subject complex or compound (e.g., precursor compound), R⁴ is independently - OC(O)NH₂. In embodiments of the subject complex or compound (e.g., precursor compound), R⁴ is independently -C(O)CH₃.

The embodiments of R¹⁹ as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. In select embodiments of the subject complex or compound (e.g., precursor compound), R¹⁹ is a C₃₋₁₂cycloalkyl optionally substituted with one, two, three, four, five, six, or seven R¹ⁱ. In additional embodiments of the subject complex or compound (e.g., precursor compound), R¹⁹ is a C_{2- 11}heterocycloalkyl optionally substituted with one, two, three, four, five, six, or seven R¹ⁱ. In further embodiments of the subject complex or compound (e.g., precursor compound), R¹⁹ is a C₆₋₁₂aryl optionally substituted with one, two, three, four, five, six, or seven R¹ⁱ. In some embodiments of the subject complex or compound (e.g., precursor compound), R¹⁹ is a C₂₋₁₂heteroaryl optionally substituted with one, two, three, four, five, six, or seven R¹ⁱ. In embodiments of the subject complex or compound (e.g., precursor compound), R¹⁹ is a C₃₋₁₂cycloalkyl. In select embodiments of the subject complex or compound (e.g., precursor compound), R¹⁹ is a C₂₋₁₁heterocycloalkyl. In additional embodiments of the subject complex or compound (e.g., precursor compound), R¹⁹ is a C₆₋₁₂aryl. In some embodiments of the subject complex or compound (e.g., precursor compound), R¹⁹ is a C₂₋₁₂heteroaryl. In some embodiments of the subject complex or compound (e.g., precursor compound), or a pharmaceutically acceptable salt or solvate thereof, R¹⁹ is a monocyclic C₃₋₉cycloalkyl optionally substituted with one, two, three, four, five, six, or seven R¹ⁱ. In embodiments of the subject complex or compound (e.g., precursor compound), or a pharmaceutically acceptable salt or solvate thereof, R¹⁹ is a monocyclic C₁₋₈heterocycloalkyl optionally substituted with one, two, three, four, five, six, or seven R¹ⁱ. In additional embodiments of the subject complex or compound (e.g., precursor compound), or a pharmaceutically acceptable salt or solvate thereof, R¹⁹ is a monocyclic phenyl optionally substituted with one, two, three, four, or five R¹ⁱ. In further embodiments of the subject complex or compound (e.g., precursor compound), or a pharmaceutically acceptable salt or solvate thereof, R¹⁹ is a monocyclic C₁₋₅heteroaryl optionally substituted with one, two, three, four, or five R¹ⁱ. In some embodiments of the subject complex or compound (e.g., precursor compound), or a pharmaceutically acceptable salt or solvate thereof, R¹⁹ is a spirocyclic C₅₋₁₂cycloalkyl optionally substituted with one, two, three, four, five, six, or seven R¹ⁱ. In embodiments of the subject complex or compound (e.g., precursor compound), or a pharmaceutically acceptable salt or solvate thereof, R¹⁹ is a spirocyclic C_{2- 11}heterocycloalkyl optionally substituted with one, two, three, four, five, six, or seven R¹ⁱ. In additional embodiments of the subject complex or compound (e.g., precursor compound), or a pharmaceutically acceptable salt or solvate thereof, R¹⁹ is a fused C₄₋₁₂cycloalkyl optionally substituted with one, two, three, four, five, six, or seven R¹ⁱ. In further embodiments of the subject complex or compound (e.g., precursor compound), or a pharmaceutically acceptable salt or solvate thereof, R¹⁹ is a fused C₂₋₁₁heterocycloalkyl optionally substituted with one, two, three, four, five, six, or seven R¹ⁱ. In further embodiments of the subject complex or compound (e.g., precursor compound), or a pharmaceutically acceptable salt or solvate thereof, R¹⁹ is a fused C₆₋₁₂aryl, optionally substituted with one, two, three, four, five, six, or seven R¹ⁱ. In some embodiments of the subject complex or compound (e.g., precursor compound), or a pharmaceutically acceptable salt or solvate thereof, R¹⁹ is a fused 5 to 12 membered heteroaryl optionally substituted with one, two, three, four, five, six, or seven R¹ⁱ.

In select embodiments of the subject complex or compound (e.g., precursor compound), R^{12b} is C₁₋₆alkyl optionally substituted with one, two, or three R^{20d}. In embodiments of the subject complex or compound (e.g., precursor compound), R^{12b} is methylene optionally substituted with one or two R^{20d}. In further embodiments of the subject complex or compound (e.g., precursor compound), R^{12b} is methylene. In some embodiments of the subject complex or compound (e.g., precursor compound), R^{12b} is ethylene optionally substituted with one, two, or three R^{20d}. In embodiments of the subject complex or compound (e.g., precursor compound), R^{12b} is ethylene. In some embodiments of the subject complex or compound (e.g., precursor compound), R^{12b} is propylene optionally substituted with one, two, or three R^{20d}. In embodiments of the subject complex or compound (e.g., precursor compound), R^{12b} is propylene. In some embodiments, R^{12b} is -CH₂-C₂₋₉heterocycloalkyl optionally substituted with one, two, or three R^{20d}. In some embodiments, R^{12b} is -CH₂-(monocyclic C₂₋₈heterocycloalkyl) optionally substituted with one, two, or three R^{20d}. In some embodiments, R^{12b} is -CH₂-(monocyclic C₃₋₅heterocycloalkyl) optionally substituted with one, two, or three R^{20d}. In some embodiments, R^{12b} is -CH₂-(spirocyclic C₂₋₁₁heterocycloalkyl) optionally substituted with one, two, or three R^{20d}. In some embodiments, R^{12b} is -CH₂-(spirocyclic C_{3- 11}heterocycloalkyl) optionally substituted with one, two, or three R^{20d}. In some embodiments, R^{12b} is -CH₂-(fused C₂₋₁₁heterocycloalkyl) optionally substituted with one, two, or three R^{20d}. In some embodiments, R^{12b} is -CH₂-(spirocyclic C₆₋₈heterocycloalkyl) optionally substituted with one, two, or three R^{20d}.

In additional embodiments of the subject complex or compound (e.g., precursor compound), R^{12b} is hydrogen.

In further embodiments of the subject complex or compound (e.g., precursor compound), each R¹² is independently selected from hydrogen, C₁₋₆alkyl, and C₃₋₆cycloalkyl. In some embodiments of the subject complex or compound (e.g., precursor compound), each R¹² is independently selected from hydrogen or C_{1- 6}alkyl. In embodiments of the subject complex or compound (e.g., precursor compound), R¹² is independently hydrogen. In select embodiments of the subject complex or compound (e.g., precursor compound), each R¹³ is independently selected from hydrogen and C₁₋₄alkyl. In some embodiments of the subject complex or compound (e.g., precursor compound), each R¹⁴ is independently selected from hydrogen and C₁₋₄alkyl. In additional embodiments of the subject complex or compound (e.g., precursor compound), each R¹⁵ is independently C₁₋₄alkyl.

In embodiments of the subject complex or compound (e.g., precursor compound), R¹⁹ is 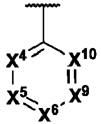 wherein X⁴, X⁵, X⁶, X⁹, and X¹⁰ are as described herein. In embodiments of the subject complex or compound (e.g., precursor compound), R¹⁹ is 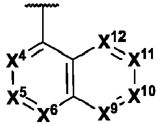 wherein X⁴, X⁵, X⁶, X⁹, X¹⁰, X¹¹, and X¹² are as described herein. In embodiments of the subject complex or compound (e.g., precursor compound), R¹⁹ is 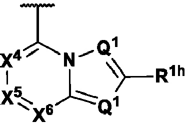 wherein X⁴, X⁵, X⁶, Q¹, and R^{1h} are as described herein. In embodiments of the subject complex or compound (e.g., precursor compound), R¹⁹ is 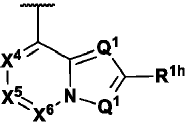 wherein X⁴, X⁵, X⁶, Q¹, and R^{1h} are as described herein. In embodiments of the subject complex or compound (e.g., precursor compound), R¹⁹ is 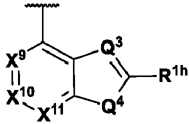 wherein X⁹, X¹⁰, X¹¹, Q³, Q⁴, and R^{1h} are as described herein. In embodiments of the subject complex or compound (e.g., precursor compound), R¹⁹ is 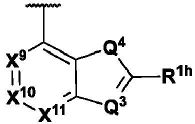 wherein X⁹, X¹⁰, X¹¹, Q³, Q⁴, and R^{1h} are as described herein. In embodiments of the subject complex or compound (e.g., precursor compound), R¹⁹ is 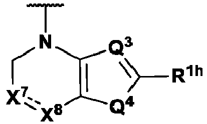 wherein X⁷, X⁸, X¹², Q³, Q⁴, and R^{1h} are as described herein. In embodiments of the subject complex or compound (e.g., precursor compound), R¹⁹ is 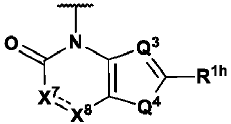 wherein X⁷, X⁸, Q³, Q⁴, and R^{1h} are as described herein. In embodiments of the subject complex or compound (e.g., precursor compound), R¹⁹ is 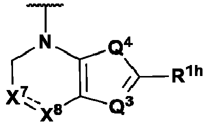 wherein X⁷, X⁸, Q³, Q⁴, and R^{1h} are as described herein. In embodiments of the subject complex or compound (e.g., precursor compound), R¹⁹ is 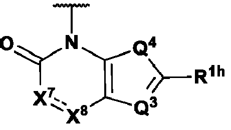 wherein X⁷, X⁸, Q³, Q⁴, and R^{1h} are as described herein. In embodiments of the subject complex or compound (e.g., precursor compound), R¹⁹ is 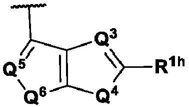 wherein Q³, Q⁴, Q⁵, Q⁶, and R^{1h} are as described herein. In embodiments of the subject complex or compound (e.g., precursor compound), R¹⁹ is 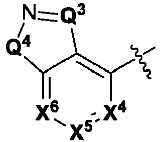 wherein X⁴, X⁵, X⁶, Q³, and Q⁴ are as described herein. In embodiments of the subject complex or compound (e.g., precursor compound), R¹⁹ is 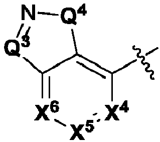 wherein X⁴, X⁵, X⁶, Q³, and Q⁴ are as described herein. In embodiments of the subject complex or compound (e.g., precursor compound), R¹⁹ is 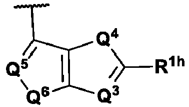 wherein, Q³, Q⁴, Q⁵, Q⁶, and R^{1h} are as described herein.

In embodiments of the subject complex or compound (e.g., precursor compound), R¹⁹ is 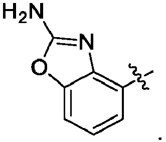 In some embodiments of the subject complex or compound (e.g., precursor compound), R¹⁹ is 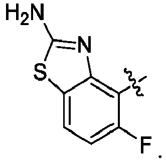 In further embodiments of the subject complex or compound (e.g., precursor compound), R¹⁹ is 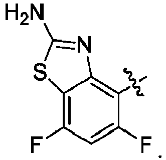 In additional embodiments of the subject complex or compound (e.g., precursor compound), R¹⁹ is 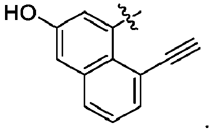 In select embodiments of the subject complex or compound (e.g., precursor compound), R¹⁹ is 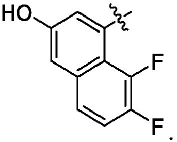 In embodiments of the subject complex or compound (e.g., precursor compound), R¹⁹ is 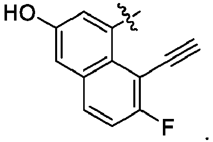 In some embodiments of the subject complex or compound (e.g., precursor compound), R¹⁹ is 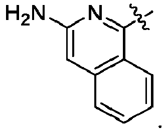 In some embodiments of the subject complex or compound (e.g., precursor compound), R¹⁹ is 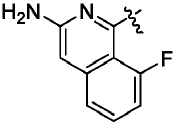 . In further embodiments of the subject complex or compound (e.g., precursor compound), R¹⁹ is 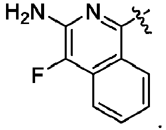 In select embodiments of the subject complex or compound (e.g., precursor compound), R¹⁹ is 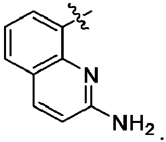 In embodiments of the subject complex or compound (e.g., precursor compound), R¹⁹ is 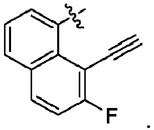 In some embodiments of the subject complex or compound (e.g., precursor compound), R¹⁹ is 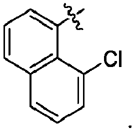 In additional embodiments of the subject complex or compound (e.g., precursor compound), R¹⁹ is 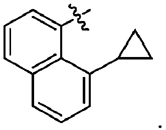 In some embodiments of the subject complex or compound (e.g., precursor compound), R¹⁹ is 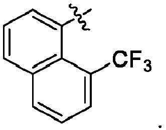 In further embodiments of the subject complex or compound (e.g., precursor compound), R¹⁹ is 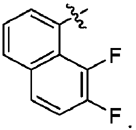 In embodiments of the subject complex or compound (e.g., precursor compound), R¹⁹ is 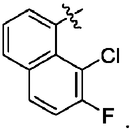 In select embodiments of the subject complex or compound (e.g., precursor compound), R¹⁹ is 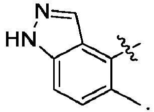 In some embodiments of the subject complex or compound (e.g., precursor compound), R¹⁹ is 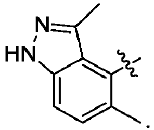 In additional embodiments of the subject complex or compound (e.g., precursor compound), R¹⁹ is 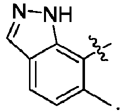 In embodiments of the subject complex or compound (e.g., precursor compound), R¹⁹ is 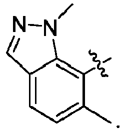 In embodiments of the subject complex or compound (e.g., precursor compound), R¹⁹ is 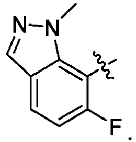 In further embodiments of the subject complex or compound (e.g., precursor compound), R¹⁹ is 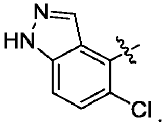 In select embodiments of the subject complex or compound (e.g., precursor compound), R¹⁹ is 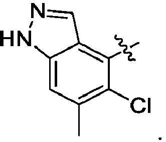 In some embodiments of the subject complex or compound (e.g., precursor compound), R¹⁹ is 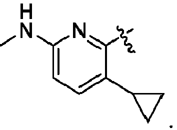 In additional embodiments of the subject complex or compound (e.g., precursor compound), R¹⁹ is 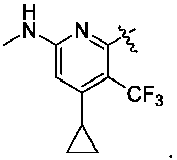 In embodiments of the subject complex or compound (e.g., precursor compound), R¹⁹ is 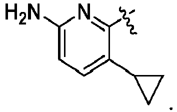 In some embodiments of the subject complex or compound (e.g., precursor compound), R¹⁹ is 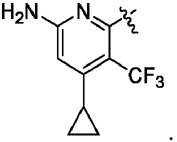 In further embodiments of the subject complex or compound (e.g., precursor compound), R¹⁹ is 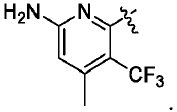 In select embodiments of the subject complex or compound (e.g., precursor compound), R¹⁹ is 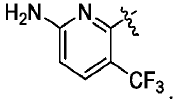 In some embodiments of the subject complex or compound (e.g., precursor compound), R¹⁹ is 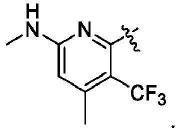 In embodiments of the subject complex or compound (e.g., precursor compound), R¹⁹ is 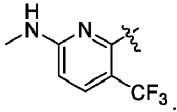 In some embodiments of the subject complex or compound (e.g., precursor compound), R¹⁹ is 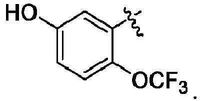 In additional embodiments of the subject complex or compound (e.g., precursor compound), R¹⁹ is 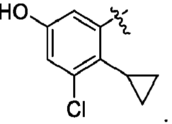 In further embodiments of the subject complex or compound (e.g., precursor compound), R¹⁹ is 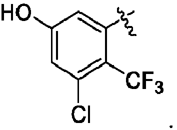 In select embodiments of the subject complex or compound (e.g., precursor compound), R¹⁹ is 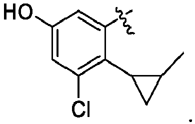 In embodiments of the subject complex or compound (e.g., precursor compound), R¹⁹ is 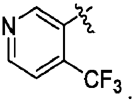 In some embodiments of the subject complex or compound (e.g., precursor compound), R¹⁹ is

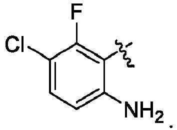

In embodiments of the subject complex or compound (e.g., precursor compound), R¹⁷ is 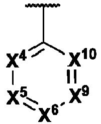 wherein X⁴, X⁵, X⁶, X⁹, and X¹⁰ are as described herein. In embodiments of the subject complex or compound (e.g., precursor compound), R¹⁷ is 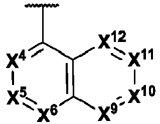 wherein X⁴, X⁵, X⁶, X⁹, X¹⁰, X¹¹, and X¹² are as described herein. In embodiments of the subject complex or compound (e.g., precursor compound), R¹⁷ is 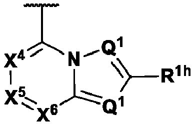 wherein X⁴, X⁵, X⁶, Q¹, and R^{1h} are as described herein. In embodiments of the subject complex or compound (e.g., precursor compound), R¹⁷ is 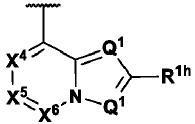 wherein X⁴, X⁵, X⁶, Q¹, and R^{1h} are as described herein. In embodiments of the subject complex or compound (e.g., precursor compound), R¹⁷ is 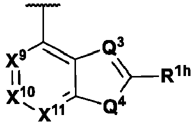 wherein X⁹, X¹⁰, X¹¹, Q³, Q⁴, and R^{1h} are as described herein. In embodiments of the subject complex or compound (e.g., precursor compound), R¹⁷ is 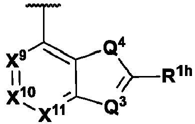 wherein X⁹, X¹⁰, X¹¹, Q³, Q⁴, and R^{1h} are as described herein. In embodiments of the subject complex or compound (e.g., precursor compound), R¹⁷ is 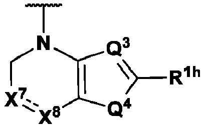 wherein X⁷, X⁸, X¹², Q³, Q⁴, and R^{1h} are as described herein. In embodiments of the subject complex or compound (e.g., precursor compound), R¹⁷ is 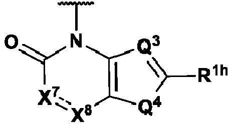 wherein X⁷, X⁸, Q³, Q⁴, and R^{1h} are as described herein. In embodiments of the subject complex or compound (e.g., precursor compound), R¹⁷ is 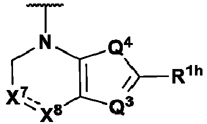 wherein X⁷, X⁸, Q³, Q⁴, and R^{1h} are as described herein. In embodiments of the subject complex or compound (e.g., precursor compound), R¹⁷ is 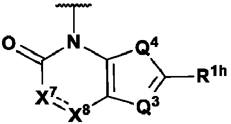 wherein X⁷, X⁸, Q³, Q⁴, and R^{1h} are as described herein. In embodiments of the subject complex or compound (e.g., precursor compound), R¹⁷ is 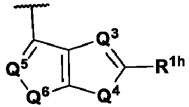 wherein Q³, Q⁴, Q⁵, Q⁶, and R^{1h} are as described herein. In embodiments of the subject complex or compound (e.g., precursor compound), R¹⁷ is 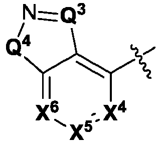 wherein X⁴, X⁵, X⁶, Q³, and Q⁴ are as described herein. In embodiments of the subject complex or compound (e.g., precursor compound), R¹⁷ is 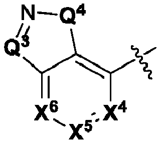 wherein X⁴, X⁵, X⁶, Q³, and Q⁴ are as described herein. In embodiments of the subject complex or compound (e.g., precursor compound), R¹⁷ is 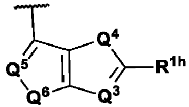 wherein, Q³, Q⁴, Q⁵, Q⁶, and R^{1h} are as described herein.

In some embodiments of the subject complex or compound (e.g., precursor compound), R¹⁷ is 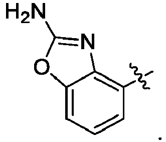 In select embodiments of the subject complex or compound (e.g., precursor compound), R¹⁷ is 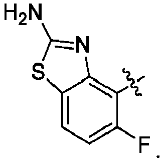 In further embodiments of the subject complex or compound (e.g., precursor compound), R¹⁷ is 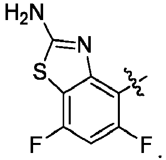 In embodiments of the subject complex or compound (e.g., precursor compound), R¹⁷ is 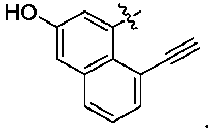 In additional embodiments of the subject complex or compound (e.g., precursor compound), R¹⁷ is 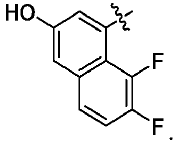 In some embodiments of the subject complex or compound (e.g., precursor compound), R¹⁷ is 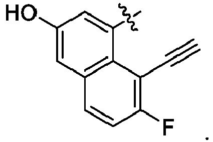 In embodiments of the subject complex or compound (e.g., precursor compound), R¹⁷ is 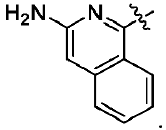 In select embodiments of the subject complex or compound (e.g., precursor compound), R¹⁷ is 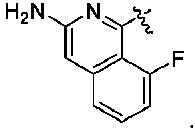 In embodiments of the subject complex or compound (e.g., precursor compound), R¹⁷ is 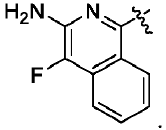 In further embodiments of the subject complex or compound (e.g., precursor compound), R¹⁷ is 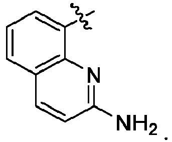 In some embodiments of the subject complex or compound (e.g., precursor compound), R¹⁷ is 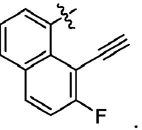 In additional embodiments of the subject complex or compound (e.g., precursor compound), R¹⁷ is 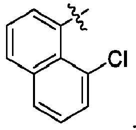 In some embodiments of the subject complex or compound (e.g., precursor compound), R¹⁷ is 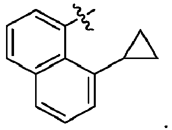 In embodiments of the subject complex or compound (e.g., precursor compound), R¹⁷ is 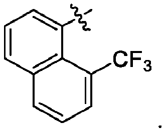 In select embodiments of the subject complex or compound (e.g., precursor compound), R¹⁷ is 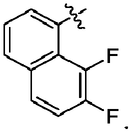 In further embodiments of the subject complex or compound (e.g., precursor compound), R¹⁷ is 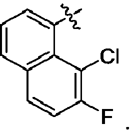 In some embodiments of the subject complex or compound (e.g., precursor compound), R¹⁷ is 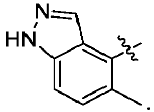 In embodiments of the subject complex or compound (e.g., precursor compound), R¹⁷ is 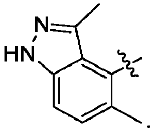 In embodiments of the subject complex or compound (e.g., precursor compound), R¹⁷ is 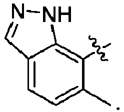 In additional embodiments of the subject complex or compound (e.g., precursor compound), R¹⁷ is 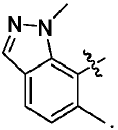 In select embodiments of the subject complex or compound (e.g., precursor compound), R¹⁷ is 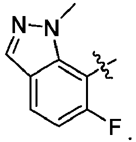 In further embodiments of the subject complex or compound (e.g., precursor compound), R¹⁷ is 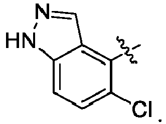 In some embodiments of the subject complex or compound (e.g., precursor compound), R¹⁷ is 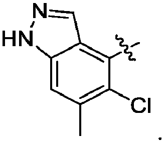 In embodiments of the subject complex or compound (e.g., precursor compound), R¹⁷ is 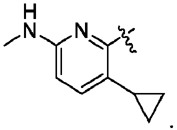 In some embodiments of the subject complex or compound (e.g., precursor compound), R¹⁷ is 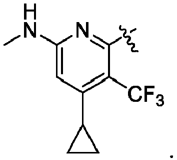 In additional embodiments of the subject complex or compound (e.g., precursor compound), R¹⁷ is 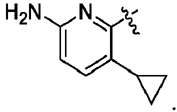 In select embodiments of the subject complex or compound (e.g., precursor compound), R¹⁷ is 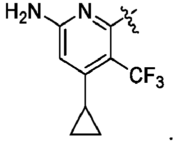 In further embodiments of the subject complex or compound (e.g., precursor compound), R¹⁷ is 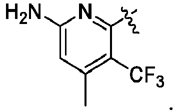 In embodiments of the subject complex or compound (e.g., precursor compound), R¹⁷ is 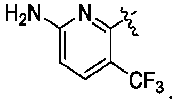 In some embodiments of the subject complex or compound (e.g., precursor compound), R¹⁷ is 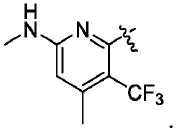 In some embodiments of the subject complex or compound (e.g., precursor compound), R¹⁷ is 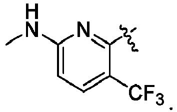 In additional embodiments of the subject complex or compound (e.g., precursor compound), R¹⁷ is 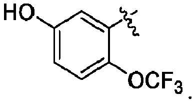 In select embodiments of the subject complex or compound (e.g., precursor compound), R¹⁷ is 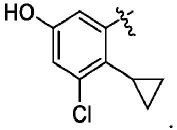 In embodiments of the subject complex or compound (e.g., precursor compound), R¹⁷ is 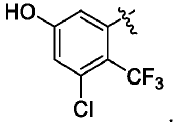 In further embodiments of the subject complex or compound (e.g., precursor compound), R¹⁷ is 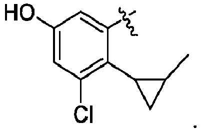 In some embodiments of the subject complex or compound (e.g., precursor compound), R¹⁷ is 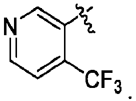 In embodiments of the subject complex or compound (e.g., precursor compound), R¹⁷ is

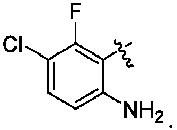

The embodiments of R² as applied to Formulae (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In') can include the following. In additional embodiments of the subject complex or compound (e.g., precursor compound), R² is 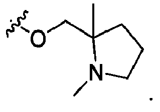 In embodiments of the subject complex or compound (e.g., precursor compound), R² is 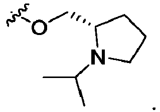 In select embodiments of the subject complex or compound (e.g., precursor compound), R² is 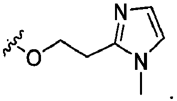 In further embodiments of the subject complex or compound (e.g., precursor compound), R² is 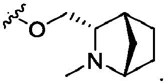 In some embodiments of the subject complex or compound (e.g., precursor compound), R² is 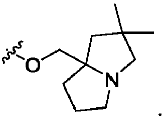 In some embodiments of the subject complex or compound (e.g., precursor compound), R² is 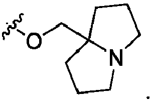 In embodiments of the subject complex or compound (e.g., precursor compound), R² is 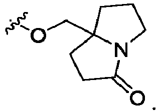 In additional embodiments of the subject complex or compound (e.g., precursor compound), R² is 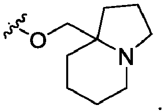 In select embodiments of the subject complex or compound (e.g., precursor compound), R² is 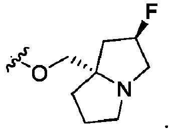 In further embodiments of the subject complex or compound (e.g., precursor compound), R² is 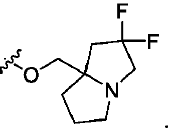 In some embodiments of the subject complex or compound (e.g., precursor compound), R² is 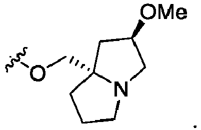 In embodiments of the subject complex or compound (e.g., precursor compound), R² is 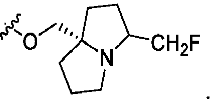 In some embodiments of the subject complex or compound (e.g., precursor compound), R² is 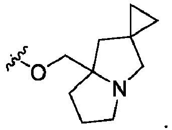 In additional embodiments of the subject complex or compound (e.g., precursor compound), R² is 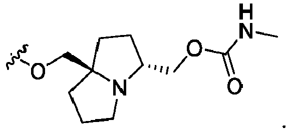 In select embodiments of the subject complex or compound (e.g., precursor compound), R² is 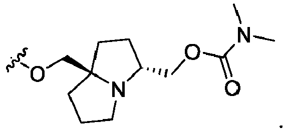 In further embodiments of the subject complex or compound (e.g., precursor compound), R² is 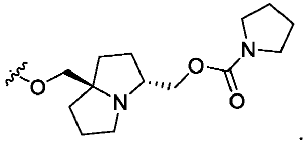 In embodiments of the subject complex or compound (e.g., precursor compound), R² is 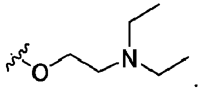 In some embodiments of the subject complex or compound (e.g., precursor compound), R² is 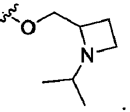 In embodiments of the subject complex or compound (e.g., precursor compound), R² is 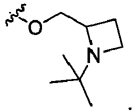 In additional embodiments of the subject complex or compound (e.g., precursor compound), R² is 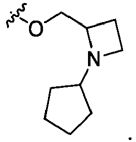 In select embodiments of the subject complex or compound (e.g., precursor compound), R² is 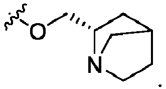 In embodiments of the subject complex or compound (e.g., precursor compound), R² is 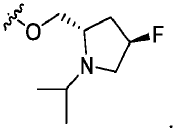 In further embodiments of the subject complex or compound (e.g., precursor compound), R² is 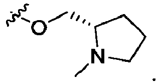 In some embodiments of the subject complex or compound (e.g., precursor compound), R² is 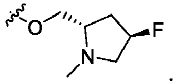 In embodiments of the subject complex or compound (e.g., precursor compound), R² is 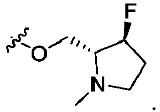 In additional embodiments of the subject complex or compound (e.g., precursor compound), R² is 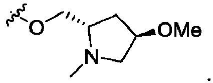 In embodiments of the subject complex or compound (e.g., precursor compound), R² is 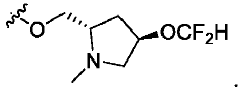 In select embodiments of the subject complex or compound (e.g., precursor compound), R² is 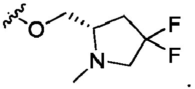 In further embodiments of the subject complex or compound (e.g., precursor compound), R² is 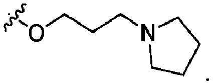 In some embodiments of the subject complex or compound (e.g., precursor compound), R² is 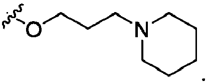 In some embodiments of the subject complex or compound (e.g., precursor compound), R² is 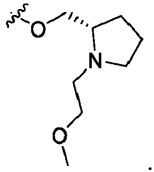 In embodiments of the subject complex or compound (e.g., precursor compound), R² is 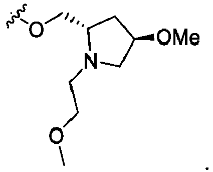 In additional embodiments of the subject complex or compound (e.g., precursor compound), R² is 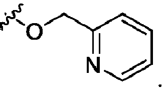 In select embodiments of the subject complex or compound (e.g., precursor compound), R² is 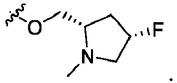 In further embodiments of the subject complex or compound (e.g., precursor compound), R² is 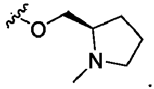 In some embodiments of the subject complex or compound (e.g., precursor compound), R² is 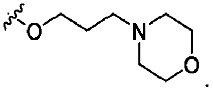 In embodiments of the subject complex or compound (e.g., precursor compound), R² is 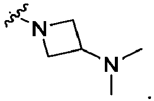 In embodiments of the subject complex or compound (e.g., precursor compound), R² is 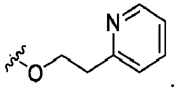 In additional embodiments of the subject complex or compound (e.g., precursor compound), R² is 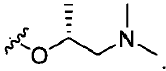 In select embodiments of the subject complex or compound (e.g., precursor compound), R² is 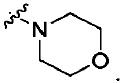 In further embodiments of the subject complex or compound (e.g., precursor compound), R² is 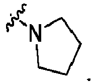 In embodiments of the subject complex or compound (e.g., precursor compound), R² is 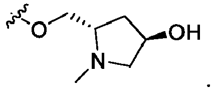 In some embodiments of the subject complex or compound (e.g., precursor compound), R² is 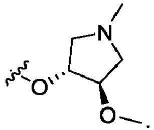 In some embodiments of the subject complex or compound (e.g., precursor compound), R² is 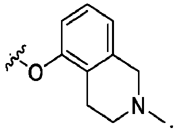 In additional embodiments of the subject complex or compound (e.g., precursor compound), R² is 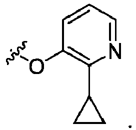 In select embodiments of the subject complex or compound (e.g., precursor compound), R² is 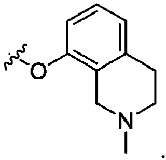 In embodiments of the subject complex or compound (e.g., precursor compound), R² is 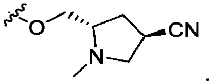 In further embodiments of the subject complex or compound (e.g., precursor compound), R² is 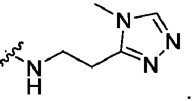 In some embodiments of the subject complex or compound (e.g., precursor compound), R² is 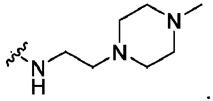 In embodiments of the subject complex or compound (e.g., precursor compound), 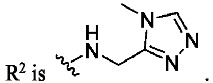 In additional embodiments of the subject complex or compound (e.g., precursor compound), R² is 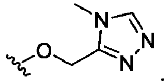 In embodiments of the subject complex or compound (e.g., precursor compound), R² is 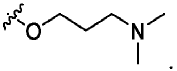 In select embodiments of the subject complex or compound (e.g., precursor compound), R² is 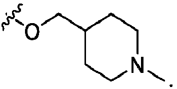 In further embodiments of the subject complex or compound (e.g., precursor compound), R² is 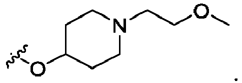 In some embodiments of the subject complex or compound (e.g., precursor compound), R² is 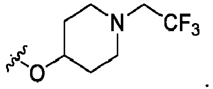 In embodiments of the subject complex or compound (e.g., precursor compound), R² is 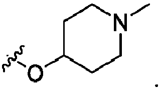 In embodiments of the subject complex or compound (e.g., precursor compound), R² is 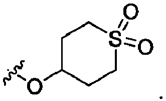 In additional embodiments of the subject complex or compound (e.g., precursor compound), R² is 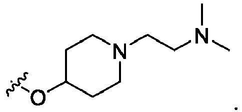 In select embodiments of the subject complex or compound (e.g., precursor compound), R² is 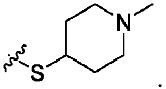 In further embodiments of the subject complex or compound (e.g., precursor compound), R² is 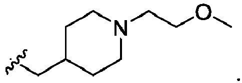 In some embodiments of the subject complex or compound (e.g., precursor compound), R² is 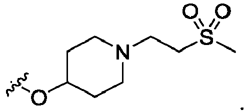 In some embodiments of the subject complex or compound (e.g., precursor compound), R² is 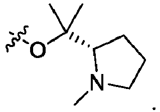 In embodiments of the subject complex or compound (e.g., precursor compound), R² is 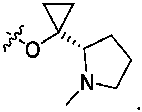 In additional embodiments of the subject complex or compound (e.g., precursor compound), R² is 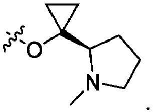 In select embodiments of the subject complex or compound (e.g., precursor compound), R² is 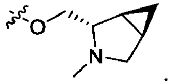 In further embodiments of the subject complex or compound (e.g., precursor compound), R² is 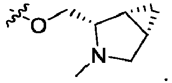 In some embodiments of the subject complex or compound (e.g., precursor compound), R² is 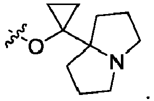 In embodiments of the subject complex or compound (e.g., precursor compound), R² is 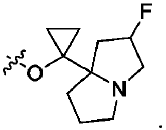 In some embodiments of the subject complex or compound (e.g., precursor compound), R² is 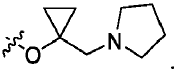 In additional embodiments of the subject complex or compound (e.g., precursor compound), R² is 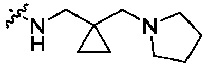 . In select embodiments of the subject complex or compound (e.g., precursor compound), R² is 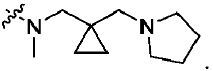 In further embodiments of the subject complex or compound (e.g., precursor compound), R² is 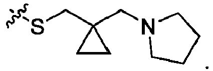 In embodiments of the subject complex or compound (e.g., precursor compound), R² is 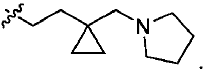 In some embodiments of the subject complex or compound (e.g., precursor compound), R² is 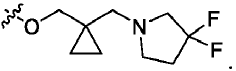 In some embodiments of the subject complex or compound (e.g., precursor compound), R² is 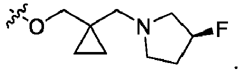 In select embodiments of the subject complex or compound (e.g., precursor compound), R² is 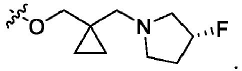 In additional embodiments of the subject complex or compound (e.g., precursor compound), R² is 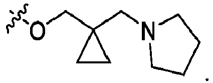 In embodiments of the subject complex or compound (e.g., precursor compound), R² is 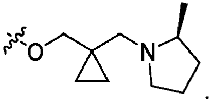 In further embodiments of the subject complex or compound (e.g., precursor compound), R² is 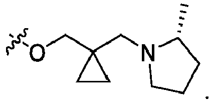 In some embodiments of the subject complex or compound (e.g., precursor compound), R² is 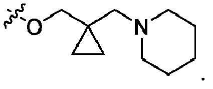 In embodiments of the subject complex or compound (e.g., precursor compound), R² is 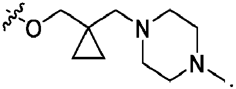 In select embodiments of the subject complex or compound (e.g., precursor compound), R² is 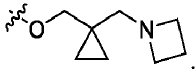 In embodiments of the subject complex or compound (e.g., precursor compound), R² is 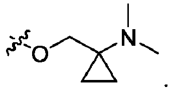 In some embodiments of the subject complex or compound (e.g., precursor compound), R² is 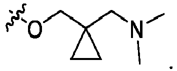 In further embodiments of the subject complex or compound (e.g., precursor compound), R² is 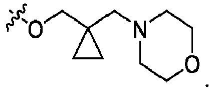 In additional embodiments of the subject complex or compound (e.g., precursor compound), R² is 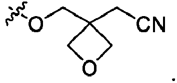 In some embodiments of the subject complex or compound (e.g., precursor compound), R² is 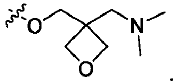 In embodiments of the subject complex or compound (e.g., precursor compound), R² is 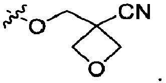 In select embodiments of the subject complex or compound (e.g., precursor compound), R² is 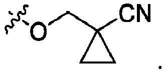 In some embodiments of the subject complex or compound (e.g., precursor compound), R² is 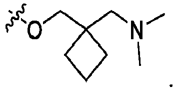 In further embodiments of the subject complex or compound (e.g., precursor compound), R² is 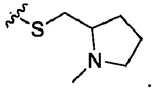 In some embodiments of the subject complex or compound (e.g., precursor compound), R² is 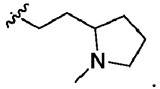 In embodiments of the subject complex or compound (e.g., precursor compound), R² is 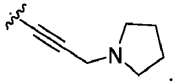 In additional embodiments of the subject complex or compound (e.g., precursor compound), R² is 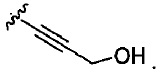 In select embodiments of the subject complex or compound (e.g., precursor compound), R² is 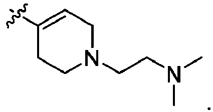 In some embodiments of the subject complex or compound (e.g., precursor compound), R² is 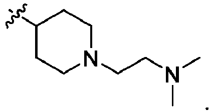 In further embodiments of the subject complex or compound (e.g., precursor compound), R² is 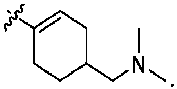 In embodiments of the subject complex or compound (e.g., precursor compound), R² is 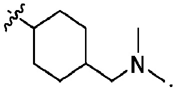 In embodiments of the subject complex or compound (e.g., precursor compound), R² is 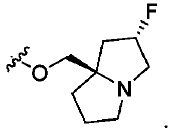 In select embodiments of the subject complex or compound (e.g., precursor compound), R² is

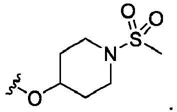

In some embodiments, R^{20a} is independently selected from halogen, -CN, C₁₋₆alkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR²¹, -SR²¹, -N(R²²)(R²³), - C(O)OR²², -C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and-OC(O)R²⁵, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵. In some embodiments, R^{20a} is independently selected from halogen, -CN, C₁₋₆alkyl, -OR²¹, - N(R²²)(R²³), -C(O)OR²², -C(O)N(R²²)(R²³), -C(O)R²⁵, and -OC(O)R²⁵, wherein C₁₋₆alkyl is optionally substituted with one, two, or three groups independently selected from halogen, oxo, -CN, C₁₋₆alkyl, C_{1- 6}haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, - N(R²⁴)S(O)₂R²⁵, -C(O)R²⁵, -S(O)₂R²⁵, -S(O)₂N(R²²)(R²³), and -OC(O)R²⁵. In some embodiments, R^{20d} is independently selected from halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR²¹, -SR²¹, -N(R²²)(R²³), -C(O)OR²², -C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, - C(O)R²⁵, -S(O)₂R²⁵, -S(O)₂N(R²²)(R²³), -OCH₂C(O)OR²², and -OC(O)R²⁵, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three groups independently selected from halogen, oxo, -CN, C₁₋₆alkyl, C₁₋₆haloalkyl, C_{1- 6}alkoxy, C₁₋₆haloalkoxy, -OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, - C(O)R²⁵, -S(O)₂R²⁵, -S(O)₂N(R²²)(R²³), and -OC(O)R²⁵. In some embodiments, R^{20d} is independently selected from halogen, -CN, C₁₋₆alkyl, -OR²¹, -N(R²²)(R²³), -C(O)OR²², -C(O)N(R²²)(R²³), -C(O)R²⁵, and - OC(O)R²⁵, wherein C₁₋₆alkyl is optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵. In some embodiments, R^{20f} is independently selected from halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR²¹, -SR²¹, -N(R²²)(R²³), - C(O)OR²², -C(O)N(R²²)(R²³), -OC(O)N(R²²)(R²³), -N(R²⁴)C(O)N(R²²)(R²³), -N(R²⁴)C(O)OR²⁵, _ N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and-OC(O)R²⁵, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and-OC(O)R²⁵. In some embodiments, R^{20f} is independently selected from halogen, -CN, C₁₋₆alkyl, -OR²¹, - N(R²²)(R²³), ^{_}C(O)OR²², -C(O)N(R²²)(R²³), -C(O)R²⁵, and -OC(O)R²⁵, wherein C₁₋₆alkyl is optionally substituted with one, two, or three groups independently selected from halogen, oxo, -CN, C₁₋₆alkyl, C_{1- 6}haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, _ N(R²⁴)S(O)₂R²⁵, -C(O)R²⁵, -S(O)₂R²⁵, -S(O)₂N(R²²)(R²³), and -OC(O)R²⁵.

In embodiments, the compound (e.g., precursor compound) is 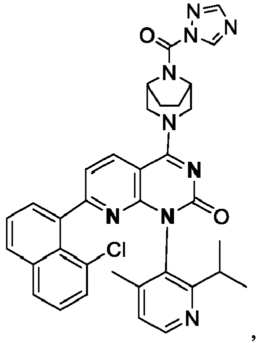 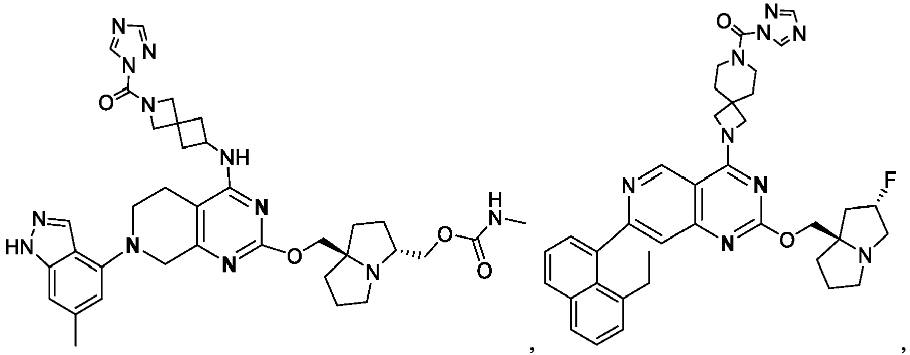 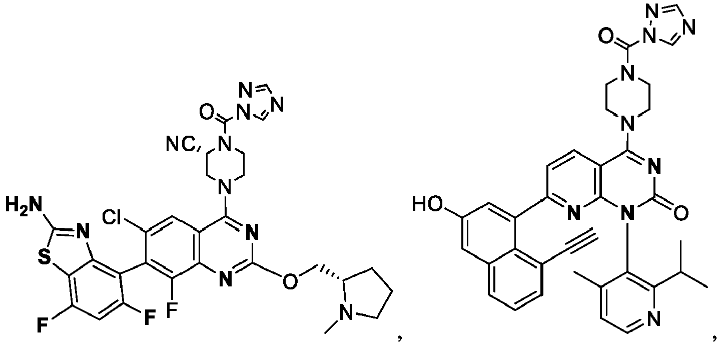 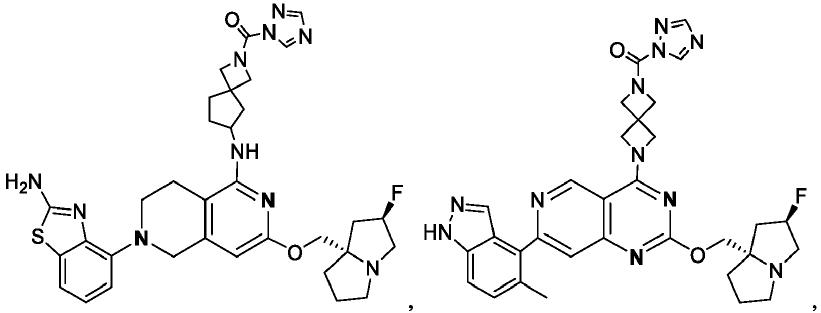 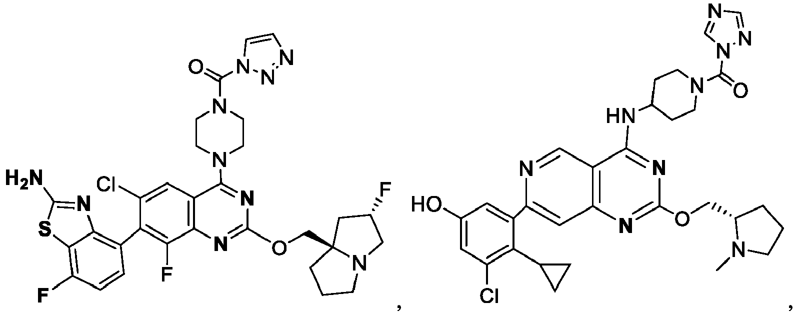 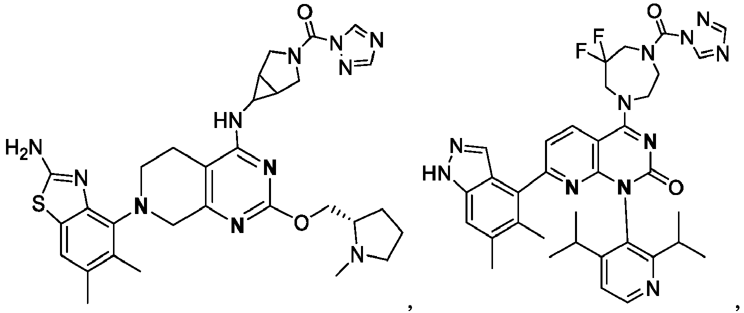 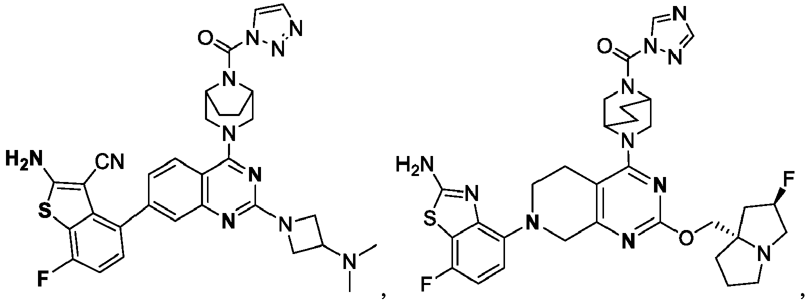 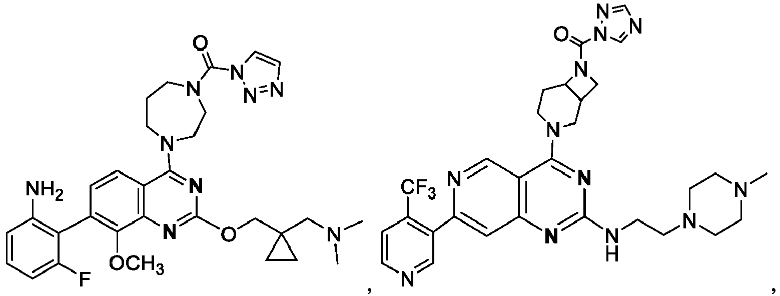 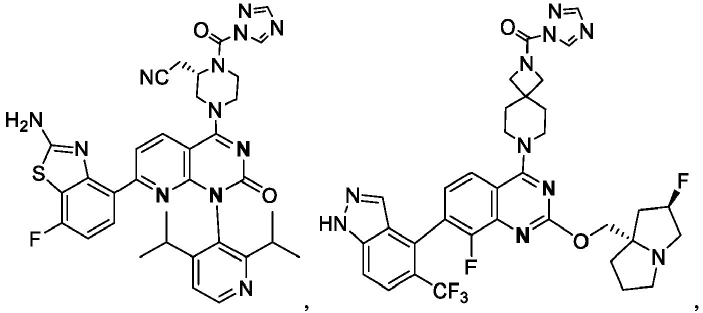 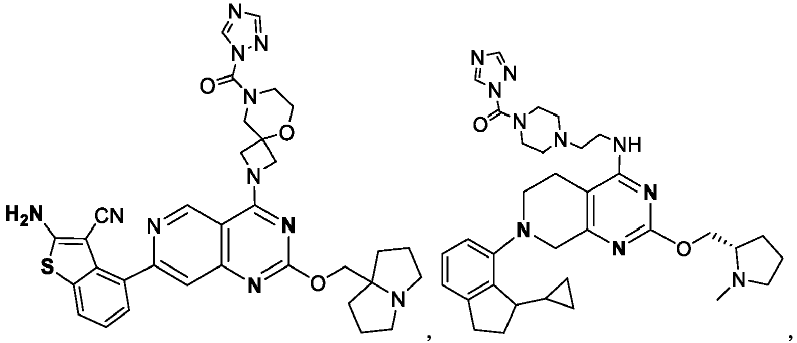 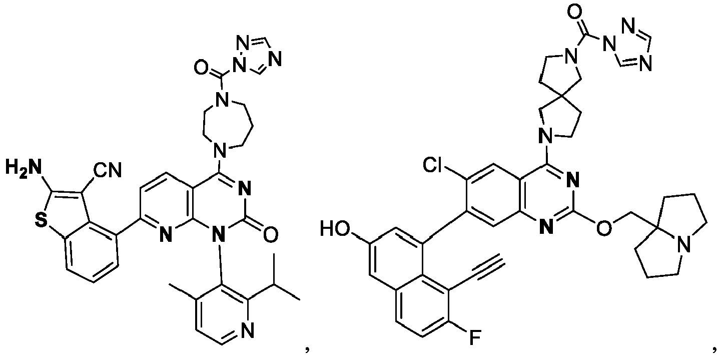 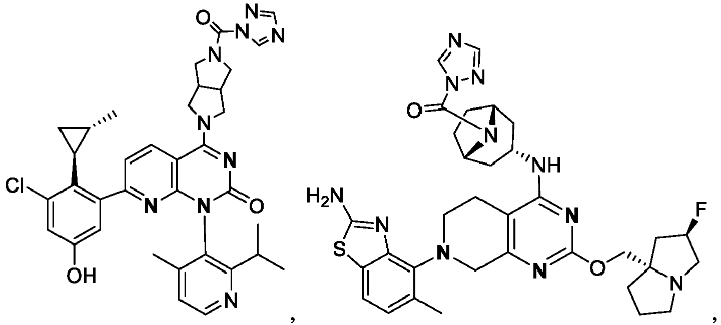 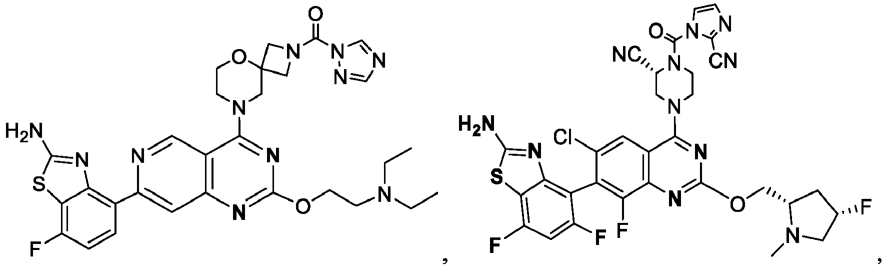 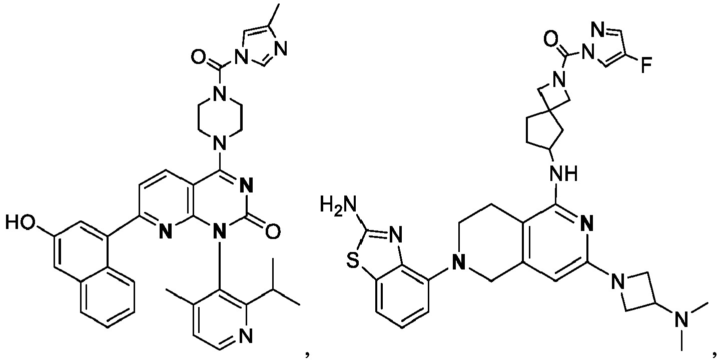 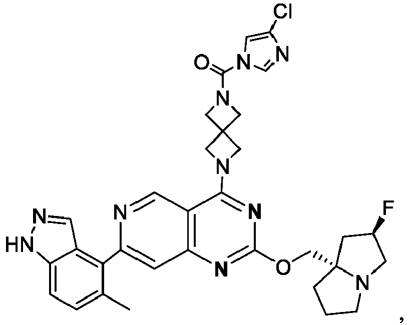 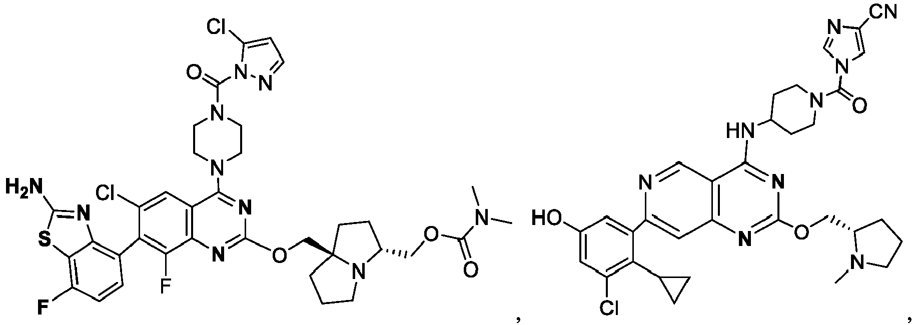 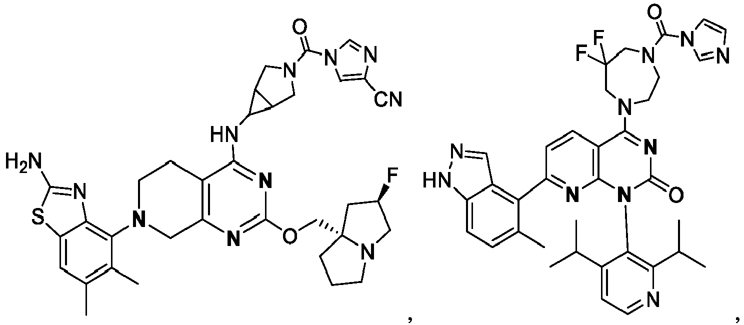 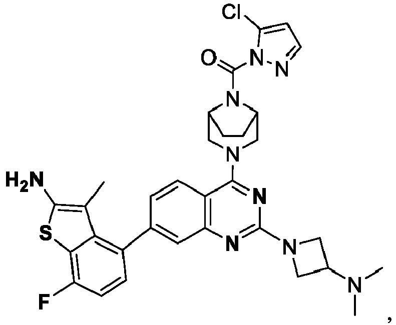 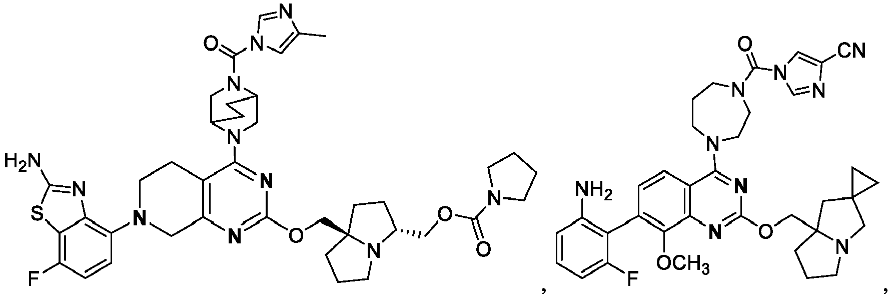 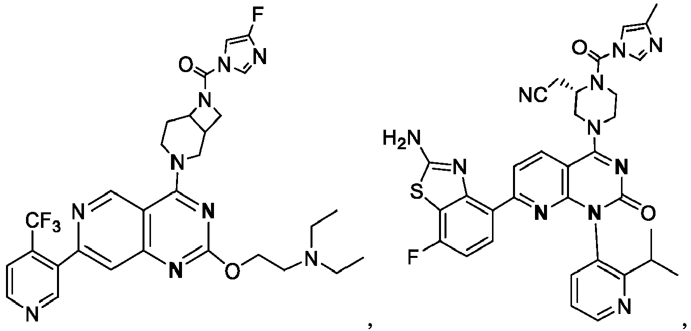 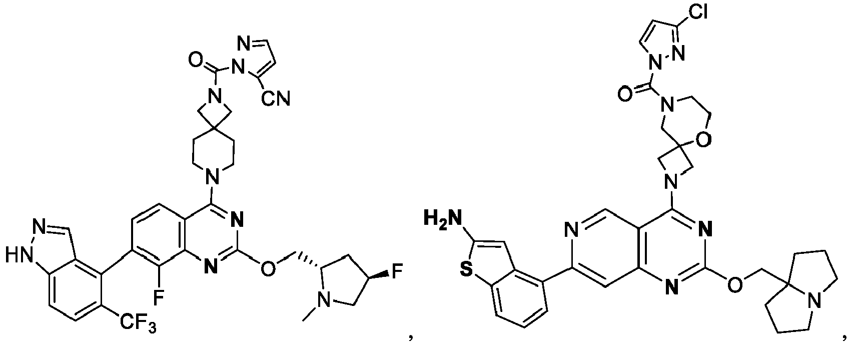 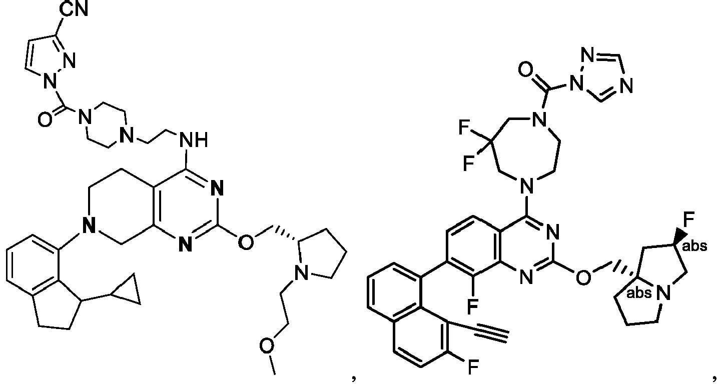 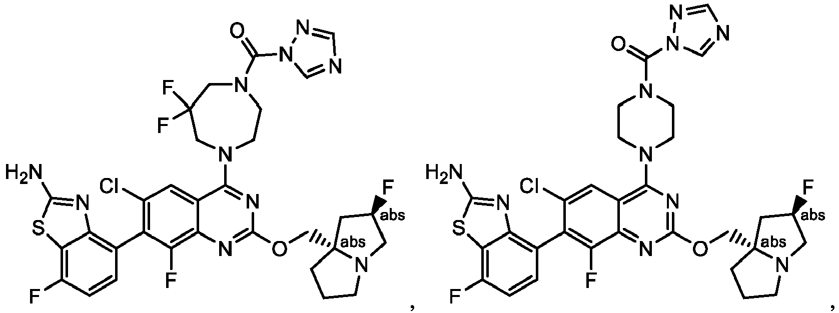 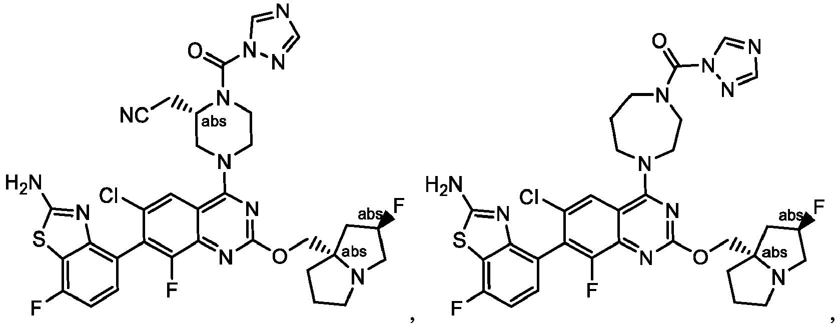 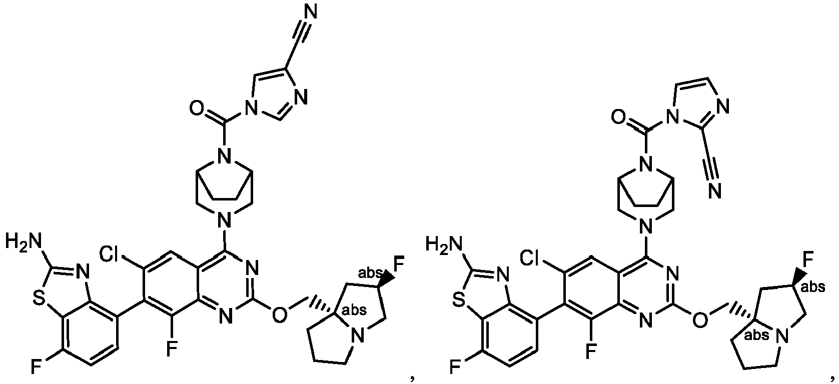 or

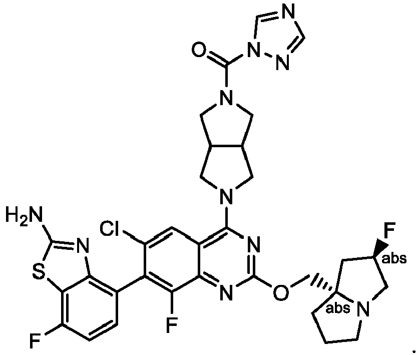

In embodiments, the compound (e.g., precursor compound) is 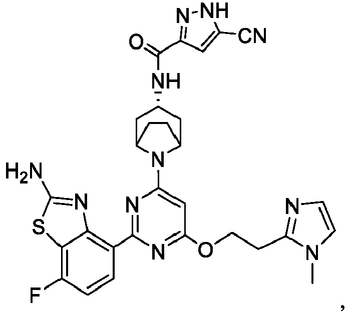 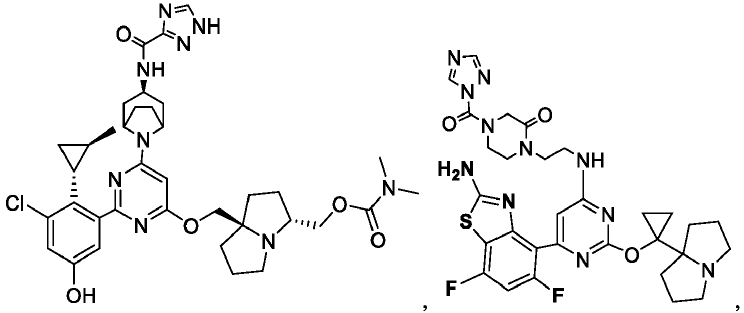 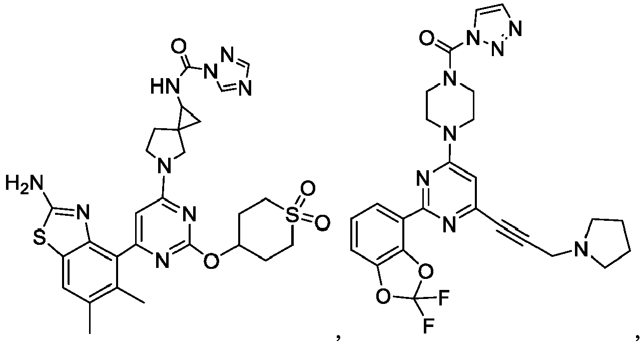 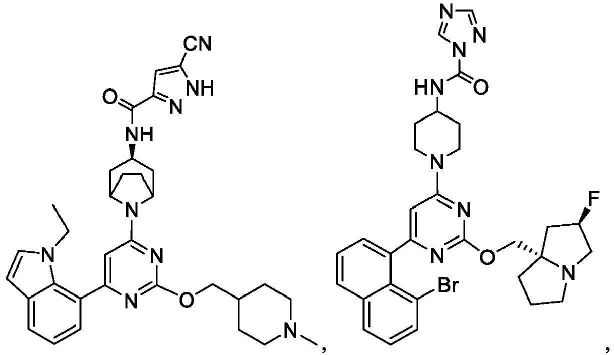 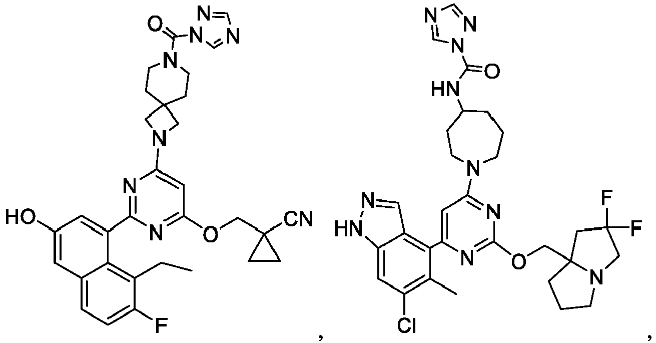 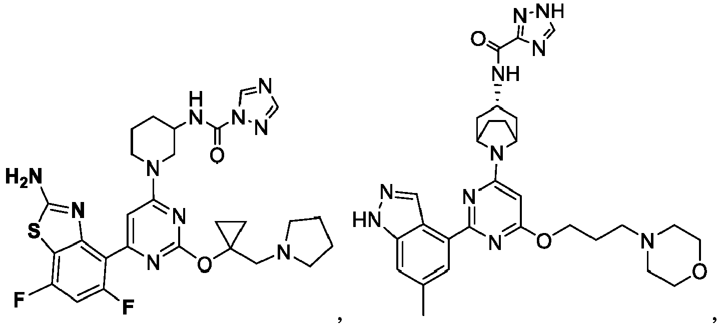 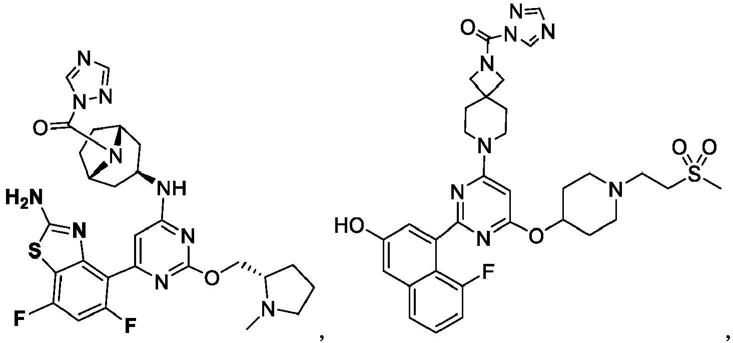 or

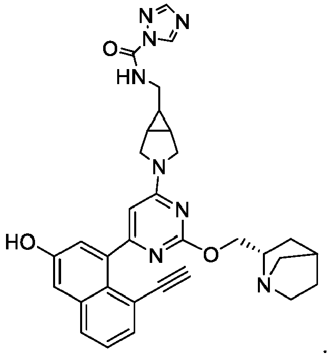

In embodiments, the compound (e.g., precursor compound) is selected from 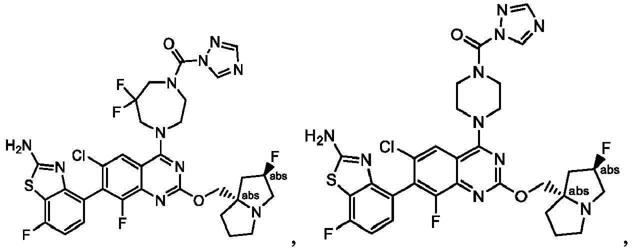 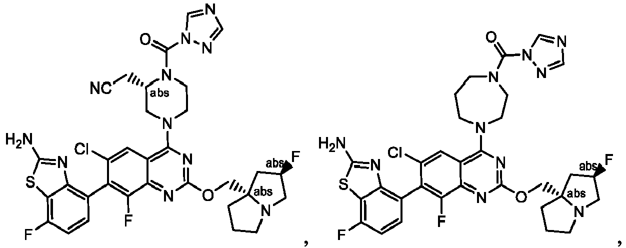 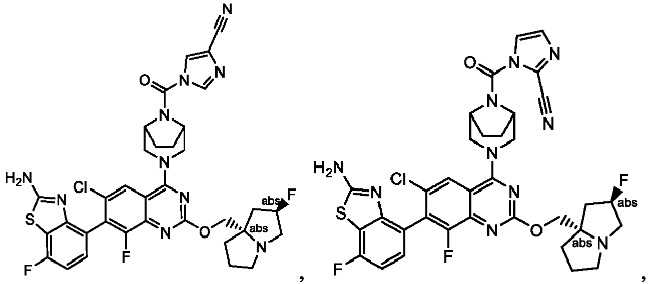 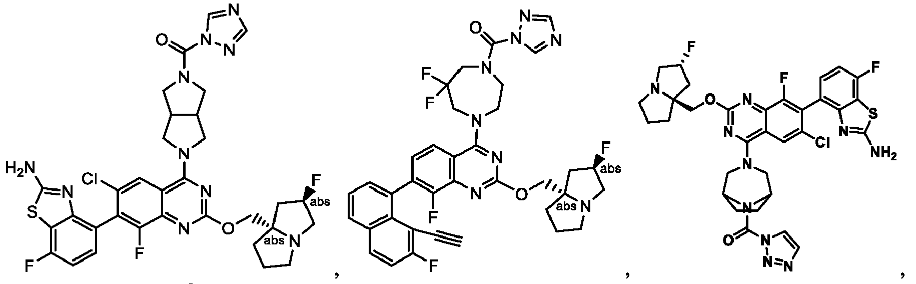 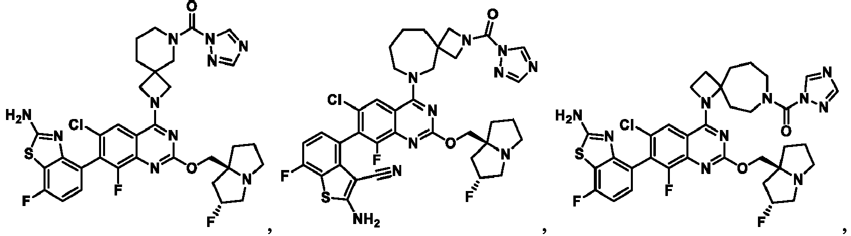 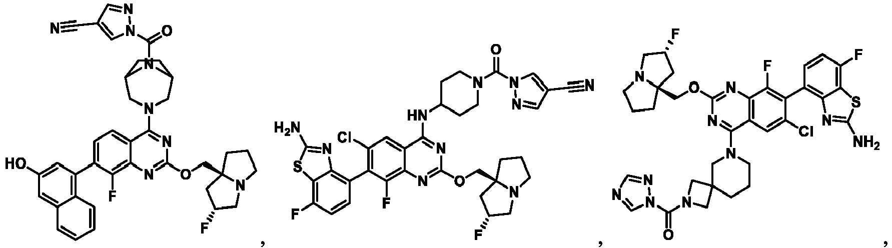 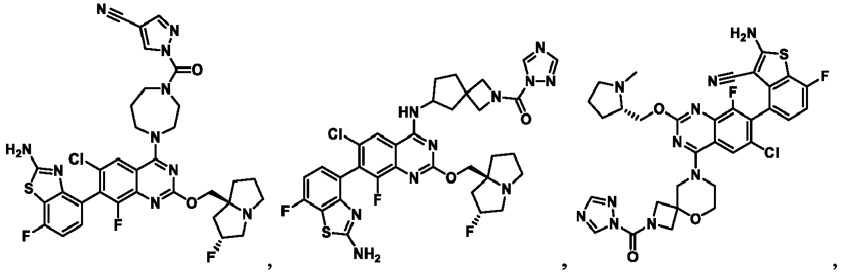 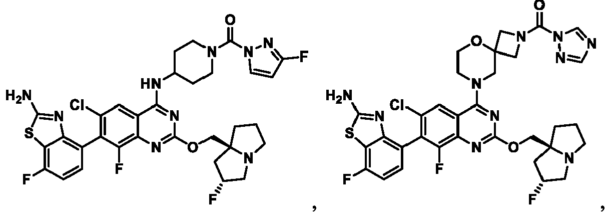 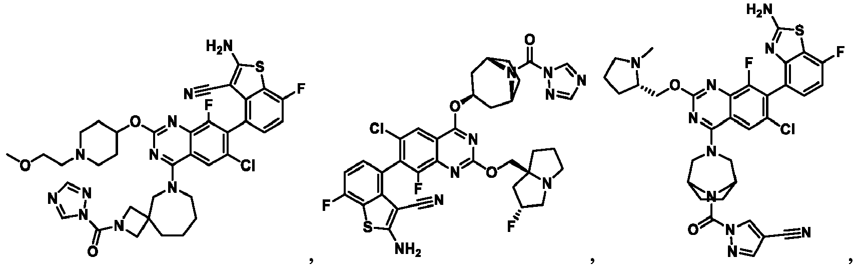 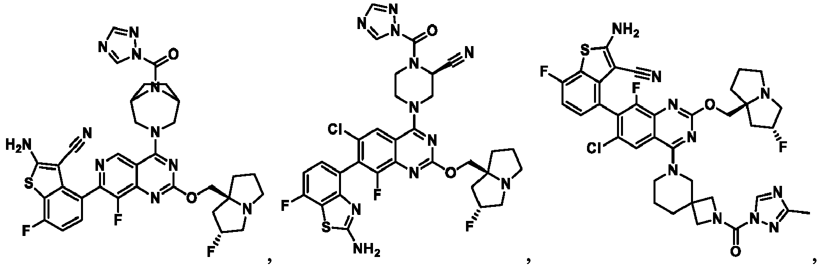 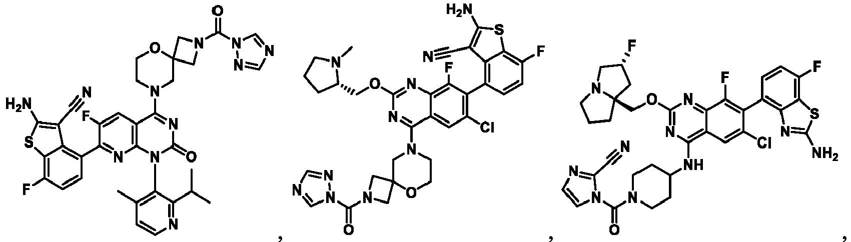 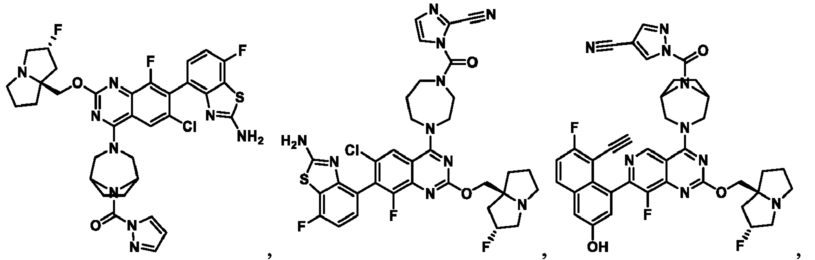 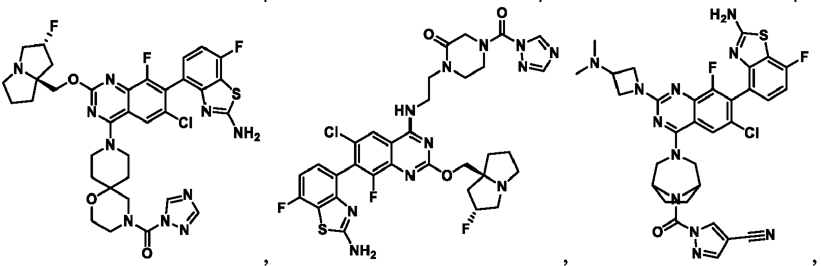 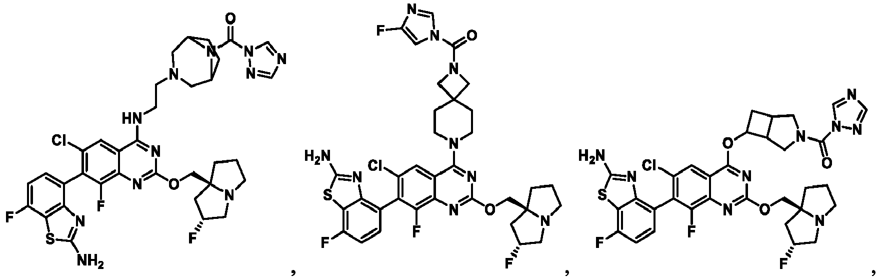 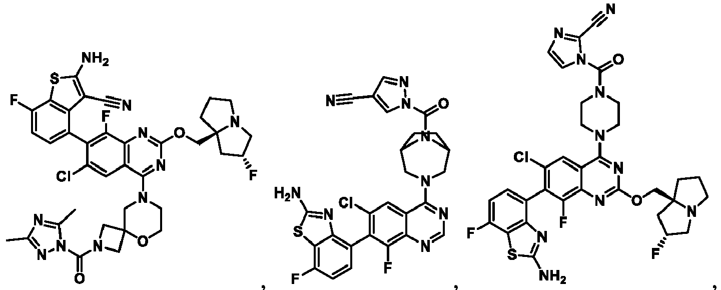 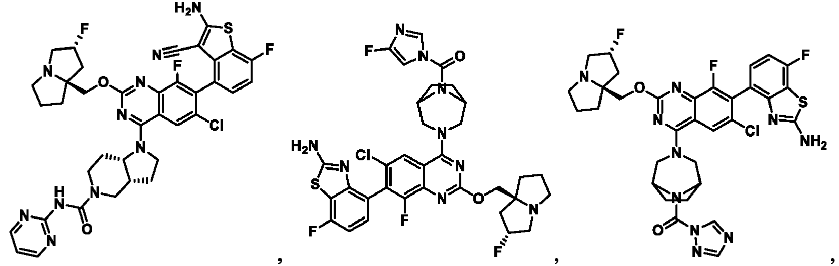 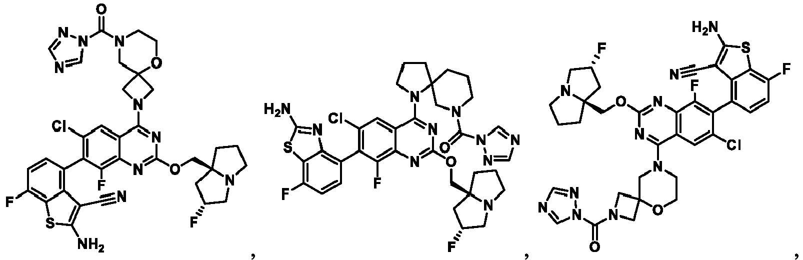 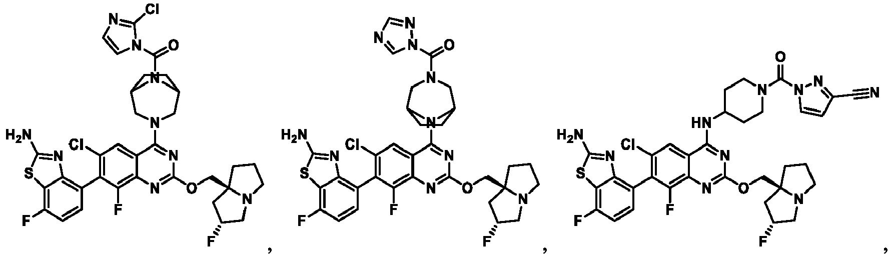 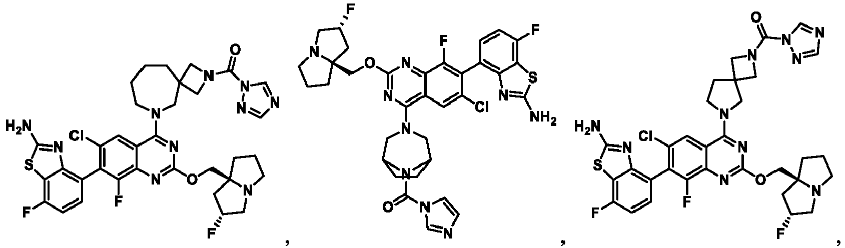 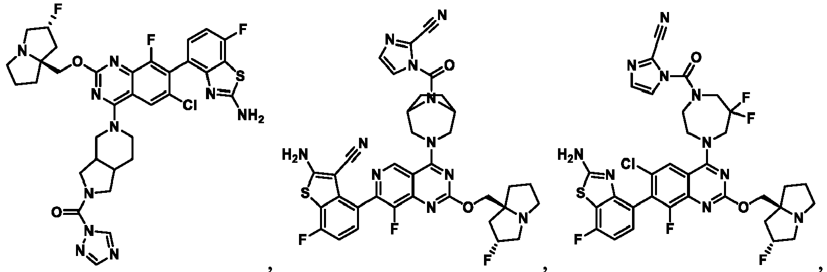 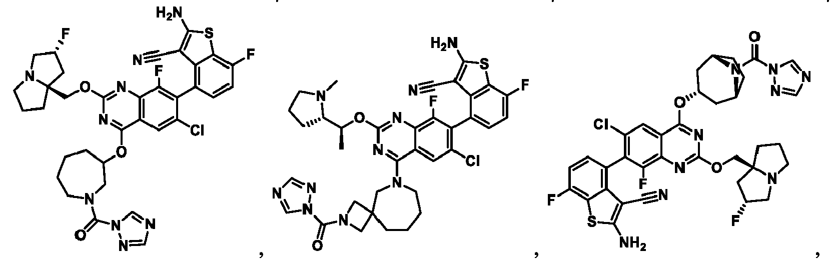 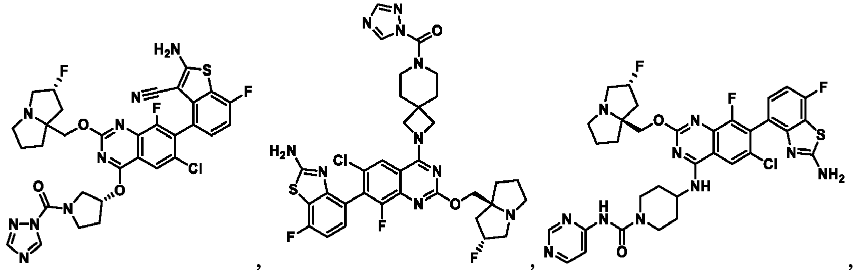 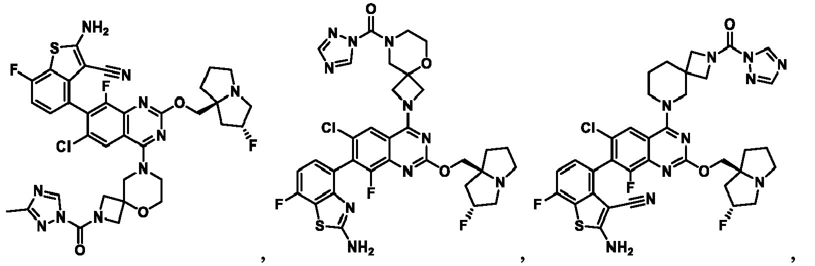 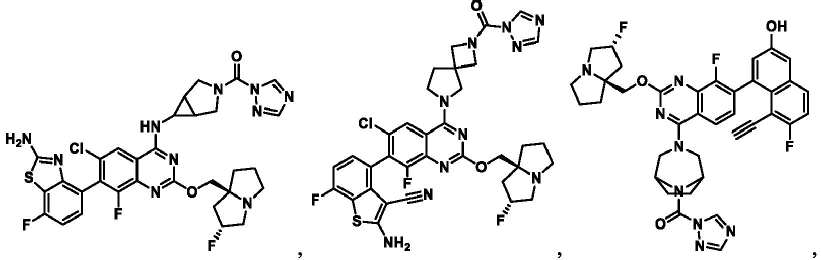 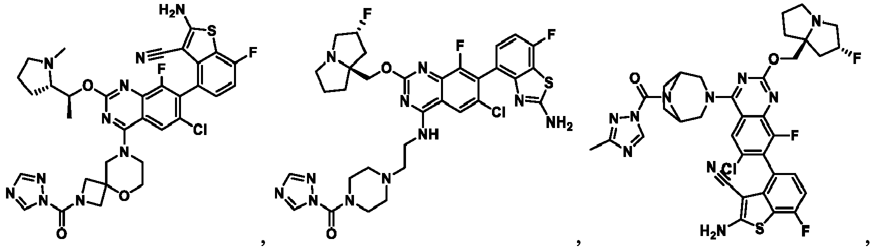 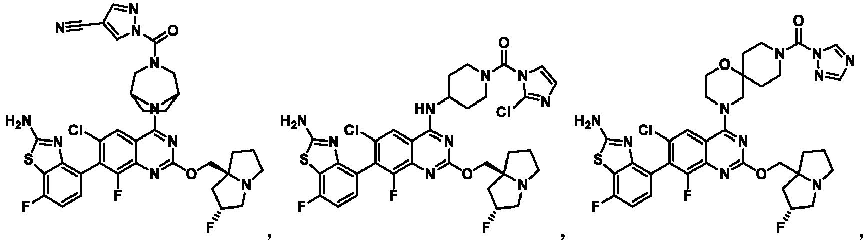 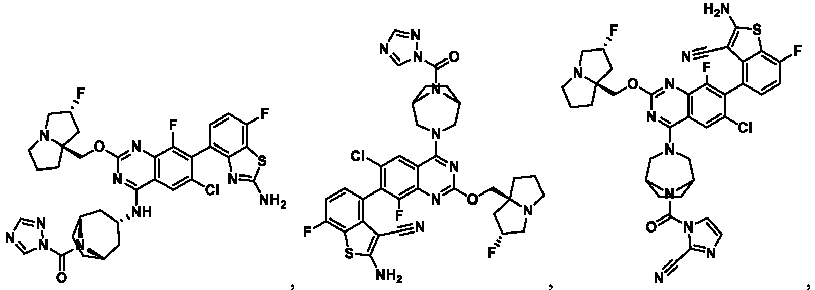 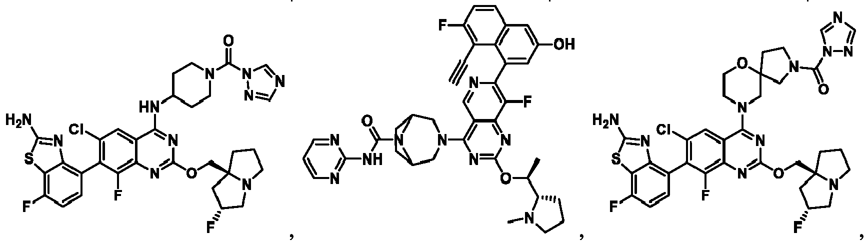 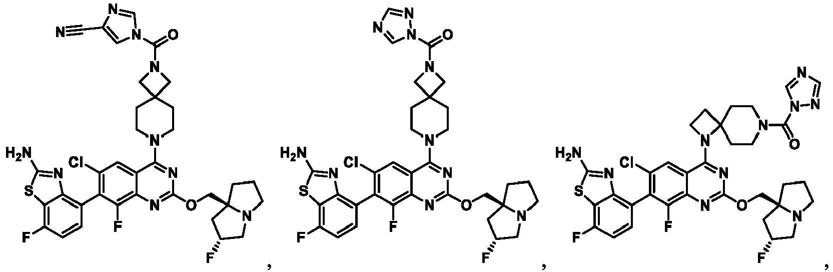 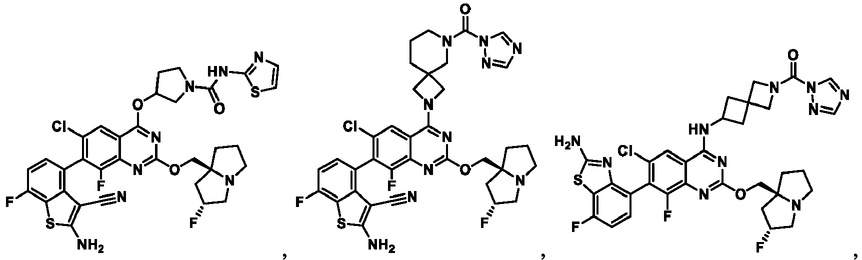 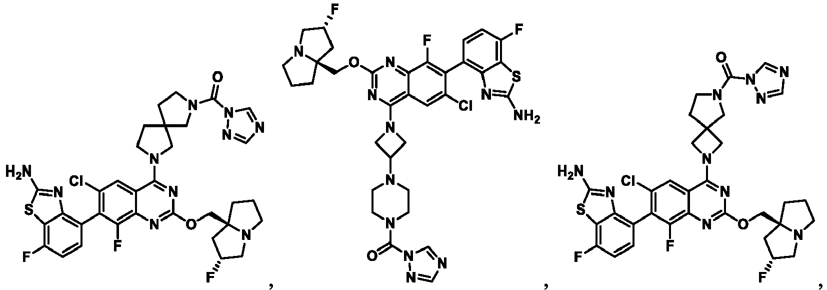 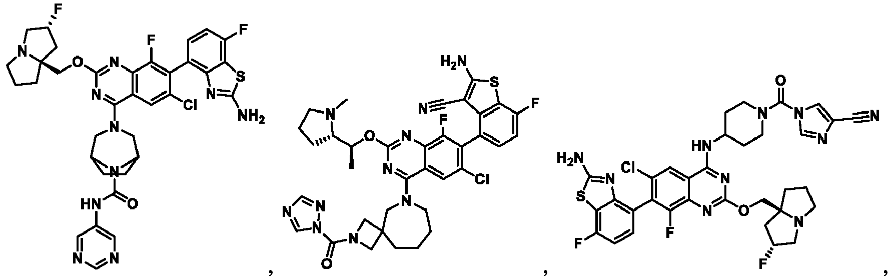 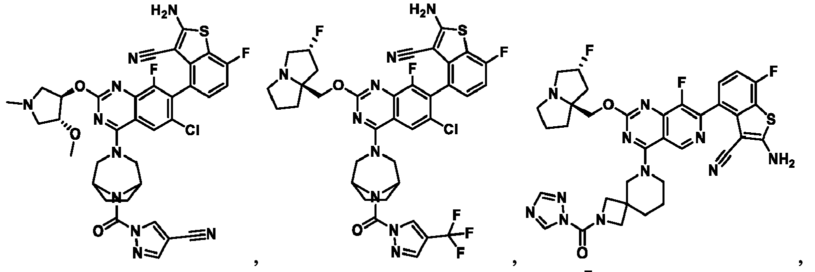 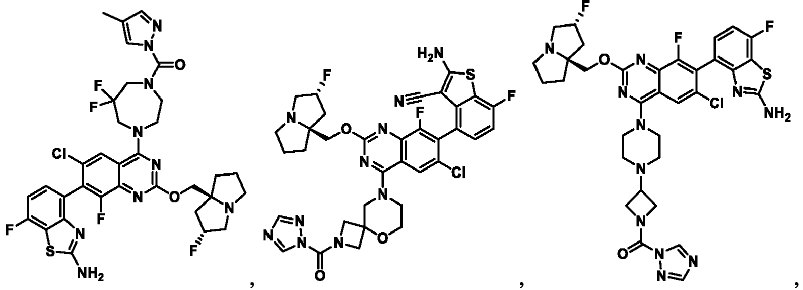 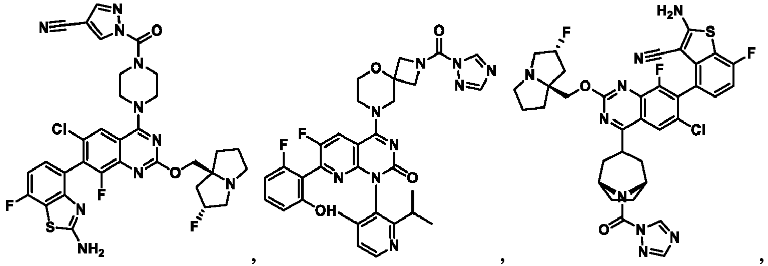 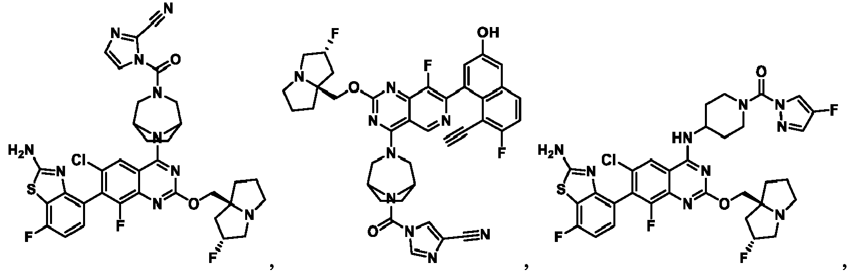 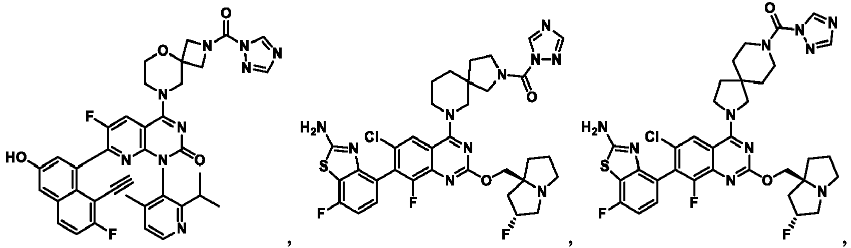 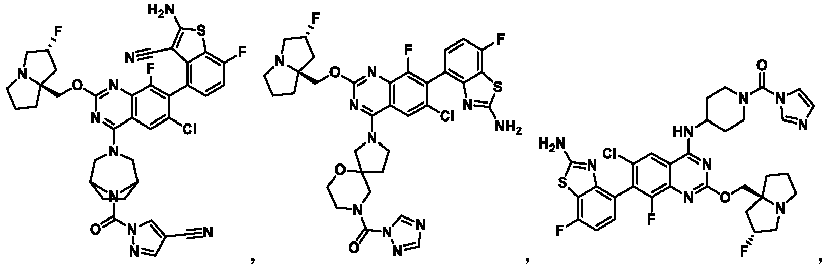 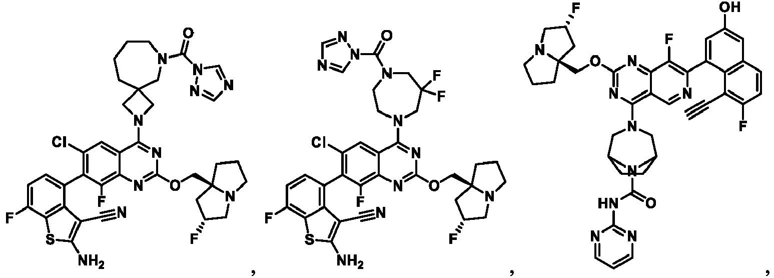 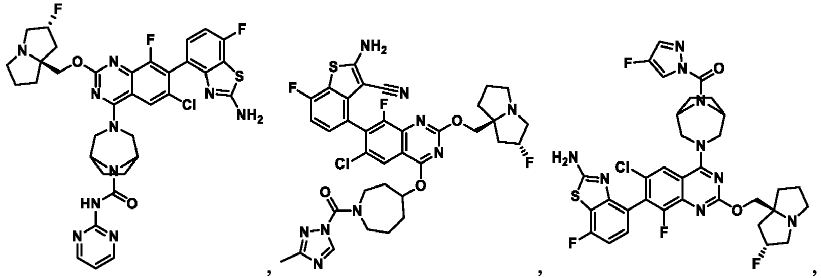 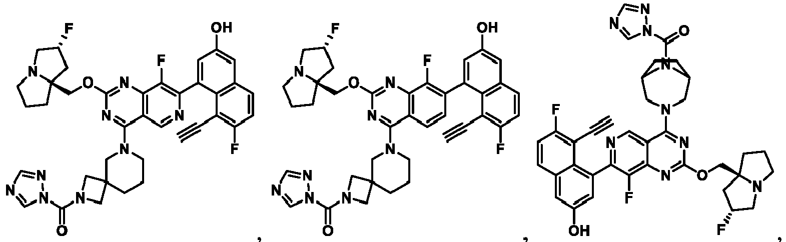 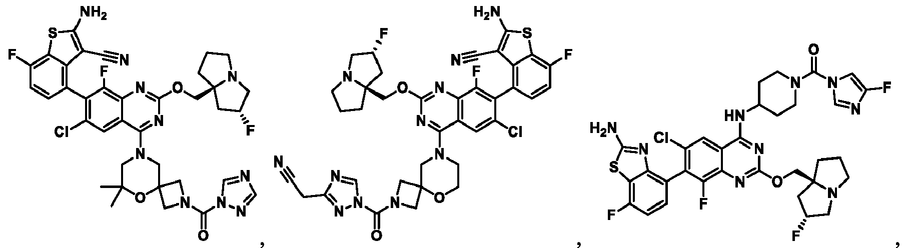 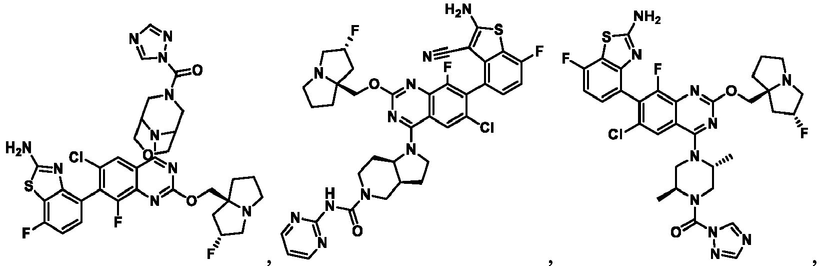 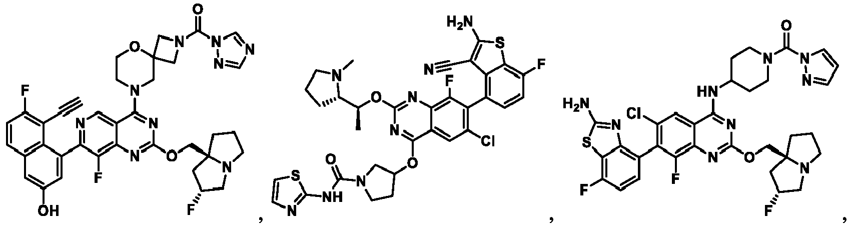 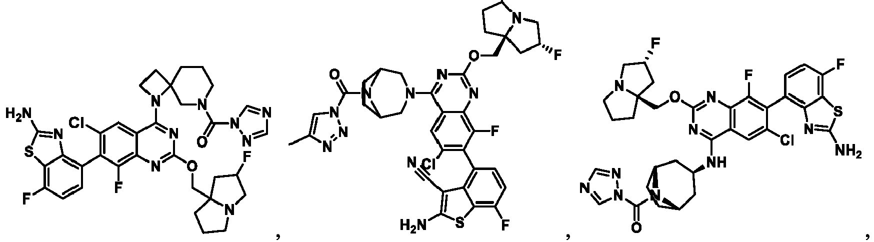 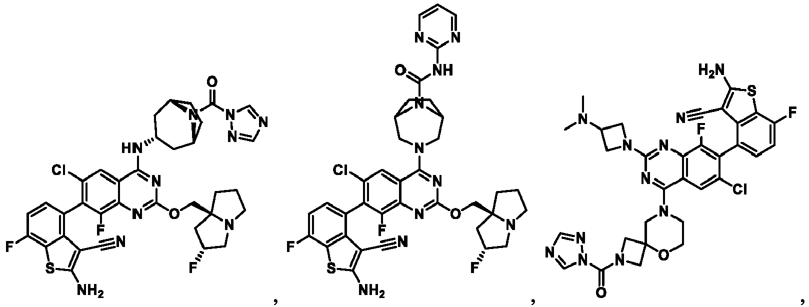 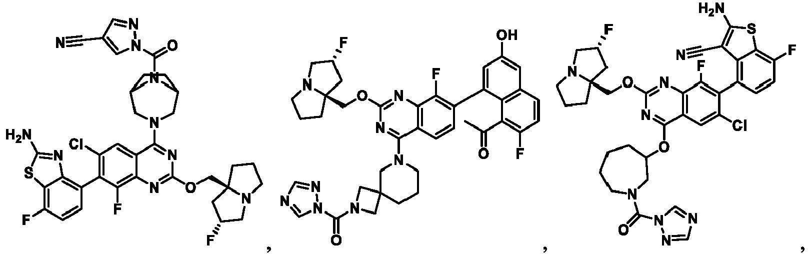 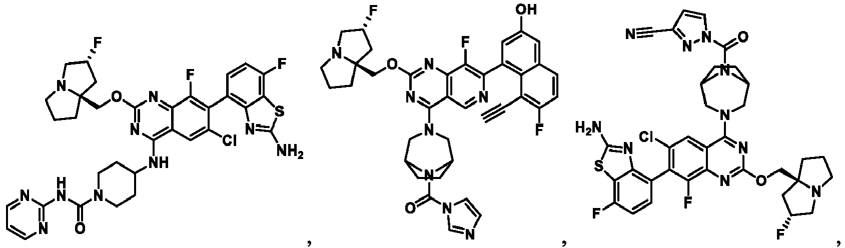 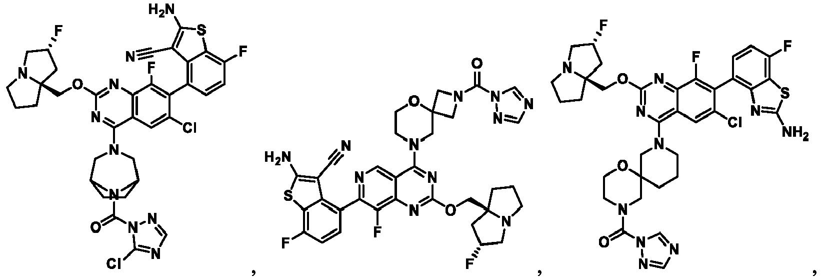 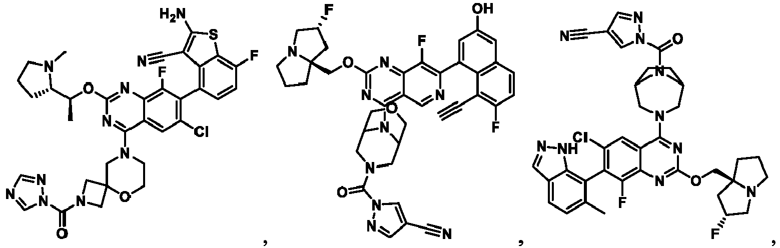 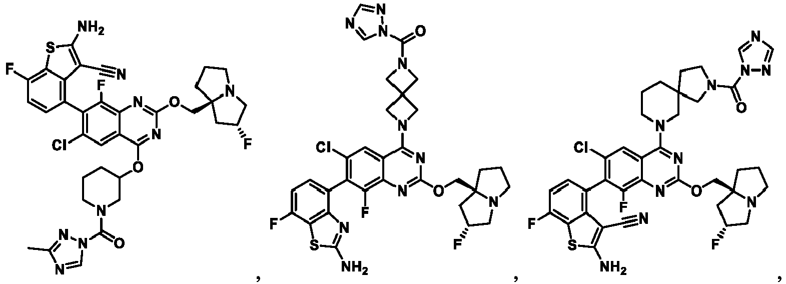 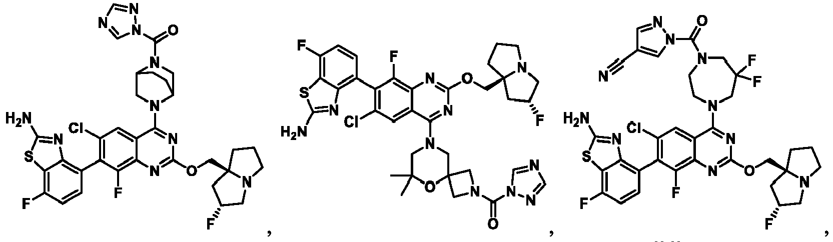 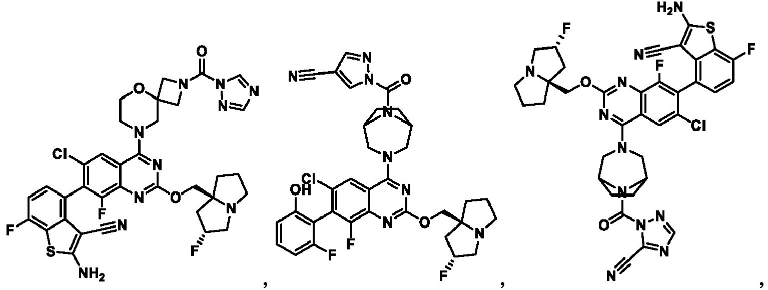 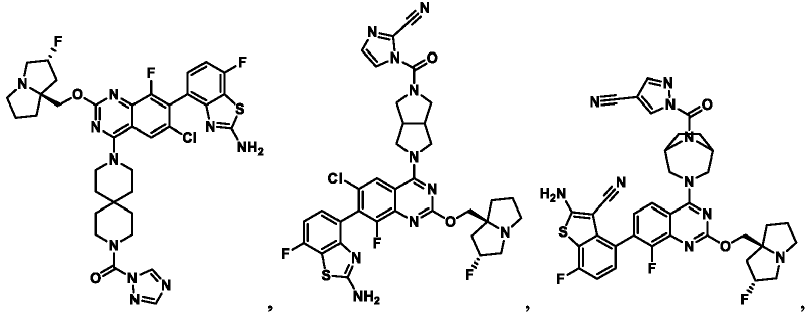 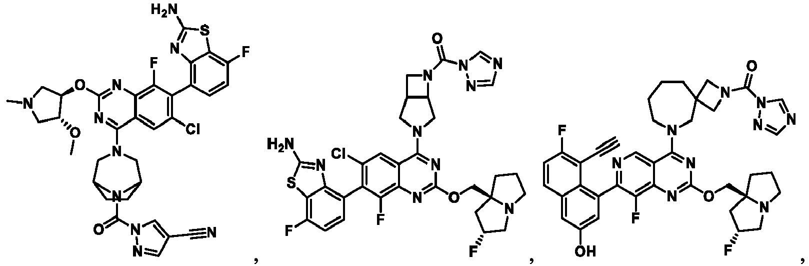 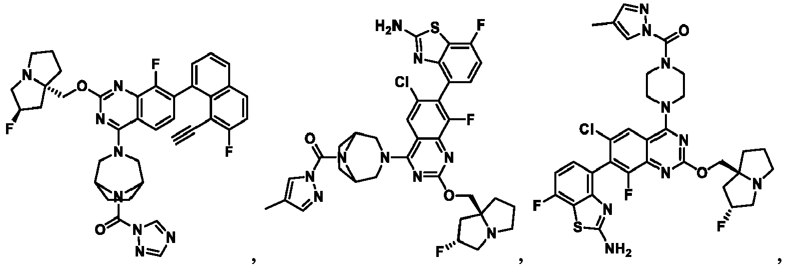 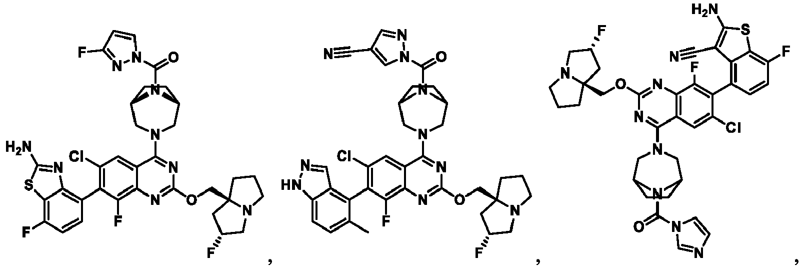 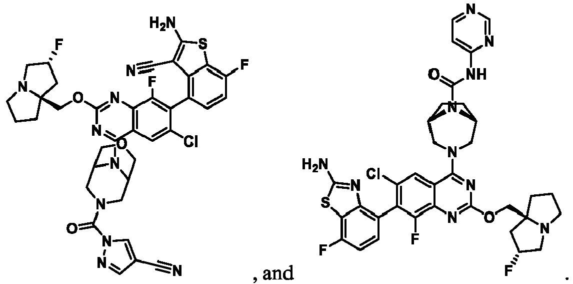 In embodiments, the compound is selected from 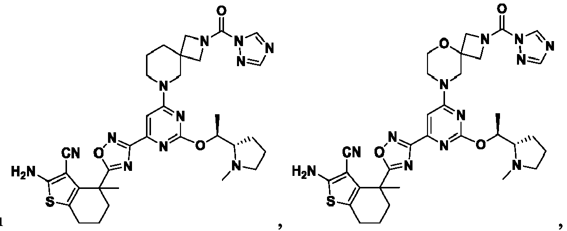 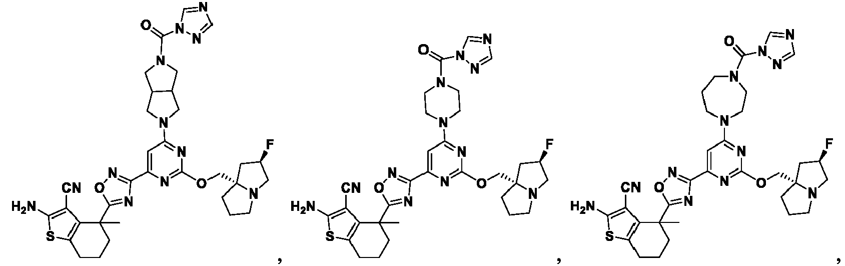 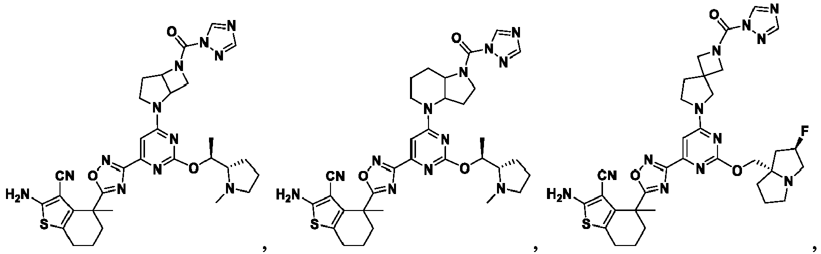 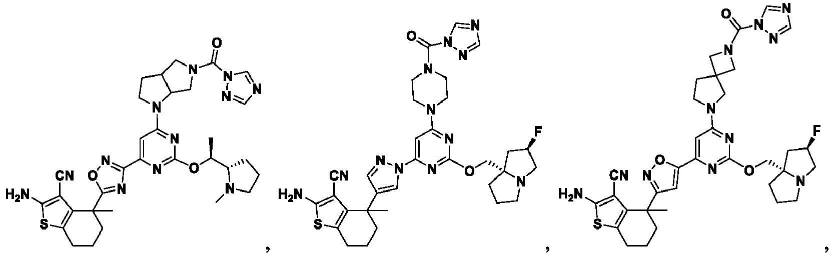 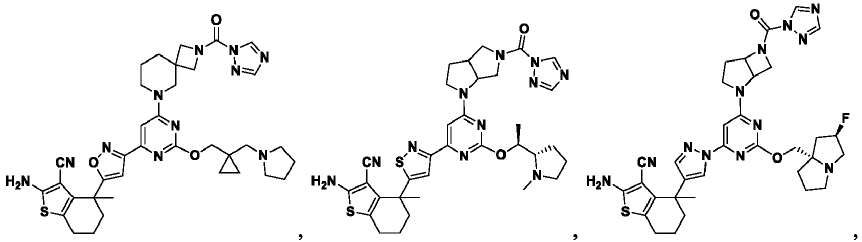 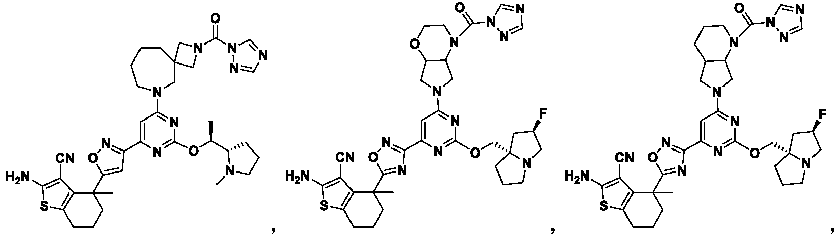 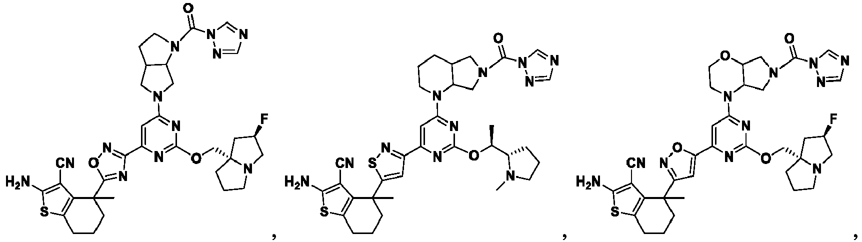 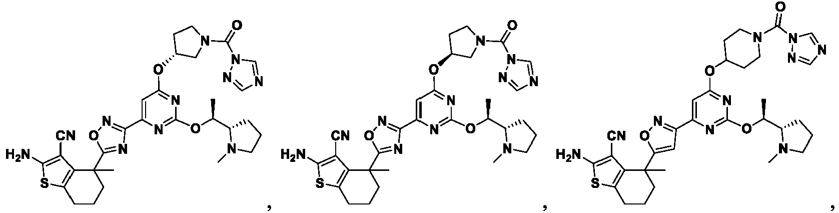 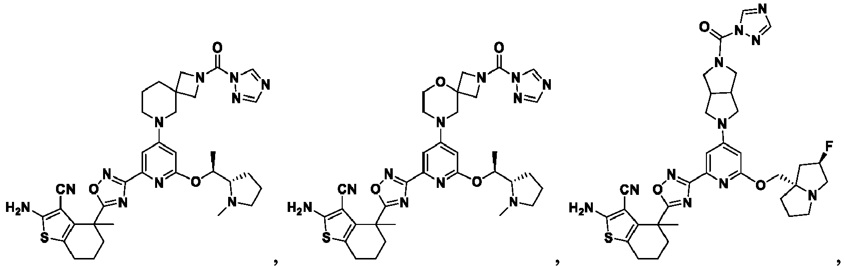 and

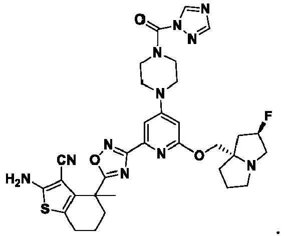

In embodiments, the compound is selected from 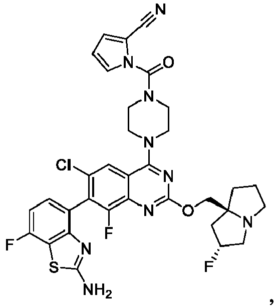 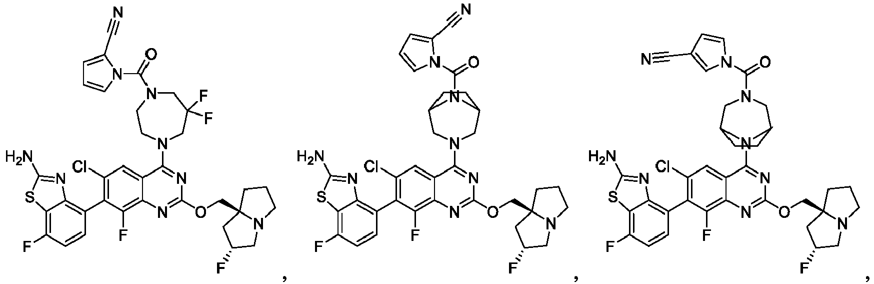 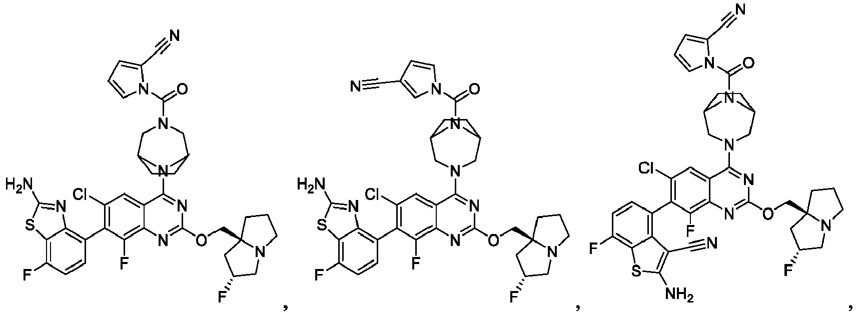 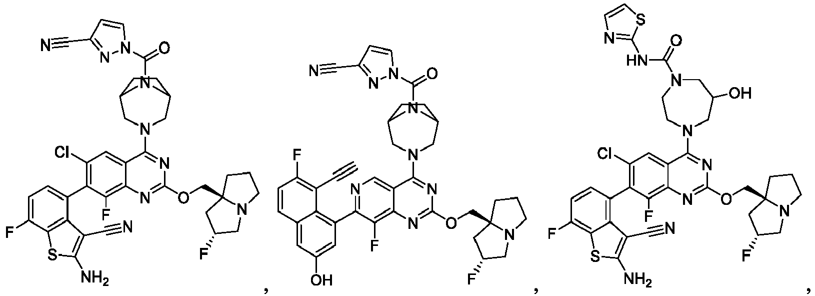 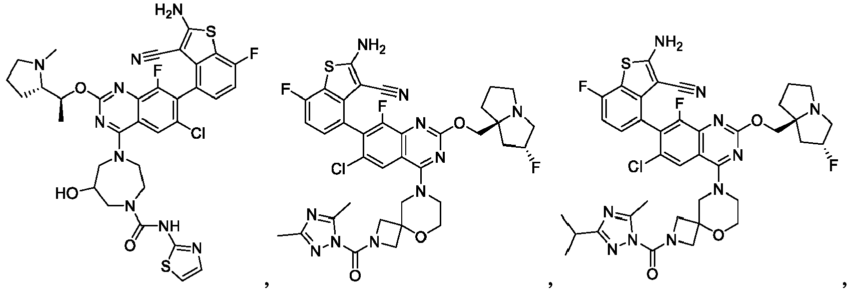 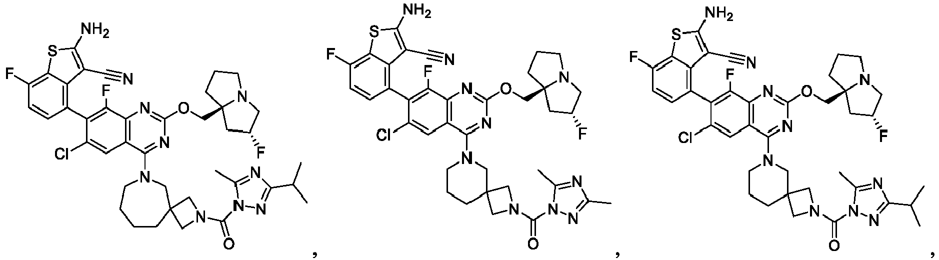 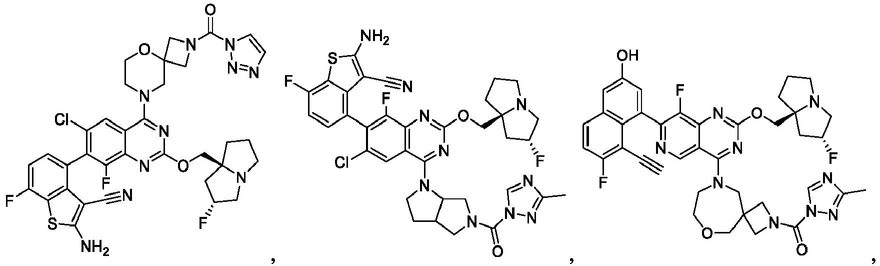 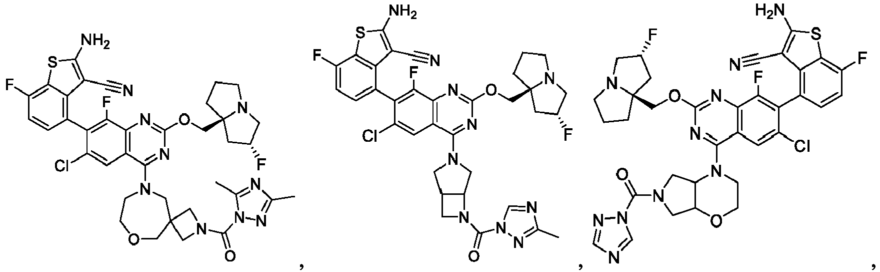 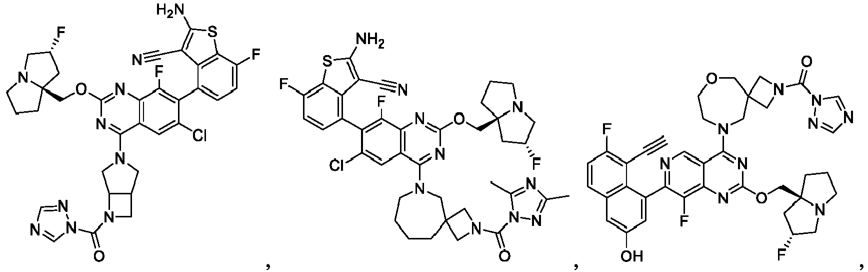 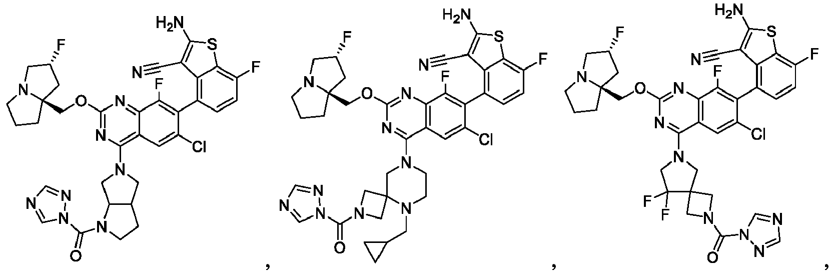 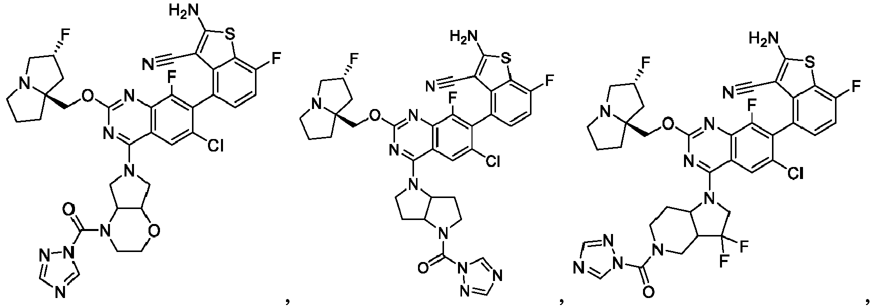 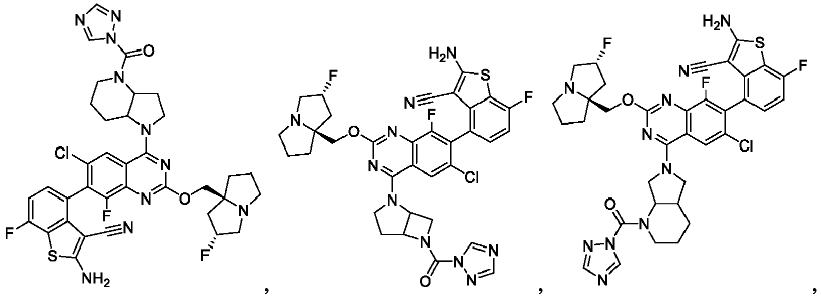 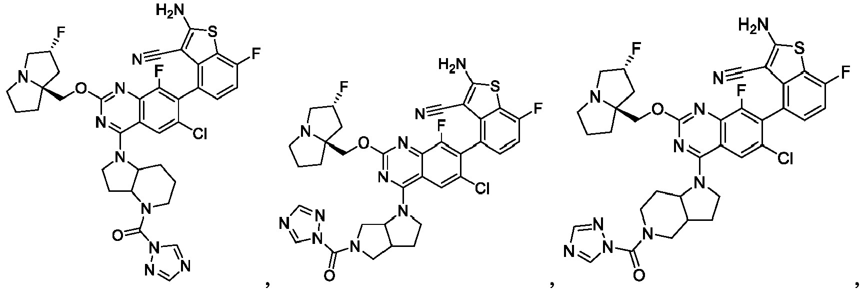 and 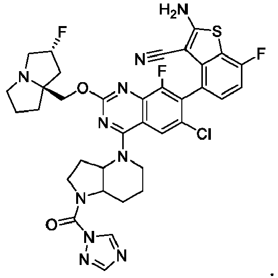 In embodiments, the compound or precursor compound is a compound described herein, for example in a table or example.

In an aspect is provided a compound having the formula A-L^{AB}-B wherein
A is a monovalent form of a precursor compound described herein;
L^{AB} is a covalent linker bonded to A and B; and
B is a monovalent form of a degradation enhancer.

A "degradation enhancer" is a compound capable of binding a ubiquitin ligase protein (e.g., E3 ubiquitin ligase protein) or a compound capable of binding a protein that is capable of binding to a ubiquitin ligase protein to form a protein complex capable of conjugating a ubiquitin protein to a target protein. In embodiments, the degradation enhancer is capable of binding to an E3 ubiquitin ligase protein or a protein complex comprising an E3 ubiquitin ligase protein. In embodiments, the degradation enhancer is capable of binding to an E2 ubiquitin-conjugating enzyme. In embodiments, the degradation enhancer is capable of binding to a protein complex comprising an E2 ubiquitin-conjugating enzyme and an E3 ubiquitin ligase protein.

In embodiments, the degradation enhancer is capable of binding a protein selected from E3A, mdm2, APC, EDD1, SOCS/BC-box/eloBC/CUL5/RING, LNXp80, CBX4, CBLL1, HACE1, HECTD1, HECTD2, HECTD3, HECTD4, HECW1, HECW2, HERC1, HERC2, HERC3, HERC4, HERS, HERC6, HUWE1, ITCH, NEDD4, NEDD4L, PPIL2, PRPF19, PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UBOX5, UBRS, VHL (von-Hippel-Lindau ubiquitin ligase), WWP1, WWP2, Parkin, MKRN1, CMA (chaperon-mediated autophage), SCFb-TRCP (Skip-Cullin-F box (Beta-TRCP) ubiquitin complex), b-TRCP (b-transducing repeat-containing protein), cIAP1 (cellular inhibitor of apoptosis protein 1), APC/C (anaphase-promoting complex/cyclosome), CRBN (cereblon), CUL4-RBX1-DDB1-CRBN (CRL4^{CRBN}) ubiquitin ligase, XIAP, IAP, KEAP1, DCAF15, RNF114, DCAF16, AhR, SOCS2, KLHL12, UBR2, SPOP, KLHL3, KLHL20, KLHDC2, SPSB1, SPSB2, SPSB4, SOCS6, FBXO4, FBXO31, BTRC, FBW7, CDC20, PML, TRIM21, TRIM24, TRIM33, GID4, avadomide, iberdomide, and CC-885.

In embodiments, the degradation enhancer is capable of binding a protein selected from UBE2A, UBE2B, UBE2C, UBE2D1, UBE2D2, UBE2D3, UBE2DR, UBE2E1, UBE2E2, UBE2E3, UBE2F, UBE2G1, UBE2G2, UBE2H, UBE2I, UBE2J1, UBE2J2, UBE2K, UBE2L3, UBE2L6, UBE2L1, UBE2L2, UBE2L4, UBE2M, UBE2N, UBE2O, UBE2Q1, UBE2Q2, UBE2R1, UBE2R2, UBE2S, UBE2T, UBE2U, UBE2V1, UBE2V2, UBE2W, UBE2Z, ATG3, BIRC6, and UFC1.

In embodiments, the degradation enhancer is a compound described in Ishida and Ciulli, SLAS Discovery 2021, Vol. 25(4) 484-502, which is incorporated by reference in its entirety for any purpose, for example VH032, VH101, VH298, thalidomide, bestatin, methyl bestatin, nutlin, idasanutlin, bardoxolone, bardoxolone methyl, indisulam (E7070), E7820, chloroquinoxaline sulfonamide (CQS), nimbolide, KB02, ASTX660, lenalidomide, or pomalidomide.

In embodiments, the degradation enhancer is a compound described in US20180050021, WO2016146985, WO2018189554, WO2018119441, WO2018140809, WO2018119448, WO2018119357, WO2018118598, WO2018102067, WO201898280, WO201889736, WO201881530, WO201871606, WO201864589, WO201852949, WO2017223452, WO2017204445, WO2017197055, WO2017197046, WO2017180417, WO2017176958, WO201711371, WO2018226542, WO2018223909, WO2018189554, WO2016169989, WO2016146985, CN105085620B, CN106543185B, US10040804, US9938302, US10144745, US10145848, US9938264, US9632089, US9821068, US9758522, US9500653, US9765019, US8507488, US8299057, US20180298027, US20180215731, US20170065719, US20170037004, US20160272639, US20150291562, or US20140356322, which are incorporated by reference in their entirety for any purpose.

In embodiments L^{AB} is -L^{AB1}-L^{AB2}-L^{AB3}-L^{AB4}-L^{AB5}-;
L^{AB1}, L^{AB2}, L^{AB3}, L^{AB4}, and L^{AB5} are independently a bond, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, C₁₋₆alkylene, (-O-C₁₋₆alkyl)_{z}-, (-C₁₋₆alkyl-O)_{z}-, C₂₋₆alkenylene, C₂₋₆alkynylene, C₁₋₆haloalkylene, C₃₋₁₂cycloalkylene, C_{1- 11}heterocycloalkylene, C₆₋₁₂arylene, or C₁₋₁₁heteroarylene, wherein C₁₋₆alkylene, C₂₋₆alkenylene, C_{2- 6}alkynylene, C₁₋₆haloalkylene, C₃₋₁₂cycloalkylene, C₁₋₁₁heterocycloalkylene, C₆₋₁₂arylene, or C_{1- 11}heteroarylene,are optionally substituted with one, two, or three R^{20j}; wherein each C₁₋₆alkyl of (-O-C_{1- 6}alkyl)_{z}- and (-C₁₋₆alkyl-O)_{z}- is optionally substituted with one, two, or three R^{20j};
z is independently an integer from 0 to 10;
each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
each R^{20d}, R^{20e}, R^{20f}, and R^{20j} are each independently selected from halogen, -CN, C₁₋₆alkyl, C₂₋₆alkenyl, C_{2- 6}alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, - CH₂-C₆₋₁₀aryl, -CH₂-C₁₋₉heteroaryl, C₁₋₉heteroaryl, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂₍R²²)(R²³), -OCH₂C(O)OR²², and-OC(O)R²⁵, wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², - C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)OR²⁵, -N(R²⁴)S(O)₂R²⁵, -C(O)R²⁵, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from Hand C₁₋₆alkyl;
each R²⁴ is independently selected from Hand C₁₋₆alkyl; and
each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl.

In embodiments, L^{AB} is -(O-C₂alkyl)_{z}- and z is an integer from 1 to 10.

In embodiments, L^{AB} is -(C₂alkyl-O-)_{z}- and z is an integer from 1 to 10.

In embodiments, L^{AB} is -(CH₂)_{zz1}L^{AB2}(CH₂O)_{zz2}-, wherein L^{AB2} is a bond, a 5 or 6 membered heterocycloalkylene or heteroarylene, phenylene, -(C₂-C₄)alkynylene, -SO₂- or -NH-; and zz1 and zz2 are independently an integer from 0 to 10.

In embodiments, L^{AB} is -(CH₂)_{zz1}(CH₂O)_{zz2}-, wherein zz1 and zz2 are each independently an integer from 0 to 10.

In embodiments, L^{AB} is a PEG linker (e.g., divalent linker of 1 to 10 ethylene glycol subunits).

In embodiments, B is a monovalent form of a compound selected from 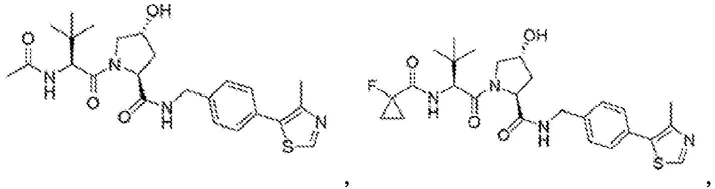 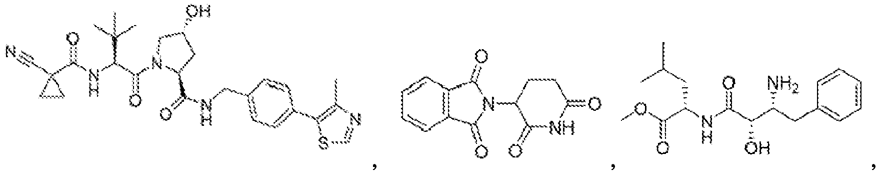 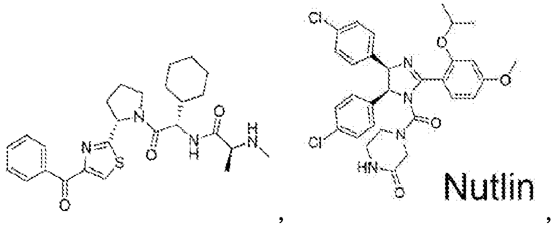 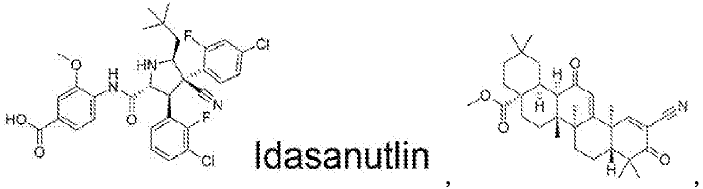 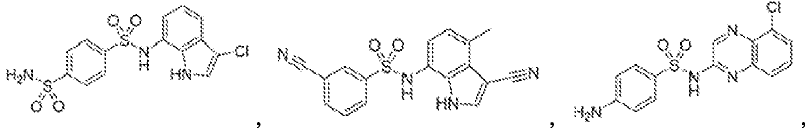 and

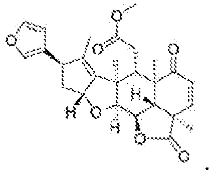

### Further Forms of Compounds Disclosed Herein

### Isomers

Furthermore, in some embodiments, the compounds described herein exist as geometric isomers. In some embodiments, the compounds described herein possess one or more double bonds. The compounds presented herein include all cis, trans, syn, anti, entgegen (*E*), and zusammen (*Z*) isomers as well as the corresponding mixtures thereof. In some situations, compounds exist as tautomers. The compounds described herein include all possible tautomers within the formulas described herein. In some situations, the compounds described herein possess one or more chiral centers and each center exists in the R configuration or S configuration. The compounds described herein include all diastereomeric, enantiomeric, and epimeric forms as well as the corresponding mixtures thereof. The skilled person would understand that stereoisomers include conformational stereoisomers, such as atropisomers. In additional embodiments of the compounds and methods provided herein, mixtures of enantiomers and/or diastereoisomers, resulting from a single preparative step, combination, or interconversion, are useful for the applications described herein. In some embodiments, the compounds described herein are prepared as optically pure enantiomers by chiral chromatographic resolution of the racemic mixture. In some embodiments, the compounds described herein are prepared as their individual stereoisomers by reacting a racemic mixture of the compound with an optically active resolving agent to form a pair of diastereoisomeric compounds, separating the diastereomers, and recovering the optically pure enantiomers. In some embodiments, dissociable complexes are preferred (e.g., crystalline diastereomeric salts). In some embodiments, the diastereomers have distinct physical properties (e.g., melting points, boiling points, solubilities, reactivity, etc.) and are separated by taking advantage of these dissimilarities. In some embodiments, the diastereomers are separated by chiral chromatography, or preferably, by separation/resolution techniques based upon differences in solubility. In some embodiments, the optically pure enantiomer is then recovered, along with the resolving agent, by any practical means that does not result in racemization.

The term "atropisomers" refers to conformational stereoisomers which occur when rotation about a single bond in the molecule is prevented, restricted, or greatly slowed as a result of steric interactions with other parts of the molecule and wherein the substituents at both ends of the single bond are asymmetrical (i.e., optical activity arises without requiring an asymmetric carbon center or stereocenter). Where the rotational barrier about the single bond is high enough, and interconversion between conformations is slow enough, separation and isolation of the isomeric species may be permitted. Atropisomers are enantiomers (or epimers) without a single asymmetric atom. Atropisomers are typically considered stable if the barrier to interconversion is high enough to permit the atropisomers to undergo little or no interconversion at room temperature for a least a week, preferably at least a year. In some embodiments, an atropisomeric compound of the disclosure does not undergo more than about 5% interconversion to its opposite atropisomer at room temperature during one week when the atropisomeric compound is in substantially pure form, which is generally a solid state. In some embodiments, an atropisomeric compound of the disclosure does not undergo more than about 5% interconversion to its opposite atropisomer at room temperature (approximately 25 °C) during one year. The present chemical entities, pharmaceutical compositions, and methods may include optically pure forms of single atropisomers or mixtures of atropisomers, such as racemic mixtures, diastereomeric mixtures, epimeric mixtures, and intermediate mixtures thereof.

In some embodiments, a compound of formula (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In'), such as a compound of Formula (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), or (I-1n),, is provided as a substantially pure stereoisomer. In some embodiments, the stereoisomer is provided in at least 80% enantiomeric excess, such as at least 85%, at least 90%, at least 95%, at least 96%, at least 97%, at least 98%, at least 99%, or at least 99.9% enantiomeric excess.

In some embodiments, the present disclosure provides an atropisomer as a stereoisomer of a compound of formula (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In'). In some embodiments, the atropisomer is provided in enantiomeric excess. In some embodiments, the atropisomer is provided in at least 80% enantiomeric excess, such as at least 85%, at least 90%, at least 95%, at least 96%, at least 97%, at least 98%, at least 99%, or at least 99.9% enantiomeric excess. In some embodiments, the compound of formula (Ib), (Id), (Ie), (Ib-1), (Ib-2). (Ib-3), (Ib-4), (Id-1), (Id-3), (Ie-1), (Ie-3), (I-1), (I-2), (I-1a), (I-1b), (I-1d), (I-1e), (I-1f), ((I-1g), (I-1h), (I-1i), (I-1k), (I-1m), (I-1n), (Ic), (If), (Ih), (Ii), (Ij), (Ik), (If-1), (Ih-1), (Ii-1), (Ij-1), (Ik-1), (Ih-3), (Ii-3), (Ij-3), (Ik-3), (I-3), or (In')is preferably used as a non-racemic mixture, wherein one atropisomer is present in excess of its corresponding enantiomer or epimer. Typically, such mixture will contain a mixture of the two isomers in a ratio of at least 9:1, preferably at least 19:1. In some embodiments, the atropisomer is provided in at least 96% enantiomeric excess, meaning the compound has less than 2% of the corresponding enantiomer. In some embodiments, the atropisomer is provided in at least 96% diastereomeric excess, meaning the compound has less than 2% of the corresponding diastereomer.

### Labeled compounds

In some embodiments, the compounds described herein exist in their isotopically-labeled forms. In some embodiments, the methods disclosed herein include methods of treating diseases by administering such isotopically-labeled compounds. In some embodiments, the methods disclosed herein include methods of treating diseases by administering such isotopically-labeled compounds as pharmaceutical compositions. Thus, in some embodiments, the compounds disclosed herein include isotopically-labeled compounds, which are identical to those recited herein, but for the fact that one or more atoms are replaced by an atom having an atomic mass or mass number different from the atomic mass or mass number usually found in nature. Examples of isotopes that are incorporated into compounds described herein include isotopes of hydrogen, carbon, nitrogen, oxygen, phosphorous, sulfur, fluorine, and chloride, such as ²H, ³H, ¹³C, ¹⁴C, ¹⁵N, ¹⁷O, ¹⁸O, ³¹P, ³²P, ³⁵S, ¹⁸F, and ³⁶Cl, respectively. Compounds described herein, and pharmaceutically acceptable salts, esters, solvate, hydrates, or derivatives thereof which contain the aforementioned isotopes and/or other isotopes of other atoms are within the scope of this invention. Certain isotopically-labeled compounds, for example those into which radioactive isotopes such as ³H and ¹⁴C are incorporated, are useful in drug and/or substrate tissue distribution assays. Tritiated, i. e., ³H and carbon-14, *i.e.,* ¹⁴C, isotopes are particularly preferred for their ease of preparation and detectability. Further, substitution with heavy isotopes such as deuterium, *i.e*., ²H, produces certain therapeutic advantages resulting from greater metabolic stability, for example increased *in vivo* half-life or reduced dosage requirements. In some embodiments, the isotopically labeled compounds, pharmaceutically acceptable salt, ester, solvate, hydrate, or derivative thereof is prepared by any suitable method.

In some embodiments, the compounds described herein are labeled by other means, including, but not limited to, the use of chromophores or fluorescent moieties, bioluminescent labels, or chemiluminescent labels.

### Pharmaceutically acceptable salts

In some embodiments, the compounds described herein exist as their pharmaceutically acceptable salts. In some embodiments, the methods disclosed herein include methods of treating diseases by administering such pharmaceutically acceptable salts. In some embodiments, the methods disclosed herein include methods of treating diseases by administering such pharmaceutically acceptable salts as pharmaceutical compositions.

In some embodiments, the compounds described herein possess acidic or basic groups and therefore react with any of a number of inorganic or organic bases, and inorganic and organic acids, to form a pharmaceutically acceptable salt. In some embodiments, these salts are prepared *in situ* during the final isolation and purification of the compounds described herein, or by separately reacting a purified compound in its free form with a suitable acid or base, and isolating the salt thus formed.

### Solvates

In some embodiments, the compounds described herein exist as solvates. In some embodiments are methods of treating diseases by administering such solvates. Further described herein are methods of treating diseases by administering such solvates as pharmaceutical compositions.

Solvates contain either stoichiometric or non-stoichiometric amounts of a solvent, and, in some embodiments, are formed during the process of crystallization with pharmaceutically acceptable solvents such as water, ethanol, and the like. Hydrates are formed when the solvent is water, or alcoholates are formed when the solvent is alcohol. Solvates of the compounds described herein are conveniently prepared or formed during the processes described herein. By way of example only, hydrates of the compounds described herein are conveniently prepared by recrystallization from an aqueous/organic solvent mixture, using organic solvents including, but not limited to, dioxane, tetrahydrofuran, or MeOH. In addition, the compounds provided herein exist in unsolvated as well as solvated forms. In general, the solvated forms are considered equivalent to the unsolvated forms for the purposes of the compounds and methods provided herein.

### Synthesis of Compounds

In some embodiments, the synthesis of compounds described herein are accomplished using means described in the chemical literature, using the methods described herein, or by a combination thereof. In addition, solvents, temperatures and other reaction conditions presented herein may vary.

In other embodiments, the starting materials and reagents used for the synthesis of the compounds described herein are synthesized or are obtained from commercial sources, such as, but not limited to, Sigma-Aldrich, FischerScientific (Fischer Chemicals), and AcrosOrganics.

In further embodiments, the compounds described herein, and other related compounds having different substituents are synthesized using techniques and materials described herein as well as those that are recognized in the field, such as described, for example, in Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), Larock's Comprehensive Organic Transformations (VCH Publishers Inc., 1989), March, Advanced Organic Chemistry 4th Ed., (Wiley 1992); Carey and Sundberg, Advanced Organic Chemistry 4th Ed., Vols. A and B (Plenum 2000, 2001), and Green and Wuts, Protective Groups in Organic Synthesis 3rd Ed., (Wiley 1999) (all of which are incorporated by reference for such disclosure). General methods for the preparation of compound as disclosed herein may be derived from reactions and the reactions may be modified by the use of appropriate reagents and conditions, for the introduction of the various moieties found in the formulae as provided herein. In some embodiments, the following synthetic method may be utilized.

In some embodiments, the compounds of the present invention exhibit one or more functional characteristics disclosed herein. For example, a subject compound binds to a Ras protein, Kras protein or a mutant form thereof. In some embodiments, a subject compound binds specifically and also inhibits a Ras protein, Kras protein or a mutant form thereof. In some embodiments, a subject compound selectively inhibits a Kras mutant relative to a wildtype Kras. In some embodiments, a subject compound selectively inhibits KrasG12D and/or KrasG12V relative to wildtype Kras. In some embodiments, the IC50 of a subject compound for a Kras mutant (e.g., including G12D) is less than about 5 µM, less than about 1 µM, less than about 50n nM, less than about 10 nM, less than about 1 nM, less than about 0.5 nM, less than about 100 pM, or less than about 50 pM, as measured in an in vitro assay known in the art or exemplified herein.

In some embodiments, a subject compound of the present disclosure is capable of reducing Ras signaling output. Such reduction can be evidenced by one or more members of the following: (i) an increase in steady state level of GDP-bound Ras protein; (ii) a reduction of phosphorylated AKTs473, (iii) a reduction of phosphorylated ERKT202/y204, (iv) a reduction of phosphorylated S6S235/236, and (v) reduction (e.g., inhibition) of cell growth of Ras-driven tumor cells (e.g., those derived from a tumor cell line disclosed herein). In some cases, the reduction in Ras signaling output can be evidenced by two, three, four or all of (i)-(v) above.

It shall be understood that different aspects of the invention can be appreciated individually, collectively, or in combination with each other. Various aspects of the invention described herein may be applied to any of the particular applications disclosed herein. The compositions of matter including compounds of any formulae disclosed herein in the composition section of the present disclosure may be utilized in the method section including methods of use and production disclosed herein, or vice versa.

### Methods

The compounds described herein, or a pharmaceutically acceptable salt or solvate thereof, are Ras modulators (including Ras inhibitors) capable of covalently modifying a Ras protein. Ras proteins being modified can be Ras G12S mutants from K-Ras, H-Ras or N-Ras. The compounds, a pharmaceutically acceptable salt or solvate thereof disclosed herein, have a wide range of applications in therapeutics, diagnostics, and other biomedical research.

In an aspect is provided a method of treating cancer in a subject in need thereof, comprising administering to the subject a therapeutically effective amount of a precursor compound described herein, or a pharmaceutically acceptable salt or solvate thereof.

In an aspect is provided a method of treating cancer in a subject comprising a a Ras G12S mutant protein, comprising modifying the Ras G12S mutant protein of said subject by administering to said subject a precursor compound, wherein precursor compound is characterized in that upon contacting the Ras G12S mutant protein, said the Ras G12S mutant protein is modified covalently at a serine residue corresponding to reside 12 of SEQ ID No: 1, such that said modified K-Ras G12S protein exhibits reduced Ras signaling output (e.g., compared to a corresponding unmodified Ras protein unbound to the covalent compound).

In an aspect is provided a method of modulating activity of a Ras protein (e.g., K-Ras, mutant K-Ras, K-Ras G12S), comprising contacting a Ras protein with an effective amount of a precursor compound described herein, or a pharmaceutically acceptable salt or solvate thereof, thereby modulating the activity of the Ras protein.

In an aspect is provided a method of inhibiting cell growth, comprising administering an effective amount of a precursor compound described herein, or a pharmaceutically acceptable salt or solvate thereof, to a cell expressing a Ras (e.g., K-Ras) protein, thereby inhibiting growth of said cells. In embodiments, the subject method comprises administering an additional agent to said cell.

In embodiments, the cancer is a solid tumor.

In embodiments, the cancer is a hematological cancer.

In practicing any of the methods disclosed herein, the Ras target to which a subject precursor compound binds covalently can be a Ras G12S mutant, including K-Ras G12S, H-Ras G12S, and N-Ras G12S. In some embodiments, the methods of treating cancer can be applied to treata solid tumor or a hematological cancer. In some embodiments, the cancer being treated can be, without limitation, prostate cancer, brain cancer, colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers. In some embodiments is a method of treating cancer in a subject in need thereof, comprising administering to the subject a therapeutically effective amount of a precursor compound described herein, or a pharmaceutically acceptable salt or solvate thereof, wherein the cancer is a hematological cancer. In some embodiments is a method of treating cancer in a subject in need thereof, comprising administering to the subject a therapeutically effective amount of a precursor compound described herein, or a pharmaceutically acceptable salt or solvate thereof, wherein the cancer is a hematological cancer selected from one or more of chronic lymphocytic leukemia (CLL), acute leukemias, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, and pre-leukemia. In some embodiments is a method of treating cancer in a subject in need thereof, comprising administering to the subject a therapeutically effective amount of a precursor compound described herein, or a pharmaceutically acceptable salt or solvate thereof, wherein the cancer is one or more cancers selected from the group consisting of chronic lymphocytic leukemia (CLL), acute myeloid leukemia (AML), T-cell acute lymphoblastic leukemia (*T-ALL*), B cell acute lymphoblastic leukemia (B-ALL), and/or acute lymphoblastic leukemia (ALL).

Any of the treatment methods disclosed herein can be administered alone or in combination or in conjunction with another therapy or another agent. By "combination" it is meant to include (a) formulating a subject composition containing a subject precursor compound together with another agent, and (b) using the subject composition separate from the another agent as an overall treatment regimen. By "conjunction" it is meant that the another therapy or agent is administered either simultaneously, concurrently or sequentially with a subject composition comprising a precursor compound disclosed herein, with no specific time limits, wherein such conjunctive administration provides a therapeutic effect.

In some embodiment, a subject treatment method is combined with surgery, cellular therapy, chemotherapy, radiation, and/or immunosuppressive agents. Additionally, compositions of the present disclosure can be combined with other therapeutic agents, such as other anti-cancer agents, anti-allergic agents, anti-nausea agents (or anti-emetics), pain relievers, cytoprotective agents, immunostimulants, and combinations thereof.

In one embodiment, a subject treatment method is combined with a chemotherapeutic agent.

Exemplary chemotherapeutic agents include an anthracycline (e.g., doxorubicin (e.g., liposomal doxorubicin)), a vinca alkaloid (e.g., vinblastine, vincristine, vindesine, vinorelbine), an alkylating agent (e.g., cyclophosphamide, decarbazine, melphalan, ifosfamide, temozolomide), an immune cell antibody (e.g., alemtuzamab, gemtuzumab, rituximab, ofatumumab, tositumomab, brentuximab), an antimetabolite (including, e.g., folic acid antagonists, pyrimidine analogs, purine analogs and adenosine deaminase inhibitors (e.g., fludarabine)), a TNFR glucocorticoid induced TNFR related protein (GITR) agonist, a proteasome inhibitor (e.g., aclacinomycin A, gliotoxin or bortezomib), an immunomodulator such as thalidomide or a thalidomide derivative (e.g., lenalidomide). Additional chemotherapeutic agents contemplated for use in combination include busulfan (Myleran^{®}), busulfan injection (Busulfex^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), daunorubicin hydrochloride (Cerubidine^{®}), daunorubicin citrate liposome injection (DaunoXome^{®}), dexamethasone, doxorubicin hydrochloride (Adriamycin^{®}, Rubex^{®}), etoposide (Vepesid^{®}), fludarabine phosphate (Fludara^{®}), hydroxyurea (Hydrea^{®}), Idarubicin (Idamycin^{®}), mitoxantrone (Novantrone^{®}), Gemtuzumab Ozogamicin (Mylotarg^{®}), anastrozole (Arimidex^{®}), bicalutamide (Casodex^{®}), bleomycin sulfate (Blenoxane^{®}), , busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), , dacarbazine (DTIC-Dome^{®}), dactinomycin (Actinomycin D, Cosmegan), dexamethasone, docetaxel (Taxotere^{®}), 5-fluorouracil (Adrucil^{®}, Efudex^{®}), flutamide (Eulexin^{®}), tezacitibine, Gemcitabine (difluorodeoxycitidine), , ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}), tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}).

Anti-cancer agents of particular interest for combinations with a precursor compound of the present invention include: anthracyclines; alkylating agents; antimetabolites; drugs that inhibit either the calcium dependent phosphatase calcineurin or the p70S6 kinase FK506) or inhibit the p70S6 kinase; mTOR inhibitors; immunomodulators; anthracyclines; vinca alkaloids; proteosome inhibitors; GITR agonists; protein tyrosine phosphatase inhibitors; a CDK4 kinase inhibitor; a BTK inhibitor; a MKN kinase inhibitor; a DGK kinase inhibitor; or an oncolytic virus.

Exemplary antimetabolites include, without limitation, pyrimidine analogs, purine analogs and adenosine deaminase inhibitors): methotrexate (Rheumatrex^{®}, Trexall^{®}), 5-fluorouracil (Adrucil^{®}, Efudex^{®}, Fluoroplex^{®}), floxuridine (FUDF^{®}), cytarabine (Cytosar-U^{®}, Tarabine PFS), 6-mercaptopurine (Puri-Nethol^{®})), 6-thioguanine (Thioguanine Tabloid^{®}), fludarabine phosphate (Fludara^{®}), pentostatin (Nipent^{®}), pemetrexed (Alimta^{®}), raltitrexed (Tomudex^{®}), cladribine (Leustatin^{®}), clofarabine (Clofarex^{®}, Clolar^{®}), azacitidine (Vidaza^{®}), decitabine and gemcitabine (Gemzar^{®}). Preferred antimetabolites include, cytarabine, clofarabine and fludarabine.

Exemplary alkylating agents include, without limitation, nitrogen mustards, ethylenimine derivatives, alkyl sulfonates, nitrosoureas and triazenes): uracil mustard (Aminouracil Mustard^{®}, Chlorethaminacil^{®}, Demethyldopan^{®}, Desmethyldopan^{®}, Haemanthamine^{®}, Nordopan^{®}, Uracil nitrogen Mustard^{®}, Uracillost^{®}, Uracilmostaza^{®}, Uramustin^{®}, Uramustine^{®}), chlormethine (Mustargen^{®}), cyclophosphamide (Cytoxan^{®}, Neosar^{®}, Clafen^{®}, Endoxan^{®}, Procytox^{®}, Revimmune^{™}), ifosfamide (Mitoxana^{®}), melphalan (Alkeran^{®}), Chlorambucil (Leukeran^{®}), pipobroman (Amedel^{®}, Vercyte^{®}), triethylenemelamine (Hemel^{®}, Hexalen^{®}, Hexastat^{®}), triethylenethiophosphoramine, Temozolomide (Temodar^{®}), thiotepa (Thioplex^{®}), busulfan (Busilvex^{®}, Myleran^{®}), carmustine (BiCNU^{®}), lomustine (CeeNU^{®}), streptozocin (Zanosar^{®}), and Dacarbazine (DTIC-Dome^{®}). Additional exemplary alkylating agents include, without limitation, Oxaliplatin (Eloxatin^{®}); Temozolomide (Temodar^{®} and Temodal^{®}); Dactinomycin (also known as actinomycin-D, Cosmegen^{®}); Melphalan (also known as L-PAM, L-sarcolysin, and phenylalanine mustard, Alkeran^{®}); Altretamine (also known as hexamethylmelamine (HMM), Hexalen^{®}); Carmustine (BiCNU^{®}); Bendamustine (Treanda^{®}); Busulfan (Busulfex^{®} and Myleran^{®}); Carboplatin (Paraplatin^{®}); Lomustine (also known as CCNU, CeeNU^{®}); Cisplatin (also known as CDDP, Platinol^{®} and Platinol^{®}-AQ); Chlorambucil (Leukeran^{®}); Cyclophosphamide (Cytoxan^{®} and Neosar^{®}); Dacarbazine (also known as DTIC, DIC and imidazole carboxamide, DTIC-Dome^{®}); Altretamine (also known as hexamethylmelamine (HMM), Hexalen^{®}); Ifosfamide (Ifex^{®}); Prednumustine; Procarbazine (Matulane^{®}); Mechlorethamine (also known as nitrogen mustard, mustine and mechloroethamine hydrochloride, Mustargen^{®}); Streptozocin (Zanosar^{®}); Thiotepa (also known as thiophosphoamide, TESPA and TSPA, Thioplex^{®}); Cyclophosphamide (Endoxan^{®}, Cytoxan^{®}, Neosar^{®}, Procytox^{®}, Revimmune^{®}); and Bendamustine HCl (Treanda^{®}).

In an aspect, compositions provided herein can be administered in combination with radiotherapy such as radiation. Whole body radiation may be administered at 12 Gy. A radiation dose may comprise a cumulative dose of 12 Gy to the whole body, including healthy tissues. A radiation dose may comprise from 5 Gy to 20 Gy. A radiation dose may be 5 Gy, 6 Gy, 7 Gy, 8 Gy, 9 Gy, 10 Gy, 11 Gy, 12, Gy, 13 Gy, 14 Gy, 15 Gy, 16 Gy, 17 Gy, 18 Gy, 19 Gy, or up to 20 Gy. Radiation may be whole body radiation or partial body radiation. In the case that radiation is whole body radiation it may be uniform or not uniform. For example, when radiation may not be uniform, narrower regions of a body such as the neck may receive a higher dose than broader regions such as the hips.

Where desirable, an immunosuppressive agent can be used in conjunction with a subject treatment method. Exemplary immunosuppressive agents include but are not limited to cyclosporin, azathioprine, methotrexate, mycophenolate, and FK506, antibodies, or other immunoablative agents such as CAMPATH, anti-CD3 antibodies (e.g., muromonab, otelixizumab) or other antibody therapies, cytoxin, fludarabine, cyclosporin, FK506, rapamycin, mycophenolic acid, steroids, FR901228, cytokines, and irradiation, peptide vaccine, and any combination thereof. In accordance with the presently disclosed subject matter, the above-described various methods can comprise administering at least one immunomodulatory agent. In certain embodiments, the at least one immunomodulatory agent is selected from the group consisting of immunostimulatory agents, checkpoint immune blockade agents (e.g., blockade agents or inhibitors of immune checkpoint genes, such as, for example, PD-1, PD-L1, CTLA-4, IDO, TIM3, LAG3, TIGIT, BTLA, VISTA, ICOS, KIRs and CD39), radiation therapy agents, chemotherapy agents, and combinations thereof. In some embodiments, the immunostimulatory agents are selected from the group consisting of IL-12, an agonist costimulatory monoclonal antibody, and combinations thereof. In one embodiment, the immunostimulatory agent is IL-12. In some embodiments, the agonist costimulatory monoclonal antibody is selected from the group consisting of an anti-4-lBB antibody (e.g., urelumab, P.F-05082566), an anti-OX40 antibody (pogalizumab, tavolixizumab, PF-04518600), an anti-ICOS antibody (BMS986226, MEDI-570, GSK3359609, JTX-2011), and combinations thereof. In one embodiment, the agonist costimulatory monoclonal antibody is an anti-4-1 BB antibody. In some embodiments, the checkpoint immune blockade agents are selected from the group consisting of anti-PD-Ll antibodies (atezolizumab, avelumab, durvalumab, BMS-936559), anti-CTLA-4 antibodies (e.g., tremelimumab, ipilimumab), anti-PD-1 antibodies (e.g., pembrolizumab, nivolumab), anti-LAG3 antibodies (e.g., C9B7W, 410C9), anti-B7-H3 antibodies (e.g., DS-5573a), anti-TIM3 antibodies (e.g., F38-2E2), and combinations thereof. In one embodiment, the checkpoint immune blockade agent is an anti-PD-Ll antibody. In some cases, a precursor compound of the present disclosure can be administered to a subject in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In some cases, expanded cells can be administered before or following surgery. Alternatively, compositions comprising a precursor compound described herein can be administered with immunostimulants. Immunostimulants can be vaccines, colony stimulating agents, interferons, interleukins, viruses, antigens, costimulatory agents, immunogenicity agents, immunomodulators, or immunotherapeutic agents. An immunostimulant can be a cytokine such as an interleukin. One or more cytokines can be introduced with modified cells provided herein. Cytokines can be utilized to boost function of modified T lymphocytes (including adoptively transferred tumor-specific cytotoxic T lymphocytes) to expand within a tumor microenvironment. In some cases, IL-2 can be used to facilitate expansion of the modified cells described herein. Cytokines such as IL-15 can also be employed. Other relevant cytokines in the field of immunotherapy can also be utilized, such as IL-2, IL-7, IL-12, IL-15, IL-21, or any combination thereof. An interleukin can be IL-2, or aldeskeukin. Aldesleukin can be administered in low dose or high dose. A high dose aldesleukin regimen can involve administering aldesleukin intravenously every 8 hours, as tolerated, for up to about 14 doses at about 0.037 mg/kg (600,000 IU/kg). An immunostimulant (e.g., aldesleukin) can be administered within 24 hours after a cellular administration. An immunostimulant (e.g., aldesleukin) can be administered in as an infusion over about 15 minutes about every 8 hours for up to about 4 days after a cellular infusion. An immunostimulant (e.g., aldesleukin) can be administered at a dose from about 100,000 IU/kg, 200,000 IU/kg, 300,000 IU/kg, 400,000 IU/kg, 500,000 IU/kg, 600,000 IU/kg, 700,000 IU/kg, 800,000 IU/kg, 900,000 IU/kg, or up to about 1,000,000 IU/kg. In some cases, aldesleukin can be administered at a dose from about 100,000 IU/kg to 300,000 IU/kg, from 300,000 IU/kg to 500,000 IU/kg, from 500,000 IU/kg to 700,000 IU/kg, from 700,000 IU/kg to about 1,000,000 IU/kg.

In some embodiments, any of the precursor compounds herein that is capable of binding a Ras protein (e.g., KRAS) to modulate activity of such Ras protein may be administered in combination or in conjunction with one or more pharmacologically active agents comprising (1) an inhibitor of MEK (e.g., MEK1, MEK2) or of mutants thereof (e.g., trametinib, cobimetinib, binimetinib, selumetinib, refametinib); (2) an inhibitor of epidermal growth factor receptor (EGFR) and/or of mutants thereof (e.g., afatinib, erlotinib, gefitinib, lapatinib, cetuximab panitumumab, osimertinib, olmutinib, EGF-816); (3) an immunotherapeutic agent (e.g., checkpoint immune blockade agents, as disclosed herein); (4) a taxane (e.g., paclitaxel, docetaxel); (5) an anti-metabolite (e.g. antifolates such as methotrexate, raltitrexed, pyrimidine analogues such as 5-fluorouracil (5-FU), ribonucleoside and deoxyribonucleoside analogues, capecitabine and gemcitabine, purine and adenosine analogues such as mercaptopurine, thioguanine, cladribine and pentostatin, cytarabine (ara C), fludarabine); (6) an inhibitor of FGFR1 and/or FGFR2 and/or FGFR3 and/or of mutants thereof (e.g., nintedanib); (7) a mitotic kinase inhibitor (e.g., a CDK4/6 inhibitor, such as, for example, palbociclib, ribociclib, abemaciclib); (8) an anti-angiogenic drug (e.g., an anti-VEGF antibody, such as, for example, bevacizumab); (9) a topoisomerase inhibitor (e.g. epipodophyllotoxins such as for example etoposide and etopophos, teniposide, amsacrin, topotecan, irinotecan, mitoxantrone); (10) a platinum-containing compound (e.g. cisplatin, oxaliplatin, carboplatin); (11) an inhibitor of ALK and/or of mutants thereof (e.g. crizotinib, alectinib, entrectinib, brigatinib); (12) an inhibitor of c-MET and/or of mutants thereof (e.g., K252a, SU11274, PHA665752, PF2341066); (13) an inhibitor of BCR-ABL and/or of mutants thereof (e.g., imatinib, dasatinib, nilotinib); (14) an inhibitor of ErbB2 (Her2) and/or of mutants thereof (e.g., afatinib, lapatinib, trastuzumab, pertuzumab); (15) an inhibitor of AXL and/or of mutants thereof (e.g., R428, amuvatinib, XL-880); (16) an inhibitor of NTRK1 and/or of mutants thereof (e.g., Merestinib); (17) an inhibitor of RET and/or of mutants thereof (e.g., BLU-667, Lenvatinib); (18) an inhibitor of A-Raf and/or B-Raf and/or C-Raf and/or of mutants thereof (RAF-709, LY-3009120); (19) an inhibitor of ERK and/or of mutants thereof (e.g., uliacertinib); (20) an MDM2 inhibitor (e.g., HDM-201 , NVP-CGM097, RG-71 12, MK-8242, RG-7388, SAR405838, AMG-232, DS-3032, RG-7775, APG-115); (21) an inhibitor of mTOR (e.g., rapamycin, temsirolimus, everolimus, ridaforolimus); (22) an inhibitor of BET (e.g., I-BET 151, I-BET 762, OTX-015, TEN-010, CPI-203, CPI-0610, olionon, RVX-208, ABBC-744, LY294002, AZD5153, MT-1, MS645); (23) an inhibitor of IGF1/2 and/or of IGF1-R (e.g., xentuzumab, MEDI-573); (24) an inhibitor of CDK9 (e.g., DRB, flavopiridol, CR8, AZD 5438, purvalanol B, AT7519, dinaciclib, SNS-032); (25) an inhibitor of farnesyl transferase (e.g., tipifamib); (26) an inhibitor of SHIP pathway including SHIP2 inhibitor, as well as SHIP1 inhibitors; (27) an inhibitor of SRC (e.g., dasatinib); (28) an inhibitor of JAK (e.g., tofacitinib); (29) a PARP inhibitor (e.g. Olaparib, Rucaparib, Niraparib, Talazoparib), (30) a BTK inhibitor (e.g. Ibrutinib, Acalabrutinib, Zanubrutinib), (31) a ROS1 inhibitor (e.g., entrectinib), (32) an inhibitor of SHP pathway including SHP2 inhibitor (e.g., 6-(4-amino-4-methylpiperidin-1-yl)-3-(2,3-dichlorophenyl)pyrazin-2-amine, as well as SHP1 inhibitors, or (33) an inhibitor of Src, FLT3, HDAC, VEGFR, PDGFR, LCK, Bcr-Abl or AKT or (34) an inhibitor of KrasG12C mutant (e.g., including but not limited to AMG510, MRTX849, and any covalent inhibitors binding to the cysteine residue 12 of Kras, the structures of these compounds are publically known)( e.g., an inhibitor of Ras G12C as described in US20180334454, US20190144444, US20150239900, US10246424, US20180086753, WO2018143315, WO2018206539, WO20191107519, WO2019141250, WO2019150305, US9862701, US20170197945, US20180086753, US10144724, US20190055211, US20190092767, US20180127396, US20180273523, US10280172, US20180319775, US20180273515, US20180282307, US20180282308, WO2019051291, WO2019213526, WO2019213516, WO2019217691, WO2019241157, WO2019217307, WO2020047192, WO2017087528, WO2018218070, WO2018218069, WO2018218071, WO2020027083, WO2020027084, WO2019215203, WO2019155399, WO2020035031, WO2014160200, WO2018195349, WO2018112240, WO2019204442, WO2019204449, WO2019104505, WO2016179558, WO2016176338, or related patents and applications, each of which is incorporated by reference in its entirety),), (35) a SHC inhibitor (e.g., PP2, AID371185), (36) a GAB inhibitor (e.g., GAB-0001), (37) a GRB inhibitor, (38) a PI-3 kinase inhibitor (e.g., Idelalisib, Copanlisib, Duvelisib, Alpelisib, Taselisib, Perifosine, Buparlisib, Umbralisib, NVP-BEZ235-AN), (39) a MARPK inhibitor, (40) CDK4/6 (e.g., palbociclib, ribociclib, abemaciclib), or (41) MAPK inhibitor (e.g., VX-745, VX-702, RO-4402257, SCIO-469, BIRB-796, SD-0006, PH-797804, AMG-548, LY2228820, SB-681323, GW-856553, RWJ67657, BCT-197), or (42) an inhibitor of SHP pathway including SHP2 inhibitor (e.g., 6-(4-amino-4-methylpiperidin-1-yl)-3-(2,3-dichlorophenyl)pyrazin-2-amine, RMC-4630, TNO155 ( 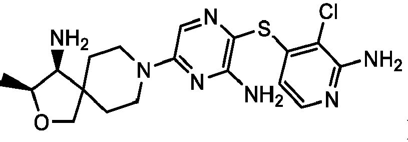 ), JAB-3068 ( 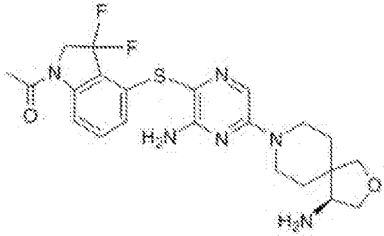 ), IACS-13909BBP-398 ( 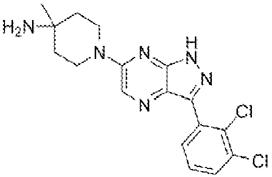 ), SHP099 ( 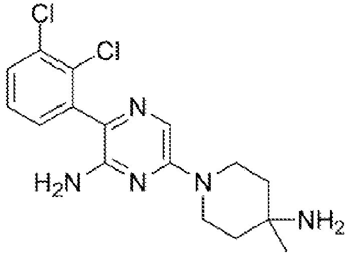 ), ERAS-601, and RMC-4550 ( 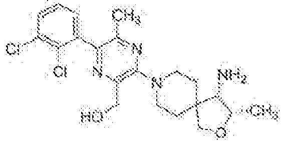 ), as well as SHP1 inhibitors.. In some embodiments, any of the precursor compounds herein that is capable of binding a Ras protein (e.g., Kras) to modulate activity of such Ras protein may be administered in combination or in conjunction with one or more checkpoint immune blockade agents (e.g., anti-PD-1 and/or anti-PD-L1 antibody, anti-CLTA-4 antibody). In some embodiments, any of the precursor compounds herein that is capable of binding a Ras protein (e.g., KRAS) to modulate activity of such Ras protein may be administered in combination or in conjunction with one or more pharmacologically active agents comprising an inhibitor against one or more targets selected from the group of MEK, epidermal growth factor receptor (EGFR), FGFR1, FGFR2, FGFR3, mitotic kinase, topoisomerase, ALK, c-MET, ErbB2, AXL, NTRK1, RET, A-Raf, B-Raf, C-Raf, ERK, MDM2, mTOR, BET, IGF1/2, IGF1-R, CDK9, SHIP1, SHIP2, SHP2, SRC, JAK, PARP, BTK, FLT3, HDAC, VEGFR, PDGFR, LCK, Bcr-Abl, AKT, KrasG12C mutant, and ROS1. Where desired, the additional agent can be an inhibitor against one or more targets selected from the group of MEK, epidermal growth factor receptor (EGFR), FGFR1, FGFR2, FGFR3, mitotic kinase, topoisomerase, ALK, c-MET, ErbB2, AXL, NTRK1, RET, A-Raf, B-Raf, C-Raf, ERK, MDM2, mTOR, BET, IGF1/2, IGF1-R, CDK9, SHP2, SRC, JAK, PARP, BTK, FLT3, HDAC, VEGFR, PDGFR, LCK, Bcr-Abl, AKT, KrasG12C mutant, and ROS1. In some embodiments, any of the precursor compounds herein that is capable of binding a Ras protein (e.g., KRAS, mutant Ras protein) to modulate activity of such Ras protein (e.g., mutant Ras protein such as G12D or G12S mutant KRas protein) may be administered in combination or in conjunction with one or more additional pharmacologically active agents comprising an inhibitor of SOS (e.g., SOS1, SOS2) or of mutants thereof. In embodiments, the additional pharmacologically active agent administered in combination or in conjunction with a precursor compound described herein (e.g., precursor compound capable of binding a Ras protein) is an inhibitor of SOS (e.g., SOS1, SOS2). In embodiments, the additional pharmacologically active agent administered in combination or in conjunction with a precursor compound (e.g., precursor compound capable of binding a Ras protein) described herein is an inhibitor of SOS (e.g., SOS1, SOS2). In embodiments, the additional pharmacologically active agent administered in combination or in conjunction with a precursor compound (e.g., precursor compound capable of binding a Ras protein) described herein is an inhibitor of SOS (e.g., SOS1, SOS2) selected from RMC-5845, BI-3406 ( 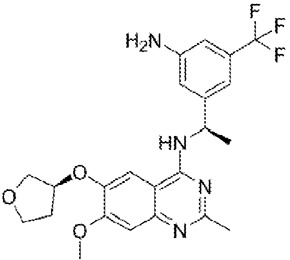 ), BI-1701963, and BAY 293 ( 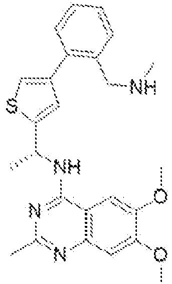 ). In embodiments, the additional pharmacologically active agent administered in combination or in conjunction with a precursor compound described herein (e.g., precursor compound capable of binding a Ras protein) is an inhibitor of SOS (e.g., SOS1, SOS2) described in WO2021092115, WO2018172250, WO2019201848, WO2019122129, WO2018115380, WO2021127429, WO2020180768, or WO2020180770, all of which are herein incorporated by reference in their entirety for all purposes.

. In some embodiments, any of the precursor compounds herein that is capable of binding a Ras protein (e.g., Kras) to modulate activity of such Ras protein may be administered in combination or in conjunction with one or more checkpoint immune blockade agents (e.g., anti-PD-1 and/or anti-PD-L1 antibody, anti-CLTA-4 antibody).

In some embodiments, any of the precursor compounds described herein that is capable of binding a Ras protein (e.g., KRAS) may be administered in combination or in conjunction with one or more pharmacologically active agents comprising an inhibitor of (1) SOS1 or a mutant thereof (e.g., RMC-5845, BI-3406, BAY-293, BI-1701963); (2) SHP2 or a mutant thereof (e.g., 6-(4-amino-4-methylpiperidin-1-yl)-3-(2,3-dichlorophenyl)pyrazin-2-amine, TNO155, RMC-4630, ERAS-601, JAB-3068, IACS-13909BBP-398, SHP099, RMC-4550); (3) SHC or a mutant thereof (e.g., PP2, AID371185); (4) GAB or a mutant thereof (e.g., GAB-0001); (5) GRB or a mutant thereof; (6) JAK or a mutant thereof (e.g., tofacitinib); (7) A-RAF, B-RAF, C-RAF, or a mutant thereof (e.g., RAF-709, LY-3009120); (8) BRAF or a mutant thereof (e.g., Sorafenib, Vemurafenib, Dabrafenib, Encorafenib, regorafenib, GDC-879); (9) MEK or a mutant thereof (e.g., trametinib, cobimetinib, binimetinib, selumetinib, refametinib, AZD6244); (10) ERK or a mutant thereof (e.g., uliacertinib, MK-8353, LTT462, AZD0364, SCH772984, BIX02189, LY3214996, ravoxertinib);; (11) PI3K or a mutant thereof (e.g., Idelalisib, Copanlisib, Duvelisib, Alpelisib, Taselisib, Perifosine, Buparlisib, Umbralisib, NVP-BEZ235-AN); (12) MAPK or a mutant thereof (e.g., VX-745, VX-702, RO-4402257, SCIO-469, BIRB-796, SD-0006, PH-797804, AMG-548, LY2228820, SB-681323, GW-856553, RWJ67657, BCT-197); (13) EGFR or a mutant thereof (e.g., afatinib, erlotinib, gefitinib, lapatinib, cetuximab panitumumab, osimertinib, olmutinib, EGF-816); (14) c-MET or a mutant thereof (e.g., K252a, SU11274, PHA665752, PF2341066); (15) ALK or a mutant thereof (e.g. crizotinib, alectinib, entrectinib, brigatinib); (16) FGFR1, FGFR-2, FGFR-3, FGFR-4 or a mutant thereof (e.g., nintedanib); (17) BCR-ABL or a mutant thereof (e.g., imatinib, dasatinib, nilotinib); (18) ErbB2 (Her2) or a mutant thereof (e.g., afatinib, lapatinib, trastuzumab, pertuzumab); (19) AXL or a mutant thereof (e.g., R428, amuvatinib, XL-880); (20) NTRK1 or a mutant thereof (e.g., merestinib); (21) ROS1 or a mutant thereof (e.g., entrectinib); (22) RET or a mutant thereof (e.g., BLU-667, Lenvatinib); (23) MDM2 or a mutant thereof (e.g., HDM-201 , NVP-CGM097, RG-71 12, MK-8242, RG-7388, SAR405838, AMG-232, DS-3032, RG-7775, APG-115); (24) mTOR or a mutant thereof (e.g., rapamycin, temsirolimus, everolimus, ridaforolimus); (25) BET or a mutant thereof (e.g., I-BET 151, I-BET 762, OTX-015, TEN-010, CPI-203, CPI-0610, olionon, RVX-208, ABBC-744, LY294002, AZD5153, MT-1, MS645); (26) IGF1, IGF2, IGF1R, or a mutant thereof (e.g., xentuzumab, MEDI-573); (27) CDK9 or a mutant thereof (e.g., DRB, flavopiridol, CR8, AZD 5438, purvalanol B, AT7519, dinaciclib, SNS-032); or (28) CDK4/6 (e.g., palbociclib, ribociclib, abemaciclib).

In combination therapy, a precursor compound provided herein and other anti-cancer agent(s) may be administered either simultaneously, concurrently or sequentially with no specific time limits, wherein such administration provides therapeutically effective levels of the two compounds in the body of the patient.

In some embodiments, the precursor compound of the present disclosure and the other anti-cancer agent(s) are generally administered sequentially in any order by infusion or orally. The dosing regimen may vary depending upon the stage of the disease, physical fitness of the patient, safety profiles of the individual drugs, and tolerance of the individual drugs, as well as other criteria well-known to the attending physician and medical practitioner(s) administering the combination. The precursor compound of the present invention and other anti-cancer agent(s) may be administered within minutes of each other, hours, days, or even weeks apart depending upon the particular cycle being used for treatment. In addition, the cycle could include administration of one drug more often than the other during the treatment cycle and at different doses per administration of the drug.

An antibiotic can be administered to a subject as part of a therapeutic regime. An antibiotic can be administered at a therapeutically effective dose. An antibiotic can kill or inhibit growth of bacteria. An antibiotic can be a broad spectrum antibiotic that can target a wide range of bacteria. Broad spectrum antibiotics, either a 3^{rd} or 4^{th} generation, can be cephalosporin or a quinolone. An antibiotic can also be a narrow spectrum antibiotic that can target specific types of bacteria. An antibiotic can target a bacterial cell wall such as penicillins and cephalosporins. An antibiotic can target a cellular membrane such as polymyacins. An antibiotic can interfere with essential bacterial enzymes such as antibiotics: rifamycins, lipiarmycins, quinolones, and sulfonamides. An antibiotic can also be a protein synthesis inhibitor such as macrolides, lincosamides, and tetracyclines. An antibiotic can also be a cyclic lipopeptide such as daptomycin, glycylcyclines such as tigecycline, oxazolidiones such as linezolid, and lipiarmycins such as fidaxomicin. In some cases, an antibiotic can be 1^{st} generation, 2^{nd} generation, 3^{rd} generation, 4^{th} generation, or 5^{th} generation. A first-generation antibiotic can have a narrow spectrum. Examples of 1^{st} generation antibiotics can be penicillins (Penicillin G or Penicillin V), Cephalosporins (Cephazolin, Cephalothin, Cephapirin, Cephalethin, Cephradin, or Cephadroxin). In some cases, an antibiotic can be 2^{nd} generation. 2^{nd} generation antibiotics can be a penicillin (Amoxicillin or Ampicillin), Cephalosporin (Cefuroxime, Cephamandole, Cephoxitin, Cephaclor, Cephrozil, Loracarbef). In some cases, an antibiotic can be 3^{rd} generation. A 3^{rd} generation antibiotic can be penicillin (carbenicillin and ticarcillin) or cephalosporin (Cephixime, Cephtriaxone, Cephotaxime, Cephtizoxime, and Cephtazidime). An antibiotic can also be a 4^{th} generation antibiotic. A 4^{th} generation antibiotic can be Cephipime. An antibiotic can also be 5^{th} generation. 5^{th} generation antibiotics can be Cephtaroline or Cephtobiprole.

In some cases, an anti-viral agent may be administered as part of a treatment regime. In some cases, a herpes virus prophylaxis can be administered to a subject as part of a treatment regime. A herpes virus prophylaxis can be valacyclovir (Valtrex). Valtrex can be used orally to prevent the occurrence of herpes virus infections in subjects with positive HSV serology. It can be supplied in 500 mg tablets. Valacyclovir can be administered at a therapeutically effective amount.

In some cases, a treatment regime may be dosed according to a body weight of a subject. In subjects who are determined obese (BMI > 35) a practical weight may need to be utilized. BMI is calculated by: BMI = weight (kg)/ [height (m)]².

Body weight may be calculated for men as 50 kg+2.3*(number of inches over 60 inches) or for women 45.5kg + 2.3 (number of inches over 60 inches). An adjusted body weight may be calculated for subjects who are more than 20% of their ideal body weight. An adjusted body weight may be the sum of an ideal body weight + (0.4 x (Actual body weight - ideal body weight)). In some cases, a body surface area may be utilized to calculate a dosage. A body surface area (BSA) may be calculated by: BSA (m2) =^Height (cm) *Weight (kg)/3600.

In an aspect is provided a method of modulating activity of a Ras (e.g., K-Ras) protein, comprising contacting a Ras protein with an effective amount of a precursor compound described herein, or a pharmaceutically acceptable salt or solvate thereof, thereby modulating the activity of the Ras (e.g., K-Ras) protein.

In some embodiments, the subject method comprises administering an additional agent or therapy.

In some embodiments is a method of modulating activity of a Ras protein, comprising contacting a Ras protein with an effective amount of a precursor compound described, or a pharmaceutically acceptable salt or solvate thereof, wherein said modulating comprises inhibiting the Ras (e.g., K-Ras) protein activity. In some embodiments is a method of modulating activity of a Ras protein including Ras G12S mutant proteins such as K-Ras G12S, H-Ras G12S, and N-Ras G12S, comprising contacting the Ras protein with an effective amount of a precursor compound described herein, or a pharmaceutically acceptable salt or solvate thereof.

In some embodiments, provided is a method of reducing Ras signaling output in a cell by contacting the cell with a precursor compound described herein. A reduction in Ras signalling can be evidenced by one or more members of the following: (i) an increase in steady state level of GDP-bound modified protein; (ii) a reduction of phosphorylated AKTs473, (iii) a reduction of phosphorylated ERKT202/y204, (iv) a reduction of phosphorylated S6S235/236, and (v) reduction of cell growth of a tumor cell expressing a Ras G12S mutant protein, and (vi) reduction in Ras interaction with a Ras-pathway signaling protein. Non-limiting examples of Ras-pathway signaling protein include SOS (including SOS1 and SOS2), RAF, SHC, SHP (including SHP1 and SHP2), MEK, MAPK, ERK, GRB, RASA1, and GNAQ. In some cases, the reduction in Ras signaling output can be evidenced by two, three, four or all of (i)-(v) above. In some embodiments, the reduction any one or more of (i)-(v) can be 0.1-fold, 0.2-fold, 0.3-fold, 0.4-fold, 0.5-fold, 0.6-fold, 0.7-fold, 0.8-fold, 0.9-fold, 1-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, 200-fold, 300-fold, 400-fold, 500-fold, 600-fold, 700-fold, 800-fold, 900-fold, 1000-fold, 2000-fold, 3000-fold, 4000-fold, 5000-fold, or more as compared to control untreated with a subject precursor compound. A reduction in cell growth can be demonstrated with the use of tumor cells or cell lines. A tumor cell line can be derived from a tumor in one or more tissues, e.g., pancreas, lung, ovary, biliary tract, intestine (e.g., small intestine, large intestine (i.e. colon)), endometrium, stomach, hematopoietic tissue (e.g., lymphoid tissue), etc. Examples of the tumor cell line with a K-Ras mutation may include, but are not limited to, A549 (e.g., K-Ras G12S), AGS (e.g., K-Ras G12D), ASPC1 (e.g., K-Ras G12D), Calu-6 (e.g., K-Ras Q61K), CFPAC-1 (e.g., K-Ras G12V), CL40 (e.g., K-Ras G12D), COLO678 (e.g., K-Ras G12D), COR-L23 (e.g., K-Ras G12V), DAN-G (e.g., K-Ras G12V), GP2D (e.g., K-Ras G12D), GSU (e.g., K-Ras G12F), HCT116 (e.g., K-Ras G13D), HEC1A (e.g., K-Ras G12D), HEC1B (e.g., K-Ras G12F), HEC50B (e.g., K-Ras G12F), HEYA8 (e.g., K-Ras G12D or G13D), HPAC (e.g., K-Ras G12D), HPAFII (e.g., K-Ras G12D), HUCCT1 (e.g., K-Ras G12D), KARPAS620 (e.g., K-Ras G13D), KOPN8 (e.g., K-Ras G13D), KP-3 (e.g., K-Ras G12V), KP-4 (e.g., K-Ras G12D), L3.3 (e.g., K-Ras G12D), LoVo (e.g., K-Ras G13D), LS180 (e.g., K-Ras G12D), LS513 (e.g., K-Ras G12D), MCAS (e.g., K-Ras G12D), NB4 (e.g., K-Ras A18D), NCI-H1355 (e.g., K-Ras G13C), NCI-H1573 (e.g., K-Ras G12A), NCI-H1944 (e.g., K-Ras G13D), NCI-H2009 (e.g., K-Ras G12A), NCI-H441 (e.g., K-Ras G12V), NCI-H747 (e.g., K-Ras G13D), NOMO-1 (e.g., K-Ras G12D), OV7 (e.g., K-Ras G12D), PANC0203 (e.g., K-Ras G12D), PANC0403 (e.g., K-Ras G12D), PANC0504 (e.g., K-Ras G12D), PANC0813 (e.g., K-Ras G12D), PANC1 (e.g., K-Ras G12D), Panc-10.05 (e.g., K-Ras G12D), PaTu-8902 (e.g., K-Ras G12V), PK1 (e.g., K-Ras G12D), PK45H (e.g., K-Ras G12D), PK59 (e.g., K-Ras G12D), SK-CO-1 (e.g., K-Ras G12V), SKLU1 (e.g., K-Ras G12D), SKM-1 (e.g., K-Ras K117N), SNU1 (e.g., K-Ras G12D), SNU1033 (e.g., K-Ras G12D), SNU1197 (e.g., K-Ras G12D), SNU407 (e.g., K-Ras G12D), SNU410 (e.g., K-Ras G12D), SNU601 (e.g., K-Ras G12D), SNU61 (e.g., K-Ras G12D), SNU8 (e.g., K-Ras G12D), SNU869 (e.g., K-Ras G12D), SNU-C2A (e.g., K-Ras G12D), SU.86.86 (e.g., K-Ras G12D), SUIT2 (e.g., K-Ras G12D), SW1990 (e.g., K-Ras G12D), SW403 (e.g., K-Ras G12V), SW480 (e.g., K-Ras G12V), SW620 (e.g., K-Ras G12V), SW948 (e.g., K-Ras Q61L), T3M10 (e.g., K-Ras G12D), TCC-PAN2 (e.g., K-Ras G12R), TGBC11TKB (e.g., K-Ras G12D), and MIA Pa-Ca (e.g., MIA Pa-Ca 2 (e.g., K-Ras G12C)).

### Pharmaceutical compositions and methods of administration

In an aspect is provided a pharmaceutical composition comprising a precursor compound described herein, or a pharmaceutically acceptable salt or solvate thereof, and a pharmaceutically acceptable excipient.

The precursor compounds described herein, or a pharmaceutically acceptable salt or solvate thereof, are administered to subjects in a biologically compatible form suitable for administration to treat or prevent diseases, disorders or conditions. Administration of the precursor compounds described herein can be in any pharmacological form including a therapeutically effective amount of a precursor compound described herein, or a pharmaceutically acceptable salt or solvate thereof, alone or in combination with a pharmaceutically acceptable carrier.

In certain embodiments, the precursor compounds described herein are administered as a pure chemical. In other embodiments, the precursor compounds described herein are combined with a pharmaceutically suitable or acceptable carrier (also referred to herein as a pharmaceutically suitable (or acceptable) excipient, physiologically suitable (or acceptable) excipient, or physiologically suitable (or acceptable) carrier) selected on the basis of a chosen route of administration and standard pharmaceutical practice as described, for example, in Remington: The Science and Practice of Pharmacy (Gennaro, 21st Ed. Mack Pub. Co., Easton, PA (2005)).

Accordingly, provided herein is a pharmaceutical composition comprising at least one precursor compound described herein, or a pharmaceutically acceptable salt, together with one or more pharmaceutically acceptable excipients. The excipient(s) (or carrier(s)) is acceptable or suitable if the excipient is compatible with the other ingredients of the composition and not deleterious to the recipient (i.e., the subject) of the composition.

In some embodiments of the methods described herein, the precursor compounds described herein are administered either alone or in combination with pharmaceutically acceptable carriers, excipients or diluents, in a pharmaceutical composition. Administration of the precursor compounds and compositions described herein can be affected by any method that enables delivery of the precursor compounds to the site of action. These methods include, though are not limited to delivery via enteral routes (including oral, gastric or duodenal feeding tube, rectal suppository and rectal enema), parenteral routes (injection or infusion, including intraarterial, intracardiac, intradermal, intraduodenal, intramedullary, intramuscular, intraosseous, intraperitoneal, intrathecal, intravascular, intravenous, intravitreal, epidural and subcutaneous), inhalational, transdermal, transmucosal, sublingual, buccal and topical (including epicutaneous, dermal, enema, eye drops, ear drops, intranasal, vaginal) administration, although the most suitable route may depend upon for example the condition and disorder of the recipient. By way of example only, precursor compounds described herein can be administered locally to the area in need of treatment, by for example, local infusion during surgery, topical application such as creams or ointments, injection, catheter, or implant. The administration can also be by direct injection at the site of a diseased tissue or organ.

In some embodiments of the methods described herein, pharmaceutical compositions suitable for oral administration are presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. In some embodiments, the active ingredient is presented as a bolus, electuary or paste.

Pharmaceutical compositions which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered precursor compound moistened with an inert liquid diluent. In some embodiments, the tablets are coated or scored and are formulated so as to provide slow or controlled release of the active ingredient therein. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active precursor compounds may be dissolved or suspended in suitable liquids, such as fatty oils, liquid paraffin, or liquid polyethylene glycols. In some embodiments, stabilizers are added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or Dragee coatings for identification or to characterize different combinations of active precursor compound doses.

In some embodiments of the methods described herein, pharmaceutical compositions are formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The compositions may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Pharmaceutical compositions for parenteral administration include aqueous and non-aqueous (oily) sterile injection solutions of the active precursor compounds which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the precursor compounds to allow for the preparation of highly concentrated solutions.

Pharmaceutical compositions may also be formulated as a depot preparation. Such long acting formulations may be administered by implantation (for example subcutaneously or intramuscularly) or by intramuscular injection. Thus, for example, the precursor compounds may be formulated with suitable polymeric or hydrophobic materials (for example, as an emulsion in an acceptable oil) or ion exchange resins, or as sparingly soluble derivatives, for example, as a sparingly soluble salt.

### EXAMPLES

The following examples are provided for illustrative purposes only and not to limit the scope of the claims provided herein.

As used herein, the following abbreviations, unless otherwise indicated, shall be understood to have the following meanings:
- ACN or MeCN: acetonitrile
- AcOH: acetic acid
- Ac: acetyl
- BINAP: 2,2'-bis(diphenylphosphino)-1,1'-binaphthalene
- Bn: benzyl
- BOC or Boc: *tert*-butyl carbamate
- i-Bu: iso-butyl
- t-Bu: *tert*-butyl
- DCM: dichloromethane (CH₂Cl₂)
- DIBAL-H: diisobutylaluminum hydride
- DIPEA: or DIEA diisopropylethylamine
- DMAP: 4-(*N*,*N*-dimethylamino)pyridine
- DME: 1,2-dimethoxyethane
- DMF: *N*,*N-*dimethylformamide
- DMA: *N*,*N*-dimethylacetamide
- DMSO: dimethylsulfoxide
- Dppf: or dppf 1,1 '-bis(diphenylphosphino)ferrocene
- EDC or EDCI: *N*-(3-dimethylaminopropyl)-*N*'-ethylcarbodiimide hydrochloride
- eq: equivalent(s)
- Et: ethyl
- Et2O: diethyl ether
- EtOH: ethanol
- EtOAc: ethyl acetate
- HPLC: high performance liquid chromatography
- KHMDS: potassium bis(trimethylsilyl)amide
- NaHMDS: sodium bis(trimethylsilyl)amide
- LiHMDS: lithium bis(trimethylsilyl)amide
- LAH: lithium aluminum anhydride
- LCMS: liquid chromatography mass spectrometry
- Me: methyl
- MeOH: methanol
- MS: mass spectroscopy
- Ms: mesyl
- NMR: nuclear magnetic resonance
- Ph: phenyl
- iPr/i-Pr: *iso*-propyl
- RP-HPLC: reverse-phase high-pressure liquid chromatography
- RT: room temperature
- TBS: *tert*-butyldimethylsilyl
- TEA: triethylamine
- TFA: trifluoroacetic acid
- THF: tetrahydrofuran
- TLC: thin layer chromatography
- TMS: trimethylsilyl
- TsOH/p-TsOH: p-toluenesulfonic acid.

### Example 1: General Synthesis

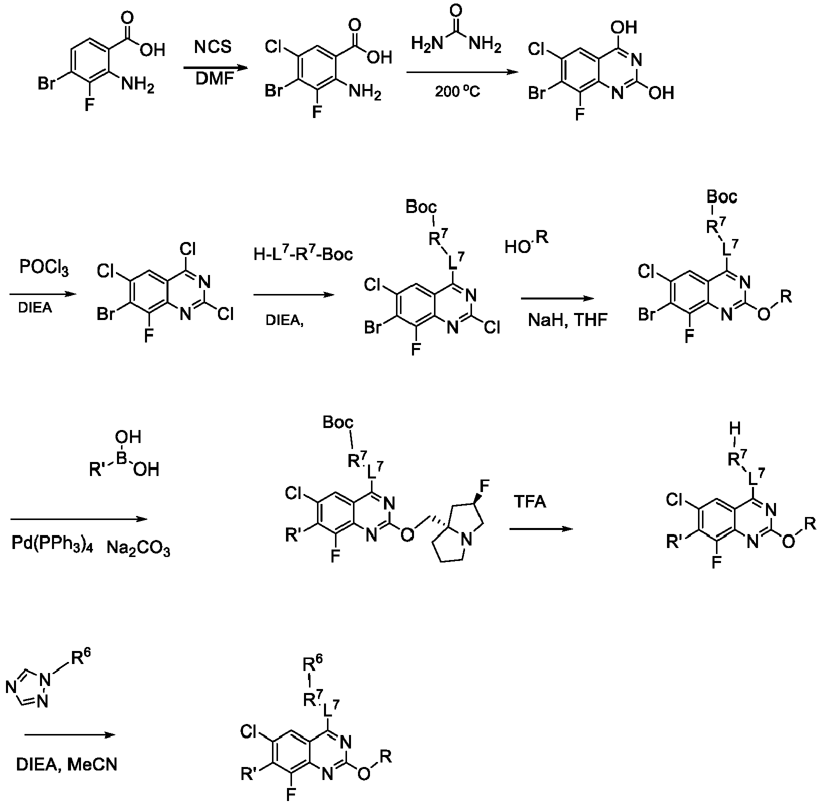

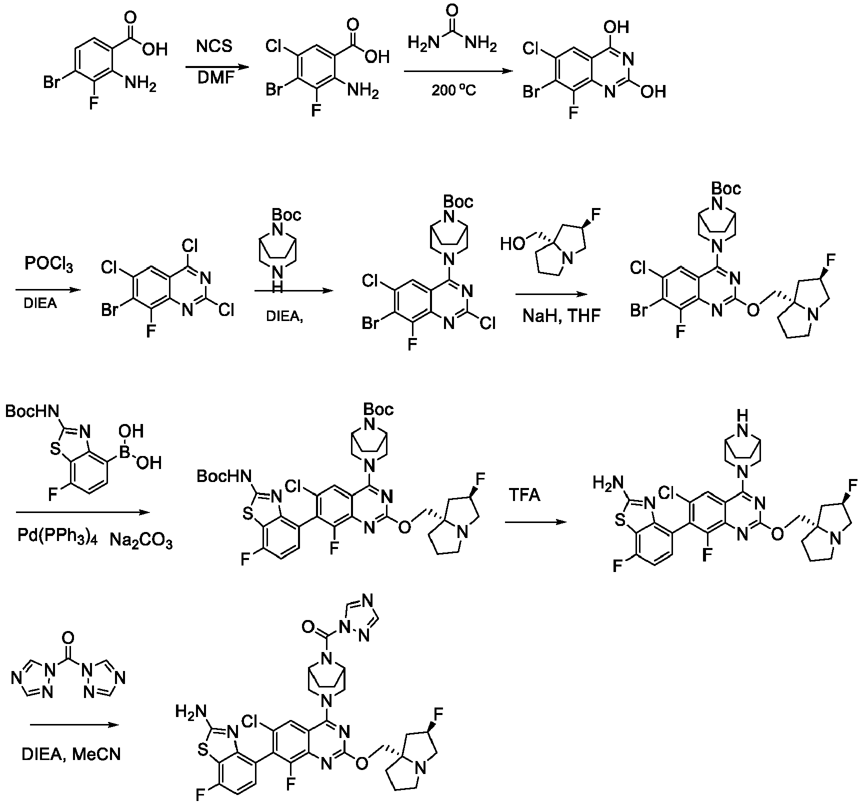

### Example 1b: General Synthesis 2

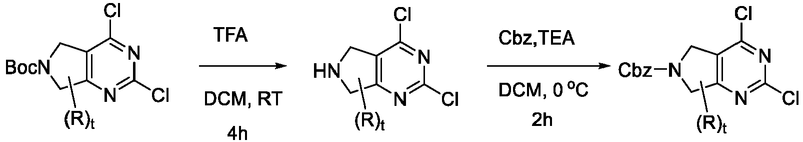

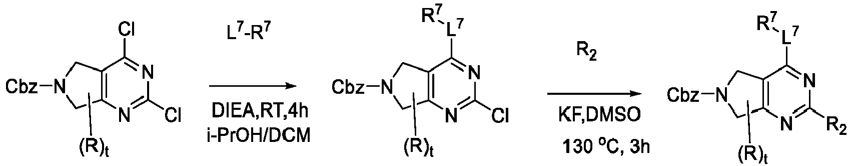

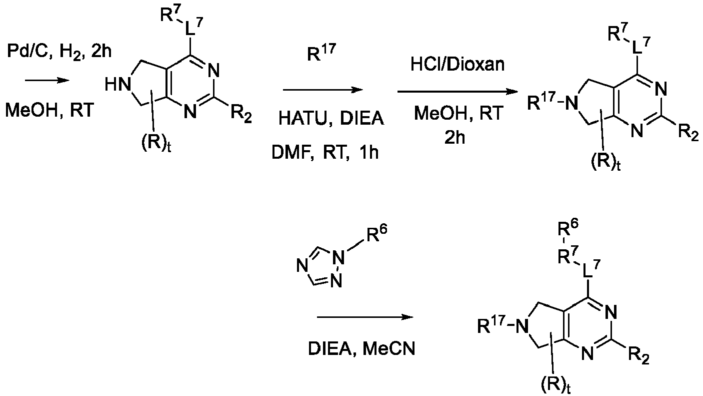

### Example 1c: General Synthesis 3

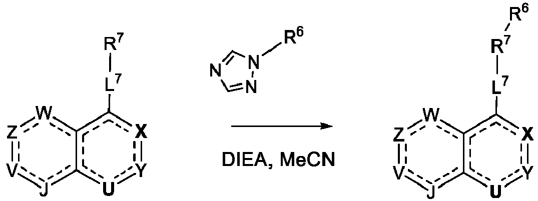

General Synthetic scheme for adding R6 to compounds described herein that may be synthesize using well known and understood synthetic schemes.

### Example 1d: General Synthesis 4

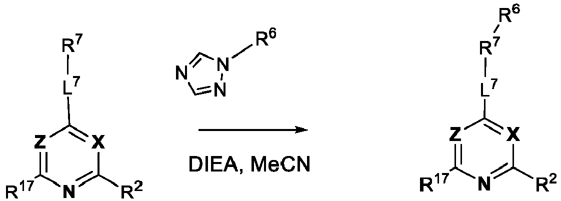

General Synthetic scheme for adding R6 to compounds described herein that may be synthesize using well known and understood synthetic schemes.

### Synthesis of Compound A103

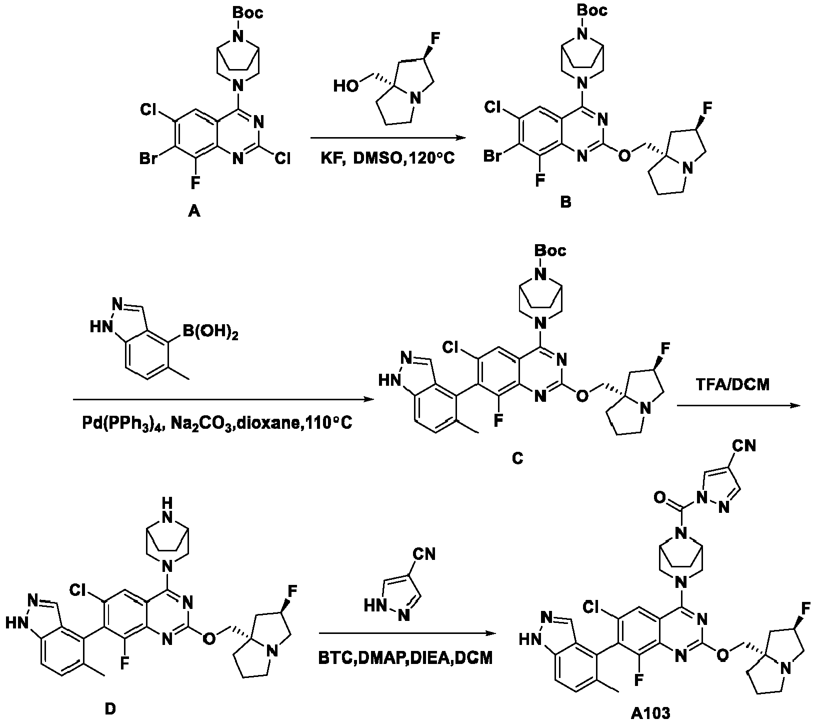

### (1R,5S)-tert-butyl 3-(7-bromo-6-chloro-8-fluoro-2-(((2R,7aS)-2-fluorohexahydro-1H-pyrrolizin-7a-yl)methoxy)quinazolin-4-yl)-3,8-diazabicyclo[3.2.1]octane-8-carboxylate(B)

To a mixture of compound A (2.5 g, 4.97 mmol) in DMSO (4 mL) was added ((2R,7aS)-2-fluorohexahydro-1H-pyrrolizin-7a-yl)methanol (1.58 g, 9.94 mmol) at rt followed by KF (2.3 g, 39.76 mmoil).The mixture was stirred at 120 °C for 4 hours under N2, the mixture was poured to ice water. The mixture was extracted with EA. The organic layer was washed with brine, dried over Na₂SO₄ and concentrated. The residue was purified by flash column chromatography on silica gel (DCM : CH₃OH =10:1) to afford the desired product (2.9 g, 93.5%yield). ESI-MS *m*/*z:* 630.2 [M+H]⁺.

### (1R,5S)-tert-butyl 3-(6-chloro-8-fluoro-2-(((2R,7aS)-2-fluorohexahydro-1H-pyrrolizin-7a-yl)methoxy)-7-(5-methyl-1H-indazol-4-yl)quinazolin-4-yl)-3,8-diazabicyclo[3.2.1]octane-8-carboxylate(C)

To a solution of B (500 mg, 0.8 mmol) in 1,4 - dioxane/H₂O (10 mL / 2mL) at rt, (5-methyl-1H-indazol-4-yl)boronic acid (280 mg, 1.6mmol), Tetrakis ( triphenylphosphine ) palladium (180 mg, 0.16 mmol) and Na₂CO₃ ( 250 mg, 2.4mmol) were added, took place air with N₂ several times, then heated to 110°C and stirred overnight. The mixture was partitioned between water and ethyl acetate. The organic layer was dried over anhydrous Na₂SO₄, filtered and concentrated in vacuo. The residue was purified by flash column chromatography on silica gel to afford the desired product (450 mg, 83.5% yield) as a yellow solid. ESI-MS m/z: 680.3 [M+H]⁺.

### 4-((1R,SS)-3,8-diazabicyclo[3.2.1]octan-3-yl)-6-chloro-8-fluoro-2-(((2R,7aS)-2-fluorohexahydro-1H-pyrrolizin-7a-yl)methoxy)-7-(5-methyl-1H-indazol-4-yl)quinazoline(D)

To a stirred solution of C (450 g, 0.66 mmol) in DCM (12 mL) was added TFA (4 mL) at RT and then stirred at RT for 1.5 h. LC-MS shows no SM left. Concentrated the solvent under reduced pressure. The residue was extracted by EtOAc (2 X 50 mL), washed with NaHCO₃ andbrine, dried over Na₂SO₄ and concentrated. The residue was purified by flash column chromatography on silica gel (DCM : CH₃OH =10:1) to afford the desired product (400 mg). ESI-MS m/z: 580.1 [M+H]⁺.

### 1-(3-(6-chloro-8-fluoro-2-(((2R,7aS)-2-fluorotetrahydro-1H-pyrrolizin-7a(5H)-yl)methoxy)-7-(5-methyl-1H-indazol-4-yl)quinazolin-4-yl)-3,8-diazabicyclo[3.2.1]octane-8-carbonyl)-1H-pyrazole-4-carbonitrile (A103)

To a mixture of 1H-pyrazole-4-carbonitrile (9.6 mg, 0.103 mmol) in DCM (5 mL) were added BTC (10.0 mg, 0.034 mmol) and DIEA (34.75 mg, 0.25 mmol) at rt and stirred for 30 min, then B (50 mg, 0.086 mmol) was added at rt. The mixture was stirred for 10 min and poured to water. The mixture was extracted with EA. The organic layer was washed with brine, dried over Na₂SO₄ and concentrated. The residue was purified by flash column chromatography on silica gel (DCM : CH₃OH =10:1) to afford the desired product (15 mg, 25% yield). ESI-MS m/z: 699.4 [M+H]⁺.

¹H NMR (400MHz, DMSO-d6) δ: 13.24 (s,1H), 9.21(s, 1H), 8.43 (s, 1H), 8.02 (s, 1H), 7.60-7.53 (m, 2H), 7.40-7.37 (d, 1H), 5.33 (m, 1H), 4. 57-4.34 (m, 2H), 3.84-3.76 (m, 2H), 3.60 (s, 2H), 3.11 (m, 2H), 2.16 (s, 3H), 2.07-1.86 (m, 4H), 1.28 (s, 8H), 0.87-0.84 (m,1H).

### Synthesis of Compound A122

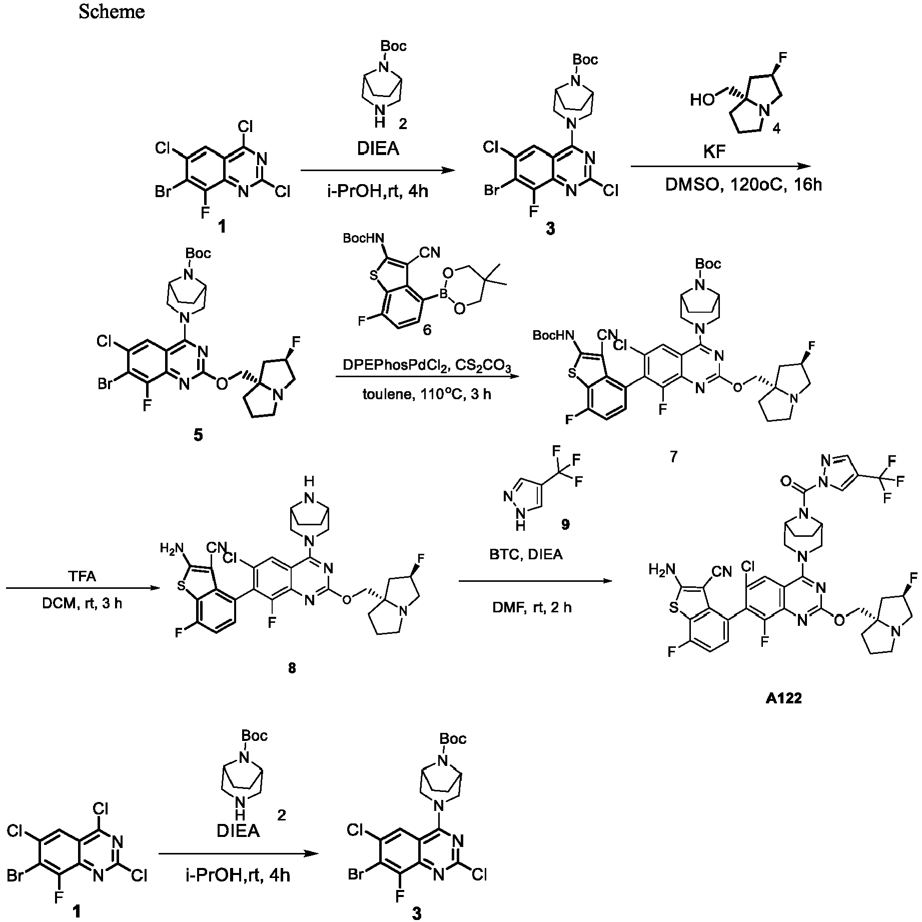

To a solution of compound 1 (5 g, 15.25 mmol) and compound 2 (3.2 g, 15.25 mmol) in i-PrOH (70 ml) was added DIEA (5.9 g 45.8 mmol). The reaction mixture was stirred at 25 °C for 3 h. The mixture was poured into ice water (200 mL). The mixture was filtered and the solid was diluted with MeOH (150 ml). The organic solution was concentrated to dryness under reduced pressure to give compound 3 (5.1 g, Yield: 66 %) as a white solid. MS m/z (ESI): 505.0 [M+H]⁺.

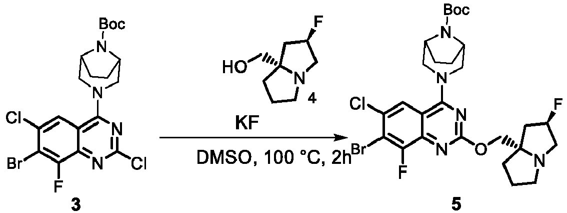

To a solution of compound 3 (2 g, 3.9 mmol) and compound 4 (3.14 g, 19.7 mmol) in DMSO (10 ml) was added KF (1.15 g, 19.7 mmol). The reaction mixture was stirred at 120 °C for 12 h. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (PE/EA=1/1) to give compound 5 (1.1 g, Yield: 45 %) as a white solid. MS m/z (ESI): 628.1 [M+H]⁺.

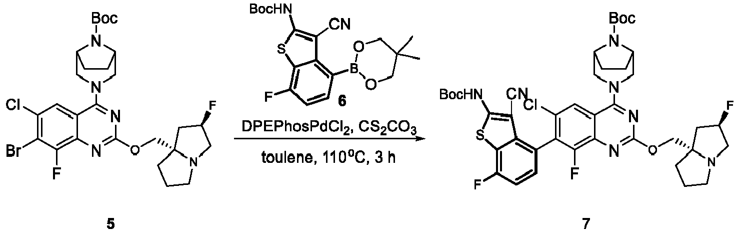

To a solution of compound 5 (400 mg, 0.64 mmol), compound 2 (400 mg, 0.99 mmol), and Cs₂CO₃ (622 mg, 1.91 mmol) in toluene (5 mL) was added Pd(DPEPhos)Cl₂ (92 mg, 0.13 mmol). The mixture was stirred at 110 °C under nitrogen for 3 hours. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The residue was purified by silica gel column (PE/EA=1/1) to give compound 7 (400 mg, Yield: 74.8 %) as a white solid. MS m/z (ESI): 840.4 [M+H]⁺.

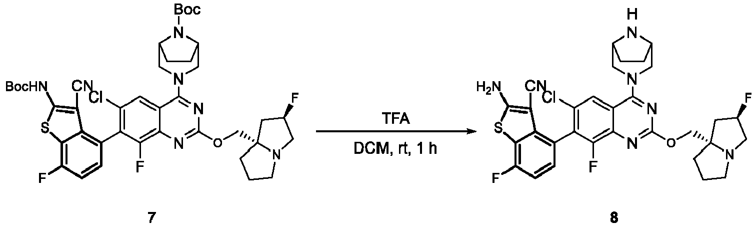

To a solution of compound 7 (100 mg, 0.12 mmol) in DCM (2 ml) was added TFA (1 ml). The reaction mixture was stirred at 20 °C for 2 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated to afford compound 8 (80 mg, 95%) as a yellow solid. MS m/z (ESI): 640.1 [M+H]⁺.

¹H NMR (400 MHz, DMSO-*d₆*): δ 8.08 (s, 2H), 7.84 (s, 1H), 7.26 (dd, *J* = 8.4, 5.2 Hz, 1H), 7.18 - 7.09 (m, 1H), 5.27 (d, *J* = 54.0 Hz, 1H), 4.32 - 4.22 (m, 2H), 4.08 (d, *J* = 10.4 Hz, 1H), 3.97 (d, *J=* 2.8 Hz, 1H), 3.60 - 3.40 (m, 4H), 3.08 (d, *J=* 8.4 Hz, 2H), 3.01 (s, 1H), 2.85 - 2.75 (m, 1H), 2.16 - 1.50 (m, 10H).

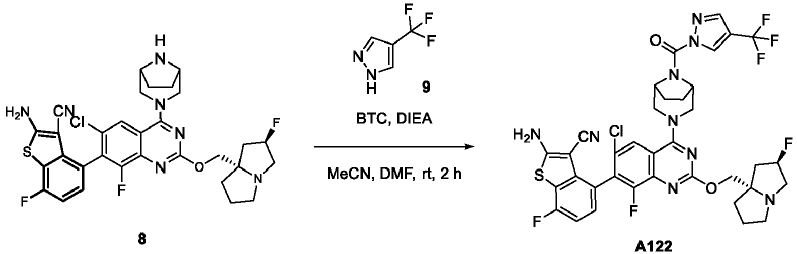

To a solution of compound 9 (13 mg, 0.09 mmol) in MeCN (0.5 mL) was added DIEA (36 mg, 0.28 mmol) and Triphosgene (25 mg, 0.08 mmol) under N₂ at 0 °C. The mixture was stirred at r.t for 2 h. Then the mixture was added to compound 8 (60 mg, 0.09 mmol) in MeCN (0.5 ml) and the reaction stirred under N₂ for 10 min. The mixture was poured into water (20 mL), and the solution was extracted with ethyl acetate (25 mL x 3). The organic layer was washed with brine (25 mL), dried over anhydrous sodium sulfate, filtered and the filtrate was concentrated to dryness under reduced pressure. The residue was purified by Prep-HPLC to give compound A122 (2.85 mg, yield: 4 %) as a white solid. MS m/z (ESI): 802.2 [M+H]⁺.

¹H NMR (400 MHz, DMSO-*d₆*): δ 8.98 (s, 1H), 8.32 (s, 1H), 8.09 (s, 2H), 7.89 (s, 1H), 7.3 - 7.2 (m, 1H), 7.2 - 7.1 (m, 1H), 5.28 (d, *J* = 54.8 Hz, 1H), 4.63 - 4.53 (m, 1H), 4.45 - 4.35 (m, 1H), 4.11 (d, *J=* 10.4 Hz, 1H), 4.03 (d, *J* = 10.4 Hz, 1H), 3.96 - 3.86 (m, 1H), 3.80 - 3.68 (m, 1H), 3.35 - 3.33 (m, 1H), 3.30 - 3.26 (m, 1H), 3.14 - 3.06 (m, 2H), 3.02 - 2.99 (m, 1H), 2.85 - 2.80 (m, 1H), 2.20 - 1.96 (m, 5H), 1.87 - 1.73 (m, 5H).

### Synthesis of Compound A123

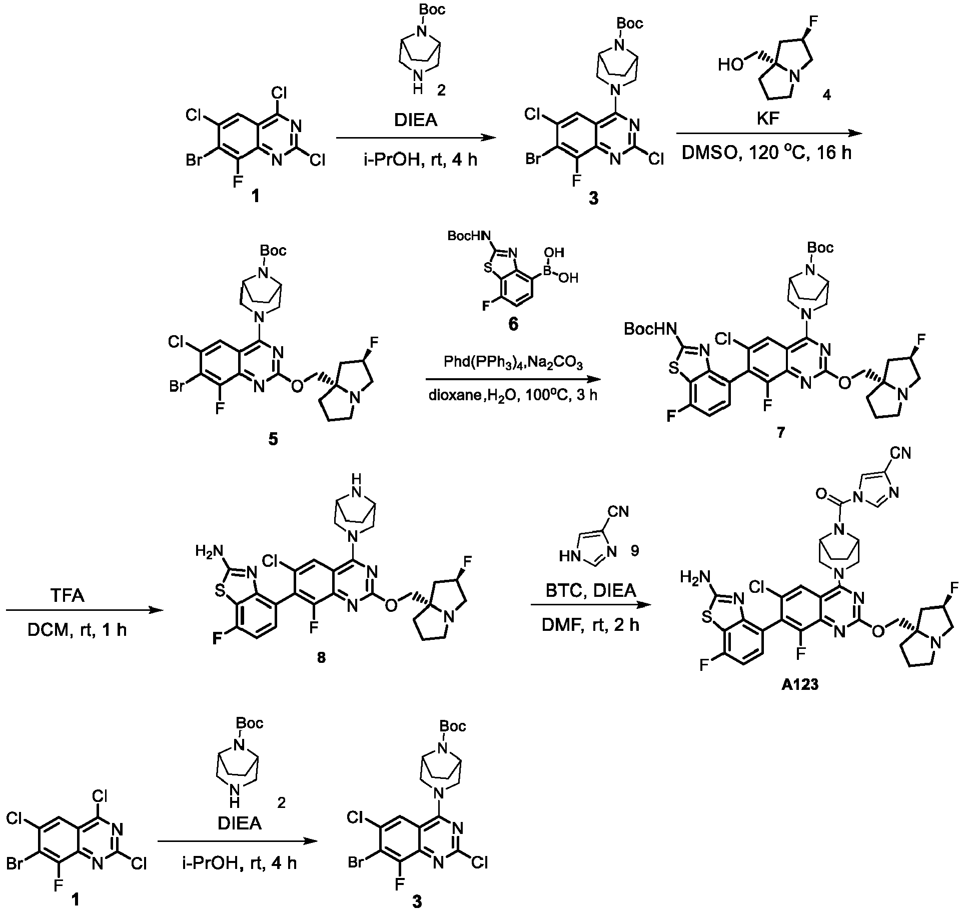

To a solution of compound 1 (5 g, 15.25 mmol) and compound 2 (3.2 g, 15.25 mmol) in i-PrOH (70 ml) was added DIEA (5.9 g 45.8 mmol). The reaction mixture was stirred at 25 °C for 4 h. LCMS showed the reaction was completed. The mixture was poured into ice water (200 mL). The mixture was filtered and the solid was diluted with MeOH (150 ml). The organic solution was concentrated to dryness under reduced pressure to give compound 3 (5.1 g, Yield: 66 %) as a white solid. MS m/z (ESI): 505.2 [M+H]⁺.

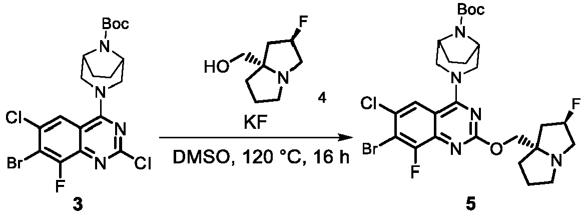

To a solution of compound 3 (2 g, 3.9 mmol) and 4 (3.14 g, 19.7 mmol,) and KF (1.15 g, 19.7 mmol) in DMSO (10 ml). The reaction mixture was then stirred at 120 °C for 16 h. LCMS showed the reaction was completed. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The crude was purified by SGC (PE/EA=1/1) to give compound 5 (1.1 g, Yield: 45 %) as a white solid. MS m/z (ESI): 628.9 [M+H]⁺.

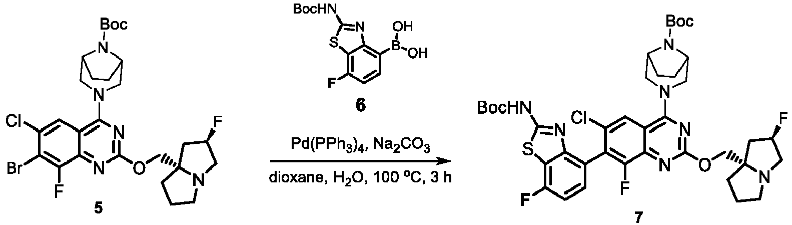

To a solution of compound 5 (3.7 g, 5.90 mmol), compound 6 (3.68 g, 11.8 mmol), and Na₂CO₃ (1.25 g, 11.8 mmol) in 1,4-dioxane/H₂O (40 mL/ 10 mL) was added Pd(PPh₃)₄ (1.36 g, 1.18 mmol). The mixture was stirred at 100 °C under nitrogen for 3 hours. LCMS showed the reaction was completed. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The crude was purified by SGC (PE/EA=1/1) to give compound 7 (2.5 g, Yield: 52 %) as a white solid.

MS m/z (ESI): 816.1 [M+H]⁺.

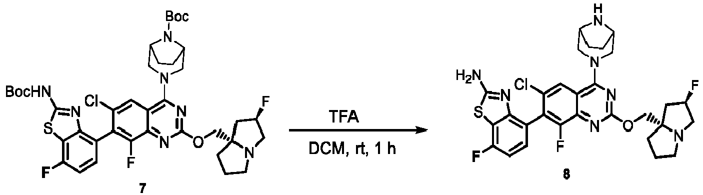

To a solution of compound 7 (2.5 g, 3.06 mmol) in DCM (8 ml) was added TFA (8 ml). The reaction mixture was stirred at rt for 1 h. LCMS showed the reaction was completed. The reaction mixture was concentrated under the reduced pressure. The residue was diluted with dichloromethane (100 mL). The solution was basified with NaHCO₃. The organic layer was washed with brine, dried over sodium sulfate, filtered, and concentrated to dryness under reduced pressure to give compound 8 (1.5 g, Yield: 79 %) as a white solid. MS m/z (ESI): 616.2 [M+H]⁺.

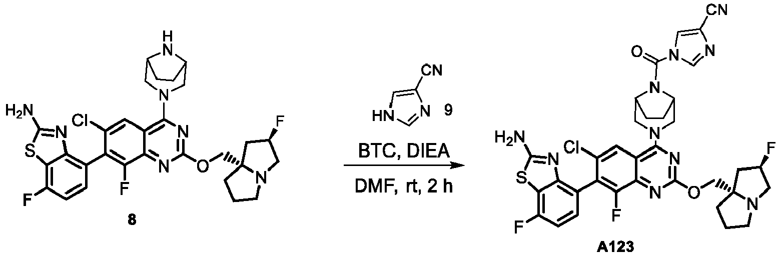

To a solution of compound 9 (303 mg, 3.25 mmol) and DIEA (1.7 mL, 9.76 mmol) in 18 mL MeCN, was added Triphosgen (322 mg, 1.084 mmol) at 0 °C under N₂ atmosphere. The reaction mixture was then stirred at rt for 2 h. To a solution of compound 8 (70 mg, 0.114 mmol) and DIEA (44 mg, 0.342 mmol) in DMF (1 mL), was added above reaction mixture (1.5 mL) at rt under N₂ atmosphere. LCMS showed the reaction was completed. The crude was purified by prep-HPLC to give A123 (6.62 mg, Yield: 5.2 %). MS:735.3 [M+H]⁺.

¹H NMR (400 MHz, DMSO-*d₆*): δ 8.62 (d, J = 1.2 Hz, 1H), 8.42 (d, J = 1.2 Hz, 1H), 7.93 (s, 1H), 7.90 (s, 2H), 7.22 (dd, J = 8.4, 5.6 Hz, 1H), 7.08 (t, J = 8.8 Hz, 1H), 5.57 (d, J = 53.2 Hz, 1H), 4.70 - 4.40 (m, 6H), 3.90 - 3.50 (m, 6H), 2.34 - 1.72 (m, 10H).

### Synthesis of Compound A135

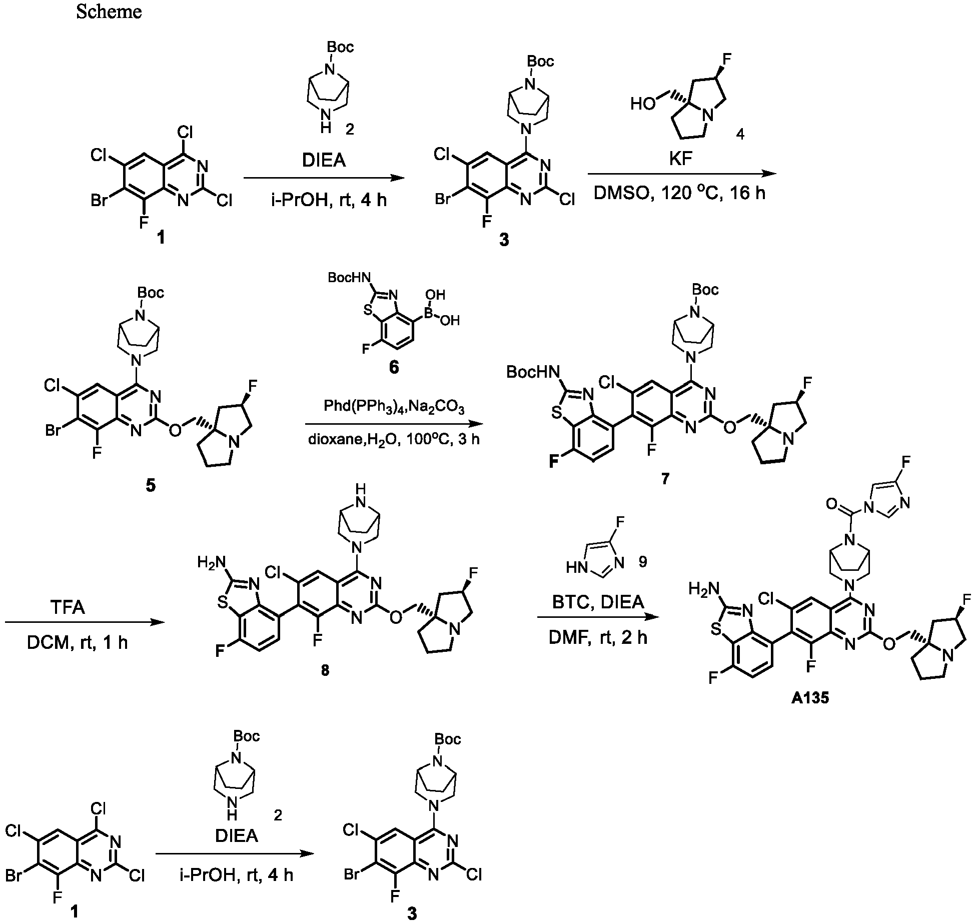

To a solution of compound 1 (5 g, 15.25 mmol) and compound 2 (3.2 g, 15.25 mmol) in i-PrOH (70 ml) was added DIEA (5.9 g 45.8 mmol). The reaction mixture was stirred at 25 °C for 3 h. LCMS showed the reaction was completed. The mixture was poured into ice water (200 mL). The mixture was filtered and the solid was diluted with MeOH (150 ml). The organic solution was concentrated to dryness under reduced pressure to give compound 3 (5.1 g, Yield: 66 %) as a white solid. MS m/z (ESI): 505.2 [M+H]⁺.

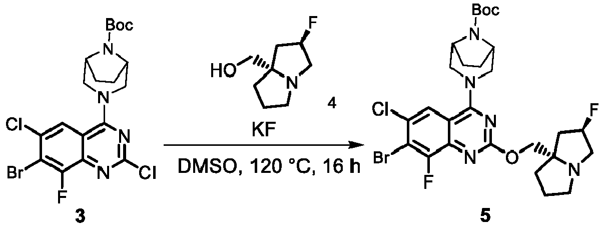

To a solution of compound 3 (2 g, 3.9 mmol) and 4 (3.14 g, 19.7 mmol,) and KF (1.15 g, 19.7 mmol) in DMSO (10 ml). The reaction mixture was then stirred at 120 °C for 16 h. LCMS showed the reaction was completed. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The crude was purified by SGC (PE/EA=1/1) to give compound 5 (1.1 g, Yield: 45 %) as a white solid. MS m/z (ESI): 628.9 [M+H]⁺.

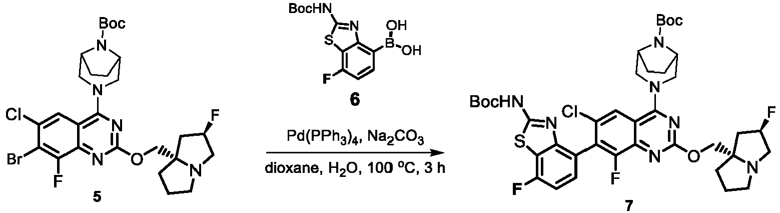

To a solution of compound 5 (3.7 g, 5.90 mmol), compound 6 (3.68 g, 11.8 mmol), and Na₂CO₃ (1.25 g, 11.8 mmol) in 1,4-dioxane/H₂O (40 mL/ 10 mL) was added Pd(PPh₃)₄ (1.36 g, 1.18 mmol). The mixture was stirred at 100 °C under nitrogen for 3 hours. LCMS showed the reaction was completed. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The crude was purified by SGC (PE/EA=1/1) to give compound 7 (2.5 g, Yield: 52 %) as a white solid.

MS m/z (ESI): 816.1 [M+H]⁺.

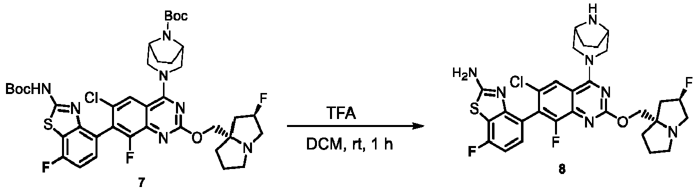

To a solution of compound 7 (2.5 g, 3.06 mmol) in DCM (8 ml) was added TFA (8 ml). The reaction mixture was stirred at rt for 1 h. LCMS showed the reaction was completed. The reaction mixture removed under the reduced pressure. The residue was diluted with dichloromethane (100 mL). The solution was basified with NaHCO₃. The organic layer was washed with brine, dried over sodium sulfate, filtered, and concentrated to dryness under reduced pressure to give compound 8 (1.5 g, Yield: 79 %) as a white solid. MS m/z (ESI): 616.2 [M+H]⁺.

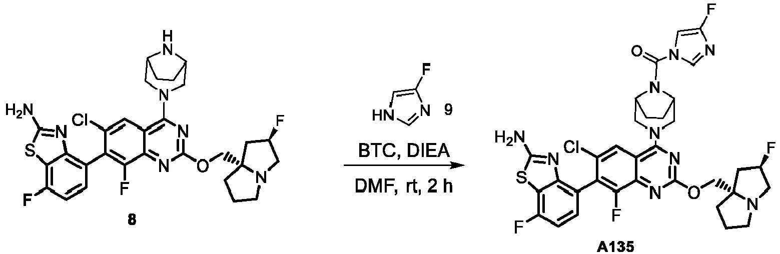

To a solution of compound 9 (168 mg, 1.95 mmol) and DIEA (1.35 mL, 7.8 mmol) in 18 mL MeCN, was added BTC (521 mg, 1.756) at 0 °C under N₂ atmosphere. The reaction mixture was then stirred at rt for 2 h. To a solution of compound 8 (60 mg, 0.098 mmol) and DIEA (38 mg, 0.294 mmol) in DMF (1 mL), was added above reaction mixture (1.5 mL) at rt under N₂ atmosphere. LCMS showed the reaction was completed. The crude was purified by prep-HPLC to give A135 (6.07 mg, Yield: 8.5 %). MS:728.2 [M+H]⁺.

¹H NMR (400 MHz, DMSO-*d₆*): δ 7.99 (s, 1H), 7.94 (s, 1H), 7.90 (s, 2H), 7.41 (dd, J = 8.2, 1.8 Hz, 1H), 7.22 (dd, J = 8.4, 5.6 Hz, 1H), 7.08 (t, J = 8.8 Hz, 1H), 5.57 (d, J = 50.8 Hz, 1H), 4.65 - 4.44 (m, 6H), 3.90 - 3.60 (m, 3H), 3.17 (s, 3H), 2.40 - 1.68 (m, 10H).

### Synthesis of Compound A141

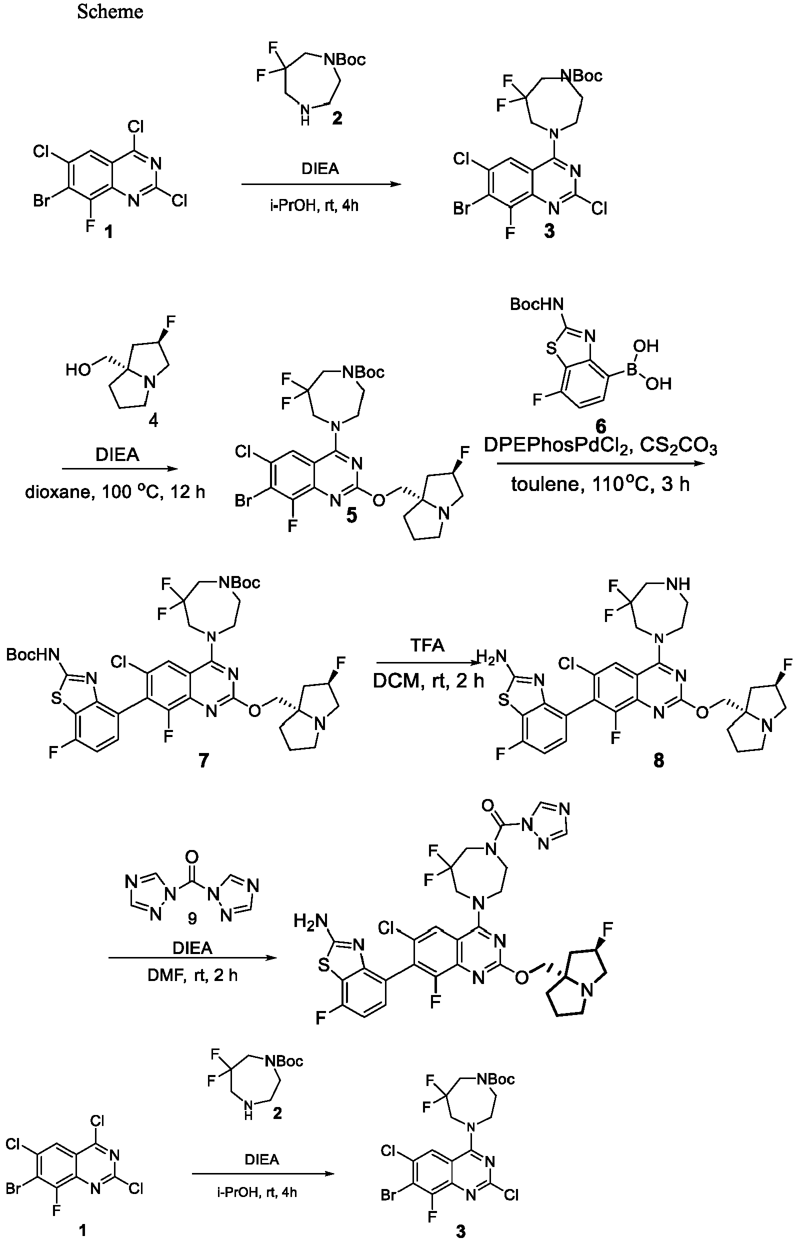

To a solution of compound 1 (650 mg, 1.8 mmol) and compound 2 (500 mg, 2.0 mmol) in i-PrOH (8 ml) was added DIEA (600 mg, 5.0 mmol). The reaction mixture was stirred at 25 °C for 4 h. The mixture was poured into ice water (200 mL). The mixture was filtered and the solid was diluted with MeOH (150 ml). The organic solution was concentrated to dryness under reduced pressure to give compound 3 (760 mg, Yield: 78%) as a white solid. MS m/z (ESI): 530.2 [M+H]⁺.

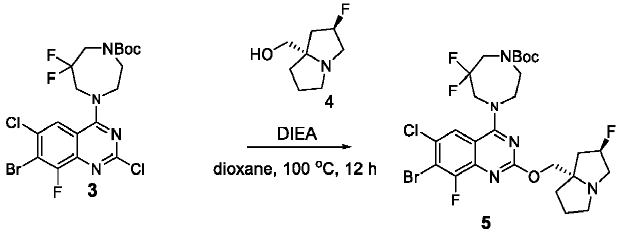

A mixture of compound 3 (500 mg, 1.0 mmol), compound 4 (710 mg, 4.2 mmol) and DIEA (583.04 mg, 4.51 mmol) in dioxane (10 ml) was stirred at 100 °C for 12 h. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (PE/EA=1/1) to give compound 5 (410 mg, Yield: 50.8 %) as a white solid. MS m/z (ESI): 654.3 [M+H]⁺.

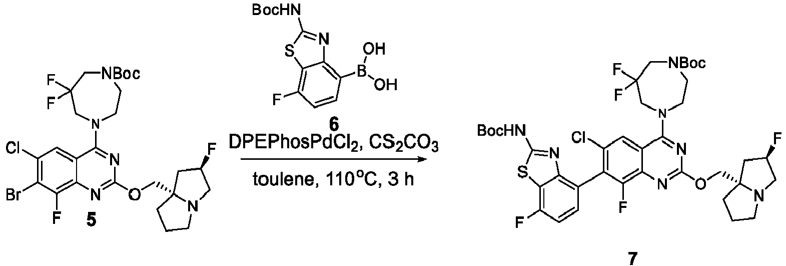

To a solution of compound 5 (350 mg, 0.45 mmol), compound 6 (400 mg, 1.0 mmol), and Cs₂CO₃ (671 mg, 2.06 mmol) in toluene (8 mL) was added Pd(DPEPhos)Cl₂ (98 mg, 0.14 mmol). The mixture was stirred at 110 °C under nitrogen for 3 hours. The mixture was diluted with EtOAc (100 mL) and water (100 ml). The organic layer was washed with brine (50 mL), dried over Na₂SO₄, filtered and concentrated. The residue was purified by silica gel column (PE/EA=1/1) to give compound 7 (280 mg, Yield: 50%) as a yellow solid. MS m/z (ESI): 841.31 [M+H]⁺.

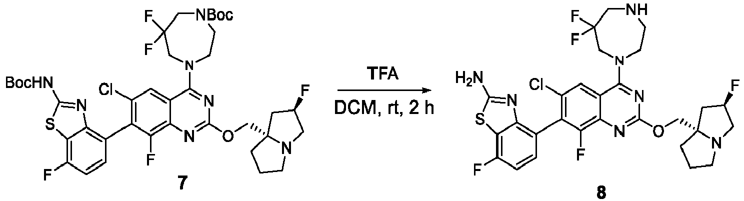

To a solution of compound 7 (250 mg, 0.3 mmol) in DCM (10 ml) was added TFA (10 ml). The reaction mixture was stirred at rt for 2 h. The solvent was removed under reduced pressure. The reaction mixture was diluted with a.q. NaHCO₃ (20 mL) and DCM (50 mL). The organic layer was washed with brine (50 mL), dried over anhydrous sodium sulfate, filtered and the filtrate was concentrated to dryness under reduced pressure. The residue was purified by Pre-HPLC (FA) to give compound 8 (180 mg, Yield: 93.4 %) as a white solid. MS m/z (ESI): 741.2 [M+H]⁺.

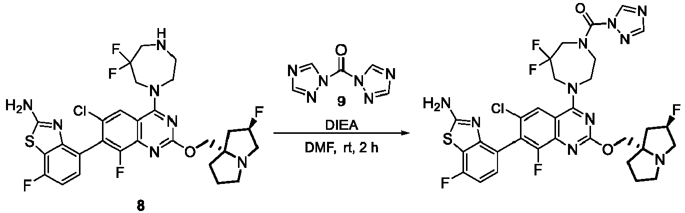

To a solution of compound 8 (50 mg, 0.1 mmol) and DIEA (40 mg, 0.1 mmol) in 0.8 mL DMF, was added compound 9 (40 mg, 0.1 mmol). The reaction mixture was then stirred at rt for 1 h. LCMS showed the reaction was completed. The crude was purified by prep-HPLC to give compound A141 (15 mg, Yield: 30 %). MS m/z (ESI): 735.14 [M+H]⁺.

¹H NMR (400 MHz, DMSO-*d₆*): δ 8.17 (s, 1H), 8.10 (s, 1H), 8.02 (s, 1H), 7.29 - 7.12 (m, 2H), 3.58 (m, 3H), 4.73 - 3.91 (m, 6H), 2.43 - 1.57 (m, 10H).

### Synthesis of Compound A146

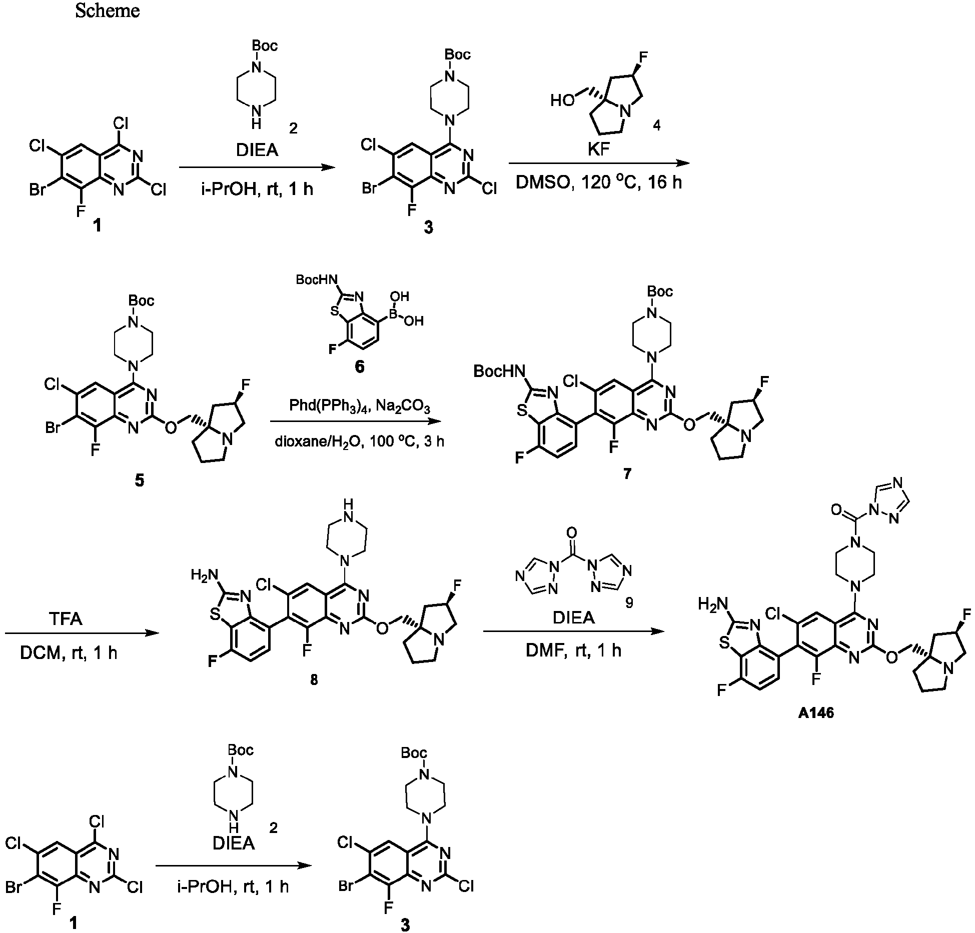

To a solution of compound 1 (5 g, 6.1 mmol) and compound 2 (1.14 g, 6.1 mmol) in i-PrOH (15 ml) was added DIEA (2.36 g, 18.3 mmol). The reaction mixture was stirred at 25 °C for 1 h. LCMS showed the reaction was completed. The mixture was poured into ice water (200 mL). The mixture was filtered and the solid was diluted with PE (150 ml). The organic solution was concentrated to dryness under reduced pressure to give compound 3 (4.0g, Yield: 69%) as a white solid. MS m/z (ESI): 479.0 [M+H]⁺.

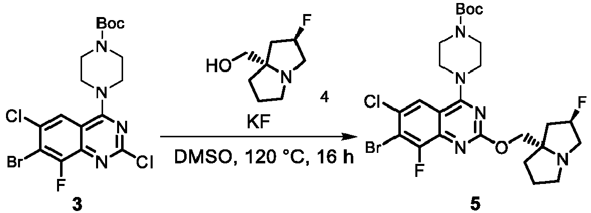

To a solution of compound 3 (2 g, 4.18 mmol) and compound 4 (3.33 g, 20.9 mmol) and KF (1.21 g, 20.9 mmol) in DMSO (10 mL). The reaction mixture was then stirred at 120 °C for 16 h. LCMS showed the reaction was completed. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The crude was purified by SGC (PE/EA=1/1) to give compound 5 (700 mg, Yield: 28 %) as a yellow solid. MS m/z (ESI): 602.6 [M+H]⁺.

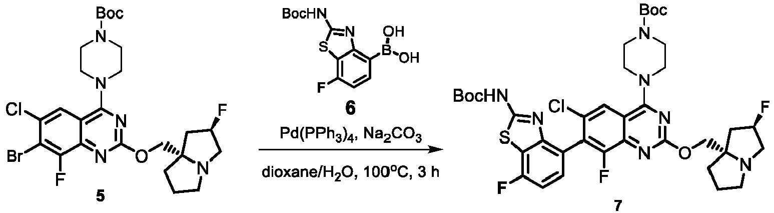

To a solution of compound 5 (500 mg, 0.83 mmol), compound 6 (519 mg, 1.66 mmol), and Na₂CO₃ (176 mg, 1.66 mmol) in 1,4-dioxane/ H₂O (12 mL/3 mL) was added Pd(PPh₃)₄ (192 mg, 0.17 mmol). The mixture was stirred at 100 °C under nitrogen for 3 hours. LCMS showed the reaction was completed. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The crude was purified by SGC (PE/EA=1/1) to give compound 7 (250 mg, Yield: 38 %) as a white solid. MS m/z (ESI): 790.5 [M+H]⁺.

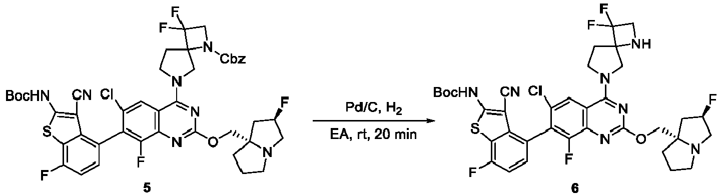

To a solution of compound 7 (80 mg, 0.10 mmol) in DCM (1 mL) was added TFA (1 mL). The reaction mixture was stirred at rt for 1 h. LCMS showed the reaction was completed. The reaction mixture removed under the reduced pressure. The residue was diluted with dichloromethane (100 mL). The solution was basified with NaHCO₃. The organic layer was washed with brine, dried over sodium sulfate, filtered, and concentrated to dryness under reduced pressure to give compound 8 (55 mg, Yield: 93 %) as a white solid. MS m/z (ESI): 590.7 [M+H]⁺.

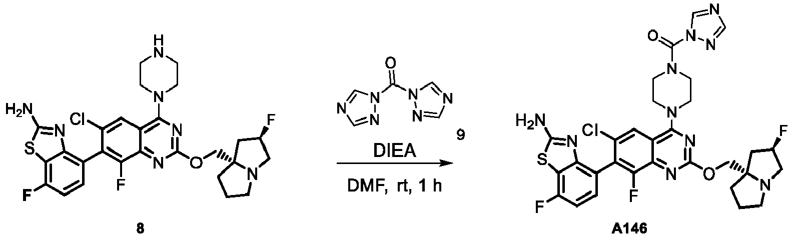

To a solution of compound 8 (90 mg, 0.153 mmol) and DIEA (59 mg, 0.459 mmol) in 1 mL DMF, was added compound 9 (75 mg, 0.459 mmol). The reaction mixture was then stirred at rt for 1 h. LCMS showed the reaction was completed. The crude was purified by prep-HPLC to give A146 (12.79 mg, Yield: 12 %). MS: 685.3 [M+H]⁺.

¹H NMR (400 MHz, DMSO-*d*₆) δ 9.12 (s, 1H), 8.29 (s, 1H), 8.26 (s, 1H), 7.94 (d, J = 5.8 Hz, 3H), 7.23 (dd, J = 7.6, 5.6 Hz, 1H), 7.06 (t, J = 8.8 Hz, 1H), 5.28 (d, J = 54.2 Hz, 1H), 4.24 - 3.88 (m, 11H), 3.13 - 2.90 (m, 4H), 2.12 - 1.61 (m,6H).

### Synthesis of Compound A139

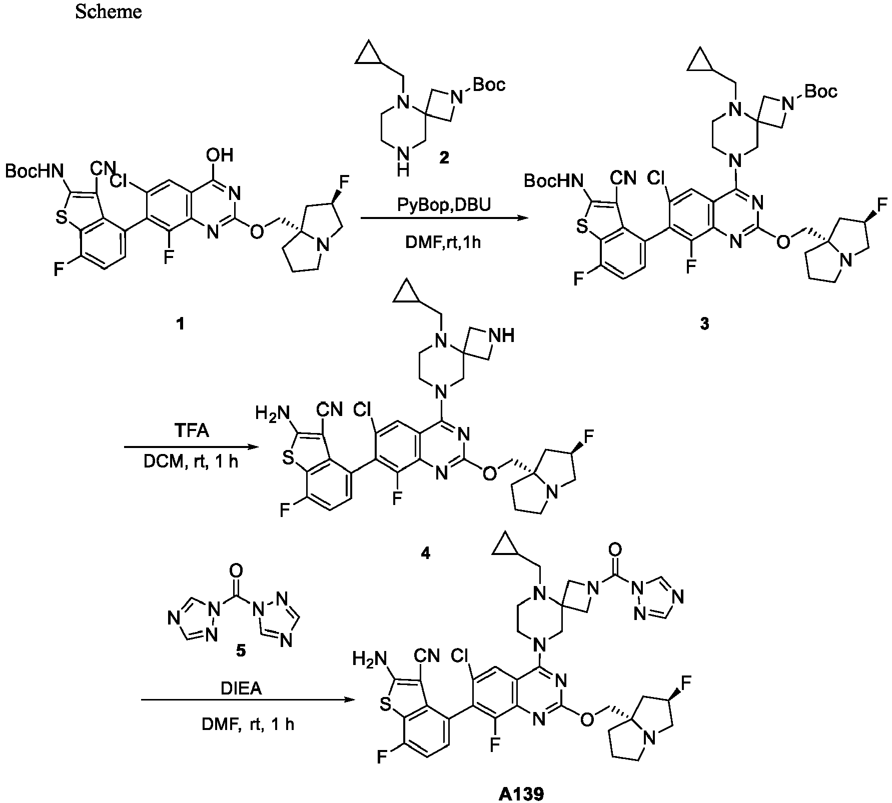

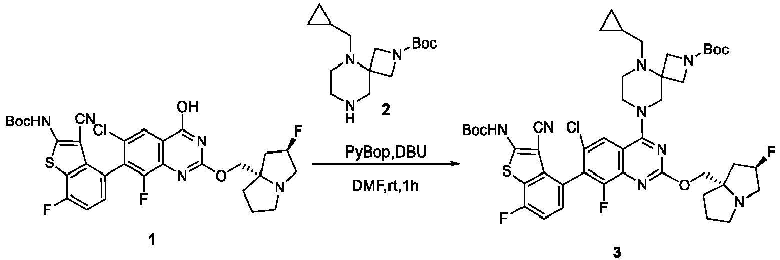

To a solution of compound 1 (150 mg, 0.23 mmol), compound 2 (85 mg, 0.30 mmol) and PyBop (180 mg, 0.35 mmol) in DMF (3 ml) was added DBU (43 mg, 0.35 mmol). The reaction mixture was stirred at 25 °C for 1 h. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by prep-TLC (DCM/MeOH=15/1) to give compound 3 (120 mg, Yield: 56.9 %) as a yellow solid.

MS m/z (ESI): 909.4 [M+H]⁺.

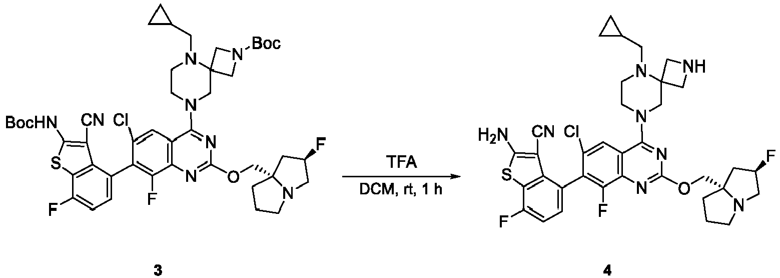

To a solution of compound 3 (120 mg, 0.13 mmol) in DCM (2 ml) was added TFA (2 ml). The reaction mixture was stirred at 20 °C for 3 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated. The residue was purified by work up to give compound 4 (110 mg, Yield: 100%) as a yellow solid.

MS m/z (ESI): 709.3 [M+H]⁺.

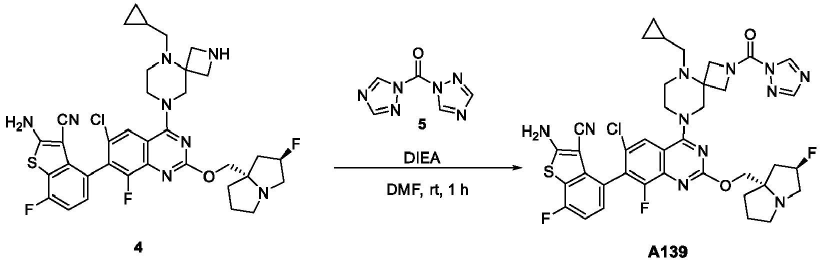

To a solution of compound 4 (40 mg, 0.06 mmol) and compound 5 (20 mg, 0.12 mmol) in 2 ml DMF was added DIEA (23 mg, 0.18 mmol) at rt. The reaction mixture was then stirred at rt for 30 min. The mixture was poured into water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by Prep-HPLC to give compound A139 (8.41 mg, Yield: 18.5%) as a white solid.

MS m/z (ESI): 804.2 [M+H]⁺.

¹H NMR (400 MHz, DMSO-*d₆*): δ 9.15 (d, J = 5.2 Hz, 1H), 8.23 (s, 1H), 8.12 (d, J = 12.0 Hz, 2H), 7.90 (s, 1H), 7.29 - 7.21 (m, 1H), 7.19 - 7.12 (m, 1H), 5.27 (d, J = 54.4 Hz, 1H), 4.58 (s, 1H), 4.47 - 4.35 (m, 1H), 4.24 - 3.75 (m, 8H), 3.16 - 2.78 (m, 6H), 2.69 - 2.56 (m, 2H), 2.18 - 1.91 (m, 3H), 1.85 - 1.65 (m, 3H), 0.92 (s, 1H), 0.49 (d, J = 8.0 Hz, 2H), 0.20 (d, J = 4.4 Hz, 2H).

### Synthesis of Compound A140

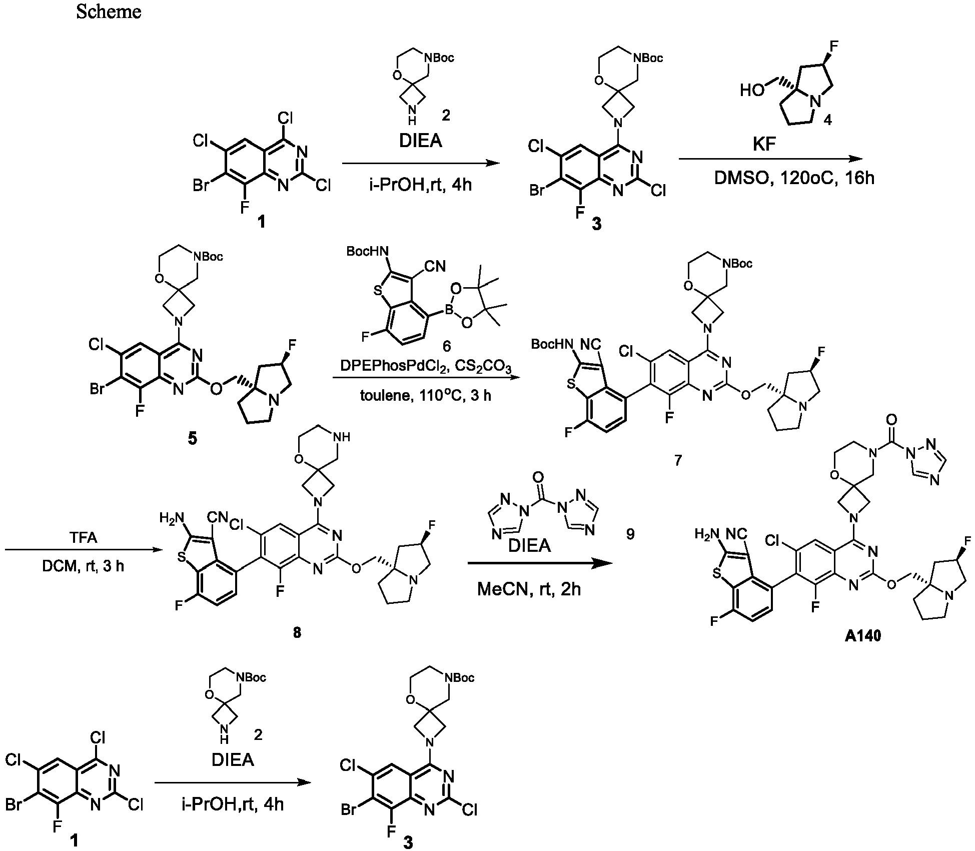

To a solution of compound 1 (1.1 g, 3.36 mmol) and compound 2 (765 mg, 3.36 mmol) in i-PrOH (12 ml) was added DIEA (1.3 g 10.07 mmol). The reaction mixture was stirred at 25 °C for 3 h. The mixture was filtered and the solid was diluted with MeOH (150 ml). The organic solution was concentrated to dryness under reduced pressure to give compound 3 (1.3 g, Yield: 75 %) as a yellow solid.

LCMS: Rt: 1.424 min; MS m/z (ESI): 521.0 [M+H]⁺.

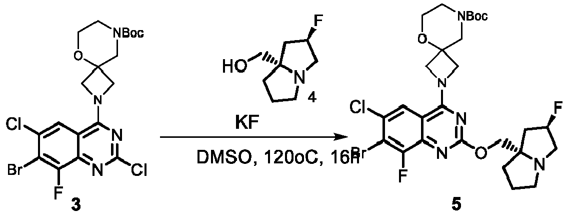

To a solution of compound 3 (1.3 g, 2.5 mmol) and 4 (1.99 g, 12.50 mmol) in DMSO (20 ml) was added KF (726 mg, 12.50 mmol). The reaction mixture was then stirred at 120 °C for 12 h. LCMS showed the reaction was completed. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The crude was purified by SGC (PE/EA=1/1) to give compound 5 (580 mg, Yield: 36 %) as a white solid.

LCMS: Rt: 1.489 min; MS m/z (ESI): 644.1 [M+H]⁺.

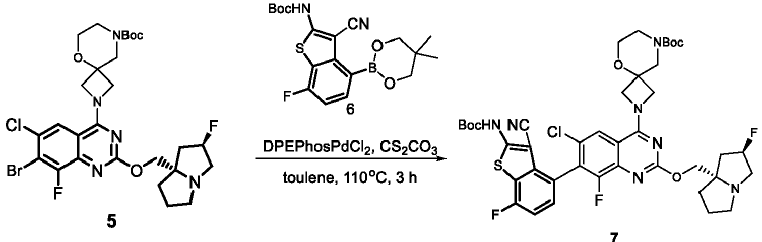

To a solution of compound 5 (400 mg, 0.62 mmol), compound 6 (375 mg, 0.93 mmol), and Cs₂CO₃ (607 mg, 1.86 mmol) in toluene (10 mL) was added Pd(DPEPhos)Cl₂ (90 mg, 0.124 mmol). The mixture was stirred at 110 °C under nitrogen for 3 hours. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The residue was purified by silica gel column (PE/EA=1/1) to give compound 7 (300 mg, Yield: 57%) as a white solid.

LCMS: Rt: 1.468 min; MS m/z (ESI): 856.2 [M+H]⁺.

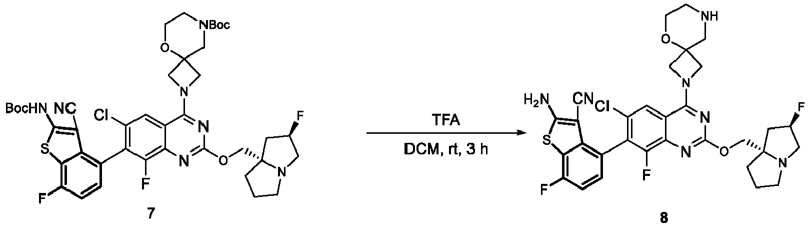

To a solution of compound 7 (300 mg, 0.35 mmol) in DCM (3 ml) was added TFA (3 ml). The reaction mixture was stirred at rt for 3 h. LCMS showed the reaction was completed. The reaction mixture removed under the reduced pressure. The residue was diluted with dichloromethane (100 mL). The solution was basified with NaHCO₃. The organic layer was washed with brine, dried over sodium sulfate, filtered, and concentrated to dryness under reduced pressure to give compound 8 (220 mg, Yield: 96 %) as a white solid.

LCMS: Rt: 1.045 min; MS m/z (ESI): 656.1 [M+H]⁺.

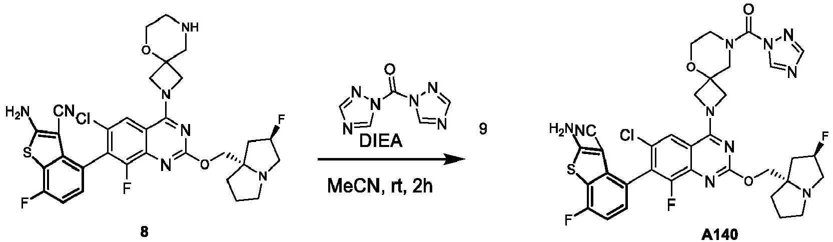

To a solution of compound 8 (50 mg, 0.076 mmol) and DIEA (30.00 mg, 0.23 mmol) in 1 mL DMF, was added compound 9 (31.30 mg, 0.19 mmol). The reaction mixture was then stirred at rt for 1 h. LCMS showed the reaction was completed. The crude was purified by prep-HPLC to give A140 (20.6 mg, Yield: 36 %).

LCMS: Rt : 1.278 min ; MS:751.2 [M+H]⁺.

¹H NMR (400 MHz, DMSO-*d₆*): δ 9.12 (s, 1H), 8.25 (s, 1H), 8.13 (s, 2H), 7.87 (s, 1H), 7.25 - 7.09 (m, 2H), 5.56 (d, *J* = 53.6 Hz, 1H), 5.20 - 4.30 (m, 7H), 3.81-3.26 (m, 9H), 2.45 - 2.00 (m, 6H).

### Synthesis of Compound A150

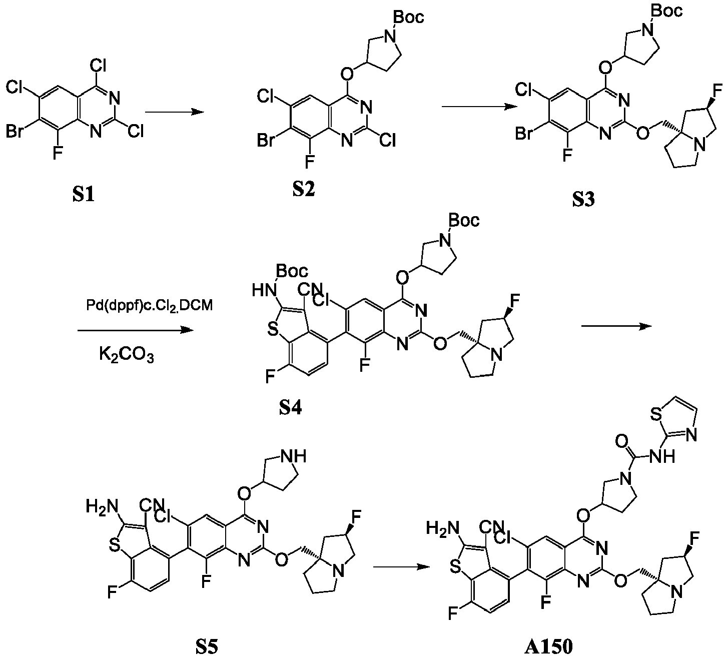

### tert-butyl 3-((7-bromo-2,6-dichloro-8-fluoroquinazolin-4-yl)oxy)pyrrolidine-1-carboxylate ( Step 1)

To a stirred solution of tert-butyl 3-hydroxypyrrolidine-1-carboxylate (2.57 g) and t-BuOK (3.4 g) in THF (30 mL) was added 7-bromo-2,4,6-trichloro-8-fluoroquinazoline (5 g) slowly at -78°C. The mixture was stirred for 1 hour at -78°C, then the mixture was extracted by EA and NaCl_{(aq)}. The extracts were combined, washed with brine and dried over sodium sulfate. It was filtered and solvent was removed under reduced pressure to give a residue. The residue was washed by 4% ethyl acetate/petroleum ether to get the desired product (6.2 g) ESI-MS *m*/*z:* 480.1 [M+H]⁺.

### tert-butyl 3-((7-bromo-6-chloro-8-fluoro-2-(((2R,7aS)-2-fluorotetrahydro-1H-pyrrolizin-7a(5H)-yl)methoxy)quinazolin-4-yl)oxy)pyrrolidine-1-carboxylate ( step 2)

To a stirred solution of tert-butyl 3-((7-bromo-2,6-dichloro-8-fluoroquinazolin-4-yl)oxy)pyrrolidine-1-carboxylate (3 g) and ((2R,7aS)-2-fluorotetrahydro-1H-pyrrolizin-7a(5H)-yl)methanol (1.5 g) in dioxane (20 mL) was added molecular sieve (500 mg) and DIEA (6 mL). The mixture was stirred for 16 hours at 100°C. It was then cooled to room temperature and the mixture was extracted by ethyl acetate and water. Organics were separated, washed with brine, dried over sodium sulfate. It was filtered and solvent was removed under reduced pressure to give a residue. The residue was purified by flash chromatography to afford the desired product (2.3 g) ESI-MS *m*/*z:* 603.2 [M+H]⁺.

### tert-butyl 3-((7-(2-((tert-butoxycarbonyl)amino)-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)oxy)pyrrolidine-1 - carboxylate (step 3)

To a stirred solution of tert-butyl 3-((7-bromo-6-chloro-8-fluoro-2-(((2R,7aS)-2-fluorotetrahydro-1H-pyrrolizin-7a(5H)-yl)methoxy)quinazolin-4-yl)oxy)pyrrolidine-1-carboxylate (1.5 g, 2.49 mmol) in Dioxane (25 mL) was added tert-butyl (3-cyano-4-(5,5-dimethyl-1,3,2-dioxaborinan-2-yl)-7-fluorobenzo[b]thiophen-2-yl)carbamate (1.51 g, 3.74 mmol), K₂CO₃ (1.7 g, 12.5 mmol) and Pd(dppf)Cl₂.DCM (205 mg) under argon. It was degassed for 3 times and back filled with argon. The mixture was stirred overnight at 105°C under argon. It was cooled to room temperature and extracted with ethyl acetate. The organics were combined and washed with brine, dried over sodium sulfate. It was filtered and the solvent was removed to give a residue. The residue was purified by flash chromatography to afford the desired product. ESI-MS *m*/*z:* 815.2 [M+H]⁺.

### 2-amino-4-(6-chloro-8-fluoro-2-(((2R,7aS)-2-fluorotetrahydro-1H-pyrrolizin-7a(5H)-yl)methoxy)-4-(pyrrolidin-3-yloxy)quinazolin-7-yl)-7-fluorobenzo[b]thiophene-3-carbonitrile ( step 4)

To a stirred solution of tert-butyl 3-((7-(2-((tert-butoxycarbonyl)amino)-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)oxy)pyrrolidine-1-carboxylate (445 mg) in DCM (4 mL) was added TFA (2 mL). The mixture was stirred for 2 hours at room temperature. The solvent was removed, the residue was extracted by ethyl acetate and washed with NaHCO_{3(aq)}. The organics were dried over sodium sulfate. It was filtered and the solvent was removed to get the desired product which was used in the next step without further purification. ESI-MS *m*/*z:* 615.1 [M+H]⁺.

### 3-((7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)oxy)-N-(thiazol-2-yl)pyrrolidine-1-carboxamide ( step5)

To a stirred solution of thiazol-5-amine (15 mg, 0.15 mmol) in DCM (15 mL) was added Et₃N (0.1 mL) and Triphosgene (22 mg, 0.074 mmol) at room temperature. The mixture was stirred for 30 min, then 2-amino-4-(6-chloro-8-fluoro-2-(((2R,7aS)-2-fluorotetrahydro-1H-pyrrolizin-7a(5H)-yl)methoxy)-4-(pyrrolidin-3-yloxy)quinazolin-7-yl)-7-fluorobenzo[b]thiophene-3-carbonitrile (100 mg, 0.16 mmol) was added, the mixture was stirred for additional 30 min at room temperature. The mixture was extracted by DCM and water. The solvent was removed, the residue was purified by flash chromatography to afford the product. ESI-MS *m*/*z:* 741.2 [M+H]⁺, ¹H NMR(400 MHz, CD₃OD): 7.94-7.92 (m, 1 H), 7.23-7.22 (m, 1 H), 7.12-7.09 (m, 1 H), 6.96~6.91 (m, 1 H), 6.87~6.86 (m, 1 H), 5.86 (brs, 1 H), 5.39-5.26 (m, 1 H), 4.45-4.35 (m, 2 H), 3.89 (brs, 2 H) 3.75-3.70 (m, 2 H), 3.43-3.38 (m, 2 H), 3.11 (brs, 1 H), 2.41-2.27 (m, 4 H), 2.04-1.94 (m, 4 H), 1.23-1.19 (m, 2 H).

### Synthesis of Compound A153

### Scheme

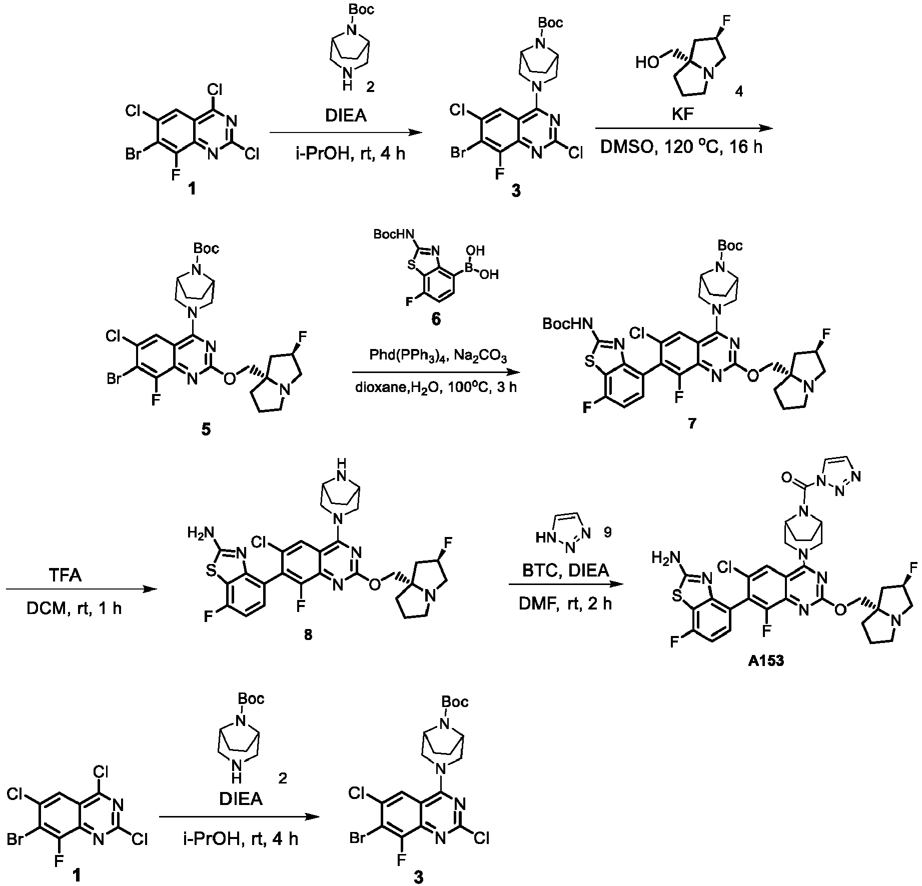

To a solution of compound 1 (5 g, 15.25 mmol) and compound 2 (3.2 g, 15.25 mmol) in i-PrOH (70 ml) was added DIEA (5.9 g 45.8 mmol). The reaction mixture was stirred at 25 °C for 4 h. LCMS showed the reaction was completed. The mixture was poured into ice water (200 mL). The mixture was filtered and the solid was diluted with MeOH (150 ml). The organic solution was concentrated to dryness under reduced pressure to give compound 3 (5.1 g, Yield: 66 %) as a white solid. MS m/z (ESI): 505.2 [M+H]⁺.

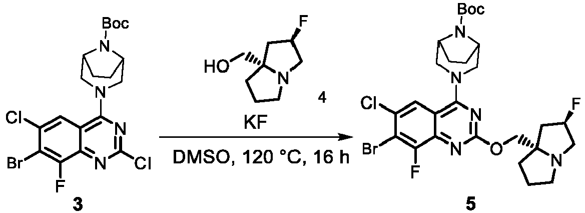

To a solution of compound 3 (2 g, 3.9 mmol) and 4 (3.14 g, 19.7 mmol,) and KF (1.15 g, 19.7 mmol) in DMSO (10 ml). The reaction mixture was then stirred at 120 °C for 16 h. LCMS showed the reaction was completed. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The crude was purified by SGC (PE/EA=1/1) to give compound 5 (1.1 g, Yield: 45 %) as a white solid. MS m/z (ESI): 628.94 [M+H]⁺.

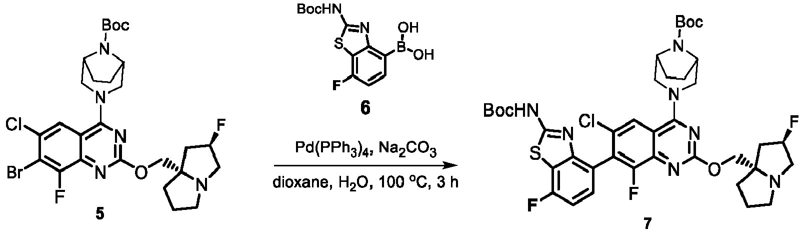

To a solution of compound 5 (3.7 g, 5.90 mmol), compound 6 (3.68 g, 11.8 mmol), and Na₂CO₃ (1.25 g, 11.8 mmol) in 1,4-dioxane/H₂O (40 mL/10 mL) was added Pd(PPh₃)₄ (1.36 g, 1.18 mmol). The mixture was stirred at 100 °C under nitrogen for 3 hours. LCMS showed the reaction was completed. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The crude was purified by SGC (PE/EA=1/1) to give compound 7 (2.5 g, Yield: 52 %) as a white solid.

MS m/z (ESI): 816.1 [M+H]⁺.

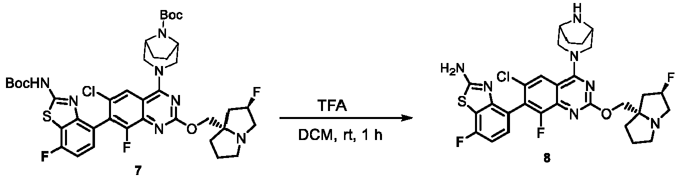

To a solution of compound 7 (2.5 g, 3.06 mmol) in DCM (8 ml) was added TFA (8 ml). The reaction mixture was stirred at rt for 1 h. LCMS showed the reaction was completed. The reaction mixture removed under the reduced pressure. The residue was diluted with dichloromethane (100 mL). The solution was basified with NaHCO₃ (100 mL). The organic layer was washed with brine (100 mL), dried over sodium sulfate, filtered, and concentrated to dryness under reduced pressure to give compound 8 (1.5 g, Yield: 79 %) as a white solid. MS m/z (ESI): 616.2 [M+H]⁺.

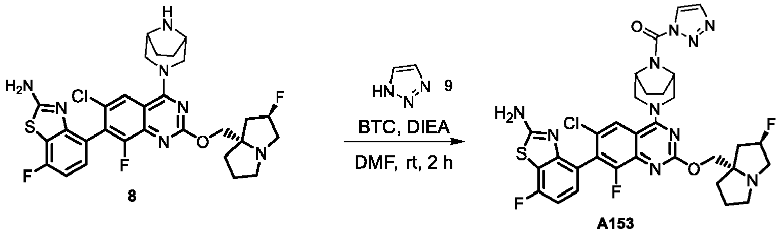

To a solution of compound 9 (141 mg, 2.037 mmol) and DIEA (2.6 g, 20.37 mmol) in 18 mL MeCN, was added BTC (181 mg, 0.611 mmol) at 0 °C under N₂ atmosphere. The reaction mixture was then stirred at rt for 2 h. To a solution of compound 8 (70 mg, 0.114 mmol) and DIEA (44 mg, 0.342 mmol) in DMF (1 mL), was added above reaction mixture (1.5 mL) at rt under N₂ atmosphere. LCMS showed the reaction was completed. The crude was purified by prep-HPLC to give A153 (4.41 mg, Yield: 5.4 %). MS: 711.2 [M+H]⁺.

¹H NMR (400 MHz, DMSO-*d₆*): δ 8.65 and 7.97 (d, 1H), 8.20 (s, 1H), 7.92 (s, 2H), 7.90 (s, 1H), 7.37 - 7.16 (m, 1H), 7.06 (t, J = 8.8 Hz, 1H), 5.28 (d, J = 53.4 Hz, 1H), 5.10 - 4.25 (m, 4H), 4.22 - 3.74 (m, 4H), 3.18 - 2.90 (m, 4H), 2.22 - 1.65 (m, 10H).

### Synthesis of Compound A178

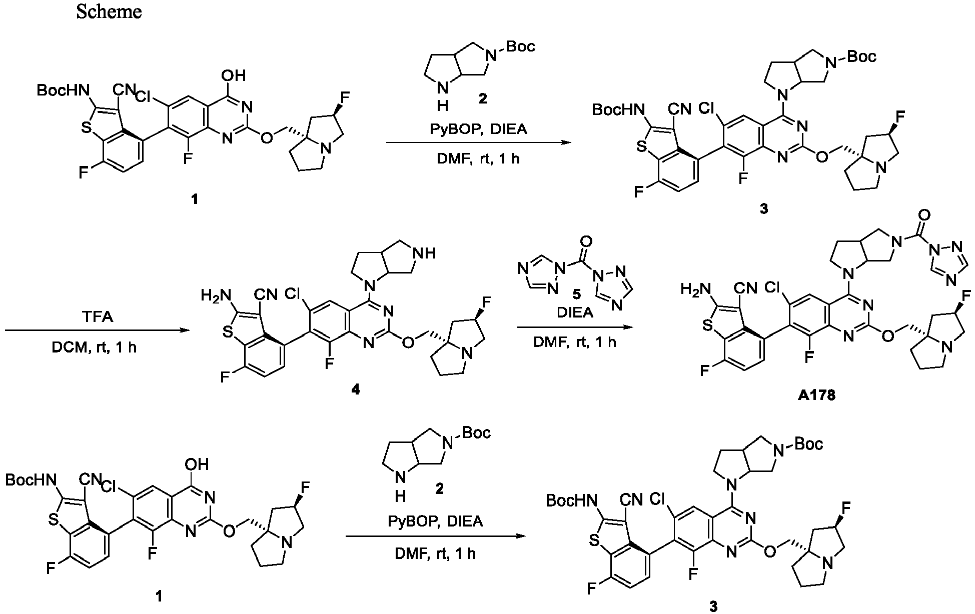

To a solution of compound 1 (150 mg, 0.23 mmol), compound 2 (64 mg, 0.30 mmol) and PyBop (182 mg, 0.35 mmol) in DMF (2 ml) was added DIEA (60 mg, 0.46 mmol). The reaction mixture was stirred at 25 °C for 1 h. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by prep-TLC (DCM/MeOH=15/1) to give compound 3 (150 mg, Yield: 76.9 %) as a yellow solid.

MS m/z (ESI): 840.2 [M+H]⁺.

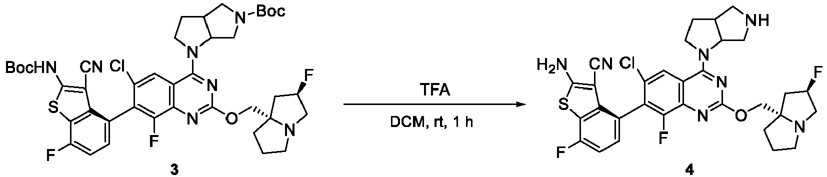

To a solution of compound 3 (150 mg, 0.18 mmol) in DCM (2 ml) was added TFA (2 ml). The reaction mixture was stirred at 20 °C for 1 h. The solvent was removed under reduced pressure. The reaction mixture was diluted with a.q. NaHCO₃ (80 mL) and extracted with DCM:MeOH=10:1 (50 x 3 mL). The organic layer was washed with brine, dried over sodium sulfate, filtered, and concentrated. The residue was purified by work up to give compound 4 (105 mg, Yield: 92.1%) as a yellow solid.

MS m/z (ESI): 640.5 [M+H]⁺.

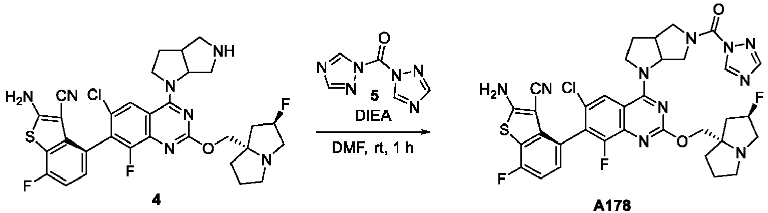

To a solution of compound 4 (70 mg, 0.11 mmol), and compound 5 (53.9 mg, 0.33 mmol) in DMF (2 mL) was added DIEA (42 mg, 0.33 mmol). The mixture was stirred at room temperature for 1h. The mixture was concentrated and purified by prep-HPLC (FA) to give A178 (23.51 mg, Yield: 29.24%) as a white solid.

MS m/z (ESI): 735.1 [M+H]⁺.

¹H NMR (400 MHz, DMSO-*d₆*): δ 9.14 - 9.06 (m, 1H), 8.21 - 8.06 (m, 4H), 7.26 - 7.19 (m, 1H), 7.17 - 7.11 (m, 1H), 5.28 (d, *J=* 54.0 Hz, 1H), 5.02 - 4.91 (m, 1H), 4.55 - 4.25 (m, 2H), 4.24 - 3.62 (m, 6H), 3.17 - 3.07 (m, 3H), 3.04 - 2.99 (m, 1H), 2.88 - 2.78 (m, 1H), 2.24 - 1.94 (m, 5H), 1.88 - 1.68 (m, 3H).

### Synthesis of Compound A199

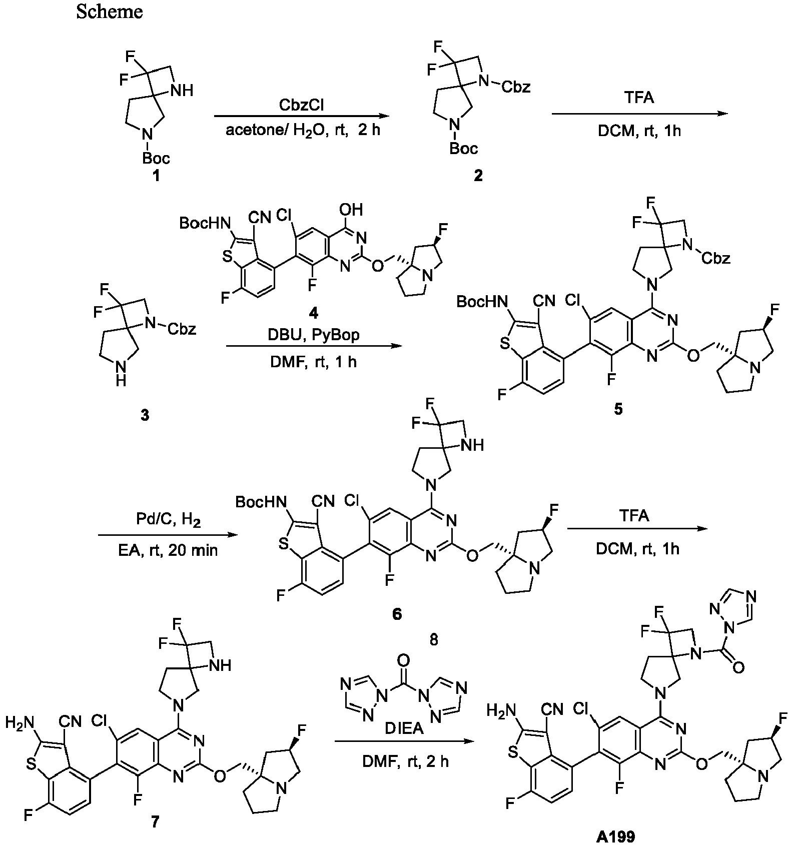

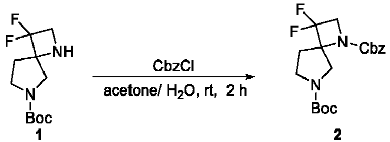

To a solution of compound 1 (100 mg, 0.40 mmol) and NaHCO₃ (68 mg, 0.80 mmol) in acetone (2 ml) was added H₂O (1 ml). The reaction mixture was added CbzCl (137 mg, 0.80 mmol) and stirred at 25 °C for 1 h. The solution was extracted with ethyl acetate (50 mL x 3). The organic layer was washed with brine (50 mL), dried over anhydrous sodium sulfate, filtered and the filtrate was concentrated to dryness under reduced pressure. The residue was purified by silica gel column (PE/EA=6/1) to give compound 2 (80 mg, Yield: 52 %) as colorless oily liquid.

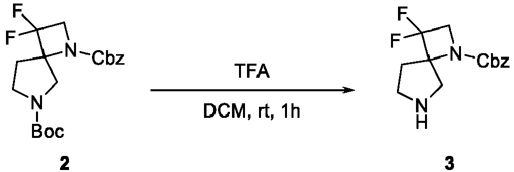

To a solution of compound 2 (80 mg, 0.21 mmol) in DCM (1 ml) was added TFA (1 ml). The reaction mixture was stirred at 20 °C for 1 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated to afford compound 3 (50 mg, 85 %) as colorless oily liquid.

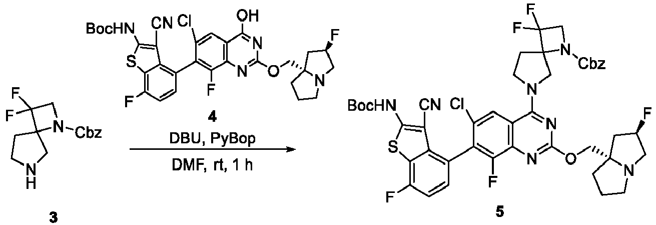

To a solution of compound 3 (119 mg, 0.42 mmol), compound 4 (210 mg, 0.32 mmol), and PyBop (253 mg, 0.49 mmol) in DMF (3 mL) was added DBU (61 mg, 0.49 mmol). The mixture was stirred at 25 °C for 1 h. The solution was extracted with ethyl acetate (80 mL X 3). The organic layer was washed with brine (100 mL), dried over anhydrous sodium sulfate, filtered and the filtrate was concentrated to dryness under reduced pressure. The residue was purified by silica gel column (DCM/MeOH =15/1) to give compound 5 (170 mg, Yield: 58 %) as a white solid. MS m/z (ESI): 910.2 [M+H]⁺.

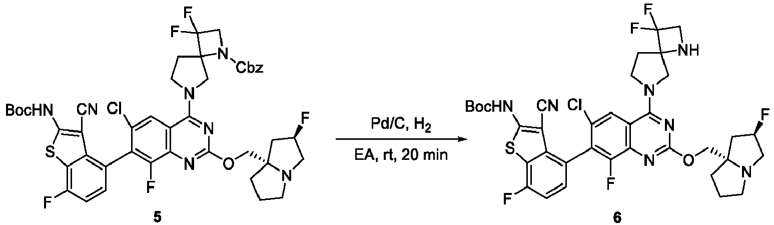

To a solution of compound 5 (120 mg, 0.13 mmol) in ethyl acetate (3 ml) was added Pd/C (60 mg). The reaction mixture was stirred at rt under H₂ atmosphere for 20 min. The reaction mixture was diluted with ethyl acetate (30 ml). The organic layer was filtered, and concentrated to afford compound 6 (90 mg, 89 %) as a yellow solid. MS m/z (ESI): 776.2 [M+H]⁺.

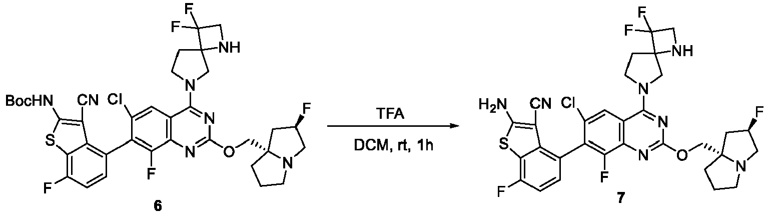

To a solution of compound 6 (90 mg, 0.17 mmol) in DCM (2 ml) was added TFA (2 ml). The reaction mixture was stirred at 20 °C for 30 min. The solvent was removed under reduced pressure. The reaction mixture was diluted with a.q. NaHCO₃ (20 mL) and DCM (50 mL). The organic layer was washed with brine, dried over sodium sulfate, filtered, and concentrated to afford 7 (70 mg, 89 %) as colorless oily liquid. MS m/z (ESI): 676.3 [M+H]⁺.

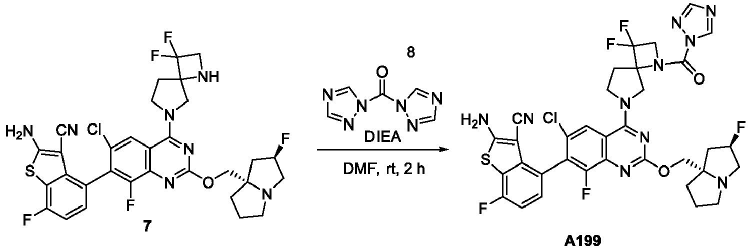

To a solution of compound 7 (30 mg, 0.044 mmol) and DIEA (17 mg, 0.132 mmol) in 0.8 mL DMF, was added compound 8 (22 mg, 0.132 mmol). The reaction mixture was then stirred at rt for 2 h. LCMS showed the reaction was completed. The crude was purified by prep-HPLC to give A199 (5.42 mg, Yield: 16 %). MS m/z (ESI): 771.4 [M+H]⁺.

¹H NMR (400 MHz, DMSO-*d₆*): δ 9.25 (s, 1H), 8.30 (d, J = 5.2 Hz, 1H), 8.17 - 8.07 (m, 3H), 7.27 - 7.12 (m, 2H), 5.27 (d, J = 54.0 Hz, 1H), 5.15 - 4.11 (m, 8H), 3.12 - 2.77 (m, 6H), 2.25 - 1.75 (m, 6H).

### Synthesis of Compound A208

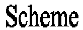

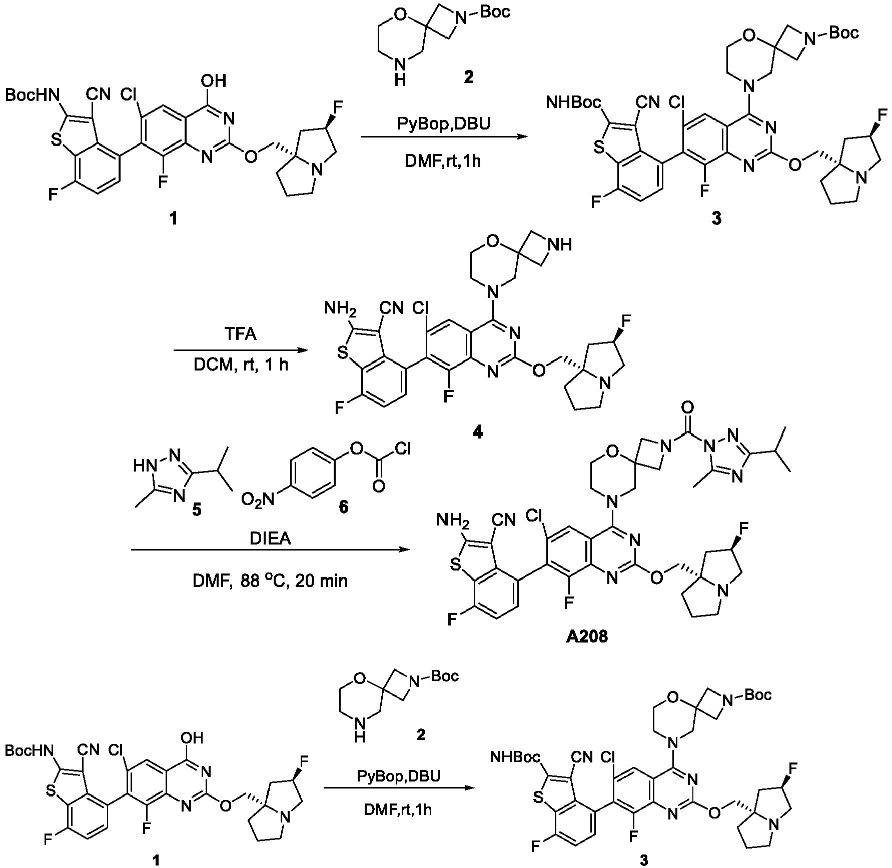

To a solution of compound 1 (300 mg, 0.465 mmol), compound 2 (137 mg, 0.600 mmol) and PyBop (604 mg, 1.163 mmol) in DMF (4 ml) was added DBU (232 mg, 1.860 mmol). The reaction mixture was stirred at r.t for 1 h. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by prep-TLC (DCM/MeOH=15/1) to give compound 3 (300 mg, Yield: 75.46 %) as a white solid.

MS m/z (ESI): 856.2 [M+H]⁺.

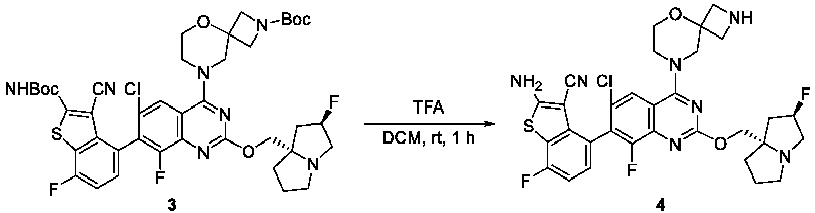

To a solution of compound 3 (300 mg, 0.351 mmol) in DCM (3 ml) was added TFA (3 ml). The reaction mixture was stirred at r.t for 1 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated. The residue was purified by work up to give compound 4 (210 mg, Yield: 91.34%) as a yellow solid.

MS m/z (ESI): 656.0 [M+H]⁺.

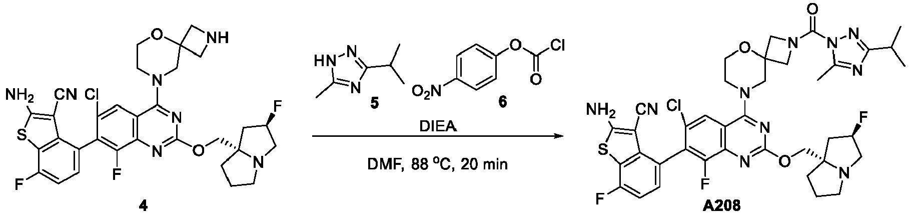

To a solution of compound 5 (793 mg, 6.35 mmol) and DIEA (1.23 g, 9.53 mmol) in 10 mL MeCN, was added 4-nitrophenyl carbonochloridate (1.41 g, 6.99 mmol) at 0 °C under N₂ atmosphere. The reaction mixture was then stirred at rt for 1 h. To a solution of compound 4 (50 mg, 0.076 mmol) in DMF (0.5 mL) was added above reaction mixture (0.15 mL) at 60 °C under N₂ atmosphere.

The solution was stirred at 60 °C for 20 min. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by Prep-HPLC to give A208 (10.98 mg, Yield: 17.93%) as a white solid.

MS m/z (ESI): 807.5 [M+H]⁺.

¹H NMR (400 MHz, DMSO-*d₆*): δ 8.12 (s, 2H), 7.95 (s, 1H), 7.26 - 7.13 (m, 2H), 5.32 (d, *J* = 53.6 Hz, 1H), 4.58 - 4.49 (m, 1H), 4.39 (d, *J=* 10.4 Hz, 1H), 4.20 - 3.69 (m, 11H), 3.20 - 3.00 (m, 2H), 2.85 -2.80 (m, 2H), 2.58 (s, 3H), 2.15 -1.73 (m, 6H), 1.30 - 1.20 (m, 6H).

### Synthesis of Compound A209

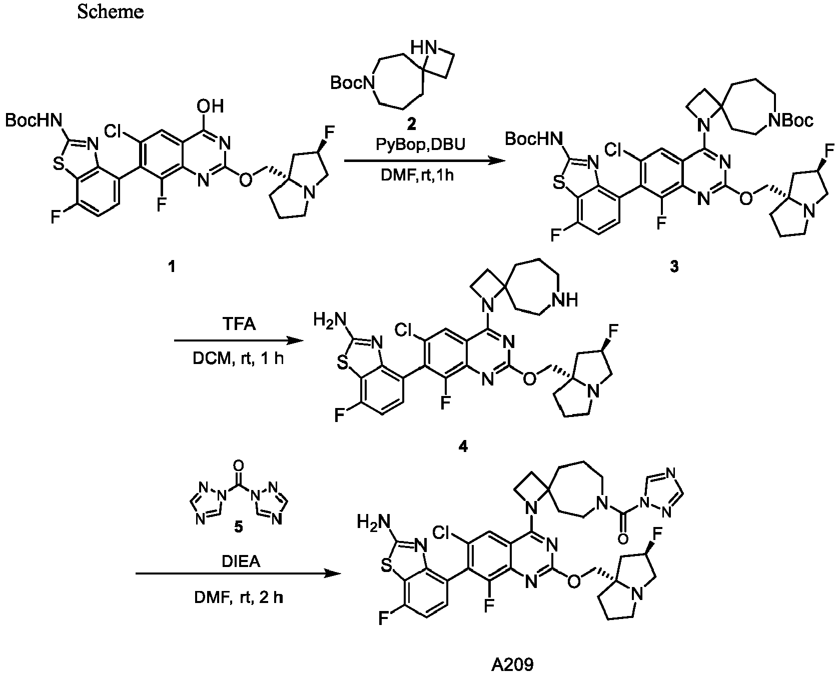

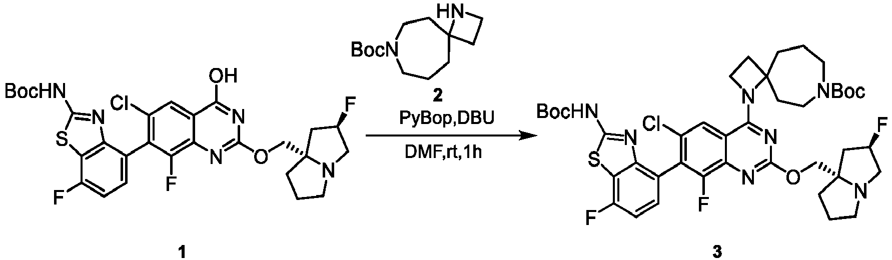

To a solution of compound 1 (120 mg, 0.19 mmol), compound 2 (51 mg, 0.21 mmol) and PyBop (151 mg, 0.29 mmol) in DMF (4 ml) was added DBU (88 mg, 0.58 mmol). The reaction mixture was stirred at 25 °C for 1 h. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by prep-TLC (DCM/MeOH=15/1) to give compound 3 (100 mg, Yield: 61.3 %) as a solid.

MS m/z (ESI): 844.5 [M+H]⁺.

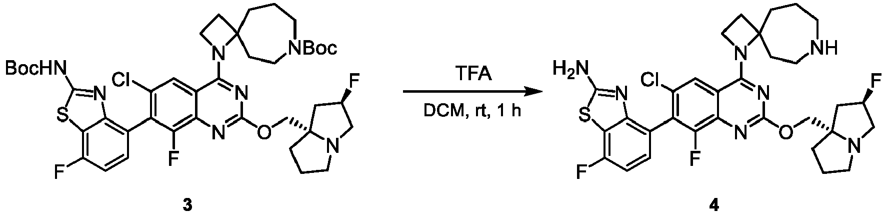

To a solution of compound 3 (100 mg, 0.12 mmol) in DCM (4 ml) was added TFA (2 ml). The reaction mixture was stirred at 25 °C for 1 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated. The residue was purified by work up to give compound 4 (20 mg, Yield: 26%) as a solid.

MS m/z (ESI): 644.2 [M+H]⁺.

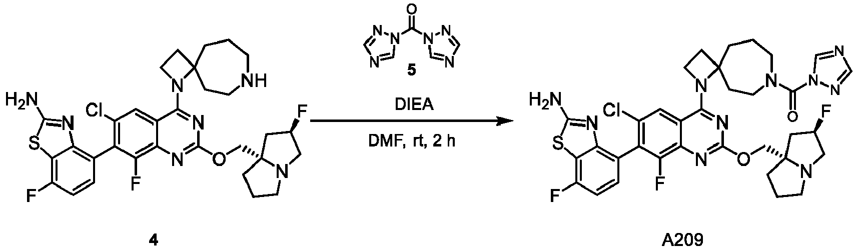

To a solution of compound 4 (20 mg, 0.03 mmol), compound 5 (15 mg, 0.09 mmol), and DIEA (12 mg, 0.09 mmol) in DMF (1 mL). The solution was stirred at 25 °C for 2 hours. The mixture was poured into water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by Pre-HPLC (FA) to give A209 (6.26 mg, Yield: 27.28%) as a solid.

MS m/z (ESI): 739.3 [M+H]⁺.

¹H NMR (400 MHz, DMSO-*d₆*): δ 9.19 (d, J= 33.2 Hz, 1H), 8.26 (s, 1H), 7.89 (s, 2H), 7.78 (s, 1H), 7.20 (s, 1H), 7.05 (t, J= 8.8 Hz, 1H), 5.30 - 5.00 (m, 1H), 4.75 (s, 2H), 4.02 - 3.75 (m, 3H), 3.70 - 3.45 (m, 2H), 3.20 - 2.60 (m, 5H), 2.30 - 1.50 (m, 14H).

### Synthesis of Compound A219

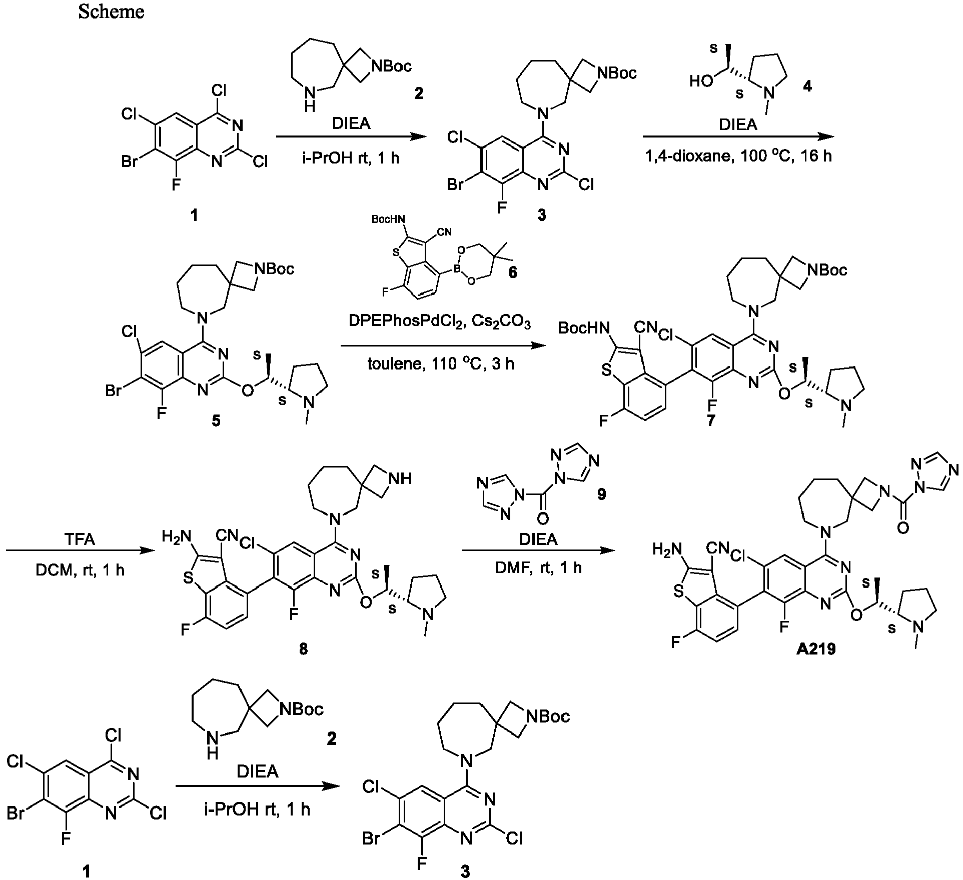

To a solution of compound 1 (1.1 g, 3.33 mmol) and 2 (780 mg, 3.33 mmol) in i-PrOH (10 ml) was added DIEA (1.28 g, 9.99 mmol). The reaction mixture stirred at rt for 1 h. The reaction solution was filtered with petroleum ether. The filter cake is concentrated to dry state under reduced pressure to give compound 3 (1.4 g, Yield: 79 %) as yellow solid. MS m/z (ESI): 533.0 [M+H]⁺.

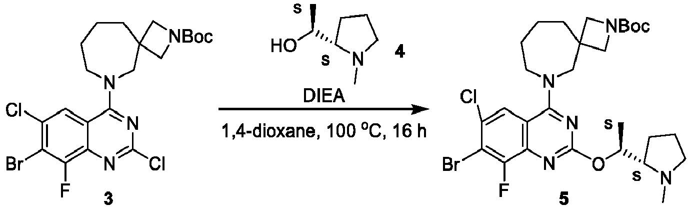

To a solution of compound 3 (800 mg, 1.5 mmol), 4 (387.1 mg, 3 mmol) in 1,4-dioxane (15 mL) was added DIEA (581.4 mg, 4.5 mmol). The mixture was stirred at 100 °C for 16 h. The solution was extracted with ethyl acetate (100 mL X 3). The organic layer was washed with brine (100 mL), dried over anhydrous sodium sulfate, filtered and the filtrate was concentrated to dryness under reduced pressure. The residue was purified by silica gel column (DCM/ MeOH =10/1) to give compound 5 (230 mg, Yield: 24.5 %) as a yellow solid. MS m/z (ESI): 626.0 [M+H]⁺.

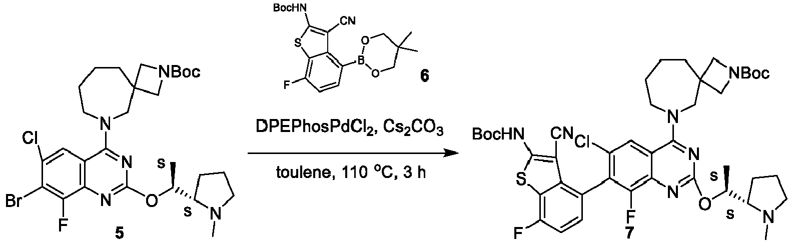

To a solution of compound 5 (200 mg, 0.318 mmol), 6 (192 mg, 0.478 mmol), and Cs₂CO₃ (310.7 mg, 0.956 mmol) in toluene (5 mL) was added DPEPhosPdCl₂ (45.4 mg, 0.062 mmol). The mixture was stirred at 110 °C for 3 h. The solution was extracted with ethyl acetate (100 mL X 3). The organic layer was washed with brine (100 mL), dried over anhydrous sodium sulfate, filtered and the filtrate was concentrated to dryness under reduced pressure. The residue was purified by reverse column (MeCN/ H₂O (4 %oFA) =1/3) to give compound 7 (70 mg, Yield: 26.3 %) as a yellow solid. MS m/z (ESI): 838.4 [M+H]⁺.

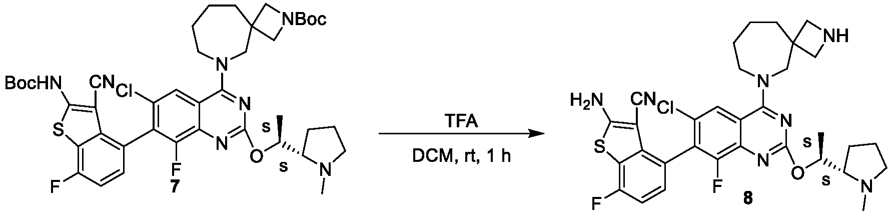

To a solution of compound 7 (70 mg, 0.083 mmol) in DCM (1 ml) was added TFA (1 ml). The reaction mixture was stirred at rt for 30 min. The solvent was removed under reduced pressure. The reaction mixture was diluted with a.q. NaHCO₃ (200 mL) and DCM/ MeOH=10/ 1(300 mL). The organic layer was washed with brine, dried over sodium sulfate, filtered, and concentrated to give compound 8 (50 mg, Yield: 94 %) as a yellow solid. MS m/z (ESI): 638.2 [M+H]⁺.

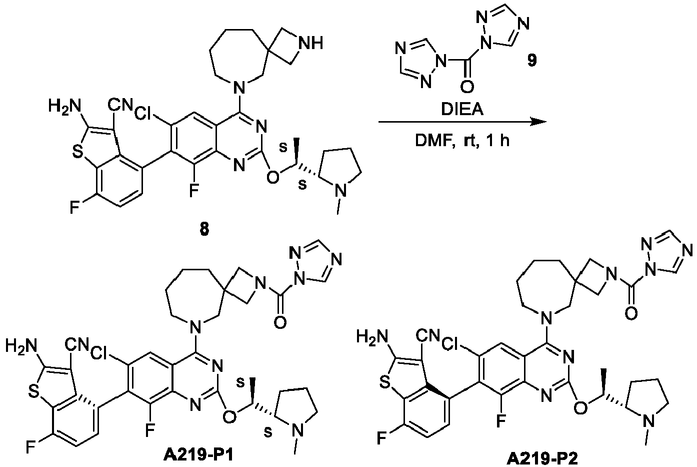

To a solution of compound 8 (50 mg, 0.078 mmol) and DIEA (30.23 mg, 0.234 mmol) in 1 mL DMF, was added 9 (32.16 mg, 0.196 mmol). The reaction mixture was then stirred at rt for 1 h. LCMS showed the reaction was completed. The crude was purified by prep-HPLC to give A219-P1 (9 mg, Yield: 15 %) and A219-P2 (8 mg, Yield: 14 %). MS m/z (ESI): 733.3 [M+H].

¹H NMR (400 MHz, DMSO-*d₆*): δ 9.12 (s, 1H), 8.21 (d, *J* = 10.0 Hz, 1H), 8.09 (s, 2H), 7.96 (s, 1H), 7.30 - 7.10 (m, 2H), 5.26 - 5.15 (m, 1H), 4.60 - 4.50 (m, 1H), 4.40 - 4.10 (m, 4H), 4.05 - 3.80 (m, 5H), 3.00 (s, 2H), 2.70 - 2.60 (m, 1H), 2.00 - 1.75 (m, 5H), 1.70 - 1.55 (m, 5H), 1.30 - 1.15 (m, 3H).

¹H NMR (400 MHz, DMSO-*d₆*): δ 9.12 (s, 1H), 8.21 (d, *J* = 10.0 Hz, 1H), 8.09 (s, 2H), 7.96 (s, 1H), 7.30 - 7.10 (m, 2H), 5.26 - 5.15 (m, 1H), 4.60 - 4.50 (m, 1H), 4.40 - 4.10 (m, 4H), 4.05 - 3.80 (m, 5H), 3.00 (s, 2H), 2.70 - 2.60 (m, 1H), 2.00 - 1.75 (m, 5H), 1.70 - 1.55 (m, 5H), 1.30 - 1.15 (m, 3H).

### Synthesis of Compound A228

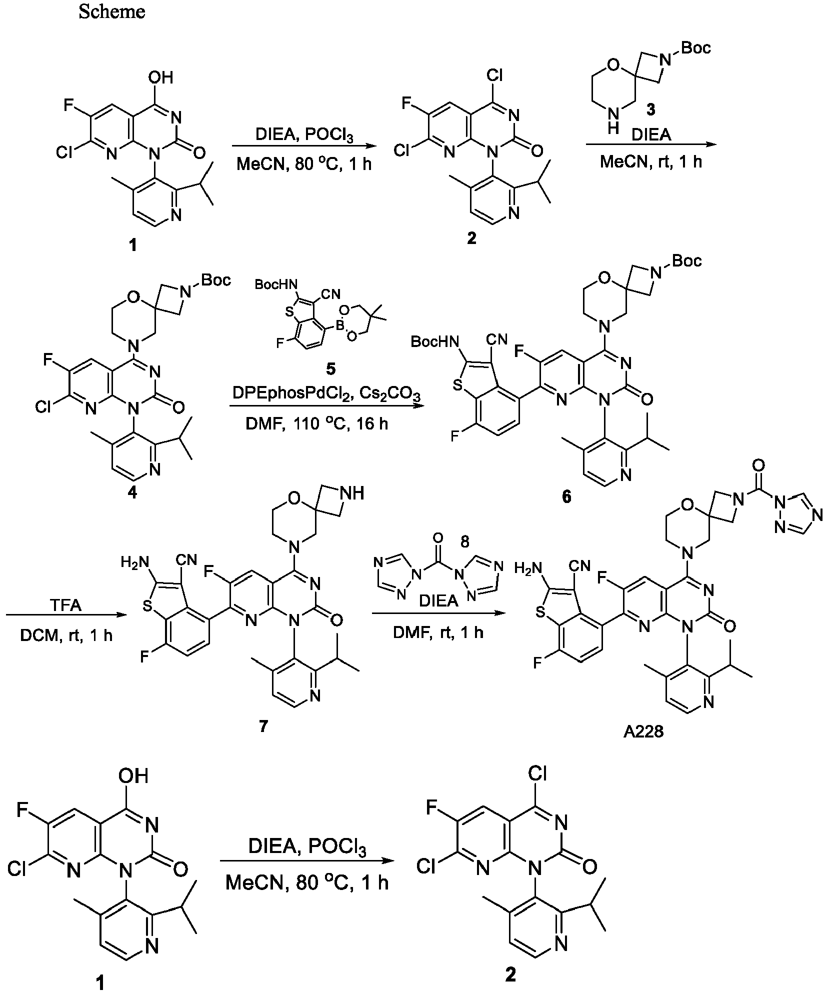

To a solution of compound 1 (3 g, 8.619 mmol) and N,N-Diisopropylethylamine (1.67 g, 12.929 mmol) in dry MeCN (20 mL) was added POCl₃ (1.72 g, 11.205 mmol). The mixture was stirred at 80 °C for 1 hr under N₂ atmosphere. The mixture was concentrated to dryness under reduced pressure to obtain compound 2 (2.5 g, Yield: 79 %) as a black oily liquid.

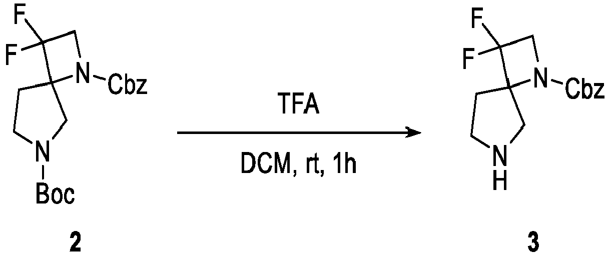

To a solution of compound 2 (1.25 g, 3.415 mmol) and N,N-Diisopropylethylamine (1.32 g, 10.245 mmol) in dry MeCN (15 mL) was added compound 3 (779 mg, 3.415 mmol). The reaction was stirred at room temperature for 1 hr. The mixture was poured into ice water (200 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (PE/EA=8/1) to give compound 4 (800 mg, Yield: 42 %) as a yellow solid. MS m/z (ESI): 559.2 [M+H]⁺.

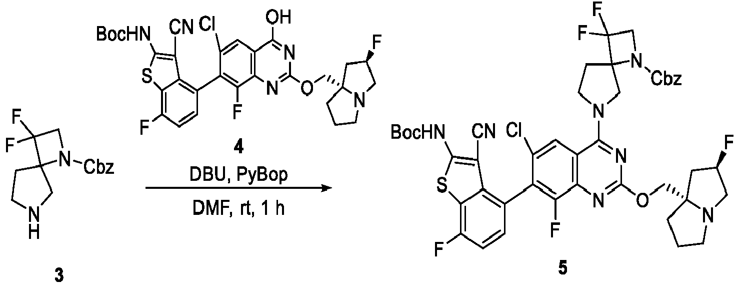

To a solution of compound 4 (200 mg, 0.358 mmol), compound 5 (217 mg, 0.537 mmol) and Cs₂CO₃ (350 mg, 1.075 mmol) in dry DMF (5 mL) was added DPEPhosPdCl₂ (52 mg, 0.0717 mmol). The mixture was stirred 110 °C for 16 h under N₂ atmosphere. The mixture was diluted with ethyl acetate (100 mL) and water (100 ml). The organic layer was washed with brine (50 mL), dried over Na₂SO₄, filtered and concentrated. The residue was purified by silica gel column (PE:EA=1:1) to get compound 6 (160 mg, Yield: 54.9 %) as a yellow solid. MS m/z (ESI): 815.4 [M+H]⁺.

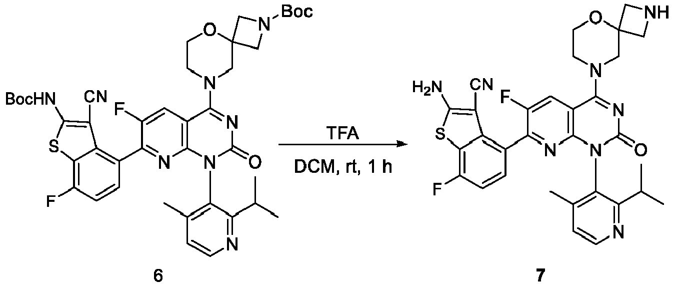

To a solution of compound 6 (180 mg, 0.221 mmol) in dry DCM (2 mL) was added TFA (2 mL). The mixture was stirred at room temperature for 1 hr. The solvent was removed under reduced pressure. The reaction mixture was diluted with a.q. NaHCO₃ (10 mL) and DCM/MeOH (10:1) (50 mL), and the solution was extracted with DCM/MeOH (10:1) (100 mL x 3). The organic layer was washed with brine (50 mL), dried over anhydro us sodium sulfate, filtered and the filtrate was concentrated to dryness under reduced pressure. The residue was purified by prep-HPLC to give compound 7 (9.40 mg, Yield: 11.46 %) as a white solid. MS m/z (ESI): 615.4 [M+H]⁺.

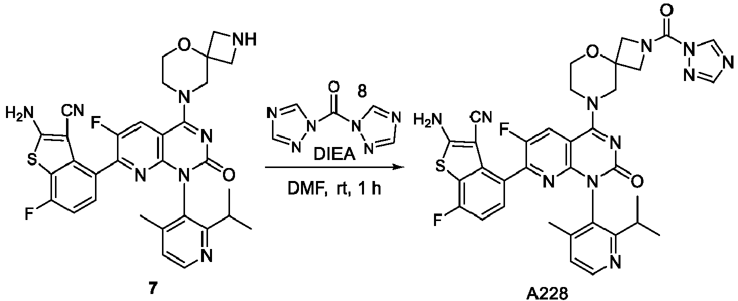

To a solution of compound 7 (50 mg, 0.0814 mmol) and DIEA (32 mg, 0.2442 mmol) in 0.8 mL DMF, was added compound 8 (13 mg, 0.0814 mmol). The reaction mixture was then stirred at rt for 1 h. LCMS showed the reaction was completed. The crude was purified by prep-HPLC to give A228 (5.47 mg, Yield: 9.5 %). MS m/z (ESI): 710.2 [M+H].

¹H NMR (400 MHz, DMSO-*d₆*): δ 9.18 (d, J = 10.0 Hz, 1H), 8.51 - 8.39 (m, 2H), 8.25 - 8.10 (m, 3H), 7.35 - 6.94 (m, 3H), 4.73 - 4.13 (m, 10H), 2.70 (s, 1H), 2.10 -1.90 (m, 3H), 1.28 - 0.94 (m, 6H).

### Synthesis of Compound A250

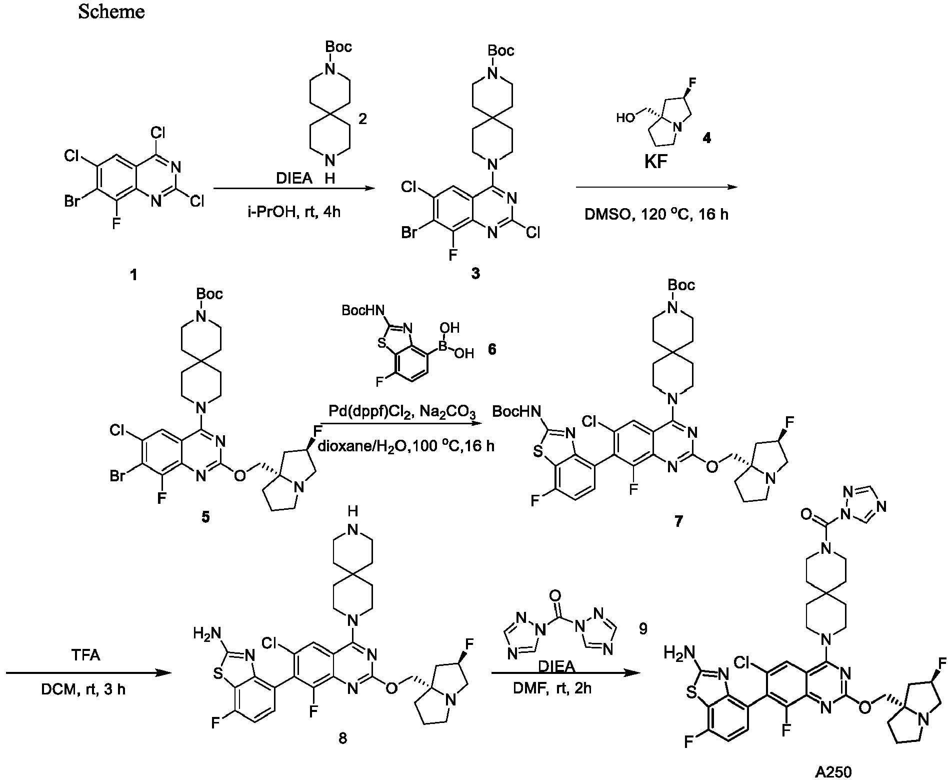

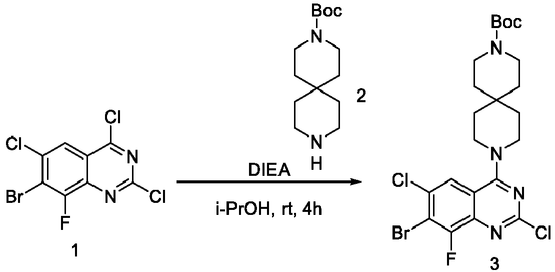

To a solution of compound 2 (1.3 g, 5.11 mmol) and compound 1 (1.68 g, 5.11 mmol) in i-PrOH (20 ml) was added DIEA (1.98 g, 15.33 mmol). The reaction mixture was stirred at 25 °C for 4 h. The mixture was poured into ice water (200 mL). The mixture was filtered and the solid was diluted with MeOH (150 ml). The organic solution was concentrated to dryness under reduced pressure to give compound 3 (1.9 g, Yield: 68 %) as a white solid. MS m/z (ESI): 547.0 [M+H]⁺.

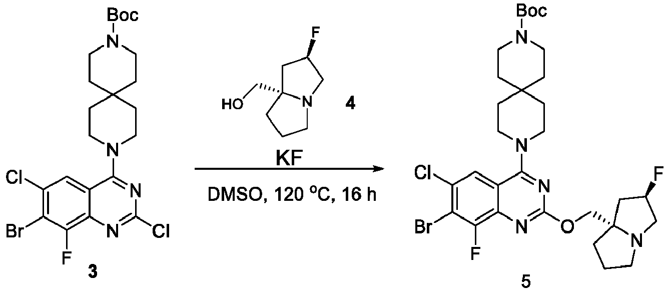

To a solution of compound 3 (1.2 g, 2.20 mmol) and compound 4 (1.75 g, 11.00 mmol) in DMSO (10 ml) was added KF (638 mg, 11.00 mmol). The reaction mixture was stirred at 120 °C for 16 h. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (PE/EA=1/1) to give compound 5 (700 mg, Yield: 50 %) as a white solid. MS m/z (ESI): 670.2 [M+H] ⁺.

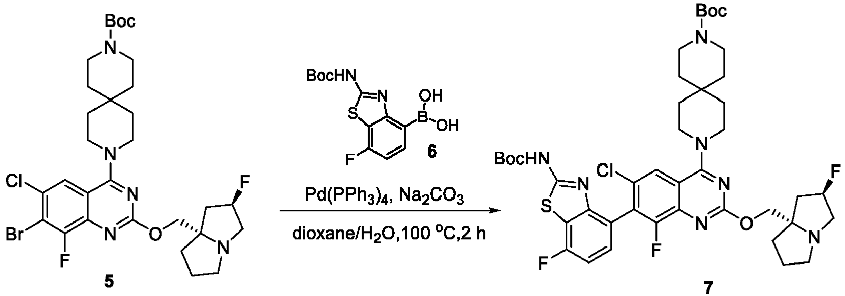

To a solution of compound 5 (700 mg, 1.05 mmol), compound 6 (424 mg, 1.36 mmol), and Na₂CO₃ (223 mg, 2.10 mmol) in 1,4-dioxane/H₂O (8 mL/ 2 mL) was added Pd(PPh₃)₄ (243 mg, 0.31 mmol). The mixture was stirred at 100 °C under nitrogen for 2 hours. The mixture was poured into water (200 mL), and the solution was extracted with ethyl acetate (100 mL X 3). The organic layer was washed with brine (100 mL), dried over anhydrous sodium sulfate, filtered and the filtrate was concentrated to dryness under reduced pressure. The residue was purified by silica gel column (PE/EA=1/1) to give compound 7 (450 mg, Yield: 50 %) as a yellow solid.

MS m/z (ESI): 858.3 [M+H]⁺.

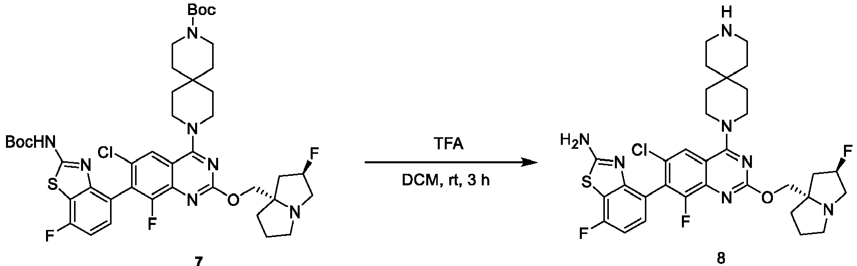

To a solution of compound 7 (400 mg, 0.467 mmol) in DCM (4 ml) was added TFA (2 ml). The reaction mixture was stirred at 20 °C for 3 h. The solvent was removed under reduced pressure. The reaction mixture was diluted with a.q. NaHCO₃ (20 mL) and DCM (40 mL). The organic layer was washed with brine, dried over sodium sulfate, filtered, and concentrated to afford compound 8 (200 mg, 65 %) as a yellow solid. MS m/z (ESI): 658.2 [M+H]⁺.

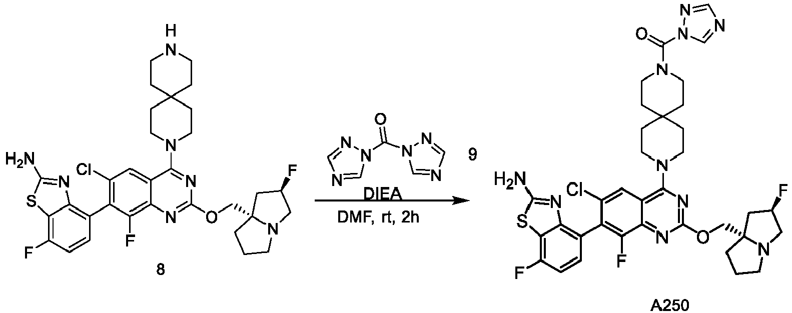

To a solution of compound 8 (50 mg, 0.076 mmol) and compound 9 (37 mg, 0.228 mmol) in DMF (2 mL) was added and DIEA (29 mg, 0.228 mmol) at 25 °C under N₂. The mixture was stirred at 25 °C for 1 h. The mixture was purified by Prep-HPLC (FA) to give compound A250 (16.34 mg, yield: 28.55 %) as a white solid.

MS m/z (ESI): 753.3 [M+H]⁺.

¹H NMR (400 MHz, DMSO-*d*₆): δ 9.06 (s, 1H), 8.25 (s, 1H), 7.91 (s, 3H), 7.25 - 7.21 (m, 1H), 7.10 - 7.05 (m, 1H), 5.56 (d, J = 52.0 Hz, 1H), 4.55 (s, 2H), 3.86 (s, 6H), 3.68 (s, 6H), 2.47 (m, 1H), 2.33 (s, 2H), 2.17 (s, 2H), 1.74 - 1.67 (m, 9H).

### Synthesis of Compound A251

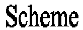

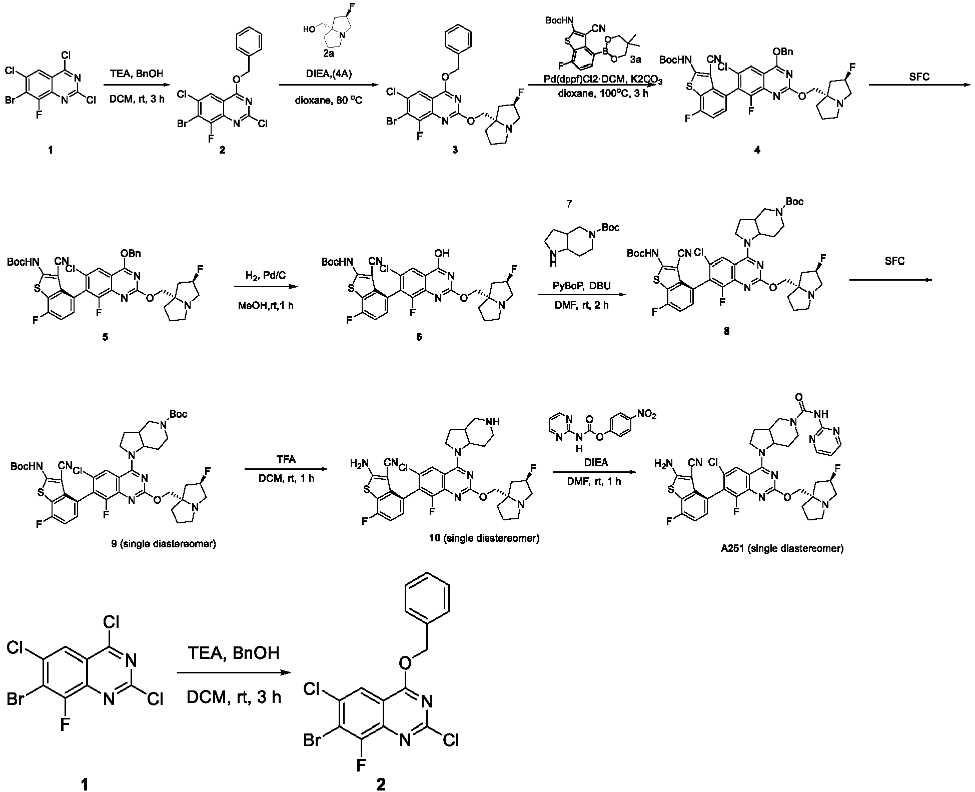

To a solution of compound 1 (5 g, 15.1 mmol) and TEA (4.59 g, 45.40 mmol) in DCM (50 mL) was added BnOH (1.96 g, 18.2 mmol). The mixture was stirred room temperature for 3 h. The mixture was poured into water (100 mL), and the solution was extracted with DCM (100 mL). The organic layer was washed with brine (50 mL), dried over anhydrous sodium sulfate, filtered and the filtrate was concentrated to dryness under reduced pressure, which was purified by silica gel column (PE/EA=10:1) to give compound 2 (4 g, Yield: 65.7 %) as a yellow solid.

MS m/z (ESI): 401.0 [M+H] ⁺.

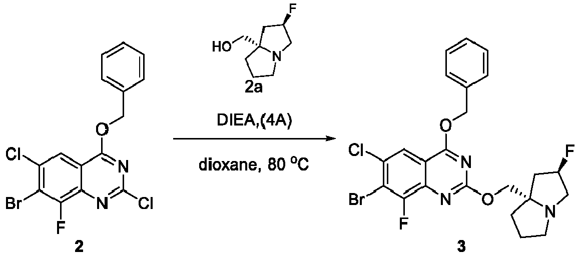

A mixture of compound 2 (5 g, 12.44 mmol), compound 2a (5.94 g, 37.31 mmol), molecular sieve (4A) (500 mg), DIEA (4.82 g, 37.31 mmol) in DMSO (30 ml) was stirred at 80 °C for 16 h under N₂. The mixture was filtered and filtrate was extracted with EA (100 mL), washed with brine (50 mL x 3), dried over anhydrous sodium sulfate, filtered and the filtrate was concentrated to dryness under reduced pressure. The residue was purified by silica gel column (PE/EA=1:1) to give compound 3 (2.3 g, Yield: 35 %) as a white solid.

MS m/z (ESI): 523.8 [M+H] ⁺.

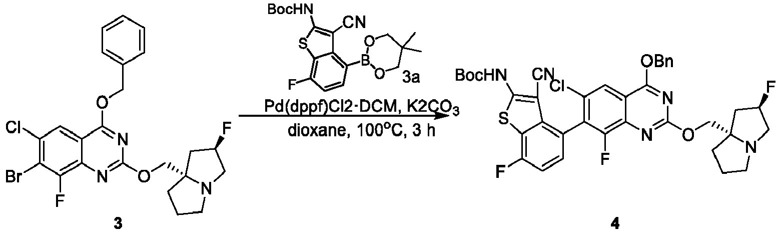

To a solution of compound 3 (2 g, 3.82 mmol), compound 3a (2.32 g, 5.73 mmol) and K₂CO₃ (3.16 g, 22.94 mmol) in dioxane (30 mL) was added Pd(dppf)Cl₂ (624 mg, 0.76 mmol). The mixture was stirred 100 °C for 3 h under N₂. The mixture was poured into water (100 mL), and the solution was extracted with ethyl acetate (100 mL x 3). The organic layer was washed with brine (50 mL), dried over anhydrous sodium sulfate, filtered and the filtrate was concentrated to dryness under reduced pressure. The residue was purified by silica gel column (MeOH:DCM=15:1) to get compound 4 (1.4 g, Yield: 49.9 %) as a yellow solid.

MS m/z (ESI): 736.3 [M+H]⁺.

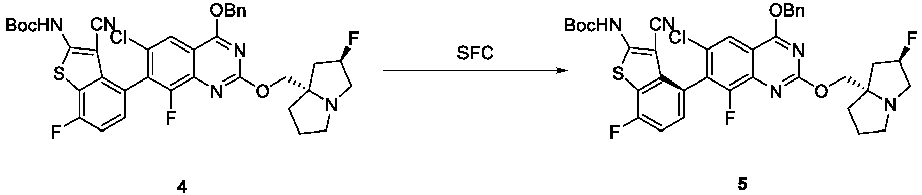

Compound 2 (4 g, 5.44 mmol) was purified by SFC to obtain Compound 5 (1.8 g, 45%) as yellow solid.

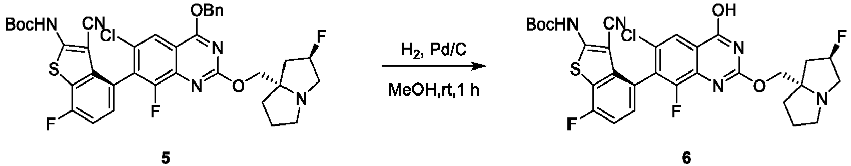

To a solution of compound 5 (1.8 g, 2.44 mmol) in MeOH (50 mL) was added Pd/C (180 mg). The mixture was stirred 25 °C for 15 min under H₂. The mixture was filtered and the filtrate was concentrated to dryness under reduced pressure to get compound 6 (1.4 g, Yield: 88.6 %) as a grey solid. MS m/z (ESI): 646.4 [M+H]⁺.

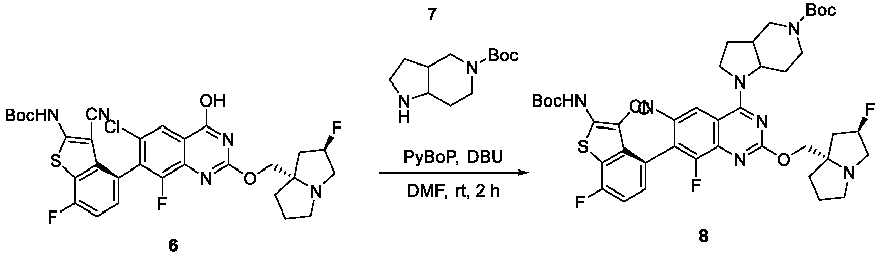

To a solution of compound 6 (230 mg, 0.356 mmol), compound 7 (114 mg, 0.506 mmol) and PyBop (303 mg, 0.584 mmol) in DMF (2 mL) was added DBU (118 mg, 0.778 mmol). The mixture was stirred at room temperature for 1h. The mixture was poured into water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by prep-TLC (MeOH:DCM=1:15) to get compound 8 (270 mg, Yield: 81.3 %) as a yellow solid. MS m/z (ESI): 854.0 [M+H]⁺.

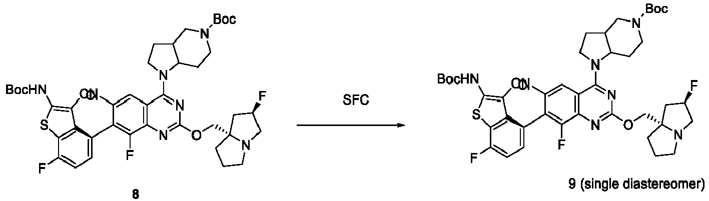

Compound 8 (270 mg, 0.317 mmol) was purified by SFC to obtain Compound 9 (110 mg, 40.7 %) as yellow solid.

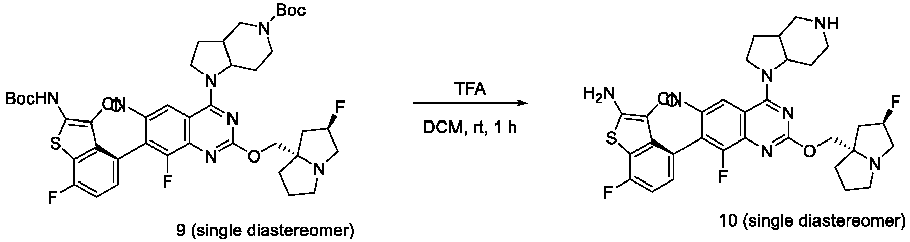

To a solution of compound 9 (110 mg, 0.129 mmol) in DCM (2 ml) was added TFA (1 ml). The reaction mixture was stirred at 20 °C for 1 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated to afford compound 10 (80 mg, 95.2%) as a yellow solid. MS m/z (ESI): 654.2 [M+H]⁺.

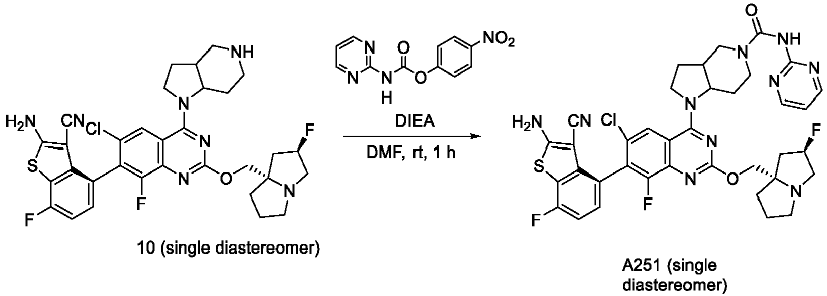

To a solution of compound 10 (20 mg, 0.0306 mmol) and DIEA (12 mg, 0.0918 mmol) in 0.5 mL DMF, was added compound 11 (24 mg, 0.0918 mmol). The reaction mixture was then stirred at rt for 1 h. LCMS showed the reaction was completed. The crude was purified by prep-HPLC to give A251 (2.82 mg, Yield: 11.9 %). MS m/z (ESI): 775.2 [M+H]⁺.

¹H NMR (400 MHz, DMSO-*d*₆): δ 9.51 (s, 1H), 8.55 (s, 1H), 8.54 (s, 1H), 8.17 (s, 1H), 8.11 (s, 2H), 7.22 (dd, J = 8.4, 5.8 Hz, 1H), 7.18 - 7.10 (m, 1H), 7.01 (t, J = 4.8 Hz, 1H), 5.27 (d, J = 54.0 Hz, 1H), 4.78 (d, J = 6.8 Hz, 1H), 4.36 - 3.55 (m, 7H), 3.27 - 2.74 (m, 6H), 2.24 - 1.71 (m, 10H).

### Synthesis of Compound A255

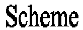

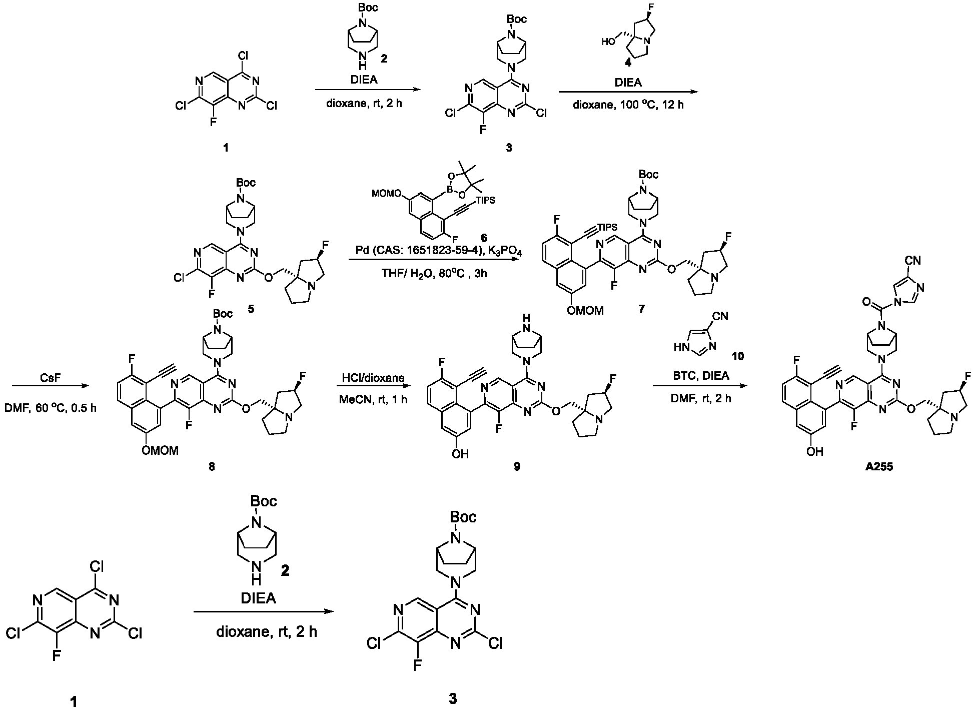

To a solution of compound 2 (507 mg, 2.39 mmol) and N,N-Diisopropylethylamine (927 mg, 7.17 mmol) in dry dioxane (5 mL) was added compound 1 (600 mg, 2.39 mmol). The reaction was stirred at room temperature for 2 hr. The mixture was poured into ice water (100 mL). The mixture was filtered and the solid was collected to obtain compound 3 as a yellow solid. (800 mg, Yield: 78.3 %). MS m/z (ESI): 428.1 [M+H] ⁺.

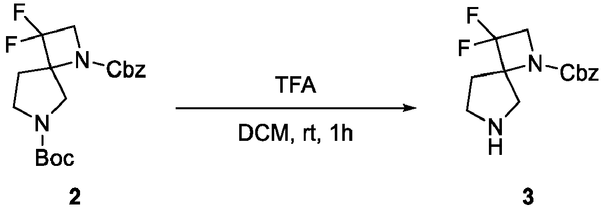

To a solution of compound 3 (400 mg, 0.936 mmol) and DIEA (363 mg, 2.808 mmol) in dioxane (10 ml) was added compound 4 (149 mg, 1.872 mmol). The reaction mixture was stirred at 100 °C for 12 h. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (DCM/MeOH=20/1) to give compound 5 (400 mg, Yield: 77.6 %) as a white solid. MS m/z (ESI): 551.2 [M+H] ⁺.

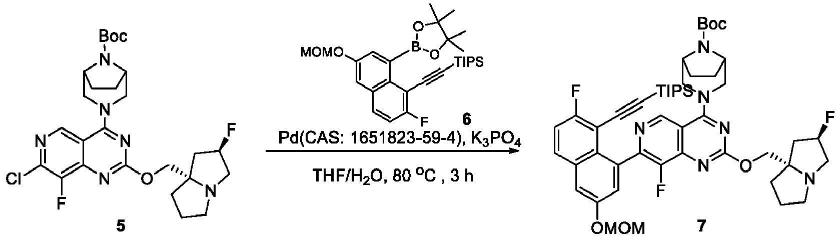

To a solution of compound 5 (900 mg, 1.636 mmol), compound 6 (1006 mg, 1.963 mmol) and K₃PO₄ (1042 mg, 4.908 mmol) in dry THF/H₂O (16 mL/4 mL) was added cataCXium A Pd G3 (120 mg, 0.164 mmol). The mixture was stirred at 80 °C for 3 hr under N₂. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (EA: MeOH=20:1) to give compound 7 (800 mg, Yield: 54.3 %) as a white solid. MS m/z (ESI): 901.5 [M+H]⁺.

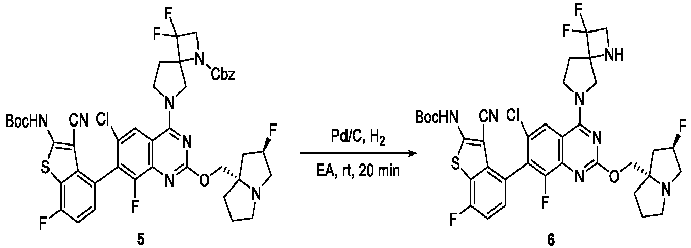

To a solution of compound 7 (200 mg, 0.222 mmol) in dry DMF (5 mL) was added CsF (337 mg, 2.22 mmol). The mixture was stirred at 60 °C for 0.5 hr. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure to give compound 8 (150 mg, Yield: 90.7 %) as a white solid. MS m/z (ESI): 745.9 [M+H]⁺.

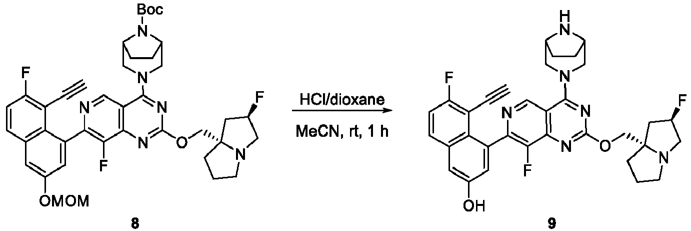

To a solution of compound 8 (150 mg, 0.2 mmol) in CH₃CN (6 mL) was added HCl/dioxane (3 mL, 12 mmol). The solution was stirred at 20 °C for 1 h. Then the solution was added dropwise to n-hexane (50 mL), the upper clear liquid was removed and the residue was dissolved in a solution of DCM (100 mL) and MeOH (10 mL). The organic layer was washed by aqueous a.q. NaHCO₃ (50 mL), and brine (50 mL). The organic layer was separated, dried over Na₂SO₄, concentrated to give compound 9 (110 mg, Yield: 90.9 %) as a yellow solid. MS m/z (ESI): 601.2 [M+H]⁺.

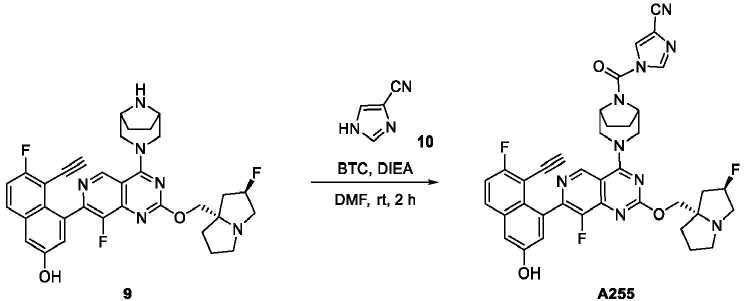

To a solution of compound 10 (248 mg, 2.66 mmol) and DIEA (1.38 g, 10.66 mmol) in 18 mL MeCN, was added BTC (712 mg, 2.40 mmol) at 0 °C under N₂ atmosphere. The reaction mixture was then stirred at rt for 2 h. To a solution of compound 9 (140 mg, 0.23 mmol) and DIEA (90 mg, 0.70 mmol) in DMF (1 mL), was added above reaction mixture (0.9 mL) at rt under N₂ atmosphere. LCMS showed the reaction was completed. The crude was purified by prep-HPLC to give A255 (21.15 mg, Yield: 12.6 %). MS m/z (ESI): 720.2 [M+H]⁺.

¹H NMR (400 MHz, DMSO-*d*₆): δ 10.21 (s, 1H), 9.07 (s, 1H), 8.62 (d, J = 1.2 Hz, 1H), 8.41 (d, J = 1.2 Hz, 1H), 8.17 (s, 1H), 7.98 (dd, J = 9.2, 6.0 Hz, 1H), 7.47 (t, J = 9.0 Hz, 1H), 7.40 (d, J = 2.4 Hz, 1H), 7.18 (d, J = 2.4 Hz, 1H), 5.28 (d, J = 55.8 Hz, 1H), 4.73 - 4.46 (m, 4H), 4.17 - 3.86 (m, 5H), 3.12 - 2.81 (m, 4H), 2.16 - 1.78 (m, 10H).

Synthesis of Compound A256

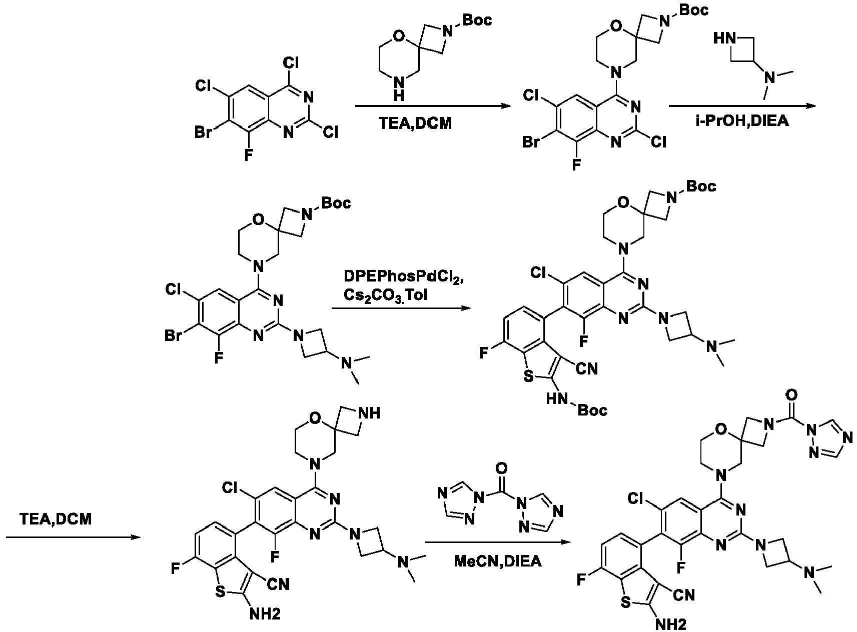

### tert-butyl 8-(7-bromo-2,6-dichloro-8-fluoroquinazolin-4-yl) -5-oxa-2,8-diazaspiro[3.5]nonane-2-carboxylate

To a solution of 7-bromo-2,4,6-trichloro-8-fluoroquinazoline(1g, 3.0mmol) and tert-butyl 5-oxa-2,8-diazaspiro[3.5]nonane-2-carboxylate (726 mg, 3.2 mmol) in DCM (15 ml) was added TEA (459 mg, 4.5 mmol). The resulting mixture was stirred for 5 hours at room temperature. TLC showed the reaction was completed, the reaction mixture was extracted with DCM washed with brine, and dried over Na₂SO₄. It was filtered and solvent was removed under reduced pressure to give a crude. The crude was purified by flash chromatography on silica gel (dichloromethane : methyl alcohol = 60: 1) to give the desired product (1.5 g, 94.9% yield) as a solid. ESI-MS m/z: 523 [M+H]⁺.

### tert-butyl 8-(7-bromo-6-chloro-2-(3-(dimethylamino) azetidin-1-yl)-8-fluoroquinazolin-4-yl)-5-oxa-2,8-diazaspiro[3.5]nonane-2-carboxylate

A solution of tert-butyl 8-(7-bromo-2,6-dichloro-8-fluoroquinazolin-4-yl) -5-oxa-2,8-diazaspiro[3.5]nonane-2-carboxylate (1.5 g, 2.9 mmol) and N,N-dimethylazetidin-3-amine (345 mg, 3.4 mmol) in i-PrOH was added DIPEA (741 mg, 5.7 mmol), The resulting mixture was heated to 80 °C and stirred for 16 h, then the solvent was removed in *vacuo,* the residue was dissolved in EA washed with brine, and dried over Na₂SO₄. It was filtered and solvent was removed under reduced pressure to give a crude. The crude was purified by flash chromatography on silica gel (dichloromethane : methyl alcohol = 60: 1) to give the desired product (1.2 g, 71.4% yield) as a solid. ESI-MS m/z: 587[M+H]⁺.

### tert-butyl 8-(7-(2-((tert-butoxycarbonyl)amino)-3-cyano -7-fluorobenzo[b]thiophen-4-yl)-6-chloro-2-(3-(dimethylamino)azetidin-1-yl)-8-fluoroquinazolin-4-yl)-5-oxa-2,8-diazaspiro[3.5]nonane-2-carboxylate

To a solution of tert-butyl 8-(7-bromo-6-chloro-2-(3-(dimethylamino)azetidin-1-yl)-8-fluoroquinazolin-4-yl)-5-oxa-2,8-diazaspiro[3.5]nonane-2-carboxylate (200 mg, 0.34 mmol), tert-butyl (3-cyano-4-(5,5-dimethyl-1,3,2-dioxaborinan-2-yl)-7-fluorobenzo[b]thiophen-2-yl)carbamate (275 mg, 0.68 mmol) and Cs₂CO₃ (334 mg, 1.02 mmol) in toluene (10 ml) was added DPEPhosPdCl₂ (24 mg, 0.07 mmol). The resulting mixture was heated at 95°C under argon and stirred overnight. It was cooled to room temperature and ethyl acetate (60 mL) was added. The organics were washed with brine, dried over Na₂SO₄. It was filtered and solvent was removed under reduced pressure to give a crude. The crude was purified by flash chromatography on silica gel (dichloromethane : methyl alcohol = 95 : 5)to afford the desired product (100 mg, 36.7% yield) as a solid. ESI-MS m/z: 798[M+H]⁺;

### 2-amino-4-(6-chloro-2-(3-(dimethylamino)azetidin-1-yl)-8-fluoro-4-(5-oxa-2,8-diazaspiro[3.5]nonan-8-yl)quinazolin-7-yl)-7-fluorobenzo[b]thiophene-3-carbonitrile

To a solution of tert-butyl 8-(7-(2-((tert-butoxycarbonyl)amino)-3-cyano-7-fluorobenzo[b]thiophen-4-yl)-6-chloro-2-(3-(dimethylamino)azetidin-1-yl)-8-fluoroquinazolin-4-yl)-5-oxa-2,8-diazaspiro[3.5]nonane-2-carboxylate (100 mg, 0.13mmol) in DCM (10 ml) was added TFA (2.5 ml). The mixture was stirred for 1 h at RT. Then the solvent was removed in *vacuo,* the residue was dissolved in DCM (15 ml), The organics were washed with aq.NaHCO3, dried over Na₂SO₄. It was filtered and solvent was removed under reduced pressure to give a crude. The crude was purified by flash chromatography on silica gel (dichloromethane : methyl alcohol = 15 :1) to afford the desired product (50 mg, 66.7% yield) as a solid. ESI-MS m/z: 598[M+H]⁺.

### 4-(4-(2-(1H-1,2,4-triazole-1-carbonyl)-5-oxa-2,8-diazaspiro[3.5]nonan-8-yl)-6-chloro-2-(3-(dimethylamino)azetidin-1-yl)-8-fluoroquinazolin-7-yl)-2-amino-7-fluorobenzo[b]thiophene-3-carbonitrile

To a solution of 2-amino-4-(6-chloro-2-(3-(dimethylamino)azetidin-1-yl)-8-fluoro-4-(5-oxa-2,8-diazaspiro[3.5]nonan-8-yl)quinazolin-7-yl)-7-fluorobenzo[b]thiophene-3-carbonitrile (50 mg, 0.08 mmol) and di(1H-1,2,4-triazol-1-yl)methanone (14 mg, 0.09 mmol) in MeCN (10 ml) was added DIPEA(16 mg, 0.12 mmol). Then the resulting mixture was stirred for 4 hours. The mixture was extracted with ethyl acetate (20 mL x 2), washed with brine, and dried over Na₂SO₄. It was filtered and solvent was removed under reduced pressure to give a crude. The crude was purified by flash chromatography on silica gel (dichloromethane : methyl alcohol = 20 : 1)to afford the desired product (21 mg, 36% yield) as a solid. ESI-MS m/z: 693[M+H]⁺.

### Synthesis of Compound A261

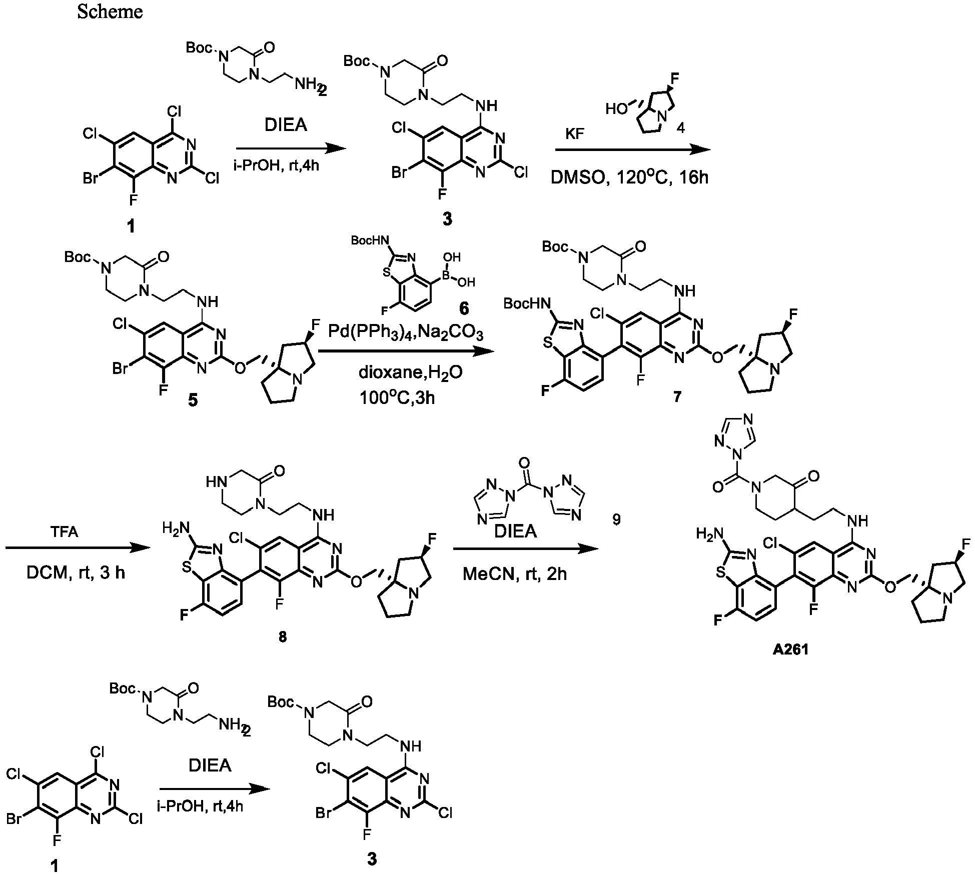

To a solution of compound 1 (1.3 g, 3.97 mmol) and compound 2 (964 mg, 3.94 mmol) in i-PrOH (12 ml) was added DIEA (1.538 g 11.9 mmol). The reaction mixture was stirred at 25 °C for 3 h. The mixture was filtered and the solid was diluted with MeOH (150 ml). The organic solution was concentrated to dryness under reduced pressure to give compound 3 (1.6 g, Yield: 75 %) as a yellow solid.

LCMS: Rt: 2.095 min; MS m/z (ESI): 536.0 [M+H]⁺.

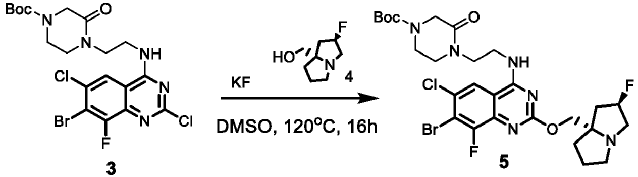

To a solution of compound 3 (1.3 g, 2.43 mmol) and 4 (1.93 g, 12.14 mmol) in DMSO (20 ml) was added KF (710 mg, 12.14 mmol). The reaction mixture was then stirred at 120 °C for 12 h. LCMS showed the reaction was completed. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The crude was purified by SGC (PE/EA=1/1) to give compound 5 (780 mg, Yield: 49 %) as a white solid.

LCMS: Rt: 1.432 min; MS m/z (ESI): 659.7 [M+H]⁺.

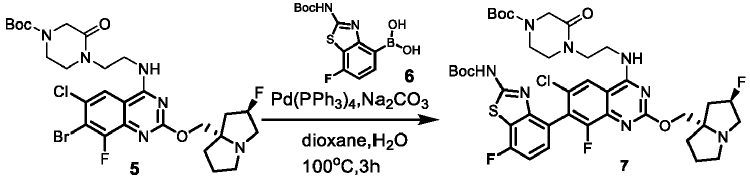

To a solution of compound 5 (400 mg, 0.61 mmol), compound 6 (379.3 mg, 1.22 mmol), and Na₂CO₃ (128.9 mg, 1.22 mmol) in 1,4-dioxane/H₂O (8 mL/ 2 mL) was added Pd(PPh₃)₄ (140.3 mg, 0.12 mmol). The mixture was stirred at 100 °C under nitrogen for 3 hours. LCMS showed the reaction was completed. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The crude was purified by SGC (PE/EA=1/1) to give compound 7 (320 mg, Yield: 62 %) as a white solid.

LCMS: Rt: 1.468 min; MS m/z (ESI): 847.2 [M+H]⁺.

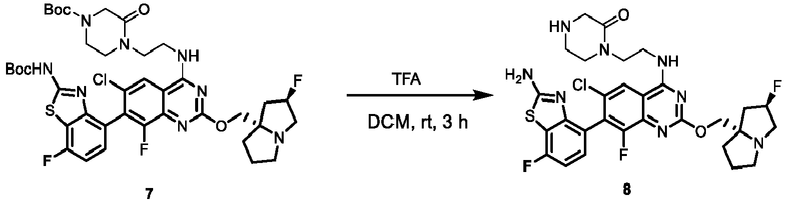

To a solution of compound 7 (300 mg, 0.354 mmol) in DCM (3 ml) was added TFA (3 ml). The reaction mixture was stirred at rt for 3 h. LCMS showed the reaction was completed. The reaction mixture removed under the reduced pressure. The residue was diluted with dichloromethane (100 mL). The solution was basified with NaHCO₃. The organic layer was washed with brine, dried over sodium sulfate, filtered, and concentrated to dryness under reduced pressure to give compound 8 (220 mg, Yield: 96 %) as a white solid.

LCMS: Rt: 1.015 min; MS m/z (ESI): 647.1 [M+H]⁺.

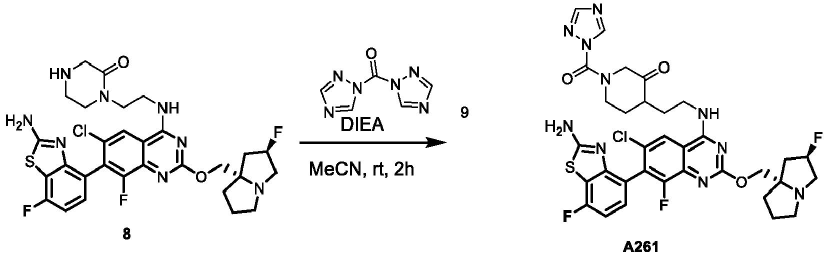

To a solution of compound 8 (50 mg, 0.077 mmol) and DIEA (30.00 mg, 0.23 mmol) in 1 mL DMF, was added compound 9 (31.74 mg, 0.19 mmol). The reaction mixture was then stirred at rt for 1 h. LCMS showed the reaction was completed. The crude was purified by prep-HPLC to give A261 (27.34 mg, Yield: 48 %).

LCMS: Rt : 1.192 min ; MS:742.1 [M+H]⁺.

¹H NMR (400 MHz, DMSO-*d*₆): δ 9.09 (s, 1H), 8.90 (s, 1H), 8.25 (s, 2H), 7.95 (s, 2H), 7.24 (dd, *J* = 8.8, 6.0 Hz, 1H), 7.07 (t, *J* = 8.8 Hz, 1H), 5.56 (d, *J* = 53.6 Hz, 1H), 4.72 - 3.96 (m, 8H), 3.66 - 3.15 (m, 8H), 2.49 - 1.95 (m, 6H).

### Synthesis of Compound A270

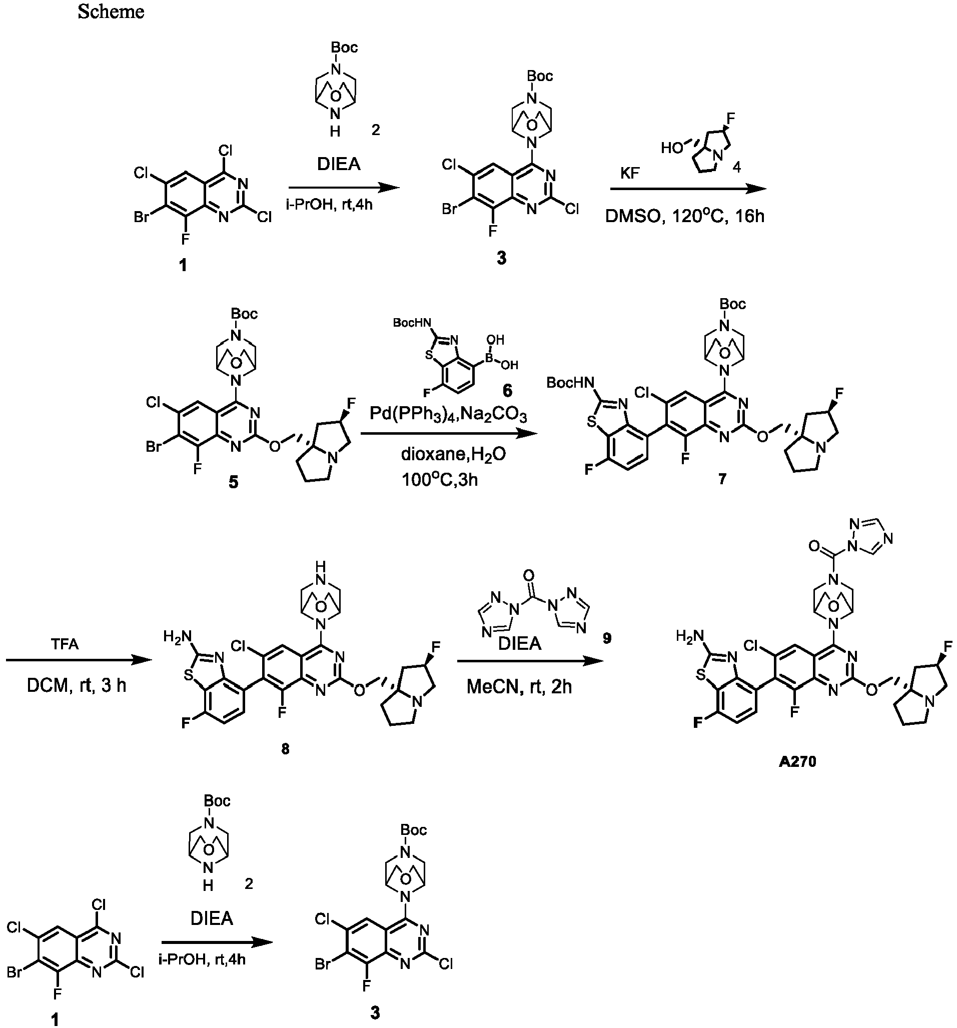

To a solution of compound 1 (1 g, 3.05 mmol) and compound 2 (696.3 mg, 3.05 mmol) in i-PrOH (10 ml) was added DIEA (1.183 g, 9.15 mmol). The reaction mixture was stirred at 25 °C for 3 h. The mixture was filtered and the solid was diluted with MeOH (150 ml). The organic solution was concentrated to dryness under reduced pressure to give compound 3 (1.5 g, Yield: 95 %) as a yellow solid.

LCMS: Rt: 2.271 min; MS m/z (ESI): 521.01 [M+H]⁺.

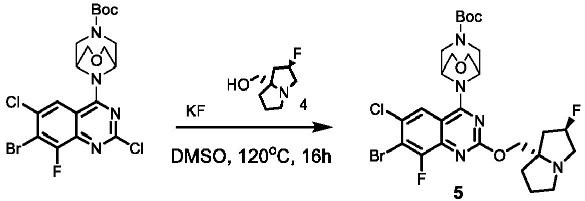

To a solution of compound 3 (1.5 g, 2.88 mmol), compound 4 (2.30 g, 14.42 mmol) in DMSO (20 ml) was added KF (782 mg, 14.42 mmol). The reaction mixture was then stirred at 120 °C for 12 h. LCMS showed the reaction was completed. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The crude was purified by SGC (PE/EA=1/1) to give compound 5 (680 mg, Yield: 37 %) as a white solid.

LCMS: Rt: 1.465 min; MS m/z (ESI): 644.1 [M+H]⁺.

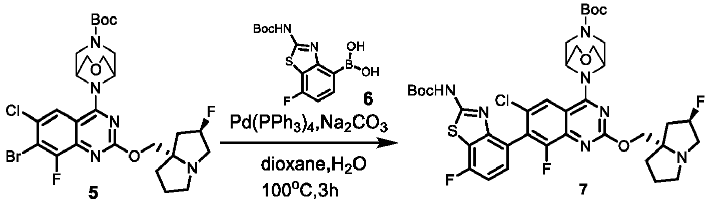

To a solution of compound 5 (300 mg, 0.466 mmol), compound 6 (291 mg, 0.933 mmol), and Na₂CO₃ (95.85 mg, 0.933 mmol) in 1,4-dioxane/H₂O (4 mL/ 1 mL) was added Pd(PPh₃)₄ (107.7 mg, 0.093 mmol). The mixture was stirred at 100 °C under nitrogen for 16 hours. LCMS showed the reaction was completed. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The crude was purified by SGC (PE/EA=1/1) to give compound 7 (200 mg, Yield: 52 %) as a white solid.

LCMS: Rt: 1.639 min; MS m/z (ESI): 832.2 [M+H]⁺.

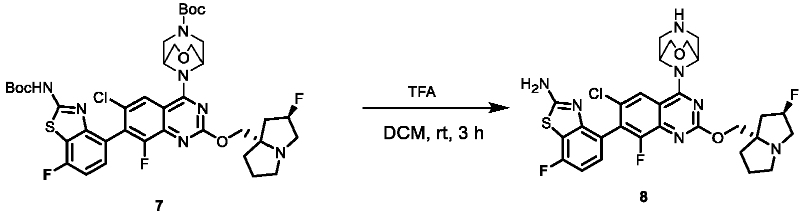

To a solution of compound 7 (200 mg, 0.24 mmol) in DCM (2 ml) was added TFA (2 ml). The reaction mixture was stirred at rt for 3 h. LCMS showed the reaction was completed. The reaction mixture removed under the reduced pressure. The residue was diluted with dichloromethane (100 mL). The solution was basified with NaHCO₃. The organic layer was washed with brine, dried over sodium sulfate, filtered, and concentrated to dryness under reduced pressure to give compound 8 (150 mg, Yield: 99 %) as a white solid.

LCMS: Rt: 1.237 min; MS m/z (ESI): 632.1 [M+H]⁺.

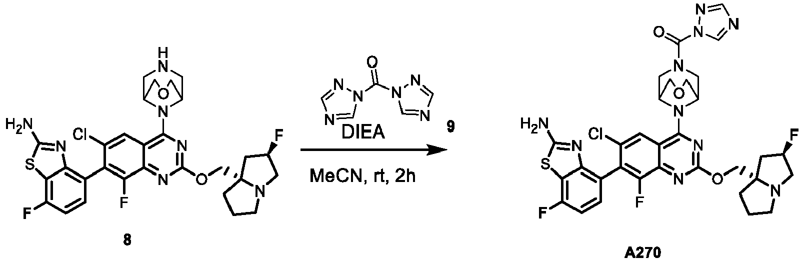

To a solution of compound 8 (50 mg, 0.079 mmol) and DIEA (30.72 mg, 0.24 mmol) in 2 mL DMF, was added compound 9 (51.9 mg, 0.32 mmol). The reaction mixture was then stirred at rt for 1 h. LCMS showed the reaction was completed. The crude was purified by prep-HPLC to give A270 (23.16 mg, Yield: 40 %).

LCMS: Rt : 7.868 min ; MS:727.3 [M+H]⁺.

¹H NMR (400 MHz, DMSO-*d6*): δ 9.12 (s, 1H), 8.31 (s, 1H), 7.90 (s, 2H), 7.82 (s, 1H), 7.27 - 6.91 (m, 2H), 5.57 (d, *J =* 52.4 Hz, 1H), 4.71 - 4.51 (m, 6H), 4.18 -3.81 (m, 7H), 3.50 - 3.20 (m, 3H). 2.40 - 1.25 (m, 6H).

### Synthesis of Compound A277

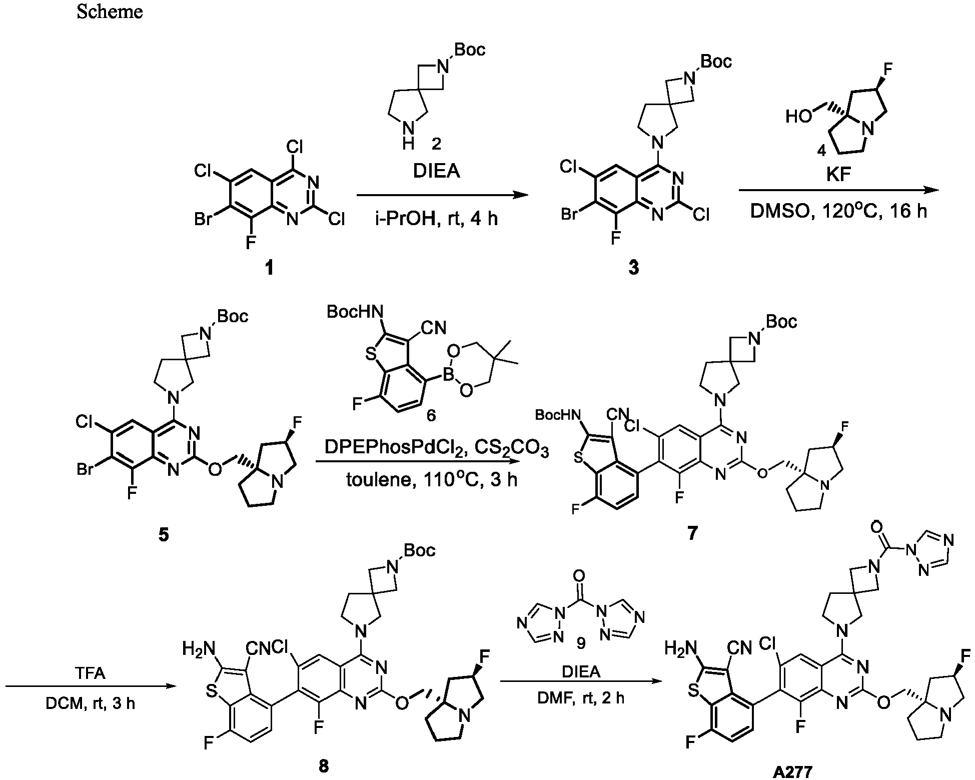

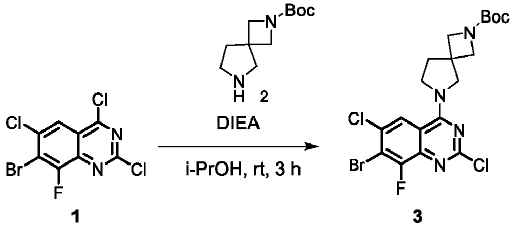

To a solution of compound 1 (1.5 g, 4.54 mmol) and compound 2 (964 mg, 4.54 mmol) in i-PrOH (15 ml) was added DIEA (1.76 g 13.62 mmol). The reaction mixture was stirred at 25 °C for 3 h. The mixture was filtered and the solid was diluted with PE (200 ml). The organic solution was concentrated to dryness under reduced pressure to give compound 3 (2 g, Yield: 87 %) as a yellow solid. MS m/z (ESI): 505.1 [M+H]⁺.

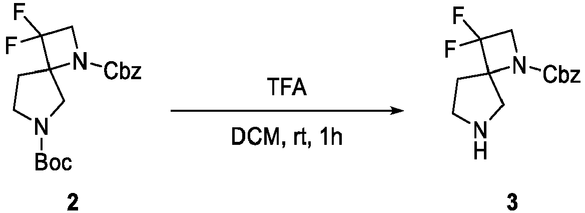

To a solution of compound 3 (1 g, 1.98 mmol) and compound 4 (1.57 g, 9.88 mmol) in DMSO (15 ml) was added KF (574 mg, 9.88 mmol). The reaction mixture was stirred at 120 °C for 16 h. The mixture was poured into ice water (100 mL), and the solution was extracted with ethyl acetate (200 mL x 3). The organic layer was washed with brine (200 mL), dried over anhydrous sodium sulfate, filtered and the filtrate was concentrated to dryness under reduced pressure. The residue was purified by silica gel column (PE/EA=1/1) to give compound 5 (550 mg, Yield: 44 %) as a white solid. MS m/z (ESI): 628.9 [M+H]⁺.

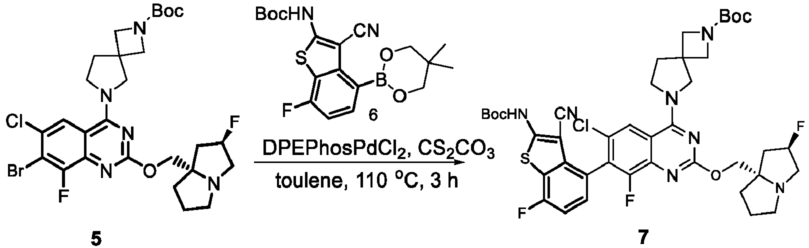

To a solution of compound 5 (450 mg, 0.72 mmol), compound 6 (434 mg, 1.07 mmol), and Cs₂CO₃ (103 mg, 2.15 mmol) in toluene (10 mL) was added DPEPhosCl₂ (92 mg, 0.14 mmol). The mixture was stirred at 110 °C under nitrogen for 3 hours. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The residue was purified by silica gel column (PE/EA=1/1) to give compound 7 (350 mg, Yield: 58 %) as a white solid. MS m/z (ESI): 840.2 [M+H]⁺.

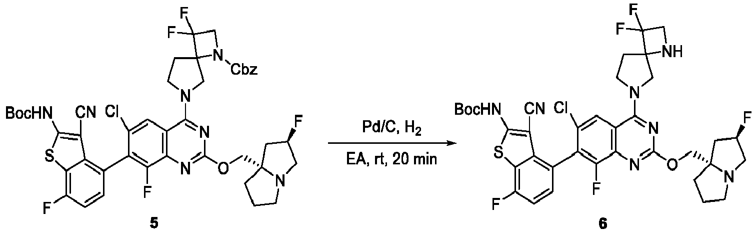

To a solution of compound 7 (350 mg, 0.42 mmol) in DCM (1 ml) was added TFA (1 ml). The reaction mixture was stirred at 25 °C for 1 h. The solvent was removed under reduced pressure. The reaction mixture was diluted with a.q. NaHCO₃ (150 mL) and DCM (50 mL). The organic layer was washed with brine, dried over sodium sulfate, filtered, and concentrated to afford compound 8 (300 mg, 82 %) as a yellow solid. MS m/z (ESI): 640.2 [M+H]⁺.

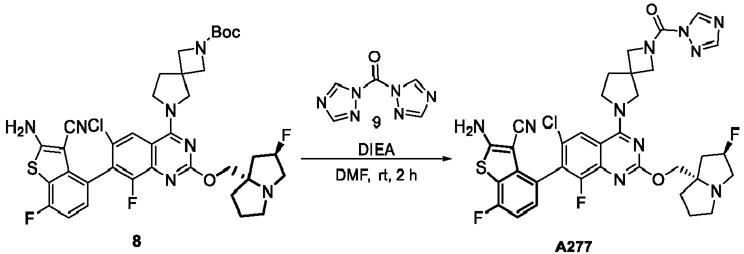

To a solution of compound 8 (70 mg, 0.11 mmol) and DIEA (43 mg, 0.33 mmol) in 1 mL DMF, was added compound 9 (54 mg, 0.65 mmol). The reaction mixture was then stirred at rt for 1 h. LCMS showed the reaction was completed. The crude was purified by prep-HPLC to give A277 (3.09 mg, Yield: 3.8 %). MS m/z (ESI): 735.3 [M+H]⁺.

¹H NMR (400 MHz, DMSO-*d*₆): δ 9.16 (s, 1H), 8.24 (d, J = 4.4 Hz, 1H), 8.19 - 8.02 (m, 3H), 7.22 (s, 1H), 7.15 (t, J = 8.8 Hz, 1H), 5.30 (d, J = 55.6 Hz, 1H), 4.78 - 4.47 (m, 2H), 4.30 - 3.80 (m, 8H), 3.20 - 2.80 (m, 4H), 2.45-1.75 (m, 8H).

### Synthesis of Compound A283

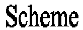

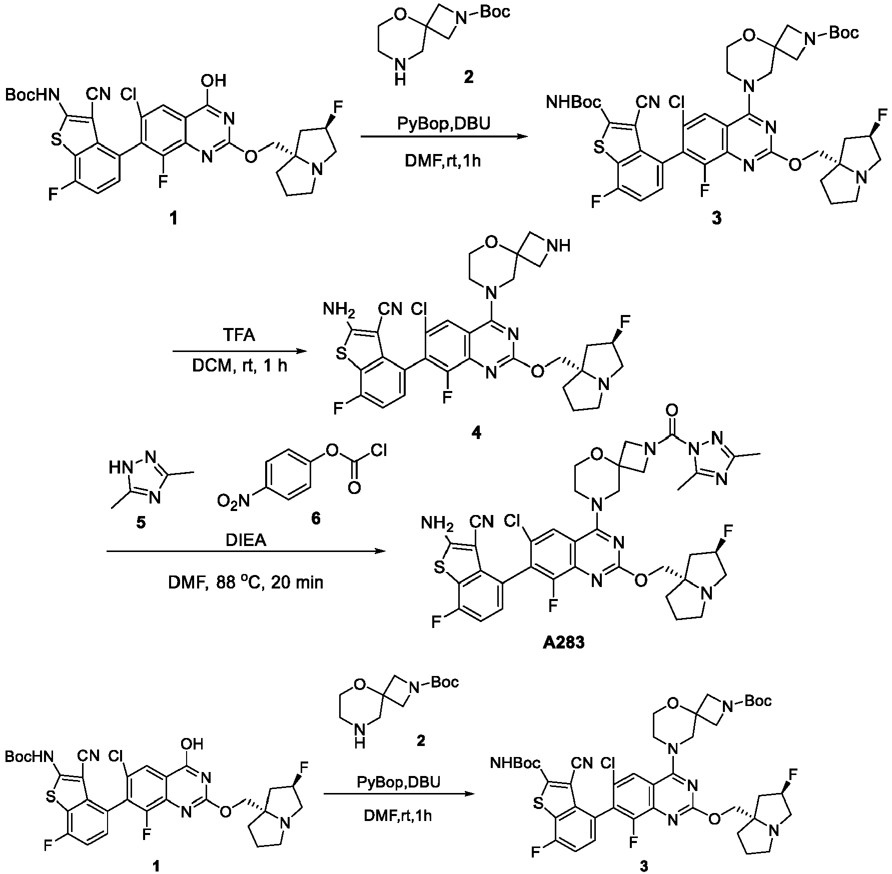

To a solution of compound 1 (300 mg, 0.465 mmol), compound 2 (137 mg, 0.600 mmol) and PyBop (604 mg, 1.163 mmol) in DMF (4 ml) was added DBU (232 mg, 1.860 mmol). The reaction mixture was stirred at r.t for 1 h. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by prep-TLC (DCM/MeOH=15/1) to give compound 3 (300 mg, Yield: 75.46 %) as a white solid.

MS m/z (ESI): 856.2 [M+H]⁺.

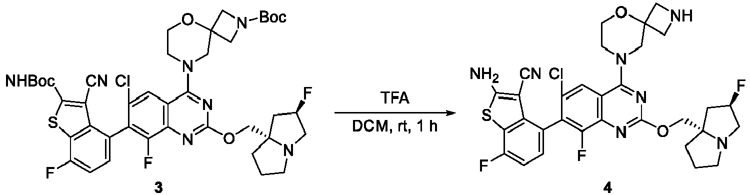

To a solution of compound 3 (300 mg, 0.351 mmol) in DCM (3 ml) was added TFA (3 ml). The reaction mixture was stirred at r.t for 1 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated. The residue was purified by work up to give compound 4 (210 mg, Yield: 91.34%) as a yellow solid.

MS m/z (ESI): 656.0 [M+H]⁺.

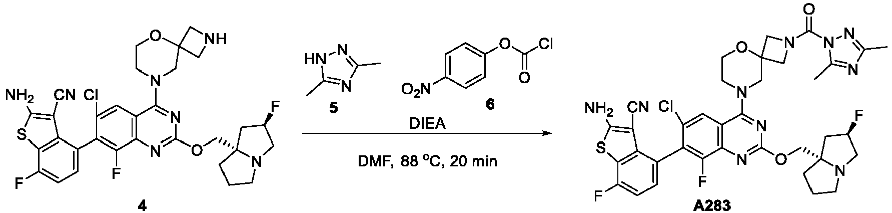

To a solution of compound 5 (615 mg, 6.35 mmol) and DIEA (1.23 g, 9.53 mmol) in 10 mL MeCN, was added 4-nitrophenyl carbonochloridate (1.41 g, 6.99 mmol) at 0 °C under N₂ atmosphere. The reaction mixture was then stirred at rt for 1 h. To a solution of compound 4 (50 mg, 0.076 mmol) in DMF (0.5 mL) was added above reaction mixture (0.15 mL) at 60 °C under N₂ atmosphere.

The solution was stirred at 60 °C for 20 min. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by Prep-HPLC (FA) to give A283 (10.17 mg, Yield: 17.20%) as a white solid.

MS m/z (ESI): 779.3 [M+H]⁺.

¹H NMR (400 MHz, DMSO-*d*₆): δ 8.11 (s, 2H), 7.95 (s, 1H), 7.26 - 7.13 (m, 2H), 5.26 (d, *J* = 53.2 Hz, 1H), 4.59 - 4.49(m, 1H), 4.38 (d, *J=* 10.8 Hz, 1H), 4.20 - 3.73 (m, 10H), 3.15 - 2.90 (m, 3H), 2.81- 2.80 (m, 1H), 2.51 (s, 3H), 2.22 (s, 3H), 2.12 (d*, J =* 9.6 Hz, 1H), 2.03 - 1.74 (m, 5H).

### Synthesis of Compound A284

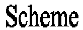

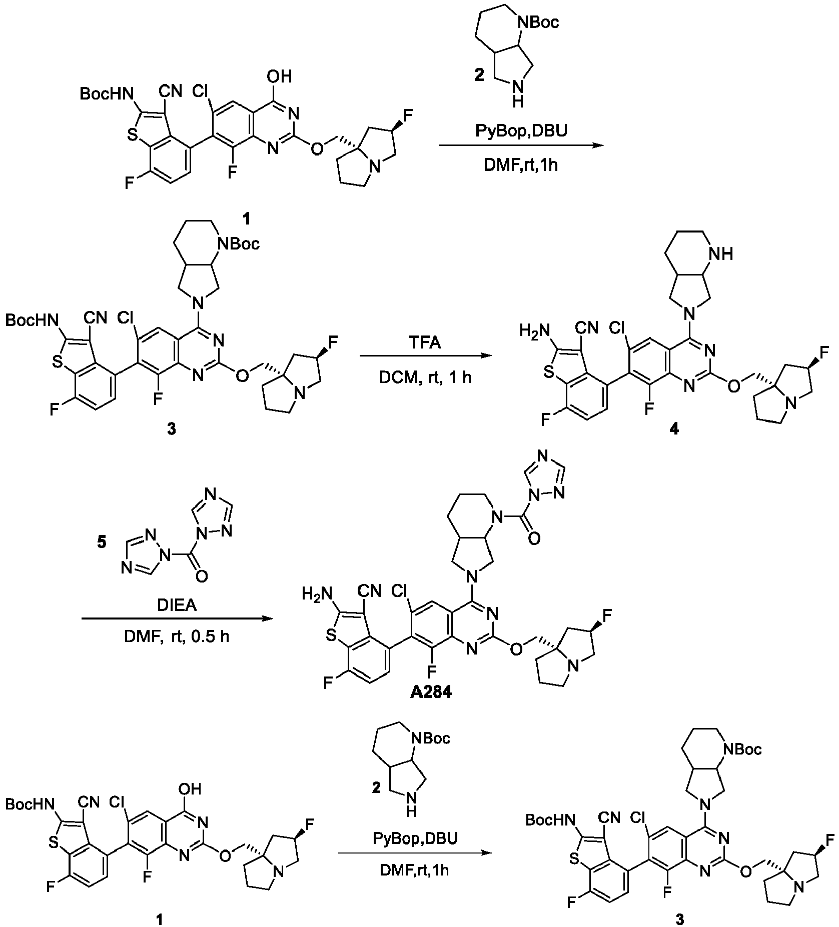

To a solution of compound 1 (100 mg, 0.155 mmol), compound 2 (45.5 mg, 0.201 mmol) and PyBop (201 mg, 0.388 mmol) in DMF (3 ml) was added DBU (77 mg, 0.620 mmol). The reaction mixture was stirred at r.t for 1 h. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by prep-TLC (DCM/MeOH=15/1) to give compound 3 (90 mg, Yield: 68.07 %) as a white solid.

MS m/z (ESI): 854.2 [M+H]⁺.

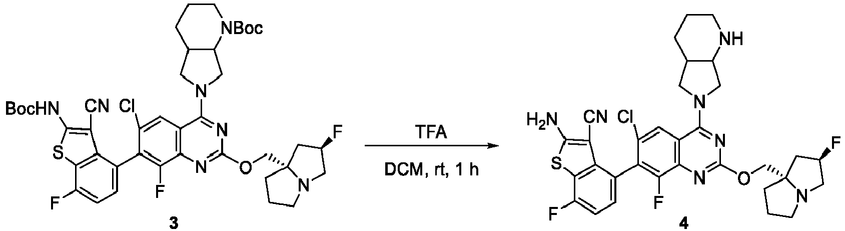

To a solution of compound 3 (90 mg, 0.105 mmol) in DCM (2 ml) was added TFA (2 ml). The reaction mixture was stirred at r.t for 1 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated. The residue was purified by work up to give compound 4 (35 mg, Yield: 51.05%) as a yellow solid.

MS m/z (ESI): 654.3 [M+H]⁺.

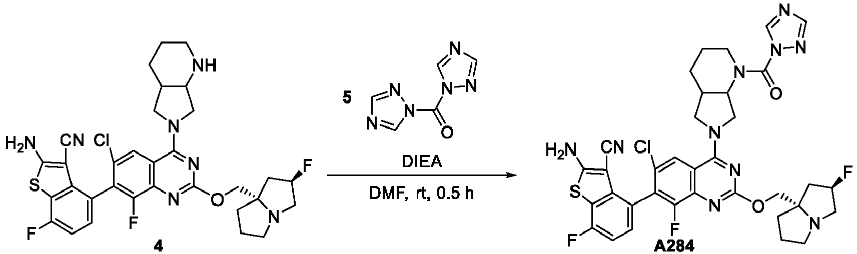

To a solution of compound 4 (35 mg, 0.054 mmol) and compound 5 (17.6 mg, 0.107 mmol) in DMF (2 mL) was added DIEA (19 mg, 0.15 mmol) at r.t. The solution was stirred at r.t for 0.5 hour. The mixture was poured into ice water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by Prep-HPLC to give A284 (10.15 mg, Yield: 25.13%) as a white solid.

MS m/z (ESI): 749.1 [M+H]⁺.

¹H NMR (400 MHz, DMSO-*d*₆): δ 9.09 (d, *J=* 2.4 Hz, 1H), 8.23 (s, 1H), 8.19 (s, 1H), 8.10 (s, 2H), 7.25 - 7.12 (m, 2H), 5.33 (d*, J =* 54.4 Hz, 1H), 4.91 (s, 1H), 4.21 (d, *J = 4.4* Hz, 2H), 4.11 - 3.97 (m, 4H), 3.72 (d*, J =* 10.0 Hz, 2H), 3.15 - 2.90 (m, 3H), 2.84 - 2.79 (m, 2H), 2.20 - 1.90 (m, 3H), 1.85 - 1.75 (m, 5H), 1.62 - 1.51 (m, 2H).

### Synthesis of Compound A287

### 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)piperazine-1-carbonyl)-1H-pyrrole-2-carbonitrile

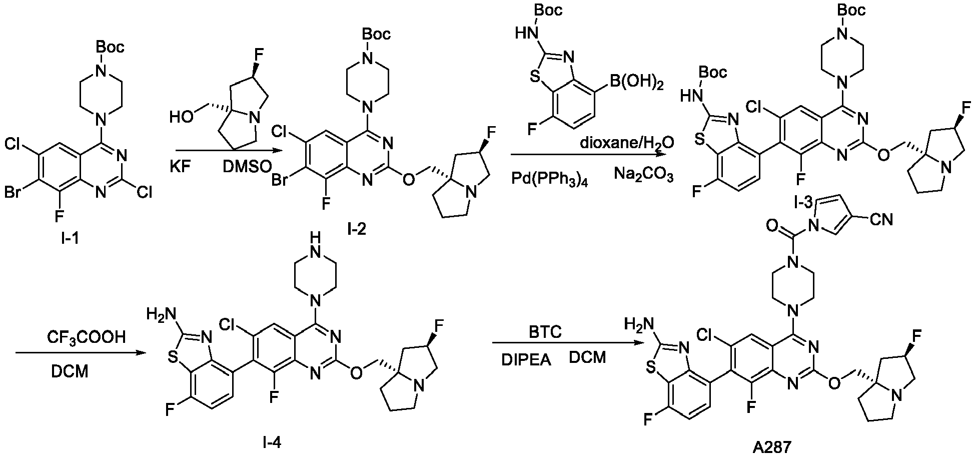

### tert-butyl 4-(7-bromo-6-chloro-8-fluoro-2-(((2R,7aS)-2-fluorotetrahydro-1H-pyrrolizin-7a(5H)-yl)methoxy)quinazolin-4-yl)piperazine-1-carboxylate

To a solution of tert-butyl4-(7-bromo-2,6-dichloro-8-fluoroquinazolin-4-yl)piperazine-1-carboxylate (1-1) (200 mg, 0.42 mmol, 1 eq) in DMSO, then added ((2R,7aS)-2-fluorotetrahydro-1H-pyrrolizin-7a(5H)-yl)methanol (133 mg,0. 82 mmol, 2eq) and KF (185 mg, 3.2 mmol, 8eq). The resulting solution was stirred to120 °C. The mixture was quenched with water, extracted with EA. The organic layer was concentrated and the residue was purified on a silica gel column to afford product (1-2) (130 mg). ESI-MS *m*/*z*: 602.13[M+H]⁺.

### tert-butyl 4-(7-(2-((tert-butoxycarbonyl)amino)-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)piperazine-1-carboxylate

To a solution of compound 1-2 (130 mg, 0.22 mmol) in dioxane/H20(3:1), then added (2-((tert-butoxycarbonyl)amino)-7-fluorobenzo[d]thiazol-4-yl)boronic acid(168mg,0.66mmol),Pd(PPh3)4(23mg,0.02mmol), Na₂CO₃ (63.6 mg, 0.6 mmol). The resulting mixture was stirred at 100 °C under argon overnight. The resulting mixture was diluted with water (20 mL) and extracted with EtOAc (15 mL x 2). The combined organic layer was washed with water and brine, dried over anhydrous Na₂SO₄, filtered and concentrated in vacuo. The residue was purified by prep-TLC to afford the desired product I-3 (100 mg) ESI-MS *m*/*z*: 790[M+H]⁺.

### 4-(6-chloro-8-fluoro-2-(((2R,7aS)-2-fluorotetrahydro-1H-pyrrolizin-7a(5H)-yl)methoxy)-4-(piperazin-1-yl)quinazolin-7-yl)-7-fluorobenzo[d]thiazol-2-amine

To a stirred solution of compound I-3 (100 mg, 0.13 mmol) in DCM (6 mL), was CF₃COOH (2 mL) and the resulting mixture was stirred at RT for 1 h. The mixture was concentrated in vacuo. The residue was extracted with DCM. The mixture was concentrated and the residue was purified by prep-TLC plate to afford the desired product I-4 (80 mg) ESI-MS *m*/*z*: 590[M+H]⁺ .

### 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)piperazine-1-carbonyl)-1H-pyrrole-3-carbonitrile

To a solution of compound 1H-pyrrole-3-carbonitrile (10.13 mg, 0.11 mmol)were added BTC (35.52 mg, 0.08 mmol) and DIPEA (30.96 mg, 0.24 mmol), and the mixture was stirred for 30 min. Compound I-4 (50 mg, 0.08 mmol) was added, and then stirred for 1h. The mixture was extracted with DCM and concentrated. The residue was purified by prep-TLC to afford the desired product I-5 (25 mg). ESI-MS *m*/*z:* 707 [M+H]⁺.

### Synthesis of Compound A294

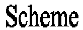

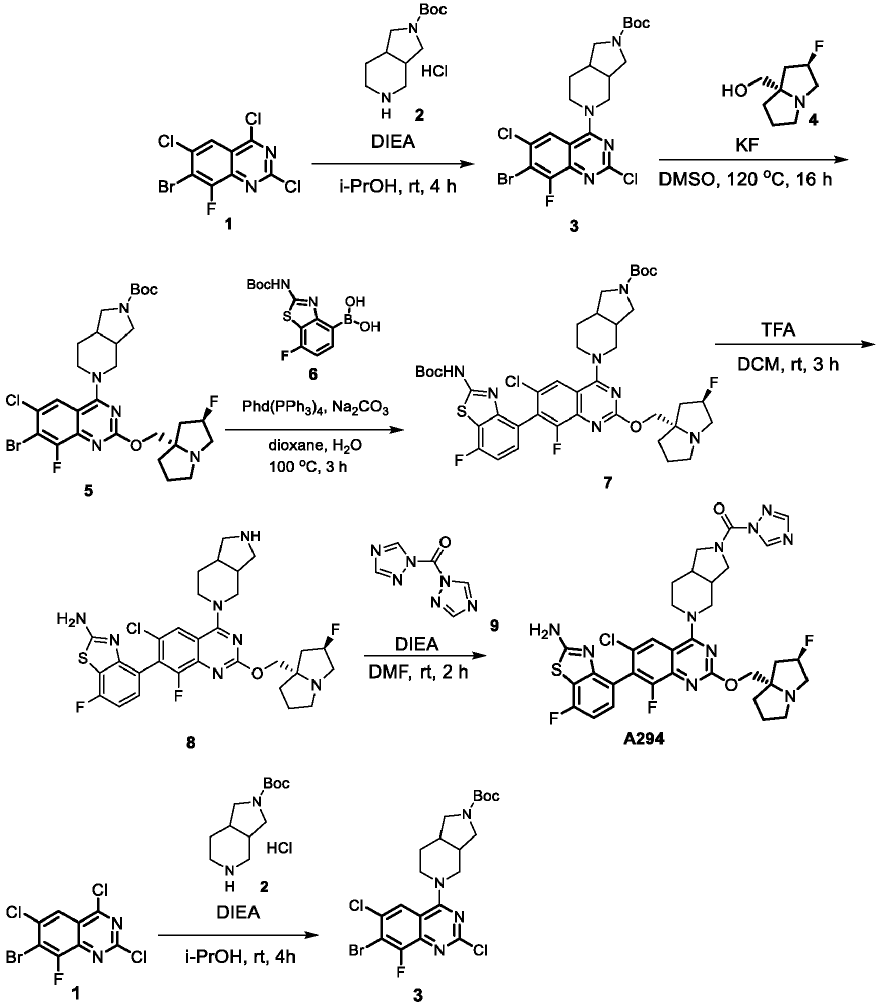

To a solution of compound 1 (1.3 g, 3.94 mmol) and compound 2 (890 mg, 3.94 mmol) in i-PrOH (20 ml) was added DIEA (2.04 g 15.76 mmol). The reaction mixture was stirred at 25 °C for 3 h. The mixture was poured into ice water (200 mL). The mixture was filtered and the solid was diluted with MeOH (150 ml). The organic solution was concentrated to dryness under reduced pressure to give compound 3 (1.8 g, Yield: 88.2 %) as a yellow solid.

LCMS: Rt: 2.147 min; MS m/z (ESI): 519.1 [M+H]⁺.

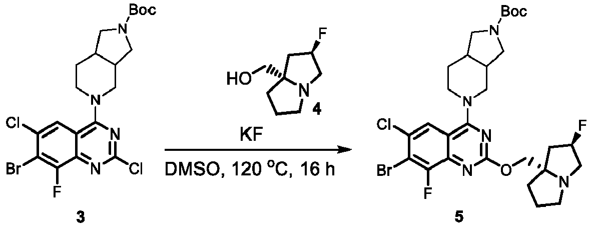

To a solution of compound 3 (1.5 g, 2.88 mmol), and 4 (2.3 g, 14.42 mmol) in DMSO (15 ml) was added KF (836 mg, 14.42 mmol). The reaction mixture was then stirred at 120 °C for 12 h. LCMS showed the reaction was completed. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The crude was purified by SGC (PE/EA=1/1) to give compound 5 (800 mg, Yield: 43.3 %) as a white solid.

LCMS: Rt: 1.380 min; MS m/z (ESI): 642.2 [M+H]⁺.

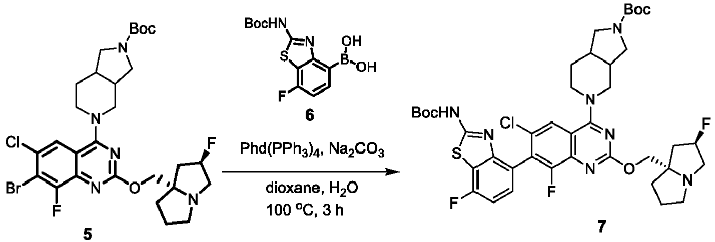

To a solution of compound 5 (800 mg, 1.25 mmol), compound 6 (587 mg, 1.88 mmol), and Na₂CO₃ (265 mg, 2.5 mmol) in 1,4-dioxane/H₂O (12 mL/ 3 mL) was added Pd(PPh₃)₄ (150 mg, 0.13 mmol). The mixture was stirred at 100 °C under nitrogen for 16 hours. LCMS showed the reaction was completed. The mixture was poured into water (200 mL), and the solution was extracted with ethyl acetate (200 mL X 3). The organic layer was washed with brine (100 mL), dried over anhydrous sodium sulfate, filtered and the filtrate was concentrated to dryness under reduced pressure. The crude was purified by SGC (PE/EA=1/1) to give compound 7 (700 mg, Yield: 67.7 %) as a white solid.

LCMS: Rt: 1.605 min; MS m/z (ESI): 831.2 [M+H]⁺.

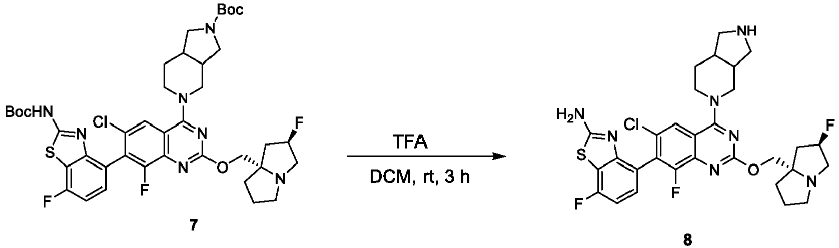

To a solution of compound 7 (700 mg, 0.84 mmol) in DCM (10 ml) was added TFA (6 ml). The reaction mixture was stirred at rt for 3 h. LCMS showed the reaction was completed. The reaction mixture removed under the reduced pressure. The crude was purified by work up to give compound 8 (570 mg, Yield: 100 %) as a white solid.

LCMS: Rt: 1.033 min; MS m/z (ESI): 630.1 [M+H]⁺.

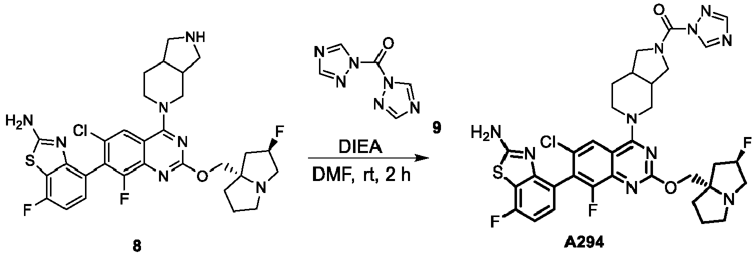

To a solution of compound 8 (50 mg, 0.08 mmol) and compound 9 (26 mg, 0.16 mmol) in 2 ml DMF was added DIEA (31 mg, 0.24 mmol) at rt. The reaction mixture was then stirred at rt for 30 min. The mixture was poured into water (50 mL), and the solution was extracted with ethyl acetate (50 mL x 3). The organic layer was washed with brine (50 mL), dried over anhydrous sodium sulfate, filtered and the filtrate was concentrated to dryness under reduced pressure. The residue was purified by Prep-HPLC to give compound A294 (5.17 mg, Yield: 9%) as a white solid.

LCMS: Rt : 1.260 min ; MS:725.1 [M+H]⁺.

¹H NMR (400 MHz, DMSO-*d*₆): δ 9.12 (d, J = 7.2 Hz, 1H), 8.23 (d, J = 23.6 Hz, 1H), 7.92 (s, 1H), 7.90 (s, 2H), 7.27 - 7.20 (m, 1H), 7.12 - 7.04 (m, 1H), 5.57 (d, J = 52.4 Hz, 2H), 4.57 (d, J = 5.2 Hz, 2H), 4.02 (s, 3H), 3.92 - 3.81 (m, 3H), 3.76 (s, 2H), 3.65 - 3.45 (m, 2H), 3.31 (s, 1H), 2.71 - 2.54 (m, 3H), 2.47 (s, 1H), 2.39 - 2.13 (m, 3H), 2.09 - 1.72 (m, 3H).

### Synthesis of Compound A295

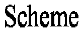

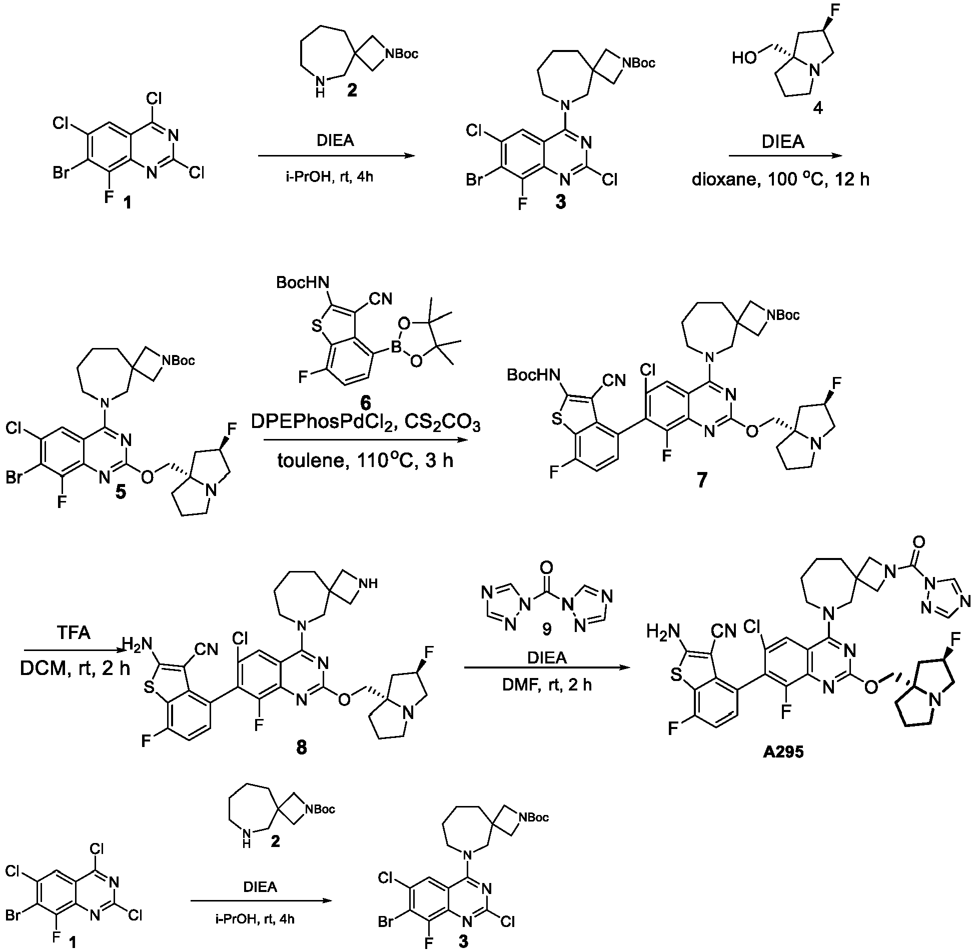

To a solution of compound 1 (684 mg, 2.09 mmol) and compound 2 (500 mg, 2.09 mmol) in i-PrOH (8 ml) was added DIEA (807 mg, 6.27 mmol). The reaction mixture was stirred at 25 °C for 4 h. The mixture was poured into ice water (200 mL). The mixture was filtered and the solid was diluted with MeOH (150 ml). The organic solution was concentrated to dryness under reduced pressure to give compound 3 (850 mg, Yield: 76.4%) as a white solid.

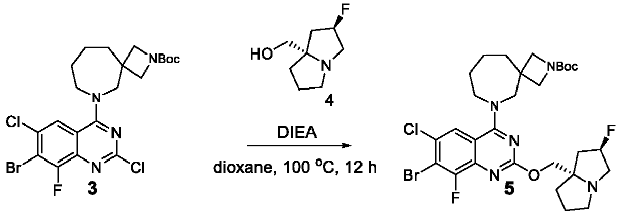

A mixture of compound 3 (800 mg, 1.50 mmol), compound 4 (717.7 mg, 4.51 mmol) and DIEA (583.04 mg, 4.51 mmol) in dioxane (10 ml) was stirred at 100 °C for 12 h. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (PE/EA=1/1) to give compound 5 (450 mg, Yield: 45.8 %) as a white solid. MS m/z (ESI): 656.2 [M+H]⁺.

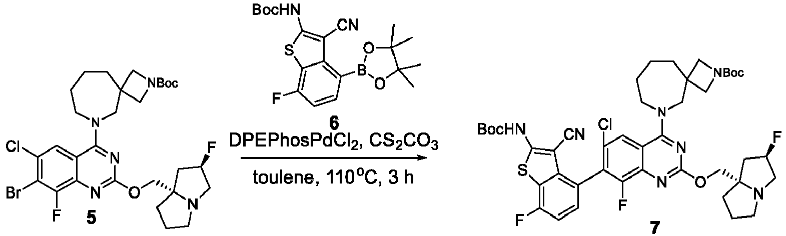

To a solution of compound 5 (450 mg, 0.69 mmol), compound 6 (431 mg, 1.03 mmol), and Cs₂CO₃ (671 mg, 2.06 mmol) in toulene (8 mL) was added Pd(DPEPhos)Cl₂ (98 mg, 0.14 mmol). The mixture was stirred at 110 °C under nitrogen for 3 hours. The mixture was diluted with EtOAc (100 mL) and water (100 ml). The organic layer was washed with brine (50 mL), dried over Na₂SO₄, filtered and concentrated. The residue was purified by silica gel column (PE/EA=1/1) to give compound 7 (320 mg, Yield: 53%) as a yellow solid. MS m/z (ESI): 868.3 [M+H]⁺.

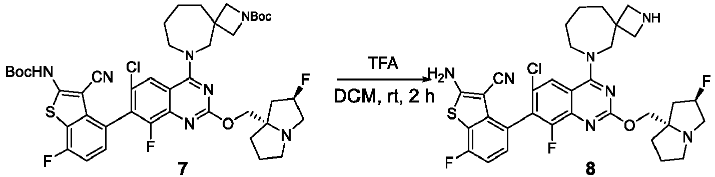

To a solution of compound 7 (320 mg, 0.369 mmol) in DCM (10 ml) was added TFA (10 ml). The reaction mixture was stirred at rt for 2 h. The solvent was removed under reduced pressure. The reaction mixture was diluted with a.q. NaHCO₃ (20 mL) and DCM (50 mL). The organic layer was washed with brine (50 mL), dried over anhydrous sodium sulfate, filtered and the filtrate was concentrated to dryness under reduced pressure. The residue was purified by Prep-HPLC (FA) to give compound 8 (230 mg, Yield: 93.4 %) as a white solid. MS m/z (ESI): 668.2 [M+H]⁺.

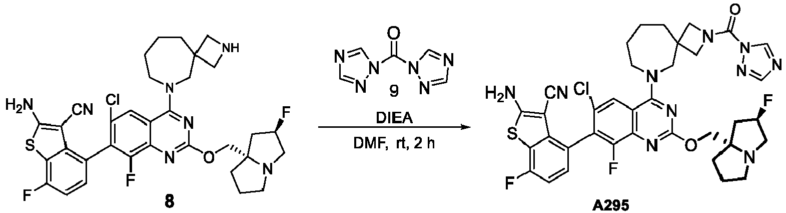

To a solution of compound 8 (25 mg, 0.037 mmol) and DIEA (43 mg, 0.111 mmol) in 0.8 mL DMF, was added compound 9 (43 mg, 0.111 mmol). The reaction mixture was then stirred at rt for 1 h. LCMS showed the reaction was completed. The crude was purified by prep-HPLC to give A295 (1.56 mg, Yield: 5.5 %). MS m/z (ESI): 763.2 [M+H]⁺.

¹H NMR (400 MHz, DMSO-*d*₆): δ 9.17 (s, 1H), 8.26 (s, 1H), 8.12 (s, 3H), 7.29 - 7.12 (m, 2H), 5.58 (dd, J = 53.4, 21.2 Hz, 1H), 4.73 - 3.91 (m, 14H), 2.43 - 1.57 (m, 12H).

### Synthesis of Compound A298

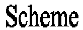

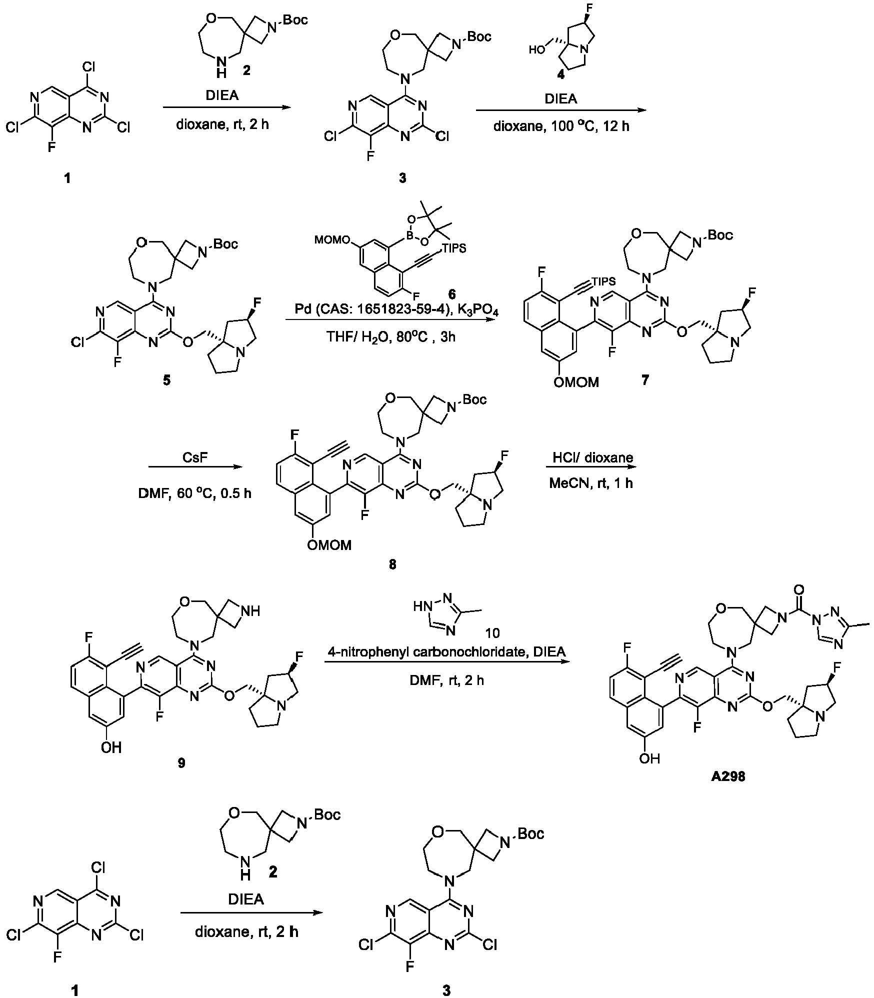

To a solution of compound 2 (579 mg, 2.391 mmol) and N,N-Diisopropylethylamine (927 mg, 7.174 mmol) in dry dioxane (5 mL) was added compound 1 (600 mg, 2.391 mmol). The reaction was stirred at room temperature for 2 hr. The mixture was poured into ice water (100 mL). The mixture was filtered and the solid was collected to obtain compound 3 as a yellow solid. (550 mg, Yield: 50 %). MS m/z (ESI): 458.2 [M+H]⁺.

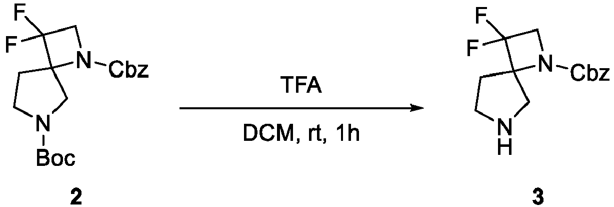

To a solution of compound 3 (520 mg, 1.138 mmol) and DIEA (441 mg, 3.413 mmol) in dioxane (10 ml) was added compound 4 (543 mg, 3.413 mmol). The reaction mixture was stirred at 100 °C for 12 h. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (DCM/MeOH=20/1) to give compound 5 (300 mg, Yield: 45 %) as a white solid. MS m/z (ESI): 581.2 [M+H]⁺.

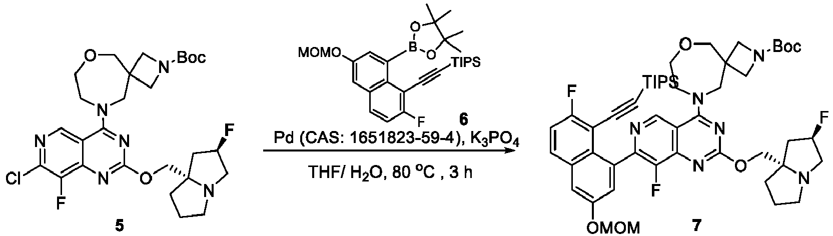

To a solution of compound 5 (300 mg, 0.517 mmol), compound 6 (318 mg, 0.620 mmol) and K₃PO₄ (329 mg, 1.551 mmol) in dry THF/H₂O (16 mL/4 mL) was added cataCXium A Pd G3 (38 mg, 0.052 mmol). The mixture was stirred at 80 °C for 3 hr under N₂. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure. The residue was purified by silica gel column (EA: MeOH=20:1) to give compound 7 (260 mg, Yield: 54 %) as a white solid. MS m/z (ESI): 931.4 [M+H]⁺.

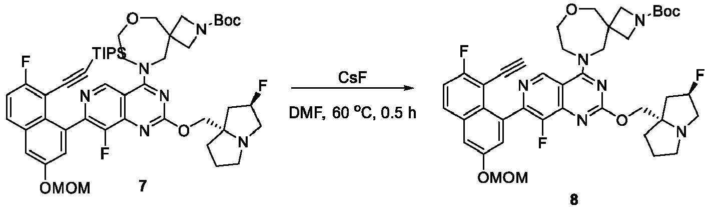

To a solution of compound 7 (230 mg, 0.247 mmol) in dry DMF (5 mL) was added CsF (376 mg, 2.472 mmol). The mixture was stirred at 60 °C for 0.5 hr. The mixture was poured into ice water (100 mL), and the solution was extracted with ethyl acetate (100 mL x 3). The organic layer was washed with brine (100 mL), dried over anhydrous sodium sulfate, filtered and the filtrate was concentrated to dryness under reduced pressure to give compound 8 (180 mg, Yield: 94 %) as a white solid. MS m/z (ESI): 775.4 [M+H]⁺.

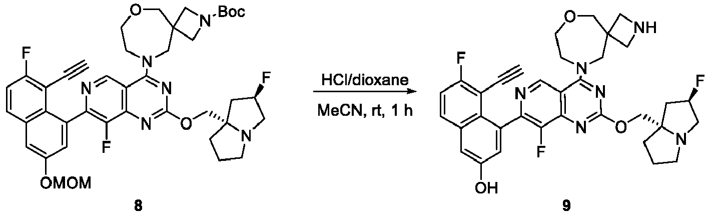

To a solution of compound 8 (180 mg, 0.232 mmol) in CH₃CN (4 mL) was added HCl/dioxane (2 mL). The solution was stirred at 20 °C for 1 h. Then the solution was added dropwise to n-hexane (50 mL), the upper clear liquid was removed and the residue was dissolved in a solution of DCM (100 mL) and MeOH (5 mL). The organic layer was washed by aqueous a.q. NaHCO₃, and brine. The organic layer was separated, dried over Na₂SO₄, concentrated to give compound 9 (130 mg, Yield: 89 %) as a yellow solid. MS m/z (ESI): 631.3 [M+H]⁺.

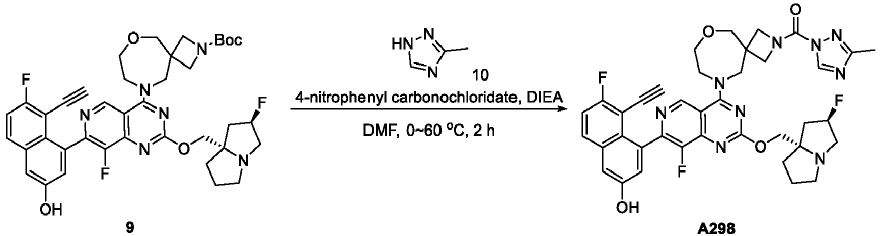

To a solution of compound 10 (527 mg, 6.35 mmol) and DIEA (1.23 g, 9.53 mmol) in 10 mL MeCN, was added 4-nitrophenyl carbonochloridate (1.41 g, 6.99 mmol) at 0 °C under N₂ atmosphere. The reaction mixture was then stirred at rt for 1 h. To a solution of compound 9 (40 mg, 0.0635 mmol) in DMF (0.5 mL), was added above reaction mixture (0.1 mL) at 60 °C under N₂ atmosphere. LCMS showed the reaction was completed. The crude was purified by prep-HPLC to give A298 (6.61 mg, Yield: 14 %). MS m/z (ESI): 740.4 [M+H]⁺.

¹H NMR (400 MHz, DMSO-*d*₆): δ 10.28 (s, 1H), 9.17 (s, 1H), 9.01 (s, 1H), 8.31 (s, 1H), 8.03 - 7.91 (m, 1H), 7.52 - 7.37 (m, 2H), 7.18 (s, 1H), 5.26 (d, J = 54.0 Hz, 1H), 4.75 - 4.25 (m, 4H), 4.21 - 3.91 (m, 11H), 3.08 - 2.82 (m, 4H), 2.32 (s, 3H), 2.25 - 1.75 (m, 6H).

### Synthesis of Compound A300

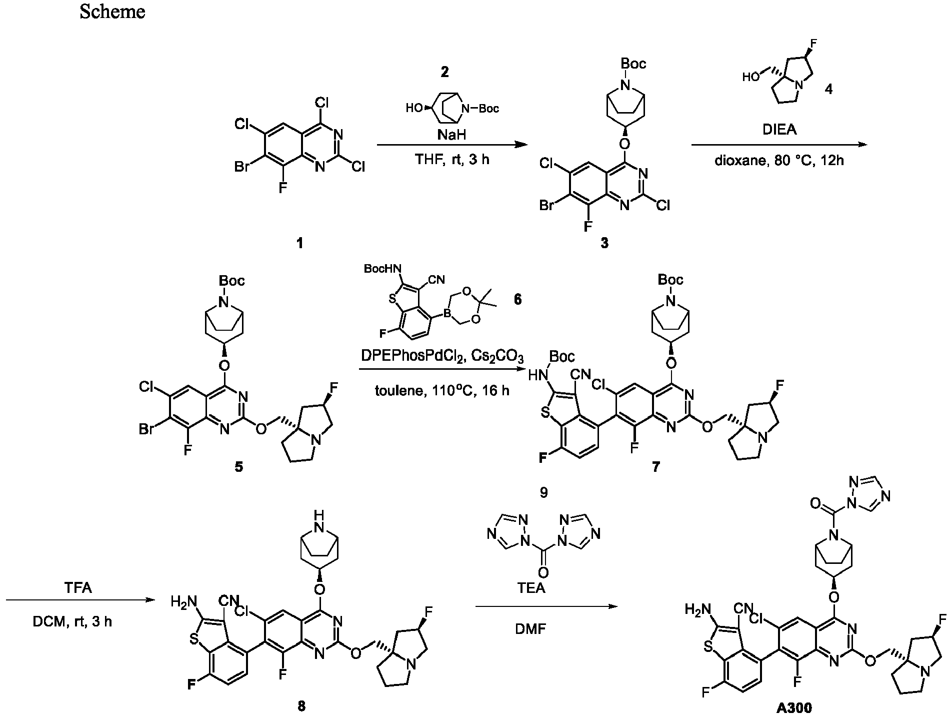

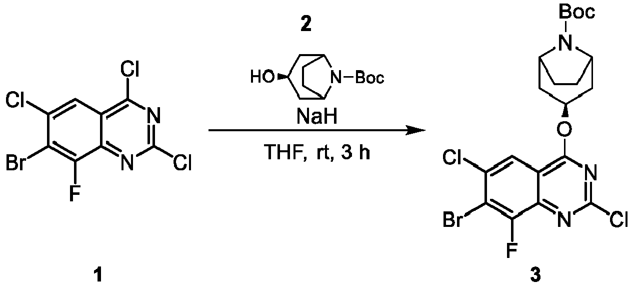

To a solution of compound 2 (1.38 g, 6.10 mmol) in dry THF (20 mL) was added NaH (60%) (366 mg, 9.15 mmol) at 0 °C. The mixture was stirred room temperature for 0.5 h. Then, compound 1 (2.0 g, 6.10 mmol) was added. The mixture was stirred room temperature for 2 h. The mixture was poured into ice water (100 mL), and the solution was extracted with ethyl acetate (100 mL x 3). The organic layer was washed with brine (50 mL), dried over anhydrous sodium sulfate, filtered and the filtrate was concentrated to dryness under reduced pressure, which was purified by SGC (MeOH:DCM=40:1) to give compound 3 (1.9 g, Yield: 60.0 %) as a white solid.

¹H NMR (400 MHz, CDCl3): δ 7.92 (d, *J* = 1.6 Hz, 1H), 5.77 (t, *J* = 4.8 Hz, 1H), 4.30 (d, *J* = 26.4 Hz, 2H), 2.36 (s, 2H), 2.19 - 2.06 (m, 4H), 2.02 (d, *J=* 15.6 Hz, 2H), 1.49 (s, 9H).

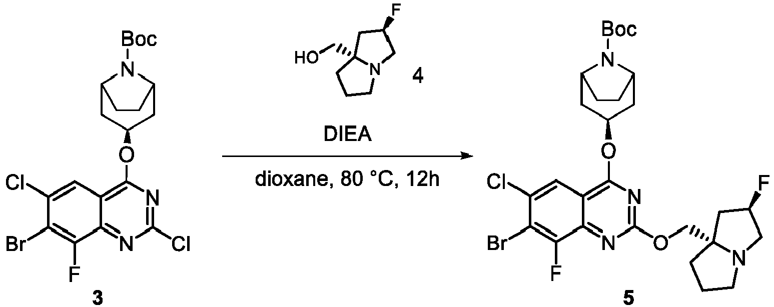

A mixture of compound 3 (1.9 g, 3.66 mmol), compound 4 (2.04 g, 12.81 mmol,) and DIEA (2.37 g, 18.3 mmol) in DMSO (10 ml) was stirred at 120 °C for 16 h. The mixture was poured into water (100 mL), and the solution was extracted with ethyl acetate (100 mL x 3). The organic layer was washed with brine (50 mL), dried over anhydrous sodium sulfate, filtered and the filtrate was concentrated to dryness under reduced pressure. The residue was purified by SGC (MeOH:DCM=20:1) to give compound 5 (1.3 g, Yield: 55.3 %) as a white solid.

MS m/z (ESI): 643.1 [M+H]⁺.

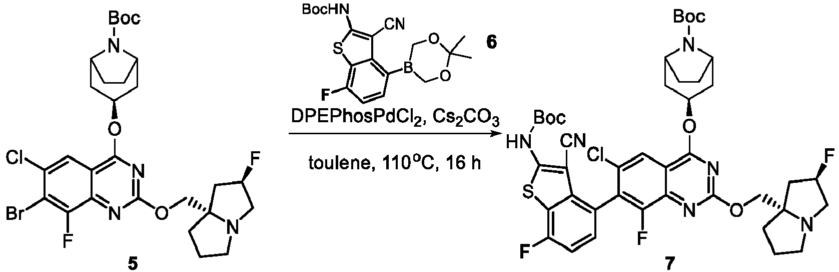

To a solution of compound 5 (1 g, 1.557 mmol), compound 6 (816 mg, 2.024 mmol) and Cs₂CO₃ (1.52 g, 4.672 mmol) in Toluene (20 mL) was added DPEPhosPdCl₂ (223 mg, 0.311 mmol). The mixture was stirred 110 °C for 16 h under N₂. The mixture was poured into water (100 mL), and the solution was extracted with ethyl acetate (100 mL x 3). The organic layer was washed with brine (50 mL), dried over anhydrous sodium sulfate, filtered and the filtrate was concentrated to dryness under reduced pressure. The residue was purified by SGC (MeOH:DCM=10:1) to get a yellow solid (480 mg, Yield: 36.1 %).

MS m/z (ESI): 855.4 [M+H]⁺.

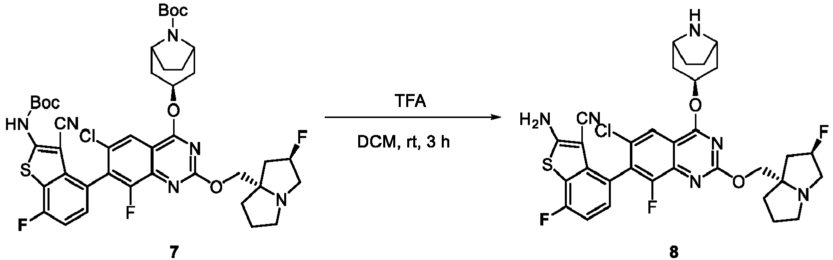

To a solution of compound 7 (480 mg, 0.56 mmol) in DCM (2 ml) was added TFA (1 ml). The reaction mixture was stirred at 20 °C for 1 h. The solvent was removed under reduced pressure. The reaction mixture was diluted with a.q. NaHCO₃ (20 mL) and DCM (50 mL). The organic layer was washed with brine, dried over sodium sulfate, filtered, and concentrated to afford compound 8 (380 mg, crude) as a yellow solid.

MS m/z (ESI): 655.3 [M+H]⁺.

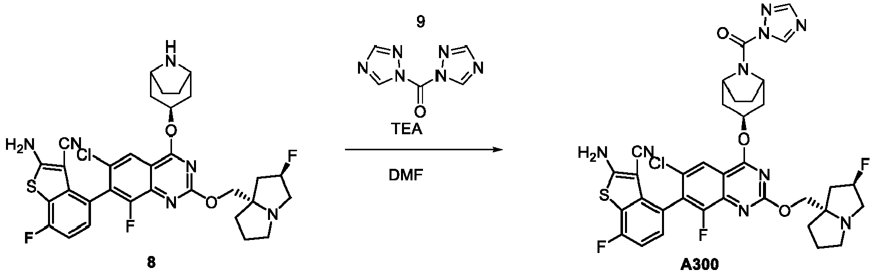

To a solution of compound 8 (140 mg, 0.214 mmol) and DIEA (110 mg, 0.856 mmol) in DMF (2 mL) was added compound 9 (87 mg, 0.535 mmol) at 0 °C under N₂. The mixture was stirred at room temperature for 1h. The mixture was concentrated and purified by prep-HPLC (FA) to give A300 (6.44 mg, Yield: 4.02 %) as a white solid.

¹H NMR (400 MHz, DMSO-*d₆*): δ 9.14 (s, 1H), 8.37 (s, 1H), 8.29 (s, 1H), 8.14 (s, 2H), 7.91 (s, 1H), 7.26 (dd, *J =* 8.4, 5.6 Hz, 1H), 7.17 (t, *J =* 8.8 Hz, 1H), 5.72 (s, 1H), 5.27 (d, *J =* 54.0 Hz, 1H), 4.19 - 4.13 (m, 1H), 4.11 - 4.05 (m, 1H), 3.17 - 2.98 (m, 5H), 2.85 - 2.75 (m, 1H), 2.47 - 2.40 (m, 2H), 2.32 - 2.13 (m, 7H), 2.10 - 1.95 (m, 2H), 1.85 - 1.70 (m, 3H).

**Table 3**

| **No.** | **Structure** | **Chemical Name** | **[M+H]⁺** |
|---|---|---|---|
| A101 | 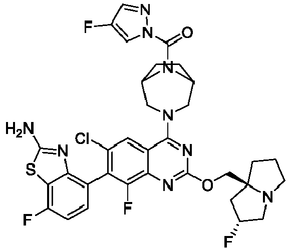 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(4-fluoro-1H-pyrazol-1-yl)methanone | 728.11 |
| A102* | 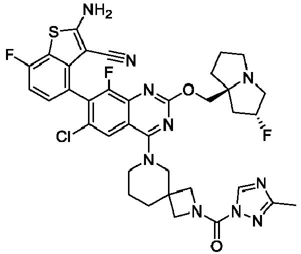 | 2-amino-4-(6-chloro-8-fluoro-2-(((2R,7aS)-2-fluorotetrahydro-1H-pyrrolizin-7 a(5H)-yl)methoxy)-4-(2-(3-methyl-1H-1,2,4-triazole-1-carbonyl)-2,6-diazaspiro[3.5]nonan-6-yl)quinazolin-7-yl)-7-fluorobenzo[b]thiophene-3-carbonitrile | 763.31 |
| A103 | 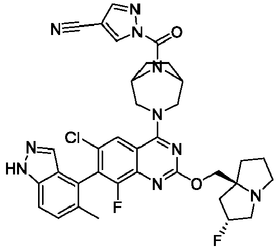 | 1-(3-(6-chloro-8-fluoro-2-(((2R,7aS)-2-fluorotetrahydro-1H-pyrrolizin-7a(5H)-yl)methoxy)-7-(5-methyl-1H-indazol-4-yl)quinazolin-4-yl)-3,8-diazabicyclo[3.2. 1]octane-8-carbonyl)-1H-pyrazole-4-carbonitrile | 699.19 |
| A104 | 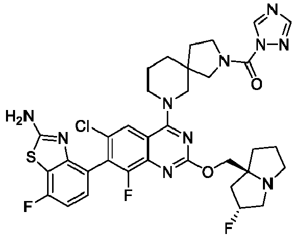 | (7-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,7-diazaspiro[4.5]decan-2-yl)(1H-1,2,4-triazol-1-yl)methanone | 739.21 |
| A105 | 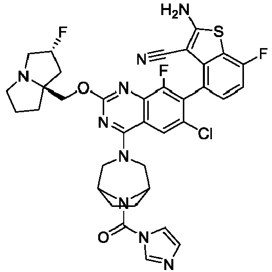 | 4-(4-(8-(1H-imidazole-1-carbonyl)-3,8-diazabicyclo[3.2.1]octan-3-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 734.21 |
| A106 | 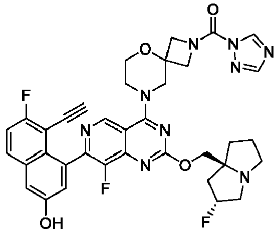 | (8-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-5-oxa-2,8-diazaspiro[3.5]nonan-2-yl)(1H-1,2,4-triazol-1-yl)methanone | 711.53 |
| A107 | 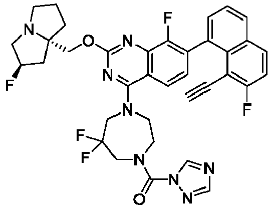 | (4-(7-(8-ethynyl-7-fluoronaphthalen-1 -yl)-8-fluoro-2-(((2R,7aS)-2-fluorotetrahydro-1H-pyrrolizin-7 a(5H)-yl)methoxy)quinazolin-4-yl)-6,6-difluoro-1 ,4-diazepan-1-yl)(1H-1,2,4-triazol-1-yl)methanone | 702 |
| A108 | 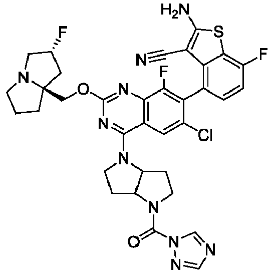 | 4-(4-(4-(1H-1,2,4-triazole-1-carbonyl)hexahydropyrrolo[3,2-b]pyrrol-1(2H)-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 735.15 |
| A109 | 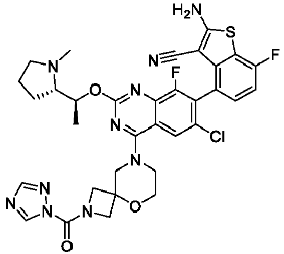 | 4-(4-(2-(1H-1,2,4-triazole-1-carbonyl)-5-oxa-2,8-diazaspiro[3.5]nonan-8-yl)-6-chloro-8-fluoro-2-((S)-1-((S)-1-methylpyrrolidin-2-yl)ethoxy)quinazolin-7-yl)-2-amino-7-fluorobenzo[b]thiophene-3-carbonitrile | 721.12 |
| A110^{†} | 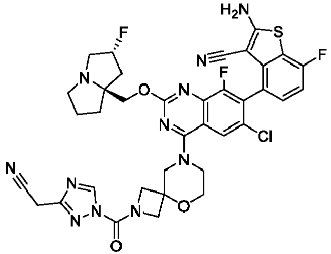 | 2-amino-4-(6-chloro-4-(2-(3-(cyanomethyl)-1H-1,2,4-triazole-1-carbonyl)-5-oxa-2,8-diazaspiro[3.5]nonan-8-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile | 790.21 |
| A111 | 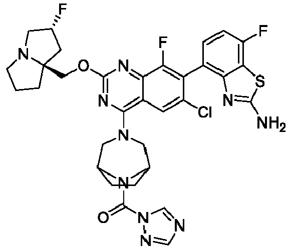 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(1H-1,2,4-triazol-1-yl)methanone | 711.2 |
| A112 | 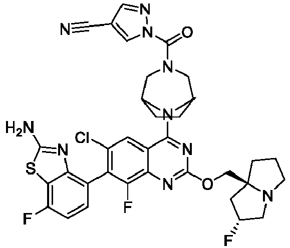 | 1-(8-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-3-carbonyl)-1H-pyrazole-4-carbonitrile | 735.11 |
| A113 | 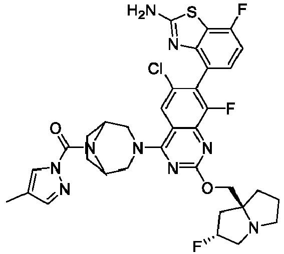 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(4-methyl-1H-pyrazol-1-yl)methanone | 724.23 |
| A114 | 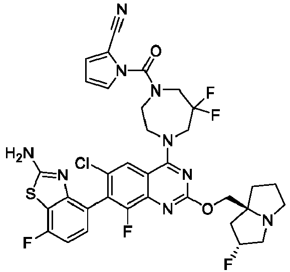 | 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-6,6-difluoro-1,4-diazepane-1-carbonyl)-1H-pyrrole-2-carbonitrile | 758.18 |
| A115 | 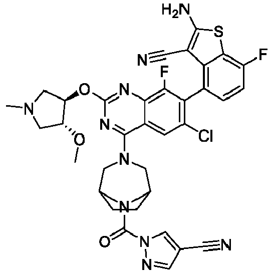 | 1-(3-(7-(2-amino-3-cyano-7-fluorobenzo [b]thiophen-4-yl)-6-chloro-8-fluoro-2-(((3R,4R)-4-methoxy-1-methylpyrrolidin-3-yl)oxy)quinazolin-4-yl)-3,8-diazabicyclo[3.2.1]octane-8-carbonyl)-1H-pyrazole-4-carbonitrile | 731.11 |
| A116 | 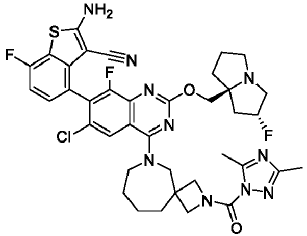 | 2-amino-4-(6-chloro-4-(2-(3,5-dimethyl-1H-1,2,4-triazole-1-carbonyl)-2,6-diazaspiro[3.6]decan-6-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile | 791.25 |
| A117 | 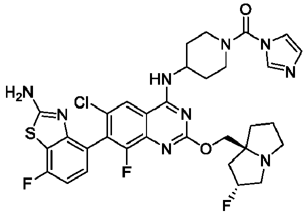 | (4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidin-1-yl)(1H-imidazol-1-yl)methanone | 698.19 |
| A118 | 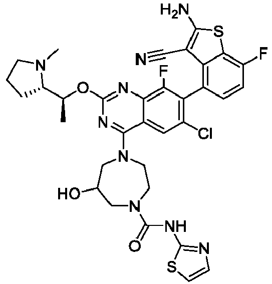 | 4-(7-(2-amino-3-cyano-7-fluorobenzo[b]thiophen-4-yl)-6-chloro-8-fluoro-2-((S)-1-((S)-1-methylpyrrolidin-2-yl)ethoxy)quinazolin-4-yl)-6-hydroxy-N-(thiazol-2-yl)-1,4-diazepane-1-carboxamide | 740.15 |
| A119 | 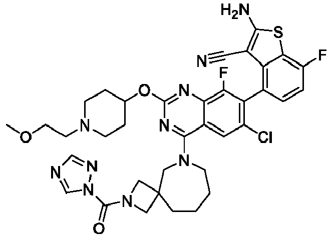 | 4-(4-(2-(1H-1,2,4-triazole-1-carbonyl)-2,6-diazaspiro[3.6]decan-6-yl)-6-chloro-8-fluoro-2-((1-(2-methoxyethyl)piperidin-4-yl)oxy)quinazolin-7-yl)-2-amino-7-fluorobenzo[b]thiophene-3-carbonitrile | 763.21 |
| A120 | 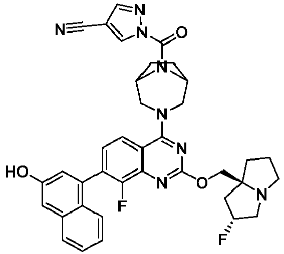 | 1-(3-(8-fluoro-2-(((2R,7aS)-2-fluorotetrahydro-1H-pyrrolizin-7a(5H)-yl)methoxy)-7-(3-hydroxynaphthalen-1-yl)quinazolin-4-yl)-3,8-diazabicyclo[3.2. 1]octane-8-carbonyl)-1H-pyrazole-4-carbonitrile | 676.79 |
| A121 | 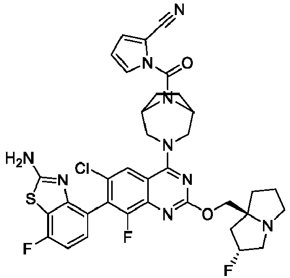 | 1-(3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-pyrrole-2-carbonitrile | 734.2 |
| A122 | 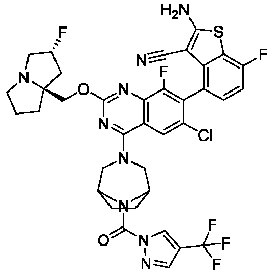 | 2-amino-4-(6-chloro-8-fluoro-2-(((2R,7aS)-2-fluorotetrahydro-1H-pyrrolizin-7 a(5H)-yl)methoxy)-4-(8-(4-(trifluoromethyl)-1H-pyrazole-1-carbonyl)-3,8-diazabicyclo[3.2.1]octan-3-yl)quinazolin-7-yl)-7-fluorobenzo[b]thiophene-3-carbonitrile | 802.21 |
| A123 | 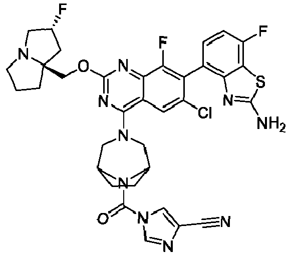 | 1-(3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-imidazole-4-carbonitrile | 735.19 |
| A124 | 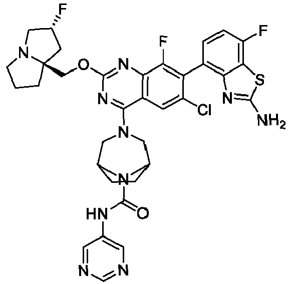 | 3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-N-(pyrimidin-5-yl)-3,8-diazabicyclo[3.2. 1]octane-8-carboxamide | 737.21 |
| A125 | 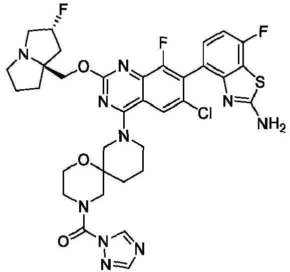 | (8-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1-oxa-4,8-diazaspiro [5.5]undecan-4-yl)(1H-1,2,4-triazol-1-yl)methanone | 755.24 |
| A126 | 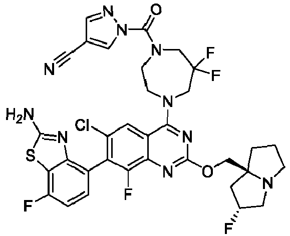 | 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-6,6-difluoro-1,4-diazepane-1-carbonyl)-1Hpyrazole-4-carbonitrile | 759.11 |
| A127 | 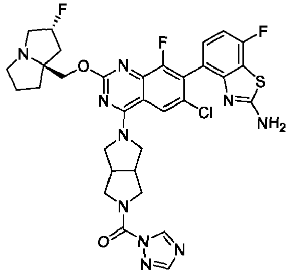 | (5-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)hexahydropyrrolo[3,4-c]pyrrol-2(1H)-yl)(1H-1,2,4-triazol-1-yl)methanone | 711.17 |
| A128 | 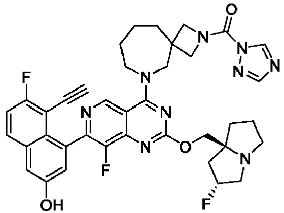 | (6-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-2,6-diazaspiro[3.6]decan-2-yl)(1H-1,2,4-triazol-1-yl)methanone | 723.73 |
| A129 | 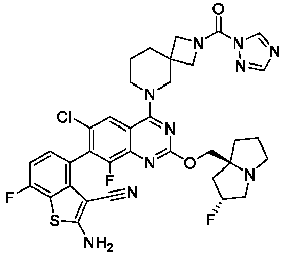 | 4-(4-(2-(1H-1,2,4-triazole-1-carbonyl)-2,6-diazaspiro[3.5]nonan-6-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 749.21 |
| A130 | 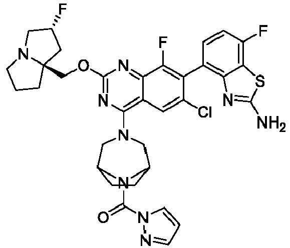 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(1H-pyrazol-1-yl)methanone | 710.19 |
| A131 | 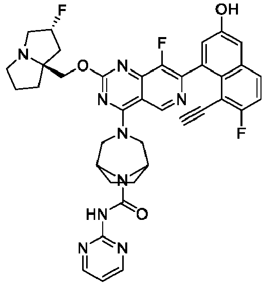 | 3-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-N-(pyrimidin-2-yl)-3,8-diazabicyclo[3.2. 1]octane-8-carboxamide | 721.66 |
| A132 | 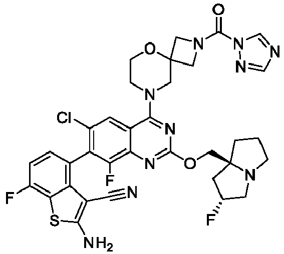 | 4-(4-(2-(1H-1,2,4-triazole-1-carbonyl)-5-oxa-2,8-diazaspiro[3.5]nonan-8-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 751.11 |
| A133 | 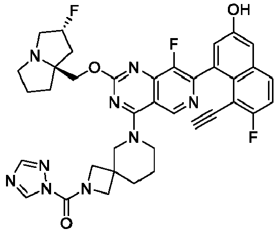 | (6-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-2,6-diazaspiro[3.5]nonan-2-yl)(1H-1,2,4-triazol-1-yl)methanone | 709.73 |
| A134 | 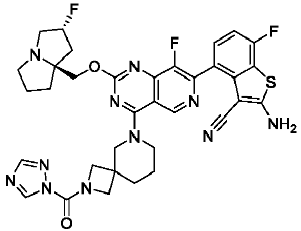 | 4-(4-(2-(1H-1,2,4-triazole-1-carbonyl)-2,6-diazaspiro[3.5]nonan-6-yl)-8-fluoro-2-(((2R,7aS)-2-fluorotetrahydro-1H-pyrrolizin-7a(5H)-yl)methoxy)pyrido[4,3-d]pyrimidin-7-yl)-2-amino-7-fluorobenzo[b]thiophene-3-carbonitrile | 715.71 |
| A135 | 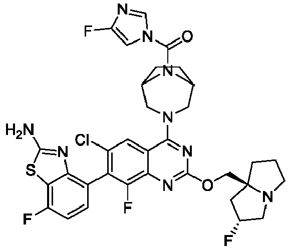 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(4-fluoro-1H-imidazol-1-yl)methanone | 728.11 |
| A136 | 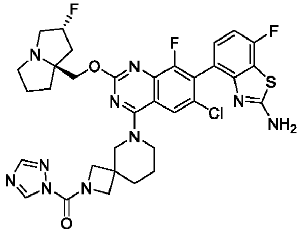 | (6-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,6-diazaspiro[3.5]nonan-2-yl)(1H-1,2,4-triazol-1-yl)methanone | 725.11 |
| A137 | 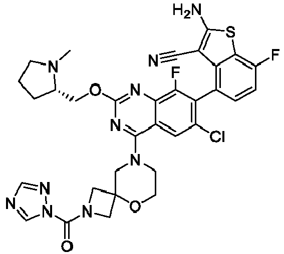 | 4-(4-(2-(1H-1,2,4-triazole-1-carbonyl)-5-oxa-2,8-diazaspiro[3.5]nonan-8-yl)-6-chloro-8-fluoro-2-(((S)-1-methylpyrrolidin-2-yl)methoxy)quinazolin-7-yl)-2-amino-7-fluorobenzo[b]thiophene-3-carbonitrile | 707.11 |
| A138 | 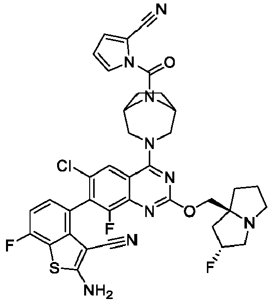 | 1-(3-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-pyrrole-2-carbonitrile | 758.19 |
| A139 | 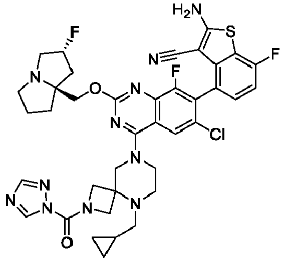 | 2-amino-4-(6-chloro-4-(5-(cyclopropylmethyl)-2-(1H-1,2,4-triazole-1-carbonyl)-2,5,8-triazaspiro[3.5]nonan-8-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile | 804.25 |
| A140 | 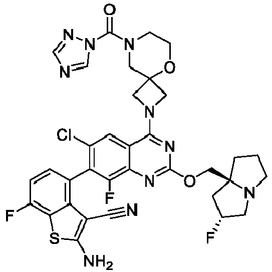 | 4-(4-(8-(1H-1,2,4-triazole-1-carbonyl)-5-oxa-2,8-diazaspiro[3.5]nonan-2-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 751.11 |
| A141 | 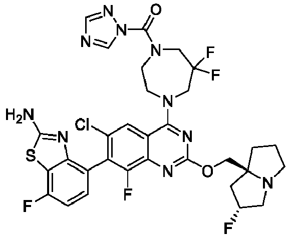 | (4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-6,6-difluoro-1,4-diazepan-1-yl)(1H-1,2,4-triazol-1-yl)methanone | 735.14 |
| A142 | 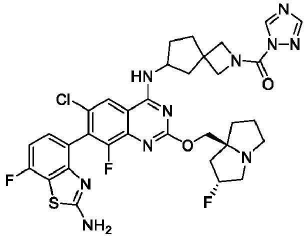 | (6-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)-2-azaspiro[3.4]octan-2-yl)(1H-1,2,4-triazol-1-yl)methanone | 725.13 |
| A143 | 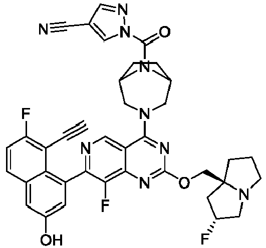 | 1-(3-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2. 1]octane-8-carbonyl)-1H-pyrazole-4-carbonitrile | 719.73 |
| A144 | 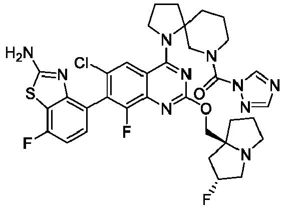 | (1-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1,7-diazaspiro[4.5]decan-7-yl)(1H-1,2,4-triazol-1-yl)methanone | 739.29 |
| A145 | 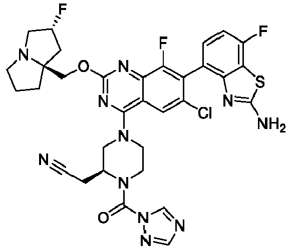 | 2-((2S)-4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1-(1H-1,2,4-triazole-1-carbonyl)piperazin-2-yl)acetonitrile | 724.16 |
| A146 | 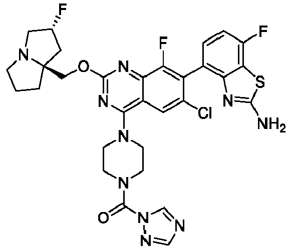 | (4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)piperazin-1-yl)(1H-1,2,4-triazol-1-yl)methanone | 685.13 |
| A147 | 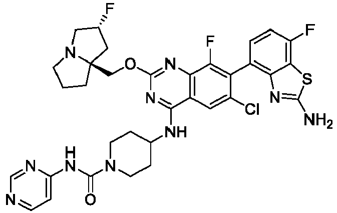 | 4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)-N-(pyrimidin-4-yl)piperidine-1-carboxamide | 725.113 |
| A148* | 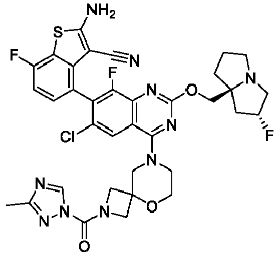 | 2-amino-4-(6-chloro-8-fluoro-2-(((2R,7aS)-2-fluorotetrahydro-1H-pyrrolizin-7 a(5H)-yl)methoxy)-4-(2-(3-methyl-1H-1,2,4-triazole-1-carbonyl)-5-oxa-2,8-diazaspiro[3.5]nonan-8-yl)quinazolin-7-yl)-7-fluorobenzo[b]thiophene-3-carbonitrile | 765.21 |
| A149 | 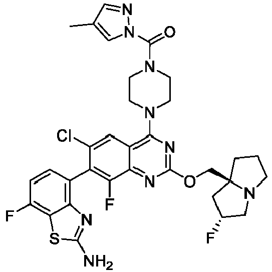 | (4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)piperazin-1-yl)(4-methyl-1H-pyrazol-1-yl)methanone | 698.2 |
| A150 | 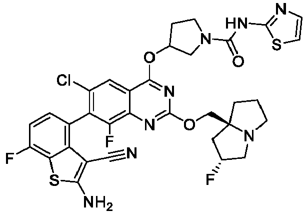 | 3-((7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)oxy)-N-(thiazol-2-yl)pyrrolidine-1-carboxamide | 741.23 |
| A151 | 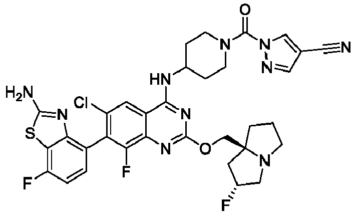 | 1-(4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidine-1-carbonyl)-1H-pyrazole-4-carbonitrile | 723.13 |
| A152 | 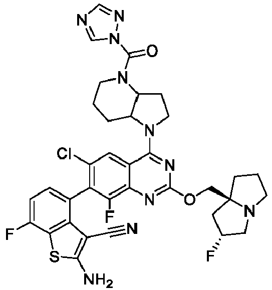 | 4-(4-(4-(1H-1,2,4-triazole-1-carbonyl)octahydro-1H-pyrrolo[3,2-b]pyridin-1 -yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 749.21 |
| A153* | 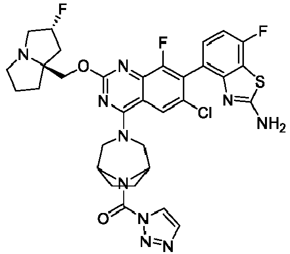 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(1H-1,2,3-triazol-1-yl)methanone | 711.19 |
| A154 | 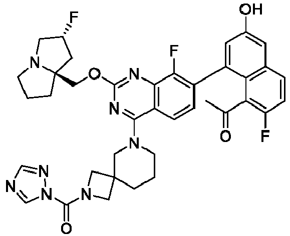 | 1-(8-(4-(2-(1H-1,2,4-triazole-1-carbonyl)-2,6-diazaspiro[3.5]nonan-6-yl)-8-fluoro-2-(((2R,7aS)-2-fluorotetrahydro-1H-pyrrolizin-7a(5H)-yl)methoxy)quinazolin-7-yl)-2-fluoro-6-hydroxynaphthalen-1-yl)ethan-1-one | 726.71 |
| A155 | 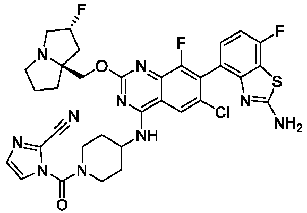 | 1-(4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidine-1-carbonyl)-1H-imidazole-2-carbonitrile | 723.19 |
| A156 | 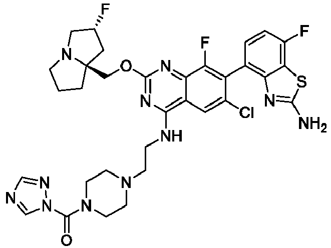 | (4-(2-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)ethyl)piperazin-1-yl)(1H-1,2,4-triazol-1-yl)methanone | 728.29 |
| A157 | 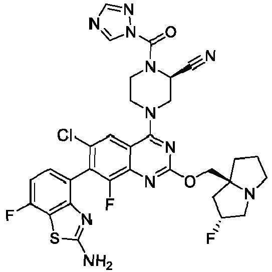 | (2R)-4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1-(1H-1,2,4-triazole-1-carbonyl)piperazine-2-carbonitrile | 710.11 |
| A158 | 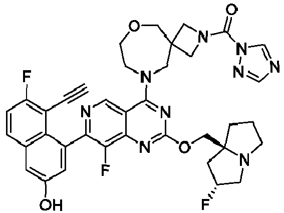 | (9-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-6-oxa-2,9-diazaspiro[3.6]decan-2-yl)(1H-1,2,4-triazol-1-yl)methanone | 725.8 |
| A159^{†} | 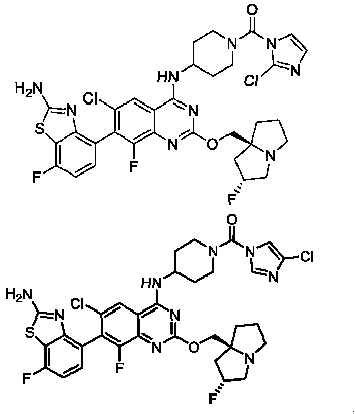 | (4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidin-1-yl)(2-chloro-1H-imidazol-1-yl)methanone (4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidin-1 -yl)(4-chloro-1H-imidazol-1-yl)methanone | 732.69 |
| A160* | 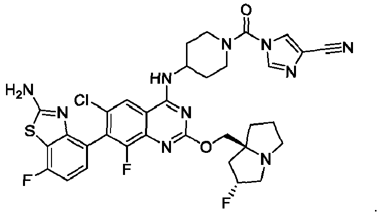 | 1-(4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)amino)piperidine-1-carbonyl)-1H-imidazole-4-carbonitrile | 723.13 |
| A161 | 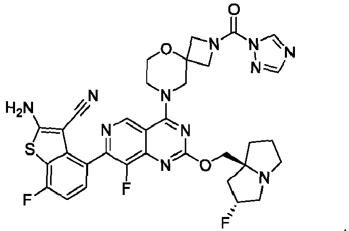 | 4-(4-(2-(1H-1,2,4-triazole-1-carbonyl)-5-oxa-2,8-diazaspiro[3.5]nonan-8-yl)-8-fluoro-2-(((2R,7aS)-2-fluorotetrahydro-1H-pyrrolizin-7a(5H)-yl)methoxy)pyrido[4,3-d]pyrimidin-7-yl)-2-amino-7-fluorobenzo[b]thiophene-3-carbonitrile | 717.71 |
| A162 | 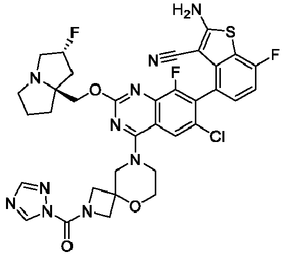 | 4-(4-(2-(1H-1,2,4-triazole-1-carbonyl)-5-oxa-2,8-diazaspiro[3.5]nonan-8-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 751.11 |
| A163 | 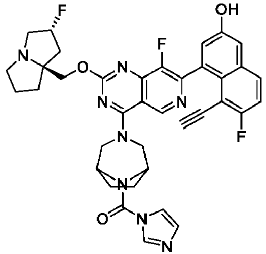 | (3-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]octan-8-yl)(1H-imidazol-1-yl)methanone | 694.73 |
| A164 | 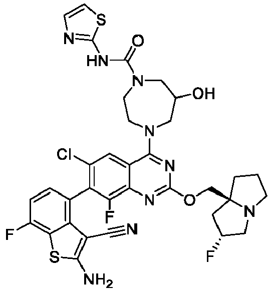 | 4-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-6-hydroxy-N-(thiazol-2-yl)-1,4-diazepane-1-carboxamide | 770.16 |
| A165 | 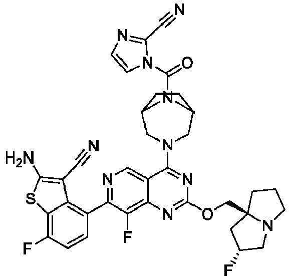 | 1-(3-(7-(2-amino-3-cyano-7-fluorobenzo[b]thiophen-4-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2. 1]octane-8-carbonyl)-1H-imidazole-2-carbonitrile | 725.79 |
| A166 | 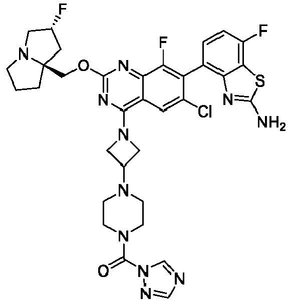 | (4-(1-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)azetidin-3-yl)piperazin-1-yl)(1H-1,2,4-triazol-1-yl)methanone | 740.23 |
| A167 | 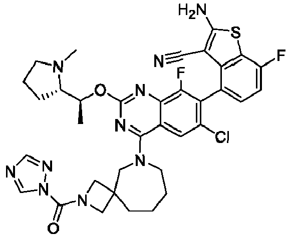 | 4-(4-(2-(1H-1,2,4-triazole-1-carbonyl)-2,6-diazaspiro[3.6]decan-6-yl)-6-chloro-8-fluoro-2-((S)-1-((S)-1-methylpyrrolidin-2-yl)ethoxy)quinazolin-7-yl)-2-amino-7-fluorobenzo[b]thiophene-3-carbonitrile | 733.22 |
| A168 | 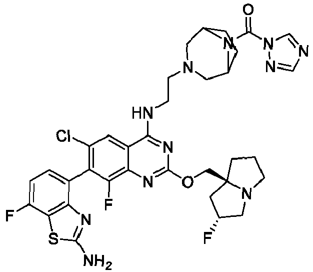 | (3-(2-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)ethyl)-3,8-diazabicyclo[3.2.1]octan-8-yl)(1H-1,2,4-triazol-1-yl)methanone | 754.29 |
| A169 | 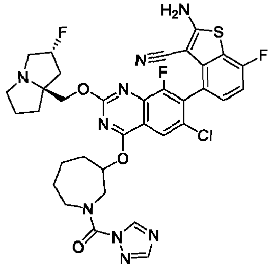 | 4-(4-((1-(1H-1,2,4-triazole-1-carbonyl)azepan-3-yl)oxy)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 738.23 |
| A170 | 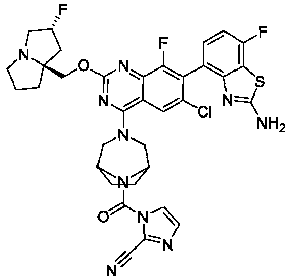 | 1-(3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-imidazole-2-carbonitrile | 735.19 |
| A171 | 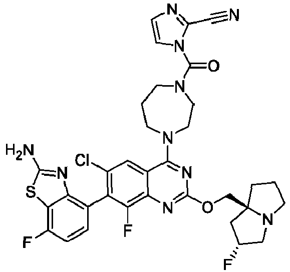 | 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1,4-diazepane-1-carbonyl)-1H-imidazole-2-carbonitrile | 723.19 |
| A172 | 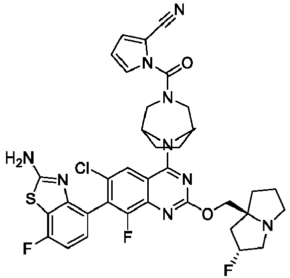 | 1-(8-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-3-carbonyl)-1H-pyrrole-2-carbonitrile | 734.3 |
| A173* | 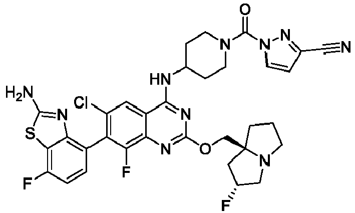 | 1-(4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidine-1-carbonyl)-1H-pyrazole-3-carbonitrile | 723.13 |
| A174* | 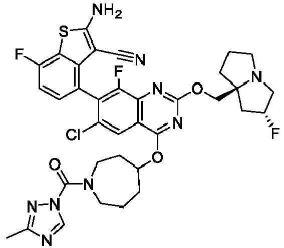 | 2-amino-4-(6-chloro-8-fluoro-2-(((2R,7aS)-2-fluorotetrahydro-1H-pyrrolizin-7 a(5H)-yl)methoxy)-4-((1-(3-methyl-1H-1 ,2,4-triazole-1-carbonyl)azepan-4-yl)oxy)quinazolin-7-yl)-7-fluorobenzo[b]thiophene-3-carbonitrile | 752.25 |
| A175 | 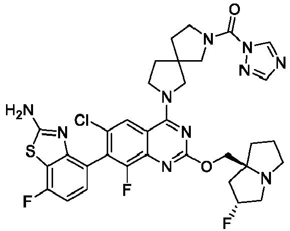 | (7-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,7-diazaspiro[4.4]nonan-2-yl)(1H-1,2,4-triazol-1-yl)methanone | 725.11 |
| A176 | 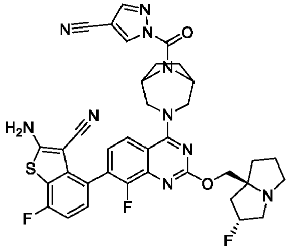 | 1-(3-(7-(2-amino-3-cyano-7-fluorobenzo[b]thiophen-4-yl)-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-pyrazole-4-carbonitrile | 724.71 |
| A177 | 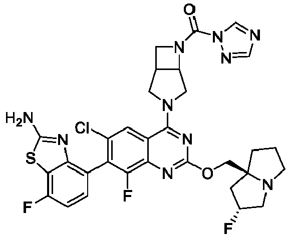 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,6-diazabicyclo[3.2.0]heptan-6-yl)(1H-1,2,4-triazol-1-yl)methanone | 697.19 |
| A178 | 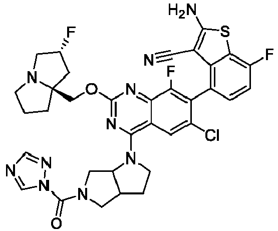 | 4-(4-(5-(1H-1,2,4-triazole-1-carbonyl)hexahydropyrrolo[3,4-b]pyrrol-1(2H)-yl)-6-chloro-8-fluoro-2-(((2R,7aS)-2-fluorotetrahydro-1H-pyrrolizin-7a(5H)-yl)methoxy)quinazolm-7-yl)-2-amino-7-fluorobenzo[b]thiophene-3-carbonitrile | 735.14 |
| A179^{t} | 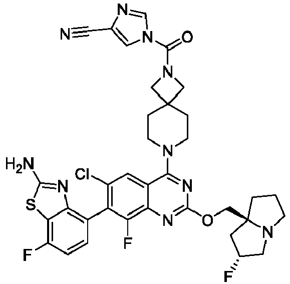 | 1-(7-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)-2,7-diazaspiro[3.5]nonane-2-carbonyl)-1H-imidazole-4-carbonitrile | 749.21 |
| A180 | 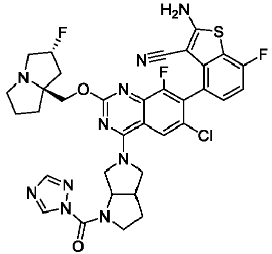 | 4-(4-(1-(1H-1,2,4-triazole-1-carbonyl)hexahydropyrrolo[3,4-b]pyrrol-5(1H)-yl)-6-chloro-8-fluoro-2-(((2R,7aS)-2-fluorotetrahydro-1H-pyrrolizin-7a(5H)-yl)methoxy)quinazolm-7-yl)-2-amino-7-fluorobenzo[b]thiophene-3-carbonitrile | 735.14 |
| A181 | 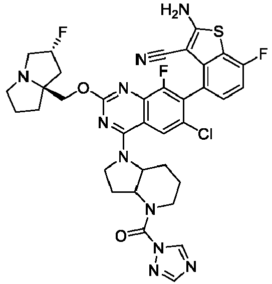 | 4-(4-(4-(1H-1,2,4-triazole-1-carbonyl)octahydro-1H-pyrrolo[3,2-b]pyridin-1 -yl)-6-chloro-8-fluoro-2-(((2R,7aS)-2-fluorotetrahydro-1H-pyrrolizin-7a(5H)-yl)methoxy)quinazolm-7-yl)-2-amino-7-fluorobenzo[b]thiophene-3-carbonitrile | 749.2 |
| A182 | 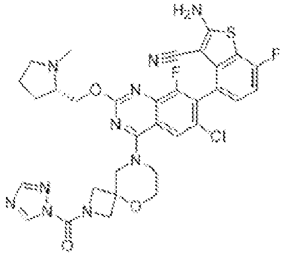 | 4-(4-(2-(1H-1,2,4-triazole-1-carbonyl)-5-oxa-2,8-diazaspiro[3.5]nonan-8-yl)-6-chloro-8-fluoro-2-(((S)-1-methylpyrrolidin-2-yl)methoxy)quinazolin-7-yl)-2-amino-7-fluorobenzo[b]thiophene-3-carbonitrile | 707.11 |
| A183 | 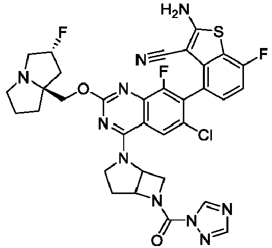 | 4-(4-(6-(1H-1,2,4-triazole-1-carbonyl)-2,6-diazabicyclo[3.2.0]heptan-2-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 721.18 |
| A184 | 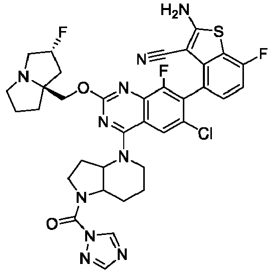 | 4-(4-(1-(1H-1,2,4-triazole-1-carbonyl)octahydro-4H-pyrrolo[3,2-b]pyridin-4-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 749.23 |
| A185 | 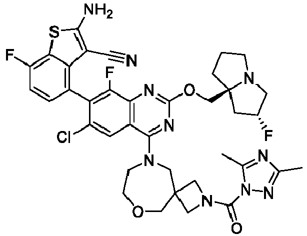 | 2-amino-4-(6-chloro-4-(2-(3,5-dimethyl-1H-1,2,4-triazole-1-carbonyl)-6-oxa-2,9-diazaspiro[3.6]decan-9-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile | 793.22 |
| A186* | 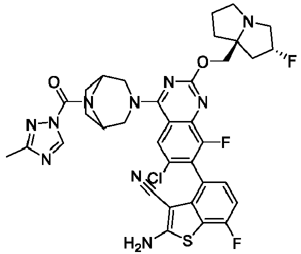 | 2-amino-4-(6-chloro-8-fluoro-2-(((2R,7aS)-2-fluorotetrahydro-1H-pyrrolizin-7a(5H)-yl)methoxy)-4-(8-(3-methyl-1H-1,2,4-triazole-1 -carbonyl)-3,8-diazabicyclo[3.2.1]octan-3-yl)quinazolin-7-yl)-7-fluorobenzo[b]thiophene-3-carbonitrile | 749.21 |
| A187 | 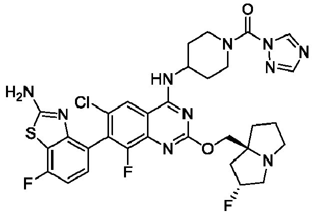 | (4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidin-1 -yl)(1H-1,2,4-triazol-1-yl)methanone | 699.19 |
| A188 | 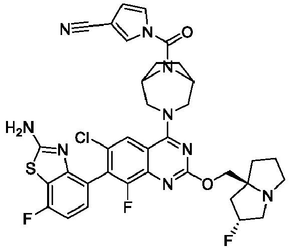 | 1-(3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-pyrrole-3-carbonitrile | 734.17 |
| A189* | 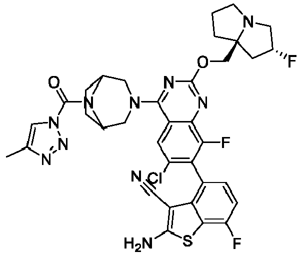 | 2-amino-4-(6-chloro-8-fluoro-2-(((2R,7aS)-2-fluorotetrahydro-1H-pyrrolizin-7 a(5H)-yl)methoxy)-4-(8-(4-methyl-1H-1,2,3-triazole-1-carbonyl)-3,8-diazabicyclo[3.2.1]octan-3-yl)quinazolin-7-yl)-7-fluorobenzo[b]thiophene-3-carbonitrile | 738.13 |
| A190 | 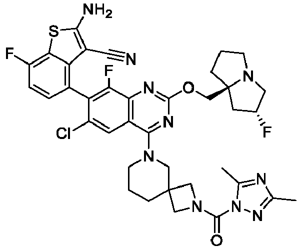 | 2-amino-4-(6-chloro-4-(2-(3,5-dimethyl-1H-1,2,4-triazole-1-carbonyl)-2,6-diazaspiro[3.5]nonan-6-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile | 777.17 |
| A191 | 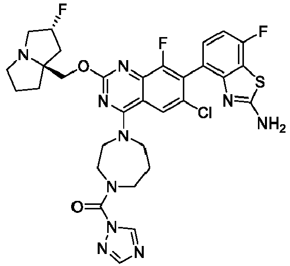 | (4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1,4-diazepan-1-yl)(1H-1,2,4-triazol-1-yl)methanone | 699.15 |
| A192 | 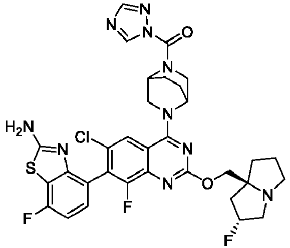 | (5-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,5-diazabicyclo[2.2.2]octan-2-yl)(1H-1,2,4-triazol-1-yl)methanone | 711.19 |
| A193 | 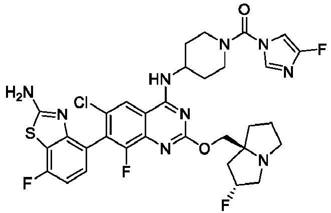 | (4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidin-1-yl)(4-fluoro-1H-imidazol-1- yl)methanone | 716.13 |
| A194 | 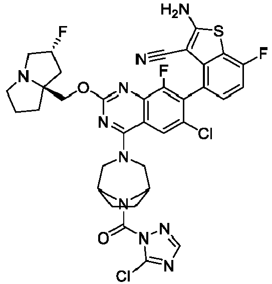 | 2-amino-4-(6-chloro-4-(8-(5-chloro-1H-1,2,4-triazole-1-carbonyl)-3,8-diazabicyclo[3.2.1]octan-3-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile 2-amino-4-(6-chloro-4-(8-(3-chloro-1H-1,2,4-triazole-1- | 769.61 |
| | 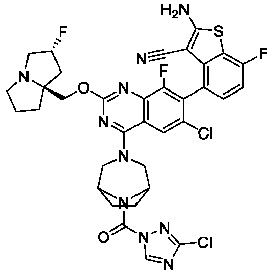 | carbonyl)-3,8-diazabicyclo[3.2. 1]octan-3-yl)-8-fluoro-2-(((2R,7aS)-2-fluorotetrahydro-1H-pyrrolizin-7a(5H)-yl)methoxy)quinazolm-7-yl)-7-fluorobenzo[b]thiophene-3-carbonitrile | |
| A195* | 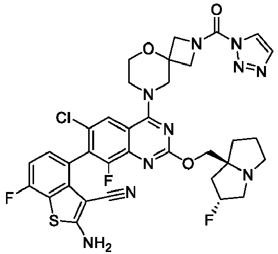 | 4-(4-(2-(1H-1,2,3-triazole-1-carbonyl)-5-oxa-2,8-diazaspiro[3.5]nonan-8-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 751.23 |
| A196* | 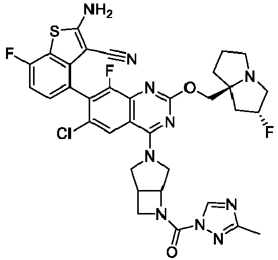 | 2-amino-4-(6-chloro-8-fluoro-2-(((2R,7aS)-2-fluorotetrahydro-1H-pyrrolizin-7 a(5H)-yl)methoxy)-4-(6-(3 -methyl-1H-1,2,4-triazole-1-carbonyl)-3,6-diazabicyclo[3.2.0]heptan-3-yl)quinazolin-7-yl)-7-fluorobenzo[b]thiophene-3-carbonitrile | 735.15 |
| A197 | 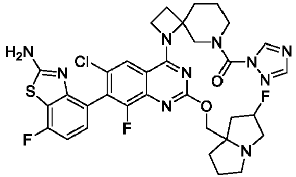 | (1-(7-(2-amino-7-fluorobenzo[d]thiazol-4-yl)-6-chloro-8-fluoro-2-((2-fluorotetrahydro-1H-pyrrolizin-7a(5H)-yl)methoxy)quinazolin-4-yl)-1,6-diazaspiro[3.5]nonan-6-yl)(1H-1,2,4-triazol-1-yl)methanone | 725.19 |
| A198 | 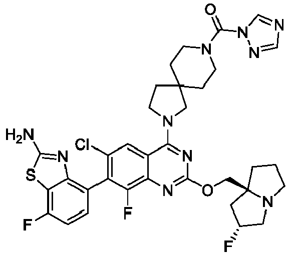 | (2-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,8-diazaspiro[4.5]decan-8-yl)(1H-1,2,4-triazol-1-yl)methanone | 739.21 |
| A199 | 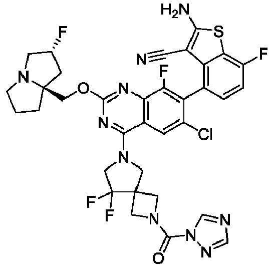 | 2-amino-4-(6-chloro-4-(8,8-difluoro-2-(1H-1,2,4-triazole-1-carbonyl)-2,6-diazaspiro[3.4]octan-6-yl)-8-fluoro-2-(((2R,7aS)-2-fluorotetrahydro-1H-pyrrolizin-7a(5H)-yl)methoxy)quinazolm-7-yl)-7-fluorobenzo[b]thiophene-3-carbonitrile | 771.11 |
| A200 | 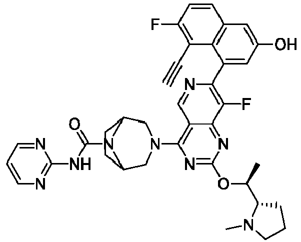 | 3-(7-(8-ethynyl-7-fluoro-3-hydroxynaphthalen-1-yl)-8-fluoro-2-((S)-1-((S)-1-methylpyrrolidin-2-yl)ethoxy)pyrido[4,3-d]pyrimidin-4-yl)-N-(pyrimidin-2-yl)-3,8-diazabicyclo[3.2. 1]octane-8-carboxamide | 691.72 |
| A201 | 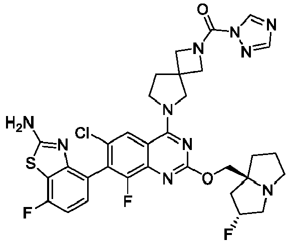 | (6-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)-2,6-diazaspiro[3.4]octan-2-yl)(1H-1,2,4-triazol-1-yl)methanone | 711.11 |
| A202 | 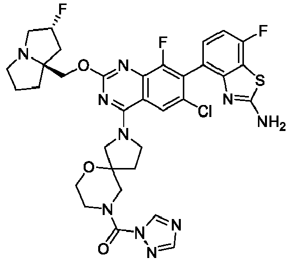 | (2-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)-6-oxa-2,9-diazaspiro[4.5]decan-9-yl)(1H-1,2,4-triazol-1-yl)methanone | 741.11 |
| A203 | 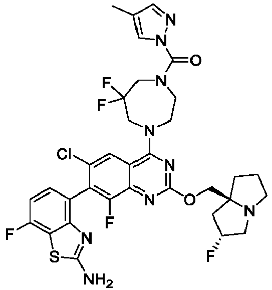 | (4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)-6,6-difluoro-1,4-diazepan-1-yl)(4-methyl-1H-pyrazol-1-yl)methanone | 748.11 |
| A204 | 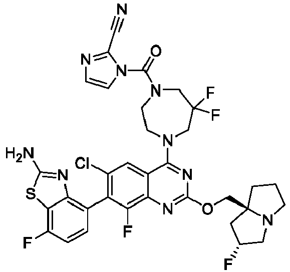 | 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-6,6-difluoro-1,4-diazepane-1 - carbonyl)-1H-imidazole-2-carbonitrile | 759.11 |
| A205 | 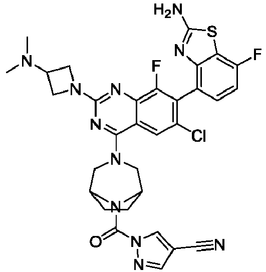 | 1-(3-(7-(2-amino-7-fluorobenzo[d]thiazol-4-yl)-6-chloro-2-(3-(dimethylamino)azetidin-1-yl)-8-fluoroquinazolin-4-yl)-3,8-diazabicyclo[3.2. 1]octane-8-carbonyl)-1H-pyrazole-4-carbonitrile | 676.11 |
| A206 | 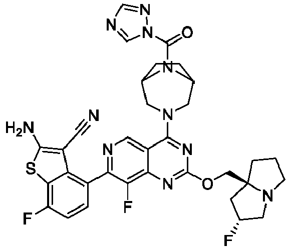 | 4-(4-(8-(1H-1,2,4-triazole-1-carbonyl)-3,8-diazabicyclo[3.2.1]octan-3-yl)-8-fluoro-2-(((2R,7aS)-2-fluorotetrahydro-1H-pyrrolizin-7a(5H)-yl)methoxy)pyrido[4,3-d]pyrimidin-7-yl)-2-amino-7-fluorobenzo[b]thiophene-3-carbonitrile | 701.79 |
| A207 | 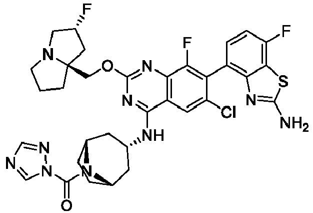 | ((1R,3r,5S)-3-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)-8-azabicyclo[3.2.1]octan-8-yl)(1H-1,2,4-triazol-1-yl)methanone | 725.11 |
| A208* | 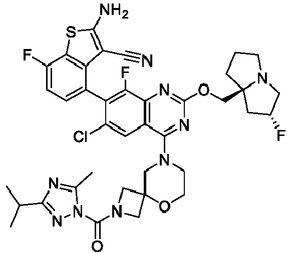 | 2-amino-4-(6-chloro-8-fluoro-2-(((2R,7aS)-2-fluorotetrahydro-1H-pyrrolizin-7 a(5H)-yl)methoxy)-4-(2-(3-isopropyl-5-methyl-1H-1,2,4-triazole-1-carbonyl)-5-oxa-2,8-diazaspiro[3.5]nonan-8-yl)quinazolin-7-yl)-7-fluorobenzo[b]thiophene-3-carbonitrile | 807.24 |
| A209 | 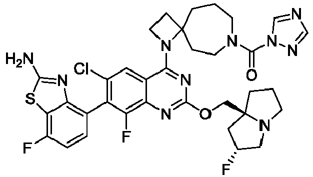 | (1-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1,7-diazaspiro[3.6]decan-7-yl)(1H-1,2,4-triazol-1-yl)methanone | 739.29 |
| A210 | 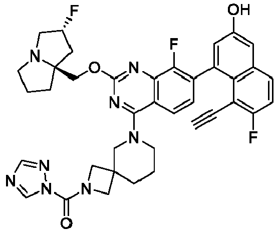 | (6-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)quinazolin-4-yl)-2,6-diazaspiro[3.5]nonan-2-yl)(1H-1,2,4-triazol-1-yl)methanone | 708.73 |
| A211 | 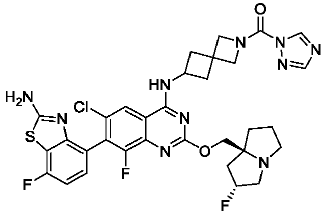 | (6-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)-2-azaspiro[3.3]heptan-2-yl)(1H-1,2,4-triazol-1-yl)methanone | 711.13 |
| A212 | 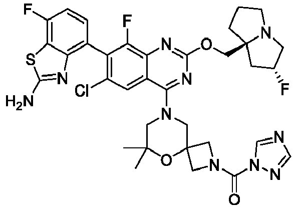 | (8-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-6,6-dimethyl-5-oxa-2,8-diazaspiro[3.5]nonan-2-yl)(1H-1,2,4-triazol-1-yl)methanone | 755.212 |
| A213 | 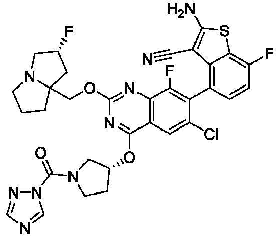 | 4-(4-(((R)-1-(1H-1,2,4-triazole-1-carbonyl)pyrrolidin-3-yl)oxy)-6-chloro-8-fluoro-2-(((2R)-2-fluorotetrahydro-1H-pyrrolizin-7a(5H)-yl)methoxy)quinazolin-7-yl)-2-amino-7-fluorobenzo[b]thiophene-3-carbonitrile | 710.13 |
| A214^{†} | 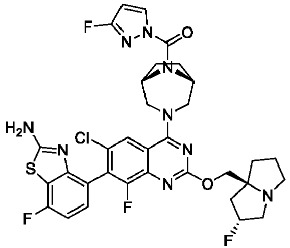 | ((1R,5S)-3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(3-fluoro-1H-pyrazol-1-yl)methanone | 728.11 |
| A215 | 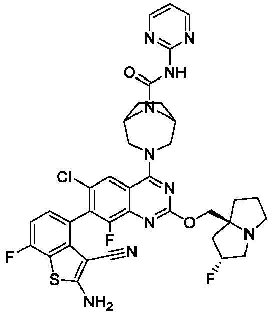 | 3-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-N-(pyrimidin-2-yl)-3,8-diazabicyclo[3.2. 1]octane-8-carboxamide | 761.21 |
| A216^{†} | 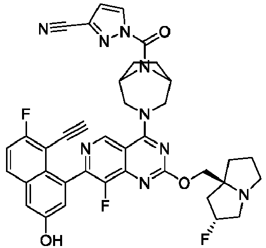 | 1-(3-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2. 1]octane-8-carbonyl)-1H-pyrazole-3-carbonitrile | 719.71 |
| A217 | 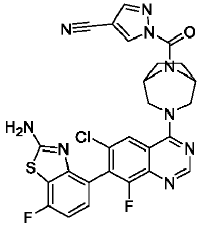 | 1-(3-(7-(2-amino-7-fluorobenzo[d]thiazol-4-yl)-6-chloro-8-fluoroquinazolin-4-yl)-3,8-diazabicyclo[3.2. 1]octane-8-carbonyl)-1H-pyrazole-4-carbonitrile | 578.09 |
| A218 | 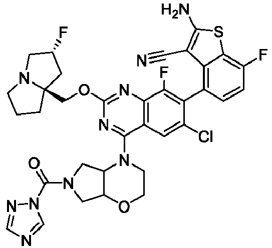 | 4-(4-(6-(1H-1,2,4-triazole-1-carbonyl)hexahydropyrrolo [3,4-b][1,4]oxazin-4(4aH)-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 751.34 |
| A219 | 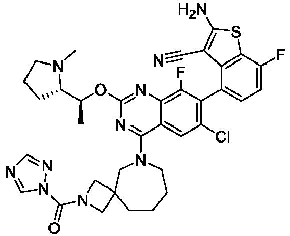 | 4-(4-(2-(1H-1,2,4-triazole-1-carbonyl)-2,6-diazaspiro[3.6]decan-6-yl)-6-chloro-8-fluoro-2-((S)-1-((S)-1-methylpyrrolidin-2-yl)ethoxy)quinazolin-7-yl)-2-amino-7-fluorobenzo[b]thiophene-3-carbonitrile | 733.18 |
| A220 | 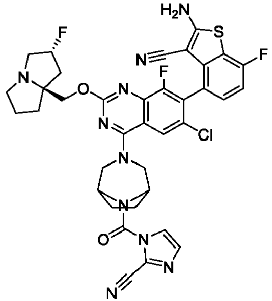 | 1-(3-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-imidazole-2-carbonitrile | 759.21 |
| A221 | 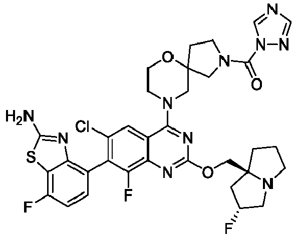 | (9-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-6-oxa-2,9-diazaspiro[4.5]decan-2-yl)(1H-1,2,4-triazol-1-yl)methanone | 741.11 |
| A222* | 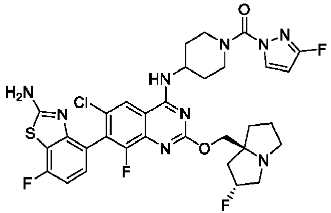 | (4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidin-1-yl)(3-fluoro-1H-pyrazol-1-yl)methanone | 716.13 |
| A223 | 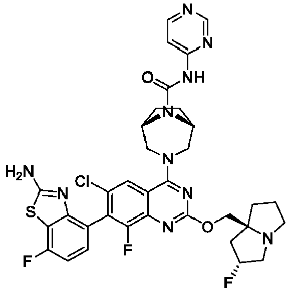 | (1R,5S)-3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-N-(pyrimidin-4-yl)-3,8-diazabicyclo[3.2. 1]octane-8-carboxamide | 737.29 |
| A224^{†} | 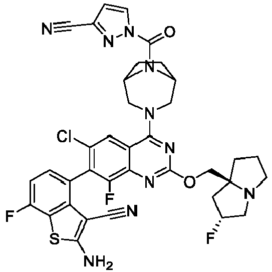 | 1-(3-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-pyrazole-3-carbonitrile | 759.2 |
| A225 | 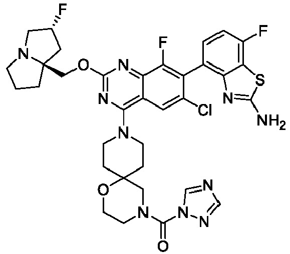 | (9-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1-oxa-4,9-diazaspiro [5.5]undecan-4-yl)(1H-1,2,4-triazol-1-yl)methanone | 755.23 |
| A226 | 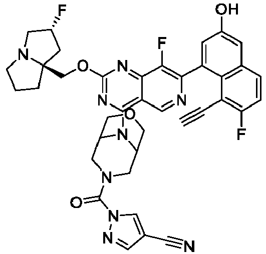 | 1-(9-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3-oxa-7,9-diazabicyclo[3.3.1]nonane-7-carbonyl)-1H-pyrazole-4-carbonitrile | 735.75 |
| A227 | 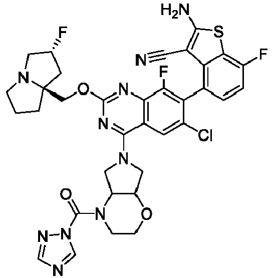 | 4-(4-(4-(1H-1,2,4-triazole-1-carbonyl)hexahydropyrrolo[3,4-b][1,4]oxazin-6(2H)-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 751.4 |
| A228 | 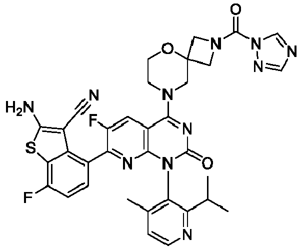 | 4-(4-(2-(1H-1,2,4-triazole-1-carbonyl)-5-oxa-2,8-diazaspiro[3.5]nonan-8-yl)-6-fluoro-1-(2-isopropyl-4-methylpyridin-3-yl)-2-oxo-1,2-dihydropyrido[2,3-d]pyrimidin-7-yl)-2-amino-7-fluorobenzo[b]thiophene-3-carbonitrile | 709.72 |
| A229 | 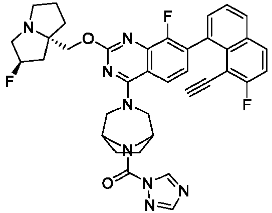 | (3-(7-(8-ethynyl-7-fluoronaphthalen-1-yl)-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(1H-1,2,4-triazol-1-yl)methanone | 678.79 |
| A230 | 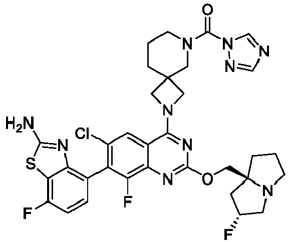 | (2-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,6-diazaspiro[3.5]nonan-6-yl)(1H-1,2,4-triazol-1-yl)methanone | 725.11 |
| A231 | 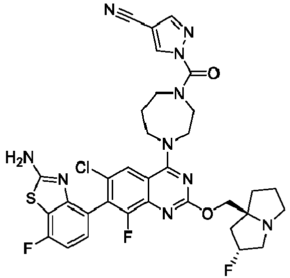 | 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1,4-diazepane-1-carbonyl)-1H-pyrazole-4-carbonitrile | 723.11 |
| A232 | 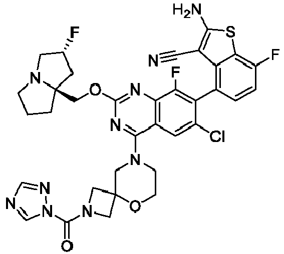 | 4-(4-(2-(1H-1,2,4-triazole-1-carbonyl)-5-oxa-2,8-diazaspiro[3.5]nonan-8-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 751.23 |
| A233 | 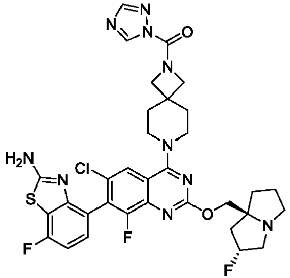 | (7-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,7-diazaspiro[3.5]nonan-2-yl)(1H-1,2,4-triazol-1-yl)methanone | 725.11 |
| A234 | 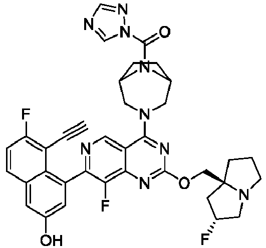 | (3-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]octan-8-yl)(1H-1,2,4-triazol-1-yl)methanone | 695.79 |
| A235 | 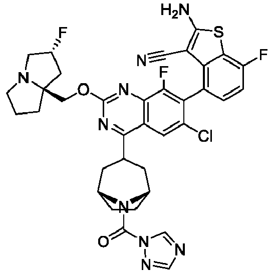 | 4-(4-((1R,5S)-8-(1H-1,2,4-triazole-1-carbonyl)-8-azabicyclo[3.2.1]octan-3-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 734.21 |
| A236 | 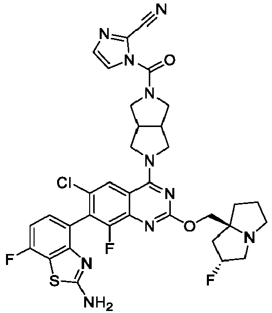 | 1-(5-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)octahydropyrrolo[3,4-c]pyrrole-2-carbonyl)-1H-imidazole-2-carbonitrile | 735.19 |
| A237 | 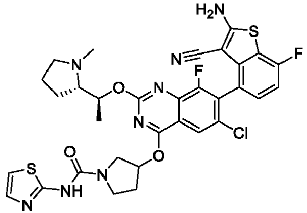 | 3-((7-(2-amino-3-cyano-7-fluorobenzo[b]thiophen-4-yl)-6-chloro-8-fluoro-2-((S)-1-((S)-1-methylpyrrolidin-2-yl)ethoxy)quinazolin-4-yl)oxy)-N-(thiazol-2-yl)pyrrolidine-1-carboxamide | 711.21 |
| A238 | 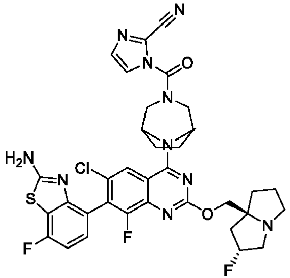 | 1-(8-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)-3,8-diazabicyclo[3.2. 1]octane-3-carbonyl)-1H-imidazole-2-carbonitrile | 735.19 |
| A239 | 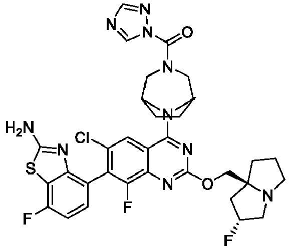 | (8-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)-3,8-diazabicyclo[3.2.1]octan-3-yl)(1H-1,2,4-triazol-1-yl)methanone | 711.19 |
| A240* | 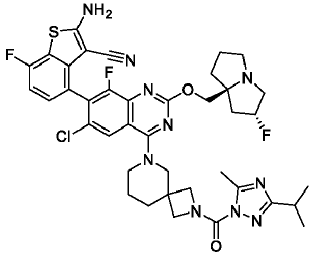 | 2-amino-4-(6-chloro-8-fluoro-2-(((2R,7aS)-2-fluorotetrahydro-1H-pyrrolizin-7 a(5H)-yl)methoxy)-4-(2-(3-isopropyl-5-methyl-1H-1,2,4-triazole-1-carbonyl)-2,6-diazaspiro[3.5]nonan-6-yl)quinazolin-7-yl)-7-fluorobenzo[b]thiophene-3-carbonitrile | 805.27 |
| A241 | 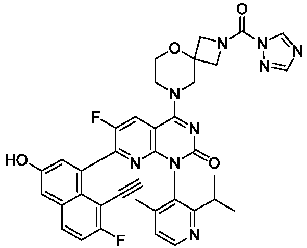 | 4-(2-(1H-1,2,4-triazole-1-carbonyl)-5-oxa-2,8-diazaspiro[3.5]nonan-8-yl)-7-(8-ethynyl-7-fluoro-3-hydroxynaphthalen-1-yl)-6-fluoro-1-(2-isopropyl-4-methylpyridin-3-yl)pyrido[2,3-d]pyrimidin-2(1H)-one | 703.72 |
| A242 | 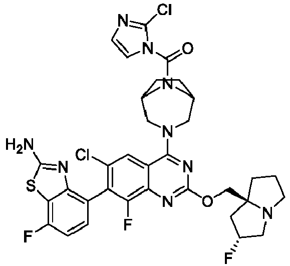 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(2-chloro-1H-imidazol-1-yl)methanone | 744.69 |
| A243 | 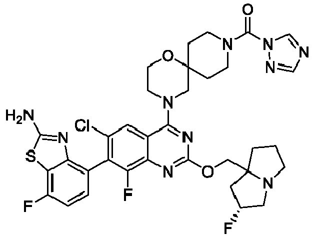 | (4-(7-(2-amino-7-fluorobenzo[d]thiazol-4-yl)-6-chloro-8-fluoro-2-(((2R)-2-fluorotetrahydro-1H-pyrrolizin-7a(5H)-yl)methoxy)quinazolin-4-yl)-1-oxa-4,9-diazaspiro [5.5]undecan-9-yl)(1H-1,2,4-triazol-1-yl)methanone | 755.21 |
| A244 | 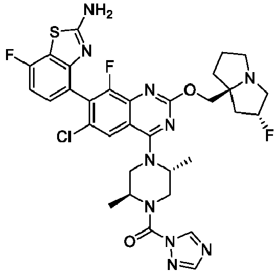 | ((2S,5R)-4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,5-dimethylpiperazin-1-yl)(1H-1,2,4-triazol-1-yl)methanone | 713.12 |
| A245^{†} | 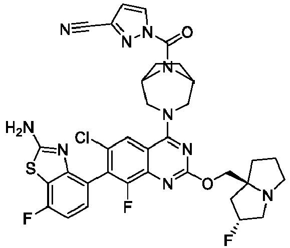 | 1-(3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-pyrazole-3-carbonitrile | 735.11 |
| A246^{†} | 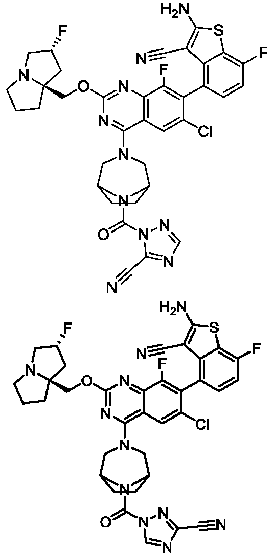 | 1-(3-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carbonyl)-1H-1,2,4-triazole-5-carbonitrile 1-(3-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-1,2,4-triazole-3-carbonitrile | 760.21 |
| A247 | 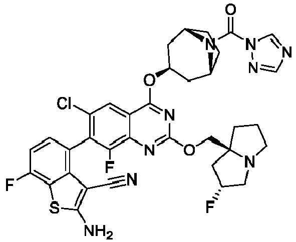 | 4-(4-(((1R,3s,5S)-8-(1H-1,2,4-triazole-1-carbonyl)-8-azabicyclo[3.2.1]octan-3-yl)oxy)-6-chloro-8-fluoro-2-(((2R,7aS)-2-fluorotetrahydro-1H-pyrrolizin-7 a(5H)-yl)methoxy)quinazolin-7-yl)-2-amino-7-fluorobenzo[b]thiophene-3-carbonitrile | 750.23 |
| A248 | 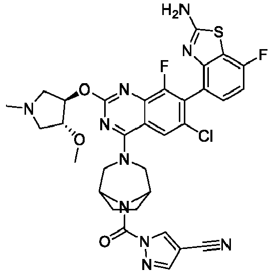 | 1-(3-(7-(2-amino-7-fluorobenzo[d]thiazol-4-yl)-6-chloro-8-fluoro-2-(((3R,4R)-4-methoxy-1-methylpyrrolidin-3-yl)oxy)quinazolin-4-yl)-3,8-diazabicyclo[3.2.1]octane-8-carbonyl)-1H-pyrazole-4-carbonitrile | 707.11 |
| A249 | 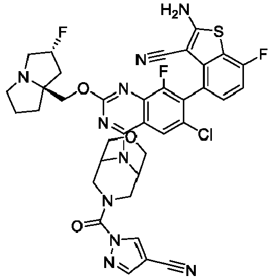 | 1-(9-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-3-oxa-7,9-diazabicyclo[3.3.1]nonane-7-carbonyl)-1H-pyrazole-4-carbonitrile | 775.21 |
| A250 | 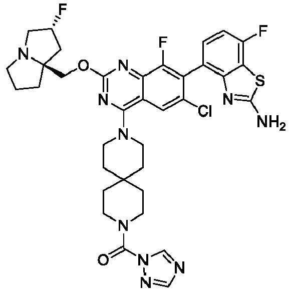 | (9-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,9-diazaspiro[5.5]undecan-3-yl)(1H-1,2,4-triazol-1-yl)methanone | 753.24 |
| A251 | 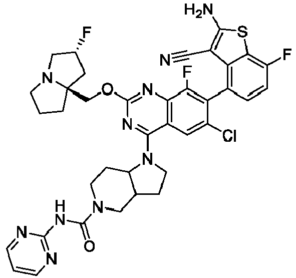 | 1-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-N-(pyrimidin-2-yl)octahydro-5H-pyrrolo[3,2-c]pyridine-5-carboxamide (first single octahydro-5H-pyrrolo[3,2-c]pyridine diastereomer) | 775.23 |
| A252 | 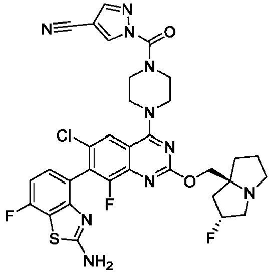 | 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)piperazine-1-carbonyl)-1H-pyrazole-4-carbonitrile | 709.11 |
| A253 | 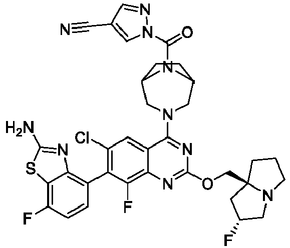 | 1-(3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-8-carbonyl)-1H-pyrazole-4-carbonitrile | 735.11 |
| A254 | 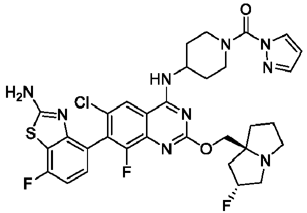 | (4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidin-1-yl)(1H-pyrazol-1-yl)methanone | 698.19 |
| A255^{†} | 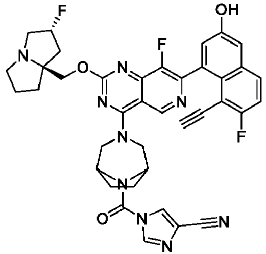 | 1-(3-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-3,8-diazabicyclo[3.2.1]octane-8-carbonyl)-1H-imidazole-4-carbonitrile | 719.75 |
| A256 | 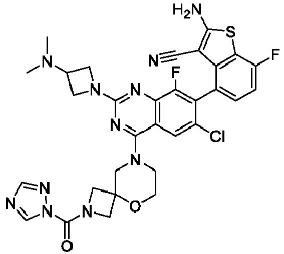 | 4-(4-(2-(1H-1,2,4-triazole-1-carbonyl)-5-oxa-2,8-diazaspiro[3.5]nonan-8-yl)-6-chloro-2-(3-(dimethylamino)azetidin-1-yl)-8-fluoroquinazolin-7-yl)-2-amino-7-fluorobenzo[b]thiophene-3-carbonitrile | 692.12 |
| A257 | 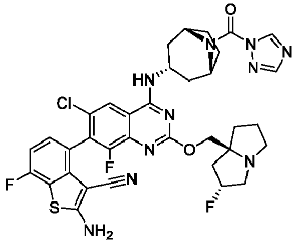 | 4-(4-(((1R,3r,5S)-8-(1H-1,2,4-triazole-1-carbonyl)-8-azabicyclo[3.2.1]octan-3-yl)amino)-6-chloro-8-fluoro-2-(((2R,7aS)-2-fluorotetrahydro-1H-pyrrolizin-7 a(5H)-yl)methoxy)quinazolin-7-yl)-2-amino-7-fluorobenzo[b]thiophene-3-carbonitrile | 749.23 |
| A258 | 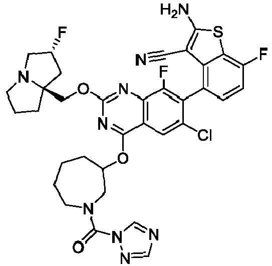 | 4-(4-((1-(1H-1,2,4-triazole-1-carbonyl)azepan-3-yl)oxy)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 738.13 |
| A259 | 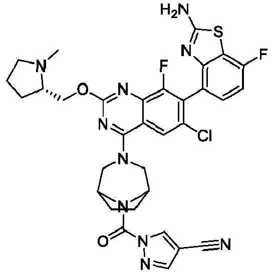 | 1-(3-(7-(2-amino-7-fluorobenzo[d]thiazol-4-yl)-6-chloro-8-fluoro-2-(((S)-1-methylpyrrolidin-2-yl)methoxy)quinazolin-4-yl)-3,8-diazabicyclo[3.2.1]octane-8-carbonyl)-1H-pyrazole-4-carbonitrile | 691.11 |
| A260 | 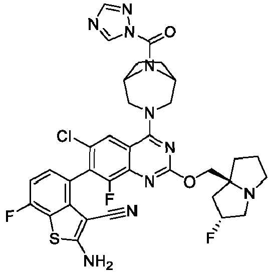 | 4-(4-(8-(1H-1,2,4-triazole-1-carbonyl)-3,8-diazabicyclo[3.2.1]octan-3-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 735.11 |
| A261 | 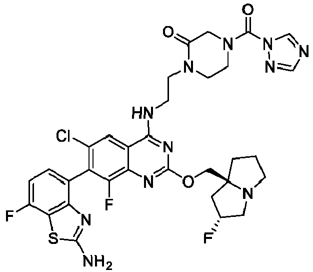 | 1-(2-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)ethyl)-4-(1H-1,2,4-triazole-1-carbonyl)piperazin-2-one | 742.19 |
| A262 | 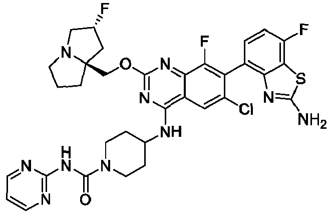 | 4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)-N-(pyrimidin-2-yl)piperidine-1-carboxamide | 725.19 |
| A263 | 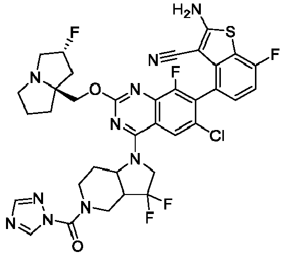 | 2-amino-4-(6-chloro-4-(3,3-difluoro-5-(1H-1,2,4-triazole-1-carbonyl)octahydro-1H-pyrrolo[3,2-c]pyridin-1-yl)-8-fluoro-2-(((2R,7aS)-2-fluorotetrahydro-1H-pyrrolizin-7a(5H)-yl)methoxy)quinazolm-7-yl)-7-fluorobenzo[b]thiophene-3-carbonitrile | 785.11 |
| A264 | 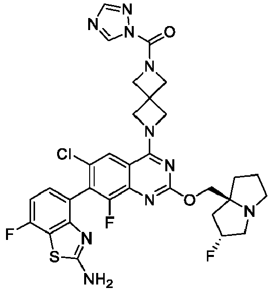 | (6-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)-2,6-diazaspiro[3.3]heptan-2-yl)(1H-1,2,4-triazol-1-yl)methanone | 697.11 |
| A265 | 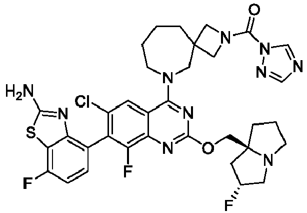 | (6-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)-2,6-diazaspiro[3.6]decan-2-yl)(1H-1,2,4-triazol-1-yl)methanone | 739.21 |
| A266 | 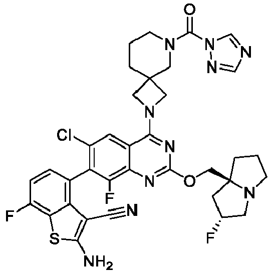 | 4-(4-(6-(1H-1,2,4-triazole-1-carbonyl)-2,6-diazaspiro[3.5]nonan-2-yl)-6-chloro-8-fluoro-2-(((2R,7aS)-2-fluorotetrahydro-1H-pyrrolizin-7a(5H)-yl)methoxy)quinazolm-7-yl)-2-amino-7-fluorobenzo[b]thiophene-3-carbonitrile | 749.21 |
| A267 | 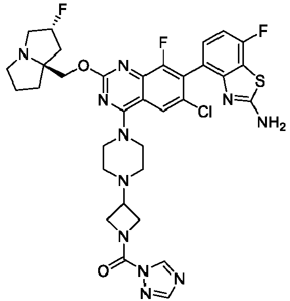 | (3-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolm-4-yl)piperazin-1-yl)azetidin-1-yl)(1H-1,2,4-triazol-1-yl)methanone | 740.23 |
| A268 | 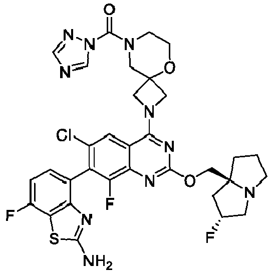 | (2-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-5-oxa-2,8-diazaspiro[3.5]nonan-8-yl)(1H-1,2,4-triazol-1-yl)methanone | 727.11 |
| A269 | 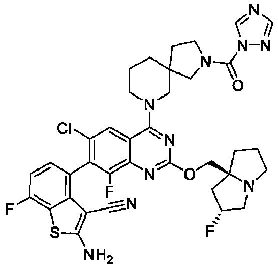 | 4-(4-(2-(1H-1,2,4-triazole-1-carbonyl)-2,7-diazaspiro[4.5]decan-7-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 763.21 |
| A270 | 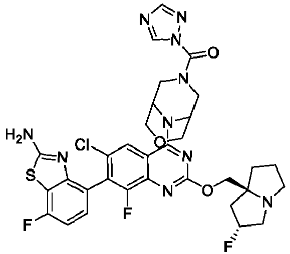 | (9-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3-oxa-7,9-diazabicyclo[3.3.1]nonan-7-yl)(1H-1,2,4-triazol-1-yl)methanone | 727.19 |
| A271 | 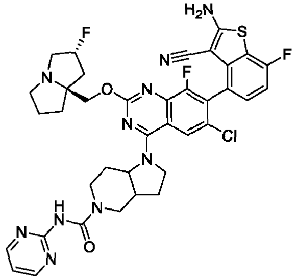 | 1-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-N-(pyrimidin-2-yl)octahydro-5H-pyrrolo[3,2-c]pyridine-5-carboxamide (second single octahydro-5H-pyrrolo[3,2-c]pyridine diastereomer) | 775.21 |
| A272 | 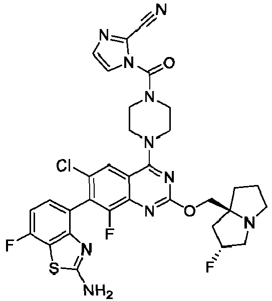 | 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)piperazine-1-carbonyl)-1H-imidazole-2-carbonitrile | 709.19 |
| A273 | 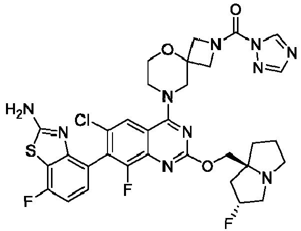 | (8-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-5-oxa-2,8-diazaspiro[3.5]nonan-2-yl)(1H-1,2,4-triazol-1-yl)methanone | 727.11 |
| A274 | 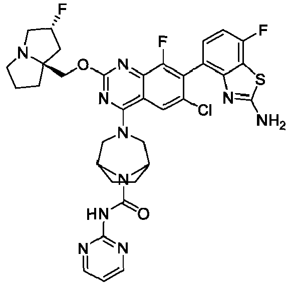 | 3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-N-(pyrimidin-2-yl)-3,8-diazabicyclo[3.2. 1]octane-8-carboxamide | 737.29 |
| A275 | 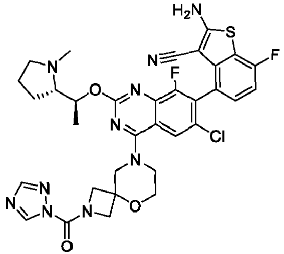 | 4-(4-(2-(1H-1,2,4-triazole-1-carbonyl)-5-oxa-2,8-diazaspiro[3.5]nonan-8-yl)-6-chloro-8-fluoro-2-((S)-1-((S)-1-methylpyrrolidin-2-yl)ethoxy)quinazolin-7-yl)-2-amino-7-fluorobenzo[b]thiophene-3-carbonitrile | 721.12 |
| A276 | 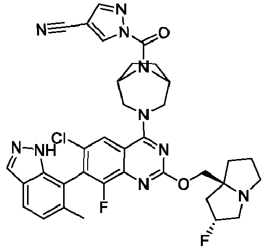 | 1-(3-(6-chloro-8-fluoro-2-(((2R,7aS)-2-fluorotetrahydro-1H-pyrrolizin-7 a(5H)-yl)methoxy)-7-(6-methyl-1H-indazol-7-yl)quinazolin-4-yl)-3,8-diazabicyclo[3.2.1]octane-8-carbonyl)-1H-pyrazole-4-carbonitrile | 699.19 |
| A277 | 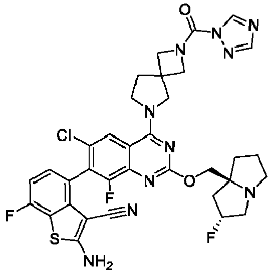 | 4-(4-(2-(1H-1,2,4-triazole-1-carbonyl)-2,6-diazaspiro[3.4]octan-6-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 735.11 |
| A278 | 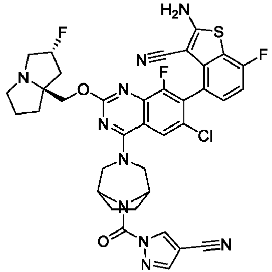 | 1-(3-(7-(2-amino-3-cyano-7-fluorobenzo[b]thiophen-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carbonyl)-1H-pyrazole-4-carbonitrile | 759.21 |
| A279 | 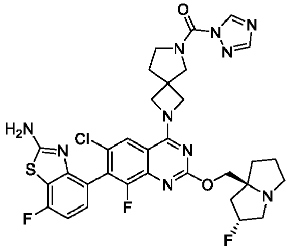 | (2-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,6-diazaspiro[3.4]octan-6-yl)(1H-1,2,4-triazol-1-yl)methanone | 711.11 |
| A280 | 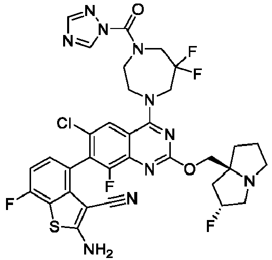 | 2-amino-4-(6-chloro-4-(6,6-difluoro-4-(1H-1,2,4-triazole-1-carbonyl)-1,4-diazepan-1-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile | 759.11 |
| A281 | 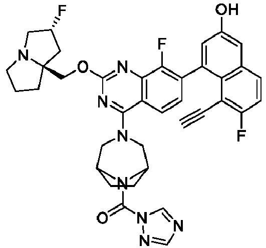 | (3-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(1H-1,2,4-triazol-1-yl)methanone | 694.73 |
| A282 | 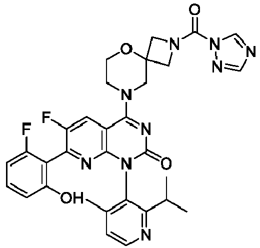 | 4-(2-(1H-1,2,4-triazole-1-carbonyl)-5-oxa-2,8-diazaspiro[3.5]nonan-8-yl)-6-fluoro-7-(2-fluoro-6-hydroxyphenyl)-1-(2-isopropyl-4-methylpyridin-3-yl)pyrido[2,3-d]pyrimidin-2(1H)-one | 629.62 |
| A283 | 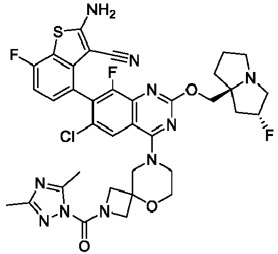 | 2-amino-4-(6-chloro-4-(2-(3,5-dimethyl-1H-1,2,4-triazole-1-carbonyl)-5-oxa-2,8-diazaspiro[3.5]nonan-8-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile | 779.13 |
| A284 | 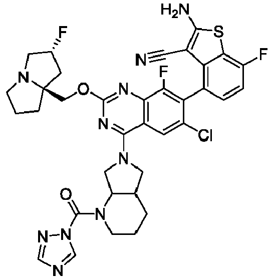 | 4-(4-(1-(1H-1,2,4-triazole-1-carbonyl)octahydro-6H-pyrrolo[3,4-b]pyridin-6-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 749.23 |
| A285 | 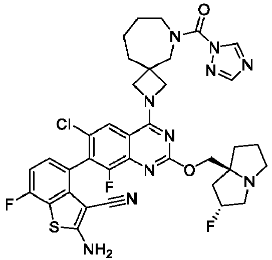 | 4-(4-(6-(1H-1,2,4-triazole-1-carbonyl)-2,6-diazaspiro[3.6]decan-2-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 763.31 |
| A286 | 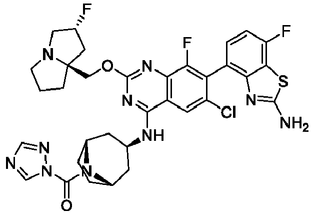 | ((1R,3s,5S)-3-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)-8-azabicyclo[3.2.1]octan-8-yl)(1H-1,2,4-triazol-1-yl)methanone | 725.11 |
| A287 | 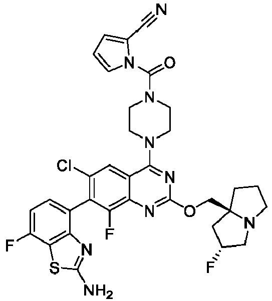 | 1-(4-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)piperazine-1-carbonyl)-1H-pyrrole-2-carbonitrile | 708.18 |
| A288 | 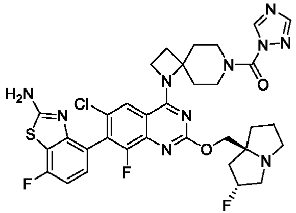 | (1-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-1,7-diazaspiro[3.5]nonan-7-yl)(1H-1,2,4-triazol-1-yl)methanone | 725.19 |
| A289 | 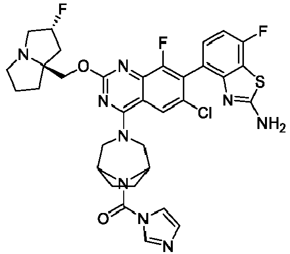 | (3-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octan-8-yl)(1H-imidazol-1-yl)methanone | 710.19 |
| A290 | 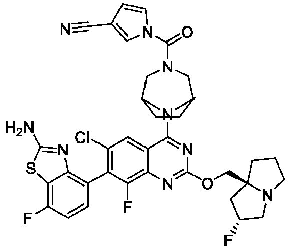 | 1-(8-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2. 1]octane-3-carbonyl)-1H-pyrrole-3-carbonitrile | 734.13 |
| A291 | 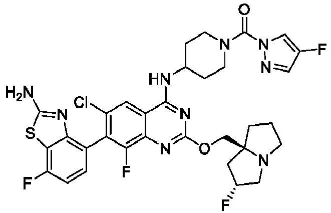 | (4-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)piperidin-1-yl)(4-fluoro-1H-pyrazol-1-yl)methanone | 716.13 |
| A292 | 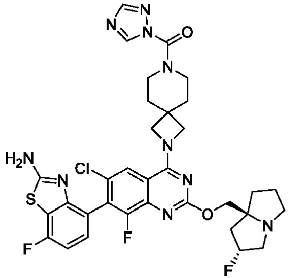 | (2-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,7-diazaspiro[3.5]nonan-7-yl)(1H-1,2,4-triazol-1-yl)methanone | 725.11 |
| A293 | 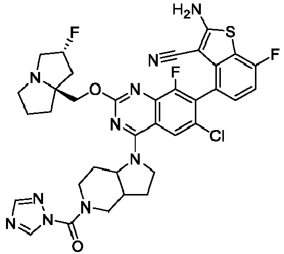 | 4-(4-(5-(1H-1,2,4-triazole-1-carbonyl)octahydro-1H-pyrrolo[3,2-c]pyridin-1-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 749.24 |
| A294 | 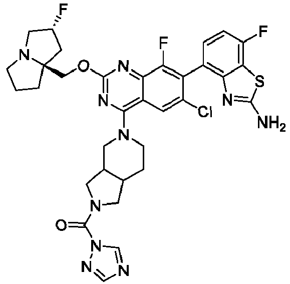 | (5-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)octahydro-2H-pyrrolo[3,4-c]pyridin-2-yl)(1H-1,2,4-triazol-1-yl)methanone | 725.19 |
| A295 | 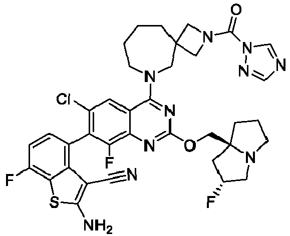 | 4-(4-(2-(1H-1,2,4-triazole-1-carbonyl)-2,6-diazaspiro[3.6]decan-6-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 763.21 |
| A296 | 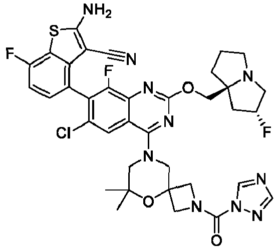 | 2-amino-4-(6-chloro-4-(6,6-dimethyl-2-(1H-1,2,4-triazole-1-carbonyl)-5-oxa-2,8-diazaspiro[3.5]nonan-8-yl)-8-fluoro-2-(((2R,7aS)-2-fluorotetrahydro-1H-pyrrolizin-7a(5H)-yl)methoxy)quinazolin-7-yl)-7-fluorobenzo[b]thiophene-3-carbonitrile | 779.21 |
| A297 | 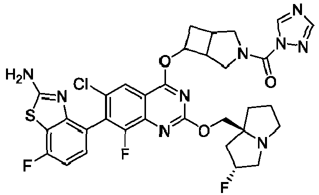 | (6-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)oxy)-3-azabicyclo[3.2.0]heptan-3-yl)(1H-1,2,4-triazol-1-yl)methanone | 712.19 |
| A298 | 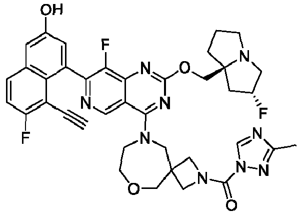 | (9-(7-(8-ethynyl-7-fluoro-3-hydroxynaphthalen-1-yl)-8-fluoro-2-(((2R,7aS)-2-fluorotetrahydro-1H-pyrrolizin-7a(5H)-yl)methoxy)pyrido[4,3-d]pyrimidin-4-yl)-6-oxa-2,9-diazaspiro[3.6]decan-2-yl)(3-methyl-1H-1,2,4-triazol-1-yl)methanone | 739.75 |
| A299 | 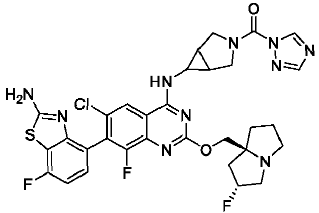 | (6-((7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)amino)-3-azabicyclo[3.1.0]hexan-3-yl)(1H-1,2,4-triazol-1-yl)methanone | 697.19 |
| A300 | 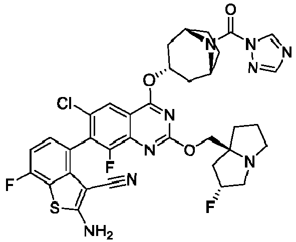 | 4-(4-(((1R,3r,5S)-8-(1H-1,2,4-triazole-1-carbonyl)-8-azabicyclo[3.2.1]octan-3-yl)oxy)-6-chloro-8-fluoro-2-(((2R,7aS)-2-fluorotetrahydro-1H-pyrrolizin-7 a(5H)-yl)methoxy)quinazolin-7-yl)-2-amino-7-fluorobenzo[b]thiophene-3-carbonitrile | 750.12 |
| A301 | 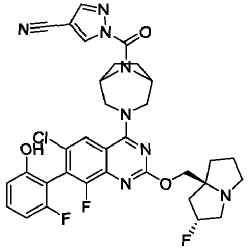 | 1-(3-(6-chloro-8-fluoro-7-(2-fluoro-6-hydroxyphenyl)-2-(((2R,7aS)-2-fluorotetrahydro-1H-pyrrolizin-7 a(5H)-yl)methoxy)quinazolin-4-yl)-3,8-diazabicyclo[3.2.1]octane-8-carbonyl)-1H-pyrazole-4-carbonitrile | 679.11 |
| A302* | 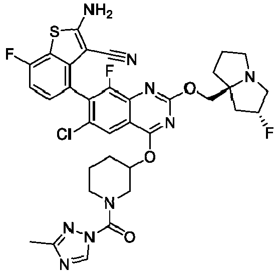 | 2-amino-4-(6-chloro-8-fluoro-2-(((2R,7aS)-2-fluorotetrahydro-1H-pyrrolizin-7a(5H)-yl)methoxy)-4-((1-(3-methyl-1H-1,2,4-triazole-1-carbonyl)piperidin-3-yl)oxy)quinazolin-7-yl)-7-fluorobenzo[b]thiophene-3-carbonitrile | 738.12 |
| A303^{†} | 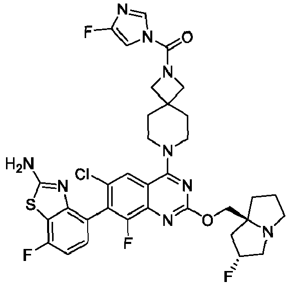 | (7-(7-(2-amino-7-fluorobenzo[d]thiazol-4-yl)-6-chloro-8-fluoro-2-(((2R,7aS)-2-fluorotetrahydro-1H-pyrrolizin-7a(5H)-yl)methoxy)quinazolin-4-yl)-2,7-diazaspiro[3.5]nonan-2-yl)(4-fluoro-1H-imidazol-1-yl)methanone | 742.11 |
| A304 | 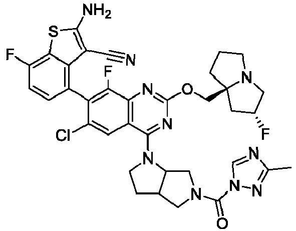 | 2-amino-4-(6-chloro-8-fluoro-2-(((2R,7aS)-2-fluorotetrahydro-1H-pyrrolizin-7a(5H)-yl)methoxy)-4-(5-(3 -methyl-1 H-1,2,4-triazole-1-carbonyl)hexahydropyrrolo[3,4-b]pyrrol-1(2H)-yl)quinazolin-7-yl)-7-fluorobenzo[b]thiophene-3-carbonitrile | 749.28 |
| A305* | 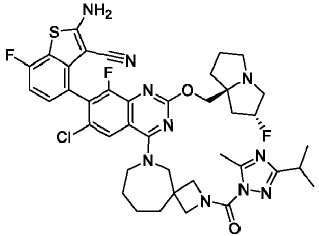 | 2-amino-4-(6-chloro-8-fluoro-2-(((2R,7aS)-2-fluorotetrahydro-1H-pyrrolizin-7 a(5H)-yl)methoxy)-4-(2-(3-isopropyl-5-methyl-1H-1,2,4-triazole-1-carbonyl)-2,6-diazaspiro[3.6]decan-6-yl)quinazolin-7-yl)-7-fluorobenzo[b]thiophene-3- | 819.28 |
| | | carbonitrile | |
| A306 | 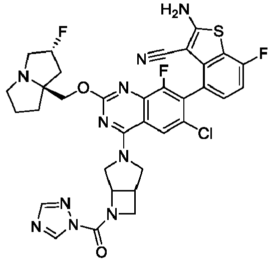 | 4-(4-(6-(1H-1,2,4-triazole-1-carbonyl)-3,6-diazabicyclo[3.2.0]heptan-3-yl)-6-chloro-8-fluoro-2-(((2R,7aS)-2-fluorotetrahydro-1H-pyrrolizin-7a(5H)-yl)methoxy)quinazolin-7-yl)-2-amino-7-fluorobenzo[b]thiophene-3-carbonitrile | 721.18 |

| | | | |
|---|---|---|---|
| *Compound provided as a mixture of regioisomers; ^{†}Compound provided as a substantially pure single regioisomer, though the structure provided has been tentatively assigned. | | | |

In some instances, particularly for compounds denoted with * or ^{†}, two or more regioisomers may exist and the compound structures and corresponding chemical names provided in Table 3 have been tentatively assigned. In the preparation of certain compounds herein, such as compounds comprising a pyrazole, imidazole, or triazole, the starting material may exist in two or more tautomeric forms, thus the products may be isolated from the respective reaction mixtures as a single regioisomer or as a mixture of regioisomers that result from reaction with the two or more tautomeric forms of the starting material. For example, Compound A102 was isolated and screened as a mixture of the two regioisomers depicted below, though for convenience only one regioisomer (A102-1) is depicted in Table 3:

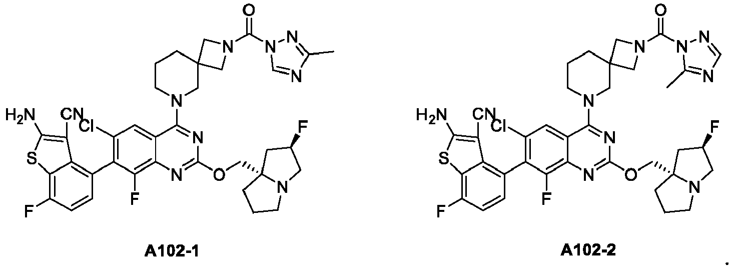

In another example, Compound A153 was isolated and screened as a mixture of the two regioisomers depicted below, though for convenience only one regioisomer (A153-1) is depicted in Table 3:

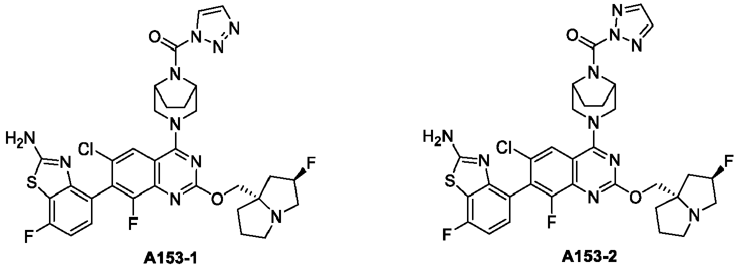

### EXAMPLE 2: Ras sequences

Human K-RasG12S (SEQ ID NO. 1): 
1 
51 
101 
151 
Human K-Ras Wildtype sequence (SEQ ID NO. 2) 
1 
51 
101 
151 
H-Ras G12S (SEQ ID NO. 3) 
1 MTEYKLVVVG ASGVGKSALT IQLIQNHFVD EYDPTIEDSY RKQVVIDGET
51 CLLDILDTAG QEEYSAMRDQ YMRTGEGFLC VFAINNTKSF EDIHQYREQI
101 KRVKDSDDVP MVLVGNKCDL AARTVESRQA QDLARSYGIP YIETSAKTRQ
151 GVEDAFYTLV REIRQHKLRK LNPPDESGPG CMSCKCVLS
H-Ras wildtype (SEQ ID NO. 4) 
1 
51 
101 
151 
Human N-Ras G12S (SEQ ID NO. 5) 
1 MTEYKLVVVG ASGVGKSALT IQLIQNHFVD EYDPTIEDSY RKQVVIDGET
51 CLLDILDTAG QEEYSAMRDQ YMRTGEGFLC VFAINNSKSF ADINLYREQI
101 KRVKDSDDVP MVLVGNKCDL PTRTVDTKQA HELAKSYGIP FIETSAKTRQ
151 GVEDAFYTLV REIRQYRMKK LNSSDDGTQG CMGLPCVVM
Human N-Ras wildtype (SEQ ID NO. 6) 
1 MTEYKLVVVG AGGVGKSALT IQLIQNHFVD EYDPTIEDSY RKQVVIDGET
51 CLLDILDTAG QEEYSAMRDQ YMRTGEGFLC VFAINNSKSF ADINLYREQI
101 KRVKDSDDVP MVLVGNKCDL PTRTVDTKQA HELAKSYGIP FIETSAKTRQ
151 GVEDAFYTLV REIRQYRMKK LNSSDDGTQG CMGLPCVVM
Human K-Ras G12D (SEQ ID NO. 7) 
1 
51 
101 
151 
Human K-Ras G12V (SEQ ID NO. 8)

1 MTEYKLVVVG AVGVGKSALT IQLIQNHFVD EYDPTIEDSY RKQVVIDGET
51 CLLDILDTAG QEEYSAMRDQ YMRTGEGFLC VFAINNTKSF EDIHHYREQI
101 KRVKDSEDVP MVLVGNKCDL PSRTVDTKQA QDLARSYGIP FIETSAKTRQ
151 GVDDAFYTLV REIRKHKEKM SKDGKKKKKK SKTKCVIM

### EXAMPLE 3: Protein expression

DNA expression constructs encoding one or more protein sequences of interest (e.g., Kras fragments thereof, mutant variants thereof, etc.) and its corresponding DNA sequences are optimized for expression in E. coli and synthesized by, for example, the GeneArt Technology at Life Technologies. In some cases, the protein sequences of interest are fused with a tag (e.g., glutathione S-transferase (GST), histidine (His), or any other affinity tags) to facilitate recombinant expression and purification of the protein of interest. Such tag can be cleaved subsequent to purification. Alternatively, such tag may remain intact to the protein of interest and may not interfere with activities (e.g., target binding and/or phosphorylation) of the protein of interest

A resulting expression construct is additionally encoded with (i) att-site sequences at the 5'and 3' ends for subcloning into various destination vectors using, for example, the Gateway Technology, as well as (ii) a Tobacco Etch Virus (TEV) protease site for proteolytic cleavage of one or more tag sequences. The applied destination vectors can be a pET vector series from Novagen (e.g., with ampicillin resistance gene), which provides an N-terminal fusion of a GST-tag to the integrated gene of interest and/or a pET vector series (e.g., with ampicillin resistance gene), which provides a N-terminal fusion of a HIS-tag to the integrated gene. To generate the final expression vectors, the expression construct of the protein of interest is cloned into any of the applied destination ventors. The expression vectors are transformed into E. coli strain, e.g., BL21 (DE3). Cultivation of the transformed strains for expression is performed in 10 L and 1 L fermenter. The cultures are grown, for example, in Terrific Broth media (MP Biomedicals, Kat. #1 13045032) with 200 ug/mL ampicillin at a temperature of 37 °C to a density of 0.6 (OD600), shifted to a temperature of ~27 °C (for K-Ras expression vectors) induced for expression with 100 mM IPTG, and further cultivated for 24 hours. After cultivation, the transformed E. coli cells are harvested by centrifugation and the resulting pellet is suspended in a lysis buffer, as provided below, and lysed by passing three-times through a high pressure device. The lysate is centrifuged (49000g, 45 min, 4 °C) and the supernatant is used for further purification.

### EXAMPLE 4: Ras protein purification

A Ras (e.g., K-Ras wildtype or a mutant such as K-Ras G12S, K-Ras G12D, K-Ras G12V or K-RasG12C) construct or a variant thereof is tagged with GST. E. coli culture from a 10L fermenter is lysed in lysis buffer (50mM Tris HCI 7.5, 500mM NaCI,1 mM DTT, 0,5% CHAPS, Complete Protease Inhibitor Cocktail-(Roche)). As a first chromatography step, the centrifuged lysate is incubated with 50mL Glutathione Agarose 4B (Macherey-Nagel; 745500.100) in a spinner flask (16 h, 10'). The Glutathione Agarose 4B loaded with protein is transferred to a chromatography column connected to a chromatography system, e.g., an Akta chromatography system. The column is washed with wash buffer (50mM Tris HCI 7.5, 500mM NaCI, 1 mM DTT) and the bound protein is eluted with elution buffer (50mM Tris HCI 7.5, 500mM NaCI, 1 mM DTT, 15mM Glutathione). The main fractions of the elution peak (monitored by OD280) is pooled. For further purification by size-exclusion chromatography, the above eluate volume is applied to a column Superdex 200 HR prep grade (GE Healthcare) and the resulting peak fractions of the eluted fusion protein is collected. Native mass spectrometry analyses of the final purified protein construct can be performed to assess its homogeneous load with GDP.

### EXAMPLE 5: HTRF (homogenous time-resolved fluorescence resonance energy transfer assay

The ability of a compound of the present disclosure to reduce a Ras signaling output can be demonstrated by an HTRF assay. This assay can be also used to assess a selective inhibition or reduction of signaling output of a mutant Ras protein relative to a wildtype, or relative to a different mutant Ras protein. For example, the equilibrium interaction of wildtype Kras or K-Ras mutant (e.g., wildtype or a mutant thereof including those mentioned in Example 6) with SOS1 (e.g., hSOS1) can be assessed as a proxy or an indication for a subject compound's ability to bind and inhibit Ras protein. HTRF assay detects from (i) a fluorescence resonance energy transfer (FRET) donor (e.g., antiGST-Europium) that is bound to GST-tagged K-Ras mutant to (ii) a FRET acceptor (e.g., anti-6His-XL665) bound to a His-tagged hSOS 1.

The assay buffer can contain ~5 mM HEPES pH 7.4, -150 mM NaCI, - 1 mM DTT, 0.05% BSA and 0.0025% (v/v) Igepal. A Ras working solution is prepared in an assay buffer containing typically a suitable amount of the protein construct (e.g., GST-tagged K-Ras mutant) and the FRET donor (e.g., antiGST-Eu(K) from Cisbio, France). A SOS1 working solution is prepared in an assay buffer containing suitable amount of the protein construct (e.g., His-hSOS1) and the FRET acceptor (e.g., anti-6His-XL665 from Cisbio, France). A suitable amount of the protein construct will depend on the range of activity or range of IC50 values being detected or under investigation. For detecting IC50 within a range of 500 nM, the protein constructs of the same range of molarity can be utilized. An inhibitor control solution is prepared in an assay buffer containing comparable amount of the FRET acceptor without the SOS 1 protein.

A fixed volume of DMSO with or without test compound is transferred into a 384-well plate. Ras working solution is added to all wells of the test plate. SOS1 working solution is added to all wells except for those that are subsequently filled the inhibitor control solution. Upon incubation for about 10 minutes or longer, the fluorescence is measured with a M1000Pro plate reader (Tecan) using HTRF detection (excitation 337nm, emission 1 : 620nm, emission 2: 665nm). Compounds are tested in duplicates at different concentrations (for example, 10 µM, 2.5 µM, 0.63 µM, 0.16 µM, 0.04 µM, 0.01 µM test compound). The ratiometric data (i.e., emission 2 divided by emission 1) is used to calculate IC50 values against Ras using GraphPad Prism (GraphPad software). Following this general procedure, samples were tested with or without a subject compound disclosed herein including compounds exemplified in Table 3 to assess their abilities to inhibit K-Ras G12S relative to another mutant (e.g., K-Ras G12D). Signaling output measured in terms of IC50 values can be obtained, a ratio of IC50 against one mutant relative to another mutant can be calculated. For instance, a selective reduction of K-Ras G12S signaling output can be evidenced by a ratio greater than one. In particular, a selective reduction of K-Ras G12S signaling relative to K-Ras G12D signaling is evidenced as the ratio of IC50 (against K-Ras G12D) to IC50 (against K-Ras G12S) is greater than 1. As is summarized below in Table 4, one or more subject precursor compounds disclosed herein (including compounds shown in Table 3) exhibit selective inhibition of K-Rras G12S relative to K-Ras G12D by at least 1-fold, and in some instances greater than 2-, 3-, 4- or 5-fold. A subject precursor compounds including the compounds disclosed in Table 3 exhibit an IC50 against K-Ras G12S less than 500 nM, less than 100 nM, 50 nM, 10 nM or even less.

**Table 4**

| **Compound** | **Ratio of IC50 KRAS G12D vs. IC50 KRAS G12S** | **% G12S Modification at 24 hr.** | **% G12D Modification at 24 hr.** | **%WT Modification at 24 hr.** |
|---|---|---|---|---|
| A101 | A | - | - | - |
| A102 | A | +++ | ND | ND |
| A103 | - | ND | - | - |
| A104 | A | ++ | - | - |
| A105 | - | - | - | - |
| A106 | - | +++ | ND | - |
| A107 | A | ++ | ND | ND |
| A108 | A | ND | ND | ND |
| A109 | - | ND | ND | ND |
| A110 | A | +++ | ND | ND |
| A111 | A | ND | - | - |
| A112 | A | ND | - | - |
| A113 | A | - | - | - |
| A114 | A | +++ | - | - |
| A115 | B | ND | ND | - |
| A116 | B | - | - | - |
| A117 | A | - | - | - |
| A118 | A | - | - | - |
| A119 | A | +++ | ND | ND |
| A120 | A | ND | - | - |
| A121 | A | ND | - | - |
| A122 | A | ND | ND | ND |
| A123 | A | ++ | ND | ND |
| A124 | A | - | - | - |
| A125 | A | ++ | ND | - |
| A126 | A | +++ | - | - |
| A127 | A | ++ | ND | ND |
| A128 | - | +++ | ND | - |
| A129 | A | +++ | - | - |
| A130 | A | - | - | - |
| A131 | - | ND | ND | ND |
| A132 | A | +++ | - | - |
| A133 | - | +++ | ND | - |
| A134 | A | +++ | ND | - |
| A135 | A | ND | - | - |
| A136 | A | +++ | - | - |
| A137 | A | +++ | ND | ND |
| A138 | A | ND | - | - |
| A139 | A | - | - | - |
| A140 | A | +++ | - | - |
| A141 | A | +++ | ND | ND |
| A142 | A | ND | - | - |
| A143 | - | ND | - | - |
| A144 | A | ND | ND | - |
| A145 | A | +++ | ND | ND |
| A146 | A | +++ | ND | ND |
| A147 | A | - | - | - |
| A148 | A | +++ | ND | ND |
| A149 | A | - | - | - |
| A150 | A | - | - | - |
| A151 | A | ND | - | - |
| A152 | A | +++ | - | - |
| A153 | A | - | - | - |
| A154 | A | +++ | ND | - |
| A155 | A | ND | - | - |
| A156 | A | - | - | - |
| A157 | A | ++ | - | - |
| A158 | A | - | - | - |
| A159 | A | - | - | - |
| A160 | A | ND | - | - |
| A161 | A | +++ | ND | - |
| A162 | A | +++ | ND | - |
| A163 | B | - | - | - |
| A164 | A | - | - | - |
| A165 | A | ++ | ND | - |
| A166 | A | ND | - | - |
| A167 | A | ND | ND | ND |
| A168 | A | ND | - | - |
| A169 | A | +++ | ND | ND |
| A170 | A | ++ | ND | ND |
| A171 | A | +++ | - | - |
| A172 | A | ND | - | - |
| A173 | A | - | - | - |
| A174 | A | ND | ND | ND |
| A175 | A | ND | - | - |
| A176 | B | ND | - | - |
| A177 | B | - | - | - |
| A178 | - | +++ | - | - |
| A179 | A | ND | - | - |
| A180 | A | - | - | - |
| A181 | A | +++ | - | - |
| A182 | A | ++ | ND | ND |
| A183 | A | ++ | - | - |
| A184 | A | ND | - | - |
| A185 | A | ++ | ND | ND |
| A186 | A | ND | ND | ND |
| A187 | A | ND | - | - |
| A188 | A | ND | - | - |
| A189 | A | ND | ND | ND |
| A190 | A | - | - | - |
| A191 | A | +++ | ND | ND |
| A192 | A | ND | - | - |
| A193 | A | - | - | - |
| A194 | A | ++ | ND | ND |
| A195 | A | +++ | ND | ND |
| A196 | - | ND | ND | ND |
| A197 | A | +++ | ND | - |
| A198 | A | +++ | - | - |
| A199 | A | +++ | ND | ND |
| A200 | B | - | - | - |
| A201 | A | +++ | - | - |
| A202 | B | +++ | ND | - |
| A203 | A | - | - | - |
| A204 | A | ++ | - | - |
| A205 | - | ND | - | - |
| A206 | A | ND | ND | - |
| A207 | A | +++ | - | - |
| A208 | A | - | - | - |
| A209 | A | ++ | ND | - |
| A210 | - | +++ | ND | - |
| A211 | A | ND | - | - |
| A212 | - | +++ | - | - |
| A213 | A | ++ | ND | ND |
| A214 | A | - | - | - |
| A215 | - | - | - | - |
| A216 | - | ND | ND | ND |
| A217 | - | ND | - | - |
| A218 | A | +++ | - | - |
| A219 | - | +++ | ND | ND |
| A220 | - | ND | - | - |
| A221 | A | ++ | ND | - |
| A222 | A | - | - | - |
| A223 | A | - | - | - |
| A224 | A | ++ | ++ | ND |
| A225 | A | ++ | - | - |
| A226 | A | ND | ND | - |
| A227 | A | +++ | - | - |
| A228 | - | ND | ND | - |
| A229 | B | - | - | - |
| A230 | A | +++ | ND | - |
| A231 | A | ++ | - | - |
| A232 | A | ND | ND | - |
| A233 | A | ND | - | - |
| A234 | - | ND | - | - |
| A235 | A | ND | ND | - |
| A236 | A | ++ | - | - |
| A237 | A | - | - | - |
| A238 | A | ND | - | - |
| A239 | A | ND | - | - |
| A240 | A | - | - | - |
| A241 | B | ND | ND | - |
| A242 | A | ND | - | - |
| A243 | A | +++ | ND | - |
| A244 | A | - | - | - |
| A245 | A | ND | - | - |
| A246 | B | ND | ND | ND |
| A247 | B | ND | - | - |
| A248 | A | ND | - | - |
| A249 | A | ND | - | - |
| A250 | A | ND | - | - |
| A251 | - | ND | ND | ND |
| A252 | A | +++ | - | - |
| A253 | B | ND | ND | - |
| A254 | A | - | - | - |
| A255 | - | ++ | ND | ND |
| A256 | A | +++ | ND | - |
| A257 | A | ++ | ND | - |
| A258 | A | +++ | ND | ND |
| A259 | A | ND | - | - |
| A260 | A | ND | ND | - |
| A261 | A | - | - | - |
| A262 | A | - | - | - |
| A263 | A | ++ | - | - |
| A264 | A | - | - | - |
| A265 | - | +++ | ND | - |
| A266 | A | +++ | ND | - |
| A267 | A | ND | - | - |
| A268 | A | +++ | - | - |
| A269 | A | ++ | ND | - |
| A270 | A | ND | - | - |
| A271 | A | ND | ND | ND |
| A272 | B | +++ | - | - |
| A273 | A | +++ | - | - |
| A274 | A | ND | - | - |
| A275 | - | +++ | ND | ND |
| A276 | B | ND | - | - |
| A277 | A | +++ | - | - |
| A278 | A | ND | - | - |
| A279 | A | +++ | ND | - |
| A280 | A | +++ | ND | - |
| A281 | - | ND | ND | - |
| A282 | - | ND | ND | - |
| A283 | A | +++ | ND | ND |
| A284 | A | +++ | - | - |
| A285 | A | +++ | ND | ND |
| A286 | A | ND | - | - |
| A287 | B | ++ | - | - |
| A288 | A | ND | ND | - |
| A289 | A | ND | - | - |
| A290 | A | ND | - | - |
| A291 | A | ND | - | - |
| A292 | A | ND | - | - |
| A293 | - | - | - | - |
| A294 | A | ND | - | - |
| A295 | B | +++ | ND | - |
| A296 | A | +++ | ND | - |
| A297 | A | ND | ND | - |
| A298 | A | +++ | ND | ND |
| A299 | A | - | - | - |
| A300 | A | ND | ND | - |
| A301 | B | ND | ND | - |
| A302 | A | ++ | ND | ND |
| A303 | A | ND | - | - |
| A304 | A | +++ | ND | ND |
| A305 | B | - | - | - |
| A306 | B | ND | ND | ND |

| | | | | |
|---|---|---|---|---|
| ++ indicates between about 1%- 20% labelling +++ indicates greater than 20% labelling ND indicates no detectable labeling - indicates not tested A: indicates that the ratio of IC50 against KrasG12D vs. IC50 against KrasG12S is 1-fold or greater | | | | |

### EXAMPLE 6: GTPase activity assay

The ability of any compound of the present disclosure to inhibit a Ras protein signalling can be demonstrated by a reduced GTPase activity. This assay can be also used to assess a selective inhibition of a mutant Ras protein relative to a wildtype, or relative to a different mutant Ras protein. For instance, the assay can be used to establish a subject compound's ability to selectively inhibit Kras G12D relative to wildtype, G12S relative to wildtype, Kras G12V relative to wildtype, KrasG12S relative KrasG12V, KrasG12S relative KrasG12D, or KrasG12D relative KrasG12V. In particular, intrinsic and GTPase-activating protein (GAP)-stimulated GTPase activity for K-Ras construct or a mutant thereof can be measured using EnzCheck phosphate assay system (Life Technologies). For example K-Ras WT, K-Ras D154Q mutant, K-Ras G12D mutant, K-Ras G12S mutant, and K-Ras G12D/D154Q mutant proteins (2.5 mg/ml) in buffer (20 mmol/L Tris, pH 8.0, 50 mM NaCl) is loaded with GTP at room temperature for 2 hours by exposing to exchange buffer containing EDTA. Proteins are buffer exchanged to assay buffer (30 mM Tris, pH 7.5, 1 mM DTT) and the concentration is adjusted to 2 mg/ml. GTP loading is verified by back extraction of nucleotide using 6M urea and evaluation of nucleotide peaks by HPLC using an ion-exchange column. The assay is performed in a clear 384-well plate (Costar) by combining GTP-loaded K-Ras proteins (50 mM final) with 2-amino-6-mercapto-7-methylpurine ribonucleoside (MESG) (200 mM final), and purine nucleotide phosphorylase (5 U/ml final). GTP hydrolysis is initiated by the addition of MgC12 at a working concentration of 40 mM. For GAP stimulation, Ras p21 protein activator 1 (P120GAP) can be included at 50 mM. Absorbance at 360 nm can be measured every 8 to 15 s for 1,000 s at 20 °C. Samples are tested with or without a subject compound disclosed herein including compounds exemplified in Table 1 to assess each compound's ability to inhibit signaling of a given Ras protein (e.g., a given mutant Kras) of interest.

### EXAMPLE 7: Nucleotide exchange assay

The ability of a compound of the present disclosure to inhibit a Ras protein signaling can be demonstrated by a reduced nucleotide exchange activity. This assay can be also used to assess a selective inhibition of a mutant Ras protein relative to a wildtype, or relative to a different mutant Ras protein. For example, 250 nM or 500 nM GDP-loaded K-Ras proteins (e.g., wildtype or a mutant thereof including those mentioned in Example 6), each is incubated with different concentrations of compounds (for example -60 µM, -20 µM, -6.7 µM, -2.2 µM, -0.7 µM, -0.2 µM subject compound). A control reaction without subject compound is also included. SOS1 (catalytic domain) protein is added to the K-Ras protein solution. The nucleotide exchange reaction is initiated by adding fluorescent labelled GDP (Guanosine 5'-Diphosphate, BODIPY^{™} FL 2'-(or-3')-O-(N-(2-Aminoethyl) Urethane) to a final concentration of 0.36 µM. Fluorescence is measured every 30 s for 70 minutes at 490nm/515mn (excitation/emission) in a M1000Pro plate reader (Tecan). Data is exported and analyzed to calculate an IC50 using GraphPad Prism (GraphPad Software). Sample(s) can be tested with or without a subject compound disclosed herein including compound(s) exemplified in Table 1 to assess compound's ability to inhibit K-Ras signaling or its IC50 against a given Ras protein (e.g., a given mutant K-Ras) of interest.

### EXAMPLE 8: Testing for modification of Ras protein via covalent binding

Test compounds are prepared as 10 mM stock solutions in DMSO (Fisher cat#BP231-100). KRAS protein (His-tagged GDP-loaded wildtype 1-169, His-tagged GDP-loaded G12S 1-169 or His-tagged GDP-loaded G12D 1-169) is diluted to ~2 µM in appropriate buffer (e.g., a Hepes buffer at physiological conditions). For testing KRAS modification, compounds are diluted to 50X final test concentration in DMSO in 96- well storage plates. 2 µl of the diluted 50X compounds are added to appropriate wells in the PCR plate (Fisher cat#AB-0800). -49 µl of the stock protein solution is added to each well of the 96-well PCR plate. Reactions are mixed carefully. The plate is sealed well with aluminum plate seal, and stored in drawer at room temperature for 24hrs. 5 µl of 2% formic acid (Fisher cat#A1 17-50) in MilliQ H2O is then added to each well followed by mixing with a pipette. The plate is then resealed with aluminum seal and stored until mass spectrometry analysis.

The extent of covalent modification of KRAS proteins was determined by liquid chromatography electrospray mass spectrometry analysis of the intact proteins on a Thermo Q-Exactive Plus mass spectrometer. 20 µl of sample was injected onto a bioZen 3.6 µm Intact C4 column (Phenomenex cat#00B-4767-AN) placed in a column oven set to 40°C and separated using a suitable LC gradient from -20% to ~60% solvent B. Solvent A was 0.1% formic acid and solvent B was 0.1% formic acid in acetonitrile. HESI source settings were set to 40, 5 and 1 for the sheath, auxiliary and sweep gas flow, respectively. The spray voltage was 4 kV, and the capillary temperature was 320°C. S-lens RF level was 50 and auxiliary gas heater temperature was set to 200°C. The mass spectrometry was acquired using a scan range from 650 to 1750 m/z using positive polarity at a mass resolution of 70,000, AGC target of 1e6 ions and maximum injection time of 250ms. The recorded protein mass spectrum was deconvoluted from the raw data file using Protein Deconvolution v4.0 (Thermo). The protein mass and adduct masses were exported with their peak intensities. The peak intensities for the unmodified and modified protein were used to calculate the percent covalent modification of the KRAS protein based on the following equation: %KRAS protein modification = ((KRAS-compound) / (KRAS) + (KRAS-Compound)) *100. Applying the method exemplified herein, subject precursor compounds exhibit selective labeling of Ras G12S mutant relative to Ras G12D or wildtype mutant. Table 4 disclosed herein summarizes the results that exemplary precursor compounds including those shown in Table 3 can covalently label K-Ras G12S mutant by at least 1%, 10%, 20%, 50% or more, whereas no detectable labeling was registered for K-Ras G12D or wildtype when tested under comparable or same conditions.

One problem in the art is that conventional warheads, such as acrylamides, chlorofluoracetamides, epoxides, and nitriles, substantially lack the ability to engage with the serine residue at position 12 of K-Ras G12S mutant, despite that many of these engage with cysteine residue at position 12 of K-Ras. The present disclosure provides warheads that are particularly suited for reacting with the serine residue at position 12 of K-Ras G12S mutant as a solution to this problem. Table 5 below summarizes sets of direct comparative data, assessed according to the method described above, demonstrating the advantages of a suitable warhead for targeting K-Ras G12S covalently.

**Table 5**

| Compound | K-Ras12S engagement after 24-hour treatment |
|---|---|
| 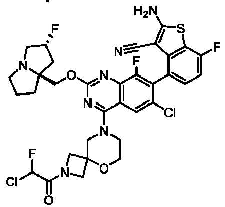 | ND |
| 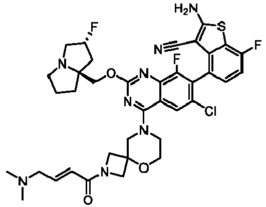 | ND |
| 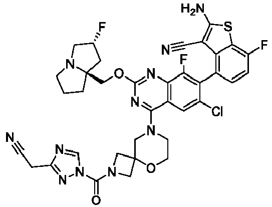 Compound No. A110 disclosed herein | +++ |
| | |
| 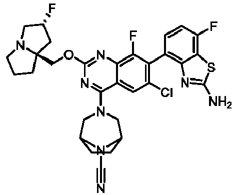 | ND |
| 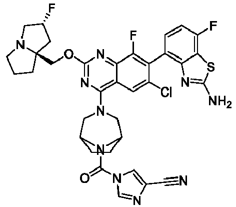 Compound No. A123 disclosed herein | ++ |
| | |
| 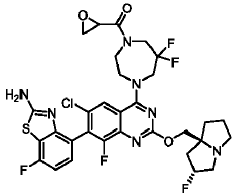 | ND |
| 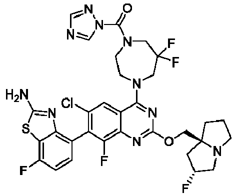 Compound No. A141 disclosed herein | +++ |
| | |
| 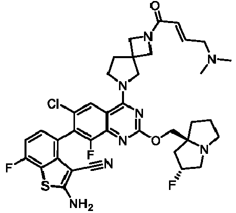 | ND |
| 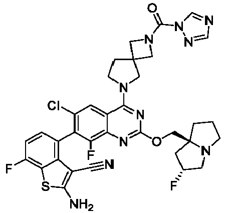 Compound No. A277 disclosed herein | +++ |

| | |
|---|---|
| +++: indicates equal to or greater than 20% labelling ++: indicates between 1% to 20% labelling ND: indicates no detectable engagement | |

The above direct comparison, wherein compound pairs differ only at R⁶, clearly shows that a subject compound having a warhead described herein is capable of engaging or labeling K-Ras G12S covalently. Additional disclosure and data provided herein demonstrate that such covalent engagement enhances K-Ras G12S inhibition potency, ascertained either by a biochemical assay or cellular proliferation assay. Such potency enhancement is also selective against K-Ras G12S.

### EXAMPLE 9: Ras cellular assay

The ability of any compound of the present disclosure to inhibit a Ras protein signaling can be demonstrated by inhibiting growth of a given Kras mutant cell line. For example, this assay can be used to assess a selective growth inhibition of a mutant Ras protein relative to a wildtype, or relative to a different mutant Ras protein.

### a. Growth of cells with K-Ras G12C mutation

MIA PaCa-2 (ATCC CRL-1420) and NCI-H1792 (ATCC CRL-5895) cell lines comprise a G12C mutation and can be used to assess Ras cellular signaling in vitro, e.g., in response to a subject inhibitor compound of the present disclosure. This cellular assay can also be used to discern selective inhibition of a subject compounds against certain types of Kras mutants, e.g., more potent inhibition against KrasG12D relative to KrasG12C mutant, by using MIA PaCa-2 (G12C driven tumor cell line) as a comparison. MIA PaCa-2 culture medium is prepared with DMEM/Ham's F12 (e.g., with stable Glutamine, 10% FCS, and 2.5% Horse Serum. NCI-H1792 culture medium is prepared with RPMI 1640 (e.g., with stable Glutamine) and 10% FCS.

On a first day (e.g., Day 1), Softagar (Select Agar, Invitrogen, 3% in ddH₂0 autoclaved) is boiled and tempered at 48 °C. Appropriate culture medium (i.e., medium) is tempered to 37 °C. Agar (3%) is diluted 1:5 in medium (=0.6%) and 50 mI/well plated into 96 well plates (Corning, #3904), then incubated at room temperature for agar solidification. A 3% agar is diluted to 0.25% in medium (1:12 dilution) and tempered at 42 °C. Cells are trypsinized, counted, and tempered at 37 °C. The cells (e.g., MIA PaCa-2 at about 125-150 cells, NCI-H1792 at about 1000 cells) are resuspended in 100 mL 0.25% Agar and plated, followed by incubation at room temperature for agar solidification. The wells are overlaid with 50 mL of the medium. Sister wells in a separate plate are plated for time zero determination. All plates are incubated overnight at 37 °C and 5% CO₂.

On a second day (e.g., Day 2), time zero values are measured. A 40 mL volume of Cell Titer 96 Aqueous Solution (Promega) is added to each well and incubated in the dark at 37 °C and 5% CO₂. Absorption can be measured at 490 nm and reference wavelength 660 nm. DMSO-prediluted test compounds are added to wells of interest, e.g., with HP Dispenser, to one or more desired concentrations (e.g., a final DMSO concentration of 0.3%).

On a tenth day (e.g., Day 10), absorption by wells treated with the test compounds and control wells are measured with, for example, Cell Titer 96 AQueous and analyzed in comparison to the time zero measurements. The IC50 values are determined using the four parameter fit. The resulting IC50 value is a measurement of the ability of the compounds herein to reduce cell growth of Ras-driven cells (e.g., tumor cell lines) in vitro and/or in vivo.

### b. Growth of cells with K-Ras G12D mutation

ASPC-1 (ATCC CRL-1682), Panc-10.05 (ATCC CRL-2547), A427, and GP2D (Sigma 95090714) cell lines comprise a G12D mutation and can be used to assess Ras cellular signaling in vitro, e.g., in response to the compounds herein. This cellular assay can also be used to discern selective inhibition of a subject compound against certain types of Kras mutants, e.g., more potent inhibition against KrasG12S relative to KrasG12D mutant, by using GP2D (G12D driven tumor cell line) as a comparison. ASPC-1 culture medium is prepared with RPMI-1640 and 10% heat-inactivated FBS. Panc-10.05 culture medium is prepared with RPMI-1640, 10 Units/ml human recombinant insulin, and 10% FBS. A427 cell culture is prepared with RPMI-1640 and 10% heat-inactivated FBS. GP2D culture medium is prepared with DMEM with 2mM glutamine and 10% heat-inactivated FBS. A Cell Titer-Glo (CTG) luminescent based assay (Promega) is used to assess growth of the cells, as a measurement of the ability of the compounds herein to inhibit Ras signaling in the cells. The cells (e.g., 800 per well) are seeded in their respective culture medium in standard tissue culture-treated 384-well format plates (Falcon #08-772-116) or ultra-low attachment surface 384-well format plates (S-Bio # MS-9384UZ ). The day after plating, cells are treated with a dilution series (e.g., a 9 point 3-fold dilution series) of the compounds herein (e.g., approximately 40 µl final volume per well). Cell viability can be monitored (e.g., approximately 5 days later) according to the manufacturer's recommended instructions, where the CellTiter-Glo reagent is added (e.g., approximately 10 µl), vigorously mixed, covered, and placed on a plate shaker (e.g., approximately for 20 min) to ensure sufficient cell lysis prior to assessment of luminescent signal. The IC50 values are determined using the four parameter fit. The resulting IC50 value is a measurement of the ability of the compounds herein to reduce cell growth of Ras-driven cells (e.g., tumor cell lines) in vitro and/or in vivo. The ability of one or more compounds exemplified in Table 3 to inhibit growth of one or more cell lines comprising a given Kras mutation is demonstrated utilizing the procedures described above.

Table 6 shows the resulting IC50 values of exemplary compounds against GP2D cells using the cell proliferation assays described herein. Compound numbers correspond to the numbers and structures provided in Table 3 and Example 1.

**Table 6**

| | ≤ 1 µM | 1 µM to 10 µM |
|---|---|---|
| Inhibition of GP2D Cells (IC₅₀) | A105, A108, A111, A112, A116, A121, A122, A123, A124, A128, A131, A135, A138, A143, A146, A152, A153, A170, A177, A178, A181, A183, A184, A186, A188, A189, A194, A196, A206, A215, A216, A219, A220, A223, A224, A234, A238, A239, A242, A245, A246, A249, A253, A255, A258, A260, A265, A270, A274, A278, A280, A281, A289, A295, A306 | A101, A102, A104, A106, A107, A110, A117, A119, A126, A127, A129, A130, A132, A133, A134, A136, A137, A140, A141, A144, A145, A148, A149, A151, A154, A156, A157, A158, A162, A165, A168, A171, A174, A176, A179, A180, A185, A187, A190, A191, A193, A195, A197, A199, A201, A202, A203, A204, A207, A209, A210, A211, A212, A218, A221, A222, A226, A227, A229, A230, A233, A235, A243, A244, A247, A250, |
| | | A251, A252, A256, A257, A264, A266, A268, A269, A271, A272, A273, A275, A277, A279, A283, A285, A286, A287, A288, A291, A294, A296, A298, A300, A303, A304, A305 |

### c. Growth of cells with K-Ras G12S mutation

A549 (ATCC CRL-185) and LS123 (ATCC CRL-255) cell lines comprise a G12S mutation and can be used to assess Ras cellular signaling in vitro, e.g., in response to the compounds herein. A549 culture medium is prepared with RPMI-1640 and 10% heat-inactivated FBS. LS123 culture medium is prepared with RPMI-1640 and 10% heat-inactivated FBS. A CellTiter-Glo (CTG) luminescent based assay (Promega) is used to assess growth of the cells, as a measurement of the ability of the compounds herein to inhibit Ras signaling in the cells. The cells (e.g., 800 per well) are seeded in their respective culture medium in standard tissue culture-treated 384-well format plates (Falcon #08-772-116) or ultra-low attachment surface 384-well format plates (S-Bio # MS-9384WZ). The day after plating, cells are treated with a dilution series (e.g., a 10 point 3-fold dilution series) of the compounds herein (e.g., approximately 40 µl final volume per well). Cell viability can be monitored (e.g., approximately 6 days later) according to the manufacturer's recommended instructions, where the CellTiter-Glo reagent is added (e.g., approximately 10 µl), vigorously mixed, covered, and placed on a plate shaker (e.g., approximately for 20 min) to ensure sufficient cell lysis prior to assessment of luminescent signal. The IC50 values are determined using the four parameter fit. The resulting IC50 value is a measurement of the ability of the compounds herein to reduce cell growth of Ras-driven cells (e.g., tumor cell lines) in vitro and/or in vivo. The ability of one or more compounds exemplified in Table 1 to inhibit growth of one or more cell lines comprising a given Kras mutation is demonstrated utilizing the procedures described above. One or more compounds disclosed herein selectively inhibits growth of cells with a K-Ras G12S mutation compared to cells with a K-Ras G12D mutation. For example, compounds A102, A110, A128, A136, A152, A183, A184, A199, A210, A218, A219, A251, A277, A298, A304, and A305 selectively inhibit growth of K-Ras G12S mutant cells as evidenced by an IC50 against a K-Ras G12D cell line such as GP2D of greater than 1-fold, 2-fold, 3-fold, 4-fold, 5-fold, 10-fold, 15-fold, or even higher relative to an IC50 against a K-Ras G12S cell line such as A549 (i.e., the ratio of IC50 against a G12D cell line to IC50 against a G12S cell line is greater than 1, 2, 3, 4, 5, 10, 15 or even greater).

Table 7 shows the resulting IC50 values of exemplary compounds against A549 cells using the cell proliferation assays described herein. Compound numbers correspond to the numbers and structures provided in Table 3 and Example 1.

**Table 7**

| | ≤ 500 nM | 500 nM to 5 µM |
|---|---|---|
| Inhibition of A549 Cells (IC₅₀) | A102, A105, A106, A108, A110, A116, A128, A129, A132, A133, A137, A143, A148, A152, A162, A178, A180, A181, A183, A184, A185, A189, A195, A196, A199, A210, A215, A216, A218, A219, A251, A256, A258, A260, A275, A277, A283, A285, A295, A304, A305 | A111, A119, A122, A123, A134, A136, A141, A146, A153, A158, A170, A174, A177, A186, A190, A191, A194, A223, A224, A233, A242, A245, A246, A253, A255, A257, A271, A289, A298, A306 |

One problem in the art is that reversible compounds substantially lack the ability to bind to the serine residue at position 12 of K-Ras G12S mutant and inhibit K-Ras G12S signaling. The present disclosure provides warheads that are particularly suited for reacting with the serine residue at position 12 of K-Ras G12S mutant as a solution to this problem. Table 8 below summarizes sets of direct comparative data, assessed according to the cell proliferation inhibition assays described herein, that demonstrate the advantages of a suitable warhead for targeting K-Ras G12S covalently.

**Table 8**

| Comparative | Compound A Compound B Compound | IC₅₀ Ratio IC₅₀ (Cpd A): IC₅₀(Cpd B) |
|---|---|---|
| 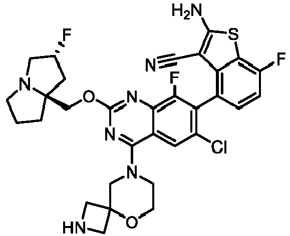 | 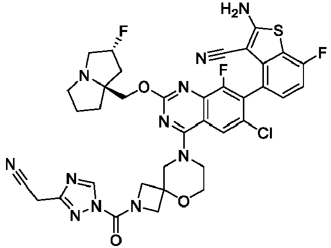 Compound No. A110 disclosed herein | >4 (A549 cells) |
| 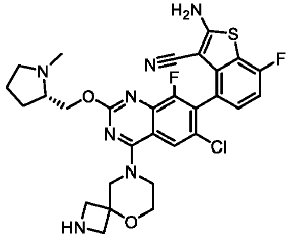 | 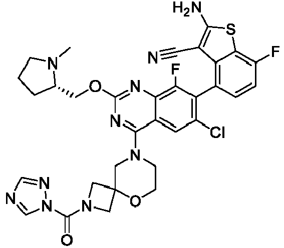 Compound No. A137 disclosed herein | >4 (A549 cells) |
| 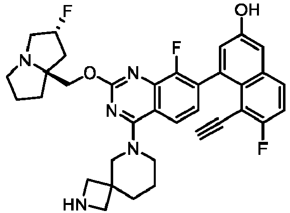 | 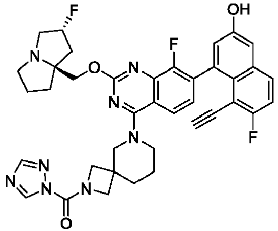 Compound No. A210 disclosed herein | >4 (LS123 cells) |
| 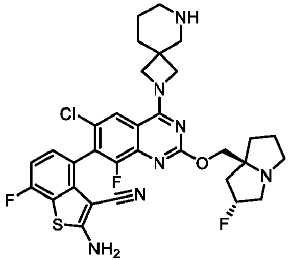 | 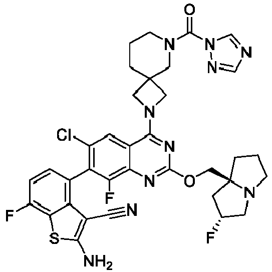 Compound No. A266 disclosed herein | >4 (LS123 cells) |
| 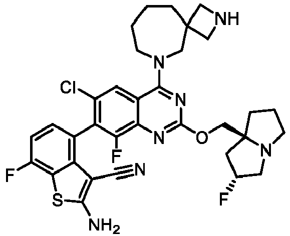 | 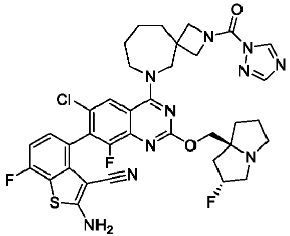 Compound No. A295 disclosed herein | >4 (LS123 cells) |

The above direct comparison, wherein compound pairs differ only at R⁶, clearly shows that a subject compound having a warhead described herein is capable of inhibiting proliferation of cells that comprise a G12S mutation, such as A549 and LS123 cells, much more potently than comparative compounds that lack the warhead. In particular, the exemplified compounds including compounds listed in Table 8 show greater than 1-fold, 3-fold, 4-fold, 5-fold, 10-fold, 50-fold, or even more than 100-fold increase in potency against K-Ras G12S mutant cell line, as ascertained in the cell proliferation inhibition assay disclosed herein. Additional disclosure and data provided herein demonstrate that such covalent engagement enhances K-Ras G12S inhibition potency *(see* Table 7), engagement (*see* Table 5), and selectivity (*see* Table 4), ascertained either by biochemical or cellular proliferation inhibition assays.

### EXAMPLE 10: In vivo Ras inhibition

The in vivo reduction in Ras signaling output by a compound of the present disclosure is determined in a mouse tumor xenograft model, utilizing a Kras-driven tumor cell line. Of particular interest is a K-Ras G12D model or K-Ras G12S model utilizing cells including a K-Ras G12D mutant or K-Ras G12S mutant to generate a xenograft model comparable to the xenograft model exemplified below.

### Xenograft with K-Ras G12D mutation

In another example, tumor xenografts are established by administration of tumor cells with K-Ras G12D mutation (e.g., ASPC-1 cells) or K-Ras G12S mutation (e.g., A549 cells) into mice. Female 6- to 8-week-old athymic BALB/c nude (NCr) nu/nu mice are used for xenografts. The tumor cells (e.g., approximately 5×10⁶) are harvested on the day of use and injected in growth-factor-reduced Matrigel/PBS (e.g., 50% final concentration in 100 µl). One flank is inoculated subcutaneously per mouse. Mice are monitored daily, weighed twice weekly, and caliper measurements begin when tumors become visible. For efficacy studies, animals are randomly assigned to treatment groups by an algorithm that assigns animals to groups to achieve best case distributions of mean tumor size with lowest possible standard deviation. Tumor volume can be calculated by measuring two perpendicular diameters using the following formula: (L × w²) / 2 in which L and w refer to the length and width tumor diameter, respectively. Percent tumor volume change can be calculated using the following formula: (V_{final} -Vᵢₙᵢₜᵢₐₗ)Nᵢₙᵢₜᵢₐₗ × 100. Percent of tumor growth inhibition (%TGI) can be calculated using the following formula: %TGI = 100 × (1 - (average V_{final} -Vᵢₙᵢₜᵢₐₗ of treatment group) / (average V_{final} -Vᵢₙᵢₜᵢₐₗ of control group). When tumors reach a threshold average size (e.g., approximately 200-400 mm³). mice are randomized into 3-10 mice per group and are treated with vehicle (e.g., 100% Labrasol^{®}) or a subject compound disclosed herein using, for example, a daily schedule by oral gavage. Results can be expressed as mean and standard deviation of the mean.

### EXAMPLE 11: Metabolic (Microsomal) Stability Assay

The metabolic stability of a test compound is assayed at 37 °C using pooled liver microsomes (mouse or human liver microsomes). An aliquot of 10 µL of 50 µM test compound is mixed with 490 µL of 0.611 mg/mL liver microsomes, then 50 µL of the mixtures are dispensed to the 96 well tubes and warmed at 37 °C for 10 minutes. The reactions are initiated by adding 50 µL of the pre-warmed NADPH regeneration system solution (add 1.2 µL solution, 240 µl solution B, mix with 10.56 ml KPBS) and then incubated at 37 °C. The final incubation solution contains 100 mM potassium phosphate (pH 7.4), 1.3 mM NADP+, 3.3 mM glucose 6-phosphate, 0.4 Unit/mL of glucose 6-phosphate dehydrogenase, 3.3 mM magnesium chloride, 0.3 mg/mL liver microsomes and 0.5 µM test article. After 0, 15, 30 and 60 minutes in a shaking incubator, the reactions are terminated by adding 100 µL of acetonitrile containing 200 nM buspirone as an internal standard. All incubations are conducted in duplicate. Plates are vortexed vigorously by using Fisher Scientific microplate vortex mixer (Henry Troemner, US). Samples are then centrifuged at 3500 rpm for 10 minutes (4 °C) using Sorvall Legend XRT Centrifuge (Thermo Scientific, GE). Supernatants (40 µL) are transferred into clean 96-deep well plates. Each well is added with 160 µL of ultrapure water (Milli-Q, Millipore Corporation) with 0.1% (v/v) formic acid (Fisher Chemical), mixed thoroughly and subjected to LC/MS/MS analysis in MRM positive ionization mode.

All the samples are measured using a mass spectrometer (QTrap 5500 quadrupole/ion trap) coupled with a Shimadzu HPLC system. The HPLC system consisted of a Shimadzu series degasser, binary quaternary gradient pumps, column heater coupled to an autosampler, and a Phenomenex Gemini-NX, C18, 3.0 µm or Phenomenex Lunar, C8, 5.0 µM HPLC column (Phenomenex, Torrance, CA), and eluted with a mobile phase gradient consisting of Solution A (0.1% formic acid water) and Solution B (0.1% formic acid acetonitrile). The column temperature is maintained at 40 °C. All the analytes are detected with positive-mode electrospray ionization (ES+).

The half-life for the metabolic degradation of the test compound is calculated by plotting the time-course disappearance of the test compound during the incubation with liver microsomes. Each plot is fitted to a first-order equation for the elimination of the test compound (% remaining compound) versus time using non-linear regression (Equation 1). $\frac{{C}_{t}}{{C}_{0}} = {e}^{- kt}$

where Cₜ is the mean relative substrate concentration at time t and C₀ is the initial concentration (0.5 µM) at time 0. Note that the area ratio of the substrate peak to an internal standard peak is proportional to the analyte concentration and is used for regression analysis to derive a value of k.

The half-life t_{1/2} for metabolic (microsome) stability is derived from the test compound elimination constant k using Equation 2 below. ${t}_{1 / 2} = \frac{0.693}{k}$

### EXAMPLE 12: CYP2C19 Inhibition Assay

Some xenobiotics can inhibit cytochrome P450 (CYP) enzyme function, which alters their ability to metabolize drugs. Administration of a CYP inhibitor with a drug whose clearance is dependent on CYP metabolism can result in increased plasma concentrations of this concomitant drug, leading to potential toxicity. The inhibition of CYP2C19 by a test compound is assayed in human liver microsomes using S-Mephenytoin as a CYP2C19 substrate. The stock solution of the test compound or known CYP2C19 inhibitor as a positive control (10 mM) is diluted with KPBS to 40 µM. In a similar way, the stock solutions of the human liver microsomes and S-Mephenytoin are diluted with KPBS buffer. The pre-incubations are started by incubating a plate containing 25 µL human liver microsomes (final concentration of 0.2 mg/mL), 25 µL NADPH-generating system, and a 25 µL test compound (final concentration 10 µM) or the positive control for 30 min at 37±1 °C. After the pre-incubation, 25 µL S-Mephenytoin (final concentration 200 µM) is added and incubated another 12 minutes at 37±1 °C for substrate metabolism. The reactions are terminated by addition of 100 µL of ice-cold acetonitrile containing an internal standard (buspirone). Precipitated proteins are removed by centrifugation at 3500rpm for 10 minutes at 4 °C (Allegra 25R, Beckman Co. Fullerton, CA) and then aliquot of the supernatant is transferred to an assay plate.

All the samples are assessed using a mass spectrometer (QTrap 5500 quadrupole/ion trap) coupled with a Shimadzu HPLC system, following the manufacturer's instructions. The metabolism of S-Mephenytoin in human liver microsomes is monitored by LC/MS/MS as representative of CYP2C19 inhibitory activity. The amount of metabolite formed is assessed by the peak area ratio (metabolite/IS) and % inhibition at 10 µM is expressed as a percentage of the metabolite signal reduced compared to the control (i.e. an incubation that contained no inhibitor and represented 100% enzyme activity): % inhibition = (1-A/B) × 100%, where A is the metabolite peak area ratio formed in the presence of test compound or inhibitor at 10 µM and B is the metabolite peak area ratio formed without test compound or inhibitor in the incubation.

### EXAMPLE 13: Mouse and Human Protein Binding Assay to Assess Free Drug Concentration

This assay can be used to determine the plasma protein binding of the test compound in the plasma of human and animal species using a Rapid Equilibrium Dialysis (RED) device for equilibrium dialysis and LC-MS/MS for sample analysis. Test compound is spiked in. The stock solution of the test compound is prepared at 5 mM concentration. One µL of 5 mM working solution is added into 1000 µL plasma to achieve a final concentration of 5 µM. The spiked plasma is placed on a rocker, and gently agitated for approximately 20 minutes. A volume of 300 µL of the plasma sample containing 5 µM test compound from each species is added to designate RED device donor chambers followed by addition of 500 µL of potassium phosphate buffer to the corresponding receiver chambers in duplicate. The RED device is then sealed with sealing tape and shaken at 150 RPM for 4 hours at 37 °C. Post-dialysis donor and receiver compartment samples are prepared for LC-MS/MS analysis, including spiking samples with an internal standard for the bioanalytical analysis. Warfarin and propranolol are purchased from Sigma-Aldrich (St. Louis, MO), and used as positive controls for low and high plasma protein binding, respectively.

All the samples are analyzed using an Agilent Technologies 6430 Triple Quad LC/MS system. The HPLC system consists of an Agilent 1290 Infinity Liquid Chromatograph coupled to an autosampler (Agilent 1290 Infinity LC Injector HTC), and a Phenomenex Gemini-NX, C18, 3.0 µm or Phenomenex Lunar, C8, 5.0 µM HPLC column (Phenomenex, Torrance, CA), eluting with a mobile phase gradient consisting of Solution A (0.1% formic acid water) and Solution B (0.1% formic acid acetonitrile). The column temperature is maintained at 40 °C. All the analytes are detected with positive-mode electrospray ionization (ES+). The percentage of the test compound bound to plasma is calculated following Equation 3 and 4. $% Free test compound = \frac{Peak ratio \left(\frac{test compound}{Internal standard}\right) , receiver compartment}{Peak ratio \left(\frac{test compound}{Internal standard}\right) , donor compartment} ∗ 100$ $% Plasma protein bound test compound = 100 - % Free test compound$

### EXAMPLE 14: bERG (automated patch-clamp) Assay

The human ether-a-go-go related gene (hERG) encodes the voltage gated potassium channel in the heart (IKr) which is involved in cardiac repolarization. Inhibition of the hERG causes QT interval prolongation and can lead to potential fatal events in humans. It is thus important to assess hERG inhibition early in drug discovery. A hERG automated patch-clamp assay is done using a hERG CHO-K1 cell line using an incubation time of 5 min. The degree of hERG inhibition (%) is obtained by measuring the tail current amplitude, which is induced by a one second test pulse to - 40 mV after a two second pulse to + 20 mV, before and after drug incubation (the difference current is normalized to control and multiplied by 100 to obtain the percent of inhibition). The percent hERG inhibition is measured in the presence of 10 µM test compound.

### EXAMPLE 15: Rat Oral Exposure (%F)

A pharmacokinetic profile for a test compound is measured by single dosing in jugular vein cannulated male Sprague-Dawley rats. Animal weights are typically over 200 grams, and animals are allowed to acclimate to their new environment for at least 3 days prior to the initiation of any studies. One set of animals is dosed intravenously (IV) with test compound (2 mg/kg in 20% HP-beta-CD or 20% Captisol, pH adjusted to ~4 by citric acid). The IV dosing solution concentration is 0.4 mg/mL test compound. Blood is sampled at 5 minutes, 15 minutes, 30 minutes, 90 minutes, 360 minutes, and 24 hours following IV dosing. Another set of animals is dosed oral (po) with test compound (10 mg/kg in 20% HP-beta-CD or 20% Captisol, pH adjusted to ~4 by citric acid). The oral dosing solution concentration is 1 mg/mL test compound. Blood is sampled at 15 minutes, 30 minutes, 90 minutes, 180 minutes, 360 minutes and 24 hours following oral (po) dosing. Blood samples (-0.2 mL/ sample) is collected via the jugular vein, placed in tubes containing EDTA-K2 and stored on ice until centrifuged. The blood samples are centrifuged at approximately 6800g for 6 minutes at 2-8 °C and the resulting plasma is separated and stored frozen at approximately -80 °C.

The plasma samples are analyzed using an Agilent Technologies 6430 Triple Quad LC/MS system, following the manufacturer's instructions. The analytes are detected with positive-mode electrospray ionization (ES+). A standard curve for each test compound is generated and used to measure test compound concentrations in the rat plasma samples. Based on the time course sampling, an area under the curve is calculated for the oral dose group and the intravenous dose group. Percentage rat bioavailability is calculated based on equation 5. $% F \left(rat\right) = \frac{{AUC}_{po} ∗ {Dose}_{IV}}{{AUC}_{IV} ∗ {Dose}_{po}} ,$ where F is bioavailability, AUCₚₒ is area under curve of oral drug, AUC_{IV} is area under curve of intravenous drug, Dose_{IV} is the intravenous dose and Doseₚₒ is the oral dose.

In some embodiments, a compound disclosed herein, such as a compound of Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), exhibits selective and potent inhibition of K-Ras G12S relative to wildtype K-Ras or other K-Ras mutants (e.g., K-Ras G12V or K-Ras G12D). In some embodiments, a compound disclosed herein exhibits selective and potent inhibition of K-Ras G12S relative to wildtype K-Ras or other K-Ras mutants (e.g., K-Ras G12V or K-Ras G12D), such as a compound of Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), wherein R⁵ is an optionally substituted 5-6 membered nitrogen containing heteroaryl. In some embodiments, a compound disclosed herein exhibits selective and potent inhibition of K-Ras G12S relative to wildtype K-Ras or other K-Ras mutants (e.g., K-Ras G12V or K-Ras G12D), such as a compound of Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), wherein R⁵ is a triazole optionally substituted with a methyl, -CH₂-CN, or halogen (e.g., Cl), a pyrazole optionally substituted with CF₃ or CN, an imidazole optionally substituted with CN, a pyrrole optionally substituted with CF₃, or pyrimidine. In some embodiments, a compound disclosed herein exhibits selective and potent inhibition of K-Ras G12S relative to wildtype K-Ras or other K-Ras mutants (e.g., K-Ras G12V or K-Ras G12D), such as a compound of Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), or (I-1n), wherein R⁵ is a triazole optionally substituted with a methyl, -CH₂-CN, or halogen (e.g., Cl); a pyrazole optionally substituted with CF₃ or CN; an imidazole optionally substituted with CN; a pyrrole optionally substituted with CF₃ or pyrimidine; R² is 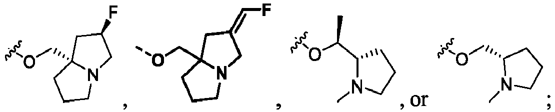 and R⁷ is 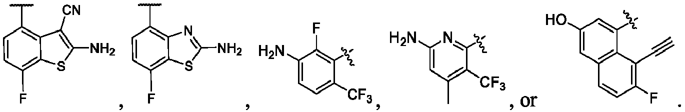 In some embodiments, the K-Ras G12S selective and on-target inhibition is attributed to (1) the warhead (e.g., R⁶ of Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi) disclosed herein) being capable of or susceptible to reacting with a serine residue that corresponds to position 12 of SEQ ID No. 1; and/or (2) the geometry of the linker (e.g., R¹⁰ of Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi) disclosed herein) that is coupled to the warhead. A linker disclosed herein may orient the warhead to specifically favor reacting with the serine residue at position 12 of K-Ras G12S mutant.

In some embodiments, a subject warhead exhibits selective engagement of K-Rras G12S relative to K-Ras G12D or wildtype K-Ras by at least 1-fold, and in some instances greater than 2-, 3-, 4-, 5-, 10-, 15-, or 20-fold, or even higher. In some embodiments, a subject warhead exhibits a selective and rapid engagement of K-Rras G12S yielding at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99%, or even higher engagement of G12S within, 10 mins, 20 mins, 30 mins, 1 hr, 2 hrs, 3 hrs, 4 hrs, 5 hrs, 6 hrs, 7 hrs, 8 hrs, 9 hrs, 10 hrs, 20 hrs, or 24 hours. In some embodiments, a selective and rapid engagement of K-Rras G12S is evidenced by at least 50% engagement within 24 hours. In some embodiments, subject compounds specifically engage K-Ras G12S covalently with essentially no detectable labeling of K-Ras G12D when assayed under comparable conditions. Such specific K-Ras G12S inhibition is observed in many of the subject compounds including but not limited to A102, A106, A110, A119, A125, A128, A133, A134, A137, A148, A154, A161, A162, A165, A169, A182, A185, A194, A195, A197, A199, A202, A209, A210, A213, A219, A221, A230, A243, A255, A256, A257, A258, A265, A266, A269, A275, A279, A280, A283, A285, A295, A296, A298, A302, and A304.

The inclusion of a warhead of the present disclosure may enhance the efficacy or potency of K-Ras G12S inhibition. In some embodiments, a subject compound comprising a subject warhead inhibits K-Ras G12S with higher potency as evidenced by an IC50 value that is at least 10%, 20%, 50%, 100%, 200%, 300%, 400%, or at least 500% lower than the IC50 value of a corresponding control compound that does not comprise the warhead. In some embodiments, a subject compound comprising a subject warhead inhibits K-Ras G12S with higher potency as evidenced by an IC50 value that is at least 1.1-times, 1.2-times, 1.5-times, 2-times, 3-times, 4-times, 5-times, 6-times, 7-times, 8-times, 9-times, 10-times, 15-times, or at least 20-times lower than the IC50 value of a corresponding control compound that does not comprise the warhead, as ascertained in a biochemical assay exemplified in Example 5.

The inclusion of a warhead of the present disclosure may enhance the efficacy or potency with which a subject compound inhibits the proliferation of cells that express a K-Ras G12S mutation. In some embodiments, a subject compound comprising a subject warhead inhibits the proliferation of cells that express a K-Ras G12S mutation with higher potency as evidenced by an IC50 value that is at least 10%, 20%, 50%, 100%, 200%, 300%, 400%, or at least 500% lower than the IC50 value of a corresponding control compound that does not comprise the warhead. In some embodiments, a subject compound comprising a subject warhead inhibits the proliferation of cells that express a K-Ras G12S mutation with higher potency as evidenced by an IC50 value that is at least 1.1-times, 1.2-times, 1.5-times, 2-times, 3-times, 4-times, 5-times, 6-times, 7-times, 8-times, 9-times, 10-times, 15-times, or at least 20-times lower than the IC50 value of a corresponding control compound that does not comprise the warhead, as ascertained in a cellular inhibition assay exemplified in Example 9.

Besides the cellular proliferation inhibitory effect and high potency in reducing K-Ras signaling, particularly signaling mediated by K-Ras G12S mutant, compounds disclosed herein exhibit advantageous ADME and/or DMPK properties. Fine-tuned pharmacological properties are of great significance for improving efficacy and safety of K-Ras inhibitors for therapeutic clinical applications.

In some embodiments, a compound of the present disclosure, such as a compound of Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), exhibits at least one, two, three or more advantageous pharmacological properties. Exemplary superior DMPK properties may include but are not limited to improved metabolic stability, reduced hERG liability, decreased CYP inhibition, increased oral exposure, and decreased serum protein binding (hence increasing the amount of free and available compound circulating in a subject's blood following administration of the compound). In some embodiments, at least one, two, three or more advantageous pharmacological properties are observed in a subject compound having Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), wherein R⁵ is an optionally substituted 5-6 membered nitrogen containing heteroaryl. In some embodiments, at least one, two, three or more advantageous pharmacological properties are observed in a subject compound having the Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), wherein R⁵ is a triazole optionally substituted with a methyl, -CH₂-CN, or halogen (e.g., Cl), a pyrazole optionally substituted with CF₃ or CN, an imidazole optionally substituted with CN, a pyrrole optionally substituted with CF₃, or pyrimidine. In some embodiments, at least one, two, three or more advantageous pharmacological properties are observed in a subject compound having the Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), or (I-1n), wherein R⁵ is a triazole optionally substituted with a methyl, -CH₂-CN, or halogen (e.g., Cl), a pyrazole optionally substituted with CF₃ or CN, an imidazole optionally substituted with CN, a pyrrole optionally substituted with CF₃, or pyrimidine, wherein R² is 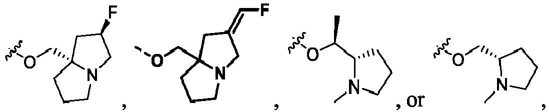 and wherein R⁷ is

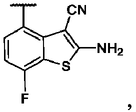

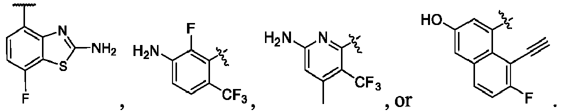

In some embodiments, a compound of the present disclosure, such as a compound of Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), exhibits suitable microsomal stability. In some embodiments, suitable microsomal stability is observed in a subject compound having Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), wherein R⁵ is an optionally substituted 5-6 membered nitrogen containing heteroaryl. In some embodiments, suitable microsomal stability is observed in a subject compound having the Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), (I-1n), (IIIa), (IIIb), (IIIc), (IIId), (IIIe), (IIIf), (IIIg), (IIIh), or (IIIi), wherein R⁵ is a triazole optionally substituted with a methyl, -CH₂-CN, or halogen (e.g., Cl), a pyrazole optionally substituted with CF₃ or CN, an imidazole optionally substituted with CN, a pyrrole optionally substituted with CF₃, or pyrimidine. In some embodiments, suitable microsomal stability is observed in a subject compound having the Formula (Ib), (Id), (Ie), (Ib-1), (Ib-2), (Ib-3), (Ib-4), (Id-1), (Id-3), (Ie-1), (Ie-3), (I-1), (I-2), (I-1a), (I-1b), (I-1d), (I-1e), (I-1f), (I-1g), (I-1h), (I-1i), (I-1k), (I-1m), or (I-1n), wherein R⁵ is a triazole optionally substituted with a methyl, -CH₂-CN, or halogen (e.g., Cl), a pyrazole optionally substituted with CF₃ or CN, an imidazole optionally substituted with CN, a pyrrole optionally substituted with CF₃, or pyrimidine, wherein R² is 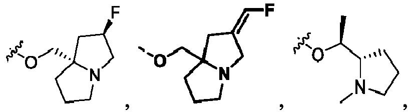 or 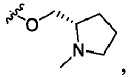 and wherein R⁷ is

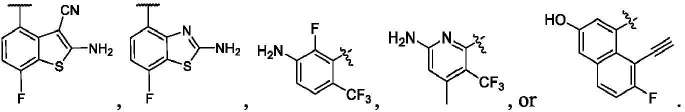

In some embodiments, a subject compound exhibits suitable metabolic stability as ascertained by a T1/2 of mouse liver microsomal metabolism greater than 10 mins, 20 mins, 30 mins, 40 mins, 50 mins, 60 mins or longer as (see Example 11 for experimental procedures). In some embodiments, a subject compound exhibits suitable metabolic stability as ascertained by a T1/2 of human liver microsomal metabolism greater than 10 mins, 20 mins, 30 mins, 40 mins, 50 mins, 60 mins, 100 mins, 120 mins or longer as (see Example 11 for experimental procedures). In yet some other embodiments, a T1/2 of at least 10 mins, 20 mins, 30 mins, 40 mins, 50 mins, 60 mins or longer is observed in both mouse and human microsomal metabolism assays. One or more compounds disclosed herein - including compounds A102, A105, A106, A110, A111, A122, A129, A131, A132, A136, A139, A140, A143, A145, A146, A148, A163, A165, A174, A178, A181, A182, A194, A201, A206, A213, A215, A216, A219, A220, A221, A224, A226, A234, A244, A249, A253, A256, A260, A266, A268, A271, A273, A277, A278, A281, A284, A288, and A296- exhibit a suitable microsomal stability with a T1/2 greater than 10 mins, 20 mins, 30 mins, 40 mins, 50 mins, 60 mins or longer in mouse and/or human liver microsomal metabolism assays.

The following numbered paragraphs set out preferred features and preferred combinations of features of the present invention:
1. A modified Ras mutant protein comprising a compound covalently bonded to its serine residue, wherein the serine residue corresponds to position 12 of SEQ ID No: 1, and wherein the modified Ras mutant protein exhibits a reduced Ras signaling output.
2. The modified Ras mutant protein of para 1 comprising an amino acid sequence selected from the group consisting of SEQ ID No. 1, SEQ ID No. 3, SEQ ID No. 5, and a respective fragment thereof comprising the serine residue corresponding to position 12 of SEQ ID No: 1.
3. The modified protein of any one of the preceding paras, wherein the reduced Ras signaling output is evidenced by one or more output selected from the group consisting of (i) an increase in steady state level of GDP-bound modified protein; (ii) a reduction of phosphorylated AKTs473, (iii) a reduction of phosphorylated ERK T202/Y204, (iv) a reduction of phosphorylated S6 S235/236, (v) reduction of cell growth of a tumor cell expressing a Ras G12S mutant protein, and (vi) reduction in Ras interaction with a Ras-pathway signaling protein.
4. The modified protein of any one of the preceding paras, comprising an amino acid sequence of SEQ ID No. 1 (K-Ras G12S) or a fragment thereof comprising the serine residue corresponding to position of SEQ ID No. 1.
5. The modified protein of any of the preceding paras that is formed by contacting a precursor compound with the serine residue of an unmodified Ras G12S mutant protein, wherein the precursor compound comprises a moiety susceptible to reacting with a serine residue corresponding to position 12 of SEQ ID No: 1.
6. The modified protein of any of the preceding paras that is formed by contacting a precursor compound with the serine residue of an unmodified Ras G12S mutant protein, wherein the precursor compound comprises a staying group and a leaving group, and upon contacting the precursor compound with an unmodified Ras G12S mutant protein, said leaving group separates from remainder of the precursor compound resulting in breaking a covalent bond between the leaving group and the remainder of the precursor compound.
7. The modified protein of any of the preceding paras that is formed by contacting a precursor compound with the serine residue of an unmodified Ras G12S mutant protein, wherein the precursor compound comprises a staying group and a leaving group, and wherein said contacting results in release of the leaving group and formation of said modified protein.
8. The modified protein of any one of paras 5 to 7, wherein the modified protein comprises an amino acid sequence of SEQ ID No. 1 or a fragment thereof that comprises the serine residue corresponding to position 12 of SEQ ID No. 1, and wherein the precursor compound selectively labels the serine residue as compared to (i) an aspartate residue of a K-Ras G12D mutant protein, said aspartate corresponding to residue 12 of SEQ ID NO: 7, (ii) a valine residue of a K-Ras G12V mutant protein, said valine corresponding to residue 12 of SEQ ID NO: 8, and/or (iii) a glycine residue corresponding to position 12 of SEQ ID No. 2 (a K-Ras wildtype protein).
9. The modified protein of any one of paras 5 to 7, wherein the the precursor compound exhibits the selective labeling by at least 2 folds when assayed under comparable conditions.
10. The modified protein of any one of paras 5 to 7, wherein the precursor compound exhibits the selective labeling by at least 5 folds when assayed under comparable conditions.
11. The modified protein of any one of paras 5-10, wherein contacting the serine residue of an unmodified Ras G12S protein corresponding to position 12 of SEQ ID No: 1 occurs in vitro.
12. The modified protein of any one of paras 5-10, wherein contacting the serine residue of an unmodified Ras G12S protein corresponding to position 12 of SEQ ID No: 1 occurs in vivo.
13. The modified protein of any one of paras 1 to 12, wherein the covalently bonded compound comprises a staying group having a formula: 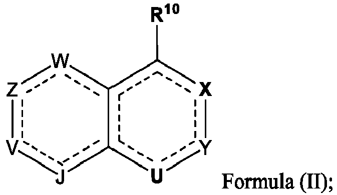 Wherein
   W is a bond, N, C(R¹⁸), N(R^{18b}), C(R¹⁸)(R^{18a}), C(O), S(O), or S(O)₂;
   Z is N, C(R⁸), N(R^{8b}), C(R⁸)(R^{8a}), C(O), S(O), or S(O)₂;
   V and J are selected from C(R¹⁷), C(R¹⁷)(R^{16a}), C(R¹⁶), C(R¹⁶)(R^{16a}), N, N(R^{17b}), and N(R^{16b});
   Y is N, C(R²), C(R²)(R^{2c}), N(R^{2b}), S(O), S(O)₂, or C(O);
   U is N, C(R^{2c}), C(R^{2c})(R^{2c}), N(R^{2b}), S(O), S(O)₂, or C(O);
   R¹⁰ is -L⁷-R⁷;
   L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
   R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl)- are substituted with one R⁶ directly bonded to a ring N and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, Ci-nheterocycloalkyl, C₆₋₁₂aryl, or C₁₋₁₁heteroaryl, wherein the C₃₋₁₂cycloalkyl, Ci-nheterocycloalkyl, C₆₋₁₂aryl, or C₁₋₁₁heteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
   R⁶ is -C(O)-; wherein R⁶ is directly bonded to the serine residue corresponding to position 12 of SEQ ID No: 1;
   R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R¹⁷ is selected from -L¹-R¹⁹, hydrogen, halogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R^{17b} is selected from -L^{1b}-R¹⁹, hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   L' is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
   R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R² is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
   R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C_{1- 9}heteroaryl are optionally substituted with one, two, or three R^{20d};
   R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C_{3- 6}cycloalkyl, C₂₋₉heterocycloalkyl, C₆₋₁₀aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
   R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
   each R^{21a}, R^{20b}, R^{20c}, R^{20d}, R^{20e}, R^{20f}, R^{20g}, R^{20h}, and R²⁰ⁱ is independently selected from halogen, -CN, C_{1- 6}alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, - N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; wherein C₁₋₆alkyl, C₂₋₆alkenyl, C₂₋₆alkynyl, C_{3- 6}cycloalkyl, -CH₂-C₃₋₆cycloalkyl, C₂₋₉heterocycloalkyl, -CH₂-C₂₋₉heterocycloalkyl, C₆₋₁₀aryl, -CH₂-C_{6- 10}aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   - - - - - - indicates a single or double bond such that all valences are satisfied.
14. The modified protein of any one of paras 6 to 13 wherein the leaving group has a formula:
   A) 5 membered partially unsaturated heterocycloalkyl or a 5 membered heteroaryl, each optionally substituted with one, two or three R^{20k}, wherein the partially unsaturated heterocycloalkyl or heteroaryl comprises two or three ring nitrogen atoms; or
   B) 5 or 6 membered partially unsaturated heterocycloalkyl or a 5 or 6 membered heteroaryl, each optionally substituted with one, two or three R^{20k}, wherein the partially unsaturated heterocycloalkyl or heteroaryl comprises two or three ring nitrogen atoms; and further wherein the partially unsaturated heterocycloalkyl or heteroaryl is substituted with a -NH(R¹⁴) bonded to a ring carbon of the partially unsaturated heterocycloalkyl or heteroaryl;

   each R¹⁴ is independently selected from hydrogen, C₁₋₆alkyl, and C₁₋₆haloalkyl;
   each R^{20k} is independently selected from halogen, -CN, C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and - OC(O)R²⁵; or two R^{20k} bonded to the same or adjacent atoms may optionally be joined to form a C_{3- 6}cycloalkyl, C₂₋₉heterocycloalkyl, C₆₋₁₀aryl, -CH₂-C₆₋₁₀aryl, or C₁₋₉heteroaryl; wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl; and
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl.
15. The modified protein of any one of paras 6 to 13 wherein the leaving group has a formula: 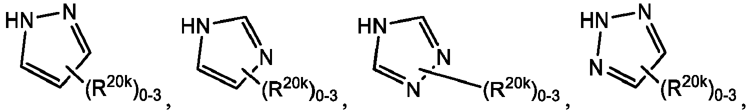 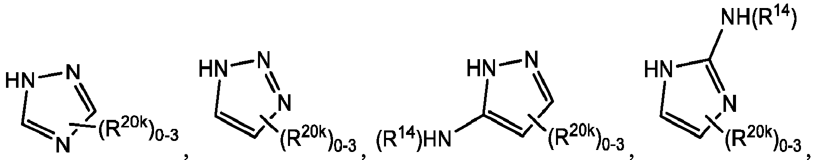 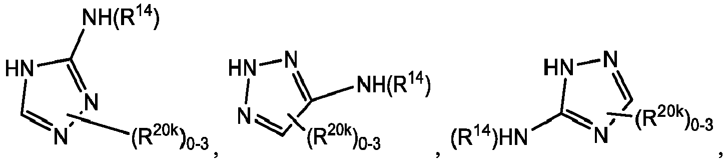 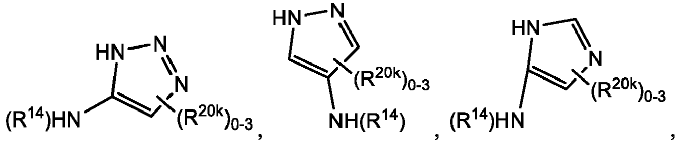 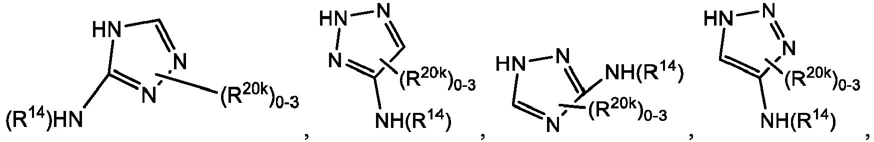 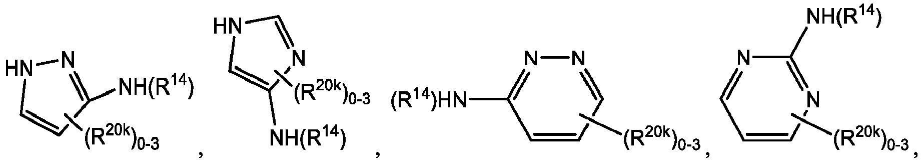 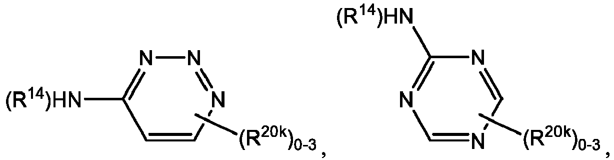 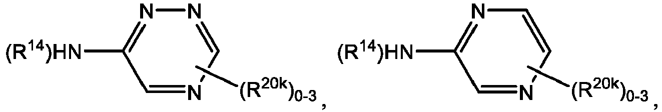 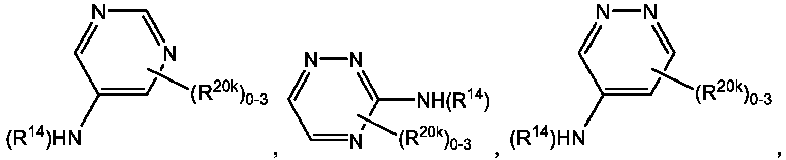 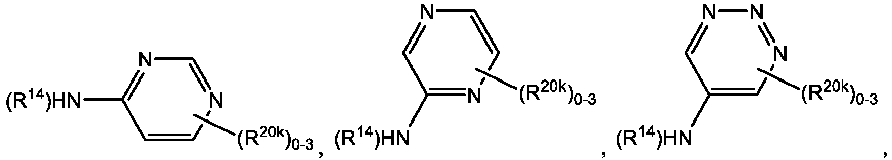 or 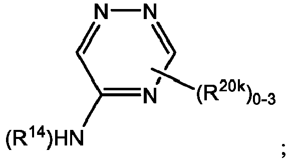
   each R¹⁴ is independently selected from hydrogen, C₁₋₆alkyl, and C₁₋₆haloalkyl;
   each R^{20k} is independently selected from halogen, -CN, C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and - OC(O)R²⁵; or two R^{20k} bonded to the same or adjacent atoms may optionally be joined to form a C_{3- 6}cycloalkyl, C₂₋₉heterocycloalkyl, C₆₋₁₀aryl, -CH₂-C₆₋₁₀aryl, or C₁₋₉heteroaryl; wherein C₁₋₆alkyl, C_{2- 6}alkenyl, C₂₋₆alkynyl, C₃₋₆cycloalkyl, -CH₂-C₃₋₆cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl; and
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl.
16. A precursor compound of Formula (Ib), or a pharmaceutically acceptable salt or solvate thereof 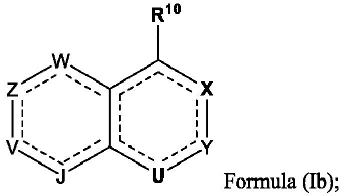 wherein
   W is N, C(R¹⁸), N(R^{18b}), C(R¹⁸)(R^{18a}), C(O), S(O), or S(O)₂;
   Z is N, C(R⁸), N(R^{8b}), C(R⁸)(R^{8a}), C(O), S(O), or S(O)₂; wherein W and Z are not both selected from C(O), S(O), and S(O)₂;
   V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
   Y is N, C(R²), C(R²)(R^{2c}), N(R^{2b}) S(O), S(O)₂, or C(O);
   U is N, C(R^{2c}), C(R^{2c})(R^{2c}), N(R^{2b}), S(O), S(O)₂, or C(O);
   R¹⁰ is -L⁷-R⁷;
   L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
   R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ directly bonded to a ring N and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, Ci-nheterocycloalkyl, C₆₋₁₂aryl, or C₁₋₁₁heteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
   R⁶ is -L²-R⁵;
   each L² is -C(O)-;
   each R⁵ is selected from:
      (i) a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is bonded through an R⁵ ring nitrogen to L²;
   R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R¹⁷ is -L¹-R¹⁹;
   R^{17b} is -L^{1b}-R¹⁹;
   L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
   R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, - (C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
   R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
   each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, - S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   - - - - - - indicates a single or double bond such that all valences are satisfied.
17. A precursor compound of Formula (Id), or a pharmaceutically acceptable salt or solvate thereof: 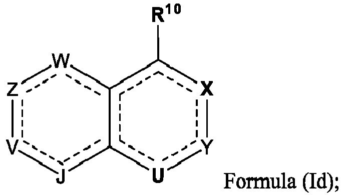 wherein
   W is a bond, N, C(R¹⁸), N(R^{18b}), C(R¹⁸)(R^{18a}), C(O), S(O), or S(O)₂;
   Z is N, C(R⁸), N(R^{8b}), C(R⁸)(R^{8a}), C(O), S(O), or S(O)₂; wherein W and Z are not both selected from C(O), S(O), and S(O)₂;
   V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
   Y is N, C(R²), C(R²)(R^{2c}), N(R^{2b}), S(O), S(O)₂, or C(O);
   U is N, C(R^{2c}), C(R^{2c})(R^{2c}), N(R^{2b}), S(O), S(O)₂, or C(O);
   R¹⁰ is -L⁷-R⁷;
   L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
   R⁷ is a 7-12 membered nitrogen containing spirocyclic heterocycloalkyl substituted with one R⁶ directly bonded to a ring N and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, Ci-nheterocycloalkyl, C₆₋₁₂aryl, or Ci-nheteroaryl, wherein the C₃₋₁₂cycloalkyl, C₁₋₁₁heterocycloalkyl, C₆₋₁₂aryl, or Ci-nheteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a}; R⁶ is -L²-R⁵;
   each L² is -C(O)-;
   each R⁵ is selected from:
      (i) a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is bonded through an R⁵ ring nitrogen to L²;
   R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R¹⁷ is -L¹-R¹⁹;
   R^{17b} is -L^{1b}-R¹⁹;
   L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
   R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, - (C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and-CH₂S(O)_{Z}N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
   R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
   each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³), - N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   - - - - - - indicates a single or double bond such that all valences are satisfied.
18. A precursor compound of Formula (Ie), or a pharmaceutically acceptable salt or solvate thereof: 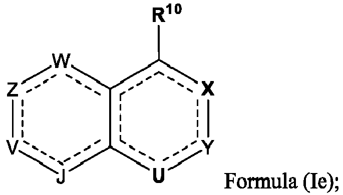 wherein
   W is a bond, N, C(R¹⁸), N(R^{18b}), C(R¹⁸)(R^{18a}), C(O), S(O), or S(O)₂;
   Z is N, C(R⁸), N(R^{8b}), C(R⁸)(R^{8a}), C(O), S(O), or S(O)₂;
   V and J are selected from C(R¹⁷), C(R¹⁷)(R^{16a}), C(R¹⁶), C(R¹⁶)(R^{16a}), N, N(R^{17b}), and N(R^{16b});
   Y is N, C(R²), C(R²)(R^{2c}), N(R^{2b}), S(O), S(O)₂, or C(O);
   U is N, C(R^{2c}), C(R^{2c})(R^{2c}), N(R^{2b}), S(O), S(O)₂, or C(O);
   R¹⁰ is -L⁷-R⁷;
   L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a};
   R⁷ is a 7-12 membered nitrogen containing fused heterocycloalkyl, 7-12 membered nitrogen containing fused aryl, or 7-12 membered nitrogen containing fused heteroaryl substituted with one R⁶ directly bonded to a ring N and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, Ci-nheterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C_{1- 6}haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, - OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
   R⁶ is -L²-R⁵;
   each L² is independently selected from a -C(O)-;
   each R⁵ is selected from:
      (i) a 5-6 membered partially unsaturated heterocycloalkyl comprising one, two, or three ring nitrogen atoms that is optionally substituted with one, two or three R^{20k}, wherein R⁵ is bonded through an R⁵ ring nitrogen to L²; and
      (ii) a 5-6 membered heteroaryl comprising one, two, or three ring nitrogen atoms that is optionally substituted with one, two or three R^{20k}; wherein R⁵ is bonded through an R⁵ ring nitrogen to L²;
   R⁸ and R^{8a} are independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(H)(R¹²), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), _ C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
   R¹⁷ is -L¹-R¹⁹;
   R^{17b} is -L^{1b}-R¹⁹;
   L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, - OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C_{2- 6}alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C_{2- 6}alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₆cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R²⁰ⁱ
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C_{2- 6}alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R20i;
   R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
   R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(O))(=NH)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, - (C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C_{6- 10}aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C_{3- 10}cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, - (C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 6}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl, wherein C₁₋₆alkyl, C₂₋₆alkenyl, C₂₋₆alkynyl, C₃₋₆cycloalkyl, -CH₂-C_{3- 6}cycloalkyl, C₂₋₉heterocycloalkyl, -CH₂-C₂₋₉heterocycloalkyl, C₆₋₁₀aryl, -CH₂-C₆₋₁₀aryl, -CH₂-C_{1- 9}heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
   R^{18b} is selected from hydrogen, -CN, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
   each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, - CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), -OC(O)N(R²²)(R²³),-N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵,-S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), and - OC(O)R²⁵;
   each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₆cycloalkyl, C_{2- 9}heterocycloalkyl, C₆₋₁₀aryl, and C₁₋₉heteroaryl; and
   - - - - - - indicates a single or double bond such that all valences are satisfied.
19. The precursor compound of para 18, wherein exactly one of V and J is substituted with R¹⁷ or R^{17b}.
20. The precursor compound of any one of paras 18 and 19, wherein R⁷ is a 7-12 membered nitrogen containing fused heterocycloalkyl or 7-12 membered nitrogen containing fused heteroaryl substituted with one R⁶ directly bonded to a ring N and optionally substituted with one or more R¹ and/or one or more R⁴.
21. The precursor compound of any one of paras 16-20, R¹⁹ is: 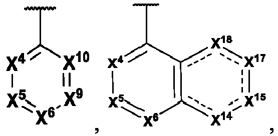 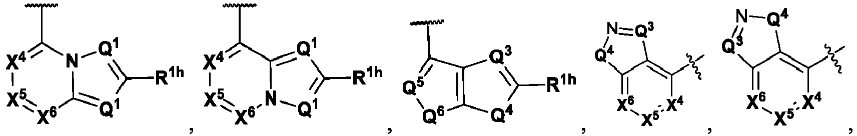 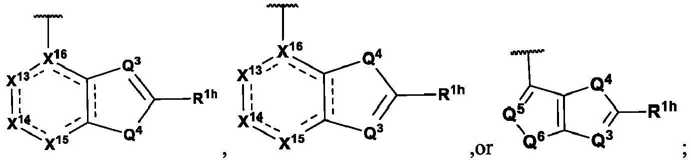
   Q¹, Q³, and Q⁵ are independently selected from N and C(R^{1d});
   Q⁴ and Q⁶ are independently selected from O, S, C(R^{1a})(R^{1b}), and N(R^{1c});
   X⁴, X⁵, X⁶, X⁹, X¹⁰ are independently selected from C(R^{1a}) and N;
   X¹³ is selected from a bond, C(R^{1a}), N, C(O), C(R^{1a})(R^{1b}), C(O)C(R^{1a})(R^{1b}), C(R^{1a})(R^{1b})C(R^{1a})(R^{1b}), C(R^{1a})(R^{1b})N(R^{1c}), and N(R^{1c});
   X¹⁴, X¹⁵, X¹⁷, X¹⁸ are independently selected from a C(O), C(R^{1a}), N, C(R^{1a})(R^{1b}), and N(R^{1c});
   X¹⁶ are independently selected from C, N, and C(R^{1a});
   each R^{1a}, R^{1b}, R^{1d}, and R^{1h} are each independently selected from hydrogen, halogen, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ; or R^{1a} and R^{1b} bonded to the same carbon are joined to form a 3-10 membered heterocycloalkyl ring or a C₃₋₁₀cycloalkyl ring, wherein the 3-10 membered heterocycloalkyl ring or C₃₋₁₀cycloalkyl ring are optionally substituted with one, two, or three R²⁰ⁱ; or two R^{1a} bonded to adjacent atoms are joined to form a 3-10 membered heterocycloalkyl ring, a C₆₋₁₀aryl ring, a 5-12 membered heteroaryl ring, or a C₃₋₁₀cycloalkyl ring, wherein the 3-10 membered heterocycloalkyl ring, C₆₋₁₀aryl ring, 5-12 membered heteroaryl ring, or C₃₋₁₀cycloalkyl ring are optionally substituted with one, two, or three R²⁰ⁱ; or R^{1h} and one of R^{1a}, R^{1b}, R^{1c}, and R^{1d} bonded to adjacent atoms are joined to form a 3-10 membered heterocycloalkyl ring, a C₆₋₁₀aryl ring, a 5-12 membered heteroaryl ring, or a C₃₋₁₀cycloalkyl ring, wherein the 3-10 membered heterocycloalkyl ring, a C₆₋₁₀aryl ring, a 5-12 membered heteroaryl ring, and C_{3- 10}cycloalkyl ring are optionally substituted with one, two, or three R²⁰ⁱ; and
   each R^{1c} is independently selected from hydrogen, C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl are optionally substituted with one, two, or three R²⁰ⁱ.
22. The precursor compound of any one of paras 16 to 21, or a pharmaceutically acceptable salt or solvate thereof, wherein R¹⁹ is selected from: 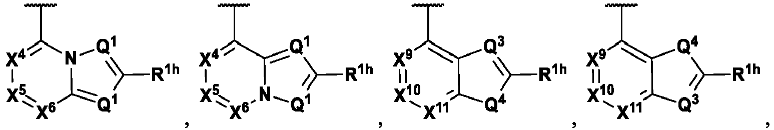 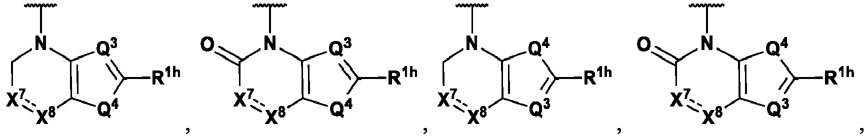 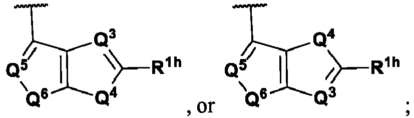
   Q', Q³, and Q⁵ are independently N or C(R^{1d});
   Q⁴ and Q⁶ are independently O, S, C(R^{1a})(R^{1b}), or N(R^{1c});
   X⁴, X⁵, X⁶, X⁹, X¹⁰, and X¹¹ are independently selected from C(R^{1a}) or N;
   X⁷ and X⁸ are independently selected from C(R^{1a}), C(R^{1a})(R^{1b}), N, or N(R^{1c});
   each R^{1a}, R^{1b}, R^{1d}, R^{1f}, R^{1g}, and R^{1h} are independently selected from hydrogen, halogen, -CN, C₁₋₆alkyl, C_{1- 6}haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, - OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C_{1- 6}alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1a} and R^{1b} bonded to the same carbon are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; or two R^{1a} bonded to adjacent atoms are joined to form a 4-7 membered heterocycloalkyl ring, a phenyl ring, a 5-6 membered heteroaryl ring, or a 4-7 membered cycloalkyl ring, wherein the 4-7 membered heterocycloalkyl ring, phenyl ring, 5-6 membered heteroaryl ring, or 4-7 membered cycloalkyl ring are optionally substituted with one, two, or three R²⁰ⁱ; or R^{1h} and one of R^{1a}, R^{1b}, R^{1c}, and R^{1d} bonded to adjacent atoms are joined to form a 4-7 membered heterocycloalkyl ring, a phenyl ring, a 5-6 membered heteroaryl ring, or a 4-7 membered cycloalkyl ring, wherein the 4-7 membered heterocycloalkyl ring, phenyl ring, 5-6 membered heteroaryl ring, or 4-7 membered cycloalkyl ring are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; and
   each R^{1c} is independently selected from hydrogen, C₁₋₆alkyl, C₂₋₆alkenyl, C₂₋₆alkynyl, C₃₋₆cycloalkyl, C_{2- 9}heterocycloalkyl, C₆₋₁₀aryl, C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ.
23. The precursor compound of any one of paras 16 to 21, or a pharmaceutically acceptable salt or solvate thereof, wherein R¹⁹ is selected from: 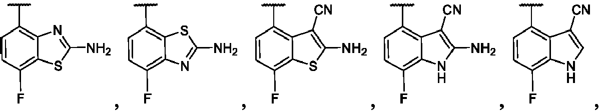 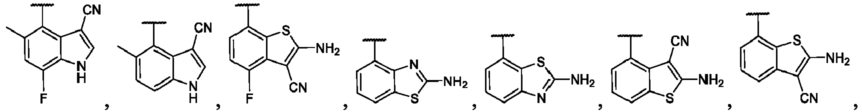 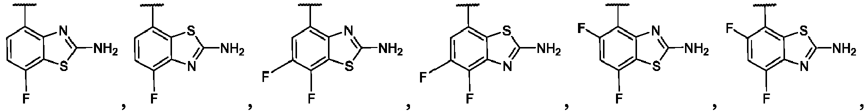 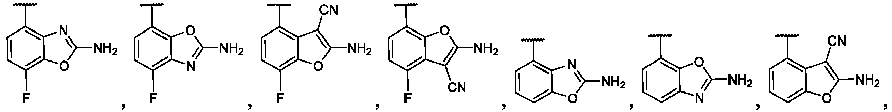 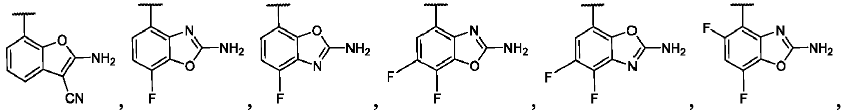 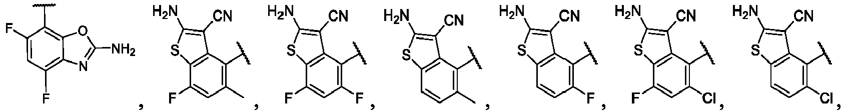 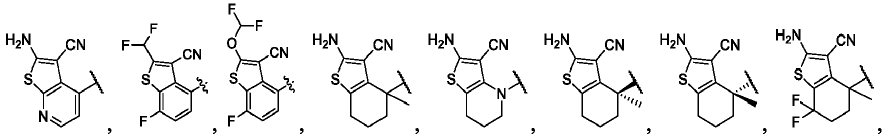 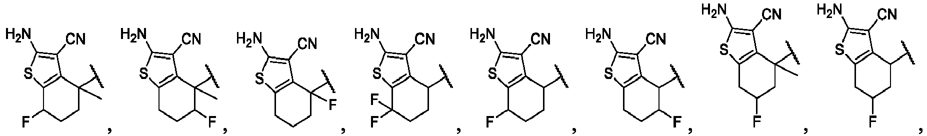 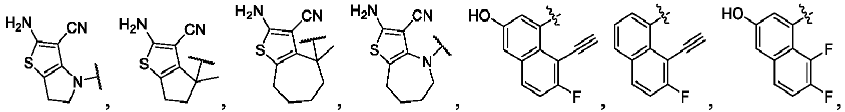 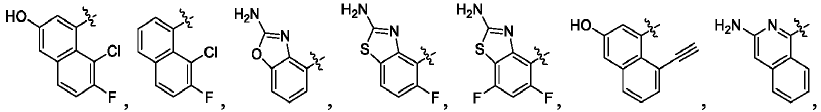 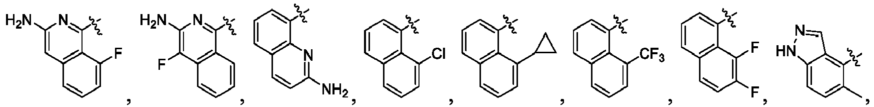 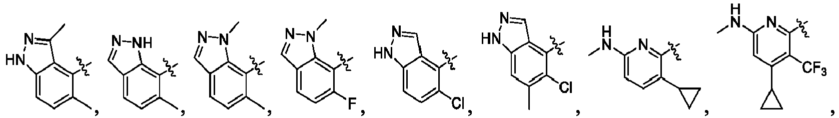 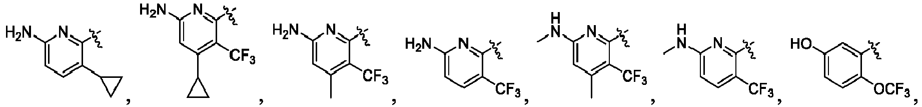 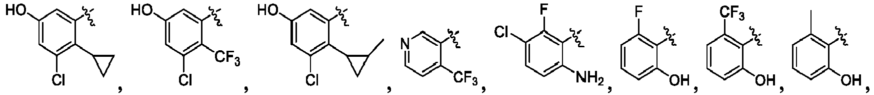 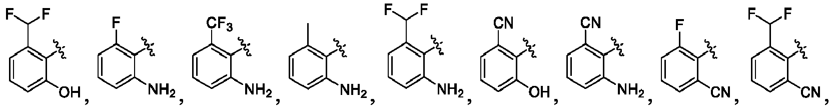 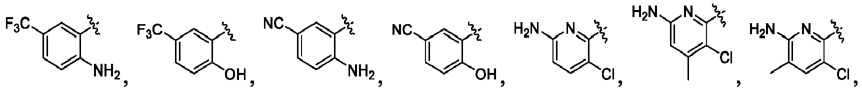 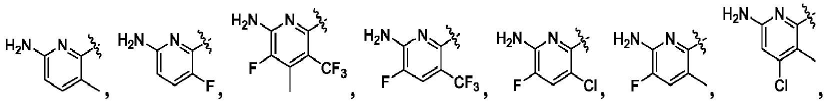 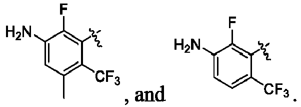
24. The precursor compound of any one of paras 16 to 23, or a pharmaceutically acceptable salt or solvate thereof, wherein the compound is substituted with one or two independently selected R¹.
25. The precursor compound of any one of paras 16 to 23, or a pharmaceutically acceptable salt or solvate thereof, wherein the compound is not substituted with R¹.
26. The precursor compound of any one of paras 16 to 25, or a pharmaceutically acceptable salt or solvate thereof, wherein the compound is substituted with one or two independently selected R⁴.
27. The precursor compound of any one of paras 16 to 25, or a pharmaceutically acceptable salt or solvate thereof, wherein R⁴ is independently unsubstituted C₁₋₆alkyl, C₁₋₆alkyl-CN, or halogen.
28. The precursor compound of any one of paras 16 to 25, or a pharmaceutically acceptable salt or solvate thereof, wherein the compound is not substituted with R⁴.
29. The precursor compound of any one of paras 16 to 28, or a pharmaceutically acceptable salt or solvate thereof, wherein R² is selected from 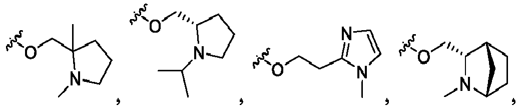 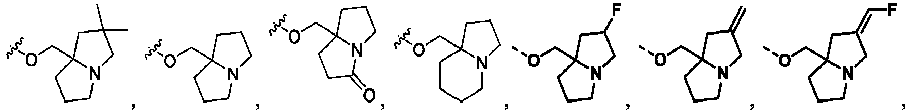 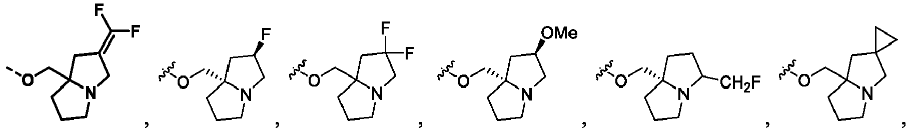 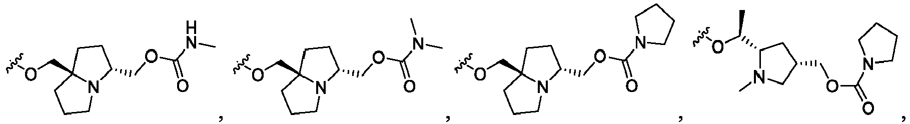 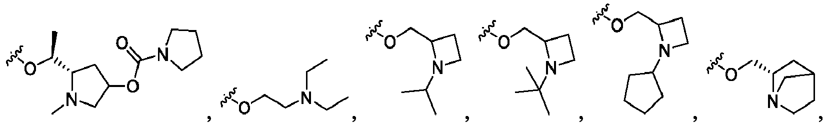 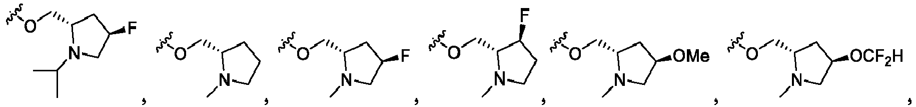 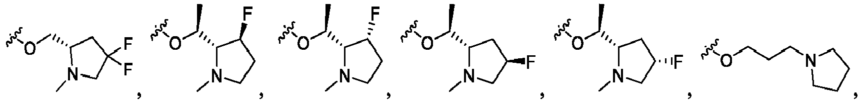 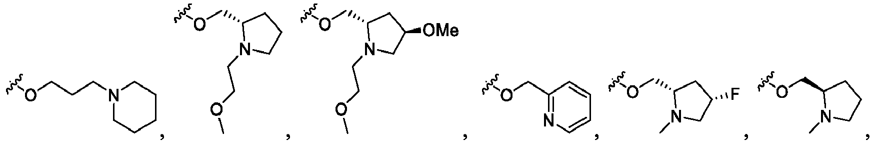 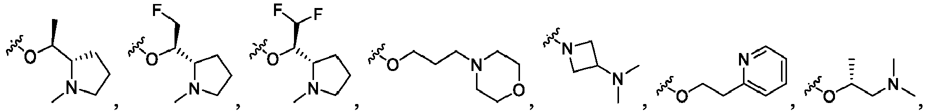 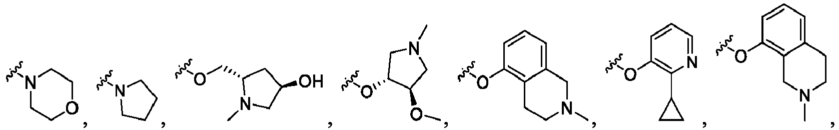 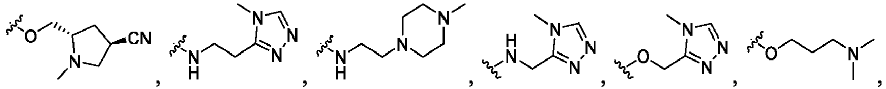 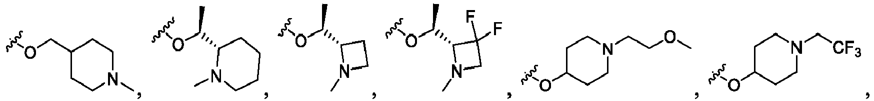 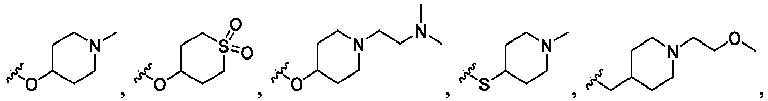 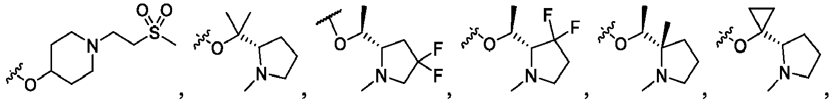 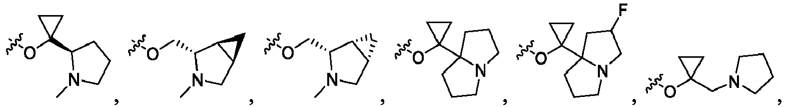 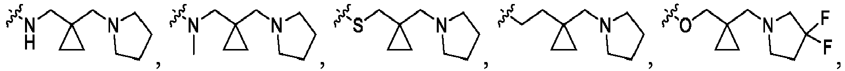 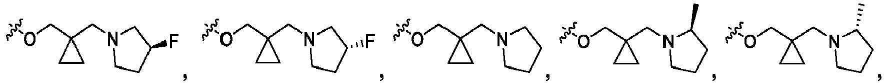 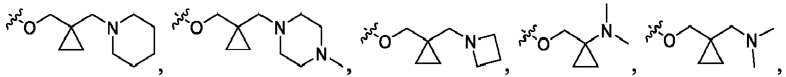 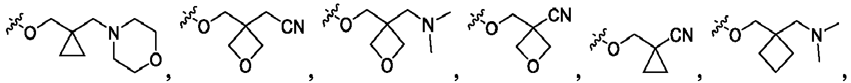 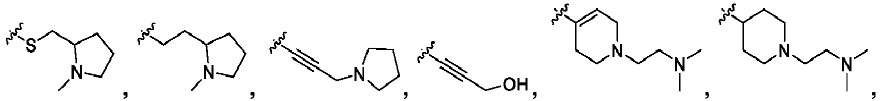 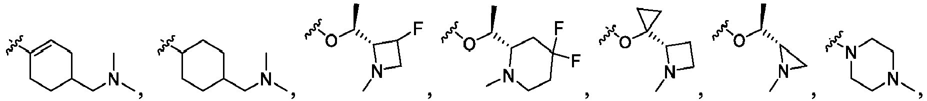 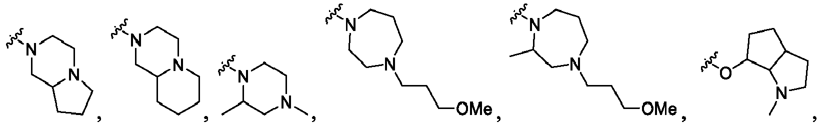 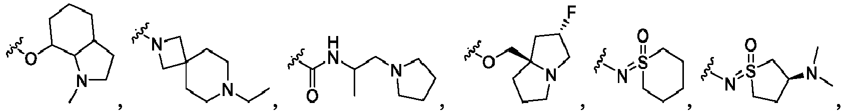 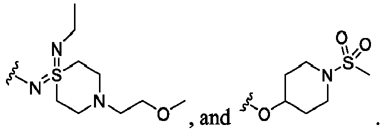
30. The precursor compound of any one of paras 16 to 28, or a pharmaceutically acceptable salt or solvate thereof, wherein R² is selected from 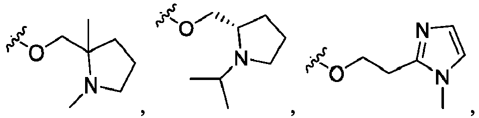 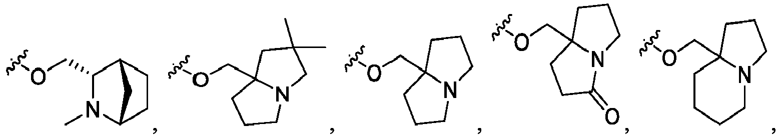 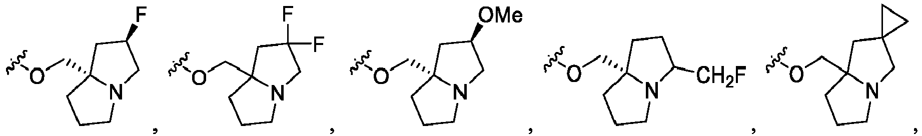 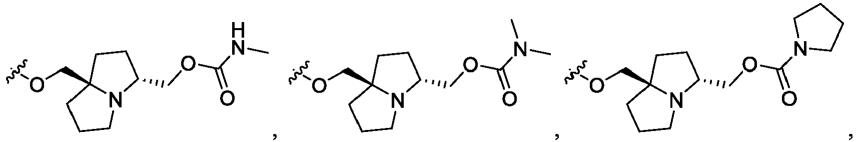 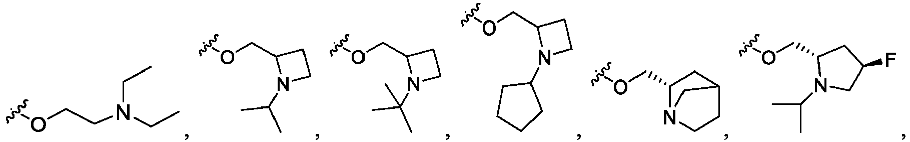 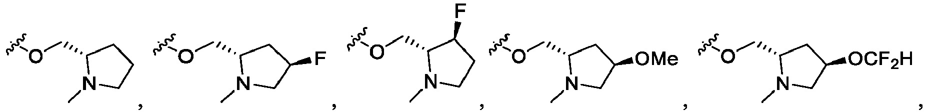 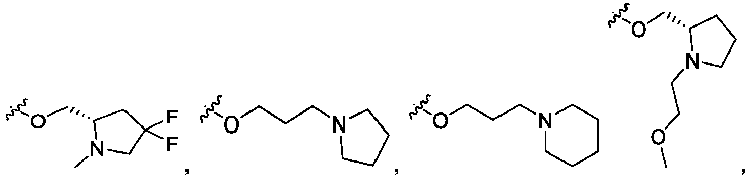 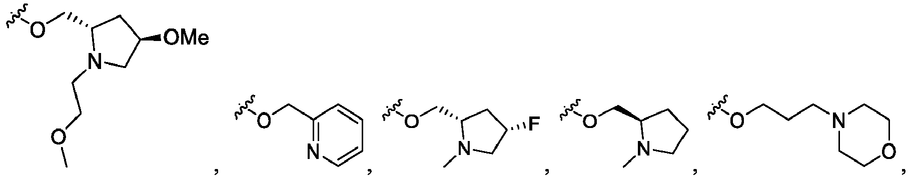 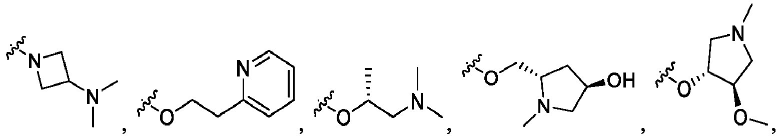 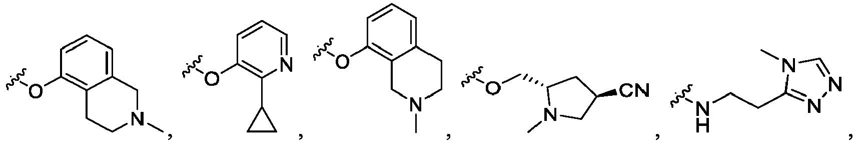 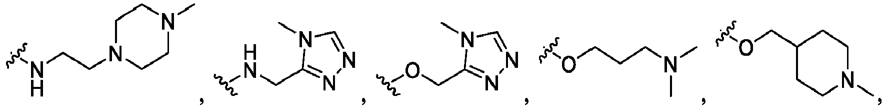 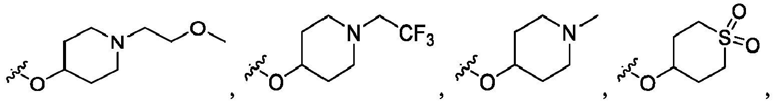 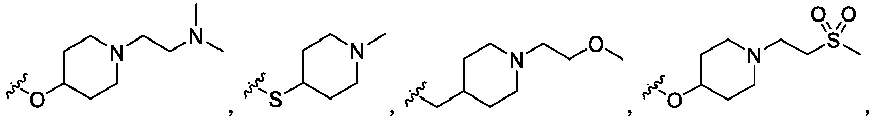 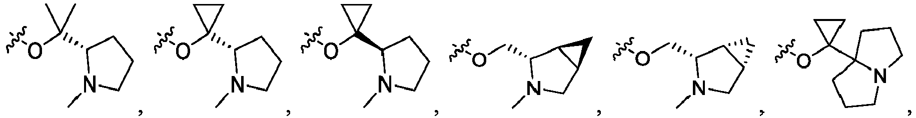 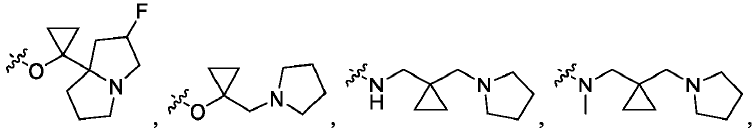 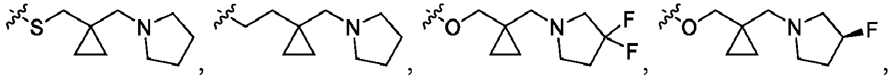 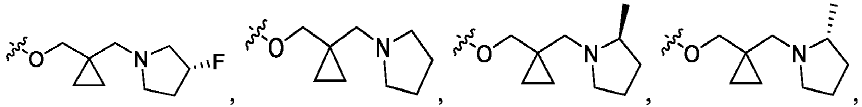 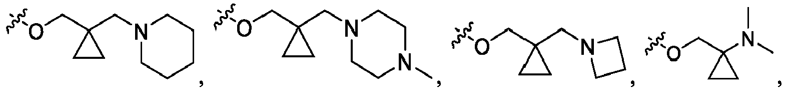 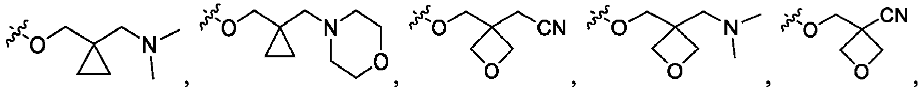 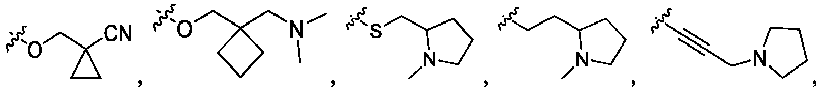 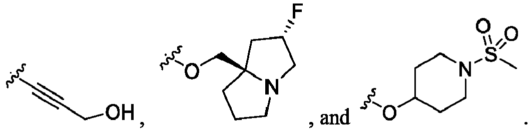
31. The precursor compound of para 16, or a pharmaceutically acceptable salt or solvate thereof, having the formula (Ib-1i): 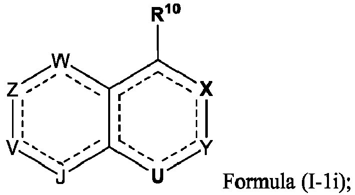 wherein
   L⁷ is a bond, -O-, or -NH-;
   R⁷ is a 6-12 membered nitrogen containing heterocycloalkyl having a formula selected from: 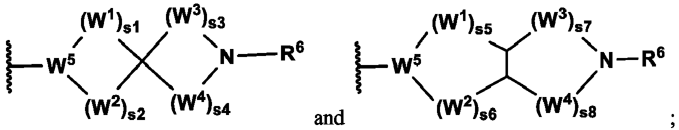
   W¹, W², W³, and W⁴ are independently selected from N(R¹), N(R⁴), C(R¹)(R¹), C(R¹)(R⁴), C(R⁴)(R⁴), C(O), S, O, S(O), and S(O)₂;
   W⁵ is selected from N, C(R¹), and C(R⁴);
   s1 is an integer from 1 to 6; s2 is an integer from 0 to 3; s3 is an integer from 1 to 3; s4 is an integer from 1 to 3;
   s5 is an integer from 0 to 6; s6 is an integer from 0 to 3; s7 is an integer from 0 to 3; s8 is an integer from 0 to 3;
   R⁶ is -L²-R⁵;
   each L² is -C(O)-;
   R⁵ is a 5 membered heteroaryl, optionally substituted with one, two, or three R^{20k}, wherein the 5 membered heteroaryl comprises one, two, or three ring nitrogen atoms and further wherein when R⁵ is directly bonded to a C(O) of L², L² is directly bonded to an N atom of R⁵;
   L¹ is a bond, L^{1b} is a bond; and
   R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ.
32. The precursor compound of para 31, or a pharmaceutically acceptable salt or solvate thereof, wherein W is C(R¹⁸) or C(O); Z is N, C(R⁸), or N(R^{8b}); V is C(R¹⁷); J is C(R¹⁶); Y is C(R²); U is N; and X is N.
33. The precursor compound of para 31, or a pharmaceutically acceptable salt or solvate thereof, wherein W is CH; Z is C(R⁸) or N(R^{8b}); V is C(R¹⁷); J is C(R¹⁶); Y is C(R²); U is N; and X is N.
34. The precursor compound of any one of paras 16 and 31-33, or a pharmaceutically acceptable salt or solvate thereof, wherein R⁷ is selected from 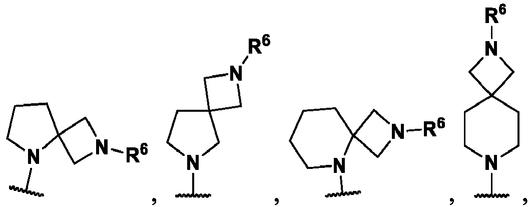 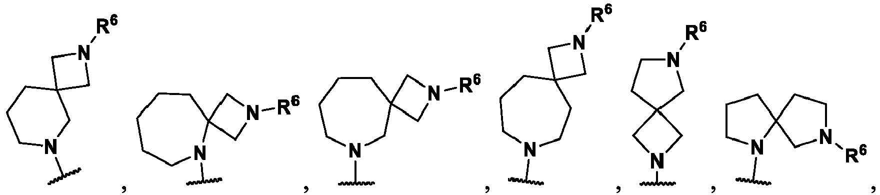 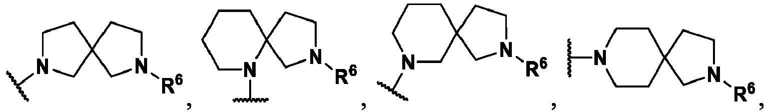 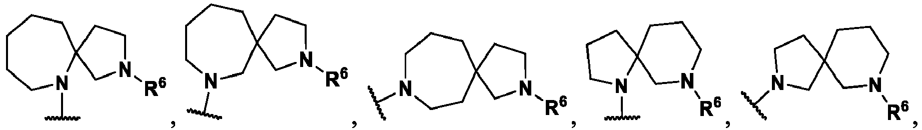 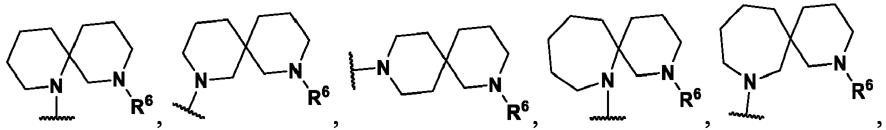 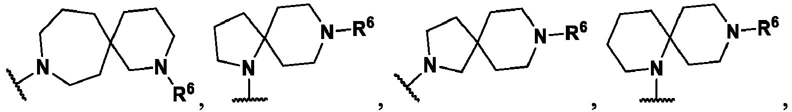 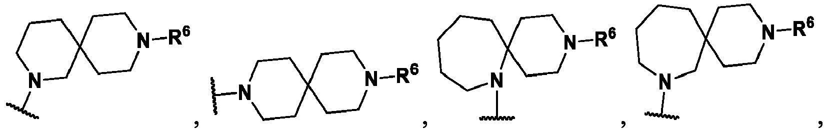 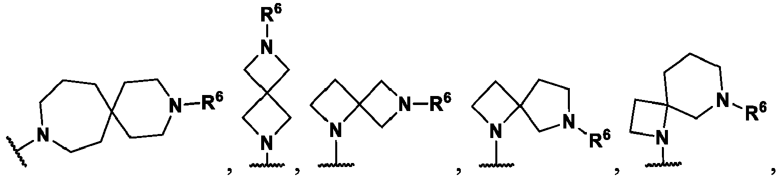 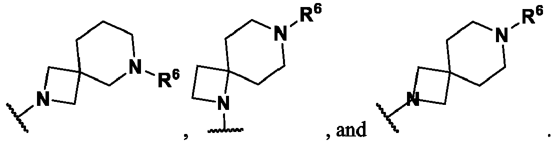
35. The precursor compound of any one of paras 16 and 31-33, or a pharmaceutically acceptable salt or solvate thereof, wherein R⁷ is selected from 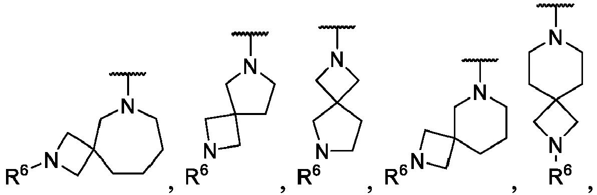 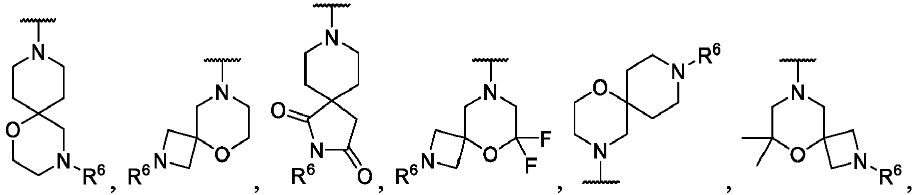 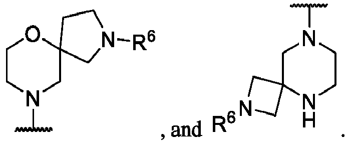
36. The precursor compound of any one of paras 16 and 31-33, or a pharmaceutically acceptable salt or solvate thereof, wherein R⁷ is selected from 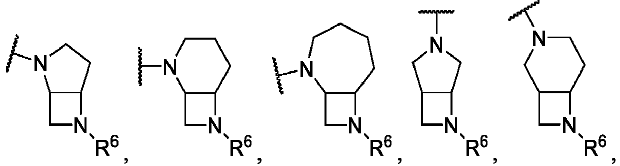 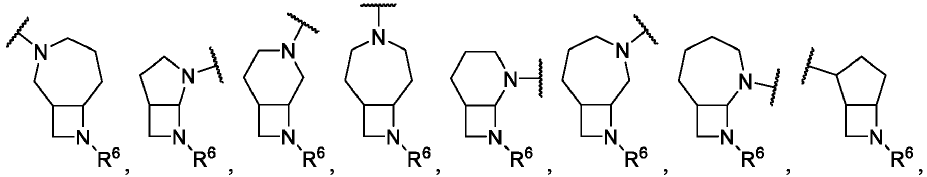 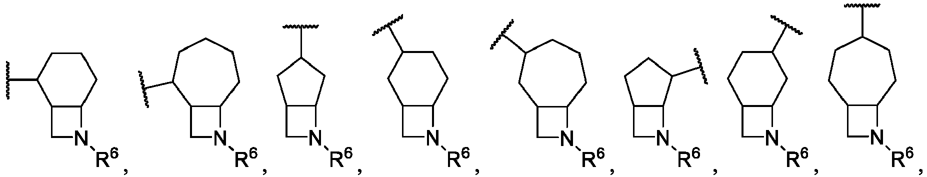 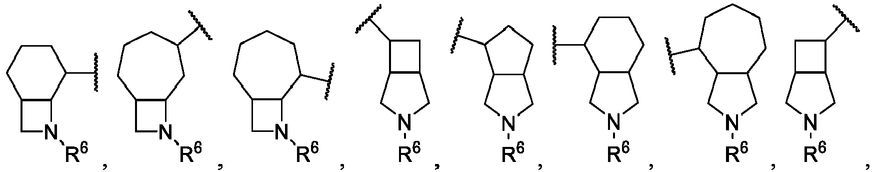 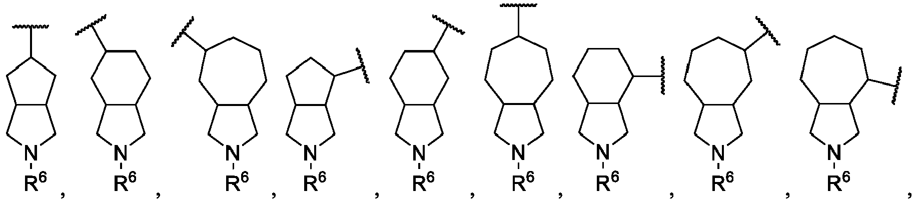 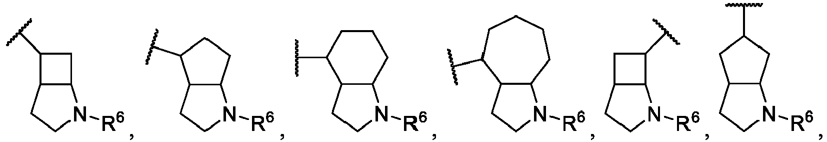 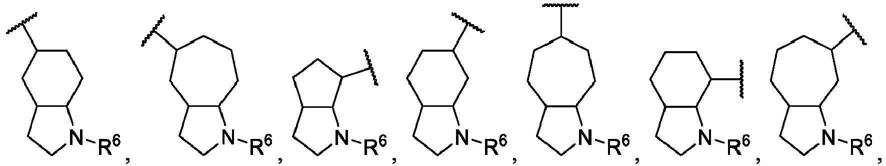 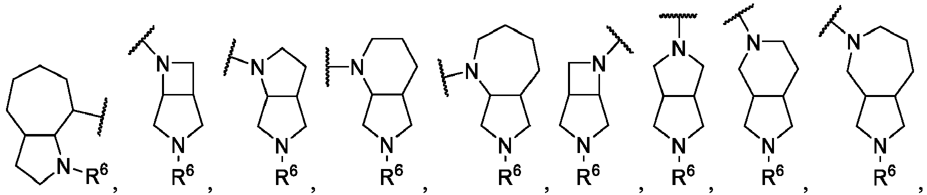 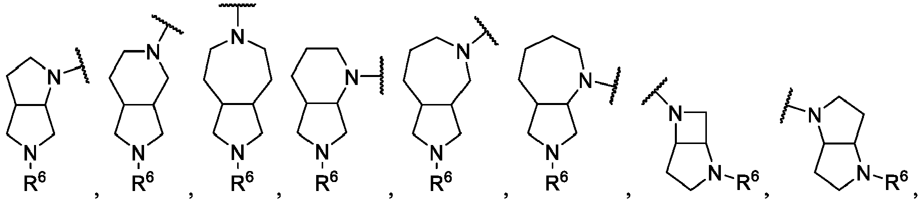 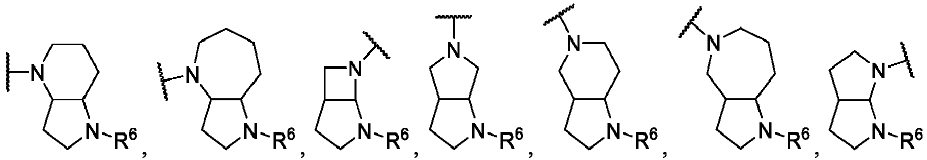 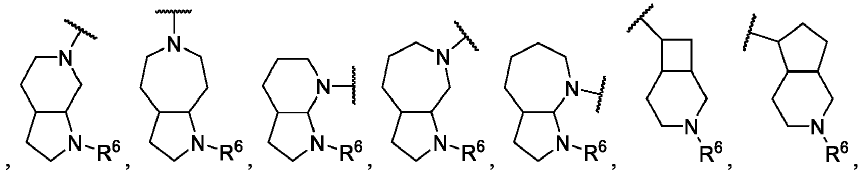 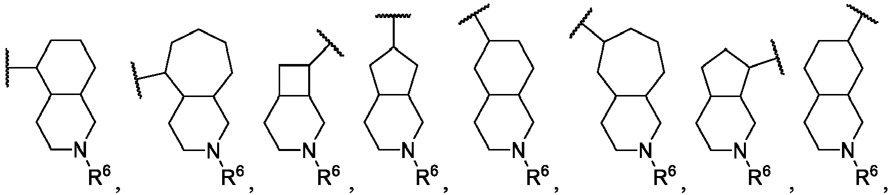 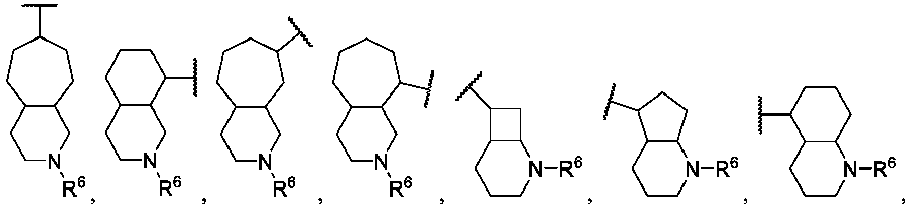 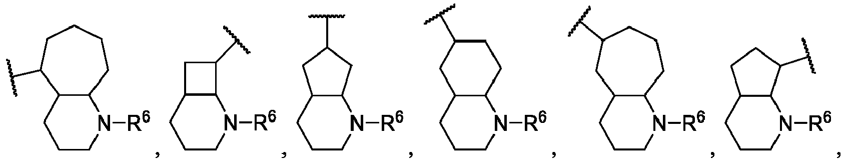 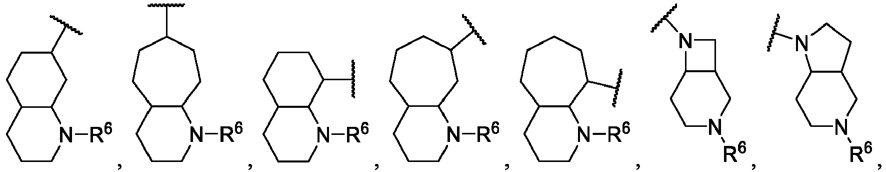 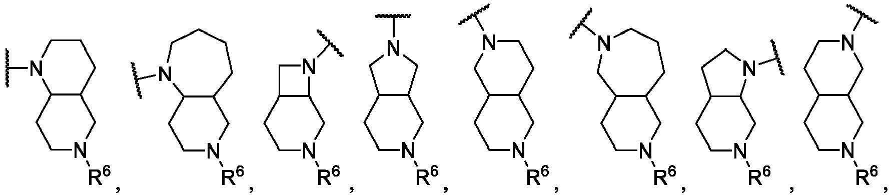 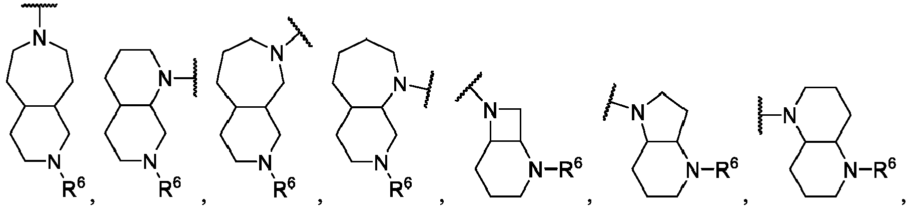 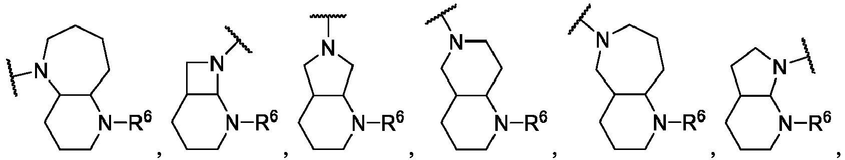 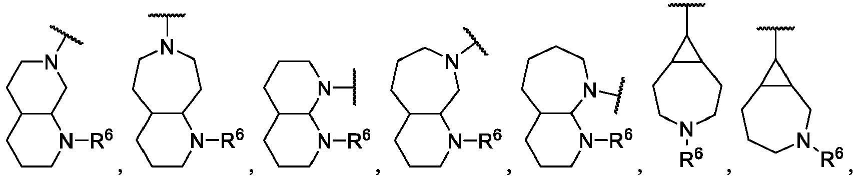 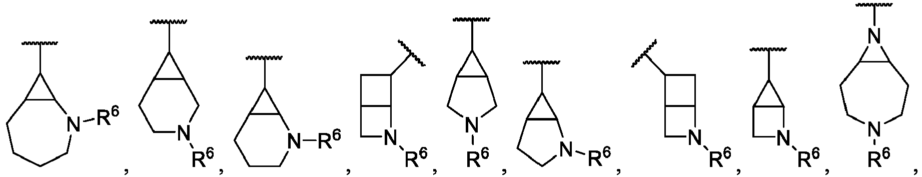 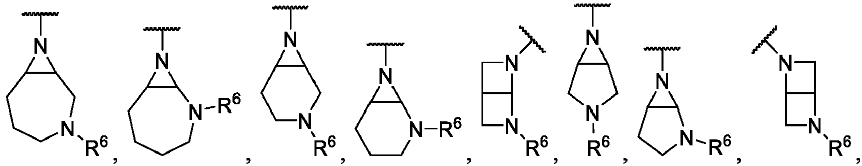 and 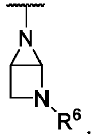
37. The precursor compound of any one of paras 16-36, or a pharmaceutically acceptable salt or solvate thereof, wherein R⁶ is selected from 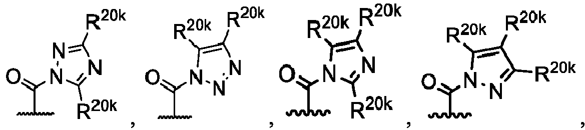 and 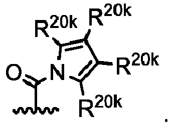
38. The precursor compound of any one of paras 16-36, or a pharmaceutically acceptable salt or solvate thereof, wherein R⁶ is selected from 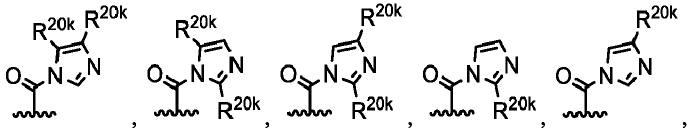 and 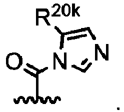
39. The precursor compound of any one of paras 16-36, or a pharmaceutically acceptable salt or solvate thereof, wherein R⁶ is selected from 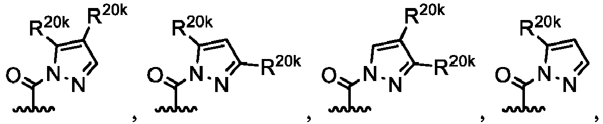 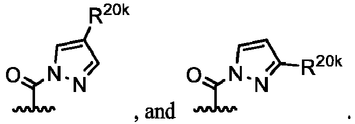
40. The precursor compound of any one of paras 16-36, or a pharmaceutically acceptable salt or solvate thereof, wherein R⁶ is selected from 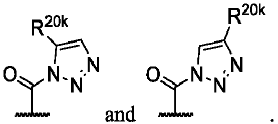
41. The precursor compound of any one of paras 16-36, or a pharmaceutically acceptable salt or solvate thereof, wherein R⁶ is selected from 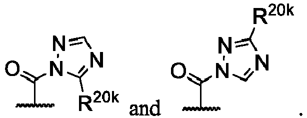
42. The precursor compound of any one of paras 16-36, or a pharmaceutically acceptable salt or solvate thereof, wherein R⁶ is selected from 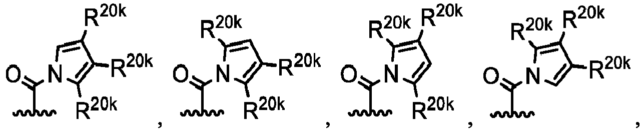 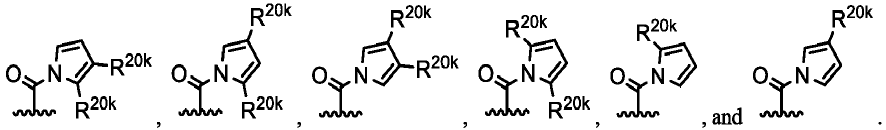
43. The precursor compound of any one of paras 16-42, or a pharmaceutically acceptable salt or solvate thereof, wherein R⁸ is independently selected from hydrogen and halogen.
44. The precursor compound of para 16, or a pharmaceutically acceptable salt or solvate thereof, having the formula (I-1k): 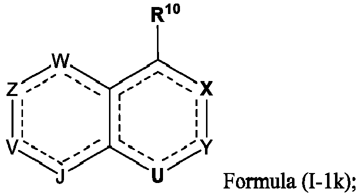 wherein
   W is CH; Z is C(R⁸), N, or N(R^{8b}); V is C(R¹⁷); J is C(R¹⁶) or N; Y is C(R²) or C(O); U is N or N(R^{2b}); X is N;
   R⁸ is hydrogen or halogen;
   R^{8b} is selected from hydrogen, C₁₋₆alkyl, C₃₋₁₀cycloalkyl, or C₂₋₉heterocycloalkyl, wherein C₁₋₆alkyl, C_{3- 10}cycloalkyl, or C₂₋₉heterocycloalkyl are optionally substituted with one, two, or three R^{20c};
   R¹⁶ is hydrogen or halogen;
   R^{2b} is phenyl or pyridyl, wherein said phenyl or pyridyl are optionally substituted with one, two, or three R^{20d};
   L⁷ is a bond, -O-, or -NH-;
   R⁷ is a 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl, wherein the 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl is each substituted with one R⁶, and optionally substituted with one, two, or three R⁴;
   each R⁴ is independently selected from halogen, oxo, -CN, C₁₋₆alkyl, C₂₋₆alkenyl, C₂₋₆alkynyl, C_{3- 6}cycloalkyl, C₂₋₅heterocycloalkyl, -OR¹², and -N(R¹²)(R¹³); wherein C₁₋₆alkyl, C₂₋₆alkenyl, C₂₋₆alkynyl, C₃₋₆cycloalkyl, and C₂₋₅heterocycloalkyl are optionally substituted with one, two, or three R^{20a};
   R⁶ is -L²-R⁵;
   each L² is -C(O)-;
   R⁵ is a 5-6 membered heteroaryl, optionally substituted with one, two, or three R^{20k}, wherein the 5-6 membered heteroaryl comprises one, two, or three ring nitrogen atoms and further wherein when R⁵ is directly bonded to a C(O) of L², L² is directly bonded to an N atom of R⁵;
   L¹ is a bond;
   R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C_{2- 6}alkynyl, -OR¹², -N(R¹²)(R¹³), wherein C₁₋₆alkyl, C₂₋₆alkenyl, and C₂₋₆alkynyl are optionally substituted with one, two, or three R²⁰ⁱ;
   R² is -O-C(R^{12e})₂-C₂₋₉heterocycloalkyl optionally substituted with one, two, or three R^{20d};
   each R^{12e} is independently selected from hydrogen and C₁₋₆alkyl;
   each R¹² is independently selected from hydrogen, C₁₋₆alkyl, C₂₋₆alkenyl, C₂₋₆alkynyl, C₃₋₁₀cycloalkyl, and C₂₋₉heterocycloalkyl, wherein C₁₋₆alkyl, C₂₋₆alkenyl, C₂₋₆alkynyl, C₃₋₁₀cycloalkyl, and C_{2- 9}heterocycloalkyl are optionally substituted with one, two, or three R^{20d};
   each R¹³ is independently selected from hydrogen, C₁₋₆alkyl, and C₁₋₆haloalkyl;
   each R¹⁴ is independently selected from hydrogen, C₁₋₆alkyl, and C₁₋₆haloalkyl;
   each R^{20a}, R^{20c}, R^{20d}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, C₂₋₉heterocycloalkyl, -OR²¹, -SR²¹, -N(R²²)(R²³), -C(O)OR²²,-C(O)N(R²²)(R²³), -N(R²⁴)C(O)R²¹, and -C(O)R²¹, wherein C₁₋₆alkyl, C₂₋₆alkenyl, C₂₋₆alkynyl, C_{3- 10}cycloalkyl, and C₂₋₉heterocycloalkyl, are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵, - S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
   - - - - - - indicates a single or double bond such that all valences are satisfied.
45. The precursor compound of para 44, or a pharmaceutically acceptable salt or solvate thereof, having the formula (I-1m): 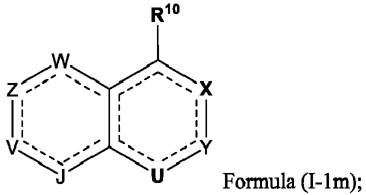 wherein
   W is CH; Z is C(R⁸) or N; V is C(R¹⁷); J is C(R¹⁶); Y is C(R²); U is N; X is N;
   R⁸ is hydrogen or halogen;
   R¹⁶ is hydrogen or halogen;
   L⁷ is a bond, -O-, or -NH-;
   R⁷ is a 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl, wherein the 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl is each substituted with one R⁶, and optionally substituted with one, two, or three R⁴;
   each R⁴ is independently selected from halogen, C₁₋₄alkyl, C₂₋₄alkenyl, C₂₋₄alkynyl, and -OH, wherein C_{1- 4}alkyl, C₂₋₄alkenyl, and C₂₋₄alkynyl are optionally substituted with one, two, or three R^{20a};
   R⁶ is -L²-R⁵;
   each L² is -C(O)-;
   R⁵ is a 5 membered heteroaryl, optionally substituted with one, two, or three R^{20k}, wherein the 5 membered heteroaryl comprises one, two, or three ring nitrogen atoms and further wherein when R⁵ is directly bonded to a C(O) of L², L² is directly bonded to an N atom of R⁵;
   L¹ is a bond;
   R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ;
   each R¹ⁱ is independently selected from halogen, -CN, C₁₋₄alkyl, C₂₋₄alkynyl, -OH, and -NH₂, wherein C_{1- 6}alkyl and C₂₋₄alkynyl are optionally substituted with one, two, or three R²⁰ⁱ;
   R² is -O-C(R^{12e})₂-C₂₋₉heterocycloalkyl optionally substituted with one, two, or three R^{20d};
   each R^{12e} is independently selected from hydrogen and methyl;
   each R¹² is independently selected from hydrogen and C₁₋₆alkyl, wherein C₁₋₆alkyl is optionally substituted with one, two, or three R^{20d};
   each R¹³ is independently selected from hydrogen, C₁₋₆alkyl, and C₁₋₆haloalkyl;
   each R¹⁴ is independently selected from hydrogen, C₁₋₆alkyl, and C₁₋₆haloalkyl;
   each R^{20a}, R^{20c}, R^{20d}, R²⁰ⁱ, and R^{20k} is independently selected from halogen, -CN, oxo, C₁₋₄alkyl, C₂₋₄alkenyl, C₂₋₄alkynyl, C₃₋₆cycloalkyl, C₂₋₅heterocycloalkyl, -OR²¹, -SR²¹, -N(R²²)(R²³), -C(O)OR²², - C(O)N(R²²)(R²³), -N(R²⁴)C(O)R²¹, and -C(O)R²¹, wherein C₁₋₄alkyl, C₂₋₄alkenyl, C₂₋₄alkynyl, C_{3- 6}cycloalkyl, and C₂₋₅heterocycloalkyl,, are optionally substituted with one, two, or three groups independently selected from halogen, oxo, -CN, C₁₋₆alkyl, C₁₋₆haloalkyl, C₁₋₆alkoxy, C₁₋₆haloalkoxy, - OR²¹, -SR²¹, -N(R²²)(R²³), -C(O)OR²², -C(O)N(R²²)(R²³), -C(O)C(O)N(R²²)(R²³), -OC(O)N(R²²)(R²³), -N(R²⁴)C(O)N(R²²)(R²³), -N(R²⁴)C(O)OR²⁵, -N(R²⁴)C(O)R²¹, -N(R²⁴)S(O)₂R²⁵, -C(O)R²¹, -S(O)₂R²⁵,-S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
   = indicates a single or double bond such that all valences are satisfied.\
46. The precursor compound of para 44, or a pharmaceutically acceptable salt or solvate thereof, having the formula (I-1n): 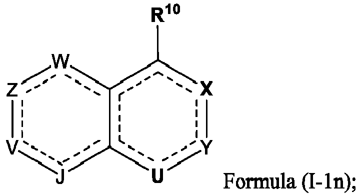 wherein
   W is CH; Z is C(R⁸) or N; V is C(R¹⁷); J is C(R¹⁶); Y is C(R²); U is N; X is N;
   R⁸ is hydrogen or Cl;
   R¹⁶ is F;
   L⁷ is a bond;
   R⁷ is a 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl, wherein the 5-7 membered monocyclic nitrogen containing heterocycloalkyl, 7-12 membered spirocyclic bicyclic nitrogen containing heterocycloalkyl, 7-12 membered fused bicyclic nitrogen containing heterocycloalkyl, or 8-10 membered bridged nitrogen containing heterocycloalkyl is each substituted with one R⁶, and optionally substituted with one, two, or three R⁴;
   each R⁴ is independently selected from halogen, C₁₋₄alkyl, and -OH, wherein C₁₋₄alkyl is optionally substituted with one, two, or three R^{20a};
   R⁶ is -L²-R⁵;
   each L² is -C(O)-;
   R⁵ is a 5 membered heteroaryl, optionally substituted with one, two, or three R^{20k}, wherein the 5 membered heteroaryl comprises one, two, or three ring nitrogen atoms and further wherein when R⁵ is directly bonded to a C(O) of L², L² is directly bonded to an N atom of R⁵;
   L¹ is a bond;
   R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ;
   each R¹ⁱ is independently selected from halogen, -CN, C₁₋₄alkyl, C₂₋₄alkynyl, -OH, and -NH₂, wherein C_{1- 6}alkyl and C₂₋₄alkynyl are optionally substituted with one, two, or three R²⁰ⁱ;
   R² is -O-C(R^{12e})₂-C₂₋₉heterocycloalkyl optionally substituted with one, two, or three R^{20d};
   each R^{12e} is independently selected from hydrogen and methyl;
   each R^{20d} is independently selected from halogen, C₁₋₄alkyl, C₂₋₄alkenyl, and C₂₋₄alkynyl wherein C₁₋₄alkyl, C₂₋₄alkenyl, and C₂₋₄alkynyl are optionally substituted with one, two, or three groups independently selected from halogen, oxo, -OH, and -NH₂;
   each R²⁰ⁱ is independently selected from halogen;
   each R^{20k} is independently selected from halogen, -CN, and C₁₋₄alkyl, wherein C₁₋₄alkyl is optionally substituted with one, two, or three groups independently selected from halogen, -CN, -OH, and -NH₂; and
   = indicates a single or double bond such that all valences are satisfied.
47. The precursor compound of any one of paras 44-46, or a pharmaceutically acceptable salt or solvate thereof, wherein R⁷ is a 7-12 membered spirocyclic bicyclic nitrogen containing heterocycloalkyl or 7-12 membered fused bicyclic nitrogen containing heterocycloalkyl, wherein the 7-12 membered spirocyclic bicyclic nitrogen containing heterocycloalkyl or 7-12 membered fused bicyclic nitrogen containing heterocycloalkyl is each substituted with one R⁶, and optionally substituted with one, two, or three R⁴.
48. The precursor compound of para 47, or a pharmaceutically acceptable salt or solvate thereof, having a formula selected from: 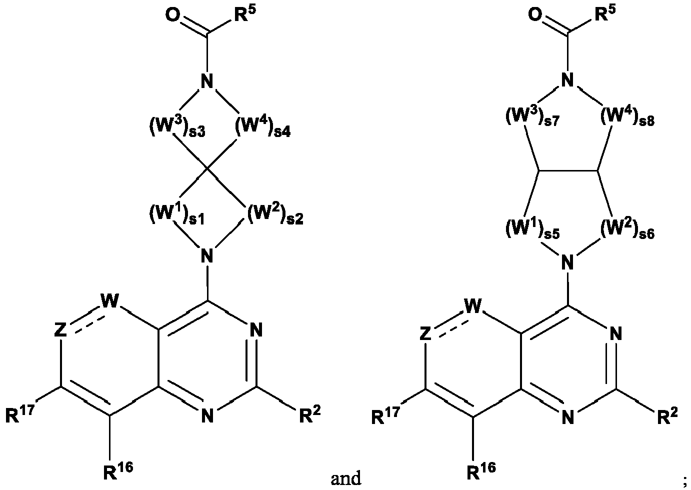 wherein
   W¹, W², W³, and W⁴ are independently selected from N(R⁴), CH₂, CH(R⁴), C(R⁴)(R⁴), C(O), S, O, S(O), and S(O)₂;
   W⁵ is selected from N, C(R¹), and C(R⁴);
   s1 is an integer from 1 to 6; s2 is an integer from 0 to 3; s3 is an integer from 1 to 3; s4 is an integer from 1 to 3; s5 is an integer from 0 to 6; s6 is an integer from 0 to 3; s7 is an integer from 0 to 3; and s8 is an integer from 0 to 3.
49. The precursor compound of para 48, or a pharmaceutically acceptable salt or solvate thereof, wherein
   W¹, W², W³, and W⁴ are independently selected from CH₂, CH(R⁴), C(R⁴)(R⁴), and O;
   W⁵ is selected from N, CH, and C(R⁴);
   s1 is an integer from 1 to 6; s2 is an integer from 0 to 3; s3 is an integer from 1 to 3; s4 is an integer from 1 to 3; s5 is an integer from 0 to 6; s6 is an integer from 0 to 3; s7 is an integer from 0 to 3; and s8 is an integer from 0 to 3.
50. The precursor compound of any one of paras 16-49, or a pharmaceutically acceptable salt or solvate thereof, wherein R⁵ is selected from 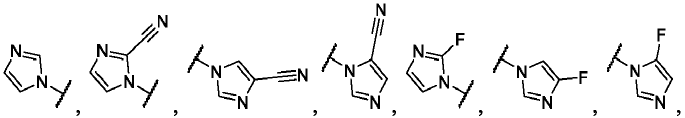 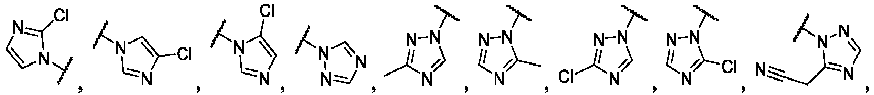 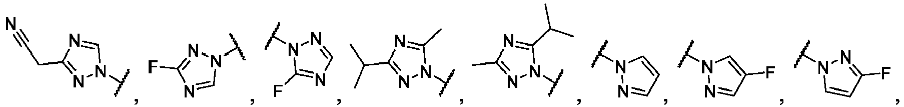 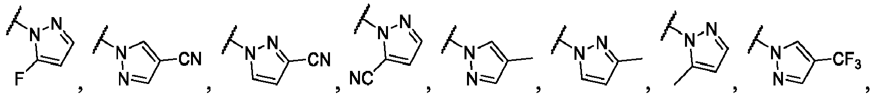 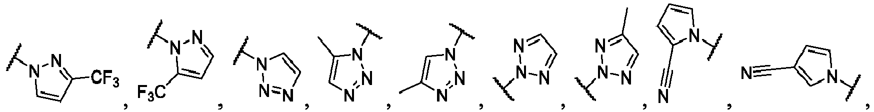 and 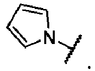
51. The precursor compound of any one of paras 16-50, or a pharmaceutically acceptable salt or solvate thereof, wherein R¹⁷ is selected from 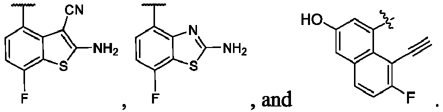
52. The precursor compound of any one of paras 16-51, or a pharmaceutically acceptable salt or solvate thereof, wherein R² is selected from 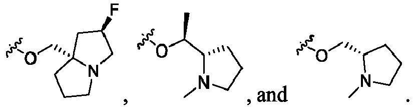
53. A modified Ras mutant protein comprising a compound covalently bonded to its serine residue, wherein the serine residue corresponds to position 12 of SEQ ID No: 1, wherein the covalently bonded compound comprises a staying group having a formula: 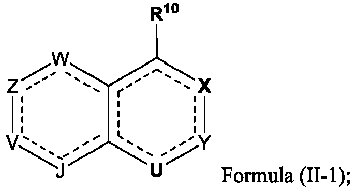 Wherein
   W is a bond, N, C(R¹⁸), N(R^{18b}), C(R¹⁸)(R^{18a}), C(O), S(O), or S(O)₂;
   Z is N, C(R⁸), N(R^{8b}), C(R⁸)(R^{8a}), O, C(O), S(O), or S(O)₂;
   V and J are independently selected from C(R¹⁷), C(R¹⁷)(R^{16a}), C(R¹⁶), C(R¹⁶)(R^{16a}), N, N(R^{17b}), and N(R^{16b});
   Y is N, C(R²), C(R²)(R^{2c}), N(R^{2b}), S(O), S(O)₂, or C(O);
   U is N, C(R^{2c}), C(R^{2c})(R^{2c}), N(R^{2b}), S(O), S(O)₂, or C(O);
   R¹⁰ is -L⁷-R⁷;
   L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C₁₋₃alkyl and C₁₋₄alkyl are optionally substituted with one, two or three R^{20a};
   R⁷ is a 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)-, wherein the 3-12 membered nitrogen containing heterocycloalkyl or 7-12 membered nitrogen containing fused aryl or 5-12 membered nitrogen containing heteroaryl or -(4-6 membered nitrogen containing saturated heterocycloalkyl)-(4-6 membered nitrogen containing saturated heterocycloalkyl or C₃₋₆cycloalkyl)- are substituted with one R⁶, and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, Ci-nheterocycloalkyl, C₆₋₁₂aryl, or C₁₋₁₁heteroaryl, wherein the C₃₋₁₂cycloalkyl, C₁₋nheterocycloalkyl, C₆₋₁₂aryl, or Ci-nheteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroary, -OR¹², -SR¹², -N(R¹²)(R¹³), -C(O)OR¹², - lOC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
   R⁶ is -C₀₋₃alkyl-C(O)-, -C₀₋₃alkyl-N(R¹²)C(O)-, -C₀₋₃alkyl-S(O)-, -C₀₋₃alkyl-N(R¹²)S(O)-, -C₀₋₃alkyl-S(O)₂-, or - C₀₋₃alkyl-N(R¹²)S(O)₂-; wherein R⁶ is directly bonded to the serine residue corresponding to position 12 of SEQ ID No: 1;
   R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-lOC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c};
   R¹⁷ is -L¹-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), - C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
   R^{17b} is -L^{1b}-R¹⁹, hydrogen, halogen, -CN, C₁₋₆alkyl, C₂₋₆alkenyl, C₂₋₆alkynyl, C₃₋₁₀cycloalkyl, C_{2- 9}heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
   L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ;
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - lOC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
   R¹⁶ and R^{16a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³),
   -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20g};
   each R² is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR^{12d}, -SR^{12d}, -N(R^{12d})(R¹³), -N=(R¹⁵), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵,-C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³), -S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), -(C₁-C₆alkyl)-R^{12b}, -(C_{2- 6}alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C_{1- 9}heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   each R^{12d} is independently selected from hydrogen, C₁₋₆alkyl, C₂₋₆alkenyl, C₂₋₆alkynyl, C₃₋₁₀cycloalkyl,-C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl,-C(R^{12e})₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl are optionally substituted with one, two, or three R^{20d};
   each R^{12e} is independently selected from hydrogen and R^{20d};
   R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R20e;
   each R¹⁴ is independently selected from hydrogen, C₁₋₆alkyl, and C₁₋₆haloalkyl;
   each R¹⁵ is independently selected C₁₋₆alkyl, C₂₋₆alkenyl, C₂₋₆alkynyl, C₃₋₁₀cycloalkyl, C₂₋₉heterocycloalkyl, C₆₋ioaryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵,-C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
   R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R20h.
   each R^{20a}, R^{20b}, R^{20c}, R^{20d}, R^{20e}, R^{20f}, R^{20g}, R^{20h}, and R²⁰ⁱ is independently selected from halogen, -CN, oxo, C_{1- 6}alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³),-N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
   - - - - - - indicates a single or double bond such that all valences are satisfied.
54. A precursor compound of Formula Ib-1, or a pharmaceutically acceptable salt or solvate thereof 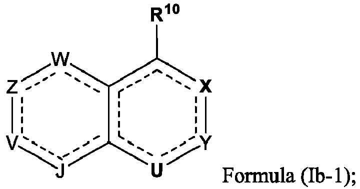 wherein
   W is N, C(R¹⁸), N(R^{18b}), C(R¹⁸)(R^{18a}), C(O), S(O), or S(O)₂;
   Z is N, C(R⁸), N(R^{8b}), C(R⁸)(R^{8a}), O, C(O), S(O), or S(O)₂; wherein W and Z are not both selected from C(O), S(O), and S(O)₂;
   V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
   Y is N, C(R²), C(R²)(R^{2c}), N(R^{2b}), S(O), S(O)₂, or C(O);
   U is N, C(R^{2c}), C(R^{2c})(R^{2c}), N(R^{2b}), S(O), S(O)₂, or C(O);
   R¹⁰ is -L⁷-R⁷;
   L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C₁₋₃alkyl and C₁₋₄alkyl are optionally substituted with one, two or three R^{20a};
   R⁷ is a C₃₋₁₂cycloalkyl, 3-12 membered nitrogen containing heterocycloalkyl, C₆₋₁₂aryl, or 5-12 membered nitrogen containing heteroaryl, wherein the C₃₋₁₂cycloalkyl, 3-12 membered nitrogen containing heterocycloalkyl, C₆₋₁₂aryl, and 5-12 membered nitrogen containing heteroaryl are substituted with one R⁶, and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, Ci-nheterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
   R⁶ is -L²-R⁵;
   each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, -C_{0- 3}alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C_{0- 3}alkyl-, or -C₀₋₃alkyl-N(R¹²)S(O)₂-;
   each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
   R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³),-CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c};
   R¹⁷ is -L¹-R¹⁹;
   R^{17b} is -L^{1b}-R¹⁹;
   L' is selected from a bond, C₁-C₆alkyl, C₂-C₆alkenyl, C₂-C₆alkynyl, -O-, -N(R¹⁴)-, -C(O)-, -N(R¹⁴)C(O)-,-C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-,-N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R¹⁸)N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ;
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹;
   each R¹¹ is independently selected from halogen, oxo, -CN, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², _ OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵,-C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
   R¹⁶ and R^{16a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵,-C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20g};
   R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), -N(R¹⁴)C(O)N(R¹²)(R¹³),-N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³),-N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³),-CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl,-C(R^{12e})₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl are optionally substituted with one, two, or three R^{20d};
   each R^{12e} is independently selected from hydrogen and R^{20d};
   R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), _ N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³),-N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³),-CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹²,-OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵,-C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
   R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20h};
   each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
   - - - - - - indicates a single or double bond such that all valences are satisfied.
55. A precursor compound of Formula Id-1, or a pharmaceutically acceptable salt or solvate thereof: 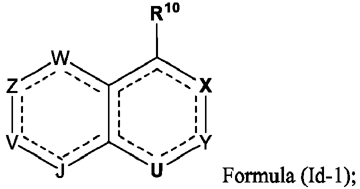 wherein
   W is a bond, N, C(R¹⁸), N(R^{18b}), C(R¹⁸)(R^{18a}), C(O), S(O), or S(O)₂;
   Z is N, C(R⁸), N(R^{8b}), C(R⁸)(R^{8a}), O, C(O), S(O), or S(O)₂; wherein W and Z are not both selected from C(O), S(O), and S(O)₂;
   V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
   Y is N, C(R²), C(R²)(R^{2c}), N(R^{2b}), S(O), S(O)₂, or C(O);
   U is N, C(R^{2c}), C(R^{2c})(R^{2c}), N(R^{2b}), S(O), S(O)₂, or C(O);
   R¹⁰ is -L⁷-R⁷;
   L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C₁₋₃alkyl and C₁₋₄alkyl are optionally substituted with one, two or three- R^{20a};
   R⁷ is a 7-12 membered nitrogen containing spirocyclic heterocycloalkyl substituted with one R⁶, and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
   R⁶ is -L²-R⁵;
   each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, -C_{0- 3}alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C_{0- 3}alkyl-, or -C₀₋₃alkyl-N(R¹²)S(O)₂-;
   each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
   R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c};
   R¹⁷ is -L¹-R¹⁹;
   R^{17b} is -L^{1b}-R¹⁹;
   L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ;
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹ⁱ;
   each R¹ⁱ is independently selected from halogen, oxo, -CN, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
   R¹⁶ and R^{16a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20g};
   R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), -N(R¹⁴)C(O)N(R¹²)(R¹³),-N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O))(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, - C(R^{12e})₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl are optionally substituted with one, two, or three R^{20d};
   each R^{12e} is independently selected from hydrogen and R^{20d};
   R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R20e.
   each R¹⁴ is independently selected from hydrogen, C₁₋₆alkyl, and C₁₋₆haloalkyl;
   each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
   R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20h};
   each R^{20a}, R^{20b}, R^{20c}, R^{20d}, R^{20e}, R^{20f}, R^{20g}, R^{20h}, R²⁰ⁱ, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³),-C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
   ------ indicates a single or double bond such that all valences are satisfied.
56. A precursor compound of Formula Ie-1, or a pharmaceutically acceptable salt or solvate thereof: 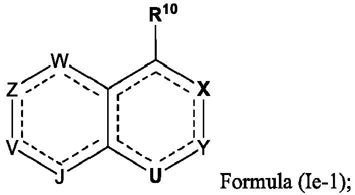 wherein
   W is a bond, N, C(R¹⁸), N(R^{18b}), C(R¹⁸)(R^{18a}), C(O), S(O), or S(O)₂;
   Z is N, C(R⁸), N(R^{8b}), C(R⁸)(R^{8a}), O, C(O), S(O), or S(O)₂;
   V and J are independently selected from C(R¹⁷), C(R¹⁷)(R^{16a}), C(R¹⁶), C(R¹⁶)(R^{16a}), N, N(R^{17b}), and N(R^{16b});
   Y is N, C(R²), C(R²)(R^{2c}), N(R^{2b}), S(O), S(O)₂, or C(O);
   U is N, C(R^{2c}), C(R^{2c})(R^{2c}), N(R^{2b}), S(O), S(O)₂, or C(O);
   R¹⁰ is -L⁷-R⁷;
   L⁷ is a bond, -O-, -N(R¹⁴)-, -C(O)-, -S-, -S(O)₂-, -S(O)-, -N(R¹⁴)-C₁₋₃alkyl-, or C₁₋₄alkyl, wherein the C₁₋₃alkyl and C₁₋₄alkyl are optionally substituted with one, two or three- R^{20a};
   R⁷ is a 7-12 membered nitrogen containing fused heterocycloalkyl, 7-12 membered nitrogen containing fused aryl, or 7-12 membered nitrogen containing fused heteroaryl substituted with one R⁶, and optionally substituted with one or more R¹ and/or one or more R⁴;
   wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C_{1- 11}heterocycloalkyl, C₆₋₁₂aryl, or C₁₋₁₁heteroaryl are optionally substituted with one, two, or three R^{20a};
   each R¹ is independently selected from hydrogen, C₁₋₆alkyl, C₂₋₆alkenyl, C₂₋₆alkynyl, C₁₋₆haloalkyl, C_{3- 12}cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, - CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, - CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C_{1- 11}heteroaryl, and C₁₋₁₁heteroaryl are optionally substituted with one, two, or three R^{20a};
   each R⁴ is independently selected from hydrogen, halogen, oxo, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20a};
   R⁶ is -L²-R⁵;
   each L² is -C₀₋₃alkyl-C(O)O-, -C₀₋₃alkyl-C(O)-, -C₀₋₃alkyl-C(O)N(R¹²)-C₀₋₃alkyl-, -C₀₋₃alkyl-N(R¹²)C(O)-, -C_{0- 3}alkyl-S(O), -C₀₋₃alkyl-S(O)N(R¹²)-C₀₋₃alkyl-, -C₀₋₃alkyl-N(R¹²)S(O), -C₀₋₃alkyl-S(O)₂-, -C₀₋₃alkyl-S(O)₂N(R¹²)-C_{0- 3}alkyl-, or -C₀₋₃alkyl-N(R¹²)S(O)₂-;
   each R⁵ is a 5 to 12 membered partially unsaturated heterocycloalkyl or a 5 to 12 membered heteroaryl, each optionally substituted with one, two, three, four, or five R^{20k}, wherein the partially unsaturated heterocycloalkyl or heteroaryl comprises one, two, three, four, or five ring nitrogen atoms and further wherein when R⁵ is directly bonded to a C(O), S(O), or S(O)₂ of L², L² is directly bonded to an N atom of R⁵;
   R⁸ and R^{8a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20c};
   R^{8b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20c};
   R¹⁷ is -L¹-R¹⁹;
   R^{17b} is -L^{1b}-R¹⁹;
   L¹ is selected from a bond, C₁-C₆alkyl, C₂-C₆alkenyl, C₂-C₆alkynyl, -O-, -N(R¹⁴)-, -C(O)-, -N(R¹⁴)C(O)-, - C(O)N(R¹⁴)-, -S-, -S(O)₂-, -S(O)-, -S(O)₂N(R¹⁴)-, -S(O)N(R¹⁴)-, -N(R¹⁴)S(O)-, -N(R¹⁴)S(O)₂-, -OCON(R¹⁴)-, - N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
   L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
   R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
   R^{1c} is selected from hydrogen, C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R²⁰ⁱ;
   R¹⁹ is selected from a C₃₋₁₂cycloalkyl, C₂₋₁₁heterocycloalkyl, C₆₋₁₂aryl, and C₂₋₁₂heteroaryl, wherein the C_{3- 12}cycloalkyl, C₂₋₁₁heterocycloalkyl, C₆₋₁₂aryl, and C₂₋₁₂heteroaryl are optionally substituted with one, two, three, four, five, six, or seven R¹¹;
   each R¹¹ is independently selected from halogen, oxo, -CN, C₁₋₆alkyl, C₁₋₆haloalkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
   R¹⁶ and R^{16a} are independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20g};
   R^{16b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20g};
   R² is halogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -OR^{12d}, -SR^{12d}, -N(R^{12d})(R¹³), -N=(R¹⁵), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   each R^{12d} is independently selected from hydrogen, C₁₋₆alkyl, C₂₋₆alkenyl, C₂₋₆alkynyl, C₃₋₁₀cycloalkyl, - C(R^{12e})₂-C₃₋₁₀cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, - C(R^{12e})₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -C(R^{12e})₂-C_{3- 10}cycloalkyl, C₂₋₉heterocycloalkyl, -C(R^{12e})₂-C₂₋₉heterocycloalkyl, C₆₋₁₀aryl, -C(R^{12e})₂-C₆₋₁₀aryl, -C(R^{12e})₂-C_{1- 9}heteroaryl, and C₁₋₉heteroaryl are optionally substituted with one, two, or three R^{20d};
   each R^{12e} is independently selected from hydrogen and R^{20d};
   R^{2c} is hydrogen, halogen, -CN, C₁₋₆alkyl, C₂₋₆alkenyl, C₂₋₆alkynyl, C₃₋₁₀cycloalkyl, C₂₋₉heterocycloalkyl, C_{6- 10}aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), - N(R¹⁴)C(O)OR¹⁵, -N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{2b} is hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C_{1- 9}heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C_{2- 6}alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
   R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C_{2- 9}heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   X is C(R³), C(R³)(R³), N(R³), or N;
   each R³ is independently selected from hydrogen, halogen, -CN, C₁₋₆alkyl, C₂₋₆alkenyl, C₂₋₆alkynyl, C_{3- 10}cycloalkyl, C₂₋₉heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20b};
   each R¹² is independently selected from hydrogen, C₁₋₆alkyl, C₂₋₆alkenyl, C₂₋₆alkynyl, C₃₋₁₀cycloalkyl, -CH₂-C_{3- 10}cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C_{1- 9}heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
   each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
   each R¹⁴ is independently selected from hydrogen, C₁₋₆alkyl, and C₁₋₆haloalkyl;
   each R¹⁵ is independently selected C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C_{6- 10}aryl, and C₁₋₉heteroaryl are optionally substituted with one, two, or three R^{20f};
   R¹⁸ and R^{18a} are independently selected from hydrogen, halogen, -CN, C₁₋₆alkyl, C₂₋₆alkenyl, C₂₋₆alkynyl, C_{3- 10}cycloalkyl, C2-9heterocycloalkyl, C₆₋₁₀aryl, C₁₋₉heteroaryl, -OR¹², -SR¹², -N(R¹²)(R¹³), -C(O)OR¹², - OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, and -CH₂S(O)₂N(R¹²)(R¹³), wherein C₁₋₆alkyl, C_{2- 6}alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20h};
   R^{18b} is selected from hydrogen, -CN, C₁₋₆alkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C_{3- 10}cycloalkyl, C₂₋₉heterocycloalkyl, C₆₋₁₀aryl, and C₁₋₉heteroaryl are optionally substituted with one, two, or three R^{20h};
   each R^{20a}, R^{20b}, R^{20c}, R^{20d}, R^{20e}, R^{20f}, R^{20g}, R^{20h}, R²⁰¹, and R^{20k} is independently selected from halogen, -CN, oxo, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C_{2- 9}heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₁₀cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C_{3- 10}cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), - C(O)OR²², -C(O)N(R²²)(R²³), -C(O)C(O)N(R²²)(R²³), -OC(O)N(R²²)(R²³), -N(R²⁴)C(O)N(R²²)(R²³), - N(R²⁴)C(O)OR²⁵, -N(R²⁴)C(O)R²¹, -N(R²⁴)S(O)₂R²⁵, -C(O)R²¹, -S(O)₂R²⁵, -S(O)₂N(R²²)(R²³), and -OC(O)R²⁵;
   each R²¹ is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
   each R²² is independently selected from H, C₁₋₆alkyl, C₁₋₆haloalkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl;
   each R²³ is independently selected from H and C₁₋₆alkyl;
   each R²⁴ is independently selected from H and C₁₋₆alkyl;
   each R²⁵ is independently selected from C₁₋₆alkyl, C₂₋₆alkenyl, C₂₋₆alkynyl, C₃₋₁₀cycloalkyl, C_{2- 9}heterocycloalkyl, C₆₋₁₀aryl, and C₁₋₉heteroaryl; and
   ------ indicates a single or double bond such that all valences are satisfied.
57. The compound or precursor compound of any one of the preceding paras, wherein the compound or precursor compound is a substantially pure single stereoisomer, single atropisomer, or single tautomer.
58. A pharmaceutical composition comprising a precursor compound of any one of paras 16-52 and 54-57, or a pharmaceutically acceptable salt or solvate thereof, and a pharmaceutically acceptable excipient.
59. A method of treating cancer in a subject in need thereof, comprising administering to the subject a therapeutically effective amount of a precursor compound of any one of paras 16-52 and 54-57, or a pharmaceutically acceptable salt or solvate thereof.
60. A method of treating cancer in a subject comprising a Ras G12S mutant protein, the method comprising: modifying the Ras G12S mutant protein of said subject by administering to said subject a precursor compound, wherein precursor compound is characterized in that upon contacting the Ras G12S mutant protein, said Ras G12S mutant protein is modified covalently at a serine residue corresponding to reside 12 of SEQ ID No: 1, such that said modified Ras G12S mutant protein exhibits reduced Ras signaling output.
61. The method of any one of paras 59-60, wherein the cancer is a solid tumor.
62. The method of any one of paras 59-60, wherein the cancer is a hematological cancer.
63. The method of any one of paras 59-62, wherein the precursor compound is a compound of any one of paras 16-52 and 54-56.
64. A method of modulating signaling output of a Ras protein, comprising contacting a Ras protein with an effective amount of a precursor compound of any one of paras 16-52 and 54-57, or a pharmaceutically acceptable salt or solvate thereof, thereby modulating the signaling output of the Ras protein.
65. A method of inhibiting cell growth, comprising administering an effective amount of a precursor compound of one of paras 16-52 and 54-57, or a pharmaceutically acceptable salt or solvate thereof, to a cell expressing a Ras protein, thereby inhibiting growth of said cells.
66. The method of para any one of paras 59-65 comprising administering an additional agent.
67. The method of para 66, wherein the additional agent comprises (1) an inhibitor of MEK; (2) an inhibitor of epidermal growth factor receptor (EGFR) and/or of mutants thereof; (3) an immunotherapeutic agent; (4) a taxane; (5) an anti-metabolite; (6) an inhibitor of FGFR1 and/or FGFR2 and/or FGFR3 and/or of mutants thereof; (7) a mitotic kinase inhibitor; (8) an anti-angiogenic drug; (9) a topoisomerase inhibitor; (10) a platinum-containing compound; (12) an inhibitor of c-MET and/or of mutants thereof; (13) an inhibitor of BCR-ABL and/or of mutants thereof; (14) an inhibitor of ErbB2 (Her2) and/or of mutants thereof; (15) an inhibitor of AXL and/or of mutants thereof; (16) an inhibitor of NTRK1 and/or of mutants thereof; (17) an inhibitor of RET and/or of mutants thereof; (18) an inhibitor of A-Raf and/or B-Raf and/or C-Raf and/or of mutants thereof; (19) an inhibitor of ERK and/or of mutants thereof; (20) an MDM2 inhibitor; (21) an inhibitor of mTOR; (23) an inhibitor of IGF1/2 and/or of IGF1-R; (24) an inhibitor of CDK9; (25) an inhibitor of farnesyl transferase; (26) an inhibitor of SHIP pathway; (27) an inhibitor of SRC; (28) an inhibitor of JAK; (29) a PARP inhibitor, (31) a ROS 1 inhibitor; (32) an inhibitor of SHP pathway, or (33) an inhibitor of Src, FLT3, HDAC, VEGFR, PDGFR, LCK, Bcr-Abl or AKT; (34) an inhibitor of KrasG12C mutant; (35) a SHC inhibitor (e.g., PP2, AID371185); (36) a GAB inhibitor; (38) a PI-3 kinase inhibitor; (39) a MARPK inhibitor; (40) CDK4/6 inhibitor; (41) MAPK inhibitor; (42) SHP2 inhibitor; (43) checkpoint immune blockade agents; (44) or SOS1 inhibitor; or (45) a SOS 2 inhibitor.

## Claims

1. A precursor compound of Formula (Ib), or a pharmaceutically acceptable salt or solvate thereof: 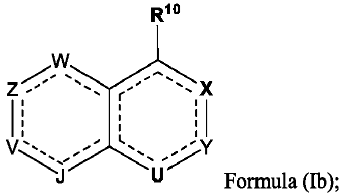 wherein
W is C(R¹⁸), N, N(R^{18b}), C(R¹⁸)(R^{18a}), C(O), S(O), or S(O)₂;
Z is C(R⁸), N, N(R^{8b}), C(R⁸)(R^{8a}), C(O), S(O), or S(O)₂; wherein W and Z are not both selected from C(O), S(O), and S(O)₂;
V and J are selected from C(R¹⁷), C(R¹⁷)(R^{16a}), C(R¹⁶), C(R¹⁶)(R^{16a}), N, N(R^{17b}), and N(R^{16b}); wherein exactly one of V and J is substituted with R¹⁷ or R^{17b};
Y is C(R²), N, C(R²)(R^{2c}), N(R^{2b}), S(O), S(O)₂, or C(O);
U is N, C(R^{2c}), C(R^{2c})(R^{2c}), N(R^{2b}), S(O), S(O)₂, or C(O);
R¹⁰ is -L⁷-R⁷;
L⁷ is a bond, -O-, -N(R¹⁴)-, -C(O)-, -S-, -S(O)₂-, -S(O)-, -N(R^{14c})-C₁₋₃alkyl-, or C₁₋₄alkyl, wherein the C₁₋₃alkyl and C₁₋₄alkyl are optionally substituted with one, two or three R^{20a};
R⁷ is a 6-12 membered nitrogen containing heterocycloalkyl or 6-12 membered nitrogen containing heteroaryl, wherein the 6-12 membered nitrogen containing heterocycloalkyl or 6-12 membered nitrogen containing heteroaryl are substituted with one R⁶ directly bonded to a ring N and optionally substituted with one or more R¹ and/or one or more R⁴;
wherein two substituents selected from R¹ and R⁴ that are bonded to the same or adjacent atoms are optionally joined to form a C₃₋₁₂cycloalkyl, C₁₋₁₁heterocycloalkyl, C₆₋₁₂aryl, or C₁₋₁₁heteroaryl, wherein the C₃₋₁₂cycloalkyl, C₁₋₁₁heterocycloalkyl, C₆₋₁₂aryl, or C₁₋₁₁heteroaryl are optionally substituted with one, two, or three R^{20a};
each R¹ is independently selected from hydrogen, C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, - CH₂-C₆₋₁₂aryl, -CH₂-C₁₋₁₁heteroaryl, and C₁₋₁₁heteroaryl, wherein C₁₋₆alkyl, C₂₋₆alkenyl, C₂₋₆alkynyl, C₁₋₆haloalkyl, C₃₋₁₂cycloalkyl, -CH₂-C₃₋₁₂cycloalkyl, C₁₋₁₁heterocycloalkyl, -CH₂-C₁₋₁₁heterocycloalkyl, C₆₋₁₂aryl, -CH₂-C₆₋₁₂aryl, -CH₂-C₁₋₁₁heteroaryl, and C₁₋₁₁heteroaryl are optionally substituted with one, two, or three R^{20a};
each R⁴ is independently selected from hydrogen, halogen, oxo, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20a};
R⁶ is -L²-R⁵;
each L² is -C(O)- or -C(O)N(R¹⁴)-;
each R⁵ is a 5- or 6-membered heteroaryl or partially unsaturated heterocycloalkyl, optionally substituted with one, two or three R^{20k}, wherein the partially unsaturated heterocycloalkyl or heteroaryl comprises one, two, or three ring nitrogen atoms; and when L² is -C(O)-, R⁵ is a 5-membered heteroaryl or partially unsaturated heterocycloalkyl, optionally substituted with one, two or three R^{20k}, bonded to L² through a ring nitrogen; and when L² is -C(O)N(R¹⁴)-, the N of L² is directly bonded to R⁵ and R⁵ is a 5- or 6-membered heteroaryl or partially unsaturated heterocycloalkyl, optionally substituted with one, two or three R^{20k}, bonded to L² through a ring carbon;
R⁸ and R^{8a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(H)(R¹²), - C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R^{8b} is selected from hydrogen, -CN, C₁₋₆alkyl, C₂₋₆alkenyl, C₂₋₆alkynyl, C₃₋₆cycloalkyl, C₂₋₉ heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20c};
R¹⁷ is -L¹-R¹⁹;
R^{17b} is -L^{1b}-R¹⁹;
L¹ is selected from a bond, C₁-C₆alkyl, C₂-C₆alkenyl, C₂-C₆alkynyl, -O-, -N(R¹⁴)-, -C(O)-, - N(R¹⁴)C(O)-, -C(O)N(R¹⁴)-, -S-, -S(O)₂-, -S(O)-, -S(O)₂N(R¹⁴)-, -S(O)N(R¹⁴)-, -N(R¹⁴)S(O)-, - N(R¹⁴)S(O)₂-, -OCON(R¹⁴)-, -N(R¹⁴)C(O)O-, N(R^{1e}), C(O)N(R^{1c}), S(O)₂N(R^{1c}), S(O)N(R^{1c}), C(R^{1f})(R^{1g})O, C(R^{1f})(R^{1g})N(R^{1c}), and C(R^{1f})(R^{1g});
L^{1b} is selected from a bond, C₁-C₆alkyl, C₂-C₆alkenyl, C₂-C₆alkynyl, -C(O)-, -C(O)N(R¹⁴)-, C(O)N(R^{1c}), C(R^{1f})(R^{1g})O, C(R^{1f})(R^{1g})N(R^{1c}), and C(R^{1f})(R^{1g});
R^{1e}, R^{1f}, and R^{1g} are independently selected from hydrogen, halogen, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², - N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ;
R^{1c} is selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁹ is selected from a C₃₋₁₂cycloalkyl, C₂₋₁₁heterocycloalkyl, C₆₋₁₂aryl, and C₂₋₁₂heteroaryl, wherein the C₃₋₁₂cycloalkyl, C₂₋₁₁heterocycloalkyl, C₆₋₁₂aryl, and C₂₋₁₂heteroaryl are optionally substituted with one, two, three, four, five, six, or seven R¹ⁱ;
each R¹ⁱ is independently selected from halogen, oxo, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ;
R¹⁶ and R^{16a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R^{16b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20g};
R² is halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2c} is hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), - N(R¹⁴)C(O)N(R¹²)(R¹³), -N(R¹⁴)C(O)OR¹⁵, -N(R¹⁴)S(O)₂R¹⁵, -C(O)R¹⁵, -S(O)R¹⁵, -OC(O)R¹⁵, - C(O)N(R¹²)(R¹³), -C(O)C(O)N(R¹²)(R¹³), -N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, -CH₂S(O)₂R¹⁵, - CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{2b} is hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), -C(O)C(O)N(R¹²)(R¹³), - CH₂C(O)N(R¹²)(R¹³), -CH₂N(R¹⁴)C(O)R¹⁵, -CH₂S(O)₂R¹⁵, -CH₂S(O)₂N(R¹²)(R¹³), -(C₁-C₆alkyl)-R^{12b}, -(C₂₋₆alkenyl)-R^{12b}, -(C₂₋₆alkynyl)-R^{12b}, -(C₃₋₁₀cycloalkyl)-R^{12b}, -(C₂₋₉heterocycloalkyl)-R^{12b}, -(C₆₋₁₀aryl)-R^{12b}, or -(C₁₋₉heteroaryl)-R^{12b}, wherein said C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R^{20d};
R^{12b} is selected from hydrogen, C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl, wherein C₁₋₆alkyl, C₂₋₆alkenyl, C₂₋₆alkynyl, C₃₋₁₀cycloalkyl, -CH₂-C₃₋₁₀cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three R^{20d};
X is N, C(R³), C(R³)(R³), or N(R³);
each R³ is independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, -C(O)R¹⁵, - S(O)R¹⁵, -OC(O)R¹⁵, -C(O)N(R¹²)(R¹³), -C(O)C(O)N(R¹²)(R¹³), -N(R¹⁴)C(O)R¹⁵, -S(O)₂R¹⁵, - S(O)₂N(R¹²)(R¹³)-, S(=O)(=NH)N(R¹²)(R¹³), -CH₂C(O)N(R¹²)(R¹³), -CH₂N(R¹⁴)C(O)R¹⁵, - CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20b};
each R¹² is independently selected from hydrogen, C₁₋₆alkyl, C₂₋₆alkenyl, C₂₋₆alkynyl, C₃₋₆cycloalkyl, - CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl, wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, - CH₂-C₁₋₉heteroaryl, and C₁₋₉heteroaryl are optionally substituted with one, two, or three R^{20d};
each R¹³ is independently selected from hydrogen, C₁₋₆alkyl, and C₁₋₆haloalkyl; or R¹² and R¹³, together with the nitrogen to which they are attached, form a C₂₋₉heterocycloalkyl ring optionally substituted with one, two, or three R^{20e};
each R¹⁴ is independently selected from hydrogen, C₁₋₆alkyl, and C₁₋₆haloalkyl;
each R^{14c} is independently selected from C₁₋₆alkyl and C₁₋₆haloalkyl;
each R¹⁵ is independently selected C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20f};
R¹⁸ and R^{18a} are independently selected from hydrogen, halogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², -SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, -N(R¹⁴)S(O)₂R¹⁵, - C(O)R¹⁵, -S(O)R¹⁵, -OC(O)R¹⁵, -C(O)N(R¹²)(R¹³), -C(O)C(O)N(R¹²)(R¹³), -N(R¹⁴)C(O)R¹⁵, - S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
R^{18b} is selected from hydrogen, -CN, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -C(O)OR¹², -C(O)R¹⁵, -C(O)N(R¹²)(R¹³), - C(O)C(O)N(R¹²)(R¹³), -CH₂C(O)N(R¹²)(R¹³), -CH₂N(R¹⁴)C(O)R¹⁵, -CH₂S(O)₂R¹⁵, and - CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R^{20h};
each R^{20a}, R^{20b}, R^{20c}, R^{20d}, R^{20e}, R^{20f}, R^{20g}, R^{20h}, R²⁰ⁱ, and R^{20k} are each independently selected from halogen, -CN, C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, C₁₋₉heteroaryl, -OR²¹, -SR²¹, -N(R²²)(R²³), -C(O)OR²², -C(O)N(R²²)(R²³), -C(O)C(O)N(R²²)(R²³), - OC(O)N(R²²)(R²³), -N(R²⁴)C(O)N(R²²)(R²³), -N(R²⁴)C(O)OR²⁵, -N(R²⁴)C(O)R²⁵, -N(R²⁴)S(O)₂R²⁵, -C(O)R²⁵, -S(O)₂R²⁵, -S(O)₂N(R²²)(R²³), -OCH₂C(O)OR²², and -OC(O)R²⁵; or two R^{20k} bonded to the same or adjacent atoms may optionally be joined to form a C₃₋₆cycloalkyl, C₂₋₉heterocycloalkyl, C₆₋₁₀aryl, -CH₂-C₆₋₁₀aryl, or C₁₋₉heteroaryl; wherein C₁₋₆alkyl, C₂₋₆alkenyl, C₂₋₆alkynyl, C₃₋₆cycloalkyl, -CH₂-C₃₋₆cycloalkyl, C₂₋₉heterocycloalkyl, -CH₂-C₂₋₉heterocycloalkyl, C₆₋₁₀aryl, -CH₂-C₆₋₁₀aryl, -CH₂-C₁₋₉heteroaryl, and C₁₋₉heteroaryl are optionally substituted with one, two, or three groups independently selected from halogen, oxo, -CN, C₁₋₆alkyl, C₁₋₆haloalkyl, C₁₋₆alkoxy, C₁₋₆haloalkoxy, -OR²¹, -SR²¹, -N(R²²)(R²³), -C(O)OR²², -C(O)N(R²²)(R²³), - C(O)C(O)N(R²²)(R²³), -OC(O)N(R²²)(R²³), -N(R²⁴)C(O)N(R²²)(R²³), -N(R²⁴)C(O)OR²⁵, - N(R²⁴)C(O)R²⁵, -N(R²⁴)S(O)₂R²⁵, -C(O)R²⁵, -S(O)₂R²⁵, -S(O)₂N(R²²)(R²³), and -OC(O)R²⁵;
each R²¹ is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²² is independently selected from H, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, and C₁₋₉heteroaryl;
each R²³ is independently selected from H and C₁₋₆alkyl;
each R²⁴ is independently selected from H and C₁₋₆alkyl;
each R²⁵ is independently selected from C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl; and
- - - - - - indicates a single or double bond such that all valences are satisfied.

2. The precursor compound of claim 1, or a pharmaceutically acceptable salt or solvate thereof, wherein
L⁷ is a bond, -O-, -N(R¹⁴)-, -C(O)-, -S-, -S(O)₂-, -S(O)-, or C₁₋₄alkyl, wherein the C₁₋₄alkyl is optionally substituted with one, two or three R^{20a}; and
L² is -C(O)-.

3. The precursor compound of any one of claims 1-2, or a pharmaceutically acceptable salt or solvate thereof, wherein R⁷ is a 7-12 membered nitrogen containing spirocyclic heterocycloalkyl substituted with one R⁶ directly bonded to a ring N and optionally substituted with one or more R¹ and/or one or more R⁴.

4. The precursor compound of any one of claims 1-2, wherein R⁷ is a 7-12 membered nitrogen containing fused heterocycloalkyl or 7-12 membered nitrogen containing fused heteroaryl substituted with one R⁶ directly bonded to a ring N and optionally substituted with one or more R¹ and/or one or more R⁴.

5. The precursor compound of any one of claims 1-4, wherein R¹⁹ is: 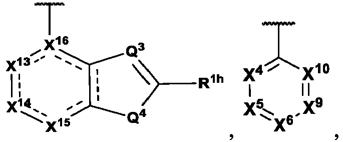 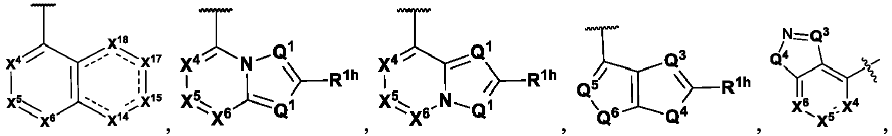 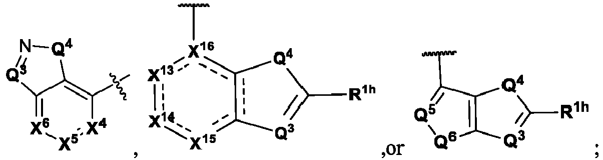
Q¹, Q³, and Q⁵ are independently selected from N and C(R^{1d});
Q⁴ and Q⁶ are independently selected from O, S, C(R^{1a})(R^{1b}), and N(R^{1c});
X⁴, X⁵, X⁶, X⁹, X¹⁰ are independently selected from C(R^{1a}) and N;
X¹³ is selected from a bond, C(R^{1a}), N, C(O), C(R^{1a})(R^{1b}), C(O)C(R^{1a})(R^{1b}), C(R^{1a})(R^{1b})C(R^{1a})(R^{1b}), C(R^{1a})(R^{1b})N(R^{1c}), and N(R^{1c});
X¹⁴, X¹⁵, X¹⁷, X¹⁸ are independently selected from a C(O), C(R^{1a}), N, C(R^{1a})(R^{1b}), and N(R^{1c});
X¹⁶ are independently selected from C, N, and C(R^{1a});
each R^{1a}, R^{1b}, R^{1d}, and R^{1h} are each independently selected from hydrogen, halogen, -CN, C₁₋₆alkyl, C₁₋₆haloalkyl, C₂₋₆alkenyl, C₂₋₆alkynyl, C₃₋₁₀cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ; or R^{1a} and R^{1b} bonded to the same carbon are joined to form a 3-10 membered heterocycloalkyl ring or a C₃₋₁₀cycloalkyl ring, wherein the 3-10 membered heterocycloalkyl ring or C₃₋₁₀cycloalkyl ring are optionally substituted with one, two, or three R²⁰ⁱ; or two R^{1a} bonded to adjacent atoms are joined to form a 3-10 membered heterocycloalkyl ring, a C₆₋₁₀aryl ring, a 5-12 membered heteroaryl ring, or a C₃₋₁₀cycloalkyl ring, wherein the 3-10 membered heterocycloalkyl ring, C₆₋₁₀aryl ring, 5-12 membered heteroaryl ring, or C₃₋₁₀cycloalkyl ring are optionally substituted with one, two, or three R²⁰ⁱ; or R^{1h} and one of R^{1a}, R^{1b}, R^{1c}, and R^{1d} bonded to adjacent atoms are joined to form a 3-10 membered heterocycloalkyl ring, a C₆₋₁₀aryl ring, a 5-12 membered heteroaryl ring, or a C₃₋₁₀cycloalkyl ring, wherein the 3-10 membered heterocycloalkyl ring, a C₆₋₁₀aryl ring, a 5-12 membered heteroaryl ring, and C₃₋₁₀cycloalkyl ring are optionally substituted with one, two, or three R²⁰ⁱ; and
each R^{1c} is independently selected from hydrogen, C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl, wherein C₁₋₆alkyl, C₂₋₆alkenyl, C₂₋₆alkynyl, C₃₋₁₀cycloalkyl, C₂₋₉heterocycloalkyl, C₆₋₁₀aryl, and C₁₋₉heteroaryl are optionally substituted with one, two, or three R²⁰ⁱ;
optionally wherein R¹⁹ is selected from: 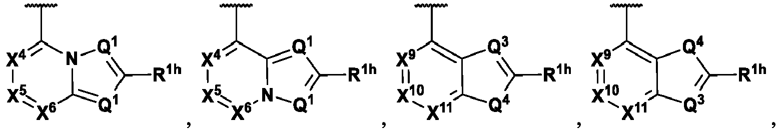 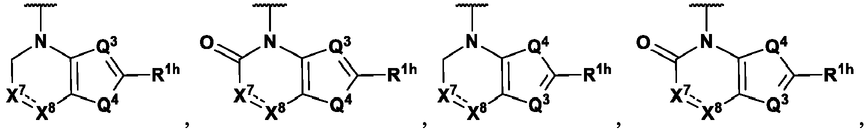 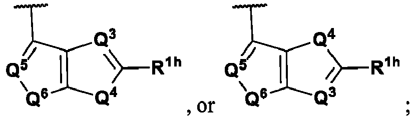
Q¹, Q³, and Q⁵ are independently N or C(R^{1d});
Q⁴ and Q⁶ are independently O, S, C(R^{1a})(R^{1b}), or N(R^{1c});
X⁴, X⁵, X⁶, X⁹, X¹⁰, and X¹¹ are independently selected from C(R^{1a}) or N;
X⁷ and X⁸ are independently selected from C(R^{1a}), C(R^{1a})(R^{1b}), N, or N(R^{1c});
each R^{1a}, R^{1b}, R^{1d}, R^{1f}, R^{1g}, and R^{1h} are independently selected from hydrogen, halogen, -CN, C₁₋₆alkyl, C₁₋₆haloalkyl, C₂₋₆alkenyl, C₂₋₆alkynyl, C₃₋₆cycloalkyl, C₂₋₉heterocycloalkyl, C₆₋₁₀aryl, C₁₋₉heteroaryl, -OR¹², - SR¹², -N(R¹²)(R¹³), -C(O)OR¹², -OC(O)N(R¹²)(R¹³), -N(R¹⁴)C(O)N(R¹²)(R¹³), -N(R¹⁴)C(O)OR¹⁵, - N(R¹⁴)S(O)₂R¹⁵, -C(O)R¹⁵, -S(O)R¹⁵, -OC(O)R¹⁵, -C(O)N(R¹²)(R¹³), -C(O)C(O)N(R¹²)(R¹³), - N(R¹⁴)C(O)R¹⁵, -S(O)₂R¹⁵, -S(O)₂N(R¹²)(R¹³)-, S(=O)(=NH)N(R¹²)(R¹³), -CH₂C(O)N(R¹²)(R¹³), - CH₂N(R¹⁴)C(O)R¹⁵, -CH₂S(O)₂R¹⁵, and -CH₂S(O)₂N(R¹²)(R¹³), wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ; or R^{1a} and R^{1b} bonded to the same carbon are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; or two R^{1a} bonded to adjacent atoms are joined to form a 4-7 membered heterocycloalkyl ring, a phenyl ring, a 5-6 membered heteroaryl ring, or a 4-7 membered cycloalkyl ring, wherein the 4-7 membered heterocycloalkyl ring, phenyl ring, 5-6 membered heteroaryl ring, or 4-7 membered cycloalkyl ring are optionally substituted with one, two, or three R²⁰ⁱ; or R^{1h} and one of R^{1a}, R^{1b}, R^{1c}, and R^{1d} bonded to adjacent atoms are joined to form a 4-7 membered heterocycloalkyl ring, a phenyl ring, a 5-6 membered heteroaryl ring, or a 4-7 membered cycloalkyl ring, wherein the 4-7 membered heterocycloalkyl ring, phenyl ring, 5-6 membered heteroaryl ring, or 4-7 membered cycloalkyl ring are optionally substituted with one, two, or three R²⁰ⁱ; or R^{1f} and R^{1g} are joined to form a 4-7 membered heterocycloalkyl ring or a 4-7 membered cycloalkyl ring, wherein the 4-7 membered heterocycloalkyl ring or 4-7 membered cycloalkyl ring are optionally substituted with one, two, or three R²⁰ⁱ; and
each R^{1c} is independently selected from hydrogen, C₁₋₆alkyl, C₂₋₆alkenyl, C₂₋₆alkynyl, C₃₋₆cycloalkyl, C₂₋₉heterocycloalkyl, C₆₋₁₀aryl, C₁₋₉heteroaryl, wherein C₁₋₆alkyl, C₂₋₆alkenyl, C₂₋₆alkynyl, C₃₋₆cycloalkyl, C₂₋₉heterocycloalkyl, C₆₋₁₀aryl, and C₁₋₉heteroaryl are optionally substituted with one, two, or three R²⁰ⁱ.

6. The precursor compound of any one of the preceding claims, or a pharmaceutically acceptable salt or solvate thereof, wherein R¹⁹ is selected from:

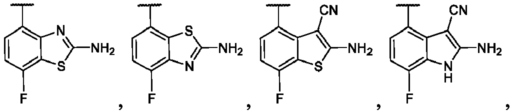

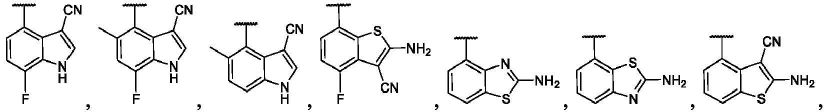

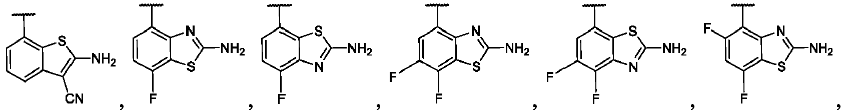

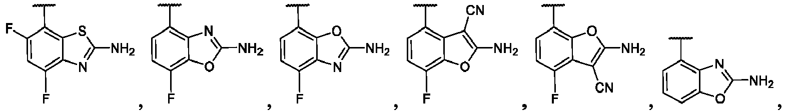

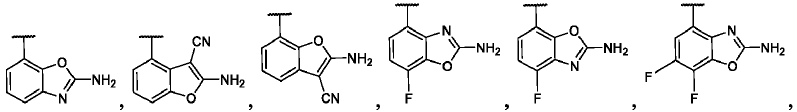

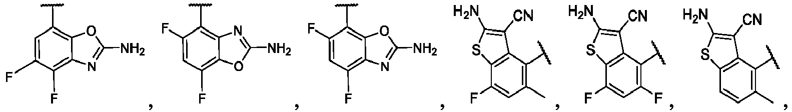

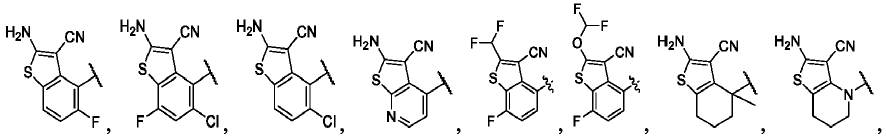

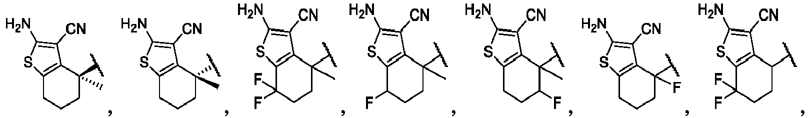

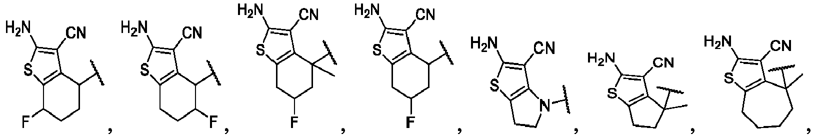

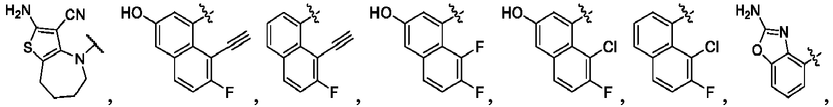

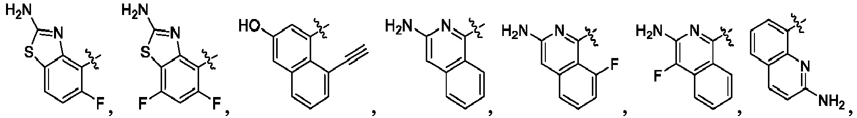

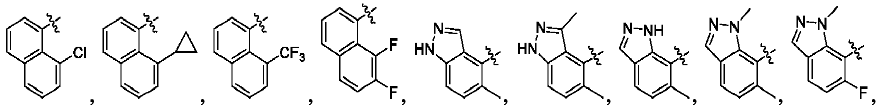

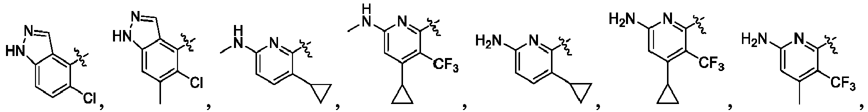

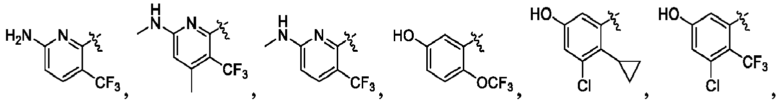

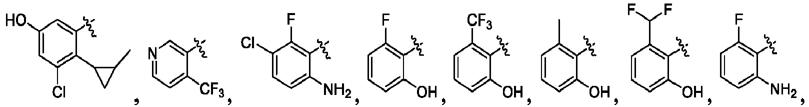

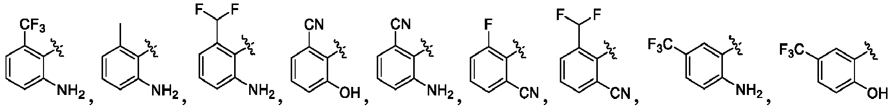

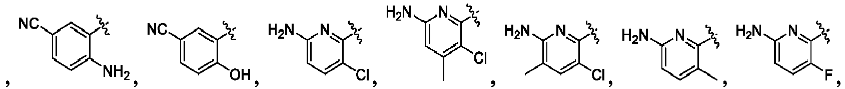

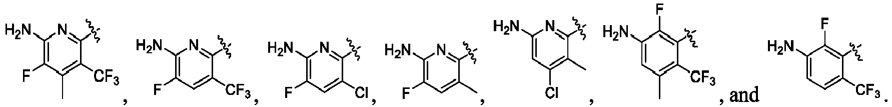

7. The precursor compound of any one of the preceding claims, or a pharmaceutically acceptable salt or solvate thereof, wherein R² is selected from

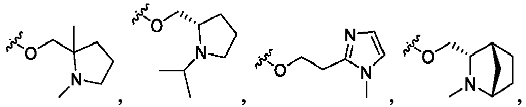

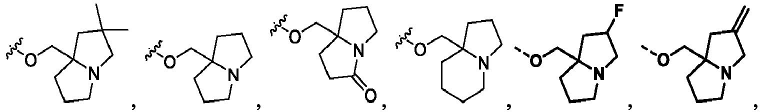

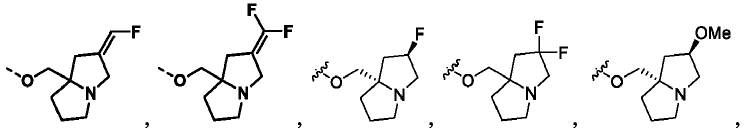

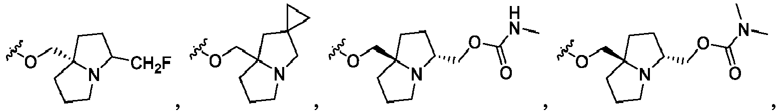

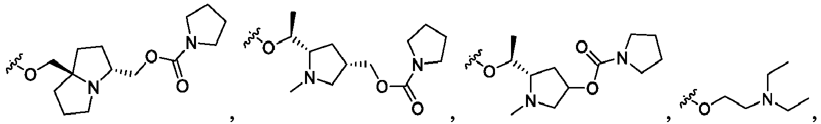

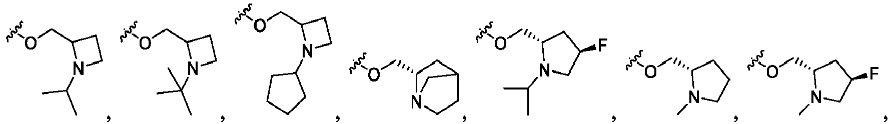

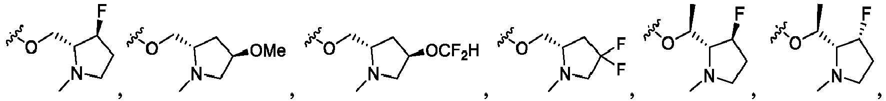

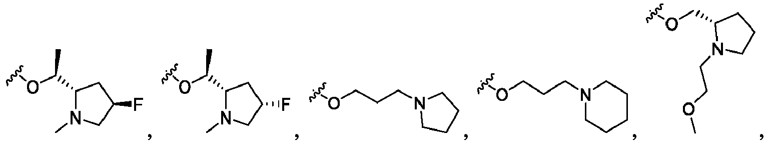

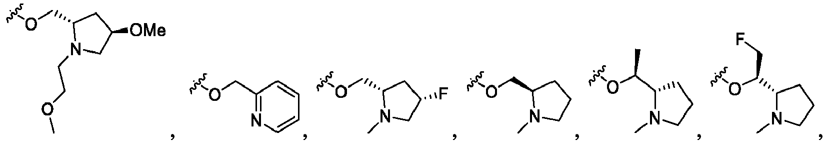

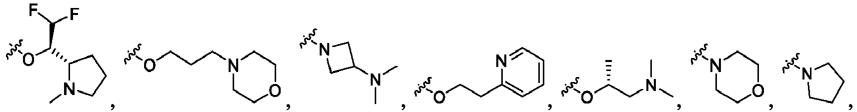

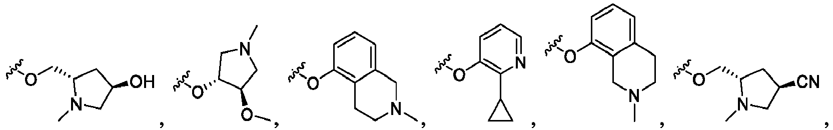

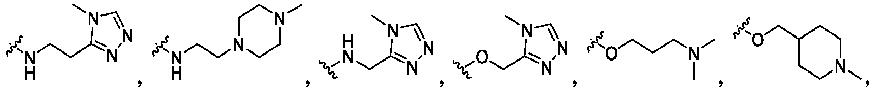

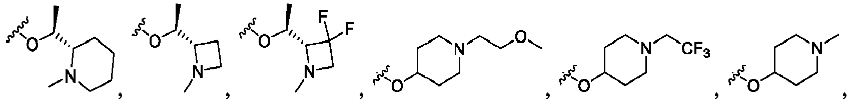

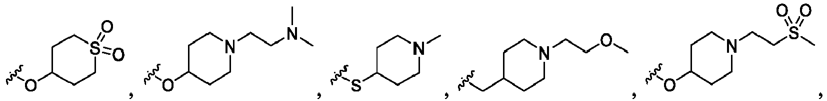

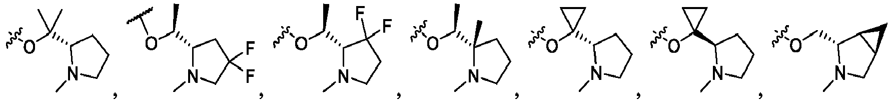

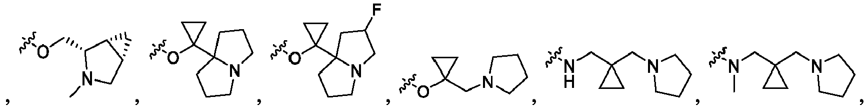

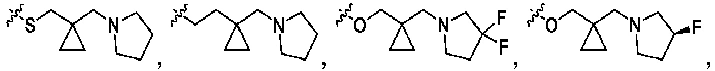

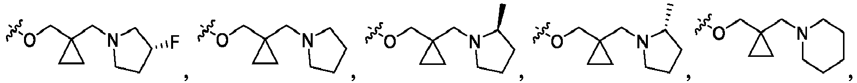

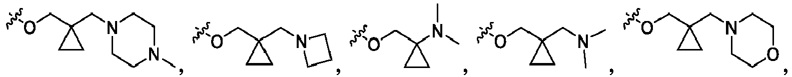

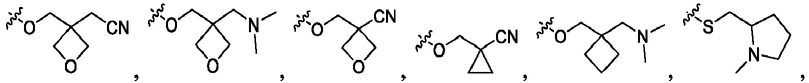

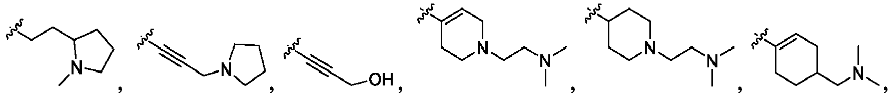

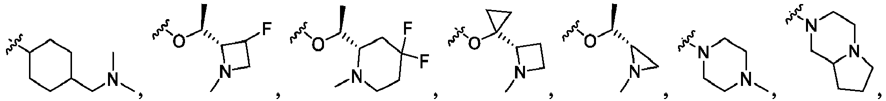

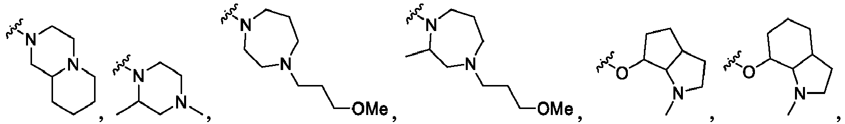

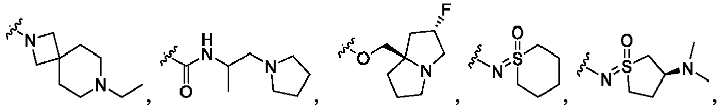

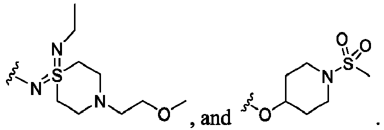

8. The precursor compound of any one of claims 1-2, or a pharmaceutically acceptable salt or solvate thereof, having the formula (I-li): 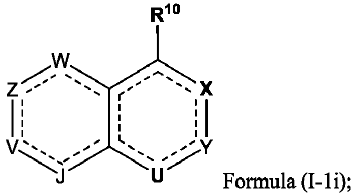 wherein
L⁷ is a bond, -O-, or -NH-;
R⁷ is a 6-12 membered nitrogen containing heterocycloalkyl having a formula selected from: 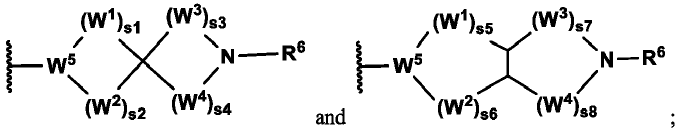
W¹, W², W³, and W⁴ are independently selected from N(R¹), N(R⁴), C(R¹)(R¹), C(R¹)(R⁴), C(R⁴)(R⁴), C(O), S, O, S(O), and S(O)₂;
W⁵ is selected from N, C(R¹), and C(R⁴);
s1 is an integer from 1 to 6; s2 is an integer from 0 to 3; s3 is an integer from 1 to 3; s4 is an integer from 1 to 3;
s5 is an integer from 0 to 6; s6 is an integer from 0 to 3; s7 is an integer from 0 to 3; s8 is an integer from 0 to 3;
R⁶ is -L²-R⁵;
each L² is -C(O)-;
R⁵ is a 5-membered heteroaryl, optionally substituted with one, two, or three R^{20k}, wherein the 5-membered heteroaryl comprises one, two, or three ring nitrogen atoms and further wherein when R⁵ is directly bonded to a C(O) of L², L² is directly bonded to an N atom of R⁵;
L¹ is a bond, L^{1b} is a bond; and
R¹⁹ is selected from a C₆₋₁₂aryl and C₂₋₁₂heteroaryl, wherein the C₆₋₁₂aryl and C₂₋₁₂heteroaryl are optionally substituted with one, two, three, four, five, six, or seven R¹ⁱ.

9. The precursor compound of claim 8, or a pharmaceutically acceptable salt or solvate thereof, wherein W is C(R¹⁸) or C(O); Z is N, C(R⁸), or N(R^{8b}); V is C(R¹⁷); J is C(R¹⁶); Y is C(R²); U is N; and X is N.

10. The precursor compound of claim 8, or a pharmaceutically acceptable salt or solvate thereof, wherein W is CH; Z is C(R⁸) or N(R^{8b}); V is C(R¹⁷); J is C(R¹⁶); Y is C(R²); U is N; and X is N.

11. The precursor compound of any one of claims 1-2 or 8-10, or a pharmaceutically acceptable salt or solvate thereof, wherein R⁷ is selected from

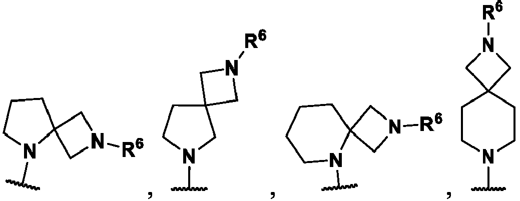

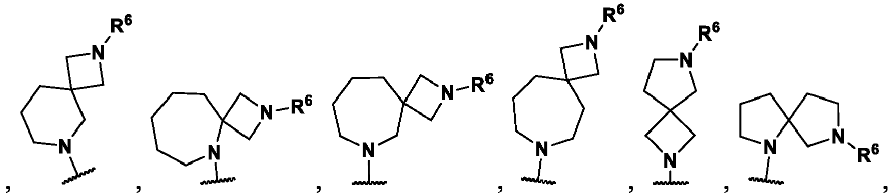

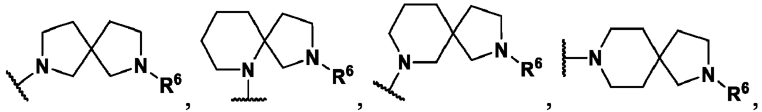

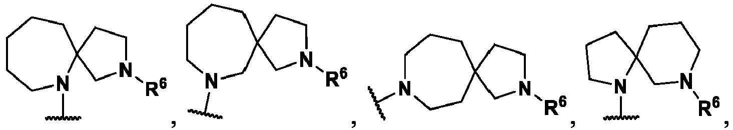

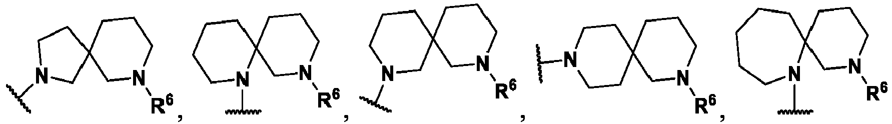

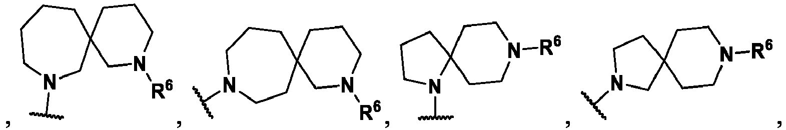

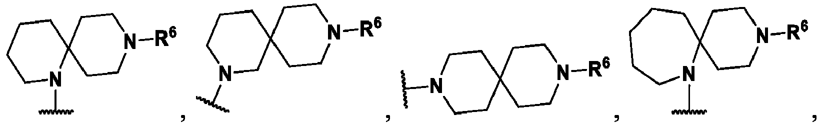

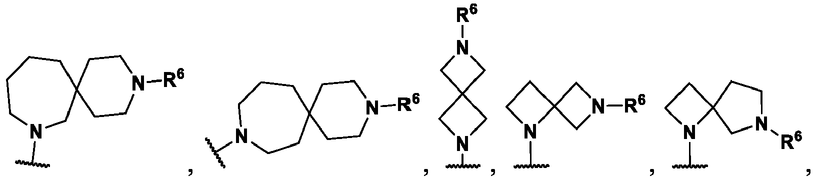

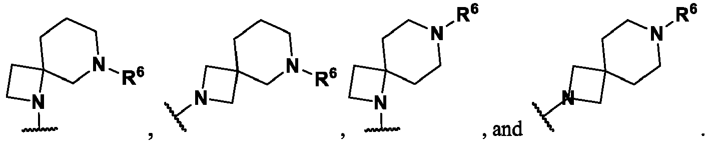

12. The precursor compound of claim 8, or a pharmaceutically acceptable salt or solvate thereof, having a formula selected from: 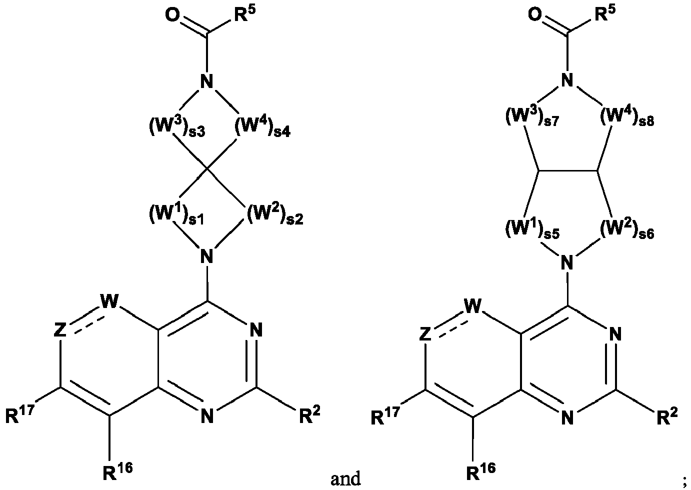 wherein
W¹, W², W³, and W⁴ are independently selected from N(R⁴), CH₂, CH(R⁴), C(R⁴)(R⁴), C(O), S, O, S(O), and S(O)₂;
W⁵ is selected from N, C(R¹), and C(R⁴);
s1 is an integer from 1 to 6; s2 is an integer from 0 to 3; s3 is an integer from 1 to 3; s4 is an integer from 1 to 3; s5 is an integer from 0 to 6; s6 is an integer from 0 to 3; s7 is an integer from 0 to 3; and s8 is an integer from 0 to 3.

13. The precursor compound of any one of claims 1-12, or a pharmaceutically acceptable salt or solvate thereof, wherein R⁵ is selected from

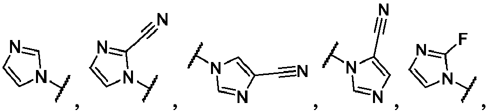

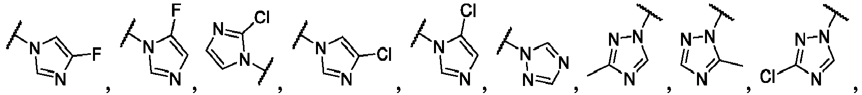

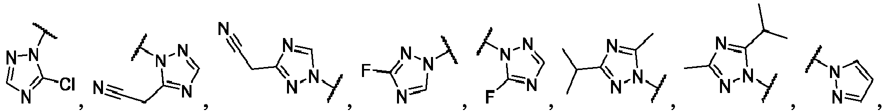

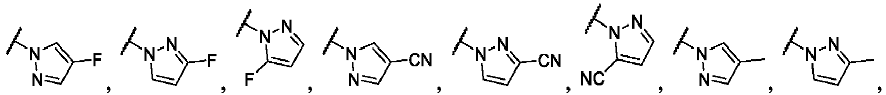

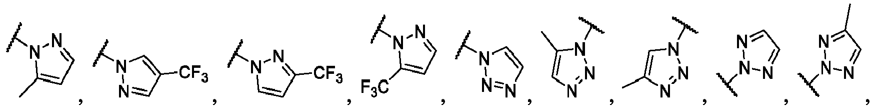

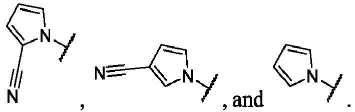

14. The precursor compound of any one of claims 1-12, or a pharmaceutically acceptable salt or solvate thereof, wherein R⁶ is selected from

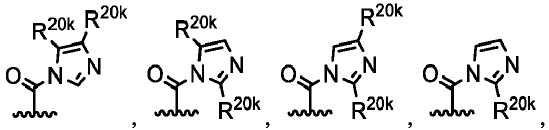

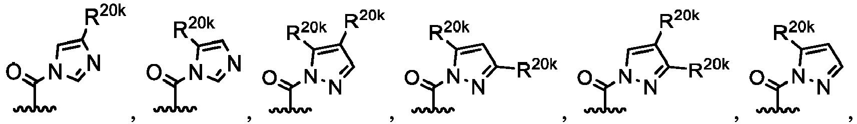

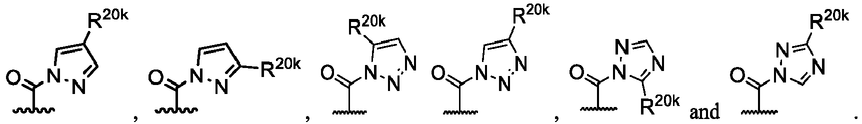

15. The precursor compound of any one of claims 1-14, or a pharmaceutically acceptable salt or solvate thereof, wherein said precursor compound is capable of reacting with a serine residue of a Ras mutant protein and covalently modifying said Ras mutant; optionally wherein said serine residue of a Ras mutant protein corresponds to serine residue 12 of SEQ ID NO. 1, 3, or 5.

16. A pharmaceutical composition comprising a precursor compound of any one of claims 1-15, or a pharmaceutically acceptable salt or solvate thereof, and a pharmaceutically acceptable excipient.

17. A precursor compound of any one of claims 1-15, or a pharmaceutically acceptable salt or solvate thereof, for use in treating cancer.

18. A precursor compound of any one of claims 1-14, or a pharmaceutically acceptable salt or solvate thereof for use in treating cancer in a subject comprising a Ras G12S mutant protein, wherein:
a. the precursor compound is **characterized in that** upon contacting the Ras G12S mutant protein, said Ras G12S mutant protein is modified covalently at a serine residue corresponding to reside 12 of SEQ ID No: 1; and
b. said modified Ras G12S mutant protein exhibits reduced Ras signaling output.
